# EUROPEAN PATENT APPLICATION

(11) **EP 1 770 171 A1**
(43) Date of publication of application: **04.04.2007**
(21) Application number: 05109025.6
(22) Date of filing: 29.09.2005
(51) Int. Cl.: C12Q 1/68

(54) **DNA microarray for rapid identification of Candida albicans in blood cultures.**

(71) Applicant: UNIVERSITÄT ZU KÖLN, D-50923 Köln (DE)
(72) Inventor: Krut, Oleg, 50937 Köln (DE); Palka-Santini, Marie, 50858 Köln (DE); Cleven, Berit, 53919 Weilerswist (DE); Krönke, Martin, 50968 Köln (DE)
(74) Representative: Helbing, Jörg

(57) **Abstract**

The present invention provides a DNA microarray for identification and characterisation of microorganisms in a sample or clinical specimen. Furthermore, it provides for a method for rapid identification and strain profiling of different microbial species in clinical specimens, especially in blood cultures, utilizing said DNA microarray.

SEQUENCE LISTING

<110>  Universität zu Köln

<120>  DNA microarray for rapid identification of pathogens in blood
       cultures

<130>  050565ep

<160>  2841

<170>  PatentIn version 3.3

<210>  1
<211>  220
<212>  DNA
<213>  Staphylococcus aureus

<400>  1
taaattgttt agattacaat cagaggacgc taaagaaaga atttttacaa atacagcaaa      60

tgcaatggaa ggcgtaacgg atgatgttaa acgacgtcat attcgtcatt gttacaaagc     120

tgacccagaa tatggtaaag gtgttgcaaa agcattaggt attgatataa attctattga     180

tcttgaaact gaaaatgatg aaacatacga aaactttgaa                           220


<210>  2
<211>  543
<212>  DNA
<213>  Staphylococcus aureus

<400>  2
cgtgtttggg ttaaattcca ttttagaacg caacaaggta ttgaaaactt aactgatgaa      60

gaagctgctg aaattatagc tacagatcgt gattcatctc aacgcgattt attcgaagcc     120

attgaaaaag gtgattatcc aaaatggaca atgtatattc aagtaatgac tgaggaacaa     180

gctaaaaacc ataaagataa tccatttgat ttaacaaaag tatggtatca cgatgagtat     240

cctctaattg aagttggaga gtttgaatta aatagaaatc cagataatta ctttacggat     300

gttgaacaag ctgcgtttgc accaactaat attattccag gattagattt ttctccagac     360

aaaatgctgc aagggcgttt attctcatat ggcgatgcgc aaagatatcg attaggagtt     420

aatcattggc agattcctgt aaaccaacct aaaggtgttg gtattgaaaa tatttgtcct     480

tttagtagag atggtcaaat gcgcgtagtt gacaataacc aaggtggagg aacacaccat     540

tat                                                                   543


<210>  3
<211>  701
<212>  DNA
<213>  Staphylococcus aureus

<400>  3
aaaagaaaaa cacgcaattc ggaaaaaatc gattggcgtg gcttcagtgc ttgtaggtac      60

gttaatcggt tttggactac tcagcagtaa agaagcagat gcaagtgaaa atagtgttac 120

gcaatctgat agcgcaagta acgaaagcaa aagtaatgat tcaagtagcg ttagtgctgc 180

acctaaaaca gacgacacaa acgtgagtga tactaaaaca tcgtcaaaca ctaataatgg 240

cgaaacgagt gtggcgcaaa atccagcaca acaggaaacg acacaatcat catcaacaaa 300

tgcaactacg gaagaaacgc cggtaactgg tgaagctact actacgacaa cgaatcaagc 360

taatacaccg gcaacaactc aatcaagcaa tacaaatgcg gaggaattag tgaatcaaac 420

aagtaatgaa acgactttta atgatactaa tacagtatca tctgtaaatt cacctcaaaa 480

ttctacaaat gcggaaaatg tttcaacaac gcaagatact tcaactgaag caacaccttc 540

aaacaatgaa tcagctccac agagtacaga tgcaagtaat aaagatgtag ttaatcaagc 600

ggttaataca agtgcgccta gaatgagagc atttagttta gcggcagtag ctgcagatgc 660

accggcagct ggcacagata ttacgaatca gttgacgaat g 701

<210> 4
<211> 700
<212> DNA
<213> Staphylococcus aureus

<400> 4
tagcatagca acaaacagtg agcttaaaaa ttctcaaaca ttagatttac cacaatcatc 60

accacaaacg atttccaatg cgcaaggaac tagtaaacca agtgttagaa cgagagctgt 120

acgtagttta gctgttgctg aaccggtagt aaatgctgct gatgctaaag gtacaaatgt 180

aaatgataaa gttacggcaa gtaatttcaa gttagaaaag actacatttg accctaatca 240

aagtggtaac acatttatgg cggcaaattt tacagtgaca gataaagtga aatcagggga 300

ttattttaca gcgaagttac cagatagttt aactggtaat ggagacgtgg attattctaa 360

ttcaaataat acgatgccaa ttgcagacat taaaagtacg aatggcgatg ttgtagctaa 420

agcaacatat gatatcttga ctaagacgta tacatttgtc tttacagatt atgtaaataa 480

taaagaaaat attaacggac aattttcatt acctttattt acagaccgag caaaggcacc 540

taaatcagga acatatgatg cgaatattaa tattgcggat gaaatgttta ataataaaat 600

tacttataac tatagttcgc caattgcagg aattgataaa ccaaatggcg cgaacatttc 660

ttctcaaatt attggtgtag atacagcttc aggtcaaaac 700

<210> 5
<211> 635
<212> DNA
<213> Staphylococcus aureus

<400> 5
aacaaagcag atgcgatagt aacaaaggat tatagcaaag agtcaagagt gaatgagaac 60

agtaaatatg ggacattaat ttcagactgg tatttaaaag ggagattaac tagtctagaa 120

tctcaattta tcaatgcatt ggatatttta gagacatatc attatggcga aaaagagtat 180

aaagatgcaa aagataaatt gatgacaaga attttagggg aagaccaata ccttttagaa 240

agaaaaaaag tgcagtatga ggaatacaaa aaattatacc aaaaatataa agaagagaat 300

ccaacctcta aaggcttaaa actgaaaaca ttcgatcaat atacaataga agatttaact 360

atgagggaat ataatgagtt aacagaatca ttaaaaagtg ctgtaaaaga ctttgagaaa 420

gatgttgaaa aaatagaaaa tcaacatcat gatttgaaac catttactga tgaaatggaa 480

gagaaggcta cttctagagt tgatgattta gcaaataaag catatagtgt ttattttgca 540

tttgttaggg atacacaaca taaaactgag gcattagagt taaaagcgaa agtagattta 600

gttttaggtg atgaggataa accgcatcgt atttc 635

<210> 6
<211> 532
<212> DNA
<213> Staphylococcus aureus

<400> 6
ctaggcgcat tagcagttgc atctagctta tttacatggg ataacaaagc agatgcgata 60

gtaacaaagg attatagcaa agagtcaaga gtgaatgaga acagtaaata tgggacatta 120

atttcagact ggtatttaaa agggagatta actagtctag aatctcaatt tatcaatgca 180

ttggatattt tagagacata tcattatggc gaaaaagagt ataaagatgc aaaagataaa 240

ttgatgacaa gaattttagg ggaagaccaa taccttttag aaagaaaaaa agtgcagtat 300

gaggaataca aaaaattata ccaaaaatat aaagaagaga atccaacctc taaaggctta 360

aaactgaaaa cattcgatca atatacaata gaagatttaa ctatgaggga atataatgag 420

ttaacagaat cattaaaaag tgctgtaaaa gactttgaga aagatgttga aaaaatagaa 480

aatcaacatc atgatttgaa accatttact gatgaaatgg aagagaaggc ta 532

<210> 7
<211> 268
<212> DNA
<213> Staphylococcus aureus

<400> 7
aatgctgcta acctgcgtga taaacaaaca aaacttgaaa agcaatatga agaagctaaa 60

aatgaatgga agaatgcaca aaatggcatg tcaacttcat tgtcagaaga agatattgct 120

gaagttattg caggatggac aggtatccca ttaactaaaa tcaatgaaac agaatctgaa 180

aaacttctta gtctagaaga tacattacat gagagagtta ttgggcaaaa agatgctgtt 240

aattcaatca gtaaagcggt tagacgtg 268

<210> 8

<211>  321
<212>  DNA
<213>  Staphylococcus aureus

<400>  8
aaagtaaaga gtagactaag cagtctgctc tttttgtatg agtaaaccga ggtgtcaata      60

aattgtttac tatactttga gcggaaatat gattgaatga agctagttga accgtaacta     120

tatgaaatgt tcccttcaaa gtagacattg aaaggaacat ttcaatcctt tgtttgtaag     180

tcgctctaga cattacattt agtacatatg ttgtttctaa tgctcattaa tggtattgat     240

tattctttaa ttaaatcttc aagtgccatt tttaaattac tatatttaaa ttggaatccc     300

aatgcttgaa ttttattagg t                                               321


<210>  9
<211>  350
<212>  DNA
<213>  Staphylococcus aureus

<400>  9
aagaatttaa aatggttagg tgtcgtagca acgttaatga tgacatttgt acaacttggt      60

ggagccttag ttaccaaaac cggatcagct gatggttgtg gttcttcttg gccactatgt     120

catggtgcgt tgattccaga attctttcct attgatacga ttattgagtt aagtcataga     180

gccgtttcag ctttgtcttt attaatggtc ttatggttag ttatcactgc atggaaacat     240

ataggctata ttaaagaaat taaaccttta tcaatcatta gtgttggatt cttattattg     300

caagcattaa tcggagctgc tgctgttatt tggcaacaaa acgattacgt                350


<210>  10
<211>  357
<212>  DNA
<213>  Staphylococcus aureus

<400>  10
aacgtcccat accattaatt ttagaaatta agaatcctaa aacattaatg attaaaggta      60

aaatctttgt gtattgaagg ataccgataa tcgctgaaat aaatacgata ggtaataata     120

cactgaagaa gaatggtggt tgcttaggat cgatatattg aataccaccg aatacaaagt     180

taacaccatc tgctgctttt aataataagt agttaaaacc gtttgaaata ccaccaataa     240

ccttgattcc cattgtagtt ttaagcaaga taaatgcaaa gataagctga attgcaagta     300

aaattcctac atatttccag cgaatatttt tcctgtctga gctaaataga aacgcaa        357


<210>  11
<211>  336
<212>  DNA
<213>  Staphylococcus aureus

<400>  11
acagagcagc aaaagcgtta gtaaaccgtt tcccaagtgc gacgatttac aacacatatg      60

gtccaactga agctacggta gcagttacaa gtattcaaat tacacaagaa atcttagatc    120

aatatccgac attacctgtt ggcgttgaaa gaccaggcgc aagattatct actacagatg    180

aaggtgaact tgttatcgaa ggtcaaagtg taagtttagg atacttaaaa aatgaccaaa    240

aaacagctga agtatttaat ttcgatgacg gtattcgtac atatcacact ggtgataaag    300

cgaagtttga aaatggtcaa tggttcattc aaggtc                              336

<210>  12
<211>  340
<212>  DNA
<213>  Staphylococcus aureus

<400>  12
catatggtga ttttacattc ttcttaattg ctttaattgc attattacca gtcattatac     60

ttggattttt aggtaagcga agttacattt ataatggcgt agttacagca tttatgattg    120

tgttaatctt ttcttctgat aaacataatc tgtttgacca aaagtattta agtgttcaat    180

taattagttt tattatttac gtcgtatggc aagttttatt gataatgttt tattatcatt    240

caaaaccaaa aaataattca ttttcaaaat ttgtaactgt aatggtttta tcaatattgc    300

cattagcact tgtgaaagtg ttacaaagta catggttagg                          340

<210>  13
<211>  210
<212>  DNA
<213>  Staphylococcus aureus

<400>  13
aaatttatta gcagaagtag cagaaaatga tattgtaaaa gaaaatccag acgtagaaat     60

ttttgaagaa ggtattattg attctttcca aacagttgga ttattattag agattcaaaa    120

taaacttgat atcgaagtat ctattatgga ctttgataga gatgagtggg caacaccaaa    180

taaaatcgtt gaagcattag aagagttacg                                     210

<210>  14
<211>  262
<212>  DNA
<213>  Staphylococcus aureus

<400>  14
tttaggcgaa aatatcggtg aagaagataa aaaatctgct gaagagaaaa aagacgctct     60

taaaactgct ttagaaggtc aagatataga agatattaaa tctaaaaaag aagaacttga    120

aaaagtgatt caagaattat cagcaaaagt atatgagcaa gcggctcaac agcaacaaca    180

agcacaaggt gcaaatgctg gtcaaaacaa cgatagtact gtagaagatg ctgaatttaa    240

agaagtaaaa gacgacgaca aa                                             262

<210>  15
<211>  224
<212>  DNA
<213>  Staphylococcus aureus

<400>  15
ggtcttatcg ttgcagctat cactatttca tcactaggga gcttaagtgg actattagtg      60

ccactgttta ctggacgaat tgtagataaa ttttccgtga gccatatcaa ttggaatcta     120

atcgcattat ttggtggtat ctttgttatc aatgctttat taagcggatt aggtttatat     180

ttattaagta aaattggtga aaagattatt tatgcgatac gctc                      224


<210>  16
<211>  435
<212>  DNA
<213>  Staphylococcus aureus

<400>  16
tcaggtgaaa tgttagaatc agcattaata gctggtttga tttcaattgg tgcagaagtg      60

atgcgattag gtattatttc aacaccaggt gttgcatatt taacacgcga tatgggtgca     120

gagttaggtg taatgatttc agcctctcat aatccagttg cagataatgg tattaaattc     180

tttggatcag atggttttaa actatcagat gaacaagaaa atgaaattga agcattattg     240

gatcaagaaa acccagaatt accaagacca gttggcaatg atattgtaca ttattcagat     300

tactttgaag gggcacaaaa atatttgagc tatttaaaat caacagtaga tgttaacttt     360

gaaggtttga aaattgcttt agatggcgca aatggttcaa catcatcact agcgccattc     420

ttatttggtg actta                                                      435


<210>  17
<211>  426
<212>  DNA
<213>  Staphylococcus aureus

<400>  17
tagtcaccat gaagttgccc ctggtcaaca tgaaattgac tttaaatatg cagatgctgt      60

tacagcatgt gataatatcc aaacatttaa attggttgtt aaaacaatcg cacgtaaaca     120

taatttacac gcaacattta tgcctaaacc attatttggt gtgaatggta gcggtatgca     180

ctttaacgtt tcattattca aaggtaaaga aaatgcattc tttgatccga atactgaaat     240

gggcttaaca gaaacagctt accaattcac agctggtgta cttaaaaacg cacgtggatt     300

tacagcggta tgtaacccat tagtaaactc atacaaacgt ttagttcctg gttatgaagc     360

accatgttat attgcatgga gcggtaaaaa ccgttcaccg ttaatccgtg taccatcttc     420

aagagg                                                                426


<210>  18
<211>  339

<212>  DNA
<213>  Staphylococcus aureus

<400>  18
cgaatgatgc aatcagacga aatatggctg tcttctctat gagtgtagta agtaagttaa      60

cggatttaac gccaaggcaa atacgttact atgaaacaca tgaactcatc aaacctgaaa     120

gaacagaagg tcaaaaacgt ctgttctcac tcaatgattt ggaaagatta ctagaaatta     180

aatcattatt agaaaaagga tttaatatca aagggattaa acaaatcatt tatgactcac     240

aagagcattt aacaacagat gaacaagaga taagaaaaaa gatgattgta gatgccacgc     300

aaaagcctat tggagaaact ttgccaataa atcgtggtg                            339


<210>  19
<211>  390
<212>  DNA
<213>  Staphylococcus aureus

<400>  19
ttgaatcacc aaattgaggt tgttgcaaat agaacgaagt ttgaattaga taatgcagaa      60

aaacgtatgc atatcgttga aggtttgatt aaagcgttgt caattttaga taaagtaatc     120

gaattgattc gtagctctaa aaacaagcgt gacgctaaag aaaaccttat cgaagtatac     180

gagttcacag aagaacaggc tgaagcaatt gtaatgttac agttatatcg tttaacaaac     240

actgatatag ttgcgcttga aggtgaacat aaagaacttg aagcattaat caaacaatta     300

cgtcatattc ttgataacca tgatgcatta ttgaatgtca taaaagaaga attgaatgaa     360

attaaaaaga aattcaaatc tgaacgactg                                      390


<210>  20
<211>  415
<212>  DNA
<213>  Staphylococcus aureus

<400>  20
aaatccatcg agatggtaat atatatcatc aaagttttaa aaacggtggt tcgccatctt      60

ctggtttagt gaaaaaaggt aaaactaaga aaacaggtac caaagtaaca tttaaacctg     120

atgacacaat ttttaaagca tctacatcat ttaattttga tgttttaagc gaacgactac     180

aagagtctgc gttcttattg aaaaatttaa aaataacgct taatgattta cgcagtggta     240

aagagcgtca agagcattac cattatgaag aaggaatcaa agagtttgtt agttatgtca     300

atgaaggaaa agaagttttg catgacgtgg ctacattttc aggtgaagca aatggtatag     360

aggtagacgt agctttccaa tataatgatc aatattcaga aagtatttta agttt          415


<210>  21
<211>  206
<212>  DNA
<213>  Staphylococcus aureus

<400>  21
gtatgcaatt tgatcgtggt tatcaatcac cgtatatggt tactgattca gataaaatgg      60

ttgctgaatt agaacgccca tacattttag taacagataa gaaaatctcg tctttccaag     120

atatcttacc tttattagaa caagtggttc aatctaatcg tccaatctta attgtagctg     180

atgaagttga aggcgatgca ttaaca                                          206


<210>  22
<211>  380
<212>  DNA
<213>  Staphylococcus aureus

<400>  22
tctatttaat ttatttatga attaagttct gtattattca ataactgcta aaatatcttc      60

ttcatttaat accagatatg tttcattatc tcgtttaact tctgtaccag catattgttg     120

gaacacgaca cggtcccctt ctttcacttc aggagtcact cttgtaccat catttaatag     180

gcgtccagtt cctactgcaa cgataacgcc ttcgtttgat ttttctttag cactatcagt      240

taaaacaata ccacttttag ttgtttgttc ttgttctttt ttctcaataa tcacacgatt     300

tccaattggt tttagcatga ttgttcctcc ttaaaaaacc taaagtttag cacttaacat     360

taaagagtgc taacatacat                                                 380


<210>  23
<211>  496
<212>  DNA
<213>  Staphylococcus aureus

<400>  23
tgtcatatta tcaacatgta atcgaactga agtatatgct gttgttgatc aaattcacac      60

aggtcgttac tatattcaac gatttctagc tcgtgcattt ggatttgaag tagatgatat     120

taaagcaatg tcagaagtaa aagtggggga cgaagcagta gaacatttat tgcgtgtcac     180

ttctggttta gattcaatcg tacttggaga aactcaaatt ttaggtcaaa taagagatgc     240

atttttctta gcgcaaagca caggtacgac aggaacaatt tttaatcatc tatttaaaca     300

ggcaattact tttgcaaaaa gagcacataa tgaaacagat atagctgata atgctgtaag     360

tgtgtcttat gctgcggtcg agttggcgaa aaaagtattt ggcaaattga aaagtaagca     420

agctatcatt attggtgcag gggaaatgag tgaattatca ctattaaatc ttcttggttc     480

tggaattact gatatt                                                     496


<210>  24
<211>  619
<212>  DNA
<213>  Staphylococcus aureus

<400>  24

aaaatgatca aaggtgaaga aacatcacat acacctgttt ggtttatgcg acaagctggc 60

cgttcgcaac cagaatatcg aaaattgaaa gaaaaatatt cactattcga tattacacat 120

cagccggagt tgtgcgctta tgtaacacat ttaccagttg ataattatca tacagatgca 180

gcaattttat acaaagatat tatgacacca ttaaagccaa ttggtgtcga tgtagaaatt 240

aaatcgggta ttggtccagt gattcataat ccaatcaaaa caattcaaga tgttgagaaa 300

ctttctcaaa tagaccccga acgagatgta ccatatgtat tagatacaat taaactttta 360

acagaagaaa agttaaatgt gccgctaata ggatttactg gggcaccatt tacattagcg 420

tcatatatga ttgaaggcgg accatcgaaa aattacaatt ttacaaaagc gatgatgtat 480

agagatgaag caacatggtt tgctttaatg aatcatttag ttgatgtatc tgttaaatat 540

gtaacagctc aagtcgaagc aggtgccgaa ttgattcaaa ttttcgattc atgggtaggt 600

gcattaaatg tcgaggatt 619

<210> 25
<211> 578
<212> DNA
<213> Staphylococcus aureus

<400> 25
aatgggatta ttagttatgg cttatggcac accttataaa gaaagtgaca tagagccata 60

ttatacagat attagacatg gtaaacgtcc atctgaagaa gaacttcaag atttgaaaga 120

tagatatgaa tttataggtg gtttatcacc attagcaggt acaacagatg accaggctga 180

tgcgctagtt tcagcattaa ataaagcata tgcagatgtt gaatttaaac tatacttagg 240

attaaaacac atttcaccat ttatcgaaga tgcggttgaa caaatgcaca atgatggcat 300

tactgaagca atcacggtag tactagcacc acattattct tcattttcag taggatcata 360

tgacaaacgt gctgatgaag aagctgcaaa atatggtatt caacttacac atgtgaaaca 420

ttattatgaa caacctaaat ttattgaata ttggacgaat aaagtcaacg aaacattagc 480

tcaaataccg gaagaggaac ataaagacac ggtattagtt gtttcggcac atagtttgcc 540

aaaaggttta atcgaaaaga ataatgatcc atatccac 578

<210> 26
<211> 382
<212> DNA
<213> Staphylococcus aureus

<400> 26
atgagatata cgaaatcaga agaagcaatg aaggttgctg aaactttaat gcctggtggt 60

gtaaatagtc cagtacgcgc atttaaatca gtagatacac cagcaatttt tatggatcac 120

ggtaaaggtt caaaaattta tgatatcgat ggtaacgagt atatcgacta tgtactaagt 180

tgggggccac ttattttagg acatagagac cctcaagtta ttagtcattt acatgaagca 240

attgataaag gtacaagttt tggtgcatca acattacttg aaaataaatt ggcgcagctc 300

gttattgacc gagtaccttc aatagaaaaa gtgcgtatgg tgtcatctgg tacagaagct 360

acattggata ctttaagatt ag 382

<210> 27
<211> 1099
<212> DNA
<213> Staphylococcus aureus

<400> 27
aaacagcaag atcctaatat tgatgtaacc atctttgaag catcgaatcg tccgggggga 60

aagattcaat cgtatcgtaa agatggttat atgattgaac tagggcctga atcttattta 120

ggtagaaaaa cgattatgac agaattagcg aaagatattg gattagaaca agatattgtt 180

acaaatacga ctggacaatc atatattttt gcgaaaaaca aattgtatcc tattccaggt 240

ggatcaatta tgggaattcc gacagatatc aaaccgtttg tgacaactaa attaatttca 300

ccacttggta aattaagagc aggattagat ttaatcaaaa agcctataca aatgcaagat 360

ggtgacattt ctgttggtgc atttttcaga gcaagattag gtaatgaggt acttgagaat 420

ttaatagagc ctttaatggg tggtatttat ggtaccgata ttgataaatt aagtttgatg 480

agtacgtttc ctaattttaa agaaaaagaa gaggcattcg gaagtctgat aaaaggtatg 540

aaggatgaga aaaataagcg tctgaaacaa agacaattat atcctggcgc accaaaagga 600

caattcaaac aatttaagca tggtttaagt tcatttattg aagcattaga acaagatgtg 660

aaaaataaag gtgtgacaat acgctacaat acgtcagtgg atgatattat tacatctcaa 720

aagcaatata aaattgttta cagtaatcaa caagaagatg tattcgatgg ggtattagtg 780

acaacaccgc atcaagtctt tttgaattgg ttcggacaag atccagcatt tgattacttt 840

aaaacgatgg atagtacgac tgttgcaact gttgtattgg catttgatga aaaagatatt 900

gaaaatactt atgatggtac tggcttcgtg attgcgagaa cgagtgatac agacattacc 960

gcatgtactt ggacatcgaa aaaatggcca tttactacac cagaaggtaa ggttttgatt 1020

cgtgcgtatg taggtaaacc aggtgatact gtggttgatg atcatacaga taatgaatta 1080

gtatcgattg tacgtagag 1099

<210> 28
<211> 629
<212> DNA
<213> Staphylococcus aureus

<400> 28
attaacaaaa ttgatttacc tgctgcagaa cctgaacgcg tgaaacaaga aattgaagat 60

atgataggtt tagaccaaga cgatgttgtt ttagcaagtg ctaaatctaa cattggaatt 120

gaagagatac tagagaaaat agttgaagtt gtgccagctc cagatggcga cccagaagca 180

ccactaaaag cgttaatatt tgattctgag tatgatccat atagaggggt aatttcatcg 240

ataagaattg tagacggtgt tgttaaagcc ggagataaaa ttcgaatgat ggcgactggt 300

aaagagttcg aagtaacaga agttggaatt aatacaccta agcagcttcc agttgatgaa 360

ttaacagttg gtgatgttgg ttatattatt gcaagtatta aaaatgttga tgattctagg 420

gttggtgaca ccatcacatt agctagtaga cctgcatcag aaccattgca aggttataag 480

aaaatgaatc caatggtata ttgcggactg ttcccaatag ataacaaaaa ttataatgat 540

ttaagagaag cattagaaaa attacaattg aatgatgcat cattagaatt tgagcctgaa 600

tcgtcacaag cattaggttt tggttatag 629

<210> 29
<211> 265
<212> DNA
<213> Staphylococcus aureus

<400> 29
aaagacgcat caaaaccagc acactttttt caccaagtca ttgtaattgc tttagtactc 60

tttgtatcga aaataattga atcatttatg ccaattccta tgcctggatc agtaatcggt 120

ttagtattat tatttgtatt attatgtact ggtgctgtta agttaggcga agtcgaaaaa 180

gtaggaacga cactaacaaa taacattggc ttactcttcg taccagccgg tatctcagtt 240

gttaactctt taggtgtcat tagcc 265

<210> 30
<211> 278
<212> DNA
<213> Staphylococcus aureus

<400> 30
gattaaccac ttagcactaa atacacctta cttcggaata ctgttatccg ttataccatt 60

tttcttagcg accatattat ttgaaaaaac taatcgtttc ttcttattcg caccgctatt 120

tgtcagtatg gtatttggtg tggccttcct ctatttaaca ggcattccgt ataagactta 180

caaaataggt ggagacatta tttacttctt cttagaaccg gcaacaatct gttttgcgat 240

tccgttatat aaaaagcgtg aagtgcttgt taaacatt 278

<210> 31
<211> 388
<212> DNA
<213> Staphylococcus aureus

<400> 31
cgacaaacac ccaacaagca catacacaaa tgtcaacaca atcacaagac gtatcttatg 60

gtacttatta tacaattgat tctaatgggg attatcatca cacacctgat ggtaactgga 120

atcaagcaat gtttgataat aaagaatata gctatacatt cgtagatgct caaggacata 180

cgcattattt ttataactgt tatccaaaaa atgcaaatgc caatggaagc ggccaaacat 240

atgtgaatcc agcaatagca ggagataaca atgactacac agcgagtcaa agccaacagc 300

atattaatca atatggttat caatcaaatg taggtccaga cgcgagctat tattcacata 360

gtaacaacaa ccaagcgtat aacagcca 388

<210> 32
<211> 203
<212> DNA
<213> Staphylococcus aureus

<400> 32
gttatcgtat taactggtga aggtgattta gcattctgtt ctggtggtga ccagaagaaa 60

cgtggacatg gtggttatgt aggtgaagac caaatccctc gcttaaatgt attagattta 120

cagcgtttaa ttcgtattat tccaaaaccg gttatcgcga tggtaaaagg ttatgctgta 180

ggtggcggta atgtactaaa tgt 203

<210> 33
<211> 1434
<212> DNA
<213> Staphylococcus aureus

<400> 33
cgtaagggaa gtagttatca gtccgggatc acgctcaacg ccacttgcac ttgcatttga 60

agcacatcca aatattaaaa catggataca ccccgatgag cgaagtgcag cattttttgc 120

agttgggtta attaaaggta gtgaaagacc tgtcgctata ttatgtacgt caggtacagc 180

agcagcgaat tatacgcctg caattgctga aagccaaatt agtagaattc cattaatcgt 240

tttaacaagt gaccgtccgc atgaattaag aagtgtaggc gcaccacaag cgattaatca 300

agtaaatatg tttaataatt atgtaagtta tgagttcgat atgcctattg cggatgatag 360

taaagagacc attaatgcaa tttattatca aatgcaaatt gctagtcaat atttatatgg 420

accacataaa gggccaattc attttaactt gccatttaga gatccgttaa cacctgattt 480

gaatgcaaca gaattgttaa cttctgagat gaagatttta ccgcactatc aaaaaagtat 540

agatgcatcg gcattaagac acattttaaa taagaaaaaa ggtttaatta ttgtagggga 600

tatgcagcac caagaagttg atcaaatact aacgtattca acgatatatg atttgcctat 660

tttagctgat cctttaagtc atttaagaaa atttgatcat ccgaatgtta tctgtacata 720

tgatttgctg tttagaagcg gcttagactt aaatgtggat ttcgtaattc gtgttgggaa 780

accagtgatt tctaaaaagt tgaatcaatg gttaaagaaa actgatgcat ttcaaatatt 840

agtgcaaaac aatgataaga ttgatgtctt tccgatagca ccagatattt catatgagat 900

ttctgcgaat gatttcttta ggtcattaat ggaagacacg accatcaatc gcgtaagttg 960

gttagaaaaa tggcaacgct tagagaaaaa agggcgtaaa gaaattaaat gttatttgga 1020

acaagctaca gatgagagtg cattcgttgg tgaattgatt aagaaaacat ctgaaaaaga 1080

tgcattattt attagtaata gtatgcctat cagagatgta gataacttgt tattgaataa 1140

aaatatagat gtctatgcga atcgtggtgc gaatggtatt gatggtatcg tttcaactgc 1200

actgggtatg gctgtgcata aacgaataac attattgata ggtgatttat cattttatca 1260

tgatatgaat ggactattaa tgtcaaaatt aaataatatt cagatgaata ttgtattatt 1320

gaacaacgat ggtggcggta ttttttcata tttaccacaa aaagaaagtg caactgacta 1380

ttttgaacgg ttgtttggca caccgacggg attggatttc gagtatacag ctaa 1434

<210> 34
<211> 1149
<212> DNA
<213> Staphylococcus aureus

<400> 34
atggactttt ggttatataa acaagcacaa caaaatggac atcatattgc gataacagac 60

ggtcaagaat cttatactta tcaaaattta tactgtgaag cgagtctatt ggctaaaaga 120

ctcaaggctt atcaacaatc tcgtgtcggg ctatacatag ataattcgat tcaatcgatc 180

attttaatac atgcttgttg gttggcaaat attgaaattg cgatgattaa tacaaggttg 240

acacctaatg agatgaagaa tcagatgagg tcaatcgatg tacaattgat tttttgtacc 300

ttgccactgg aattgcgagg gtttcaaatt gtatcgctgg atgatattga attcgctgga 360

acggatatta caatgaacgg tttgttggac aacacaatgg atatccaata tgatacatcg 420

aatgaaactg tggtgccgaa agagtcgccg tccaacatat taaatacttc atttaattta 480

gatgacattg catcgattat gtttacatca gggacaactg gccctcaaaa agcggtgccg 540

caaacgtttc gtaatcatta tgccagtgca atcggatgta aagagagctt gggatttgat 600

cgtgatacta attggctatc tgtcttgccg atttatcata tttcgggtct cagtgtactt 660

ttaagagctg ttattgaagg gtttactgtg cgcattgttg ataaattcaa tgccgaacaa 720

attttaacga taattaaaaa tgaacgcatc acgcacattt cgcttgtgcc acaaacttta 780

aattggctta tgcaacaagg tttacatgaa ccttataatt tgcaaaaaat attactcggc 840

ggtgctaaat tatctgccac tttgatagag acggcattac aatataacct gccaatttat 900

aattcatttg gtatgactga gacatgttca caatttttaa cagcaacacc ggaaatgttg 960

catgcacgtc ctgacactgt agggatgcca agtgccaatg tagacgttaa aattaaaaat 1020

cctaataaag aaggtcatgg agaattaatg attaaaggtg ccaatgtgat gaatggatat 1080

ttgtatccaa cagatttaac gggtacgttt gaaaatggtt attttaatac gggtgacatt 1140

gctgaaata 1149

<210>  35
<211>  236
<212>  DNA
<213>  Staphylococcus aureus

<400>  35
attgataatt tacatccaac acctgcttta ggtggctatc caaaagaatt tgcgatggat      60

tttattgaac agaaagaatt tggtacacga ggattatatg gtgcgccggt tggctatata     120

gatatatatg atgattgtga atttattgtt gcaattcgtt cgatgcttat taagaaagca     180

caagcaactt tatttgctgg gtgtggcatt gttaaagatt ctgatccaga tagtga         236


<210>  36
<211>  327
<212>  DNA
<213>  Staphylococcus aureus

<400>  36
atgaggtact ctttaattag tggtatcttg ataggtttta ttgcgcctct aatcggtgct      60

tttatcgttg ttagacgact atcacttata gctgatgctc taagtcatgt aactttaggt     120

ggtatatctt tcggtatgtt tttacttact attatgccaa cactagtatt tattaatcca     180

atgtggtttg gaatcttatt cgcaatagta ggtgcgcttc taattgaaaa attaagaacg     240

tcatacactg cttaccaaga aattgctatt ccaattataa tgagtgctgg tatcgccttg     300

agtgcaatct tcatttcatt agctgat                                         327


<210>  37
<211>  195
<212>  DNA
<213>  Staphylococcus aureus

<400>  37
gaaaatacag aacttgatgg tgaaatgaag tttagaatcg cttgtacaaa ccatcatcat      60

catcatttta tctgtgaaaa gtgtggagat acaaaggtaa tagattattg tccaatagat     120

cagataaagt tatcactacc tggtgttaat attcacaaac acaaacttga agtttatggt     180

gtatgtgagt cttgc                                                      195


<210>  38
<211>  313
<212>  DNA
<213>  Staphylococcus aureus

<400>  38
acacagagaa taatcaagag aagacgtttt catctgaaga aagtaacagt aagccattta      60

tggtagaaaa tcaaaacgat gaaatagtta taagagaaga ttcatataat ccattcgtaa     120

cgaaaacgtc tgaaagttta atagctgatg atgaatcttc cggttataat aatacacgtg     180

aaaaagatga agactacttc aaaaagcaac aagaaattct acaagaaatg gatcaaacat 240

ttgattcgaa tgacgataca tctgtgcaaa attatgagaa taaagcgtct gatgattatt 300

atgatgtaaa cga 313

<210> 39
<211> 322
<212> DNA
<213> Staphylococcus aureus

<400> 39
ttgtaattca cttaacttca ccaatgcctc aattggtgtc atattagata aattcaaatt 60

tttaatttgt agttcaatct cgctttcttg atcattttca aacaaatcaa atgatgcttg 120

ttcaaagtct ttttgagata aagtatcagt tgtttcttca acacttaagt ttaaattttc 180

ttgattaatt tcaggttcat tttcgaccat ttttaaattt gatatcgatg attttttacc 240

agcagacgct tcaaactcgc ttagaatcac ttgtgctctg ctaataactt tttcaggtaa 300

atcagctaat ttcgcaactt ga 322

<210> 40
<211> 432
<212> DNA
<213> Staphylococcus aureus

<400> 40
actcaaacag ttagcaagat tgctcaagtt aaaccaaaca acactggtat tcgtgcttct 60

gtttatgaaa aaacagcgaa aaacggtgcg aaatatgcag accgtacgtt ctatgtaaca 120

aaagagcgtg ctcatggtaa tgaaacgtat gtattattaa acaatacaag ccataacatc 180

ccattaggtt ggttcaatgt aaaagactta aatgttcaaa acttaggcaa agaagttaaa 240

acgactcaaa aatatactgt taataaatca aataacggct tatcaatggt tccttggggt 300

actaaaaacc aagtcatttt aacaggcaat aacattgctc aaggtacatt taatgcaacg 360

aaacaagtat ctgtaggcaa agatgtttat ttatacggta ctattaataa ccgcactggt 420

tgggtaaatg ca 432

<210> 41
<211> 353
<212> DNA
<213> Staphylococcus aureus

<400> 41
ggtgttccaa actcaaaaga tgatataggt actggtgcat acattcacga tggtgttatt 60

caacaggcaa cacgtattgc taaaaaaatg tatgatgact tattaattgt tgcagacact 120

tgtttatgtg aatatactga tcatggtcat tgtggcgtga ttgatgacca tacacatgac 180

gttgacaatg ataaatcatt gccactgctt gttaaaacag caatttctca agtggaagct 240

ggtgctgata ttattgcgcc aagtaatatg atggatggtt ttgttgctga aattcgtcgt 300

ggattagatg aagccggcta ttacaatatt cctataatga gttatggtgt caa 353

<210> 42
<211> 399
<212> DNA
<213> Staphylococcus aureus

<400> 42
aacacaatcg gaaatgttgg atacggctga aaagttatca aagctaatca agatggatac 60

taagaaaatt acagaacgtg ataagaaaga tttctggatt cagttgcatc ctaaaaaagc 120

aaaagcaatg atgacaaaag aacaagctat gttagcagat ggaagtatta aacaagatca 180

atatgataaa caactgttat cgaaaatcag aaaatcacaa ttagatgaat tgtcttctaa 240

agatttacaa gttttagcta tttttcgaga gatgaatgca ggaacagttt tagatccaca 300

aatgataaaa aatgaagatg tcagtgaaaa agagtatgca gcagtttctc agcaactttc 360

caaattacca ggtgttaaca cgtctatgga ttgggatag 399

<210> 43
<211> 329
<212> DNA
<213> Staphylococcus aureus

<220>
<221> misc_feature
<222> (56)..(56)
<223> n is a, c, g, or t

<220>
<221> misc_feature
<222> (71)..(71)
<223> n is a, c, g, or t

<400> 43
tgacatttca aatcaatcac atcggtgcaa atggttcaag aaatcaaatg aaagcncgca 60

gaagaacttg naaaaagatg gttattctgt tgaagtaatt gacttacgta ctgttcaacc 120

aatcgatgtt gatacaattg tagcttcagt tgaaaaaact ggtcgtgcag ttgtagttca 180

agaagcacaa cgtcaagctg gtgttggtgc agcagttgta gctgaattaa gtgaacgtgc 240

aatcctttca ttagaagcac ctattggaag agttgcagca gcagatacaa tttatccatt 300

cactcaagct gaaaatgttt ggttaccaa 329

<210> 44
<211> 303
<212> DNA
<213> Staphylococcus aureus

<400> 44
ctggagatac tattgaagaa gacgatgttt tagctgaggt acaaaacgat aaatcagtag 60

tagaaatccc atcaccagta tctggtactg tagaagaagt tatggtagaa gaaggtacag 120

tagctgtagt tggtgacgtt attgttaaaa tcgatgcacc tgatgcagaa gatatgcaat 180

ttaaaggtca tgatgatgat tcatcatcta aagaagaacc tgcgaaagag gaagcgccag 240

cagagcaagc acctgtagct actcaaactg aagaagtaga tgaaaacaga actgtaaaag 300

caa 303

<210> 45
<211> 302
<212> DNA
<213> Staphylococcus aureus

<400> 45
tagttatcga gattatcaaa gattggtaga taaacttcaa gttcacgata aagagataga 60

cttagcttct agcttacaac aaacaatgct taaaacagat attccacaat ttgatagtat 120

tcaaattggc gttatttcag tggcggcaca aaaagtaagt ggagattatt ttaatttaat 180

tgaccataac gatggcacaa tgagctttgc tgttgcagat gtcattggaa aaggtatacc 240

agctgcttta gcaatgagta tgataaagtt tggcatggat tcttatggac actcacaatt 300

ac 302

<210> 46
<211> 254
<212> DNA
<213> Staphylococcus aureus

<400> 46
tgaatcttaa tatagaaaca accactcaag ataaatttta cgaagttaaa gtcggtggag 60

aattagatgt ttatactgtg cctgaattag aagaggtttt aacacctatg agacaagatg 120

gaactcgtga tatttatgtt aatttagaaa atgtgagtta tatggattcg acaggtttag 180

gtttattcgt aggtacatta aaagcattaa accaaaatga taaagaacta tacattttag 240

gtgtgtcaga tcgt 254

<210> 47
<211> 191
<212> DNA
<213> Staphylococcus aureus

<400> 47
tctaaagaag attttatcga aatgcgcgtg ccagcatcgg cagagtatgt aagtttaatt 60

cgtttaacac tttctggcgt tttttcgaga gctggtgcta catatgatga tattgaagat 120

gccaagattg cagttagtga agctgtgaca aatgcagtta aacatgcata caaagaaaat 180

aacaatgtag g 191

<210> 48
<211> 204
<212> DNA
<213> Staphylococcus aureus

<400> 48
tgagatagat gcaatcatgt ttatggttaa tgccaatgag gaaattggac gaggtgatga 60

atatattata gaaatgttga aaaatgttaa gacaccagta tttttagtat taaataaaat 120

agatttagtg catccagatg aattaatgcc aaagattgaa gaatatcaaa gttatatgga 180

ctttacagag attgtaccta tttc 204


<210> 49
<211> 234
<212> DNA
<213> Staphylococcus aureus

<400> 49
aatataattg ggaagaagta catcaagaag cagaaatttt agaacatcga atttcagatt 60

tatttgttga aaggctggat agcctgttaa atttcccaga aacttgcccg cacggcggtg 120

tgattcctag aaataatgaa tataaagaga aatatataac aacgattttg aattatgaac 180

ctggtgatat cgttacaatc aaacgtgtga gagataagac cgatttgcta atat 234


<210> 50
<211> 251
<212> DNA
<213> Staphylococcus aureus

<400> 50
ttgaattacc aaaattacca tacgcatttg atgcattaga accacatttt gacaaagaaa 60

ctatggaaat tcatcatgac agacatcata acacttatgt tacgaaatta aatgctgcag 120

tagaaggtac agatttagaa tctaaatcta ttgaagaaat tgttgctaat ttagacagtg 180

taccagctaa catccaaact gctgtacgta ataatggcgg tggacattta aaccattcat 240

tattctggga g 251


<210> 51
<211> 359
<212> DNA
<213> Staphylococcus aureus

<400> 51
gcgcattttg aaaaggcata cttgagaata ctaaagtgct tgttacaatt aaagaaccta 60

ctgttgctaa ttttttcatt gtcttgtccc cttatattac aatttgatta catttacatt 120

atcatacgat tacaaaagaa atgcaacaaa atttttgaat cattacattt ttttataaaa 180

atttcacttt agattcacaa taattactta ttttgtcaat ttatttaatg tcaatatgtt 240

gattaattaa tagtgttgtc taatgtatat aatatttagg tcatcgttat agtcaacaat 300

aataaggtat ttcgagttga aatttatctt attatttttc cacttttacg tgctatccc     359

<210>  52
<211>  438
<212>  DNA
<213>  Staphylococcus aureus

<400>  52
ttcgttgttc ataggtgcga gtgaactatc aattaaagat ttactacatt taactgagtc      60

acagcggaat attttattct caagccgaat accaaggacg atgagtattt taattgctgg     120

aagttcgttg gctttagcag gcttgataat gcaacaaatg atgcaaaata agtttgttag     180

tccgactaca gctggaacga tggaatgggc taaactaggt attttaattg ctttattgtt     240

ctttccaacc ggtcatattt tattaaaact agtatttgct gttatttgta gtatttgcgg     300

tacgttttta tttgttaaaa tcattgattt tataaaagtg aaagatgtca tttttgtacc     360

gcttctagga attatgatgg gtgggattgt tgcaagtttc acaaccttca tctcattgcg     420

cacgagtgct gttcaaag                                                   438

<210>  53
<211>  288
<212>  DNA
<213>  Staphylococcus aureus

<400>  53
tattgcctta tttagatgta ttgcttttag gtcgtgctga agcaattaat ctggggatat      60

cgtatgaaaa attaacgcga attctacttg taatagtctc agttttagtt tctgtgtcaa     120

ctgcattagt aggaccaatt acatttttag gtttattaac tgtaaatcta gcgcatgaac     180

taatgaagac gtatgaacat aagtatattt taattgcgac aatttgcttg agttggatta     240

gtttatttag tgcgcaatgg gtagttgaaa atgtgtttga agctacga                  288

<210>  54
<211>  431
<212>  DNA
<213>  Staphylococcus aureus

<400>  54
aatcaaatga tattggaaga tattagcata gatatcgaaa aaggtaaatt gacttcttta      60

attggaccta atggtgcggg taagagtact ttactttcag cgatttgtag gttaattcgt     120

tttgataacg gtgaagtgaa aatagatgga cggctcatgt ctgattataa aaataatgac     180

ttgtcgaaaa aaatatctat attaaaacaa acaaaccata ctgaaatgaa tattacggta     240

gagcagttgg taaactgtgg acgattccct tattctaaag gtcgtttgac gaaagaggat     300

catgatattg tcaatgatgc gctagatttg ttgcaactac aagatatcag aaatcgtaat     360

attaagtcat tatctggtgg acaacgtcag cgtgcatata ttgcaatgac aatagcacaa     420

gatactgaat a                                                          431

<210>  55
<211>  437
<212>  DNA
<213>  Staphylococcus aureus

<400>  55
catgcggtaa caattctgat aaagaacaat caaaatcaga gactaaaggt tctaaagata      60

cagtgaaaat tgaaaataac tataaaatgc gtggcgagaa aaaagatggt agtgacgcta     120

aaaaagttaa agaaactgtt gaagtaccaa aaaatcctga aaatgcagtt gtgttagact     180

atggcgcatt agatgtaatg aaagaaatgg gcttatcaga caaagtaaaa gcattaccta     240

aaggggaagg cggtaagtca ttaccgaatt tcttagaatc atttaaagat gataaatata     300

caaacgttgg taatttaaaa gaagtgaatt ttgataaaat tgctgcgacg aaacccgaag     360

taatctttat ctctggacgt acagctaatc aaaagaattt agatgaattc aaaaaagctg     420

cacctaaagc gaaaatt                                                    437

<210>  56
<211>  163
<212>  DNA
<213>  Staphylococcus aureus

<400>  56
gctgactatg aaggtaaagc tgacatttta aaattagatg ttgatgaaaa tccatcaact      60

gcagctaaat atgaagtgat gagtattcca acattaatcg tctttaaaga cggtcaacca     120

gttgataaag ttgttggttt ccaaccaaaa gaaaacttag ctg                       163

<210>  57
<211>  471
<212>  DNA
<213>  Staphylococcus aureus

<400>  57
caattggctt tcgattattg ttgtatctat ttcgatatta ttaattgcaa tagtaatggc      60

atttattttt aaaaaaattg cacgtattaa tacagaaaca gctattttaa gtgttatacc     120

aggagcacta acacaaatgc tggtcatggc tgaacaagac aaacgtgcta atttgttagt     180

tgttagctta acgcaaacat cacgaattat atttgttgtt gttttagtac cgttcatttc     240

atattttttt catgatggta acatgcatgc gaatggaaag ttaacaaaag tcttgccttt     300

atcacaagta ttaaacatag ggcaaatagt tattttagcg atagctatct ttatagttta     360

tctaattatg tctaaaataa agtttccaac atttcaatta ttagcaccac tcattgtatt     420

aattgtttgg aatttttcta caggtttaac atttacacta gatcattggt t              471

<210>  58

<211>  713
<212>  DNA
<213>  Staphylococcus aureus

<400>  58
cttagatgtc ccatgctgat ataacaattc aatcgcttta tctggatgtt tttcaagatg      60

atatttatca atgattaaag ctagtgctcc cgaaacttta ggtgtggcta atgaagttcc     120

agcttgataa atatatcttc cgttattggc agtagttaaa atgttctcct tatgcatata     180

cccttcattc atccatttat ccacaccgaa ttgatttaaa taagcaaatg atcctccggg     240

cgcagcaata tctgtataat tcataccaaa attggaaaac tcagatagat tactcttttg     300

atctgtagat cctactgtaa cgacattgtc catagatgca ggaacatctt tcacttcgcc     360

attaccttga tattcacgct gtaattttag tttctgtttg tcattgacat caataccatc     420

attaccagct gcagcaacaa cgatagattt tttcttcttg gcgtaattga ttgctttctg     480

taacgcatcg tattctactt tttcatcttt tctaaatgtt tgatggtcat ttttgtccaa     540

aataatataa ctaccaacac taatattaat gacttgattt ccatcatttg cagcttgaac     600

aatcgctttt gatacccaaa gcagttctgt ttttttacta ccaaacacgc gatacattgt     660

aaatttgtta ttcggtgcaa cacctattaa cttaccatta gcactcgttt gac            713


<210>  59
<211>  738
<212>  DNA
<213>  Staphylococcus aureus

<400>  59
ttcaataggc gtggtgtcag tgttagcggc tacaatgttt gttgtgtcat cacatgaagc      60

acaagcctcg gaaaaaacat caactaatgc agcggcacaa aaagaaacac taaatcaacc     120

gggagaacaa gggaatgcga taacgtcaca tcaaatgcag tcaggaaagc aattagacga     180

tatgcataaa gagaatggta aaagtggaac agtgacagaa ggtaaagata cgcttcaatc     240

atcgaagcat caatcaacac aaaatagtaa aacaatcaga acgcaaaatg ataatcaagt     300

aaagcaagat tctgaacgac aaggttctaa acagtcacac caaaataatg cgactaataa     360

tactgaacgt caaaatgatc aggttcaaaa tacccatcat gctgaacgta atggatcaca     420

atcgacaacg tcacaatcga atgatgttga taaatcacaa ccatccattc cggcacaaaa     480

ggtaataccc aatcatgata aagcagcacc aacttcaact acacccccgt ctaatgataa     540

aactgcacct aaatcaacaa aagcacaaga tgcaaccacg gacaaacatc caaatcaaca     600

agatacacat caacctgcgc atcaaatcat agatgcaaag caagatgata ctgttcgcca     660

aagtgaacag aaaccacaag ttggcgattt aagtaaacat atcgatggtc aaaattcccc     720

agagaaaccg acagataa                                                   738

<210>  60
<211>  780
<212>  DNA
<213>  Staphylococcus aureus

<400>  60
aggctcgtat gattgaaaaa attgcagagc tcgttcgtga caagaaaatt gacggtatca      60

ctgatttacg tgatgaaaca agtttacgta ctggtgtgcg tgtcgttatt gatgtgcgta     120

aggatgcaaa tgctagtgtc attttaaata acttatacaa acaaacacct cttcaaacat     180

catttggtgt gaatatgatt gcacttgtaa atggtagacc gaagcttatt aatttaaaag     240

aagcgttggt acattattta gagcatcaaa agacagttgt tagaagacgt acgcaatata     300

acttacgtaa agctaaagat cgtgcccata ttttagaagg gttacgtatc gcacttgacc     360

atatcgatga aattatttca acgattcgtg agtcagatac agataaagtt gcaatggaaa     420

gcttgcaaca acgcttcaaa ctttctgaaa aacaagctca agctatttta gacatgcgtt     480

taagacgtct aacaggttta gagagaaaca aaattgaagc tgaatataat gagttattaa     540

attatattag tgaattagaa gccatcttag ctgatgaaga agtgttatta cagttagtta     600

gagatgaatt gactgaaatt agagatcgtt tcggtgatga gcgtcgtaca gaaattcaat     660

taggtggatt tgaagactta gaggacgaag acttaattcc agaagaacaa atagtaatta     720

ctttgagcca taataactac attaaacgtt tgccggtatc tacatatcgt gctcaaaacc     780


<210>  61
<211>  622
<212>  DNA
<213>  Staphylococcus aureus

<400>  61
ttggcacaac tgataagaca ggtactgtca ttcgttttaa agcagatgga gaaatcttca      60

cagagacaac tgtatacaac tatgaaacat tacagcaacg tattagagag cttgctttct     120

taaacaaagg aattcaaatc acattaagag atgaacgtga tgaagaaaac gttagagaag     180

actcctatca ctatgagggc ggtattaaat cttatgttga gttattgaac gaaaataaag     240

aacctattca tgatgagcca atttatattc atcaatctaa agatgatatt gaagtagaaa     300

ttgcgattca atataactca ggatatgcca caaatctttt aacttacgca aataacattc     360

atacgtacga aggtggtacg catgaagacg gatttaaacg tgcattaacg cgtgtcttaa     420

atagttatgg tttaagtagc aagattatga aagaagaaaa agatagactt tctggtgaag     480

atacacgtga aggtatgaca gcaattatat ctatcaaaca tggtgatcct caattcgaag     540

gtcaaacgaa gacaaaatta ggtaattctg aagtgcgtca agttgtagat aaattattct     600

cagagcactt tgaacgattt tt                                              622


<210>  62

<211>  756
<212>  DNA
<213>  Staphylococcus aureus

<400>  62
atcatcagcg acaatgagag atatggttag agagaatcat gtaagaaaag aagatttaat      60

atatccaatt tttgtagttg aaaaagacga tgtgaaaaaa gaaattaagt cattgccagg     120

tgtataccaa atcagtttga atttacttga aagtgaatta aaagaagctt atgacttagg     180

catacgtgcc attatgtttt tcggtgttcc aaactcaaaa gatgatatag gtactggtgc     240

atacattcac gatggtgtta ttcaacaggc aacacgtatt gctaaaaaaa tgtatgatga     300

cttattaatt gttgcagaca cttgtttatg tgaatatact gatcatggtc attgtggcgt     360

gattgatgac catacacatg acgttgacaa tgataaatca ttgccactac ttgttaaaac     420

agcaatttct caagtggaag ctggtgctga tattattgcg ccaagtaata tgatggatgg     480

ttttgttgct gaaattcgtc gtggattaga tgaagccggc tattacaata ttcctataat     540

gagttatggt gtcaagtatg catcaagttt ctttggacct tttagagatg cagcagattc     600

agcgccatca tttggggata gaaaaacgta tcagatggac cctgctaacc gtttggaagc     660

acttcgtgaa ttagaaagtg atcttaaaga agggtgcgac atgatgattg ttaaacctgc     720

tctaagttat ttagatatag ttcgagatgt taaaaa                               756


<210>  63
<211>  200
<212>  DNA
<213>  Staphylococcus aureus

<400>  63
gtgccaattg caggatatgc tacaatctca gatcaaaacg aaatcgaatt tacaggttta      60

attatgaccc cagatggtaa agaacgattt gaatatacaa tgaacggaac agatccggtt     120

gagttaggca aaacagtgag taacaaatta aaagagcaag gtgcttatga aattataaaa     180

cgcttaaatg aacaacatta                                                 200


<210>  64
<211>  452
<212>  DNA
<213>  Staphylococcus aureus

<400>  64
ttgataacat tgctgtgata ggaagtaaga cagcgcaata ttgtgaatca cttggcattc      60

gagttgattt tatgccaaac gacttttctc aagaaggatt tttaaaatca tttaatcaaa     120

ctaaccaaaa aatacttttg ccttcgagtg aattggcgag accattgtta ttagcagcgt     180

tatctaaaga taatgaagtt gttaaaatag atttatatac ttcagtgcct aacaaacaaa     240

atatacaaga tgttaaagaa atgatagaac atcaacaaat cgatgcatta acattttcaa     300

gttcgtcggc agtacgttat tattttaatg aaggatttgt accaaaattc aagtcgtatt     360

ttgctattgg agaacaaaca gcacggacca ttaaatcata tcaacaacca gtaacaattg     420

cagaaattca aacactcgaa tcactaattg aa                                   452


<210>  65
<211>  757
<212>  DNA
<213>  Staphylococcus aureus

<400>  65
tcttccattc tctcagtcaa agaaggttta tttgatacta aaattgcttt actttcttta      60

tttatagctt tgatataatg attcactgga ttgatattcg tataacgcac accatctaca     120

taaccacttg cacctgctcc aaatccataa tattcctcat taaaccagta aaccttatta     180

tgttctgatt catggccatc taatgcaaaa ttagatattt cgtattgatg gaaaggagat     240

tgttctatct tagacatcaa caactgatac atgtcagcac ctaaatcctc attaggaagt     300

ttaagcaacc cttttctata catattataa aattgggttt taggttcaag tattaagccg     360

taactcgaaa tatgttgaat atccatatct aaagctagat ctaaactttg ttcaaaatct     420

tcaatcgtct gtttcggtaa atgatacatt aaatctaaac tgattgattt aatacctgcg     480

tttttagcat ttaacaccga agtgtaaata tcttcagtat tgtgcgttct acctaaaaca     540

gacaataact ccggtttgaa tgtttgaacg cccattgaaa ttctatttac tccatatttc     600

tctaatagtt ggactttctc tttagttaac tcatcaggat ttgcttcaaa tgtatactcg     660

cctgtgattg taaacgtatc acgtattgct ttaagtaatc tttccaactg attaatagaa     720

agggccgttg gtgtgccgcc acctacatac atggtct                              757


<210>  66
<211>  464
<212>  DNA
<213>  Staphylococcus aureus

<400>  66
agggcaaatg ctttcagtaa ctataaatag tggcattata aaatttagtg aattggatag      60

aaaagataat tcaagtaaag ataaaagtaa ttataaagta gttaggaaaa atgatattgc     120

atataattct atgagaatgt ggcaaggggc tagtggtaaa tcaaattata atgggattgt     180

tagccctgca tatactgtgc tttatccaac acaaaatact agctcattat ttattggata     240

taagtttaaa acacatagaa tgattcataa atttaaaatt aattcacaag gattaacatc     300

agatacatgg aacttaaaat ataaacaatt aaaaaatata aatatagata tacctgtatt     360

ggaggaacaa gaaaagatag gtgatttctt taaaaaaatg gatatattga taagtaaaca     420

gaaaatgaaa attgaaatat tagaaaaaga gaaacaatcc tttt                      464

<210>  67
<211>  533
<212>  DNA
<213>  Staphylococcus aureus

<400>  67
gtgccagagt tgagattccc agggtttgaa ggcgaatggg aagagaagca gttgggggat      60

cttacagata gagtaattag gaaaaataaa aacttagaat cgaaaaagcc tttaacaata     120

tccggacagt taggtttaat tgatcaaaca gaatatttta gtaaatcagt ttcgtcgaaa     180

aatctagaaa attatacact aataaagaat ggagaattcg cgtataacaa aagttattct     240

aatggatacc cattaggggc tattaaaaga ttaactagat atgatagtgg tgtattgtcc     300

tctttgtata tttgtttttc tattaaaagt gaaatgtcta aagacttcat ggaagcatat     360

tttgattcga cacactggta tagagaagtt tctggaattg cagttgaggg tgcaagaaat     420

cacggattat taaatgtttc tgtgaatgat ttttttacta ttctaattaa atatccaagt     480

ttagaagaac agcaaaaaat aggcaagttc ttcagcaaac tcgaccgaca aat            533


<210>  68
<211>  721
<212>  DNA
<213>  Staphylococcus aureus

<400>  68
tgcatcttcc attttaatag ctacattact atttttaagt ggtggacaag cacaagcagc      60

tgagaagcaa gtgaatatgg gaaattcaca ggaggataca gttacagcac aatctattgg     120

ggatcaacaa actagggaaa atgctaatta tcaacgtgaa aacggtgttg acgaacagca     180

acatactgaa aatttaacta agaacttgca taatgataaa acaatatcag aagaaaatca     240

tcgtaaaaca gatgatttga ataaagatca actaaaggat gataaaaaag catcgcttaa     300

taataaaaat attcaacgtg atacaacaaa aaataacaat gctaatccta gcgatgtaaa     360

tcaagggtta gaacaggcta ttaatgatgg taaacaaagt aaagtggcgt cacagcaaca     420

gtcaaaagag gcagataata gtcaagattc aaacgctaat aacaatctac cttcacaaag     480

tcgaataaag gaagcaccat cattaaataa gttagatcaa acaagtcaac gagaaattgt     540

taatgagaca gaaatagaga aagtacaacc acaacaaaat aatcaagcga atgataaaat     600

tactaactac aattttaaca atgaacaaga agtgaaacct caaaaagacg aaaaaacact     660

atcagtttca gatttaaaaa acaatcaaaa atcaccagta gaaccaacaa aggacaatga     720

c                                                                     721


<210>  69
<211>  416
<212>  DNA
<213>  Staphylococcus aureus

<400> 69
ttgacagctt tgcattttta taaatatagt gagccattta agtcacaaat tgtaacaccg 60

aaagtcactt taacgcatcg tgattgtttg tttatcgaat tgattgatga caaaggaaat 120

gcatatttcg gggaatgtaa cgcttttcaa acagattggt atgatcatga aacaattgcc 180

tcagtgaaac atgtaattga gcaatggttc gaagataata gaaataaatc atttgaaacg 240

tatgaagcag cactaaaatt agtagattca ttggaaaata cgcctgctgc aagggcaact 300

attgtcatgg cattgtatca aatgtttcat gtactgcctt cattttcagt agcatatgga 360

gcgacagcga gcggcttatc aaataaacaa ctagagtcat taaaagcaac aaagcc 416

<210> 70
<211> 400
<212> DNA
<213> Staphylococcus aureus

<400> 70
gtattattgc ttggggtgat gatgaacatc tacgtaaaat tgaagcagat gttccaattt 60

attattatgg atttaaagat tcggatgaca tttatgctca aaatattcaa attacggata 120

aaggtactgc ttttgatgtg tatgtggatg gtgagtttta tgatcacttc ctgtctccac 180

aatatggtga ccatacagtt ttaaatgcat tagctgtaat tgcgattagt tatttagaga 240

agctagatgt tacaaatatt aaagaagcat tagaaacgtt tggtggtgtt aaacgtcgtt 300

tcaatgaaac tacaattgca aatcaagtta ttgtagatga ttatgcacac catccaagag 360

aaattagtgc tacaattgaa acagcacgaa agaaatatcc 400

<210> 71
<211> 613
<212> DNA
<213> Staphylococcus aureus

<400> 71
tggctatcag taatgtttcg aaagggcaat acgcaaagag gtttttcttt ttcgctacta 60

gttgcttagt gttaacttta gttgtagttt caagtctaag tagctcagca aatgcatcac 120

aaacagataa cggcgtaaat agaagtggtt ctgaagatcc aacagtatat agtgcaactt 180

caactaaaaa attacataaa gaacctgcga ctttaattaa agcgattgat ggtgatacgg 240

ttaaattaat gtacaaaggt caaccaatga cattcagact attattggtt gatacacctg 300

aaacaaagca tcctaaaaaa ggtgtagaga aatatggtcc tgaagcaagt gcatttacga 360

aaaaaatggt agaaaatgca aagaaaattg aagtcgagtt tgacaaaggt caaagaactg 420

ataaatatgg acgtggctta gcgtatattt atgctgatgg aaaaatggta aacgaagctt 480

tagttcgtca aggcttggct aaagttgctt atgtttacaa acctaacaat acacatgaac 540

aacatttaag aaaaagtgaa gcacaagcga aaaaagagaa attaaatatt tggagcgaag 600

acaacgctga ttc 613

<210> 72
<211> 212
<212> DNA
<213> Staphylococcus aureus

<400> 72
atggtcaagc tgctgaagtt gattacattg gtatgccagc agtatgcttt actgaacctg 60

aattagctac agttggttat tcagaagcgc aagctaaaga agaaggttta gcaattaaag 120

cttctaaatt tccatatgca gcaaatggtc gtgcattatc attagacgat actaacggat 180

ttgttaaact tattacactt aaagaagatg at 212

<210> 73
<211> 763
<212> DNA
<213> Staphylococcus aureus

<400> 73
tggaagacat cgtaaacgta gaaactacgc gagaatttca gaagtattag aattaccaaa 60

cttaatagaa attcaaacta aatcttacga gtggttccta agagaaggtt taatcgaaat 120

gtttagagac atttctccaa ttgaagattt tactggtaat ttgtcattag agtttgtgga 180

ttaccgttta ggagaaccaa aatatgattt agaagaatct aaaaaccgtg acgctactta 240

tgctgcacct cttcgtgtaa aagtgcgtct aatcattaaa gaaacaggag aagttaaaga 300

acaagaagtc tttatgggtg atttcccatt aatgactgat acaggtacgt tcgttatcaa 360

tggtgcagaa cgtgtaatcg tatctcaatt agttcgttca ccatccgttt atttcaatga 420

aaaaatcgac aaaaatggtc gtgaaaacta tgatgcaaca attattccaa accgtggtgc 480

atggttagaa tatgaaacag atgctaaaga tgttgtatac gtacgtattg atagaacacg 540

taaactacca ttaacagtat tgttacgtgc attaggtttc tcaagcgacc aagaaattgt 600

tgacctttta ggtgacaatg aatatttacg taatacttta gagaaagacg gcactgaaaa 660

cactgaacaa gcgttattag aaatctatga acgtttacgt ccaggtgaac caccaactgt 720

tgaaaatgct aaaagtctat tgtattcacg tttctttgat cca 763

<210> 74
<211> 500
<212> DNA
<213> Staphylococcus aureus

<400> 74
ggcagttgta ctcccacatg gtgtcttatt ccgtggtgcc gcagaaggcg tcattcgtcg 60

ttatttaatt gaagaaaaga actacttaga agccgtgatt ggcttaccag tgaatatttt 120

ctatgggaca agtattccaa catgtatctt agtatttaaa aaatgttgcc aacaagacga 180

caacgtatta tttatcgatg catccaatga ttttgaaaaa ggaaaaaatc aaaaccattt 240

aagcgatgcc caagtcgaac gtattattga cacatacaag cgtaaagaaa caattgataa 300

atacagttac agtgcgacat tacaagagat tgccgataac gattacaacc taaacatacc 360

gaggtatgtc gatacattcg aagaagaagc gccaattgat ttagatcaag tccaacaaga 420

tttgaaaaat atcgacaaag aaatcgcaga aattgaacaa gaaatcaatg catacctgaa 480

agaacttggg gtgttgaaag 500

<210> 75
<211> 468
<212> DNA
<213> Staphylococcus aureus

<400> 75
tgaatagaaa tactaggacc acaaccggtt atttttcaat agaagaaata gattcaagaa 60

aaagccttga tgaaagagaa acagaaaaaa agtatcctgt gaaaatgata aacaataaaa 120

ttattccaac tgaggagata aaagatgaaa agttgaaaaa ggaaattgaa aactttaagt 180

tttttgtgca atatggcagt tttaaaggaa tagagaatta tgaaaatggt gacatttcct 240

ataattctga agctcctatt tattcagcga aatataaact gaaaaatgat gattataatg 300

ttaaagaatt acgaaaaaga tataatattc caacagaaaa ggcgcctaaa ttgttgttga 360

aaggttcggg ggatttgaaa gggtcttcag ttggatataa ggaaattgaa tttatattta 420

tagaaaataa aaaagaaaat atatattttt cagatggatt aaacttaa 468

<210> 76
<211> 512
<212> DNA
<213> Staphylococcus aureus

<400> 76
ggtgtattag ataatgaagg tatggtttta aatttggata gaaatacacg aacggccaag 60

ggatattatt ttgtagatac tatatatgac aatcatgaaa actcttatag taaaaattat 120

agagttgaga tgaaaaacaa taaaattatt ttattagaca aggtggaaga tcaaaaactt 180

aaagaaagaa tagaaaactt taaatttttc ggacaatatg ccgatttcaa gagtttgaaa 240

agttacaacc atggcgacgt ttcaattaat agtaatgttc caagttatga cgcgaaattt 300

aaaatgagta ataaagatga aaatgttaag caattaagaa gccgttataa cattcctact 360

gataaagctc caatattaaa aatgcatatt gatggggact taaaaggcag ttccgttgga 420

tataaaaagt tagaaataga cttttcaaaa gaagaaaata gcgaattatc aatagtcgat 480

tcattaaatt ttcaacctgc caaaaataaa ga 512

<210> 77
<211> 502

<212>  DNA
<213>  Staphylococcus aureus

<400>  77
aaccaaaagg cgagagttta aaatcacgag gaatgatatt aaagttagat agaaataaga      60

gaactgctaa aggaagttat attattagag aattgaaaga agataaaaat catgatgttc     120

aaaaaaatga aaagaaatat ccagtgaaat tggtgaataa taggatagtt ttggtaaaag     180

atgttaaaga caaaaagtta aaaaatgaaa tagagtcgtt tgaattattt tcacaatatg     240

gaaactttaa tcattttgat cggaatgaga ttactaatat ttcatataat cctaatgctc     300

ccaattactc tgcagaatat aaaatgaaga aaaatgacag aaacattcaa cagttgaaaa     360

agagatttaa tctaaaaact agcaagacac caaaattatt gtttaaggga tctggagata     420

taaaggggtc ttctgtagga tataaggaaa tagaaatcat atttagtaga agtaaagaag     480

aagcatttat tatgttgaca gc                                              502


<210>  78
<211>  400
<212>  DNA
<213>  Staphylococcus aureus

<400>  78
gcgaaagagt cgaaatcagc taatgaaatt tcacctgagc aaattaacca atggattaaa      60

gaacaccaag aaaataagaa tacagatgca caggataagt tagttaaaca ttaccaaaaa     120

ctaattgagt cattggcata taaatattct aaaggacaat cacatcacga agatttagtt     180

caagttggta tggttggttt aataggtgcc ataaatagat tcgatatgtc ctttgaacgg     240

aagtttgaag cctttttagt acctactgta atcggtgaaa tcaaaagata tctacgagat     300

aaaacttgga gtgtacatgt tccgagacgt attaaagaaa ttgggccaag aatcaaaaaa     360

gtgagcgatg aactaaccgc tgaattagag cgttcacctt                           400


<210>  79
<211>  529
<212>  DNA
<213>  Staphylococcus aureus

<400>  79
ccgttacgtt gttcttcagt taagttaggt aaatgtaaaa tttcatagaa agcattttgt      60

tgttctttgt tgaatttgtt gtcagctttt ggtgcttgtg catcatttag ctttttagct     120

tctgctaaaa ggttagcgct ttggcttggg tcatctttta agctttggat gaaaccattg     180

cgttgttctt cgtttaagtt aggtaaatgt aagatttcat agaaagcatt ttgttgttct     240

ttgttgaatt tgttatccgc tttcggtgct tgagattcat ttaacttttt agcttctgac     300

aataggttag cactttgact tgggtcatct tttaagcttt ggatgaaacc attgcgttgt     360

tcttcgttca agttaggcat gttcaagatt tcatagaaag cattttgttg ttctttgttg     420

aaattgttgt cagctttcgg tgcttgagat tcgtttaatt ttttagcttc acctaaaacg     480

ttagtgcttt ggcttggatc gtctttaaga ctttgaatga aaccattgc                529


<210>  80
<211>  528
<212>  DNA
<213>  Staphylococcus aureus

<400>  80
tgatattgga agatattagc atagatatcg aaaaaggtaa attgacttct ttaattggac     60

ctaatggtgc gggtaagagt actttacttt cagcgatttg taggttaatt cgttttgata    120

acggtgaagt gaaaatagat ggacggctca tgtctgatta taaaaataat gacttgtcga    180

aaaaaatatc tatattaaaa caaacaaacc atactgaaat gaatattacg gtagagcagt    240

tggtaaactg tggacgattc ccttattcta aaggtcgttt gacgaaagag gatcatgata    300

ttgtcaatga tgcgctagat ttgttgcaac tacaagatat cagaaatcgt aatattaagt    360

cattatctgg tggacaacgt cagcgtgcat atattgcaat gacaatagca caagatactg    420

aatatatttt gctagatgaa ccattaaata atttagatat gaagcatgct gttcaaatta    480

tgcaaacgtt aaaaatgtta gcgcataaaa tgaataaagc gattgtca                528


<210>  81
<211>  513
<212>  DNA
<213>  Staphylococcus aureus

<400>  81
ttttgattta tcttctgacg gtttaaaata accattcttt acccattctt cataacgtcc     60

cgcttcaact tcacgaggat catattttgg tttcatttcc atttgctata cctcctaaaa    120

aaataaaaat atccatccta tatacaaata ggacggatat tccgtggtac cacctatatt    180

caagaaggat gattaatatc aaattcactc ttttaacata attggaataa tcataccaat    240

actatcatcg tgaaatttga aatgcttcat ctcttcaagc actctagatt atgattaacg    300

ctcaaacacg tcttagccta ctattaatca cgttcagcta agatactctg tgggctacct    360

tcagtaagaa aatcatttac atactcacac caaatcatat gctctcttta aaataatttg    420

aacttactct tcccaaatcc tatattaaac tcttaactta tagtataatg attgacaaaa    480

taagtcaatg tataggtggg aataaaatga atg                                 513


<210>  82
<211>  361
<212>  DNA
<213>  Staphylococcus aureus

<400>  82
tggatataac aatcaaaatc actcaatgct tgcataccgc gttctcggtc agtagggttt     60

ttgaaactaa tttttaaagc accgtatata tcttcgcgta cttctaagat tcttaagttg 120

cttatagata tgttatgtaa actcaggata taagtcactt tacttatcat acctgattca 180

tccggaatgt ctacatatag atcatacgca gtatttagtc cacctagttg tttagcgggt 240

agtgcgtcgc gatacgattt agcttgggca aaaaatgata acaatttttc agaatcattg 300

ctttcaatta gtctttctaa atcttgaaac tgacttttta gctgtcgaat catttctaaa 360

a 361

<210> 83
<211> 731
<212> DNA
<213> Staphylococcus aureus

<400> 83
atgagatacc taacatcagg agaatcacat ggacctcaat taacagttat tgttgaaggt 60

gtacctgcaa atttagaagt taaggttgag gatattaata aagaaatgtt taagcgtcaa 120

ggcggttacg gacgtggacg tcgtatgcaa attgaaaaag atacagtgga gattgtttcg 180

ggtgtaagaa atggttatac attaggtagc cctattacaa tggttgttac taatgatgat 240

tttacacatt ggcgaaaaat tatgggccgt gcgccaataa gcgacgaaga acgagaaaat 300

atgaaacgta caattacgaa gccaagaccg ggacatgcag atttacttgg cggtatgaaa 360

tataatcatc gtgacttacg aaatgtatta gaacgttcat ctgccagaga aacagcagca 420

cgtgtagcgg tcggtgcact atgcaaagtt ttattagaac aattagatat cgaaatatac 480

agtcgtgttg ttgagatagg tggcattaaa gataaagatt tttatgattc agaaacattt 540

aaagcaaacc ttgatcgaaa tgacgtccgt gtaattgatg atggcatcgc acaagcaatg 600

cgcgataaaa ttgatgaagc gaaaacagat ggtgattcaa tagggggcgt agttcaagtt 660

gtagttgaaa atatgcctgt tggtgtaggt agttatgtac attatgatcg taaattagat 720

ggaagaatag c 731

<210> 84
<211> 254
<212> DNA
<213> Staphylococcus aureus

<400> 84
accttcaata ttcgcatcca taagtttcaa tggtctcaag acacgatcca ttggcctttt 60

accaattgaa acatcgccag acaaaacact ttcaatacct aaaccactta acaaaccagc 120

taacaatcga gtcgttgtgc cagagtttcc agtatataaa acttgatgag gtgttttaaa 180

agctttatat ccaggtgaat tcacaaccaa tttatcttca tcttctttaa tatctacgcc 240

taataatcgg aata 254

<210> 85
<211> 716
<212> DNA
<213> Staphylococcus aureus

<400> 85
tcgaggaatt aacaaaggtc aaaggttata caacacatgt ggataacaat gatatgggca 60

acttgattgt gacgaataaa tatacgccag aaacaacatc aattagtggt gaaaaagtat 120

gggacgacaa agacaatcaa gatggtaaga gaccagaaaa agtcagtgtg aatttattgg 180

ctaacggaga gaaagtaaaa acgttagacg tgacatctga aacaaactgg aagtacgaat 240

ttaaagactt accgaagtat gatgaaggaa agaaaataga atatacagtg accgaagatc 300

acgtaaaaga ctacacaaca gacatcaacg gtacgacaat aacgaacaag tatacaccag 360

gagagacatc ggcaacagta acaaaaaatt gggatgacaa taataaccaa gacggaaaac 420

gaccaactga aatcaaagtt gagttatatc aagatggaaa agcaacagga aaaacggcaa 480

tattaaatga atctaataac tggacacata cgtggacagg attagatgaa aaagcaaaag 540

gacaacaagt aaaatacaca gtcgatgaat taacaaaagt taatggctat acaacgcatg 600

tggataacaa tgatatgggt aacttgattg tgacaaataa atatacgccg aaaaaaccga 660

ataaaccaat ctatcctgaa aaaccaaaag acaaaacacc accaactaaa cctgat 716


<210> 86
<211> 581
<212> DNA
<213> Staphylococcus aureus

<400> 86
gaacctagcc atcaagacag tacacctcaa catgaagagg aatattataa taagaatgct 60

tttgcaatgg ataaatcaca tccagaacca atcgaagaca atgataaaca cgatactatt 120

aaaaatgcag aaaataacac tgagcattca acagtttctg ataagagtga agctgaacaa 180

tctcagcaac ctaaaccata ttttacaaca ggtgctaacc aatcagaaac atcaaaaaat 240

gaacatgata atgattctgt aaaacaagat caagatgaac ctaaagaaca tcataatggt 300

aaaaaagcag cagctattgg tgctggaaca gcaggtgttg caggtgcagc tggtgcaatg 360

gctgcttcta aagctaagaa acattcaaat gacgctcaaa acaaaagtaa ttctggcaag 420

gcgaataact cgactgagga taaagcgtct caagataagt ctaaagatca tcataatggc 480

aaaaaaggtg cagcgatcgg tgctggaaca gcaggtttgg ctggaggcgc agcaagtaaa 540

agtgcttctg ccgcttcaaa accacatgcc tctaataatg c 581


<210> 87
<211> 530
<212> DNA
<213> Staphylococcus aureus

<400>  87
tcgtgcatta gtaccatcag gtgcttcaac tggtgaacac gaagctgttg aattacgtga      60

tggagataaa tcacgttatt taggtaaagg tgttactaaa gcagttgaaa acgttaatga     120

aatcatcgca ccagaaatta ttgaaggtga attttcagta ttagatcaag tatctattga     180

taaaatgatg atcgcattag acggtactcc aaacaaaggt aaattaggtg caaatgctat     240

tttaggtgta tctattgcag tagcacgtgc agcagctgac ttattaggtc aaccacttta     300

caaatattta ggtggattta atggtaagca gttaccagta ccaatgatga acatcgttaa     360

tggtggttct cactcagatg ctccaattgc attccaagaa ttcatgattt tacctgtagg     420

tgctacaacg ttcaaagaat cattacgttg gggtactgaa attttccaca acttaaaatc     480

aattttaagc aaacgtggtt tagaaactgc agtaggtgac gaaggtggtt                530


<210>  88
<211>  560
<212>  DNA
<213>  Staphylococcus aureus

<400>  88
cgccaaaata gtgcttcaat atcagatagt tattattggg atatcattaa aaatctagaa      60

ttacaattta ctgctgcatt agatttatta gaagattatc gatatggtga aaaagagtat     120

gaaaaagcaa aagatcaact aatgacaagg atattaagtg aagtcaagta tttacttgag     180

caaaaaatta aagaatatga caagtataaa gatttatata aagaatatat gagtaaaaat     240

ccaacgtcaa aggtaaaaag agcaaatttt gatcaatata atatcgaaga cctaagagaa     300

aaagaatata atgatttact aagttctatt aaagatgcgg tagaaacatt taaatcagat     360

gtacaaaaaa tagaatatga aaataaagag ttaaaatctt attcttacga agaagaaaag     420

aaggctgctt ctagagttga tgatttagca aataaagcgt atagcgttta ttttgcgttt     480

gttagggata cacaacataa aactgaggca ttagagttaa aagcgaaagt ggatttagtt     540

ttaggtgatg aggacaaacc                                                  560


<210>  89
<211>  462
<212>  DNA
<213>  Staphylococcus aureus

<400>  89
tgaaaaataa attgatagca aaatctttat taacaatagc ggcaattggt attactacaa      60

ctacaattgc gtcaacagca gatgcgagcg aaggatacgg tccaagagaa aagaaaccag     120

tgagtattaa tcacaatatc gtagagtaca atgatggtac ttttaaatat caatctagac     180

caaaatttaa ctcaacacct aaatatatta aattcaaaca tgactataat attttagaat     240

ttaacgatgg tacattcgaa tatggtgcac gtccacaatt taataaacca gcagcgaaaa     300

ctgatgcaac tattaaaaaa gaacaaaaat tgattcaagc tcaaaatctt gtgagagaat 360

ttgaaaaaac acatactgtc agtgcacaca gaaaagcaca aaaggcagtc aacttagttt 420

cgtttgaata caaagtgaag aaaatggtct tacaagagcg aa 462

<210> 90
<211> 584
<212> DNA
<213> Staphylococcus aureus

<400> 90
aatcctcata acgcagaaag agtaaccttg aaatataaat ggaaatttgg agaaggaatt 60

aaggcgggag attattttga tttcacatta agcgataatg ttgaaactca tggtatctca 120

acactgcgta aagttccgga gataaaaagt acagatggtc aagttatggc gacaggagaa 180

ataattggag aaagaaaagt tagatatacg tttaaagaat atgtacaaga aaagaaagat 240

ttaactgctg aattatcttt aaatctattt attgatccta caacagtgac gcaaaaaggt 300

aaccaaaatg ttgaagttaa attgggtgag actacggtta gcaaaatatt taatattcaa 360

tatttaggtg gagttagaga taattgggga gtaacagcta atggtcgaat tgatacttta 420

aataaagtag atgggaaatt tagtcatttt gcgtacatga aacctaacaa ccagtcgtta 480

agctctgtga cagtaactgg tcaagtaact aaaggaaata aaccaggggt taataatcca 540

acagttaagg tatataaaca cattggttca gacgatttag ctga 584

<210> 91
<211> 545
<212> DNA
<213> Staphylococcus aureus

<400> 91
gctggtgtgg tacttatcct agtggcagca tatttgtttg ctaaaccaca tatcgataat 60

tatcttcacg ataaagataa agatgaaaag attgaacaat atgataaaaa tgtaaaagaa 120

caggcgagta aagataaaaa gcagcaagct aaacctcaaa ttccgaaaga taaatcgaaa 180

gtggcaggct atattgaaat tccagatgct gatattaaag aaccagtata tccaggacca 240

gcaacacctg aacaattaaa tagaggtgta agctttgcag aagaaaatga atcactagat 300

gatcaaaata tttcaattgc aggacacact ttcattgacc gtccgaacta tcaatttaca 360

aatcttaaag cagccaaaaa aggtagtatg gtgtacttta aagttggtaa tgaaacacgt 420

aagtataaaa tgacaagtat aagagatgtt aagcctacag atgtaggagt tctagatgaa 480

caaaaaggta aagataaaca attaacatta attacttgtg atgattacaa tgaaaagaca 540

ggcgt 545

<210> 92

<211>  527
<212>  DNA
<213>  Staphylococcus aureus

<400>  92
ttaacaatag aacatttaac aaagaagata ggcaacaaaa cgattctcga agatgtatca      60

tttaagctga aacgcggaca aatagttggt ctcgttggag cgaatggtgc aggtaaaaca     120

actttaatga aagttatatt aggttactct agtttccaaa gcgggaattt taatgttatt     180

aacagcaagg acgaaaaaag caatatcggc gcattgattg aaaatccagg aatatatcct     240

tttatgtctg gatatgaaaa cttgaagtta ttgaatgaat caaaaaacac tcaagatatc     300

gataaaattg tctcacaact tcatatggat gaatacattc ataaaaaagc taaaacgtat     360

tctcttggta tgaaacaaaa attaggaatt gctatagcat ttttaaataa acctcaattc     420

attatcttag atgaaccaat gaatggctta gatccaaaag ctgtgcgaga tgtacgtgaa     480

ttgattgtcc aaaaagcgca agaaggtgtt actttcttaa tttcgag                   527


<210>  93
<211>  645
<212>  DNA
<213>  Staphylococcus aureus

<400>  93
aaatggttca gtcgtaatgg cgacaggtga agttttagaa ggtggaaaga ttagatatac      60

atttacaaat gatattgaag ataaggttga tgtaacggct gaactagaaa ttaatttatt     120

tattgatcct aaaactgtac aaactaatgg aaatcaaact ataacttcaa cactaaatga     180

agaacaaact tcaaaggaat tagatgttaa atataaagat ggtattggga attattatgc     240

caatttaaat ggatcgattg agacatttaa taaagcgaat aatagatttt cgcatgttgc     300

atttattaaa cctaataatg gtaaaacgac aagtgtgact gttactggaa ctttaatgaa     360

aggtagtaat cagaatggaa atcaaccaaa agttaggata tttgaatact tgggtaataa     420

tgaagacata gcgaagagtg tatatgcaaa tacgacagat acttctaaat ttaaagaagt     480

cacaagtaat atgagtggga atttgaattt acaaaataat ggaagctatt cattgaatat     540

agaaaatcta gataaaactt atgttgttca ctatgatgga gagtatttaa atggtactga     600

tgaagttgat tttagaacac aaatggtagg acatccagag caact                     645


<210>  94
<211>  548
<212>  DNA
<213>  Staphylococcus aureus

<400>  94
ggtattgcat ctgtaacttt aggtacatta cttatatctg gtggcgtaac acctgctgca      60

aatgctgcgc aacacgatga agctcaacaa aatgcttttt atcaagtgtt aaatatgcct     120

aacttaaacg ctgatcaacg taatggtttt atccaaagcc ttaaagatga tccaagccaa 180

agtgctaacg ttttaggtga agctcaaaaa cttaatgact ctcaagctcc aaaagctgat 240

gcgcaacaaa ataagttcaa caaagatcaa caaagcgcct tctatgaaat cttgaacatg 300

cctaacttaa acgaagagca acgcaatggt ttcattcaaa gtcttaaaga cgatccaagc 360

caaagcacta acgttttagg tgaagctaaa aaattaaacg aatctcaagc accgaaagct 420

gacaacaatt tcaacaaaga acaacaaaat gctttctatg aaatcttgaa catgcctaac 480

ttgaacgaag aacaacgcaa tggtttcatc caaagcttaa aagatgaccc aagtcaaagt 540

gctaacct 548

<210> 95
<211> 304
<212> DNA
<213> Staphylococcus aureus

<400> 95
gttatcaatt aatacaaccc ctgaagcaat tcgatacatt aaacctgcag attttcatgt 60

tcctggcgat atttcatctg cagcgttctt tattgttgca gcacttatca caccaggaag 120

tgatgtaaca attcataatg ttggaatcaa tccaacacgt tcaggtatta ttgatattgt 180

tgaaaaaatg ggcggtaata tccaactttt caatcaaaca actggtgctg aacctactgc 240

ttctattcgt attcaataca caccaatgct tcaaccaata acaatcgaag gagaattagt 300

tcca 304

<210> 96
<211> 269
<212> DNA
<213> Staphylococcus aureus

<400> 96
gtagttgaaa atatgcctgt tggtgtaggt agttatgtac attatgatcg taaattagat 60

ggaagaatag cacagggtgt cgttagtatt aatgcattta aaggtgtaag ttttggagaa 120

ggatttaaag cagctgaaaa gcctggtagc gaaattcaag acgaaattct ctacaatact 180

gaattgggct attatcgtgg gtcaaatcac ttaggtggtt tagaaggcgg tatgtcaaat 240

ggaatgccaa ttatcgttaa tggtgtaat 269

<210> 97
<211> 305
<212> DNA
<213> Staphylococcus aureus

<400> 97
agacttatta tctaaacgtg gtgaactagc acaaaaaatt ggggaagaaa aattaaaaca 60

aggtacacgt atctatgatc cacaacgtga aaaagaaatg cttaacgact taatcgatag 120

taacaaagga ccattcaacg ataatactat taagcaatta tttaaagaaa ttttcaaagc 180

ctctacagat ttacaaaaat ctgaaaatga aaaacattta tatgtatcac gtaagttgaa 240

acctgaagat acgattgtaa catttgataa tgggggcatt attggagacg gcaataaatc 300

atttg 305

<210> 98
<211> 287
<212> DNA
<213> Staphylococcus aureus

<400> 98
aaaattgctg gtatcgctgc acgtgaagtt aaaggtatct tagacatgaa aggtggctta 60

actgatacat tcactaatgc attctcaagt ggaaataacg ttactcaagg tgtatctgtt 120

gaagttggtg aaaaacaagc tgctgtagac ttaaaagtaa ttttagaata tggtgaatca 180

gcacctaaaa tcttccgtaa agtaactgaa ttagtaaaag aacaagttaa atatattact 240

ggtttagatg ttgttgaagt taacatgcaa gttgacgatg taatgac 287

<210> 99
<211> 429
<212> DNA
<213> Staphylococcus aureus

<400> 99
agctgagacg acacaagatc aaactactaa taaaaacgtt ttagatagta ataaagttaa 60

agcaactact gaacaagcaa aagctgaggt aaaaaatcca acgcaaaaca tttctggcac 120

tcaagtatat caagaccctg ctattgtcca accaaaaaca gcaaataaca aaacaggcaa 180

tgctcaagta agtcaaaaag ttgatactgc acaagtaaat ggtgacactc gtgctaatca 240

atcagcgact acaaataata cgcagcctgt tgcaaagtca acaagcacta cagcacctaa 300

aactaacact aatgttacaa atgctggtta tagtttagtt gatgatgaag atgataattc 360

agaaaatcaa attaatccag aattaattaa atcagctgct aaacctgcag ctcttgaaac 420

gcaatataa 429

<210> 100
<211> 536
<212> DNA
<213> Staphylococcus aureus

<400> 100
cgggattctc tgcattatcc cccacggcaa cacccaaaat aaactcttca atgttaaaaa 60

caagacacaa atgactgata atactaagtt tattaatatt gatacgaaca caccaaagta 120

tcgagttaat aaaaagttga gcggtatcaa tggtagagat actacataca tcaacaatat 180

tgtcaccaat aacaacatag cattaaccgg atgtggatta ataattaggt cacctatata 240

agcaataata aatactaaaa agcaatgtac caaaaatgct attgataaaa tgaaaatctt 300

tgctcttatt tcttttgtaa tcgaccaatt attacttaag taataattaa atgatttatt 360

tctcatttca attttaaata acgaattaca agccatacat aatacaatcg ggatgaaagc 420

aattggccaa atattaaata gtaaagttat atatggtgac acactattcg ctgttcccgt 480

attacttttg gcgaataaga ctgtgaaaat agcaaaacaa agaaatacca gcggac 536

<210> 101
<211> 637
<212> DNA
<213> Staphylococcus aureus

<400> 101
ttaattgttc taccgctcca tttattaaat cctttaaaga gtaaaactgc taatagcaac 60

gtgataataa tatagattgc caatgttaat gtaactggta tactcccttc gataaacata 120

taaacgtaac gtgtagcata tgtgattggt aaatagaacc acgaatgatc tccaagcact 180

tctaatccaa aataaacgtt aaaaataaac attaaaactc cgacaacaat agccattaca 240

tctttaatga aaatactaaa aataaaaagt agcagtaata taattacatt gaaaaacaat 300

gatacgccta taaacataag tgttattttc atatcatgtg aatgccacaa taaattaatt 360

gatgctaata gaatacatat ggctgtaata gtataagtaa aaatcattga tgcatttaac 420

caatttagcc tattagcttt tcctaaaata tgattaaagt gaccaatatt ttcttcaaaa 480

ttgataactt gatagacgtt tatagaaatt aatagcgatg taattgcatt aaaactcgct 540

gtaaacaaac ttatttgtcg accattccat aaatttacgt ttaaatacca atttataaat 600

aatataaaca atatggttac aataatgggt acaaatg 637

<210> 102
<211> 507
<212> DNA
<213> Staphylococcus aureus

<400> 102
aaagataatt ggtttgctga aaaaccagtc ctctaaagaa tcgaatgtta agattcatcg 60

cttggcgtat attacaaact caaaatttga tggcaataac tatatagata gatggtgtaa 120

aatcaggaat tctcacattg gtgaatacag ttatattgga tttggtagtg attttaataa 180

tgtagaagta ggaagatatt gttcgatatc ttcggatgta aaaattgggt taggaaaaca 240

tcctacacac ttttttagct catcaccgat tttttattct aataataatc catttaacat 300

aaagcaaaag tttatagact ttaatgacca accaagccgt acaacaatta aaaatgatgt 360

gtggattggt gcaaatgtaa ttattatgga tggtttaaca ataaatactg gtgcagtcat 420

agcagccggc tcagttgtta ctaaaaatgt aggagcatat gaggttgttg gtggggttcc 480

tgcaaaagtg attaagaagc gatttga 507

<210>  103
<211>  639
<212>  DNA
<213>  Staphylococcus aureus

<400>  103
caagggtact taaacaaata gaaacaatta aagacgttac ggatgattat aaaattgttg      60

gaatgaataa ttcacaagct actaataagc gattggaaaa tttagattgt aattatcgtt     120

tgttaggtag caaggtagat ccaaaaaata ttctttctaa attaattaag cgtataagat     180

ttgcaacagg tgttatccga gaaattaaag cttataaacc tgacgtgatt catgcaaatg     240

atttcgacgt attattaatg gtctatttaa gcaattataa aaaagctaat attgtttatg     300

atgcgcatga aatatatgcg aaaaatgcct ttattaataa agttccactt atttcaaagt     360

ttgtagaaag tatagaaaaa cacatagtaa aacatcgtgt taatgccttc gtaacagtaa     420

gtcatgcagc aaaagaatat tatcaatcta aaggatataa gaaggaagcg aatgttatta     480

cgaatgcacc tattttaaat gatagcagag aatttaaaga aatcgaaaac tttaaagaaa     540

ttgtatatca aggtcaaatt gtaatggaca gaggatatga agagtttatt attgcttcat     600

cagcttttaa acaaaatgct ccttcattca taattcgag                            639


<210>  104
<211>  380
<212>  DNA
<213>  Staphylococcus aureus

<400>  104
actttgtgca attatcagca tgaacatatt tatagtaatc tctacattta ctaaagaagt      60

attagggttc cctatagagc cggtgtatta ctcaaccatg gttggtatag cattaattac     120

cacggtgttt gctatttata agataattgt cacgcaagaa attccgcgag ggttaatatt     180

attaattgct atatgtttgc tttatctagc tttttattat ttttcaccag ataaggaaga     240

gaaactagct aaaaataata ttctattctt tttaacatgg gcagttccag cggcaattag     300

tggtatttat attaaatata taaacaaggc tacggtagaa agatttttta aattagtatt     360

tttcatattt tctgtttcat                                                 380


<210>  105
<211>  500
<212>  DNA
<213>  Staphylococcus aureus

<400>  105
ttatggatag cgtaaagaca ataattggta cgttgcttat agctttagga ttacaatttt      60

tagcttatcc aattattaat caacgagtag gtaatgaagc gtttggttct attttaacga     120

tttatacaat aataacaatc acgagtgttg tattaggcaa tacgcttaac aatatacgat     180

tgattaatat gaatctatac aaatccaatc attactactg gaaatttgtg tcgatacttt 240

taatttcaat tctgattgag agtatagctt taattattgt atttctttac ttttttaatt 300

tgaacaccat cgatattatc tttttaattc tacttaatat tttaatgtgt ttaaggattt 360

atctgaatgt attttttagg atgactttaa aatataatca gattttgtat attgctctta 420

ttcaattttt aggtttgctg ataggactat ttctatatta tttaatccaa aactggattg 480

tttgttttat taccagtgaa 500

<210> 106
<211> 522
<212> DNA
<213> Staphylococcus aureus

<400> 106
gattcttggc gctactaaca ttaagcatat gtcattatta tcacattatt taaaccacat 60

tgatttgaat atcaatgagg tggacattat atacactgac aaatatgata tcgaagaaca 120

tatccaaggc atcaataatt actataaata taaagtagat attaaagaag attggacatt 180

tatcaaaaaa gctattgctt actatcgatt taggccatac gctatgaaaa ttcttaaaga 240

aaatcgttat gattttgtca tagtatgggg aagttataca ggacacttat ttaaaagttt 300

tttagaaaaa cactataaaa ataaattcat tttaaatata agggactact tttttgaaaa 360

taataaactt attaagtata gaatgaaaaa aatcgttgat gctagcaggg tgacaacatt 420

atcttcagaa ggttttctta aatttttacc taaatctgaa aaatatagaa ttatttatag 480

ttataacatg agtattatta gagaaagtaa tgtaaccgat gg 522

<210> 107
<211> 655
<212> DNA
<213> Staphylococcus aureus

<400> 107
taatgtttcc ttgccttatg ttaggtgata aacctttatt atttttagca cctataagtt 60

atggagtagg aaagctcttt ataagcttct cgaataatcc gaattttaaa ttttcgaaaa 120

ttgtatacga tgttttaggt tttcttagat tagtatttat acctgctatg atagtgtttt 180

tccaggattc aactatagat aatttaccat taggacaagc ttattttaat caagcggtta 240

tttatatgag tgtggagttt atcataggct cgctatttat attgatacta tctaaattat 300

tcaaacatga agtggtatca agaaatagct ttacactttc tggatcatca atttattaca 360

ttgtgtttgg tcttgttatt tgtgggattt ttgtagcttt tcccgaagtg cgcaaaaaca 420

tatcattttt aattattaaa acagatgcaa tgggaagagg aaccgaagca acaagtggtt 480

taaatgttct ttttgtaatg ctatttcaac ttgccttagc gttattattc ttaataatcg 540

catatgcttc atataaaaag tataaagaga atcctaaaat tatttatgtt gtattaccgc 600

tagctatagg aattttaaat attagtttaa ttgttggtga aagaagaagt tatca 655

<210> 108
<211> 459
<212> DNA
<213> Staphylococcus aureus

<400> 108
gtaaaaacat ttatgaaatc gaaaatattt agattaatga atacaccact attattattt 60

tataagaaag aatatttaac tggatattat tttgaaaata aagtggctgg atggttatgg 120

gcgtggaaag ctgttccgtt caagttgtta ggaataaata caagtttgcc atttcctgca 180

gatataactg ttagaatgca taaccctaat aacattgttt ttgataaaaa tgatattcat 240

atttttcaat cgcccgggac gtattttaat aatttttcag cagttatata tataggtaga 300

ggtgtttata tagcgcctaa cgtaggtatt attacagcta atcataatat taaaaatttg 360

aagtcacatg caccaggtga agatgtcaaa atagggaatt atagttggat tggaatgaac 420

tcagttatat taccaggagt agaattgggg gaacataca 459

<210> 109
<211> 562
<212> DNA
<213> Staphylococcus aureus

<400> 109
aagatacgat ttgttgattg tgaataccaa aaatgaccgt agtgctaata tactttcaca 60

aatcagtttt ttgatatcat tgcttatttt attaatactg ataccaatat ttgcgattag 120

tgcatgttta tacccaaact ttatattaga ttttattttc attattatta tgttgttttt 180

ggtaagttta acaaacattt ttacaaatta tctaaataag gaaagaaagt ataaagtgtt 240

aagtttgatt aatgtgttta gagctggatc aatggcttta cttcaaatca ttttcggact 300

tttagcatta ggaagtttag gattaattat tggtttttca ttatcctata tcgcaggcat 360

tacactagga tataaaacgt ttaaaaagca ctttaatatt gtgagagata aagaagaaac 420

taaagcatta tttttagaaa ataaaaatca gttagtttat tcaacaccat caatattatt 480

aaatagtttg tctttctcgg ttgttgtgtt ctttataggt attttgtata ccaatacaga 540

agtgggtatt tatggtatgg cc 562

<210> 110
<211> 104
<212> DNA
<213> Staphylococcus aureus

<400> 110
ttttatctta attaaggaag gagtgatttc aatggcacaa gatatcattt caacaatcag 60

tgacttagta aaatggatta tcgacacagt gaacaaattc acta                    104

<210>  111
<211>  351
<212>  DNA
<213>  Staphylococcus aureus

<400>  111
aaatatcaaa tcgctgtggc tgatacgaat gttcaaacgc cagattatga aaagttgagg      60

aacacatggc tggacgttaa ctatggttat gataagtatg atgagaagaa tgacgcaatg     120

aagaagaagt ttgaggctac ggagaatgag gcaaagaaat tacttagtga gatgaaaact     180

gaaagtgata ggaaatactt gtgggaaaac tcaaaagatt tagatacgaa gtctgcggat     240

atgactcgta cctatcgtaa tattgagaaa atcgcagaag cgatgaagca taaagatact     300

aagttaaaaa tagatgaaaa caagaagaaa gtgaaagatg cccttgagtg g              351

<210>  112
<211>  278
<212>  DNA
<213>  Staphylococcus aureus

<400>  112
gggttcttgc tgtctttaag tgattcagag aatacttctt gtgcacgttc tgggtgttcg      60

cgtaatgttt tgatgtattg gttacgttgt tcttctgtga taccttttag atgtaatact     120

tgataaaaag ctttttgttg atctgttacg tagttgtttt gagttgtttg gtgcttagtt     180

gaagtttgtt gcgtgttttc actcgctttt gcttccccat ttgaaatcat tgtagctaaa     240

gtaattgttg ctgccccaac tagcaacttc gagatata                             278

<210>  113
<211>  226
<212>  DNA
<213>  Staphylococcus aureus

<400>  113
aaagatagtt ctaagataaa tggtccatta agactcgcag gtggagatat taataagcta      60

gattcaacaa ctcaagacaa agtaagaaga ttagattcat ctatttctaa atctactact     120

cctgaatctg tatacgttta tagactttta aatttagatt atttgacaag tatcgttgga     180

tttacaaatg aagatttata taaattacaa cagaccaata atggcc                    226

<210>  114
<211>  576
<212>  DNA
<213>  Staphylococcus aureus

<400>  114
gctagtgcat ttgttattca agacgaactg atgcaaaaaa accatgcaaa agcagaagtt      60

tcagcagaag aaataaaaaa acatgaagag aaatggaata agtactatgg tgtcaatgca     120

tttaatttac caaaagagct ttttagtaaa gttgatgaaa aagatagaca aaagtatcca 180

tataatacta taggtaatgt ttttgtaaaa ggacaaacaa gtgcaactgg tgtgttaatt 240

ggaaaaaata cagttctaac aaatagacat atcgctaaat ttgctaatgg agatccatct 300

aaagtatctt ttagaccttc tataaataca gatgataacg gtaatactga aacaccatat 360

ggagagtatg aagtcaaaga aatattacaa gaaccatttg gtgcaggtgt tgatttagca 420

ttaatcagat taaaaccaga tcaaaacggt gtttcattag gcgataaaat atcgccagca 480

aaaataggga catctaatga tttaaaagat ggagacaaac tcgaattaat aggctatcca 540

ttcgatcata aagttaacca aatgcacaga agtgaa 576

<210> 115
<211> 630
<212> DNA
<213> Staphylococcus aureus

<400> 115
ttttagcagc gtcaattttt actatttcct tacctgtgat tccttttgaa agtacattac 60

aagcaaaaga atacagcgca gaagaaatca gaaaattaaa acaaaaattt gaggttccac 120

ctacagataa agagctttat acacacatta cggataatgc aagaagtcct tataattctg 180

ttggtacagt gtttgtcaaa ggtagtacat tagctaccgg agttttaatt ggtaaaaata 240

caattgttac taattaccac gttgcaagag aagcagccaa aaacccatcg aatattattt 300

ttacacccgc tcaaaataga gatgcagaaa aaaatgaatt ccctactccg tatggaaaat 360

ttgaagctga agaaattaaa gaatctccgt atggacaagg actcgattta gctataataa 420

aattaaaacc aaacgaaaaa ggggaatcag cgggagattt aattcaacca gctaatatac 480

ctgatcatat tgatatacaa aaaggagaca aatattcttt attaggatat ccttataatt 540

attcagctta ctctttatat caaagtcaga ttgaaatgtt caatgattct caatattttg 600

gatatactga ggtaggaaac tctggatcag 630

<210> 116
<211> 330
<212> DNA
<213> Staphylococcus aureus

<400> 116
agaaagaaag tgatttctat gattaaaaat aaaatattaa cagcaacttt agcagttggt 60

ttaatagccc ctttagccaa tccatttata gaaatttcta aagcagaaaa taagatagaa 120

gatatcggcc aaggtgcaga aatcatcaaa agaacacaag acattactag caaacgatta 180

gctataactc aaaacattca atttgatttt gtaaaagata aaaaatataa caaagatgcc 240

ctagttgtta agatgcaagg cttcattagc tctagaacaa catattcaga cttaaaaaaa 300

tatccatata ttaaaagaat gatatggcca 330

<210> 117
<211> 350
<212> DNA
<213> Staphylococcus aureus

<400> 117
tcgttacacc gaatggtcaa gtatctgcat atgatcaata cttatttgca caagacccaa 60

ctggtccagc agcaagagac tatttcgtcc cagataatca actacctcct ttaattcaaa 120

gtggctttaa tccatcattt attacaacat tgtcacacga aaaaggtaaa ggtgataaaa 180

gcgagtttga aatcacttac ggcagaaaca tggatgctac atatgcatac gtgacaagac 240

ctcgtttagc cgttgataga aaacatgatg cttttaaaaa ccgaaacgtt acagttaaat 300

atgaagtgaa ctggaaaaca catgaagtaa aaattaaaag catcacacct 350

<210> 118
<211> 221
<212> DNA
<213> Staphylococcus aureus

<400> 118
tttaagcgta ctatcacaca gacaagatgg cgctaaaaaa tctaaaatta cagtaactta 60

tcaacgtgaa atggatttat accaaattcg ttggaatggc ttctactggg caggcgcgaa 120

ttataaaaac tttaaaacta gaacatttaa atcaacatat gaaattgatt gggaaaatca 180

caaagtgaaa ttgttagata caaaagaaac tgaaaacaat a 221

<210> 119
<211> 337
<212> DNA
<213> Staphylococcus aureus

<400> 119
ttgatagcga tttatttgta ggctacaaac ctcatagtaa agatcctaga gattatttcg 60

ttccagacag cgagttacca cctcttgtac aaagtggatt taacccttca tttatcgcaa 120

cagtatctca cgaaaaaggt tcaagcgaca cgagcgaatt tgaaatcact tatggaagaa 180

atatggatgt cactcatgcc attaaaagat caacacatta tggcaacagt tatttagatg 240

gtcatagagt ccataatgca tttaaaaata gaaactacac tgtgaaatat gaagtcaatt 300

ggaagactca cgaaatcaaa gtgaaaggac agaattg 337

<210> 120
<211> 752
<212> DNA
<213> Staphylococcus aureus

<400> 120
gtcagctcag taacaacaac actattgcta ggttccatat tgatgaatcc tgtcgctggt 60

gccgcagatt ctgatattaa tattaaaacc ggtactacag atattggaag caatactaca 120

gtaaaaacag gtgatttagt cacttatgat aaagaaaatg gcatgcacaa aaaagtattt 180

tatagtttta tcgatgataa aaatcacaat aaaaaactgc tagttattag aacgaaaggt 240

accattgctg gtcaatatag agtttatagc gaagaaggtg ctaacaaaag tggtttagcc 300

tggccttcag cctttaaggt acagttgcaa ctacctgata atgaagtagc tcaaatatct 360

gattactatc caagaaattc gattgataca aaagagtata tgagtacttt aacttatgga 420

ttcaacggta atgttactgg tgatgataca ggaaaaattg gcggccttat tggtgcaaat 480

gtttcgattg gtcatacact gaaatatgtt caacctgatt tcaaaacaat tttagagagc 540

ccaactgata aaaaagtagg ctggaaagtg atatttaaca atatggtgaa tcaaaattgg 600

ggaccatatg atagagattc ttggaacccg gtatatggca atcaactttt catgaaaact 660

agaaatggtt ctatgaaagc agcagataac ttccttgatc ctaacaaagc aagttctcta 720

ttatcttcag ggttttcacc agacttcgct ac 752

<210> 121
<211> 507
<212> DNA
<213> Staphylococcus aureus

<400> 121
tgttatcgac cgttttgtat ccaaattggg ggcaatataa acgcgctgat ttaatcggac 60

aatcttctta tattaaaaat aatgatgtcg taatattcaa tgaagcattt gataatggtg 120

cttcagacaa attattaagt aatgtgaaaa aagaatatcc ttaccaaaca cctgtactcg 180

gtcgttctca atcaggttgg gacaaaactg aaggtagcta ctcatcaact gttgctgaag 240

atggtggcgt agcgattgta agtaaatatc ctattaaaga gaaaatccag catgttttca 300

aaagcggttg tggattcgat aatgatagca acaaaggctt tgtttataca aaaatagaga 360

aaaatggtaa gaacgttcac gttatcggta cacatacaca atctgaagat tcacgttgtg 420

gtgctggaca tgatcgaaaa attagagctg aacaaatgaa agaaatcagt gactttgtta 480

aaaagaaaaa tatccccaaa gatgaaa 507

<210> 122
<211> 213
<212> DNA
<213> Staphylococcus aureus

<400> 122
ggtgtcctat ctcgaaaaca aaacgctgca aaaaaatcaa aaattactgt tacttatcaa 60

agtgaaatgg atagatatac aaacttttgg atcaacttca actggatagg taataattat 120

aaagatcaca taagagcaac tcatacatca atttatgaag ttgattggga aaatcataca 180

gttaaattaa tagatactca atctaaggaa aaa                                213

<210>  123
<211>  220
<212>  DNA
<213>  Staphylococcus aureus

<400>  123
ataaagaaag gaaatgattt tatggtcaaa aaaagactat tagctgcaac attgtcgtta      60

ggaataatca ctcctattgc tacttcgttt catgaatcta aagctgataa caatattgag     120

aatattggtg attgcgctga ggtagtcaaa agaacagaag atacaagttg cgataagtgg     180

ggggtcacac aaaatattca gtttgatttt gttaaagata                           220

<210>  124
<211>  359
<212>  DNA
<213>  Staphylococcus aureus

<400>  124
atcattaggt aaaatgtctg gacatgatcc aaatttattt gttggatata aaccatatag      60

tcaaaatccg agagactatt ttgtgccaga caatgaatta cccccattag tacacagtgg     120

tttcaatcct tcatttattg caactgtttc tcatgaaaaa ggctcaggag atacaagtga     180

atttgaaata acgtatggca gaaatatgga tgttactcat gctactagaa gaacaacaca     240

ctatggcaat agttatttag aaggatctag aatacacaag gcatttgtaa acagaaatta     300

cacagttaaa tatgaagtga actggaaaac tcatgaaatt aaagtgaaag gacataatt      359

<210>  125
<211>  612
<212>  DNA
<213>  Staphylococcus aureus

<400>  125
aagttgctca aatacaagct ggtttacaat ataaaccaca agtacaacgt gtaccaggta      60

agtggacaga tgctaacttt aatgatgtta agcatgcaat ggatacgaag cgtttagctc     120

aagatccagc attaaaatat caattcttac gcttagacca accacaaaat atttctattg     180

ataaaattaa tcaattctta aaaggtaaag gtgtattaga aaaccaaggt gctgcattta     240

acaaagctgc tcaaatgtat ggcattaatg aagtttatct tatctcacat gccctattag     300

aaacaggtaa cggtacttct caattagcga aaggtgcaga tgtagtgaac aacaaagttg     360

taactaactc aaacacgaaa taccataacg tatttggtat tgctgcatat gataacgatc     420

ctttacgtga aggtattaaa tatgctaaac aagctggttg ggacacagta tcaaaagcaa     480

tcgttggtgg tgctaaattc atcggcaact catatgtaaa agctggtcaa aatacacttt     540

acaaaatgag atggaatcct gcacatccag gaacacacca atatgctaca gatgtagatt     600

gggctaacat ca 612

<210> 126
<211> 401
<212> DNA
<213> Staphylococcus aureus

<400> 126
tgttattatt ctcattttct tcaattacta atgaggtaag tgcatcaagt tcattcgaca 60

aaggaaaata taaaaagggc gatgacgcga gttattttga accaacaggc ccgtatttga 120

tggtaaatgt gactggagtt gatggtaaag gaaatgaatt gctatcccct cattatgtcg 180

agtttcctat taaacctggg actacactta caaaagaaaa aattgaatac tatgtcgaat 240

gggcattaga tgcgacagca tataaagagt ttagagtagt tgaattagat ccaagcgcaa 300

agatcgaagt cacttattat gataagaata agaaaaaaga agaaacgaag tctttcccta 360

taacagaaaa aggttttgtt gtcccagatt tatcagagca t 401

<210> 127
<211> 715
<212> DNA
<213> Staphylococcus aureus

<400> 127
ttttattcat tgccctaacg ttgacaacaa gtccacttgt aaatggtagc gagaaaagcg 60

aagaaataaa tgaaaaagat ttgcgaaaaa agtctgaatt gcagggaaca gctttaggca 120

atcttaaaca aatctattat tacaatgaaa aagctaaaac tgaaaataaa gagagtcacg 180

atcaattttt acagcatact atattgttta aaggcttttt tacagatcat tcgtggtata 240

acgatttatt agtagatttt gattcaaagg atattgttga taaatataaa gggaaaaaag 300

tagacttgta tggtgcttat tatggttatc aatgtgcggg tggtacacca aacaaaacag 360

cttgtatgta tggtggtgta acgttacatg ataataatcg attgaccgaa gagaaaaaag 420

tgccgatcaa tttatggcta gacggtaaac aaaatacagt acctttggaa acggttaaaa 480

cgaataagaa aaatgtaact gttcaggagt tggatcttca agcaagacgt tatttacagg 540

aaaaatataa tttatataac tctgatgttt ttgatgggaa ggttcagagg ggattaatcg 600

tgtttcatac ttctacagaa ccttcggtta attacgattt atttggtgct caaggacagt 660

attcaaatac actattaaga atatatagag ataataaaac gattaactct gaaaa 715

<210> 128
<211> 233
<212> DNA
<213> Staphylococcus aureus

<400> 128
cgtagatgtg tttggagcta attattatta tcaatgttat ttttctaaaa aaacgaatga 60

tattaattcg catcaaactg acaaacgaaa aacttgtatg tatggtggtg taactgagca 120

taatggaaac caattagata aatatagaag tattactgtt cgggtatttg aagatggtaa 180

aaatttatta tcttttgacg tacaaactaa taagaaaaag gtgactgctc aag 233

<210> 129
<211> 360
<212> DNA
<213> Staphylococcus aureus

<400> 129
aatttttggc acatgattta atttataaca ttagtgataa aaaactgaaa aattatgaca 60

aagtgaaaac agagttatta aatgaaggtt tagcaaagaa gtacaaagat gaagtagttg 120

atgtgtatgg atcaaattac tatgtaaact gctatttttc atccaaagat aatgtaggta 180

aagttacagg tggcaaaact tgtatgtatg gaggaataac aaaacatgaa ggaaaccact 240

ttgataatgg gaacttacaa aatgtactta taagagttta tgaaaataaa agaaacacaa 300

tttcttttga agtgcaaact gataagaaaa gtgtaacagc tcaagaacta gacataaaag 360

<210> 130
<211> 501
<212> DNA
<213> Staphylococcus aureus

<400> 130
ccacctgttg aaggaagagg agttattaat tctagacagt ttttatctca tgatttaatt 60

tttccaattg agtataagag ttataatgag gttaaaactg aattagaaaa tacagaatta 120

gctaacaatt ataaagataa aaaagtagac atttttggcg ttccatattt ttatacatgt 180

ataataccta aatctgaacc ggatataaac caaaattttg gaggttgttg tatgtatggt 240

ggtcttacat ttaatagttc agaaaatgaa agagataaat taattactgt acaggtaaca 300

atcgacaata gacaatcact tggatttaca ataactacaa ataagaatat ggttactatt 360

caggaactag attacaaagc aagacactgg ctcactaaag aaaaaaagct atacgagttt 420

gatggttctg catttgaatc tggatatata aaatttactg aaaagaacaa tacaagtttt 480

tggtttgact tatttcctaa a 501

<210> 131
<211> 542
<212> DNA
<213> Staphylococcus aureus

<400> 131
gaagatttac acgataaaag tgagttaaca gatttagctt tagctaatgc atatggtcaa 60

tataatcacc cattcattaa agaaaatatt aagagtgatg aaataagtgg agaaaaagat 120

ttaatattta gaaatcaagg tgatagtggc aatgatttga gagtaaagtt tgcaactgct 180

gatttagctc agaagtttaa aaataaaaat gtagatatat atggggcatc tttttattat     240

aagtgtgaaa aaataagtga aaatatttct gaatgtctat atggaggtac aacactaaat     300

agtgaaaaat tggcacagga aagggtgatt ggtgctaatg tttgggtaga tggtattcaa     360

aaagaaacag aattaatacg aacaaataag aaaaatgtga cattgcaaga attagatata     420

aagatcagaa aaatattgtc cgataaatat aaaatttatt ataaagacag cgaaataagt     480

aaaggtctaa ttgaatttga tatgaaaact cctagagatt actcattcga catttatgat     540

tt                                                                    542

<210>  132
<211>  343
<212>  DNA
<213>  Staphylococcus aureus

<400>  132
agtcttatct aacggcgatg taggtccagg aaatctaaga aatttttata ctaaatatga      60

atatgtgaat ttaaagaatg ttaaagacaa aaattcacca gaatcacacc gcttagaata     120

ctcgtataaa aatgatacat tgtatgctga atttgacaat gaatatataa ctagtgatct     180

aaagggaaaa aatgtcgatg tttttggtat aagctataaa tatggttcta actctcgtac     240

tatatatggt ggtgttacta aagcagaaaa caataaatta gattcgccaa gaataatacc     300

tataaattta attatcaatg gcaagcatca aacagttaca act                       343

<210>  133
<211>  272
<212>  DNA
<213>  Staphylococcus aureus

<400>  133
ggatataaat acggaaataa agttacattt atagataatt ctcaacaaat tgatgttaca      60

ttgacaggaa atgaaaaatt aactgttaaa gatgatgacg aagtttctaa tgttgacgtg     120

tttgtagtaa gagaaggtag tgacaaatca gctatcacaa catcgattgg tggaattaca     180

aagacaaatg ggactcaaca taaagatact gttcaaaacg ttaatttgtc agtttctaag     240

agtacaggtc aacacactac ttctgtgact tc                                   272

<210>  134
<211>  450
<212>  DNA
<213>  Staphylococcus aureus

<400>  134
atgaaattta aagcgatagc aaaagcaagt ttagcattgg gaatgttagc aacaggtgta      60

attacatcga atgtacaatc agtacaagcg aaaacagaag ttaaacaaca aagtgaatca     120

gagttgaaac actattataa taaaccggtt ttagagcgta aaaatgttac tggatataaa     180

tatactgaaa aaggtaaaga ttatatagat gttatagtag acaatcaata ttctcaaatt     240

tctttagttg gatctgataa agacaaattt aaagatggag acaactcgaa tatagatgtg     300

tttatcctta gagaaggtga cagtagacaa gcaacaaatt actcaattgg tggcgtaaca     360

aaaacaaaca gtcaaccttt tattgactat atacacacac caatccttga aatcaagaaa     420

ggtaaagaag aaccacaaag tagtttatac                                      450


<210>  135
<211>  500
<212>  DNA
<213>  Staphylococcus aureus

<400>  135
gtattgaata taaaaatgtg acaggttata tcagtttcat tcaaccaagt attaaattta      60

tgaatatcat agatggtaat tctgttaata accttgcttt aattggcaaa gataagcaac     120

attatcatac gggtgtacat cgtaatctta atatatttta cgttaatgag gataagagat     180

ttgaaggtgc aaagtactct attgggggta tcactagtgc aaacgataaa gctgtcgacc     240

taatagcaga agcaagagtt attaaagcag atcatattgg tgaatatgat tatgactttt     300

tcccatttaa aatagttaaa gaagcgatgt cattgaaaga gattgatttt aaattaagaa     360

aataccttat tgataattat ggtctttacg gtgaaatgag tacaggaaaa attaccgtca     420

aaaagaaata ctatggaaag tatacatttg aattggataa aaagttacaa gaagaccgta     480

tgtccgatgt tatcaatgtc                                                 500


<210>  136
<211>  384
<212>  DNA
<213>  Staphylococcus aureus

<400>  136
gcgcaattac agtaacgacg caatcggtca aagcagaaaa aatacaatca actaaagttg      60

acaaagtacc aacgcttaaa gcagagcgat tagcaatgat aaacataaca gcaggtgcaa     120

attcagcgac aacacaagca gctaacacaa gacaagaacg cacgcctaaa ctcgaaaagg     180

caccaaatac taatgaggaa aaaacctcag cttccaaaat agaaaaaata tcacaaccta     240

aacaagaaga gcagaaatcg cttaatatat cagcaacgcc agcgcctaaa caagaacaat     300

cacaaacgac aaccgaatcc acaacgccga aaactaaagt gacaacacct ccatcaacaa     360

acacgccaca accaatgcaa tcta                                            384


<210>  137
<211>  270
<212>  DNA
<213>  Staphylococcus aureus

<400>  137

tttaaaagtt agttctttat tcgttgcaac tttgacaaca gcgacacttg tgagttctcc 60

agcagcaaac gcgttatctt caaaggctat ggacaatcat ccacaacaaa cgcagtcaag 120

caaacagcaa acacctaaga ttcaaaaagg cggtaacctt aaaccattag aacaacgtga 180

acacgcaaat gttatattac caaataacga tcgtcaccaa atcacagata caacgaatgg 240

tcattatgca cccgtaactt atattcaagt 270

<210> 138
<211> 556
<212> DNA
<213> Staphylococcus aureus

<400> 138
tttttatcgt aagccctttg ttgcttgcga caatcgctac agattttacc cctgttccct 60

tatcatctaa tcaaataatc aaaactgcaa aagcatctac aaacgataat ataaaggatt 120

tgctagactg gtatagtagt gggtctgaca cttttacaaa tagtgaagtt ttagataatt 180

ccttaggatc tatgcgtata aaaaacacag atggcagcat cagccttata atttttccga 240

gtccttatta tagccctgct tttacaaaag gggaaaaagt tgacttaaac acaaaaagaa 300

ctaaaaaaag ccaacatact agcgaaggaa cttatatcca tttccaaata agtggcgtta 360

caaatactga aaaattacct actccaatag aactaccttt aaaagttaag gttcatggta 420

aagatagccc cttaaagtat tggccaaagt tcgataaaaa acaattagct atatcaactt 480

tagactttga aattcgtcat cagctaactc aaatacatgg attatatcgt tcaagcgata 540

aaacgggtgg ttattg 556

<210> 139
<211> 532
<212> DNA
<213> Staphylococcus aureus

<400> 139
gaaagtattc tgtaggtact gcttcaattt tagtagggac aacattgatt tttgggttaa 60

gtggtcatga agctaaagcg gcagaacata cgaatggaga attaaatcaa tcaaaaaatg 120

aaacgacagc cccaagtgag aataaaacaa ctaaaaaagt tgatagtcgt caactaaaag 180

acaatacgca aactgcaact gcagatcagc ctaaagtgac aatgagtgat agtgcaacag 240

ttaaagaaac tagtagtaac atgcaatcac cacaaaacgc tacagctaat caatctacta 300

caaaaactag caatgtaaca acaaatgata aatcatcaac tacatatagt aatgaaactg 360

ataaaagtaa tttaacacaa gcaaaagatg tttcaactac acctaaaaca acgactatta 420

aaccaagaac tttaaatcgc atggcagtga atactgttgc agctccacaa caaggaacaa 480

atgttaatga taaagtacat ttttcaaata ttgacattgc gattgataaa gg 532

<210> 140
<211> 622
<212> DNA
<213> Staphylococcus aureus

<400> 140
cgggcaaata aataaagatg taacagatat aaaaatatat caagttccta aaggttatac 60

attaaataaa ggatacgatg tgaatactaa agagcttaca gatgtaacaa atcaatactt 120

gcagaaaatt acatatggcg acaacaatag cgctgttatt gattttggaa atgcagattc 180

tgcttatgtt gtaatggtta atacaaaatt ccaatataca aatagcgaaa gcccaacact 240

tgttcaaatg gctactttat cttcaacagg taataaatcc gtttctactg gcaatgcttt 300

aggatttact aataaccaaa gtggcggagc tggtcaagaa gtatataaaa ttggtaacta 360

cgtatgggaa gatactaata aaaacggtgt tcaagaatta ggagaaaaag gcgttggcaa 420

tgtaactgta actgtatttg ataataatac aaatacaaaa gtaggagaag cagttactaa 480

agaagatggg tcatacttga ttccaaactt acctaatgga gattaccgtg tagaattttc 540

aaacttacca aaaggttatg aagtaacccc ttcaaaacaa ggtaataacg aagaattaga 600

ttcaaacggc ttatcttcag tt 622

<210> 141
<211> 892
<212> DNA
<213> Staphylococcus aureus

<400> 141
aaagttggcg atggtaaaga taatgtggca gcagcgcatg acggtaaaga tattgaatat 60

gatacagagt ttacaattga caataaagtc aaaaaaggcg atacaatgac gattaattat 120

gataagaatg taattccttc ggatttaaca gataaaaatg atcctatcga tattactgat 180

ccatcaggag aggtcattgc taaaggaaca tttgataaag caactaagca aatcacatat 240

acatttacag actatgtaga taaatatgaa gatataaaat cacgcttaac tctatattcg 300

tatattgata aaaaaacagt tccaaatgag acaagtttga atttaacatt tgctacagca 360

ggtaaagaaa caagccaaaa tgtcactgtt gattatcaag atccaatggt ccatggtgat 420

tcaaacattc aatctatctt tacaaaatta gatgaagata agcaaactat tgaacaacaa 480

atttatgtta acccattgaa aaaatcagca accaacacta aagttgatat agctggtagt 540

caagtagatg attatggaaa tattaaacta ggaaatggta gcaccattat tgaccaaaat 600

acagaaataa aggtttataa agttaactct gatcaacaat tgcctcaaag taatagaatc 660

tatgatttta gtcaatacga agatgtaaca agtcaatttg ataataaaaa atcatttagt 720

aataatgtag caacattgga ttttggtgat attaattcag cctatattat caaagttgtt 780

agtaaatata cacctacatc agatggcgaa ctagatattg cccaaggtac tagtatgaga 840

acaactgata aatatggtta ttataattat gcaggatatt caaacttcat cg 892

<210> 142
<211> 747
<212> DNA
<213> Escherichia coli

<400> 142
gtttgggact tattgctctg gcggtgggta atgcatatgc aacacaattg ttggatgatt 60

atagtataat ttcctatatg actgatgaag aatcgccgat tgaaatcaaa gataataatc 120

cgataagtaa tggagagtat ctaaccactg aagacgaaag ccatgctgtg aaagtggatg 180

acggtgtaac tggatatata aataatgcca gtgtgatgac tagtggtgat ggatcttatg 240

gtatttctgt tgatagtcaa aacaaagtat tatatataag cgatagcgat attaagacct 300

ctggaagcgt atctgacaaa gaaaatggag ggataacagc cagcgcagta gtcagtgaat 360

ttggtggcac catctttatg aatggtgata attcagtcga gtcgggtggg gcatattcag 420

cgggactttt aagccaggtt aatgattctg aaaagatggt aaataacacc cgtcttgaaa 480

ccacagataa aacgaacatt gttacctctg gggaaaatgc agtaggtgtt cttgcatgtt 540

caagtcctgg agagtctcga acatgtgtcg atgctgtaga tgatgaagtt agtgattcta 600

acagttacga agttattagc cgtgctgatt taaaaatgaa tggtggttcc ataacaacta 660

atggcattaa tagctatggt gcttatgcta atgggaaaaa agcatatatt aatttagatt 720

atgtggcact tgaaactgtg gctgatg 747

<210> 143
<211> 621
<212> DNA
<213> Escherichia coli

<400> 143
agcctggtga cgacttatct ggtggtgctg aacttcgcga ttttgccgag cctccagcag 60

tttaataaag tcctcgcgta cgaagtgcgt atgttgatga ccgacaaact gcaactggag 120

gacggcacgc agttggttgt gcctcccgct ttccgtcggg agatctaccg tgagctgggg 180

atctctctct actccaacga ggctgccgaa gaggcaggtc tgcgttgggc gcaacactat 240

gaattcttaa gccatcagat ggcgcagcaa ctgggcggcc cgacggaagt gcgcgttgag 300

gtcaacaaaa gttcgcctgt cgtctggctg aaaacctggc tgtcgcccaa tatctgggta 360

cgcgtgccgc tgaccgaaat tcatcagggc gatttctctc cgctgttccg ctatacgctg 420

gcgattatgc tattggcgat aggcggggcg tggctgttta ttcgtatcca gaaccgaccg 480

ttggtcgatc tcgaacacgc agccttgcag gttggtaaag ggattattcc gccgccgctg 540

cgtgagtatg gcgcttcgga ggtgcgttcc gttacccgtg cctttaacca tatggcggct 600

ggtgttaagc aactggcgga t 621

<210>  144
<211>  449
<212>  DNA
<213>  Escherichia coli

<400>  144
accacgacag gtctttatga tctgaaaacc gaaaatacct tgttaactac cgatgctgca      60

ttcgataaat tagggaatgg cgataaagtc accgttggcg gcgtagatta tacttacaac     120

gctaaatctg gtgattttac taccaccaaa tctactgctg gtacgggtgt agacgccgcg     180

gcgcaggcta ctgattcagc taaaaaacgt gatgcgttag ctgccaccct tcatgctgat     240

gtgggtaaat ctgttaatgg ttcttacacc acaaaagatg gtactgtttc tttcgaaacg     300

gattcagcag gtaatatcac catcggtgga agccaggcat acgtagacga tgcaggcaac     360

ttgacgacta acaacgctgg tagcgcagct aaagctgata tgaaagcgct gcttaaagcc     420

gcgagcgaag gtagtgacgg tgcctctct                                       449


<210>  145
<211>  704
<212>  DNA
<213>  Escherichia coli

<400>  145
atggaattgc gtctgttcaa ctatctggtc gagcgtaaag atctgattca gatcccggtg      60

tatccgttcg aacgcgaatg gacgcacttc accagcatga cttacattga tgagttttca     120

gagctgcatg gcaaagatgt tccggtgcgt gaagccctcg ccggacaagt gcccagcgca     180

ggcgtcggca cctgtttcag ccgccgcgcc gtgaccgcac tgttagctga cggtgacggt     240

attgctttcg acgtgcagag tcttactgaa gattacgaca ttggcttccg cctgaaagaa     300

aaaggtatga cggaaatttt tgtccgtttt ccggtggtgg acgaagccaa agaacgcgag     360

cagcgtaaat ttttacagca cgcgcggaca tcaaacatga tctgcgtgcg cgaatatttc     420

cccgatacct tttcgactgc ggttcgacaa aaatcccgct ggatcatcgg cattgttttc     480

caaggcttta aaacccataa atggacctcc agcctgacgc tgaactactt tctctggcgc     540

gaccgcaaag gggcaatcag taactttgtc agcttcctcg cgatgctggt gatgatccag     600

cttttgctgt tgctggcgta tgaaagtttg tggcccgatg cctggcattt cctttctatt     660

ttcagcggca gcgcatggtt aatgaccctg ctgtggctaa actt                      704


<210>  146
<211>  251
<212>  DNA
<213>  Escherichia coli

<400>  146
ataatcctcg tcatttgcag attatggaac tcgagggggc gcagctcccg cgcgtactgg      60

atgatcccaa agttgatgta gcgattatca gcaccactta cattcagcag accgggcttt    120

ctccggtgca cgacagcgta tttattgaag ataagaattc gccgtatgtg aatattttgg    180

tggcacggga agataataag aatgcagaaa acgtgaagga atttctgcaa tcttatcaat    240

cacccgaagt c                                                          251

<210>  147
<211>  423
<212>  DNA
<213>  Escherichia coli

<400>  147
ctctgtccct cagttctacg acggctctgg ccgctgccac gacggttaat ggtgggaccg     60

ttcactttaa aggggaagtt gttaacgccg cttgcgcagt tgatgcaggc tctgttgatc    120

aaaccgttca gttaggacag gttcgtaccg catcgctggc acaggaagga gcaaccagtt    180

ctgctgtcgg ttttaacatt cagctgaatg attgcgatac caatgttgca tctaaagccg    240

ctgttgcctt tttaggtacg gcgattgatg cgggtcatac caacgttctg gctctgcaga    300

gttcagctgc gggtagcgca acaaacgttg gtgtgcagat cctggacaga acgggtgctg    360

cgctgacgct ggatggtgcg acatttagtt cagaaacaac cctgaataac ggaaccaata    420

cca                                                                   423

<210>  148
<211>  768
<212>  DNA
<213>  Escherichia coli

<400>  148
gactcggtac agcgattgcg gcaactggcg caacaaaccg gctcgctgaa gtcatcaacc     60

gaacagaaag ttattaccac aacgaagaaa gctgtaccgg taaaacagac agtcacggca    120

cccgtcatac catccaatac agttttaact gccaaccccg tcattacaga gccggcaaca    180

accgtcattt ccattgagcc cgccaatcct gatgtggtct atattcccaa ctacaaccca    240

accgtggttt acgggaactg ggccaatact gcgtatccgc cggtttatct gccaccacca    300

gccggagaac cgtttgttga cagctttgta cgcggattcg gctatagcat gggcgttgct    360

accacgtacg cactattcag cagcatcgac tgggacgacg acgatcatga ccatcatcat    420

catgacaatg atgattatca tcaccacgat ggcggtcatc gtgacggtaa tggctggcaa    480

cacaacggcg acaacatcaa tatcgacgtc aacaatttca accgtatcac cggtgagcat    540

cttactgata agaatatggc atggcggcac aatccaaact accgtaatgg tgtgccctat    600

catgatcagg atatggcaaa gcggtttcat caaaccgatg tcaacggcgg aatgagcgcc    660

acgcaattac ctgccccaac gcgcgacagc cagcgtcagg cggcagcaaa tcagtttcag    720

caacgaacac acgccgcacc agtcattaca cgagataccc aacgtcag 768

<210> 149
<211> 788
<212> DNA
<213> Escherichia coli

<400> 149
ctttacgacg gttctcccca ggactgaaag cccagtttgc cttcggcatg gtctttttgt 60

tcgttcagcc cgatgccagc gctgctgaca taagtgcgca gcaaataggt ggggtgatta 120

ttccgcaggc cttcagtcag gcgcttcagg acggcatgag cgtcccgctc tatattcatc 180

tcgccggtag ccagggtcgc caggacgatc agcgaatcgg cagcgctttt atctggctgg 240

acgatggaca gctacgcatc cggaaaatac agctggaaga gagtgaagat aacgccagtg 300

tcagcgaaca aactcgacag cagctgatgg ctctggccaa cgccccgttc aatgaggccc 360

ttaccatccc cctgactgac aacgcgcagc tggatctcag cttgcgccaa ctgctgctgc 420

agctagtggt caagcgcgaa gcgctgggca ccgtactacg ctcacgtagc gaagacatcg 480

ggcagtccag tgttaacacc ctcagcagta atctgagcta taacttgggc gtctataaca 540

accagttgcg taacggcggg agcaacacat ccagctatct gtcgctgaat aacgttactg 600

cactgcgcga acatcatgtg gtgctcgacg gctcgctgta cgggatcggt agcggtcaac 660

aggacagtga attatataaa gcgatgtatg aacgcgattt tgccggtcac cgatttgccg 720

gtggaatgct cgacacctgg aacttgcagt ccttagggcc gatgaccgcc atttcagcag 780

ggaagatt 788

<210> 150
<211> 750
<212> DNA
<213> Escherichia coli

<400> 150
ttgaaacttc ttactgccgc atttttagca gcgagtcccg cggcgaagag tgctgttaat 60

aacgcctatg atgcattgat tattgaagct cgcaagggta atactcagcc agctttgtca 120

tggtttgcac taaaatcagc actcagcaat aaccaaattg ctgactggtt acagattgcc 180

ttatgggccg ggcaagataa acaggttatt accgtttaca accgctaccg tcatcagcaa 240

ttaccagcgc gtggttatgc agctgtcgcc gtcgcttatc gtaacctgca acaatggcaa 300

aactcgctta cactgtggca aaaggcgctc tctctggagc cgcaaaataa ggattatcaa 360

cggggacaaa ttttaaccct ggcagatgct ggtcactatg atactgcgct ggttaaactt 420

aagcagctta actctggagc accggacaaa gccaatttac tcgcagaagc ctatatctat 480

aaactggcgg ggcgtcatca ggatgaatta cgggcgatga cagagtcatt acctgaaaat 540

gcatctacgc aacaatatcc cacagaatac gtgcaggcat tacgtaataa tcaacttgct 600

gccgcgattg acgatgccaa tttaacgcca gatattcgcg ctgatattca tgccgaactg 660

gtcagactgt cgtttatgcc tacgcgcagt gaaagtgaac gttatgccat tgccgatcgc 720

gccctcgccc aatacgctgc attagaaatt 750

<210> 151
<211> 733
<212> DNA
<213> Escherichia coli

<400> 151
atagcagggc tgtttgtatc atctctaagt tatgcagaaa acacggagat cccttcttat 60

gaagaaggga tctcgctctt tgatgttgaa gccactctgc aaccagatgg ggtgctcgac 120

atcaaagaaa atattcattt tcaggcgcga aatcagcaga ttaagcacgg cttttatcgt 180

gatttaccac gactatggat gcagcctgat ggggacgctg cactgctgaa ctatcatatt 240

gttggcgtca cccgtgatgg tattcctgaa ccctggcatc ttgactggca tatcgggtta 300

atgagtattg tcgtgggcga taaacaacgt ttcttgcctc aaggcgacta tcattatcaa 360

attcattatc aggttaaaaa tgctttcctg cgtgaggggg attctgatct gctaatctgg 420

aacgtgaccg gtaaccactg gccgtttgaa atttataaga cccgtttttc tctccagttc 480

tctaatattg cgggtaatcc atttagcgaa atcgatcttt ttaccggaga agagggcgac 540

acatatcgta atggccgcat ccttgaggac ggaagaattg aatcccgcga tccgttttat 600

cgtgaagatt tcacggtcct ctaccgctgg cctcacgctt tacttagcaa tgcctcggct 660

ccgcaaacga cgaatatttt cagccatctt cttttaccct ccacgtcatc gttgttaatt 720

tggtttccgt gtc 733

<210> 152
<211> 756
<212> DNA
<213> Escherichia coli

<400> 152
tattgtcatc gcgcagagtc tcacgatgcg ggcgatatca gctttagcga tatctttcgt 60

ggcccggctt ccatctttgg cggcattgag tatcaaacgc cgtggaatcc cctgcgtctg 120

aaactcgaat acgatggaaa caattaccag aatgatttcg ctggcaaact gcctcaggca 180

agccatttca acgtcggcgc agtttatcgc gctgccagct gggcagatct caacctgagt 240

tatgaacgcg gtaacacgtt gatgtttggc ttcacgttac ggaccaattt caacgatctg 300

cgccctgccc tgcgcgatac gccaaaaccg gcatatcaac ctgcgcctga atctgaagga 360

ttgcagtaca ccacggtagc aaaccaactt accgccctga agtataacgc gggctttgac 420

gcgccagaaa ttcagctacg cgataagaca ctgtatatgt ctggtcagca atacaaatac 480

cgtgactctc gtgaagcggt cgatcgtgcc aaccggattc tggtgaataa cctgccgcaa 540

ggcgttgaga agattagcgt gacgcaaaag cgcgagcata tggcgatggt gactaccgaa 600

accgacgtag ccagcctgcg caaacagctg gcaggtacag cgcctggtca atcagagcca 660

ctgcaacaac aacgtgttga agctgaagat ctttctgcct ttggtcgggg ctaccgtatt 720

cgtgaagatc gctttagcta ctctttcaac ccaaca 756

<210> 153
<211> 735
<212> DNA
<213> Escherichia coli

<400> 153
gaataccaaa gcagatcgtc tcgctgaatt aaaaatccgt tcgccctcaa ttcaactgat 60

aaaatttggc gctattggtt tgaatgcaat tatcttttcc cccctgctga tagctgctga 120

tacaggaagt caatatggca ccaatattac tattaatgat ggtgacagaa ttacaggaga 180

taccgccgat ccatcaggaa acctctatgg tgtaatgacc ccagcaggaa acacgcctgg 240

caatatcaac ctgggtaatg atgtcaccgt caatgtcaac gacgcctctg gatatgcaaa 300

aggaatcatt attcagggca aaaacagctc cctgacagct aaccgactca cagtagatgt 360

tgttggtcaa acctctgcca tcggcattaa cttaattggt gactataccc atgctgactt 420

aggcacaggc agcaccatta agagtaacga tgacggcatc attattgggc atagctcaac 480

actaacagcc actcaattca ccattgaaaa ctcgaacggt ataggcctaa ccatcaatga 540

ctatggcacc agtgtcgatc ttggaagcgg aagtaaaatc acgaccgatg gaagtacagg 600

tgtttatatc ggtggtctca acggcaataa cgccaatggt gctgcgcgtt ttacggctac 660

agacctgaca atcgatgttc agggctacag cgccatgggg ataaacgtac agaaaaactc 720

tgttgtcgat ctcgg 735

<210> 154
<211> 509
<212> DNA
<213> Escherichia coli

<400> 154
ctaactcatt gtggtggagc ccaaacatga ttactcatgg tttttatgcc cggacccggc 60

acaagcataa gctaaaaaaa acatttatta tgcttagtgc tggtttagga ttgttttttt 120

atgttaatca gaattcattt gcaaatggtg aaaattattt taaattgggt tcggattcaa 180

aactgttaac tcataatagc tatcagaatc gcctttttta tacgttgaaa acaggtgaaa 240

ctgttgccga tctttctaaa tcgcaagata ttaatttatc gacgatttgg tcgttgaata 300

agcatttata cagttctgaa agcgaaatga tgaaggccga gcctggtcag cagatcattt 360

tgccactcaa aaaacttccc tttgaataca gtgccttacc acttttaggt tcggcacctc 420

ttgttgctgc aggtggtgtc gctggtcata caaataaact gactaaaatg tccccggacg 480

tgaccaaaag caacatgacc gatgacaag 509

<210> 155
<211> 338
<212> DNA
<213> Escherichia coli

<400> 155
ggcgttacta tcctctctat gtgcacacgg agctcctcag tctattacag aactatgttc 60

ggaatatcac aacacacaaa tatatacgat aaatgacaag atactatcat atacggaatc 120

gatggcaggc aaaagagaaa tggttatcat tacatttaag agcggcgcaa catttcaggt 180

cgaagtcccg ggcagtcaac atatagactc ccaaaaaaaa gccattgaaa ggatgaagga 240

cacattaaga atcacatatc tgaccgagac caaaattgat aaattatgtg tatggaataa 300

taaaaccccc aattcaattg cggcaatcag tatggaaa 338

<210> 156
<211> 500
<212> DNA
<213> Escherichia coli

<400> 156
tttgttgtta ttggtacttc attcctgaaa atttctattg tactggggat attgaagaac 60

gcattaggca ttcaacaggt accaccaaac atggcgctaa catcagtgtc tttgatactg 120

acaatgttta ttatgtctcc gataatatta cagataaatg ataatatttc tcaggaacca 180

atcaattata ccgactctga tttttttcaa aaagttgatg agaaaatatt atcaccatat 240

cgcggtattt tagaaaaaaa tacagagaaa gacaatgtag agttttttga acgtgcagct 300

caaaaaaaat tgggtaatga aactatctta aaaaaagact ctctatttat actgttaccg 360

gctttcacga tggggcagct tgaagctgca ttcaagatag gttttttgct ctatttaccc 420

tttattgcga tagatttgat catttccaat atcttattgg ccttgggtat gatgatggta 480

tcgccagtaa caatttcgat 500

<210> 157
<211> 503
<212> DNA
<213> Escherichia coli

<400> 157
ttacgcttcc gatcatagta gagaactata cattttcgga aaaattgcca tctggaatct 60

ttcagttgac aggaatagtg ctaaaagaaa taagtattgg ttttttcatt gggttatcat 120

ttactattct tttttgggca atagatgcgg ctgggcagat tattgatact ctaagaggtt 180

caacaatatc ttcaattttt aacccgtcca taagtgattc atcttctatc actggcgtta 240

ttttgtacca atttatctct gtgatctttg ttattcatgg tgggatacaa agcattctgg 300

ataagctata tttatcctac gagatattac cattacaagc cgatattgca ttcaatcgtg 360

ctttaataga ttttttgttt tctctatggg attcatttat taaactgatg ttatcatttt 420

cagttcccat gattatcggt atattcttat gtgatatggg gtttgggttt cttaacaaaa 480

cagcgcctca actaaacgta ttc 503

<210> 158
<211> 617
<212> DNA
<213> Escherichia coli

<400> 158
aagtgaagag gtaatggctg cagtgcagtc attaatctta ttttcatttt tttctttata 60

tggcatgagt ttttttgttg atatagttgg gttagttaat acgacaatag actcgctaaa 120

tagaccgttt ttgtatgcca ttcgagaaat attaggtgcg gtgttaaata tatttttatt 180

atatattttg ccaatttctt tgattgtctt tgttggaact gttacgactg gtgtatcaca 240

aataggattc atctttgcgg ttgaaaaaat aaaaccatcg gctcagaaga ttagtgtaaa 300

aaataacctg aaaaatattt tttctgtaaa gagcattttt gagctactta aatcagtatt 360

taagttagtg ataattgttc tcatttttta ttttatgggg cattcatatg caaatgagtt 420

tgctaatttc acaggactga acgcatatca agctcttgtc gttgttgcct tttttgtttt 480

tcttttatgg aaaggcgtgc tattcggata tctactcttt tcagtatttg atttctggtt 540

ccagaagcat gagggactga agaaaatgaa aatgagtaaa gatgaggtga aacgagaagc 600

caaggatact gatggta 617

<210> 159
<211> 740
<212> DNA
<213> Escherichia coli

<400> 159
gatggtgact ctattgcagg attaccaaca aaaacaattg gcgcaaagct atcagattca 60

gcaggccgtt tttgagagcc agaataaagc tattgaggaa aaaaaagccg cggcaaccgc 120

tgctttggtt ggcgggatta tttcatcagc attggggatc ttaggttctt ttgcagcaat 180

gaacaacgcg gctaaagggg ctggtgagat tgctgaaaaa gcaagctctg catcttcaaa 240

ggctgctggt gcggcttctg aggttgcaaa taaagctctg gtcaaggcta cggaaagtgt 300

tgctgatgtc gcagaggagg catccagtgc gatgcagaaa gcgatggcca caacaacgaa 360

agcagccagc cgtgcatctg gcgttgcaga tgatgttgcg aaagccactg actttgctga 420

agatcttgca gacgccgccg agaagacaag cagaatcaat aagttgttga attccgtaga 480

taaactgacc aataccacag catttgttgc cgtgaccagt cttgctgaag gtacgaaaac 540

gttgccaaca acaatatctg agtccgtcaa atcgactcat gaggttaatg aacaacgtgc 600

gaagtcgctg gaaaacttcc agcaggggaa tctggagctg tataaacaag acgttcgcag 660

aacgcaggat gatatcacga ctcgtctgcg tgatataacg tccgctgtcc gcgatctcct 720

tgaggtccag aatcgtatgg 740

<210> 160
<211> 717
<212> DNA
<213> Escherichia coli

<400> 160
tgtttgaggt cactttctgg tggcgtgatc cccaaggttc tgaagaatac tcgacgataa 60

agcgcgtatg ggtctacatc actggtgtga ccgatcacca tcagaacagc cagccccagt 120

cgatgcagcg aattgcaggc actaacgtct ggcagtggac gacacaactc aatgccaact 180

ggcgcggcag ctactgcttt attcccaccg aacgcgatga cattttttct gtaccatccc 240

ccgatcgcct cgaattgcgc gaaggctggc gaaaactatt accccaggcg atagccgatc 300

cgctgaacct acaaagctgg aaaggcgggc gagggcacgc tgtttctgca ctcgaaatgc 360

cgcaagcgcc tctgcaaccg ggatgggatt gtccgcaagc gccagaaata cctgccaaag 420

aaattatctg gaaaagtgaa cggttgaaaa agtcacggcg tgtatggatt tttaccaccg 480

gcgatgcaac agcagaagaa cgcccgctgg cagttttgct cgatggcgaa ttttgggcgc 540

aaagtatgcc cgtctggcca gtgctgactt cgctgaccca tcgtcagcaa cttcctcccg 600

ccgtgtatgt gttgatcgac gctatcgaca ccacgcaccg cgcccacgaa ctgccgtgta 660

atgcggattt ctggctcgca gtacagcaag agttattacc cctggtgaaa gctattg 717

<210> 161
<211> 379
<212> DNA
<213> Escherichia coli

<400> 161
tgtttctgca ctcgaaatgc cgcaagcgcc tctgcaaccg ggatgggatt gtccgcaagc 60

gccagaaata cctgccaaag aaattatctg gaaaagtgaa cggttgaaaa agtcacggcg 120

tgtatggatt tttaccaccg gcgatgcaac agcagaagaa cgcccgctgg cagttttgct 180

cgatggcgaa ttttgggcgc aaagtatgcc cgtctggcca gtgctgactt cgctgaccca 240

tcgtcagcaa cttcctcccg ccgtgtatgt gttgatcgac gctatcgaca ccacgcaccg 300

cgcccacgaa ctgccgtgta atgcggattt ctggctcgca gtacagcaag agttattacc 360

cctggtgaaa gctattgcc 379

<210>  162
<211>  402
<212>  DNA
<213>  Escherichia coli

<400>  162
tatgctgctc caactattcc tcaggggcag ggtaaagtaa cttttaacgg aactgttgtt      60

actgctccat gcggcatttc tcagaaatca gctgatcagt ctattgattt tggcagcttt     120

tcaaaaagct tccttgcggc aggaggtgta tccaaaccaa tgaatttaga tattgaattg     180

gttaattgtg atatcacttc atttaagggg gggggaggaa gccaggcagc aaaaaaaggg     240

actgtgaagc tggcttttag tggtccaagg gtttctggtc ataatgagga gttagatacc     300

agcgggggga caggtactgc aattgcagtt caggccgcag gtaaaaacgt ttctttcgat     360

ggcacagaag gtgatgctaa taccctgaaa gatggagata at                        402


<210>  163
<211>  724
<212>  DNA
<213>  Escherichia coli

<400>  163
cttggaaatg ttggtaaagc tgtttcgcaa tatattctgg ctcagagaat ggcacagggg      60

ttatcgacaa cagctgcaag tgcgggtctg atcacatcgg ctgttatgct ggctatcagt     120

cctctttctt tcctggctgc tgcagataaa tttgagcgag ctaagcagct tgaatcatat     180

tctgaacgat ttaaaaaatt gaattatgaa ggggatgctt tactcgcagc ctttcataaa     240

gaaaccggag ctatagatgc agccctgaca acaataaata ctgtcctgag ttctgtatct     300

gcgggagtta gtgcagcctc cagtgcatcc ctcatagggg ccccgataag catgctggtg     360

agtgcattaa ccggtacgat atctggcatt ctggaagcat caaaacaggc tatgtttgag     420

cacgttgcag agaaattcgc tgctcggatc aatgaatggg aaaaggagca tggcaaaaat     480

tattttgaga atggatatga cgcaagacat gctgcgtttt tagaagactc tctgtctttg     540

cttgctgatt tttctcgtca gcatgcagta gaaagagcag tcgcaataac ccagcaacat     600

tgggatgaga agatcggtga acttgcaggc ataacccgta atgctgatcg cagtcagagt     660

ggtaaggcat atattaatta tctggaaaat ggagggcttt tagaggctca accgaaggag     720

ttta                                                                  724


<210>  164
<211>  618
<212>  DNA
<213>  Escherichia coli

<400>  164
tcaatgctga aactataagg catcagtata atacccacac acaagatttt ggggtgactg      60

aatggttact ggcagcgaaa tctattggct taaaagcaaa atatgtagaa aaacattttt     120

ccagattgtc aataatttct ttacctgcgt tgatatggcg ggatgacggt aagcattata 180

tattgtctcg tattactaaa gattcatcac gctatcttgt ttatgatcca gaacaacatc 240

agtcactaac ttttagtcgg gatgagtttg aaaaactgta tcagggaaaa gtcattctgg 300

ttacgtcaag agcaacagta gtcggagagt tagctaaatt tgatttttct tggtttatcc 360

cctctgttgt gaaatacagg aggattttac ttgaggtgtt aactgtttct gcttttattc 420

agtttcttgc gttaataaca cctctttttt ttcaggttgt aatggataag gttttagttc 480

accgggggtt ttcaacgtta aatattatca caatagcatt tattatagtg atactttttg 540

aagtgatatt aaccggagcc agaacttata ttttctctca tactacaagt cgtattgacg 600

tcgaactggg tgctaagt 618

<210> 165
<211> 768
<212> DNA
<213> Escherichia coli

<400> 165
catcaggcag ttatcctgtc gactttacca ctctctcccg gcttattatt gataagctcc 60

ggcatcaact ttttctgcca gttcccctct gcgaaacttt ccaccaacgc gtgctggaaa 120

gctacgccca tacgcaacag acaattgatg cccgccatga ctgggccatc ctgcgtgaaa 180

aagcgttgaa ttttggcgag gctgagcagg cactgctgac aggacacgct ttccaccctg 240

cacctaagtc tcatgaaccg tttaaccgcc aggaagctga acgatacctg cctgacatgg 300

cacctcactt cccgctgcgg tggttttcgg tggataaaac gcaaatcgct ggtgaaagtc 360

tgcatcttaa ccttcaacag cggttgacgc gatttgccgc agaaaatgcg ccccagttac 420

tcaacgaatt aagtgacaat caatggctgt tcccgctgcg cccgtggcag ggagaatatc 480

ttttccagca agtgtggtgc caggcacttt ttgctaaagg acttatcaga gacttaggcg 540

aggccggcac gtcgtggctg ccgaccacct cttcccgctc cctctactgt gctaccagcc 600

gcgatatgat caagttctcc ctgagcgttc ggctgaccaa ctccgtccgt actctgtctg 660

tgaaagaggt ggagcgagga atgcgcctgg cacgtctggc gcaaaccgac ggctggcaga 720

tgctacaggc ccgcttccct actttccggg taatgcagga ggacgact 768

<210> 166
<211> 501
<212> DNA
<213> Escherichia coli

<400> 166
ttcacagcgg atatggactg cgctgtgaaa aactcgacaa gcctctgaat cttggctggg 60

ggctggacaa tagcgcggtg ttgcactggc ccggggagct gccaacaggg tggctgtgcg 120

acgcgctgga tcagatattt atcgccgcac cacaactttc agcagtggtt cttccctggt 180

ccgaatggtg tgaggagcca caggcgctga cgcttttcgg acaggtacaa agcgacatta 240

tccatcgttc cgctttctgg cagttaccgt tatggctgag ttctccggca aaccgggcct 300

ccggtgaaat ggtttttgat gcagagcgtg agatttattt cccgcagcgc cccccccgtc 360

cgcagggtga agtttatcgt cgttacgatc cacgcattcg caggatgctg agtttccgca 420

ttgccgatcc cgtttctgat gcagaacgtt tcactcgctg gatgaacgat ccgcgcgttg 480

agtatttctg ggagcaaagt g 501

<210> 167
<211> 721
<212> DNA
<213> Escherichia coli

<400> 167
agactgggat ttggtcaacc gccgcctggt ggcaaaaatg ttgtctgagc tggagtatga 60

gcaggttttc cacgcggaat ctcagggcga tgaccgctac tgcattaacc tgccgggagc 120

acaatggcgc ttcatcgctg aacgtggtat ctggggctgg ctctggattg atgctcaaac 180

tctgcgctgc gcggacgagc cagtactggc tcagacgctg ctgatgcagc taaagcaggt 240

actgtcaatg agcgatgcaa ccgttgctga gcatatgcag gatttgtatg ccacgctgct 300

gggcgacctg caactactga aagcccgtcg cgggctgagc gccagtgacc tgattaatct 360

taatgccgac cgcctgcaat gcctgctgag cggtcatcct aaattcgttt ttaataaagg 420

tcgccgtggc tggggtaaag aggcgctgga acgatatgcg ccagagtatg ccaacacctt 480

cagactgcac tggctggcgg taaaacgtga acatatgatc tggcgctgtg ataacgagat 540

ggatattcat cagttgttga cggccgcaat ggatccgcag gagtttgccc gcttcagtca 600

ggtctggcag gaaaacggac tggatcataa ctggctgccg ctgccggtac atccgtggca 660

gtggcaggaa aaaatcgcta ccgacttcat cgctgatttt ggcgaaggca ggatggtgtc 720

t 721

<210> 168
<211> 719
<212> DNA
<213> Escherichia coli

<400> 168
ggagtatatt gcgtgggtag tattccccaa aaaggttatg accaaaaatg gatatccctt 60

atttattgag gttcataata aaggtagctg gagtgaggag aatactggtg acaatgacag 120

ctattttttt ctcaaggggt ataagtggga tgagcgggcc tttgatgcag gtaatttgtg 180

tcagaaacca ggagaaacaa cccgtctgac tgagaaattt gacgatatta tttttaaagt 240

cgccctacct gcagatcttc ctttagggga ttattctgtt acaattccat acacttccgg 300

catgcagcgt catttcgcga gttacttggg ggcccgtttt aaaatcccat acaatgtggc 360

caaaactctc ccaagagaga atgaaatgtt attcttattt aagaatatcg gcggatgccg 420

tccttctgca cagtctctgg aaataaagca tggtgatctg tctattaata gcgctaataa 480

tcattatgcg gctcagactc tttctgtgtc ttgcgatgtg cctgcaaata ttcgttttat 540

gctgttaaga aatacaactc cgacatacag ccatggtaag aaattttcgg ttggtctggg 600

gcatggctgg gactccattg tttcggttaa cggggtggac acaggagaga caacgatgag 660

atggtacaaa gcaggtacac aaaacctgac catcggcagt cgcctctatg gtgaatctt 719

<210> 169
<211> 561
<212> DNA
<213> Escherichia coli

<400> 169
aaatgaatgt ctggactcaa cgtggatttc atcaaaagga aactatattc agaagtttga 60

aaataaattt gcggaacaaa accatgtgca atatgcaact actgtaagta atggaacggt 120

tgctcttcat ttagctttgt tagcgttagg tatatcggaa ggagatgaag ttattgttcc 180

aacactgaca tatatagcat cagttaatgc tataaaatac acaggagcca cccccatttt 240

cgttgattca gataatgaaa cttggcaaat gtctgttagt gacatagaac aaaaaatcac 300

taataaaact aaagctatta tgtgtgtcca tttatacgga catccatgtg atatggaaca 360

aattgtagaa ctggccaaaa gtagaaattt gtttgtaatt gaagattgcg ctgaagcctt 420

tggttctaaa tataaaggta aatatgtggg aacatttgga gatatttcta cttttagctt 480

ttttggaaat aaaactatta ctacaggtga aggtggaatg gttgtcacga atgacaaaac 540

actttatgac cgttgtttac a 561

<210> 170
<211> 750
<212> DNA
<213> Escherichia coli

<400> 170
agcagcatca ggttctgagc tgcattgcga atcaaatgac aacggaagat attctggaga 60

aactgaaaat atcgctaaaa acgctctact gccataaaca caatatcatg atgatcctca 120

atcttaagcg gatcaatgag ctggtacgcc atcagcatat taattatctg gtgtgaacga 180

ttgaacaata taaagaggcc cagcaacagc cagacctccc gttaattata cgttatgcag 240

taacgccttc cggtatcaac gaagcaattt gcttacgcca ttgcgcttgc tcctgttcac 300

cttctgtacg ttgaccataa agttgcgcta tctgcgtacc atcatggcca aacaattcca 360

gactggttac gtgaccatcg ctggtcggtt tacgggtaac ccaggcttca gcaatgctct 420

cttctaatag atgaagggta aacgtcgggt tgaaaatatt cagccaacct ttcattggca 480

ccactttttc taccacaccg gtgaaaatct gtacgcagcc acggttgcca acaaacacca 540

tgatttcatt gccatcctgc tgtgcagatt caagaatttg cgccaacgca ctgttggata 600

ctttgcaggc caaatcgtct gccaccagat tgaacgcctg ttggcgcgtc aggttgtggc 660

gcttgagcaa cgtaaaaaac tgatgaacgt cggtcatcgc ccgccactct tgctcgacca 720

cactggcatc ggctcgcgtt tgaacaactg 750

<210> 171
<211> 616
<212> DNA
<213> Escherichia coli

<400> 171
ttcttcggta tcctattccc gggagtttat gatagacttt tcgacccaac aaagttatgt 60

ctcttcgtta aatagtatac ggacagagat atcgacccct cttgaacata tatctcaggg 120

gaccacatcg gtgtctgtta ttaaccacac cccaccgggc agttattttg ctgtggatat 180

acgagggctt gatgtctatc aggcgcgttt tgaccatctt cgtctgatta ttgagcaaaa 240

taatttatat gtggctgggt tcgttaatac ggcaacaaat actttctacc gtttttcaga 300

ttttacacat atatcagtgc ccggtgtgac aacggtttcc atgacaacgg acagcagtta 360

taccactctg caacgtgtcg cagcgctgga acgttccgga atgcaaatca gtcgtcactc 420

actggtttca tcatatctgg cgttaatgga gttcagtggt aatacaatga ccagagatgc 480

atccagagca gttctgcgtt ttgtcactgt cacagcagaa gccttacgct tcaggcagat 540

acagggagaa tttcgtcagg cactgtctga aactgctcct gtgtatacga tgacaccgga 600

agaagtggac ctcaca 616

<210> 172
<211> 613
<212> DNA
<213> Escherichia coli

<400> 172
aaatggcgac aaattatacc gtgctgactc tagaccccca gatgaaataa aacgttccgg 60

aggtcttatg cccagagggc ataatgagta cttcgataga ggaactcaaa tgaatattaa 120

tctttatgat cacgcgagag gaacacaaac cggctttgtc agatatgatg acggatatgt 180

ttccacttct cttagtttga gaagtgctca cttagcagga cagtctatat tatcaggata 240

ttccacttac tatatatatg ttatagcgac agcaccaaat atgtttaatg ttaatgatgt 300

attaggcgta tacagccctc acccatatga acaggaggtt tctgcgttag gtggaatacc 360

atattctcag atatatggat ggtatcgtgt taattttggt gtgattgatg aacgattaca 420

tcgtaacagg gaatatagag accggtatta cagaaatctg aatatagctc cggcagagga 480

tggttacaga ttagcaggtt tcccaccgga tcaccaagct tggagagaag aaccctggat 540

tcatcatgca ccacaaggtt gtggagattc atcaagaaca attacaggtg atacttgtaa 600

tgaggagacc cag 613

<210> 173
<211> 227
<212> DNA
<213> Escherichia coli

<400> 173
aagaagatgt ttatggcggt tttatttgca ttagtttctg ttaatgcaat ggcggcggat 60

tgcgctaaag gtaaaattga gttttccaag tataatgaga atgatacatt cacagtaaaa 120

gtggccggaa aagagtactg gaccagtcgc tggaatctgc aaccgttact gcaaagtgct 180

cagttgacag gaatgactgt cacaatcaaa tccagtacct gtgaatc 227

<210> 174
<211> 260
<212> DNA
<213> Staphylococcus epidermidis

<400> 174
ccactttttc tacttctaaa acttcagcaa gtgtttcacg taacttgcta gaacgagtaa 60

tttttatatc gtccttacca tcattttgtt ctatggtaaa tatattcata ttattttctt 120

ctttaaatat tgctgcatgt actgtaaact tatcgtagtc aatcatagtt agtactgtat 180

ctaggtgcat aaatgtgcgt gtattaggta tttcaatagc tacgattttt ttaaaacttg 240

tgtttgcatc tttgaaaata 260

<210> 175
<211> 422
<212> DNA
<213> Staphylococcus epidermidis

<400> 175
ttgataacaa acaaattctg ctatttcatt tgaagaagtc aaaatcatca aagtatcgtt 60

acctccaata gttcctaata tttctttcat ttgtaattga tctatgtaat aacttatact 120

ttgagcaaag ccaggagatg tttttattaa gacatagtta tttagcgtta taaattcaat 180

aatctcatca ctaaatattt ctaattgttt ttttgcactt aattgatttg tttgatttat 240

tttcttgtaa atatactttt tattttcaac agggattttg taaatttcta attcttgtaa 300

gtcacgagaa atagttgtca agctatagta aactccaaaa tgtcttgcca tgtaatccac 360

tatttgttgt tttttattaa actgattctg ttgtataaca gttaagataa gatttaaacg 420

tt 422

<210>  176
<211>  322
<212>  DNA
<213>  Staphylococcus epidermidis

<400>  176
taacactgaa ccccaatgac ctacaatatg ttctaatact tgtgccattg atggattagc      60

aagttttgaa atttggttct gctgaatgac accttgggct agtacagtca ttaagaaata     120

aatgactagc acagaaatca aaccaataac ggtagcagtt cctacatcct ttttagactt     180

tgcacgtcca gaaaagacaa cggctccttc aatccctgtg aatacccata cagttactaa     240

catagtactt tttacttgtg ccattgtatc tccccaacta aaaacgccaa cacttccact     300

agtcatacca taaaaaccgg at                                              322


<210>  177
<211>  733
<212>  DNA
<213>  Staphylococcus epidermidis

<400>  177
   cctcaaacaa gcagaaaaag ctaaaagcga agttacacaa tcaactacaa atgtatctgg      60
   tacacaaaca tatcaagacc ctacccaagt tcaacctaaa caagacacac aaagtactac     120
   atatgatgca tcattagatg aaatgagtac ttataatgaa atttcatcaa atcaaaagca     180
   acaatcttta tcaacagatg atgcgaatca aaatcaaacg aattctgtta caaaaaatca     240
   acaagaagaa acaaatgatt tgacacaaga agataaaaca tccactgata caaatcaatt     300
   acaggagaca caatctgtag caaaagaaaa tgagaaagat ttaggagcta acgcaaataa     360
   tgaacaacaa gacaagaaga tgactgcaag tcaaccttcc gaaaatcaag caattgaaac     420
   tcaaactgct tctaatgata atgaaagcca acaaaaaagt cagcaagtaa cttctgaaca     480
   aaatgaaact gctacaccta aagtatcaaa tacaaacgca tctggttata attttgatta     540
   cgatgatgaa gacgatgata gctcaacaga ccatttagag cctatctcat taaacaatgt     600
   gaatgctaca tctaaacaaa ctacttcata taaatataaa gaaccagctc aacgtgtaac     660
   aactaatact gtaaaaaaag aaacggcatc taatcaagcg actatagata caaagcaatt     720
   caccccattt agt                                                       733


<210>  178
<211>  507
<212>  DNA
<213>  Staphylococcus epidermidis

<400>  178
cttagggaaa aagatgggta gtaatgttaa agattctaaa attacaccga ataaaataag      60

tttatttacc ggttctttag ttactaatga aataactacg atagtacaat ataaaaatat     120

ggagagtatt ttttttcgct ttacaagacg tctaggtata ggttgtttct tagttgctgc     180

aggtgcgtat aaaatggtaa taattaatcc gactaatgcc atagataaaa gaacaaaata    240

gttaatatct aactttatta ttaagtatgg aaagataata aagaaaatta tgttctgaat    300

atgacataac aatgacgaat ttgcatgcgt accgtgtgca tgtctcctaa ttaaaaaata    360

acttaaatga gttaaaagtg tgtaaaagaa agtatgaaag attattgcta gcccatacac    420

aactatagac ttttcaatat ttatcgctag tacctgcatc cctaaacgaa tttttagaaa    480

ctgtatgtga tctaagttat ttttacg                                        507

<210>  179
<211>  512
<212>  DNA
<213>  Staphylococcus epidermidis

<400>  179
cctcgcatat cagtttgtga caccatataa agtaaaataa atgatatgaa aagtactatt     60

gttattatca ataagtatct tttattgagt gacaagtagg atacttttaa tttattgaac    120

aatagttgag ttaaataagc aataattaga gttatgatta caaaagaggt aaaatgtatt    180

aactgtaaag caaatttaaa cggaatataa tcttttatag tcaaatgtat gtatacagtt    240

atgaaattag ttatatataa gatcatagtg gtgaataata caactaatat tgaataaagc    300

tttatttttg tataaaagaa aatggtgatt attataacca aaactattaa tgctttactt    360

tgccaaaagt aatacattat tgcagaaggg attacaatcg taaaaacgat tatgtaatcc    420

ctaaaattaa atttcatatt aatgataact ttagtaaccc aaatcattaa aaagatttgt    480

aggcctgcaa acggaaatag attaatatca tc                                  512

<210>  180
<211>  534
<212>  DNA
<213>  Staphylococcus epidermidis

<400>  180
atgaagcaca accaccatac cttgtaaatt ttttttagat ttagttaaaa tggggataca     60

agatacatcg aaatatttag tatgtacgtt attaatagca acttctaatt gttcatagat    120

aactttttca gttctaaaac tttcaataat taatttttca atactatcat ctatgaaacc    180

aatgtaactt ttattttcta ccatttgatc agggttaaac acctgataat aagcatgatt    240

agcaactaca atttctccat gtttatcaat catcagtact gaactcggta tattttctaa    300

ggtagttttt aatctattgg attgaatttt ttgactattg tttaattttt gcaatcgtcg    360

tgctaagtca tttgtagtca caaataatgc cctagtttcc ttcacattac tttctggaac    420

acgaacatgg taatatccat ctgctagaag tgatgtagca taagttactt cattgatagg    480

tctaatatat gttcgattga tacttctact tgctaaatag accgtaaata atac          534

<210>  181
<211>  286
<212>  DNA
<213>  Staphylococcus epidermidis

<400>  181
ctaagcacaa gaaaggctca atattagcta tcataggttt gctaattgta tttgttgtta      60

caggttttat cttcttttca atgatttcag atcaaatatt tttcaaacat gtcaaaccag     120

ttgaaaaggt tgaaaaatta gataaaactt tagataaagc atctaaaaag caaatacaca     180

attatacgag ccaacaagta tctaacaaag caaatacagc ttggcgtgat gcgtctggta     240

cagaaattaa agaagctatg gatagtagta aattcataga tgatga                    286


<210>  182
<211>  381
<212>  DNA
<213>  Staphylococcus epidermidis

<400>  182
acgacgaatg attcataagg tttaatatgg tctaaattta tatcatttaa gtgataatta      60

tgcaatttta tatctacaga tgaaatatct aattcaaaag gtaggtttag ttctgatact     120

tcatttgtga gattggctac aattaataca gtattgtttt taaatgtgcg tgtatatgca     180

aaaacctgct tattttcagc atcgaccata ttaaacttac cgtagatgta aatcaaatca     240

gattttttta gttgaattaa cgctttataa taagaaagta tcgaaaactt atcatttagt     300

tgttgtttaa cattaatttc tgtatagtta gggtttacat gaaaccatgg cttaccagta     360

gtgaatccag cattgataga a                                               381


<210>  183
<211>  272
<212>  DNA
<213>  Staphylococcus epidermidis

<400>  183
ttaaaccatt aggaaatcgt gtgattattg agaagaaaga gcaagaacaa gcagctaaaa      60

gtggcatcgt tttaacagat agcgctaaag aaaaatcaaa tgaaggtgtg atcattgcag     120

ttggacaagg tcgtttatta gacaatggca cacaagttgc tcctcaagtc agtgaaggtg     180

acacaatcgt cttccaacaa tatgcaggta ctgaagtaaa acgtggcgcc caaacatatt     240

taattttaaa tgaagaagat atattagcta tt                                   272


<210>  184
<211>  614
<212>  DNA
<213>  Staphylococcus epidermidis

<400>  184
tcaagacacg ctttctagtg ttttatctct agaatatcct gaaaaagaaa ttatcattat      60

caatgatgga agttctgata atactgctga aatcatctat gaattcaaga aaaatcatga 120

ttttaaattt gttgacctcg aagtcaatag aggtaaagct aatgcactca atgagggaat 180

caaacaagca tcttacgaat atgttatgtg cttagatgct gacactgtca ttgatgacga 240

tgcgcctttt tatatgattg aagactttaa aaagaatcca aaattaggcg cagttacagg 300

taatccacgt attcgtaata aaagttctat cttaggaaaa atacagacca ttgaatatgc 360

aagtattatt ggttgtatca agcgaagtca atctctagca ggagcaatca atactatttc 420

aggtgttttc acactattta aaaaaagtgc actcaaagat gtaggttatt gggatactga 480

catgattact gaggatatcg ctgtttcatg gaaactccat ctttttgatt acgaaattaa 540

gtacgaacca cgcgcacttt gctggatgtt ggtgcctgaa actataggtg gtttatggaa 600

acaaagggtt cgat 614

<210> 185
<211> 329
<212> DNA
<213> Staphylococcus epidermidis

<400> 185
gttttcttat tacgaaccac attggttcta ccaattttca taatttaaat ttactttcaa 60

aaaagcaatt agatgaaatg tatgaaacag gcttatggga ctttgaatct catactcatg 120

atttacacgc tcttaagaaa ggcaataaat cgaagttttt agattcgtct caatctgttg 180

ctagtaaaga tattaaaaaa agcgaacact atttaaataa aaactaccca aaaaatgaac 240

gcgcacttgc ttacccatac ggattaatta atgacgacaa aataaaagct atgaaaaaaa 300

atggaattca atatgggttt acacttcag 329

<210> 186
<211> 220
<212> DNA
<213> Staphylococcus epidermidis

<400> 186
ttattctgct atatgatatt cacgaatatt gttatcaata gattttaaat agaaaatgtc 60

acgatctgca tttgattttt caagttcatg attcaattct aattggtcaa agcgtttgaa 120

gaaatgttca tattcatcaa cagaaacctc tattctatta tttaataaag atttgtggcg 180

ctcaacatct aattgctcct tgaatccatc tactaatgtt 220

<210> 187
<211> 210
<212> DNA
<213> Staphylococcus epidermidis

<400> 187
acattaagtc agcatttgga gaaaacatga ataaatgtct aaaccatatc gcaatggttt 60

gacgataatc aaattcaggt tgaatcgcat tggttacaag cgtagaataa caacccatta 120

ttaaaataat caacaaaacg atattcacaa atatatctga aaatgaactt aatcgtctaa 180

cgtttttgat ggatagtcgt cttaaagtta 210

<210> 188
<211> 200
<212> DNA
<213> Staphylococcus epidermidis

<400> 188
attagagcca aagtactctc caccgtaacc ttgacttcct tgcgctttat aagtatctaa 60

atatgtttct ttatgggaag aaggcacaac aaaacgatct tcatatttag caatacctag 120

taagcgatac atttcagtca tctgtctttc agtaagtcct aatcgttcta atttagaagt 180

atcgaaaggt tggtttgtta 200

<210> 189
<211> 284
<212> DNA
<213> Staphylococcus epidermidis

<400> 189
tttgatacct gtaatttgtt cttgccaagc gggagtatat ttagaagatg cgtcatcata 60

agatgtagct tctagttcgt gttcaaaacg ttgaacacca tattgactcg tcattaaatc 120

ataaaccgta gcaattttaa cttcttctcc gttagctaac tgaatagttc tcgttgcaat 180

aggtctctca aagataccat caccactgct atcaaaatat ggaaattgaa tcgtttcaac 240

atgatagtca ctttcaacca ttgataacat tggatcaatt ggtg 284

<210> 190
<211> 721
<212> DNA
<213> Staphylococcus epidermidis

<400> 190
agcttctcgc actacttgac taggatcatt agtgacctct attcctacca ttaaacctac 60

accacgtact tcaattacat ttcttttatt tactaaactt tttcttaagt tttcaataag 120

aaattgcccc tttgattgaa catcattcag caaatcagca tcattaatga tagaaagcgt 180

ttggtttgca gcagccaatg ataatctatt tccaccgaat gttgtaccat gagaaccgta 240

gccaaatgca tgacctaaat tctttttgcc taacattgct ccaataggaa ggccattacc 300

taatccttta gctaatgtga tgatatctgg agacaattga taatgttcat gagcatataa 360

cttaccggtt ctacctatgc ccgtttgaac ctcgtctaca attataagga tatctttttg 420

tttacaatac tcatttaatt gcttcataaa taaaggatca gcaggtagta ctcctgattc 480

accttgaatt atttctataa ttacagcagc agtattattt gaagttaatg atttaaatga 540

attaaaatca ttaaaaatag caaatttgaa tccaggaaca accggaccaa attgatctgt 600

aattttcttc tgtcctgttg cagacattgc gccgtacgtt ctgccgtgaa aagacttttt 660

aaaagcaata atttccgact taccagtagc tttacgtgcg agtttgatag ctgcctcatt 720

c 721

<210> 191
<211> 465
<212> DNA
<213> Staphylococcus epidermidis

<400> 191
aaagaaatta agtctctagc caaaatgtat cttggtggta gtactgaaat taaaacatca 60

caacttaaag gtaaggatga ctacttaaat gatatatact attaccaccc aagcgtaaaa 120

agtattatgg aatattcaaa tcttttacgt aatgatttag atttatctca aataacaaac 180

aaaaacgatt tcttagatca aagagtcatt aaacgatatg gttcactcgt acccttaaca 240

gaattagatg aagacttatt gcgtaagaac caaaaggaat cgactgatag tcagaaagag 300

tctgattctt catcacaaaa taatgatgaa gaagatcaaa ctaacgaaca aacagaccaa 360

aatagcttaa acggaaacga acagtaccca aatcaacaag acaacaatca aaccaatggt 420

gaaaatggta tgataaataa tgacaattat ccttacgcac aataa 465

<210> 192
<211> 362
<212> DNA
<213> Staphylococcus epidermidis

<400> 192
aaccaaacga tgctagatga ttgctttgaa ataagaaagt gtgttttcgt cgaagaacaa 60

ggcgtaccac tcgaaaatga atttgatcaa tatgaagatt actcattcca tatagtggga 120

tatataaatg gtgttcctat ggcaactgct agaattagac ctttaaatac tcatatttgt 180

aaaattgaac gtgtagcaat catcaagtgg tatcgtggtc ttgggtacgg taaaaattta 240

atacatgcta ttgaaacaat tgcaaaaaaa caccaataca atgaactcac tatgaatgct 300

caattacaag ctcgagactt ttacttaaaa ctaggttact caccttttgg taaagtattc 360

tt 362

<210> 193
<211> 320
<212> DNA
<213> Staphylococcus epidermidis

<400> 193
agttttataa tattcagtgc aaaattcaat tattgcgttt tgaagtggat aatagtattc 60

ggttgttaaa gatagttcat tatataaata aaatttttct ctattagttt tacatttgat 120

ttgttccttt ttccactgtt cttgccattt agattcttct atatttaaaa tttctaaaaa 180

tagattttct tttgttttaa agtgataata aagattccct ttactacttt ctgataattt 240

aacaatttct ccagtagtag tggcattata tccatttttt ataaataatt cctttgcgac 300

acctagtatt ttatctttca 320

<210> 194
<211> 503
<212> DNA
<213> Staphylococcus epidermidis

<400> 194
tttagagaga cagctagata atttgaaaac atttggcgta gagaaaatat ttacagagaa 60

acgatcgggg aaatcagtag aaaatagacc tgtatttcaa gaagcactta actttgtgag 120

aatgggcgat agatttgtgg tagaatcgat tgatcgctta ggtcgtaatt atgatgaagt 180

gattaacaca gttaattatt taaaagataa agaggttcaa ttgatgatta ctagcttacc 240

tatgatgaat gaagtcattg gcaatccatt attagataaa tttatgaaag acctaatcat 300

tcaaatatta gcaatggttt cagaacaaga acgaaatgaa agtaaacgta gacaagcaca 360

aggtattaaa gttgcgaaag aaaatggtgt atataaagga cgccctctat tgtactcacc 420

taatgctaaa gatcctcaaa aacgcattat ttatcataga gttgtagaaa tgttagaaga 480

aggtcaagca attagtaaga ttg 503

<210> 195
<211> 320
<212> DNA
<213> Staphylococcus epidermidis

<400> 195
tgaaagaagg gatagttttg cactttacac aacgtgaaca agacaaattg atgatagttg 60

tagctgctga ggttgcacgt cgtagaaaag caagaggact taaacttaat catcctgaag 120

cacttgcttt aatcagtgat gaattattag aaggcgcgcg tgatggtaaa acggtagctg 180

aactcatgag ctatggaaaa acaattttaa acgaggaaga tgtcatggat ggcgtagcta 240

acatgattac agaacttgaa attgaagcaa cttttccaga tggtactaag ttaataacag 300

tccatcaccc aatcgtttaa 320

<210> 196
<211> 503
<212> DNA
<213> Staphylococcus epidermidis

<400> 196
atgcaaatta tggagatgaa gctactttcg gtggcggaaa atcaattcgt gatggtatgg 60

ctcaaaatcc taatgtgaca agagatgata aaaatgtagc cgatttagtt ttaactaacg 120

cattaattat tgattatgac aagattgtta aagcagatat cggaattaaa aatggttata    180

tttttaagat cggtaaagct ggaaacccag atataatgga taacgttgac atcatcattg    240

gtgcaacaac tgatattatt gctgctgaag gtaaaattgt tactgccggc ggtatcgata    300

cacacgtgca cttcatcaat cctgaacaag ctgaagttgc acttgagagt ggtattacaa    360

cgcatatcgg tggaggaact ggtgcttctg aaggtgctaa agcgactact gtaacaccag    420

gaccttggca tattcatcgc atgttagaag cagcagaaga gatgcctatt aatgtaggat    480

ttactggtaa aggtcaagct gtc                                            503

<210>  197
<211>  452
<212>  DNA
<213>  Staphylococcus epidermidis

<400>  197
tgattataga agaaattcaa ggaaatattg ctaatttatc tcaagatgaa aagcaaaaac     60

atgtcgaaaa agtttatctt gaaaactcag atttggttaa acgtatacaa cgtgttaaaa    120

cagatcacgg taatgaaata gggatacgtc ttaaacaacc tattgaccta caatatggtg    180

atattttata tcaagacgat acaaacatga ttattgtcga tgttaatagc gaagacttat    240

tagttattaa acctagaaat ttaaaggaaa tgggagacat tgctcatcaa ctaggtaatc    300

gccatctgcc tgcccaattt acagaaactg aaatgcttat tcaatatgac tatcttgttg    360

aagatttatt aaaagagttg ggtatcccct actcacatga agacagaaag gtcaatcaag    420

catttcgaca tataggacat tcacatgatt ga                                  452

<210>  198
<211>  524
<212>  DNA
<213>  Staphylococcus epidermidis

<400>  198
ttaacttatt cagatgggat agctatgaga attgtctacc acgcattaat taacaatgac     60

aaagataaaa ttttagatat taaccaaaaa ctcttcgtac aaaatctacc taaagaaacg    120

cgtattggcg ctaagcaaat gggtacacgc atggtaaaat tagctttaga tctttatgat    180

agtgaatgga ttcaatggta ttataatcaa atgaaaaaca ataaaattaa gcttcatcct    240

gctgtgtgct ttactatgct aggacatttt ttaggtgtag atgtggaatc catcattgat    300

tattatttat atcaaaatat ctctagcctt acccaaaatg cagtaagagc gattccttta    360

ggacaaacag ctggacagca agtcgtaact gaaatgatag cccatattga gaagacacga    420

aatcacatac tagaattgga cgaaatcgat tttggtatga ctgctcccgg cttggaactt    480

aatcaaatgg aacatgaaaa tgttcatgtt cgaatcttta tttc                     524

<210> 199
<211> 500
<212> DNA
<213> Staphylococcus epidermidis

<400> 199
tcgtatatgg aatttgtagc agatcctatt attgcctatg aaaacgctaa atttttccaa 60

cataatacgt ttaatcttaa agaagatagt gctatgtttt acactgatat attgactcca 120

ggctattcat ctaatggcca agatttcacg tataattata tgcatcttat taatgaaatt 180

tacattgaca atcaattagt tgttttcgat aacatgatgt taagtcctga taaaagcaga 240

cttgacggca ttgggtatat ggaaaattat acacacttag gatcagctta ttttattcat 300

ccagatgtaa accaaagttt catagacgat atttacgcgg cggttgctga ttttcaaaaa 360

caatacgact gtagaatagg tatctcacaa ttacctactc atggattggc cgttcgtatt 420

ttgactaaaa gaactcaaat aatagaagaa attttgactc gtgttcaatc atatatcaat 480

caaacgattt atcatcgaca 500

<210> 200
<211> 363
<212> DNA
<213> Staphylococcus epidermidis

<400> 200
gcttaacaac gtaaaacaag ctggcgttga tcaaattgta actattattg gtcatggcgc 60

tgagagtgtg aaagatacat tgggtaatca atcattatat agttttcagg ataaacaact 120

tggaacagct catgctgtga aaatggcaca tgaacattta gcagataaag aaggaactac 180

tctagtagta tgtggagata caccacttat tacataccaa actttacaat cacttattga 240

acatcatgaa agtacacaat cacatgttac tgtattatct gcttctacta tcaatcctta 300

tggttatgga cgaattatta gaaatcataa tggaatatta gagcgtattg ttgaagagaa 360

aga 363

<210> 201
<211> 780
<212> DNA
<213> Staphylococcus epidermidis

<400> 201
agctcacggc tttaactatg ctatttataa agatgtggtt ttagatttct gaaaactgga 60

atatattctc tttgttgatc tactaagtct acatcgatat gtgttgttag tactccttct 120

ttatcatcta aatggtctaa aatttcacca ttaggattaa tgacaattga atttccagca 180

taattggtgt gaccatcatc accacaacta ttacaagcta caataaaaat atcattttcg 240

attgctctcg cttttagtaa tgataaccaa tgatctagtc ttgagctagg ccactgcgct 300

acataaaaag caattttagc accttttcta gctggatagc gcaatatctc tggaaatcgc 360

aagtcataac aaatgatttg cgtcacaagt gtttgatcag ataaataaaa aggttcaggg 420

actacatttc caccacataa aaagtctggc tcacgtaaca ttggcacgag atgtactttg 480

tcatattcat taatcaattc tttgttttta ttaattgcaa aagcagtatt atatatatgg 540

ttttctctta tatttgacac tgaacctgca atgatatcta cattaaatgt atgtgctaag 600

tcttttataa agagagagct gtctttaaga tttttatcag ctttttgttc taattcttct 660

aatgcataac cgttattcca catttctgga agcacgacga cactggtatc tttatctaag 720

tattgattaa acttagtttt gatattttgc atatttttat caacatttcc acgttctaca 780

<210> 202
<211> 501
<212> DNA
<213> Staphylococcus epidermidis

<400> 202
gtatttacgt gcgtttattt gtgtcataat catcgtgaca cacttactaa cgcaaatcac 60

tttagaaaat gaacagatgt ctgatagttc actcatattg caatattata tacgcaatat 120

ttttattttc ggcaccccta gttttataat attgtctcaa ttattaacaa cattaaatta 180

cgaatcagta actataaatt atcttttttc aagatttaag tatattttta ttccatatct 240

tttaatcggc ttgttctata gttatagtga atcacttatc accgcttctt cttttaaaaa 300

gcagtttatc gaaaatgttg ttttaggaca atggtatggc tatttcatta tcataattat 360

gcagttcttt gttctatctt atatcattta caaaattaat tttagattgt tcaatagtaa 420

aattttgctg cttttagcat ttatagtcca acaatcttat ctacattatt ttttgaataa 480

tgacactttt catcaattca t 501

<210> 203
<211> 300
<212> DNA
<213> Staphylococcus epidermidis

<400> 203
ggtcaagccc agacagaggc aatatccaac ggtaacctct tatttaaata tagttaggga 60

gagcttattt attactatat ccggagtatt ttggatgtat tgtatcgttg tgatgattgt 120

ttatatagga actcttatca attctcaaat ggaaagtgtt ataacaatac gtattgcatt 180

aaatgttgaa aacacggaaa tttacaaatt attcggatgg atgagtttgt ttgtacttat 240

tatatttatc ttttttacat ttagtctcgc gtttcaaaaa tataagaaag gtcgtgacat 300

<210> 204
<211> 406
<212> DNA
<213> Staphylococcus epidermidis

<400>  204
catttaacag tgaatatact tggtctttaa aacggttttt actgtcctca ataaccccga      60

atttttgtga aaaggaggct ctaaaatacc aagtctcaag aaaaagaaga attaagttta     120

taaagtcctc tttattcaaa gcgatgtgcg taggatcata atactttatc aattcatcat     180

gtaaggtagt attaatttct tgaagatggt gtttgatttc tgaattcagt gcttctggag     240

cactagataa ttgaacatat aatttaatat atctctcatc aacgtcgaat ataaatttga     300

ataaaaactg gtaaagtccg tcaatggaat aattatcgtc atggttccta agcaaaaaat     360

ctataaagta attgaaacaa ttctcaacac tttttcgata tatttc                    406


<210>  205
<211>  325
<212>  DNA
<213>  Staphylococcus epidermidis

<400>  205
atgtcaaaat tagcagaagc tattgcaaat acagtaaaag cagcacaaga tcaagattgg      60

actaaattag gaactagtat cgttgacatc gtagaaagtg gcgttagcgt attaggtaaa     120

atcttcggat tttaattaat cttagttttt taaaatataa atttaaataa ttaattaggg     180

agagataaac atgtcaaaat tagcagaagc tattgcaaat acagtaaaag cagcacaaga     240

ccaagattgg actaaattag gaactagtat cgttgatatc gtagaaagtg gcgttagcgt     300

attaggtaaa atcttcggtt tctaa                                           325


<210>  206
<211>  451
<212>  DNA
<213>  Staphylococcus epidermidis

<400>  206
tgacacaata cctcatgaac cacccaaata agttgatacc cctcacttat tttgtaaaga      60

aatttaaaca agcaaaatcg tcaattagtg aagacgttca aatcattaaa aatacgtttc     120

aaaatgaaaa attaggaact attattacta cagcaggtgc tagcggtgga gtaacctata     180

agcctatgat gagtaaatca gaggccacag aggttgttga tgaggtgata gagcaattac     240

aagagaaaga ccgtttgcta cctggaggat atttattttt atccgattta gttggtaatc     300

cttctctatt aaataaagta ggtaagttaa ttgctagtat atatatgaac gaagaacttg     360

atgctgttgt taccatagcg actaaaggga tatcacttgc gaatgcagtc gcaaacgtat     420

taaatttacc tgtagtggtt ataagaaagg a                                    451


<210>  207
<211>  300
<212>  DNA
<213>  Staphylococcus epidermidis

<400>  207
gtgacagatg taagacttag aaaaatacaa acagacggca gaatgaaagc actcgtttcc      60

attacgctag atgaagcttt tgtaattcat gatttacgtg taattgaagg aaactcaggt     120

cttttcgtcg caatgccaag taaacgtaca ccagatggtg aattccgtga catcgcgcat     180

cctatcaatt ctgatatgag acaagaaatc caagatgcag tgatgaaagt atatgatgaa     240

actgatgaag ttattccaga caaaaatgct acttcagata acgaagaatc agacgaagct     300


<210>  208
<211>  380
<212>  DNA
<213>  Staphylococcus epidermidis

<400>  208
atgaaaataa tcaactcaga taaggtaccc gaagcactag gcccatattc gcatgcaact      60

gttataaacg gttttgtctt tacatcaggt caaattccac tcacacttga tggaacaatt     120

gttagcgatg atgttcaaga acaaactaag caagttttag aaaatttaac tgtggtatta     180

aaagaagcag attctgattt gaattctgtt gttaaagcga caatctatat ttctgatatg     240

aatgattttc aacaaattaa tcaaatctat ggaaactatt tcgtcgaaca ccaaccagct     300

cgtagttgtg ttgaagtgtc acggttgcct aaagacgtaa aggtagaaat tgaattgata     360

ggtaaagtga aggaattata                                                 380


<210>  209
<211>  245
<212>  DNA
<213>  Staphylococcus haemolyticus

<400>  209
atgaacatga gcgacatcat ctttcttaat ggcatgcgtt tttatggcta tcatggagcg      60

cttcatgcag aaaatgaact tggccaaatt tttatagtag atgtaacact taaagttgat     120

ttgactgaag cagggaaaac ggataatgtc aaagacactg tgcattatgg tgaggtcttt     180

gaagatgtta aaaacattgt tgaagggcca tcttgtcaat tgatagaaca tcttgcagaa     240

cgtat                                                                 245


<210>  210
<211>  563
<212>  DNA
<213>  Staphylococcus haemolyticus

<400>  210
ttgaattggg aacgacagct ttgaaaggtg caatcgattc agcaaatatt gatcctaata      60

taatacaaca agttattttc ggtaatgtgc tacaaagtgg tgtaggacaa aacccagcac     120

gtcaaattgc gattaaagcg ggtgtacctg atacaacacc agctatgaca attaatgagg     180

tatgtggatc aggtcttaaa gcaattatat tagggaaaca gttaattcaa ttaggtgaag 240

cggatgtagt agcagtgggt ggagttgaaa gtatgacaaa tgccccacaa ttaatcttaa 300

aagaaggtca agaaccagtg gaaagcttta tgcatgatgg tttaacagat gcctttcatt 360

atgtaccaat gggtgtaaca gctgagaaca tagctgaaaa atatgacatc acgcgtgaaa 420

tgcaagatga gttcgcaaat cattcacaag ctaaagcagc taaagcgacg caagatggta 480

aatttaataa tgaaatcatc ggtatgactg acgcagaagg ggaacaaatg acttctgatg 540

aaggtgttcg cccaaatagt agt 563

<210> 211
<211> 231
<212> DNA
<213> Staphylococcus haemolyticus

<400> 211
aatgacgatg aaacttcctt tgcacaccgt gttgaagcgg atggctggga aaatgaattg 60

gctatggttt ttgttgttat taataacaaa tctaaaaagg tatccagtcg ttcaggcatg 120

tcacttacac gtgatacatc acgtttttat caatattggt tagataacgt tgaaccagat 180

ttgaaagaga ctaaagaagc cattgctcaa aaagatttca agcgtatggg t 231

<210> 212
<211> 278
<212> DNA
<213> Staphylococcus haemolyticus

<400> 212
catcaattgt gtgataatga taagaattat atgcaagttg ttaaacatat tggttcttta 60

gtgtattcag ctagtgaagc gattgagcat catagttttg atcaattagc tacaatcttt 120

aatcaatgtc aagatgactt aagaacattg acggtgagtc acgacaaaat agaaatgttt 180

cttcgcttag gagaagagaa tggttcagtc gctggcaaat taacaggtgg cggccgtggt 240

ggtagtatgc ttatcttagc taaagaattg caaacagc 278

<210> 213
<211> 200
<212> DNA
<213> Staphylococcus haemolyticus

<400> 213
acgtatatcg tcctgaatat tttctaagta gtaaatagac ttatcgtcat cagtttgttc 60

agtagcgtga tcgaattcta aatcatcgaa tcgcttgaag aaactttcat agtcttcaac 120

tgaaacttct tgacgttcat tcaataaggc tttatgtcct tcaatatcta attgtttttc 180

atagccttcg actagcgtag 200

<210> 214

<211>  565
<212>  DNA
<213>  Staphylococcus haemolyticus

<400>  214
aatcgtccac ttgtcttttg aaaatgactt catataaact ttgcctaact taatttgaaa      60

ggtaaggttt atggcgcatc aatttataca actagagaag acctttaaag cattccaaaa     120

tagtcgtagt agtcacgaac aagatagatt atttatagat atagtaaacc acatacaacc     180

taaacttttt ataaaattta aaagttatgg aatacaaaat gaagatattg aagatttagt     240

acaagaaact ttaatcagga tttatttagc acttcataca tttgatttta gtacagacgt     300

tccttttgaa cactatttga attgtatcgt acgatcgatg cgaaatgatt tttggagaag     360

aaaatatatt gagactgata agtacgatag catcattaat gactatgtta ttgactacaa     420

attgaatcaa tcaagtaaat atattgaaga tatttgtatg ataaaagaga aacgagaatt     480

gctagcgagt agtttaacag tattaagtcg attcgagcga aacgtagctg aattactaat     540

gtctgattat acgcctagtg aaatt                                           565


<210>  215
<211>  635
<212>  DNA
<213>  Staphylococcus haemolyticus

<400>  215
ccaagatgct aatgtgtctt caaaagaatc ggaaatcgac aaaaatatta ataaagtaga      60

cgacgcgcag tcttattctc aacaaaatga gcaacaatcc tcaaaagccg aaaataagga     120

aatacagaat tcaacacaag cagaacaagt tgaaaaacag gaacaacctg cttctaatca     180

gacggctaat cactcttcaa aagagtcctc cattaataat caggaaagtc ataacaaaca     240

gcaacctagt gatgacaaaa cacctaatat caaaccagaa aaaattgaaa aagtagataa     300

tcataagcgt attcaagatc agtatcaaga taaaaacaag caggttgata ataatcaatc     360

taacaattcg caattaaacc aaaaagaaca tcccaattca tcaaataata aacaacaaaa     420

gcaacgtcta gatgttaaac cacaaaacga taaccaacaa ttacaatctc gaaatgatgt     480

aaaagaaaaa ttagataacc agccaattga gcaaaaagat accaagctgc aaagtaacaa     540

taaaagcaaa gacaacacaa cttctgtaaa gtcacacagc caacaacata aaccgcattc     600

attaaagacc caatcccatt taactccagg tcaaa                                635


<210>  216
<211>  468
<212>  DNA
<213>  Staphylococcus lugdunensis

<400>  216
tgcgaattaa acagttaggc attaatgatc aaatgaattg cgtaaaattg tataatgata      60

ccaaggagcg tgacgctaat ttgaaggcga tagacaaaaa aattgaaaga tttgctagat 120

acttgcagcg tcaaaacaat ctagaccata ttcaattttt gaagatacgc ctaggcttac 180

aagtcgcatt aggtaatttt ttcaaaacta ttgttactta tggtgttgct cttttattcc 240

atacctttct ttacacatta attacacact taacgtattt tttcgttaga cgttttgcgc 300

atggtgcaca cgcaaggtca tcattgttgt gccacattca aaatttagtt ttatttgtgg 360

cattaccttg gtcaattgtg cattttcaag tgtcttggac attcatgatt tttgtagcat 420

ttatcgcatt cataattatt atatgttacg caccatcggc aactaaaa 468

<210> 217
<211> 450
<212> DNA
<213> Staphylococcus lugdunensis

<400> 217
tttaatgttg ttatttgttg ctaaaatagt agccgatatt aaatttcaaa tgagggatta 60

ttttgccatt tttggtatca taatcccttc aactatactg tttggcgtga taggtagaca 120

gtctttaata tttttgataa ttggatgttt aatattcttt tatttgaaaa taggcttata 180

ttccgtttta gcaatctttg gttctgcgct tattatgtat gttagtaatt atatttctgt 240

catccttagt gtaattgctg attatttttc tttaagttat atagttcaaa taataataat 300

attagtttcg tttactctaa tatcaataat ttgtgcttat ttcattaggt ttctattaat 360

aagctcaaaa aaaacctatc tgtatttcaa caaaatatac atatcagtaa tatctatttt 420

ccttatttta tctttgatca tgctctattt 450

<210> 218
<211> 466
<212> DNA
<213> Staphylococcus lugdunensis

<400> 218
tatcaatctt tcaagcagtt atgttaataa ttgtagctaa aattattgca aacgttaagt 60

tttacttaag ggattattta gccgttgccg gcataatagt cccttctgcc gtattatttg 120

ttgtttttgg cagacaatca attatctttt tacttattat ttgtttaata tacttttatg 180

taaaaatagg gttttattct ataatcgcta tattaggctc tgccttaata atgtacataa 240

gtaacttttt ctcagtttca ctcataatat taataggtaa ttttatcaaa tttaggataa 300

tatacgtaat aatttcttta tcatcataca tactgatagg tgttttatgt gcatttatga 360

caaaatactt aattaataaa ctcaaaaaaa catacttatt ttttaataaa gtatacataa 420

tcgtcatatc tactttttta acatttacca tcgctatatt ttattt 466

<210> 219
<211> 512

<212>  DNA
<213>  Staphylococcus lugdunensis

<400>  219
caaaggagtg tgattttatg tcaaaaatgt tagttctttt ttctacatgt attcttttaa      60

tgtcgatgtc gttaattttt atgcctgtta gtcatgcgca aggtttatcc tctaagcaag     120

caacgttgta tcagcagaat ccaaaagata ctaatactca agtttcagga aaactgaata     180

attcgaaaga aacaaaagca aatgatacag caaccttatt tgcaaactct aaagtcaatc     240

aatatattat cgacaatcat cttcagcatt cgccagtagt aaaagatcca cgtatggata     300

cacttcctaa attagaatat aaaaacggca cttacatggg tgttgttatt cacgaagtgg     360

gcgaagacaa tcgtccttta caagtatggg tagatcgcat gtatgaaact tatactagag     420

catttgtaca cgcattcgtt gataataacg aaatacatct tactgcacct gcagaatatt     480

atgtgtgggg agctggtcct aaagctaatc ca                                   512


<210>  220
<211>  646
<212>  DNA
<213>  Staphylococcus lugdunensis

<400>  220
gaagtggagc gtaatttgtc aaaacaacaa atacagcata ataatgatgc tactggtgac      60

actcaagatg ataataatta taataatgaa atatcaaatc aggaagcaac aacgcagaac     120

aaacaaataa ctcagtctga caatgtaaat agcgaggcac aagcaataaa tgaaataagc     180

gacagccatc gtacagtaaa taaagccact gaagcactag acaataactc tactttaaat     240

acatccaccg atgtatcacc tgcaacgaaa caagatacaa ctactagcaa tcaaacaact     300

caggaaaaca atgatgcaac aacacaaacc aaaacaaatt ataagcaaga tggtaataac     360

aacgtattat cccaagtagc aaccaatgac aatcagtctt caaatcaacc acgtaacagt     420

cacctaaata catccacagt aacatacaac aataatcatc aagtaagaag attagcaaaa     480

gttgaagcaa caaatacaga taataacgtt actcagactt cagacatatc gaataaactc     540

tcaaatgtaa cagcgacaat tgaagcggca gatacgattt acccacataa agcagaatat     600

gtaaatttaa attatcgttt ccaagcccca gatgatgttc aagcag                    646


<210>  221
<211>  500
<212>  DNA
<213>  Staphylococcus lugdunensis

<400>  221
tgtcaggtat cgtagatgca attactaaag cagtacaagc aggtttagat aaagattggg      60

ctacaatggc tacaagcatt gctgatgcaa tcgctaaagg tgtagacttt atcgctggtt     120

tctttaacta aaatataaat tgagacttta acaataatcg taaaaaggag cgtttacaat     180

atgtcaggta tcattgaagc aattactaaa gcagtacaag caggtttaga taaagattgg    240

gctacaatgg gcactagcat tgcagaagca cttgctaaag gcattgacgc aatttcaggc    300

ttatttggtt aatctcaaat ataataaata atactattta aaataaaaat atttttaaag    360

gagcgaacat atcatggacg gaatttttga agcaatttct aaagcagtac aagcaggttt    420

agacaaagac tgggctacaa tgggtactag cattgcagaa gcacttgcta aaggtgtaga    480

ctttattatt ggattattcc                                                500

<210>  222
<211>  500
<212>  DNA
<213>  Staphylococcus saprophyticus

<400>  222
gaaataaccg cattccaact aacactttaa ttaatggaga aaagagaacc aaaccaatcg     60

atgtgcctga aatttttaaa gtcttaagct caatgattcg tagacgttta tatcattttg    120

ctatacatcc aaatgaccaa gaagatttgt gtcaagatgt gctcgtaaga ttatactgtg    180

catttaaaaa atttgatttc actgatgaca cacctattga gcattatgta aatcgtgtga    240

ttaaaaatgt aaaaaatgat tatatccgta aaaaatgcta tggcaaccaa cgacaagaaa    300

tgctggtcaa tgaatttata gtcaatgatc aaaatagtaa aacagaacac ccacttgata    360

aacatatatt agctttagag ataggaagtc aattacaaca gggattaatg aaactgacgg    420

tcttagaaaa aagtatcgta atctatttac taaatgactt taagccgaaa gaaattgctg    480

aaacactaaa tatacaaatc                                                500

<210>  223
<211>  432
<212>  DNA
<213>  Staphylococcus saprophyticus

<400>  223
aagagaacca aaccaatcga tgtgcctgaa atttttaaag tcttaagctc aatgattcgt     60

agacgtttat atcattttgc tatacatcca aatgaccaag aagatttgtg tcaagatgtg    120

ctcgtaagat tatactgtgc atttaaaaaa tttgatttca ctgatgacac acctattgag    180

cattatgtaa atcgtgtgat taaaaatgta aaaaatgatt atatccgtaa aaaatgctat    240

ggcaaccaac gacaagaaat gctggtcaat gaatttatag tcaatgatca aaatagtaaa    300

acagaacacc cacttgataa acatatatta gctttagaga taggaagtca attacaacag    360

ggattaatga aactgacggt cttagaaaaa agtatcgtaa tctatttact aaatgacttt    420

aagccgaaag aa                                                        432

<210>  224

<211>  200
<212>  DNA
<213>  Staphylococcus warneri

<400>  224
aaaagatatg acataatgtt acgaatagtt aaactatccg gatcaaatgt taactttaca      60

cattcagcat aaccatcgta ttcaccattc aaattcgatg ttattccatt agcccttcca     120

gcttcagttg atacgatacc tggtatagtt ttaaaaaaag cttgaacgcc ccacaaacaa     180

ccgccagcta catatactat                                                 200


<210>  225
<211>  515
<212>  DNA
<213>  Staphylococcus warneri

<400>  225
catccaattt acagaaccat ctttttcatc tatgactgca ttattaatta taatgcttac      60

taaattgtcg attgcatcgt caatattgtc tgaatttact atttcatatc cataatttat     120

aaatccatta ccatcaataa ataatttatt ttgactttct aatgaaaatt ttattagttt     180

acattgaaac aacaaatctt tcaaagaata tctttgcgtt ttttctaaaa atacattgag     240

tgggtttttc aataagtgat gtaccgtatt atttttaata tcttttaccg aaacactttg     300

gaccttagta taaaaatagg gtactgaaag agtttctatt tgttttattt ctgaatttat     360

taacttatca cttaataaat tttcaccgta ctcttctagt ttgttaaaca agctctttcg     420

cttatttgca taaagaggtg atttagcagc ttgtattaat actgagtact caattgtact     480

tcttggtaaa attctcactt ctacttctga tgacg                                515


<210>  226
<211>  320
<212>  DNA
<213>  Staphylococcus warneri

<400>  226
tgtatcaact ccactttatt catattaatg acgacgcact tacactcaca aagtcaaagc      60

aagacaccat tcacttattt ataggcaatt ggattaaccc atcagcccaa aaatctatta     120

gcattcgaac tggcgttgat acgaatcaca atcaatatca aattcttcaa attgataccg     180

aacatcaacg tattaaactg acttctgaag aagatcctca actcatgtat attttagact     240

acgaagatac aaaccatata ttcatacaaa catcagttaa gaattcgtat ggcacgtcaa     300

gacccataag atacgaaaaa                                                 320


<210>  227
<211>  271
<212>  DNA
<213>  Staphylococcus warneri

<400>  227
agcaagttct ttgttaattg caactttgac atcagcgaca ttaattaatc cggcacatgc      60

agaaacgaca tcatcaaccg ataatcacca acaaaccaca caatctcaac aacaaaagac     120

accgaagatt gataaaggta ataacgtcaa acctgttgaa aagaaagaac gcgcaaatgt     180

catactacct aacaatgatc gacatcaaat taatgataca acgttaggtc actatgctcc     240

tgttactttc gttcaagttc aatcaaacga a                                    271


<210>  228
<211>  500
<212>  DNA
<213>  Staphylococcus warneri

<400>  228
tattgtcaaa gtcacaacaa ttagatatag aattaaaagc gatacttcaa caattcaatt      60

cttttattat gagaagaatt aattatattt ctcaaaatga ttttgaaaaa gacgaccttt     120

atcaagaagt gctcatcaaa atatatctag cgcttgagcg ccatcatttt caatatgatg     180

attcgtttat aaaatatata tcgcggctca tcaaatcagt taaatgtgat tactatcgac     240

ggcattacac tcaacagaag cgatatacga atgtagttaa tgatgctgtg gttgaatatc     300

aaacgaacct gcttaataga gatcgagttg aaagagaaat attaacatgt gaagcaatca     360

aactattgaa cgcggcgtgt gagaaattaa ctaaacaaga acgagaagta tttgaatttt     420

atagtaaagg ttataaacca aaagaaatcg cacatttact aggtataaaa gacaaagtag     480

tttacaatgc gatacaacgt                                                 500


<210>  229
<211>  400
<212>  DNA
<213>  Staphylococcus warneri

<400>  229
tcagatataa acaatttaac aaggatgtta tcactgtagc ggttggctac tatctaagat      60

atgcattgag ttatcgtgat atatctgaaa tattaaggga acgtggtgta aacgttcatc     120

attcaacggt ctaccgttgg gttcaagaat atgctcccgt tttgtatcaa atttggaaga     180

aaaaacataa aaaagcgtat tataagtggc gtgttgatga gacatatatc aaaattaaag     240

gacagtggtg ttatctgtat cgcgcgattg atgcagatgg acatacatta gatatttggt     300

tgcgtaagca acgagataat cattcagcat atgcgtttat caaacgtctc attaaacaat     360

ttggtaaacc tcaaaaggta attacagatc aggcaccttc                           400


<210>  230
<211>  758
<212>  DNA
<213>  Staphylococcus warneri

<400>  230
taatcaaacg caacaacaac cttcagaacc aacaaaagcg aaagattctg atacaaataa      60

tacgaatgtt gaacgtcctg aatcgaattc gacacaaaca tcaaatcaag acactgacaa     120

aatgcaggat acatcaacta atcaaacaaa cgaaaattct aaacatatta ttgataaaac     180

taatgacgtt tcacatgaaa ctacaaagac aaatgataca gatcaaacgt catctcaaga     240

caattcagaa caatctcttg aagtcgactc aaatgaggca ccagcttcaa atgacaaatc     300

aactccaacc aaacaagaac ctactaattc aaagcaagat attgatgaaa catctaaacc     360

taatgaagat tcaaaacttg taccatcaaa gtcaaatata acatctaaag cagataaaca     420

agaacagtct tctaaagaac ctgttgagga taatgctcaa aaagataaac atgtatcaca     480

agaagattca tctttagaaa agcaaggtac acaagaggtc ccgcagactg acacacataa     540

agatgtcaat gtaacacctt caaagtcatc atcagaacaa caactatcta caacacaaca     600

cattacagct aaagattcta gtgcttcaca agaggtgcca gttcattcac tagattcatc     660

taaacaagat cacacaacat cgactgagag ccatatcaat ttagataacc tagataaaca     720

agcgactaaa gatcgtacac ctacagataa tggcgatg                             758


<210>  231
<211>  562
<212>  DNA
<213>  Candida albicans

<400>  231
aaacgcattg ttaagagacc cagaaatcaa aactggtaaa gtgtctgttg cttcatactt      60

gaagtttttg gattctgttc aattcaagag ttatggagac gaacctttgg aagtattggc     120

tattgtggta gaacaaaatg acaaaattcc taaattagac gagtttttgt catccaagac     180

aggttggtta aacaatgtta ccgataatat tttcaatgct atcaagaaag attacagtca     240

attatgttgg gttgttaatg aaaacgatgc caacttacct tggtatttct ccaaatcaga     300

tggttcattt gccaagaatg gccaaatctt gttttggtac ggtttaaaca ttgacgaagc     360

tagtaaattg attaaagaat ttgattcttc atctattgga tcatcgttgt catcttctaa     420

agaatctggc gtattcacat ctgctcaaca aaagcgtggt ttccaccact ctacagtccg     480

tagaaacacc aatcctaatc ctccattatc tgaaggtaag caaaccgaga gaaaaaaagt     540

tgctttgatt ggtgctagag gt                                              562


<210>  232
<211>  524
<212>  DNA
<213>  Candida albicans

<400>  232
caggtaagtc aaagtctggt gagttatctt ctactggttc tgtgacaact aatacagcaa      60

caccagatgt tccatcaact aaagtacctt cgaatccagg ggcaccaggt actggtgttc 120

caccaccttt agcaccatcg acagaaacac aaactaccaa taatgtacca ggctcaccaa 180

atatccctgc cactggaaca actgatatta ttagagaatc aactactgtt tcacacacag 240

tgaccgggaa tggaaatact ggcgttccaa tgaatccaaa ccctgcgttg acaacaggca 300

cttcactgac tggcgcaacg aattctgcaa ctaacccatc tcatgaaaca ggtgttaata 360

caggatcagg aggctcaact aatattgtca ctccaccttc ttctgcaact gcgacagtgg 420

ttattccagg aactgataat ggtgctacta ccaagggtca agatacagct ggtggcggca 480

actctaatgg atctactgct accaccaata tacaaggtgg caat 524

<210> 233
<211> 230
<212> DNA
<213> Candida albicans

<400> 233
gattaatgac atcaagggtt tagttaaagg cattaaaggc aaaaacggga aatcctactc 60

aagtgtccca gttgggactg ttgattcttg ggatgtctta gttgatggtg ccagtaaacc 120

agccatcgat gctgcagatg ttgtctactc caactccttc tcatactggc aaaaaaacag 180

tcaagctaat gcttcatact ctcttttcga tgatgttatg caagctttgc 230

<210> 234
<211> 632
<212> DNA
<213> Candida albicans

<400> 234
tctggtgaag gtttaggaag aaagaaatca ttaattagac cagaaagatc aagaatggat 60

gaaagccatc cacgattcca ttatactcaa gttgcaaatc aagaatctaa tcatattaaa 120

gtacagccat cttcaactgg tgttgatcct cgtaaatcaa atgaattatc aacatcaaga 180

tcacatttga gtaattacgc tactccacca catcaagagg aagaagaaga cgaagggatc 240

cctttaatgg atatacacaa tgcttcaccc aatgttagca gtgaccaaaa taatgatcta 300

aaaggtggac gtgaagttta tggattaaat gatgaaatca acgattatgg tagttcaccc 360

aagaaaaacc aagtcatttc atcttcaaga ccaatgaaca acgaaaaacc agctaaacct 420

aaacatgata tatatttctg gaaagtttat tgttatgcta ttacattttg ggcaccagct 480

ccattattga aattatttgg attaccaaca aaagatcgtc aattcgcttg gagagaaaaa 540

atagggttga tttcttgtat tctttacgtt ggggcatttg ttgcttattt gacttttggt 600

ttcactaaaa ctgtttgttc gagtcaagtg gt 632

<210> 235
<211> 633

<212>  DNA
<213>  Candida albicans

<400>  235
caccaaactc aggcttattc aaacaaggat actcctcctt ctccaatgcc gacggagcca      60

ttatcagaaa tgttgaagca gttcgtgaaa tcgcctctat cttactcacc tccatgggtc     120

caagtggaag aaacaagatc atcgtcaaca agttgggcaa aaaattcatc accaacgatg     180

ccgccaccat gcttaacgaa ttggaaattg tccaccccgt agtgaaaatc ttgatccagg     240

catcaaagca gcaggaattc gaaatgggcg acaacactaa cctagtaatc atccttgctg     300

gcgagttcct caacgttgct gaaaaattgt taacattggg cttgaatgtc agtgaaatca     360

tccaggggtt caacttggca aacaagtttg tgatgaaaac attggacgag ttggtcgttg     420

aaaaagtcga gtcgttcgaa actgacctat taaaagcagt gaagccagtg atcgccgcta     480

aacagtacgg cgtagaagat accatcgcca aactcgtcgt tgatgccgtt gccctagtta     540

tgaagaacgg gtctttcaat gtcgacaaca taagagtggt caaggtcatg ggtgcatcgc     600

tctcccaatc gcaagtggtc aagggtatgg tct                                  633


<210>  236
<211>  465
<212>  DNA
<213>  Candida albicans

<400>  236
gaatgcaaag aaacattgaa atcaagagta ttttgatcca attgaccatg tatgctaagc      60

ttaacgaaag ggtcgactat ttgttggaaa agttaacatc cactgaatta ttggatagtg     120

aaaaagtcat gtcaaagttg aattcagaat ttgatcctca agaaaaattc gattatgata     180

aattgattaa agacaagggt ctgaccttga gaaaaggatt gaaagatttg aaattcgata     240

gagaagagat tgaaaatact ccttgctata atgaaatgat tgaagatttg tttgttcaaa     300

tcaaggatga tcatccagag acaaaaaccg atggcgacaa attgattgaa tacttaaaag     360

aacatagaaa caggatcgac gatgttttgt ctaaacagac tataaaattg gatgatttat     420

tgtaccagaa agctcaattg atagtaagtg atgatttgca tacgg                     465


<210>  237
<211>  504
<212>  DNA
<213>  Candida albicans

<400>  237
tgtctgctgc tagtgaatcc aaatattcta ctgaagtgct ttccgaatta ttgagcaaat      60

tacaagttgc tgataataag gatgaagctg cttccaacat ttccactttt ttaaactcat     120

ctattgttga acacgatgtt ccagttgaat ttttcgaaga tttgaaaaaa caaattcaat     180

ctaaagatgc taaagtttct cttgctgctt tggatgctta caaacacatt gcttcaacca     240

acggtttatc cccatccgtt gaaccatatg ttgttgactt ggttagtgaa gttgccgtta 300

aagctggtga caaaaacaag gatgttcaaa ctgctgcttc tgatgcttta ttggccattg 360

cttctgccat caccccaact gctgtcaaag ccatcttacc aaaattgatt gacaacttga 420

ccaacaccaa caaatggact gaaaaagttg ccatcttgag agctgtttct caattggttg 480

acactgctaa agctcaaatt gctt 504

<210> 238
<211> 526
<212> DNA
<213> Candida albicans

<400> 238
tgacaggttc attggtgtct tacaaaagtc ttggtaaaaa aggtggattt tggattttca 60

cattattcaa ttatctctgt atcggtgttt tgacatcttt gttcattgtc tccattggta 120

atagaccaca tgcatcaaag aatattttca aaacattaat catattgtta accatatgtg 180

cattatacgc attggtggtt ggatttgtgt ttgttatcaa tactattgct acttttggaa 240

ccggtggaac atctacctat gtgctcgtta gtattgtggt ttcattgttg tccacctatg 300

gtctttatac gttaatgtcc attttgtact tggacccatg gcacatgttg acttgttctg 360

tacaatactt tttgatgatt ccatcgtaca cttgtacatt acaaatattt gcattttgta 420

atactcacga tgtctcgtgg ggtacaaaag gtgacaacaa tccaaaagaa gatttgagta 480

atcagtacat tattgagaaa aatgccagtg gagaatttga ggctgt 526

<210> 239
<211> 621
<212> DNA
<213> Candida albicans

<400> 239
tcagatggtg atgaactgtc gattgaattt cttaaccaaa gaagcaacac tccattaaca 60

caaggaactt ataattatca taatacttct actaattcac ttaatttcca acaaccagaa 120

ccaatttatc gtaatcaaac tcgtacatct ttaagtgatt cttattatga tcatcccata 180

tttgacactt ctcaaacaca gatccaacct ccacatgata atccattcac tgaaagttat 240

gaaatgacag atacttcata tcaaggtaat gatcatcatt atcgtactgg tcaacctaat 300

catctcatga accccactta taaccaagct ttcattcctc atgtttatga tgaagaagat 360

aatgatgaac aagaatatga tcaacgtatt cagtataatc aatttcaagg ggatcatttt 420

gatttggcag cgattagtta tgctgatgat gaaagtcaaa gtcagttgga ctatgtcccc 480

actgaacgtg tcatacctga aggagaggaa gaagaagagg aaggtgagac gagttttgaa 540

aaagaacctg gtagtgaaac catttctggc ccatttggag aagaacgatc atttgaagaa 600

cctcctccac aacaagaagt c                                              621

<210>  240
<211>  607
<212>  DNA
<213>  Candida albicans

<400>  240
aactagggct gctaatgttg ccactgaatt aactgctgct gcaccttatg aattgggtaa      60

attatattat aatggatttg aagatattgt cttgattgat aaaaaatatg gattagaatt     120

atttgctcaa gcagcagcat taggtcattt acaatcagcc gccattttgg gtcatcatta     180

tgaaattgga gaaattgttc ctcaagattc taatttatca attcattatt atactcaagc     240

agcattagga ggtgatccaa attcaatgtt ggcaatgtgt gcttggtatt tagttggtag     300

tgaaccatat ttacctaaag atgataatga agcatttgaa tgggctaaac gtgctgccaa     360

ttgtaattta ccaaaagctc aatttgcttt agcaaatttt tatgaaaaag ggattggatg     420

tattaaaaat attaatgaag ctcaatcatg gtataaaaaa gctgctgaaa atggtgatga     480

aaaatctttg aaacgattaa ctgataaaga attggttaaa accattcaaa aacaatggaa     540

aaagaaacct ccagtaattc ataatgaaga tggaacttct acaactaatt caggatctct     600

tgctcaa                                                              607

<210>  241
<211>  693
<212>  DNA
<213>  Candida albicans

<400>  241
agtcagagca gggtcaatca tcaaaatcag atgtgatcaa gatttcgata gtgaaaaaga      60

agaggcagag aaatttacca aaattcagga tgagatttta caaacatttg ctacaaattt     120

gccacaacca ccaaatttga aaatcaagaa cgttactcaa acctcgtgtg ttttagaatg     180

ggataaacta aacttgggca ccgccacatt gaaaaatctt attttattca aagatggtaa     240

aaaattaggc tcaattcctc agccattaaa taatcgaacc tcaaaattgt ctggattgcc     300

aattgacaaa tcttttaaag tacaattacg tttggatacc actgctggta ctttcttgtc     360

gaatgaaatt gaggtaacaa cccacaaaat gactgatttg tcaggaatta ctgtgtgtct     420

tggtgacctt acacctaatg atcaattcaa caaggaggac attgaagagg cattaaagaa     480

tatgggggca aaatatccag tgcaacaaca agtcaaagtc gacactacac atttcctctg     540

tactagagaa aacaaacaaa atcctgaata tgtgaaggca aatgatatga acattccaat     600

aattagacca gagtggttga aagcctgtga gagagaaaga agaatagttg gtgttagaga     660

cttttatgtg aaagattgtg tcttacccga cat                                 693

<210> 242
<211> 511
<212> DNA
<213> Candida albicans

<400> 242
gtcaacaaca aggcaagaca attttacttt cacttggagg agccacgggc aattacgggt 60

tttcttccga ctcagaagca gttcaatttg caggaacatt atggaataaa tttggaggtg 120

ggaaagactc agaaagacct tttgacgatg caattgttga tgggtttgat tttgatattg 180

aaaataaaga ccagacaggt tatgctgctt tagcgactca attaagaaaa tattttagca 240

ctggaactaa atcttattac ttgtcagctg ctccacaatg cccataccct gatgagtcgg 300

ttggtgactt aatgtcccaa gttgatttag attttgcatt tatacaattt tataacaact 360

actgttcgct caatcagcaa ttcaactgga actcatggag caactatgcc agaggtaaaa 420

gtattaaact ttatttgggc cttcctggct catcatcgtc tgctggctcc ggatttgttg 480

gtttgtcgac tgttcaaaga gtcgtggcta g 511

<210> 243
<211> 510
<212> DNA
<213> Candida albicans

<400> 243
ctgtcaagaa actgacgttg acattgtttt attgtcattc ttgaatttgt ttccagatcc 60

attgaacgtt aattttgcca accaatgtgg taacactttt gaatctggtt tgttacactg 120

ttctcaaatt ggtgctgaca tcaaaacttg tcaatcttta ggtaaaaccg tgttgttatc 180

tttaggtggt ggtgttggtg actatggttt cagcgatgtt gcttctgcca ctaaattcgc 240

agacaccttg tggaacaaat tcggtgctgg tgaagatcca gaaagaccat ttgatgacgc 300

tgttgttgat ggtttcgatt ttgacattga acacggtggt gctactgggt accctgaatt 360

ggctactgcc ttaagaggca agttcgccaa agacacttcc aaaaactatt tcttatctgc 420

tgctccacaa tgtccatacc ctgatgcatc tcttggtgat ttattatcca aagtcccact 480

tgattttgca ttcatccaat tctacaacaa 510

<210> 244
<211> 577
<212> DNA
<213> Candida albicans

<400> 244
ttggctcgat taagaaataa attaaattca aaatatatta tcacggtagc ggctcctggt 60

ggtagtgata atattgaaat tttgaagatt caagaaatgg ataaatattt gacattttgg 120

aatttaatgt gttatgattt tgctggtgaa ggctggtctt cgaaaactgc tttccattct 180

aatttatttg gtaataatgg ggataattca ttgaatgcat ctgatgttgt ccaaacttat 240

attaacaagg gagttcatcc aacaaaattg atattaggga tgccaatgta tggaagaata 300

tttcatggtg ttgatcgacc agaaattggt attcctttta caaaagagag aaaatcaggt 360

tgtatagaag ctgatgttgt ggactataac aaatttggtg atacattcga ttatgaagat 420

tttgatccac gcaaagtggg tgcattgaaa tatgattccc atagtaagca attaattaca 480

tttgataatc cccagtgtgc tagaataaaa gctagctttg tacaactgag acaattgggt 540

ggtgggatgt ggtgggattc tgctggtgat gtttcag 577

<210> 245
<211> 909
<212> DNA
<213> Candida albicans

<400> 245
gctccatcta gcaactcatc tggtgttcca gctgcgccat ctaacaattc atctggtgct 60

tcagttgttc catcacaatc agccaacaat tcatctgctt cagctgctcc atctaacaac 120

tcatctagtg ctatttctgg aagtgttgca ccatcaagct acggaaactc taccattgca 180

caaccatcta cttctacaaa atccgatgct gcatcaatta ctggtccaat tactacagac 240

aaggttataa ccaatgagtc tggcattgtc tttacatcta cagtaatcat tacacatgtt 300

tctgaatatt gtgaccagac ttctgctgct gctgttcaat catcagcatg tgaagaacag 360

tcaagtgcta aatcagaaca agcttctgct tcatcagaac aagttaaggt cattactagt 420

gtggtttggt gtgagtcatc tattcaatct attgaatctg tcaaaacaag tgcagaagct 480

gctcataaga ctgaggttat tgctagttgt gcaagtgaat taagctcttt gagttctgct 540

aaatctgaag ctatgaagac tgtttctagt ttagttgaag ttcaaaaatc tgcagttgcc 600

aaacaaacct cgttggctgc tgtacaatca tctgctgctt ctgtacaatt aagtgctgct 660

cacgcccaaa agtcgtctga ggcagttgaa gttgcccaaa ctgctgttgc tgaagcttct 720

aaagctggtg atgaaatttc gactgaaatt gttaacatca ccaagacagt ttcttctggt 780

aaggagactg gtgtttccca agctactgtt gctgctaaca cacattcagt tgctattgct 840

aatatggcaa ataccaagtt tgccagcaca atgtcgttgt tggtcgctag tttcgtgttt 900

gttggtctc 909

<210> 246
<211> 537
<212> DNA
<213> Candida albicans

<400> 246
gacactccgt cagattcaac tccaactaaa aaaccagaac cgactataag tccagagttt 60

agaaaaccca gcataagtct gttaacttct ccaagtgttg cacataaacc tccgccacta 120

ccaccgtcac tgagtctggt tggaagtagt gagcattcga gtgcaagatc gtccccggct 180

atcacgaaga gaaactcgat tgcaaacatt atcgatgctt atgaagaacc agctactaaa 240

actgaaaaaa aggctgagct aaactcacca aagataaacc aactgacacc ggtgccaaag 300

cttgaggaac acgagaatga tacaaacaaa gtagaaaagg ttgtggatag tgcacctgaa 360

ccaaaaccaa aaaaggagcc tcaaccagtt tttgacgacc aagacgatga cttgacaaaa 420

atcaaaaagc tcaagcaatc taagaaacca cgtcggtatg aaacacctcc aatttgggcc 480

cagaggtggg ttcccccaaa tagacagaag gaggaaacta atgttgatga cgggaat 537

<210> 247
<211> 561
<212> DNA
<213> Candida albicans

<400> 247
acatagtcag ccacaaccac aaccacaagc aacacaacca agatcaaata gaagtagact 60

gcaaacgagc ttttctaaac caagaggtag caggcaagtt agtggcagtg gcaggtcaac 120

cggggccaag aaacaatcag caatcacact gggcagtact ggtactggcc ctgcccgaaa 180

tgctgataca ggtatgacat cagttgctaa tagcacttcc acaaccacta tgacaaccac 240

caacaataac aacaaattgt ctgtttcagc cccagtaaat gtgatatatg ctaatcttcc 300

tgagagactt caacaggtgt taccagcacc gccgttatca cgtgctccag taagacctga 360

tgtaacggtc aatttgacat caaaacgagc caaaagaaaa tcaaaattca ctccggaaca 420

agatgacatg atcgtgaatt tgaagaaaaa ggggaaatca tgggttgaaa ttgccgaaat 480

cactggtgtt ggatcatatt tagcggcacg gaatcgattt caagttattg ttggacagca 540

aggaaataac aattcgagtg c 561

<210> 248
<211> 351
<212> DNA
<213> Candida albicans

<400> 248
tcaagaaagc tactgatggt ggtccacacg gtgctatcaa tgtctctgtt tctgaaaaag 60

ccattgacca atctgttgaa tatgttagac cattaggtaa agttgttttg gttggtttac 120

cagctcacgc taaagtcact gctccagttt tcgatgctgt tgtcaaatcc attgaaatca 180

aaggttctta cgttggtaac agaaaagaca ctgctgaagc tattgacttc ttctccagag 240

gtttaatcaa atgtccaatc aagattgtcg gtttatctga cttgccagaa gtcttcaaat 300

tgatggaaga aggtaaaatc ttgggtagat acgtcttgga caccagtaaa t 351

<210> 249
<211> 707

<212>  DNA
<213>  Candida albicans

<400>  249
ctcagtcctt tgctacaacc actacagtta ctgctcctcc aggtggtacc gatactgtga      60

ttatcagaga gccaccaaac catactgtca ctactactga atattggtca caatcctttg     120

ctactactac tactgttact gctcctccag gtggtactga ctcagtaatt atcagagaac     180

caccaaatcc aactgtcact acaaccgagt attggtctca atcctttgct actactacta     240

cagttactgc tcctccaggt ggtactgact cagtaattat cagagaacct ccaaacccaa     300

ctgtcaccac cactgaatat tggtcccaat cttacgcaac cacaactact gtgactgctc     360

ctccaggagg cactgactca gtaattatca gagaaccacc aaaccacact gtcactacta     420

ctgaatactg gtcacaatca tatgccacca ctaccactgt aactgcacca ccaggtggta     480

ctgacactgt tatcattaga gagccaccaa accacactgt cactactact gagtattggt     540

ctcaatcgtt tgctactacc acaactgtaa ctggtccacc aagtggcact gatactgtta     600

tcattaggga accaccaaac ccaactgtca ccactactga atactggtct caatcatatg     660

caaccactac taccattacc gctccacctg gtgaaactga taccgtt                   707


<210>  250
<211>  586
<212>  DNA
<213>  Candida albicans

<400>  250
aacggtcata tccaaagaag ttactggtgt tttcaaccaa ttcaattcat tgatatggtc      60

ttacacatac agagctcgat acgaagaaat atctactctt accgctaatg ctcaattgga     120

atgggctttg gatggtacta ttgccagtcc cggtgataca tttacattag tcatgccctg     180

tgtatataaa ttcatgacgt acgaaacctc agtgcaatta actgccaact ctattgcata     240

tgccacatgt gactttgatg ctggtgaaga cactaaaagt ttttcaagtt tgaagtgtac     300

ggtgactgat gagttgacag aagataccag cgtttttgga agtgttattt tgcctattgc     360

tttcaatgtt ggaggttccg gatctaaatc tacgataaca gactccaaat gtttttcaag     420

tgggtacaac actgtcacgt tttttgacgg aaacaatcaa ctttctacaa ctgcaaattt     480

tcttccccga agagaactag cgtttggtct agttgttagt caaagacttt ccatgtcgct     540

cgatacaatg actaattttg ttatgtctac accttgtttc atgggt                    586


<210>  251
<211>  692
<212>  DNA
<213>  Candida albicans

<400>  251
aacattagaa acggaacagg ccgtcctcgt aagactccca gatccaagct ctatatggtt      60

taccccccac tttcaggtga ggactcaaca aatcctgaac cagaagaggg tagttcacag 120

gaaaacaatc ccacagaacc tagttcctca caatcaaatt cagtacaaaa tcaagaccaa 180

agtgaagacc agagtcaact accacaacaa gaactgaata cacaacaaga gctgaataca 240

caacaagaac tgaatacgcc atcacccagg gcgtcaaaca catcaactga aactcctgct 300

cctttaagtc ccatacaacc aggaattcga aatattcctc tgggattatt attaccacaa 360

gaaaaagttg gccgtcttat gggatatcca ttttaccgcg attttaattt taccctaaat 420

ccagagagat atcagaaact tatttatgtg tttcagatac ttaaaaatgc tgctcgtaat 480

cacagaaatg gagcttctct acttagaaag tatttcctgt tagcgagaag gtctaaaaga 540

acaacagaca tgtttgtaac caccatagag gaaatgcgga agaggctgtt ggaaaatagt 600

cgtaagagag agctcgagga agcgcaagaa agggaagagt caaataaaag acaacataca 660

gaatcaagtg cagaaccaaa tgcagaactg ag 692

<210> 252
<211> 506
<212> DNA
<213> Candida albicans

<400> 252
caaagttcca ccatttcaac tccagtagac tcattaccta caagtggaag aagtactcct 60

aatccgaatg catcaaccac ttcattaaca tcattgaata ctgctcttgc taaattaaat 120

gtttccaata ttccatttga agaaaatttg agtaatattg agaaagccgg taagatagct 180

gagattagac ccgaagtgga aaccattgtt aagataattg atgaacaaga agatttatgc 240

attattaatg aatggaaatt gaatgaaatt ttgaaatctt tattgaaacc taaaagtcct 300

gcattagtta aagaaggagc tttattaatc attcaacaat tggcaactaa atttggtggt 360

caaaccccca aagaagctta tttattacag tttttaagta ctgcttatga tatgtttact 420

gataaagata aaaatgttgt taaagctgct aaatctgcta ctgatgcatt atttggaatt 480

taccctgtgg aagcattagg atcaat 506

<210> 253
<211> 520
<212> DNA
<213> Candida albicans

<400> 253
atcgacatca acaggcttac cacctaattg gacgattaga gtatccagat cccataacaa 60

agagtatttc ttaaaccaat ctaccaatga gtcgtcttgg gacccacctt atggcactga 120

caaagaagta ttgaatgcat acattgcgaa gtttaaaaac aatggttaca agccacttgt 180

gaatgaggat ggccaggtta gagtttctca tttgttgatc aagaacaatc aatcaagaaa 240

acccaagtct tggaagtccc cagatggtat aagtagaact agagacgaat ctatacagat 300

attgaagaaa catttggaaa gaatattgag tggtgaggtt aaactaagtg aattggcaaa 360

taccgaaagt gattgcagct cacatgacag aggtggtgat ttagggtttt ttagcaaagg 420

acaaatgcaa ccaccattcg aagaagccgc attcaatttg catgttggag aagtcagtaa 480

cataattgaa accaatagtg gtgtccatat cctccaaaga 520

<210> 254
<211> 507
<212> DNA
<213> Candida albicans

<400> 254
caatagcaca ggcacaatct ggaactggta aaactgctac tttttctatt ggtatgcttg 60

aggttataga tactaaatca aaagagtgtc aagcacttat cttgtctcct actagagagt 120

tggcaattca aatacaaaat gtggtcatgc atttaggaga ttatatgaac attcacaccc 180

atgcctgtat tggtgggaaa aatgtcggtg aggatgttaa gaaattgcag caagggcaac 240

aaatagttag tgggacacca ggtagagtga ttgatgtgat aaaaagaaga aatctacaaa 300

ctagaaatat caaggttctt attttagatg aagctgatga actttttaca aaagggttta 360

aagaacagat ctacgaaatc tacaaacatt taccaccttc ggttcaagta gtagttgtta 420

gtgccacttt gccacgtgaa gtattggaga tgacaagtaa gtttaccact gatccagtga 480

aaatcttggt gaagagggat gagattt 507

<210> 255
<211> 535
<212> DNA
<213> Candida albicans

<400> 255
ttcattcaaa ccagccttac cacaagataa actcacgggt gtagatgata tccctgatag 60

agaacttacc gatattgaaa gaatcaacat caatgctgcc aattccaatt tacaaagaaa 120

attgaaaaca agacatttac aaatgatcgc tattggatca tctataggaa ccggtctttt 180

cgttggtact ggtggtgcat taagtactgg tggaccagct gccattgttc tagcatgggc 240

cataagtgct atatcggtat ttatgacaat gcaaggatta ggtgaattgg ccgttgcatt 300

cccagtttct ggtggattca atttatacgc aagtaaattt ttagaaccag gtattggatt 360

tgctgttggt tggaattatt tcttacaatt ctttgtatta ttgccattag aattagttgc 420

tggtgctata actatcaaat attggaatgc tagtataaat tctgatgtgt ttgttattat 480

attttggttt gtggtgcttg tgatcaccat gttgggtgta agatggtatg gtgaa 535

<210> 256
<211> 433

<212>  DNA
<213>  Candida albicans

<400>  256
cacaaggtta tacattcaga aaactaaaac ttactgatta tgataatcaa tatttagaaa      60

ctttaaaagt tttgacgaca gttggtgaaa tttccaaaga agatttcact gaattgtata     120

atcattggtc ttcattgcca tctatttatc atccatatgt aatcaccaat gcatcaggta     180

tagtggtagc cacggggatg ttatttgtgg agaaaaaatt gattcatgaa tgtggtaaag     240

ttggtcatat tgaagatatt tcagttgcta aatctgaaca aggtaaaaaa ttgggatatt     300

atttagtcac ttcattaacc aaagttgctc aagagaatga ttgttacaaa gtcattttag     360

attgttctcc tgaaaatgtt ggcttttatg aaaaatgtgg ttataaagat ggtggtgttg     420

aaatggtatg tag                                                        433


<210>  257
<211>  540
<212>  DNA
<213>  Candida albicans

<400>  257
aaaccataaa tcaacaacca cttgcttcgt caagatgggc tgcttgtgcc attggtggtg      60

ttcttgcttc atttattcaa attcttgcca cacttttcga atggattttc gtgcctagag     120

aatgggccgg tgctcaacat ttgagtcgtc gtatgctatt tttggtgtta attttcttac     180

tcaatttggt tccaccagtt tatacattcc aaattaccaa attggtgatt tattcgaaat     240

cggcatatgc tgtgtcgatt gttggatttt tcattgctgt ggccacttta gtattctttg     300

ccgtcatgcc attgggtggt ttattcactt catacatgaa caagagatca agaagatata     360

ttgcatcaca aacatttact gccaactaca ttaaattgaa aggtttagat atgtggatgt     420

cttatttgtt atggtttttg gttttccttg ccaaattggt tgaatcttat ttcttctcga     480

ctttgtcttt aagagatcct attagaaact tgtcgaccat gacaatgaga tgtgttggtg     540


<210>  258
<211>  574
<212>  DNA
<213>  Candida albicans

<400>  258
tattatggcg attccacaga gttgatattg gtgatatcac aaatatggaa cagcattatc      60

atttccatgt acagggagca tgttctctcg gttgaacaag tttgcaagtt gatttatcaa     120

cgaggagctg atgaaaacac tatacgacca ccactatttt ttgtttacga agatgataac     180

aaattttatg attttattaa aatcgaaaag gaatgggaaa gaaggatcac attttttgct     240

caatcgttat caagcccttt accagaacca tttccagtag tttctacacc aacatttacc     300

gttttgattc ctcattactc agaaaaaata ctattaagtt tacaagattt aattaaagaa     360

caaagctttt caaaactaac gttgctagat tatttgaaac aacttcattc gaaagaatgg 420

gattcatttg ttcaagatag taagatgatc caaactataa aggaaatgga tgaagacaag 480

tttgtacgcg aaaatatgga tgatttgccg tactactgta tcgggttcaa agattcttca 540

ccagaaaatg ttttacgaac aagaatttgg gctg 574

<210> 259
<211> 506
<212> DNA
<213> Candida albicans

<400> 259
cgtttgttat ttgctgttcc taaaaagggc agattatacg aaaaatgctg taacttattg 60

agtggtgccg atatacagtt tagaagatct aatagattag atatagcact ttctacaaac 120

ttgccaattg cattaatctt cttgcctgca gctgatatcc cagttttcgt tggagaaggt 180

aattgtgact tgggtataac tgggttagac caaatcaaag aagctgaaca attcgacaac 240

atcgaggact tgttggattt gaaatttggt tcatgcaaat tgcagatcca agttccagca 300

gatggcgagt acgaaaagcc agaacagctt gttggaaaga aaattgtgtc ttcatttaca 360

aaattgagta ccgactattt caaacaattg tcagacaaac ctactaatat cagatatgtc 420

ggtggttccg ttgaggcttc ttgtgccttg ggtgttgctg atgctattgt cgatttggtt 480

gaaagtggtg aaactatgaa agcagc 506

<210> 260
<211> 539
<212> DNA
<213> Candida albicans

<400> 260
agctaaatcc aaagacgatg acgcatcggc atatgtcggt gtcgggtcca tcgctgctgg 60

tggccgttac gacaatttag tgggtatgtt ctccaacggt aaatccatcc cttgtgttgg 120

tgtatcgttt ggtgttgaga gattattctc catcatcaag aaccgtgcca atctcaacaa 180

catctccgcc aaccacactg acgtgtttgt tatggcattt ggcggcggcg aaggctggaa 240

cgggttctta aaagaaagaa tggaaatcac caacaagtta tggaaagctg ggatcaacgc 300

cgagtacttg tacaaatcca aagccaacat tcgtaaacaa ttcgatgccg ccgaaaaggc 360

cggcgccaaa ttagctgtca ttcttggtaa agaagagtac ccacaaggcc aattacgaat 420

caaagtgttg ggccagggag aggaaaacga aggtgagttg gtcaccaaag atgaactact 480

tgctgctgtc caggccaagc tcagctctga catcgacgac atttctcgca taatcaagg 539

<210> 261
<211> 1030
<212> DNA

<213>  Candida albicans

<400>  261
gctaccactc caaacacttc tgttccaaca acttcttcag aatcaactac tccagctact      60

agcccagaaa gttctgttcc agttacttct ggatcatcta ttttagctac cacttcagaa     120

tcatcatctg ctccagctac tactccaaat acatctgttc caaccactac tactgaaacc     180

aaatcatcaa gtactccatt aactactact actgaacatg atacaactgt tgtcactgtt     240

acttcatgtt ctaacagtgt ttgtaccgaa agtgaagtta ctactggtgt tattgtcatc     300

acatctaaag atactattta caccacttac tgtccattga ctgaaactac tccagtttct     360

actgctccag ccactgaaac accaactggt acagtatcca cttctactga acaatcaact     420

actgttatta ctgttacttc atgttctgaa agctcttgta ccgaatctga agttactact     480

ggtgttgttg ttgttacttc tgaggaaact gtctacacta cattctgtcc attgactgaa     540

aacactccag gtactgattc aactccagaa gcttccattc cacctatgga aacaattcct     600

gctggttcag aatcatccat gcctgccggt gaaacctctc cagctgttcc aaaatcagat     660

gttccagcta ctgaatcagc tccagttcct gaaatgactc cagctggttc acaaccatct     720

attcctgccg gtgaaacctc tccagctgtt ccaaaatcag atgttccagc tactgaatct     780

gctcctgctc ctgaaatgac tccagctggt actgaaacta aaccagctgc tccaaaatca     840

tcagctcctg ccactgaacc ttccccagtt gctccaggta ctgaatccgc accagctggt     900

ccaggtgctt cttcttctcc aaaatcttct gttttggcta gtgaaacctc accaattgct     960

ccaggtgctg aaaccgctcc agctggctca agtggtgcta ttactattcc ggaatctagt    1020

gctgtcgtct                                                           1030


<210>  262
<211>  528
<212>  DNA
<213>  Candida albicans

<400>  262
ttggtgggtt agaagttgag aaaggtgctt ctttatttat taagctggac aatggtcctg      60

tcttagctct taatgtcgct ttatcaactt tagttagacc agttataaat aatggtgtta     120

tttcattaaa ttctaaatct tctacaagtt tttcaaattt tgacattggt ggatcttcat     180

tcactaataa tggtgaaatt tatcttgatt cttcgggtct tgttaaaagt acagcctatc     240

tttatgcacg tgaatggact aataatgggt taattgttgc ttatcaaaat caaaaagctg     300

ctggtaatat tgcttttggt actgcttatc aaaccatcac taataatggc caaatttgtt     360

tgcgtcatca agactttgtt ccagctacaa aaatcaaagg tactggttgt gttactgctg     420

atgaagacac atggattaaa cttggtaata ctattttatc agttgaacct actcataatt     480

tttacttgaa agatagtaaa tcgtctttga ttgttcatgc tgtttcaa                 528

<210> 263
<211> 528
<212> DNA
<213> Candida albicans

<400> 263
caagagaaag ggaaagaaga gaaaaaggac acagcctttc aaacatcttt tgatagaaat 60

tttgatcttg ataattcaat cgatatacaa caaacaattc aacatcagca acaacagcca 120

caacaacaac aacaactctc acaaaccgac aataatttaa ttgatgaatt ttcttttcaa 180

acaccgatga cttcgacttt agacctaacc aagcaaaatc caactgtgga caaagtgaat 240

gaaaatcatg caccaactta tataaatacc tcccccaaca aatcaataat gaaaaaggca 300

actcctaaag cgtcacctaa aaaagttgca tttactgtaa ctaatcccga aattcatcat 360

tatccagata atagagtcga ggaagaagat caaagtcaac aaaaagaaga ttcagttgag 420

ccacccttaa tacaacatca atggaaagat ccttctcaat tcaattattc tgatgaagat 480

acaaatgctt cagttccacc aacaccacca cttcatacga cgaaacct 528

<210> 264
<211> 360
<212> DNA
<213> Candida albicans

<400> 264
cgttaactca gtcatatact acattttatt cctttttgca tcaacaatcc ttgcggcaga 60

taaaacgtcc agttcagtat cacctacttt agtatgggtc acaggtactg atgccaatgg 120

gaaattagcc accacccaat caacatatta tcaaagcttt atgagtactt ataccacagc 180

tgaaacccca tcgtctggtt ctattggatt ggggtcaatc agtggaacag taggagaaat 240

cagaacttat agtatgacta ctatatcaca aggtaatggt gggttatcaa aattcaatca 300

aaatggttta gaaatgaaga atttgtcatt tgttaaatta attggggttt cttttattgc 360

<210> 265
<211> 701
<212> DNA
<213> Candida albicans

<400> 265
gatccagatg ctgtaaccac agccaatgga acattaaatt tacgtatgga tgcttataaa 60

aatcataatt tattctatcg ttcaggaatg gtacaaagtt ggaatcaatt gtgttatact 120

caaggtcatt tagaaattct ggctcgttta ccaaattatg gtaatgtaac agggttatgg 180

cctgggttat ggtctatggg gaatttaggt agaccagggt atttgggatc tactgatggg 240

gtatggccat attcttacga ttcatgtgat gccggtatta cacctaatca atcttctcct 300

gatgggattt cttatttacc aggtcaaaga ttaaataaat gtacatgtcc aggtgaatta 360

catcctaatc gaggtgttgg tagaggtgcc cctgaaattg atgttattga aggtgaagtg 420

atgactgata gtagtggtaa aaaagaaaat tgtggtgttg cctctcaatc cttacaattg 480

gcccctatgg atatttggta tattcctgat tataattggg tggaaatcta caatttttca 540

gtttcaacga tgaatactta tactggtgga ccattccaac aagcattatc agcaacaacc 600

atgttgaatg ttacatggta tgaatttggt gataatgccc ataatttcca aacttatggt 660

tatgaatatt taaatgaccc tgaaacgggt tatttacgat g 701

<210> 266
<211> 794
<212> DNA
<213> Candida albicans

<400> 266
taatttccct tgttgtttcc ataataagat gtgttgttgc agatgttgac atcacatcac 60

caaagagtgg agaaactttt tctggtagtt ctggatcagc aagtatcaag attacctggg 120

atgattcaga cgattcagac tcaccgaaat ctttggataa tgccaaaggg tacacaattt 180

ctttatgtac tggacctact tcagatgggg atatccagtg tttggatcca ttagtcaaga 240

acgaagctat tgcaggtaaa tctaaaacag tttctattcc ccagaactca gtacctaatg 300

gttattacta tttccaaatt tacgttactt tcactaatgg aggtaccact attcattatt 360

caccacgttt caaattgact ggtatgtctg gtccaactgc cactttagat gtcaccgaaa 420

caggatcggt gccagcggat caagcttcag gatttgatac tgcaactact gccgactcca 480

aatctttcac agttccatat accctacaaa cagggaagac cagatacgca ccaatgcaaa 540

tgcaaccagg taccaaagtg actgctacaa cctggagtat gaagttccca actagtgctg 600

ttacttacta ctcaacaaag gctggcacac caaatgtggc ctctactatt accccaggtt 660

ggagttatac tgctgaatct gccgttaact atgctagtgt tgctccatat ccaacatact 720

ggtatcctgc cagtgaacga gtgagtaagg ctacaattag tgctgctaca aagagaagaa 780

gatggttgga ttga 794

<210> 267
<211> 654
<212> DNA
<213> Candida albicans

<400> 267
acattcattg ggttcatctc cagaaaacaa taatgccctg ggtccattaa gtggagttcc 60

aactccatca ttttctaatt tgaatgatta tttccaacaa aaaagtaaca gcaataattc 120

tcgattattt aatgctagtt catcatcatt gagttcatta agtggaaaaa taagatcttc 180

ttcatcgact aatttagctg gtttacaaag attaactcca ttaactagta ctacaaacaa 240

tacaaacaac acaacaacat ctaatactaa taataataat atgacaaaac caagtataat 300

accaaaacaa ccatcttcta catcattaaa tttagaattt tataatggca acaatcaaca 360

acaacagaat tatcataccc ataagaaatc tcgaccaaat tcaccatcac aaaccccaat 420

tcatttatca agttcacgta aaagcgctaa taatctgttt ataatatcac ctaatgaaac 480

cccattacaa actccattac aatcaccaca attaaaacca tatcaagatc aaccaccaac 540

taatgtcaat atcaacgtta gtgcaccatc agatacattt attggaactg ctgttactga 600

aaaattaaat aatattagta gtattgctgg taatggaaca caattaccac caat 654

<210> 268
<211> 529
<212> DNA
<213> Candida albicans

<400> 268
tgtcccagaa agtgctaaac acattttcaa ccaagaaact ttagcatttg ttgccacttt 60

gcaccgtggt ttcgaagcca gaagacaaga attgttgaac aacagaaagg aacaacaaaa 120

attaagagat caaggtttct tgccagattt cttaccagaa actgaataca ttagaaatga 180

tgctacctgg actggtccac cattggctcc aggtttagtt gacagaagat gtgaaatcac 240

tggtccaacc gacagaaaaa tggttatcaa tgccttgaac tccaatgttg ctacttatat 300

ggccgatttt gaagattcat tgaccccagc ttggaaaaac ttggttgaag gtcaagtcaa 360

tctttacgat ggtgtcagaa gaaacttgac tgctaacatt aatggtaaaa attatgcctt 420

gaacttggac aaaggtagac acattccaac gttgattgtg agaccaagag gatggcattt 480

ggatgaaaag catgtattgg ttgacggtaa accagtttcc ggtggtatt 529

<210> 269
<211> 647
<212> DNA
<213> Candida albicans

<400> 269
ttagctcatc aacatcatca acataaagaa gaaaaaagag ctgttcatgt tgttaccacc 60

accaatgttg ttgttgtcac cattggtaat ggtgatcaaa ctaccacttt tgctgctcca 120

tctgtagctg ctgattctag tgttagtgtt tctgtcaaca ctgaaccacc tcaaaatcac 180

ccaactacta ctcaagatgt tgcttctgct tctacttatc catcttccac tgatggttct 240

gccgcttctt cttctgctgc cgcttcttct tcttctcaag ctggttctga accttctggt 300

ggtgttggat ctggtggtgc taaaggtatt acttattctc catacagtga caatggtgga 360

tgtaaatcat catctcaaat tgccagtgaa attgctcaat tatctggatt taatgtcatt 420

cgtttatacg gggttgattg tgatcaagtt gcagctgtat taatagctaa aacttcatct 480

caaaaaattt tcgctggtat tttcgatgtt tctagtatta catctggtat tgaaagttta 540

gctgaagccg ttaaaagtat ttgcggtagt tgggatgata tttacactgt ctctattggt 600

aatgaattgg ttaatgctgg ttctgccact ccaagtcaaa ttaaagc 647

<210> 270
<211> 636
<212> DNA
<213> Candida albicans

<400> 270
actgtcgttt ctggtcattc tggtaaagat acttcctctt ctaaatcaac tgttgccgaa 60

tacactgggg ttgaagaaat cactaccacc ttgaattatg actatttagt tgttggtgtt 120

ggtgctcaac catctacttt cggtattcct ggagtcgctg agaattcaac ctttttgaaa 180

gaagtcagtg atgcttctgc tattagaaga aaattgatgg atgttattga agctgccaat 240

attttaccta aagatgaccc agaaagaaag agattattgt ccattgttgt ttgtggaggt 300

ggaccaacgg gtgttgaagc tgctggtgaa atccaagatt atattgacca agatttgaag 360

aaatgggttc ctgaagttgc cgatgaattg aaagtctcct tggtggaagc tttaccaaac 420

gttttgaaca catttaacaa gaaattgatt gactatacca aagaagtttt caaagacact 480

aatatcaatt tgatgactaa taccatgatc aaaaaagtca atgataaaag tttgattgca 540

aaccataaaa accctgacgg atctactgag tctattgaaa ttccatatgg tcttttaatt 600

tgggctactg gtaatgcacc aagagatttc actcgt 636

<210> 271
<211> 666
<212> DNA
<213> Candida albicans

<400> 271
ggtacgaaca gacaaacacc tgaagaaact gacattggta tgattgccca ttattttgaa 60

aaataccagt ttgacgggtt aattattgtt ggaggttttg aagcatttgt ttcgttagag 120

caattggaaa gatcaagagc tatgtatcca tcgttcagaa ttcctatggt tttaatccct 180

gccaccattt caaataatgt tcctggtacc gaatattctt taggggctga tacctgtttg 240

aattcgttaa tggaatattg tgacattgtc aagcaatcag cttcagctac cagaggtaca 300

gcatttatta ttgatgttca aggaggtaat tccggataca ttgccacatt tgcctcatta 360

atcagtggag cacaagcatc ctatgttcca gaagaaggta tttcattaca gcaattggaa 420

atggatatca attcattgag agaagcattt gccgtggaac aaggaatgac aaagagtggt 480

aaattgatca tcaagtcgag taatgcatcc aaagtactaa ccccacacac attggctgac 540

atattcaacg atgaatgtca cggtgacttt gacactaaga cagctattcc gggacacgtc 600

caacaaggtg gattaccttc accaatagat agaagcagag gtgatagatt tgccattaga 660

gctgtt                                                              666

<210>  272
<211>  588
<212>  DNA
<213>  Candida albicans

<400>  272
ttagccaagt ttgaatcgtc caccccacca gttgaagttg ttggtaacaa attttatttt      60

tccaataatg ggtctcagtt tttaatcagg ggtatcgcct atcagcaaga tgccgcgggc     120

tcagtttcct ccggttacga cgccgatcct aatagaaaat acaatgatcc tttagccgat     180

gctgacgctt gtaaacgtga cgtcaagtat ttcaaagaat caaacaccaa tactttgaga     240

gtttatgcta ttgacccaga taaggatcat gaagagtgta tgaaaatttt cagtgacgct     300

ggtatttaca ttgttgctga tttatcagaa ccaactgtat cgattaacag aaacaaccca     360

gaatggaact tggatttata caaacgttat acaaaagtca ttgataagat gcaagaatat     420

tctaatgttt tgggattttt tgctggtaac gaagtaacta ataatcgttc aaataccgat     480

gcttctgcat ttgttaaggc tgccattaga gatatgaaga aatacatcaa ggagtctgat     540

tatagacaaa ttcctgttgg ttattcatcc aatgatgacg aagaaatt                  588

<210>  273
<211>  609
<212>  DNA
<213>  Candida albicans

<400>  273
tcaatcttgg ctgctacttc attcgtttct tccgtggctg ccgaagattt gcctgctatt      60

gaaattgttg gtaacaaatt cttctactcc aacaatggat cccaatttta catcaaaggt     120

attgcttacc aacaaaataa cttggactcc aacgaatcat ttgttgaccc attagctaat     180

cctgagcact gtaaaagaga tattccatac ttggaagctg tcgactacga ctccaatgtc     240

atcagagttt atgctttaga caccagtcaa gaccatactg aatgtatgca aatgttgcaa     300

gatgccggta tttatgtcat tgccgatttg tcccaaccag atgaatccat caacagagac     360

gacccatcct gggatttgga tctttttgaa agatacactt ctgttgtcga tttgttccac     420

aactacacta acattttagg tttctttgcc ggtaatgaag tcaccaacaa gaaatcaaac     480

actgacgctt ctgctttcgt taaggctgct atcagagata ccaaagccta catcaaaagc     540

aaaggttaca gaagtattcc agtcggttac tctgccaatg atgattccgc catcagagtt     600

tcattagcc                                                           609

<210>  274
<211>  684
<212>  DNA
<213>  Candida albicans

<400>  274
attgggtatc aacaccattc gtatttattc aataaatgca cacctaaacc acgataaatg      60

catgaccatg ttggccaaag caggaatata cttgtttcta gacgtaaact cgccattgcc     120

acaccaccac ctaaaccgat acgagccgtg gaattcgtac aacttgtact actttgaaaa     180

tgtctttaag gtggtagaac agttttccca ctacaacaac acgctagggt ttattgccgg     240

gaacgaaatt gtcaacgacc ccatctccgc cagtgtggct gccccatatg tcaaagcggt     300

ggtccgcgaa atcaaaagct atatcgaata caatgcacca agaaccatcc ccgtcggtta     360

ttcagcggcc gacgacttga actatcgaat gccactagca cagtacctcg agtgtggcga     420

cgacaacccc aaagaatcag tcgactttta tggcgtcaac tcgtaccagt ggtgtggcga     480

ccagacattc tacagcagcg ggtacaacat cttggtcaac gattacaaac atttcaccaa     540

accaatgttt ttttcggaat atgggtgcaa tgaggtgttg ccgagaaatt tcgatgaagt     600

cccagtattg tacacaaacg atatgataga tgttttcagt ggcggattgg tatacgagtt     660

cacccaggaa ccaaacaact atgg                                            684


<210>  275
<211>  532
<212>  DNA
<213>  Candida albicans

<400>  275
attagctgaa catgccagag accacacatt gagattcggt agcaaatcgc catttttcag      60

aaaatacttt ggaaatgaca ctgcaagtgc tgaggtcgtt ggtcattttg aaaatgttgt     120

cggtgctgac aaatcatcca ttttgtttct ttgtgatgac ttagatgata agtgcaaaaa     180

tgatggctgg gctggctatt ggagaggttc caaccatagt gatcaaacta ttatttgtga     240

cttatctttt gttaccagaa gatacttatc ccaactatgc tccggtggat ataccgtctc     300

gaaatctaag acaaacattt tttgggcagg tgacttgtta cacagattct ggcacttgaa     360

atcgattggt caacttgtta ttgaacatta cgctgacact tatgaggagg ttcttgaatt     420

ggctcaagaa aattcaactt atgctgtaag aaactcaaac tcattgattt attatgcttt     480

ggatgtgtat gcatatgatg tgacaattcc cggcgaaggg tgcaatggag at             532


<210>  276
<211>  506
<212>  DNA
<213>  Candida albicans

<400>  276
gatttacacg cctcacaaat tcaagggttt ttcgatgttc cagtagataa cttgtacgct      60

gaacctagtg tggttagata catcaaggaa actattgatt atagtgaagc tataattata     120

tcttctgatg ctggtggtgc caagagagct gctggattgg ccgatagact tgatttgaat     180

tttgaattga ttcataaaga aagagccaga gctaatgaag tatctcgaat ggttttagtt 240

gttgatgtca ccgataagat ttgtgttatt gttgatgata tggcggatac ttgtggtact 300

ttggctaaag ctgccgaagt attgttagat aataatgcta aagatgtcat tgccattgtc 360

actcatggta tattatctgg gaacgcaata aaaaatatca acaattctaa attgaaaaaa 420

gttgtatgta ccaacaccgt tccatttgaa gacaaattga aactttgtct taaattggat 480

acaattgata tttctgctgt tattgc 506

<210> 277
<211> 606
<212> DNA
<213> Candida albicans

<400> 277
taccacgata gctccatttc ccttagtggt tccaagaaca agagagaagc tgaaattgtc 60

aatgaagatg gtacaattga aaagagaact tttggaagcg ctggtgtaaa tgccggtttc 120

aatgccgcat ttgtcgtgtc taatgccaaa aaattatctg acggttctta tggtattgat 180

tgtaacttca agagtgattc ttctgtccaa ttgaacctgg cctttggtaa aaaagttaaa 240

caattgagta tcaccggtac tggttattct gatatttcat tattaggaaa tgttgctaat 300

ccatttgaat ggtcagcttc cttgaaagtc aaagcagaaa ttgttaaagg aaaatgttgt 360

cttccatcag gtttcagaat cgttacagat ttcgaaagca actgtcctga atttgatgcc 420

atcaaacaat tttttggcag ttctcaaata atttacaaag tcaatgccgt ttctaacgca 480

attggtactt ttgatgcttc tgcattattc aatgctcaag tcaaagcctt ccctgccaag 540

agagaattag atgaatttga agaattaagt aacgatggtg ttactcacag caagagaact 600

ttgggt 606

<210> 278
<211> 625
<212> DNA
<213> Candida albicans

<400> 278
gtggtgttac tgttggtgaa actgccaccg ttgctacaac tgttaccgtt ggtgcaactg 60

tcactggtgg tgaccaaggt caagatcaag ttcaacaatc agctgctcca gaagctggtg 120

atattcaaca atcagctgtt ccagaagctg atgatatcca acaatcagct gttccagaag 180

ctgaacccac tgccgatgct gatggtggta atggtattgc aattaccgaa gtctttacca 240

ctaccattat gggtcaagag attgtttatt ccggtgttta ttacagttat ggtgaagaac 300

atacctatgg agacgttcaa gttcaaaccc tcactattgg gggtggcggc ttcccttcag 360

atgaccaata tcctacaact gaagtttctg ctgaggctag tccatctgct gttactactt 420

cttctgctgt tgctactcct gacgccaaag tcccagactc tactaaagac gcttctcaac 480

ccgctgctac tacagctagt ggctcctctt ctggtagtaa tgactttagt ggtgttaaag 540

atacccaatt tgctcaacaa atcttggatg ctcacaacaa aaaacgtgct agacatggtg 600

ttccagattt gacttgggat gctac 625

<210> 279
<211> 220
<212> DNA
<213> Candida albicans

<400> 279
aagagatgat cctcatacta ttgaagcctt gagacaacaa caacaacaac cagtctcaac 60

ttctgaaggt caacaagttg ctcaaagaat tggtgctgct gattacttgg aatgttctgc 120

taaaaccggt agaggtgtta gagaagtgtt tgaagctgct actagagctt ctttaagagt 180

taaagaaaag aaggaaaaga agaagaaatg tgttgtcttg 220

<210> 280
<211> 531
<212> DNA
<213> Candida albicans

<400> 280
taagagagat ggccgtaaag agccagtacg tttcgacaaa atcactgcca gagttcaaag 60

attatgttac ggtttgaatc caaaccacgt tgaaccagtt gctattaccc aaaaagttat 120

atcaggtgtt taccaggggg ttactactat tgagttggac aacttggctg cagaaattgc 180

tgctacaatg acaacaattc acccagatta cgctgtctta gccgctagaa ttgccgtatc 240

aaatttacat aagcaaacca ccaaacagta ttccaaagtg tctaaggatt tatatgaata 300

cattaatcct aagactgggt tacactctcc tatgatttcc aaggaaacct acgacatcat 360

tatggaacac gaagatgaat taaactcagc cattgtttac gacagagatt ttaactacaa 420

ttattttggg ttcaagactt tggaaagatc atatttgtta cgtatcaacg gtaaggttgc 480

tgaaagacca caacatttga tcatgagggt tgctgtcggt attcacggta a 531

<210> 281
<211> 453
<212> DNA
<213> Candida albicans

<400> 281
ttttggacct caaatggacc agtatttgag agaaaaacta ttaagtgatg tggaaggtac 60

atgtacaggt caatttggtt acattgtgtg tgttttggat tcaatgaata tagatgttgg 120

caagggaaga ataattccaa gtactgggat ggctgaattt gaagtcaaat atagagctgt 180

tgtgtggaaa ccattcaaag gtgaagtggt agatgcagtt gtaacaaccg tcaataaaat 240

gggatttttc gccgatgttg gcccattatc agtgtttgtt agtacccatt tgataccttc 300

agatatgaaa tttaatcctt cagcaaaccc accagcatat gtgagtcccg atgaaaacat 360

tgaaaaggga tcgagggtta gattgaagat tgttggtaca agaactgatg tcaatgagat 420

ttacgccata ggaagcataa aagaagacta ttt 453

<210> 282
<211> 525
<212> DNA
<213> Candida albicans

<400> 282
ccaagaactt accattattg aacaaccact tcagaaagca ctggcaagaa agagtcagag 60

ttcactttga ccaagctggt aaaaaagctt caagaagaca atctagattg agaaaagctg 120

ccaagattgc cccaagacca atcgatgctt taagaccagt cgtcagagct ccaactgtca 180

aatacaacag aaaagtcaga gccggtagag gtttcacttt ggccgaattg aaagccgttg 240

gtattgctcc aaaatacgcc agaaccattg gtatctcagt tgaccacaga agacaaaaca 300

aatctcaaga aacttttgat gctaacgtcg ccagattaca agaatacaaa tctaaattag 360

ttatctttga caaaaagacc aaggcttctg aagttgcttc tttcgaacaa gttgatgtct 420

ctgccacctt cccagttgaa caaccagctc cagaatctgg tttgagagct gttgaagttc 480

cagaacaaac tgcttacaga accttgagat tggctagaaa cgaaa 525

<210> 283
<211> 400
<212> DNA
<213> Candida albicans

<400> 283
ttaaaggatt caaaaagggt gtccttaggg ccccacagac aatgcgtcag aaattcaaca 60

tgggagaaat cacccaagat gctgtttatc tcgatgctga aagaagattc aaagaaatcg 120

aaacggaaac aaaaaagttg agtgaagaat ccaagaaata tttcaatgct gtcaatggga 180

tgttagatga acaaattgat tttgccaaag ccgtggctga gatttataaa ccaatcagtg 240

gtagattatc ggaccccagt gctacggtac cagaagataa cccacaaggt attgaagcat 300

cggaactgta ccaagcagtg gttaaagatc tcaaagatac cttaaaaccc gatttggaat 360

tgattgaaaa aagaattgtt gaaccagcac aagaattatt 400

<210> 284
<211> 522
<212> DNA
<213> Candida albicans

<400> 284
catggcacca gaaagaacca ccaattataa cacccatcgt ttaatcaacc aattaattga 60

tatgaatcaa tatgagtcaa ttgaaatcaa tgggacaaca gtgacaaaat caaactgtaa 120

atatttacct acattggctg gggatatttg gtcattggga gtattgttca ttaatatcac 180

ttgttcaaga aacccatggc ccattgcatc atttgataat aatcaaaata atgaagtgtt 240

taagaattat atgttgaata ataacaaggc tgttttgagc aaaatcttac ccatttcctc 300

acaatttaat cgcttattag atagaatttt caaattgaat cctaatgata gaatagattt 360

accaacttta tacaaagaag ttattcgttg tgatttcttc aaagatgatc attactacta 420

tgcccaacat caacatcatc acaatcacaa tcaaatcaat aatgcttaca atcactatca 480

gaaacaacct aatcaagcaa gacctactgc aaaccaacaa tt 522

<210> 285
<211> 500
<212> DNA
<213> Candida albicans

<400> 285
tataatgccc cgaaaataaa gtttaccgat actgaaggac aagaagaaca tttttatttc 60

aatcggagta acaattcaac caatgattta accagtcatg actcttcatc aactcaacta 120

caagatgcca attccagaag acaagcccca ccaccaccac cacataatcc attttctgac 180

aattcccatg aaaatagtac tgaatcatta tatcaatcag aaacaagatt tcatcaacca 240

ctacttcata atgatagtaa taatagcaat agcagtatag gcaataatag acaacgtatt 300

ccatcacaac aacatgatac actgtcatta tattcagcat caccaatatc aacatcacct 360

ttagtttcta attttcaatc atatctggac aaccaagacg aaatgactcg aggtaagtat 420

aaccagaata caaatcggtc aagttcaaat tatattcaac acagtccaac atcagcaggg 480

tacgatagat atccgcttaa 500

<210> 286
<211> 279
<212> DNA
<213> Candida albicans

<400> 286
tggaacctgt ttgtacttga cgtcattgtc gaaaaaacac ccagagaaat tgtgtaaaga 60

gaaatacgtc cacggcggta acgtgttgat cgacccaact gccaagatcc acccatctgc 120

cttaatcggt ccaaacgtca ccatcggtcc aaacgttgtt gtcggtgaag gtgctagaat 180

ccgaagatca gtgttgttgg ccaactccca agtcaaagac cacgcctggg tcaaatctac 240

cattgttggt tggaactcca gaattggaaa gtgggctag 279

<210> 287
<211> 597
<212> DNA
<213> Candida albicans

<400>  287
gatttcctag ccggaatgca cgacaatcct gagacggaag tcgatcgtcg atgcccatgg      60

tgcgtggtga aaaatttctt tagaaaattt gttctttcct tcaactgctt ttaagaaaga     120

gaggttcaag tggtttaagt acgacggtca caaagattgc ggcttatgag gcccgaactg     180

agttgaaata caaaatcaag atataattat ataccttact tgtccatatt gttttataat     240

acattcttca gatatttaaa tttctgtgta tcaacctata aaacagagat acattcagtg     300

catttagtat actgagtgaa ctggtacctg tgacattcaa gataactgtt tcgcgcacgc     360

tggcagacga acagattaga agcttggtaa agttctgctt tgctcaatag gtttcagatt     420

cagaaagatt gttaaaactt agatcatctt cgttcatcac aaaccaagaa ctttacggaa     480

tgtacgaata tcactttcat tagtagataa ttcgttactt aatccagtga ttaatcttga     540

ggttcgaaag atggttaata gaaatttatt tgacaattac gactaaggtt acataat        597


<210>  288
<211>  350
<212>  DNA
<213>  Candida albicans

<400>  288
aagacgactg agcgtgtccc ttttgtataa actttataat tttcaatgaa tcttttaccc      60

cattggtttc aacaccgcca ctaacatcgt agcccaaaat gttgtcaaat gtaggcaaat     120

tctcaggggt tagcccacca gcaagtatag cttttgtagg taacttctca ataaacgtcc     180

aatcaagtaa cttcccttca cccccaactt ccgaatcaag caacggcaaa ctcacacatt     240

gcgttaacag caagctctgc tcttccaaaa ggtctagctc gtcaggaaca acatacctgg     300

gaattaaccc aaattctgta cccaaaaact ctagcttatc ttccagtcca                350


<210>  289
<211>  330
<212>  DNA
<213>  Candida albicans

<400>  289
acatgtcaag aggattgttc atgtaagaat aatgaagccc ccacaacaaa gacaactgcc      60

accacaacta atgttggtga tggccctggc cctggcccta tccctggcaa taatgatgat     120

gatgatgatg acatttggtc agatgatgat acgaaactaa tacctgaaaa tgatataata     180

cgatcacatt ataaaaaagg gtatgttgat gggataactc aagctaaaga atcttcatta     240

caacaaggat ttgatgatgg atatcctgaa ggtgcaaaat tagggattaa agttggtgaa     300

attttagcaa atttaatcaa tcaatgtaaa                                      330


<210>  290
<211>  524

<212>  DNA
<213>  Candida albicans

<400>  290
gccgaagata ctaaaccaaa gactgaagaa tcatcttcta ttccaaaacc accaacttct      60

aatgtattct ccatgtttgg tgccaaaaaa gagaaaaaac cagaacaaga agattcagac     120

aacaagaaag aatccgataa aaaggaagaa aaagatacta gcaaatcaac tggtgatgat     180

aatgaagtag ctgaagaaga agaagctgat gtcgaattta ctccagttgt tcaattggat     240

aaaaaagttg acgttaaaac caatgaagaa gatgaagaag tcttgtataa agttagagcc     300

aaattattta gattccatgg tgattcaaaa gaatggaaag aaagaggtac tggtgatgtt     360

aaatttttaa aacataaaac tactggtaaa gttagaattt taatgagaag agataaaact     420

ttgaaaattt gtgctaatca tttgatttct gctgattatg aattgaaacc aaatattggt     480

tctgatagat cttgggttta tactgttact gctgatgttt ctga                      524


<210>  291
<211>  513
<212>  DNA
<213>  Candida albicans

<400>  291
tctgatgttg ctgtttgttc ttcaagaact ttcggtcaaa gagctgtttt gaaatttgct      60

gctcacactg gtgctactgc cattgctggt agattcactc caggtaactt taccaattat     120

atcactcgtt cattcaaaga accaagatta gttgttgtta ctgacccaag aaccgatgct     180

caagccatca aagaatcatc ttatgttaac attccagtta ttgccttgac tgacatggac     240

tctccatctg aatacgttga tgttgccatt ccatgtaaca acaaaggtaa acactctatt     300

ggtttaatct ggtggttgct tgctagagaa gtcttgagat taagaggtat tatcccagac     360

agaactaccg aatggtcagt tatgccagat ttgtacttct acagagaccc agaagaaatt     420

gaacaaaatg ccgtcgaaga agctaaaact gaagaagttg aagaagctcc agttgctgaa     480

gctgaaaccg aatggactgg tgaaactgaa gat                                  513


<210>  292
<211>  613
<212>  DNA
<213>  Candida albicans

<400>  292
tcgaccatac catccaatac ttgaatcatt ggaatttcaa accaatcaac atttaattca      60

agaatattct ttagatattg tcaatacttt atctcaattg gaatcactta cattagttaa     120

tcctgccatg attgatttac aaccagaaat tcaatggttt atgcgtccat ttttattaga      180

ttttttaatt gaattgcatt cttcatttaa attacaacca acaacattat ttttatgtct      240

taatattatt gatagatatt gtgctaaaag aattgttttc aaacgtcatt atcaattagt     300

tggttgtaca gcattatgga ttgctagtaa atatgaagat aaaaaactgc gtgtacccac 360

attaaaagaa ttaacaataa tgtgtcgtaa tgcttatgat gaagaaatgt ttgttcaaat 420

ggaaatgcat attttaagta ctttagattg gtcaattggt catccaactt tagaagattg 480

tctacaatta gccattgatc tgaataattt atctaacaac accactaatg atattgaaaa 540

caaaagtgta cgtcctaatc ggaaatcaag tatatcatca gctgtaactg ctgttgctag 600

gtttctttgt gaa 613

<210> 293
<211> 251
<212> DNA
<213> Candida albicans

<400> 293
agaaatttgg cctgatgtta attatttacc agattttaaa tcaagtttcc ctcaatggaa 60

aaagaaacct ttgagtgaag cagttccaag tttggatgct aatggaattg atcttttgga 120

tcaaatgttg gtgtatgatc caagtagaag aataagtgct aaacgagctt taattcatcc 180

ttattttaat gataatgatg atcgtgatca taacaattat aatgaagata atattgggat 240

tgacaaacac c 251

<210> 294
<211> 564
<212> DNA
<213> Candida albicans

<400> 294
aacagcaacc agaaatcaag ttaggtatga gaccattgtt gttggatttc ttaatggaag 60

ttatcactat tctcaacttg tctagatcta cattcccttt gactgtcaat ttgattgatc 120

gttattgttc aaccagaatt gtcaagaaac aacattacca gttgttggga ttgactagtc 180

tttggatcag ttgtaagaac ttggattcaa agttcaaagt tcctacattg aatgatttga 240

gaaaaatttg tgttgacagt tattacaaag aattgtttgt ggaaatggag aaacatattt 300

taaaatcatt agaatgggtc gtcaatgctc cgacatttga tgcctttatt gatttgtatt 360

caaacttgtt gatttctaac agcagtaact ttgaggttgc aaacattatc aaaaaatcat 420

ctcataaaat aaaattgttt tccaattata ttggtgaatt gttccagttt tatccaaaca 480

tttattacga ttacacatcg tcacaaattg ctttgattgc tattttaatc acggtcttga 540

cgttgaagat tcctgttgat ttaa 564

<210> 295
<211> 580
<212> DNA
<213> Candida albicans

<400>  295
gctaccactt taaccgacac cggtgtatcc tcaggattga ataataccac ttctggtggc      60

ggcagtgata gtgcaacctc cacacacaac aacaatgagg catcgaccaa accaagtaat     120

ggcagtgaaa aatcgtcacc ggagtacact acaactgccc gcggtagaga tgagtttgga     180

ttccttaatg aagccacacc aagtcaatac aaagccaatt cagattatga agacgatttc     240

ccattggatt atatcaatca gaccactcaa aattctgaag attatattac tttggatgca     300

aattatcagg caggaagtta tgcaaatatg atcgaagaca attacgattc atttttggat     360

gcaacactat ttatacctcc aagtcttggc gtacctacag gtacagctgc gactgcaaca     420

acatcaaacc aagttgcctt caacgacgaa tacttgattg aacaagccca accaataagg     480

actccactac ccccaatatc atcatcaaca atatccggat tattacaacc aaaatcagct     540

gctaaattct tttcactaca gagtgctaat ggtggagaag                           580


<210>  296
<211>  604
<212>  DNA
<213>  Candida albicans

<400>  296
tttcatcacc acctcaagtc tctgtaacat catctgaagg agtttcacat gtcaatacac      60

gtcaatattt gggtgatgtt tcaaatcaat acataacaaa tgctaaacca acaaataaaa     120

gaaaaccatt gggtggagac aatgcccctc tacaaaaaca acagcataga ccatctagac     180

caatacccat tgccagtgat aacaacaata atggtagtac cagtagcagt agcaacagta     240

gcaacaacaa taacaacgac gcaaatagac tagcatcttt ggcagttcca tctcgattac     300

cccaaaaacg acaagctact gaatcgtcga caaatttagt agagaaatta agagtaccac     360

aaccagaagt aggggaaaga agtcagtcat accataagaa atcacgttta attgattatg     420

aatggcagga tttggatgaa gaagataatg acgaccaatt aatggttagt gaatatgtta     480

acgaaatatt ttcgtactat tacgaattag aaacacgaat gttacctgat ccgcaatatc     540

ttttcaaaca aacattgtta aaaccaagaa tgagatcgat attggttgat tggcttgttg     600

aaat                                                                  604


<210>  297
<211>  735
<212>  DNA
<213>  Candida albicans

<400>  297
ccagcaaaca attcctaatc aattgtcaca gccacaacct cagcattaca atggatctaa      60

tcgtaattac acaagtgctc ctagtggtgc ccccatacct tccaattcta ccagtggacc     120

ttcacaacag ccaccactac caggtcaaca agcagtacct atcccaccac atgtatcgac     180

aatgcaacaa ccaactcctg ttcaggatac gttgaacgcc tcgagcactt ccactgtggg 240

gcaattccaa ccaccaggaa tcagaccacg agtaacaact accatgtggg aagatgaaaa 300

aactttgtgc tatcaagttg atgccaataa tgtgtcggtt gtcagaagag cagataataa 360

tatgatcaac ggaaccaaat tgctcaatgt ggcccaaatg acacgtggta gaagagatgg 420

gattttgaaa tcagaaaagg tgagacacgt tgtgaaaatc ggatcaatgc atttgaaagg 480

agtctggatt ccatttgaaa gagcattggc catggctcaa cgtgaacaaa ttgtggatat 540

gttgtatcct ttgtttgtca gagatattaa acgagtgatt caaaccggag taactcctaa 600

tgcagctgct gcaacggccg ccgccgctgc cactgccact tctgcttcgg ctcctccacc 660

tccacctcca cccgttgctg ctgctactac tactgctgct actgctattt ccaaaagttc 720

tagcggtaat gggaa 735

<210> 298
<211> 563
<212> DNA
<213> Candida albicans

<400> 298
gctcgtttga ttagatttgg gatctttgcc cttgttttaa taggatgtgg ctatatcctt 60

acaagaggct catcattcca acctccaaat tatcaacaaa cacaatcacc cgccgctcat 120

gaaaaacaga ccggtaatgt tgctgctgga ggtggtgctg gttcaggttc cgcaggagct 180

caagttccat taggcaaaaa tagaggtcca ataccaaaag caattatggg agctggtgaa 240

ggtggtagtg atgctccggt tcctcaacaa gatattcctg atagttatac cctcaatgac 300

aaaattaagg ctacatttgt cactttggcc cgtaactctg atttatattc tttagctgaa 360

tcaattagac acgttgaaga tcgtttcaat aagaaattcc attatgattg ggttttcctc 420

aatgatgaag aattcaatga tgaatttaaa gaaactgttg gtagtttagt tagtggtaac 480

actaaatttg gtttgattcc aaaggaacat tggtcatatc ctccatggat tgatcaagaa 540

aaagctgctt tagtccgtga aca 563

<210> 299
<211> 554
<212> DNA
<213> Candida albicans

<400> 299
cccaactaat tcagcatcac ttaaacagaa acaacgtcaa cagctaggaa ttaaatccga 60

gattggtgct tcaacatcag acgtatatga tccccaagtt gctagttatt tgagtgctgg 120

tgattcacct agccaatttg ccaacactgc ccttcatcat agtaatagtg ttggttattc 180

tgctagtgca gctgcagctg ctgcggaatt acaacaccgt gcagaattac aaagaaggca 240

acaacaattg caacaacaag aattacaaca tcaacaggaa cagttacaac aatatcgaca 300

ggctcaagca caggctcaag cccaggcgca agctcaaaga gaacaccaac agttacagca 360

tgcttatcaa cagcaacaac agctacacca attgggtcaa ctttctcaac agttggcaca 420

accacatttg tcacaacatg agcatgtcag agatgcgctc actacggatg aatttgatac 480

taatgaagat cttcgttcac gatacattga gaatgagatt gtaaagacat ttaacagtaa 540

agccgaattg gtac 554

<210> 300
<211> 503
<212> DNA
<213> Candida albicans

<400> 300
aacagcaagc tgctcagttg cagcaacaaa tgcaacagca attgcaagcc agtgggttgc 60

caacaacacc aaactattct gaattgttag gtcaattagg ccagttgtct caacaacaat 120

cacagcaaca gcagcttcat catatacctc aacaacgtca acgaacccag agtcaacaac 180

tgcaacagca acctcaacaa actgcacatg gattggatca accagatgct gcagttattg 240

ctgcaattga agctagtgca gcagcagctg ttgcgtctca aggatcacct aatgtcactg 300

cagctgctgt agccgcatta caacacacac agggtaatga gcacgatgct caacaacaac 360

aagatcgtgg tggtaataac ggtggtgcta ttgattcaaa tgtcgatcca agtcttgacc 420

caaacgttga ccctaatgtt caagctcatg atcattctca tggattaaga aattcgtatg 480

ggaaaagaag tgggtttttg taa 503

<210> 301
<211> 724
<212> DNA
<213> Candida albicans

<400> 301
gtcctttcaa gtgtttgtgg agcaactgta acattatttt cgagactcca gaaattttgt 60

acgatcattt gtgtgacgac catgttggta gaaagtcttc gaacaatttg tcattgactt 120

gtctttggga aaattgtggc acaactacag ttaagagaga tcacattact tctcacttga 180

gagtccatgt cccattgaag cctttccatt gtgacttgtg tcccaaatcg ttcaagagac 240

ctcaagattt gaagaaacat tccaagactc acgctgaaga ccatccaaag aagttaaaaa 300

aggcacaaag agagttgatg aaacaacaac aaaaagaggc caagcaacaa cagaaattgg 360

ccaacaagcg agcaaactcg atgaatgcaa ctaccgcatc cgatttgcaa ttgaactact 420

attccggtaa ccctgctgat ggattgaact acgacgacac ctccagaaaa agaagatacg 480

aaaacaattc tcaacacaac atgtatgtgg ttaatagtat tttgaacgat ttcaacttcc 540

aacaaatggc acaagctcca cagcaaccag gcgttgttgg aacccgcagg ttctggctga 600

gttcacccac caagaggatg aaagccggca ctgagtataa cattgatgtg tttaacaagt 660

tgaatcattt ggacgaccac ttgcaccacc accaccctca acagcaacac ccacaacaac 720

aata 724

<210> 302
<211> 543
<212> DNA
<213> Candida albicans

<400> 302
ataacccaca taaggtctgg ttaccaggag aagaaatctc aggacaagtt gtattaattt 60

cgaaaaagaa tttggcaaat atagtcataa cgttgtcgtt ggtggggttt attaaaataa 120

atgcatcgtc acatctgaag ttgaggcctt tgaagcatac gttatttgat tatactatta 180

aaatctatgg taaagatgaa gaagaacaaa cagactcagc agagtttagt aatggacttt 240

tgaaaggcga acatgtgttt ccgtttattg taaagttgcc caataaaaga gtatatacgt 300

cgattgattt tgggaaaggt tccatcaact acattttgaa agcagctata ggaaactcgt 360

cgtcctatgt gatacctgcc tcgcccgaca atgccagtac tagcagttta acgaaaaaga 420

aaatactaca gaatcctagt cacacatcag aaaaagtcat aagtctagta aatccaatag 480

atgtttcgtt attgcctcga ccgaaaccaa agagattgat tctcaaagat ccacgaacta 540

gct 543

<210> 303
<211> 315
<212> DNA
<213> Candida albicans

<400> 303
tgactacgat gactactgaa gaaatattgg cttcttatcc acaaatcacc gctccaaccg 60

atcaaacagg ttacacatca aatttaacac ctgaacaaaa aaccacttta gatatattca 120

gacaacaatt aactgaattg ggttataaag acagattaga tgatgcatca cttttaagat 180

ttcttagagc aagaaaattt gatattcaaa aagctattga tatgtttgta gcttgtgaaa 240

aatggagaga agattttggt gttaatacca ttttaaaaga tttccattat gaagaaaaac 300

ccattgttgc taaaa 315

<210> 304
<211> 230
<212> DNA
<213> Candida albicans

<400> 304
attggtttca aacagttact cagcacgcca atgaggatgc acagatattt ttagtaggta 60

acaagtgtga tgatgaagta aacagacaag tttctaaaga gcaaggtcaa gaattagctg 120

ctaaattaaa tgttccattt ttggaagcca gtgccaaaag caatgaaaac gttgactcta    180

ttttttacga attggctagt attatccaag agaagcatgt tgaagagaat    230

<210>  305
<211>  575
<212>  DNA
<213>  Candida albicans

<400>  305
aaagagctaa ccacgtcaag gaaatcccac cattcttgca agatttagac attgccaaag     60

ccaaccccga gttcaagaaa cagcacctcg aatactatgt gttgtacaac ccagcgttct    120

ccaaagactt ggatattgac atggtccact ccttagacca ctcgtcagtt gtttgctgcg    180

tgagattttc cagagacggc aagttcatcg ccaccggttg caacaaaacc acccaagtgt    240

tcaatgtcac caccggagag ttggtcgcca aattgattga cgagtcctcc aacgaaaaca    300

aagacgacaa caccaccgcc tcaggcgact tgtacatcag atctgtgtgt ttctcccctg    360

acggaaaact cttggcgaca ggtgcagaag acaagttgat tagaatctgg gatttgagca    420

caaagagaat tatcaaaatc ttgaggggcc acgaacaaga catttactcg ttagactttt    480

tccctgatgg cgataggttg gtttcaggct ccggcgatag gtcagtcaga atctgggact    540

tgagaacctc ccagtgttcc ttgactttgt cgatc                               575

<210>  306
<211>  286
<212>  DNA
<213>  Candida albicans

<400>  306
aggtggtgtc atgaaattat tagttggtaa taaggctgat ttgtctgata aaaaaatcgt     60

cgaatatact gctgctaaag aatttgctga tgccttggac attccatttt tagaaacctc    120

cgctttatca tcgaccaatg ttgaacaagc tttttacact atggcaagac aaatcaaagc    180

ccaaatgaca aacaatgcca atgccggaaa tgctgccaat gccaagggca aatctaatgt    240

gaatttgaga ggtgaatctt tgacttctaa ccaatcgaat tcctgt                   286

<210>  307
<211>  558
<212>  DNA
<213>  Candida albicans

<400>  307
ttgcccaatc agcattacaa tttgcaacaa agacaacagg cacaaggaca acaactcaaa     60

ctgcaactaa acgagcaaaa tgccatgatg tctgcctcga ctcaacaata tcctgtccag    120

gattttacaa atccttaccc caatgcacag aatcccgcag aacaacagca acagcaacaa    180

cctcttcgaa cccagtcaca acaatgggac ggctaccaat ctcaaccttt gtattctgct    240

gctggtaata ctataccatc ctcaatccag cagcaaatac caccacagaa tttgtctcca 300

tcagagcagc aacaagtcaa gcaacaacag ccactgccgc cagaacaagg aacaaagaaa 360

aaacctggta gaaaaccaaa attaagaaaa ttatcggaac tgagttctga aacaccacaa 420

gttccaaaaa cagcatccag ttcttcgagc tcaccaactg cagtcaattc tggtaaacca 480

attacaaaaa gatcgcgtat gggatgtctt acatgccgtc aaagaaagaa acgttgttgt 540

gaaacaagac caaggtgt 558

<210> 308
<211> 450
<212> DNA
<213> Enterococcus faecalis

<400> 308
atatcgaagt ggtctattta gaggacttag ctgctgaagc gttgattaat gaagaggtcc 60

gccgacaatt tattgaccaa ttcttagaag aagccaatat tcgcagcgaa tcagcaaaag 120

aaaaagttag agagttaatg ttagaaattg acgacaacga agaacttatt caaaaagcga 180

ttgctggcat tcaaaaacaa gaattaccta aatatgagca agaattttta acagatatgg 240

ttgaagcgga ttatccattc attattgatc caatgcctaa cttatacttc acgcgtgata 300

actttgcgac aatggggcac gggatttctt taaatcatat gtattcagta actcgacaac 360

gggaaaccat ttttgggcaa tacatttttg attatcatcc tcgttttgct ggaaaagagg 420

ttcctagagt ctatgatcgt tcagaatcaa 450

<210> 309
<211> 280
<212> DNA
<213> Enterococcus faecalis

<400> 309
aattaaacaa agcaggaatc aagaaacaag tggctactgt tttaacacag gtggtcgtag 60

atccagcaga tgaggcattc aaaaatccaa caaaaccgat cggtccattt ttaacagaag 120

ctgaagccaa agaagcaatg caagcaggtg ctatttttaa agaagatgca ggacgtggct 180

ggcgcaaagt cgttccaagt cctaagccaa ttgacatcca cgaggctgag actattaata 240

ccttaataaa aaatgatata attaccattt catgtggtgg 280

<210> 310
<211> 600
<212> DNA
<213> Enterococcus faecalis

<400> 310
agttgcacaa gtagcgatgg cgatggcttt taatcctcaa aaagattatt ttttaccgta 60

ttatcgtgat atgaccgcgt gcttggtttg gggcatgacc tccaaagata ttttaatggg 120

ttcttttgga aaagaagcgg atccttcttc ccatggtcgt caaatgccga atcattatgg 180

ttcaaaagag cataatattg tttccttctc ttcaacagta agtacacaaa tgccattagc 240

aacaggtgtt ggttatgcag cgcaacttca aaaagctgat tttgttgcat tgaccaccac 300

tggggaaggc tctgccaatc aaggagaagt ccaagaagct attaactttg caggcgtaaa 360

aaaattacca gtcatttttg ttgttgaaaa taatgaatat gcgatttctg tcccaattga 420

agaacagtat gccaataaac gaatggccga tcgcgcgaaa gcttatggct ttgaaggtgt 480

gaccgttgat ggtagtgatt ttgctgaagt ctatctagca tttaaagaag cagtaaaagc 540

ggctcgcggg aaaaaaggac caaaattgat tgaattaatg gtttctcgct tgacttctca 600

<210> 311
<211> 528
<212> DNA
<213> Enterococcus faecalis

<400> 311
cgcagacaag aaagacaaca caacgaactc ttctagcgta gcatcttcag aaacgaaaaa 60

atcaactgaa tcatcagcac cagcgaaaaa agttgccggt ggcgatttaa aagatggtac 120

gtataaatta gaagaaaaaa atgaaaaaaa tggttaccgt gcagtctttg aaatgactgt 180

aaaagacggc aaaatcactg aatctaaata tgacaacatc aatgctgacg gcaaatctaa 240

aacagaagac actaagtatg aagaaagcat gaaagcaaaa tctggtgttg gaccaaaaga 300

atacatcaaa caattaaacg attcttttgt taaagcacaa agcgcaagcg gtgtggaagt 360

agtaactggt gcgactcatt catctgaatc attccaaaac tacgcacaac aattaatcca 420

agcagcacaa gctggtaaca cagacacaat cgaaatcgac aatggggcaa cattgaaaga 480

tggtacgtac tcattgaaag aaaaaaatga ctcaaacggc taccacac 528

<210> 312
<211> 451
<212> DNA
<213> Enterococcus faecalis

<400> 312
ttttcacttt taggagctat ttttatttta gctagttgtg gcataggaaa agatgctgtc 60

acagatacta agtacaaagt tagtttgcag caagctgctg aaatctatga aaaagaagct 120

ggcaacagca aaccattagt aaatgtccaa tttgatacag aaccagcaag tgactacagc 180

tatatcttta ctaacgatac agaaacactt tacgtgaatc ctgaaacagg aaaagtcacc 240

aaaaatactg aagcaaatca acttggcgaa aacgagacag ccttttcagc tgctgaagtc 300

aaagaattag gcgctgttaa cgacgtttta gccaaagcaa aaaaagaagt tggaggactt 360

tctccacgta ttttgacttg gaagttaacc aaaaataaca ataaacttgt ttatacagta 420

gatgttaaaa cgactacggc agatgaaaaa g 451

<210> 313
<211> 274
<212> DNA
<213> Enterococcus faecalis

<400> 313
caaaaccaac agaagaagaa ttaaaacaaa ccttgacgga tcttcaatat gccgtcacac 60

aagaaaacgc aacagaacgc cctttttcag gagaatatga tgacttttac caagacggaa 120

tctatgtaga cattgttagt ggcgagccgt tgtttagctc cctggacaaa tacgatgctg 180

gttgtggctg gccatccttt accaaaccaa ttgaaaaacg tggcgtcaaa gaaaaagctg 240

attttagtca cggcatgcac cgagtagaag ttcg 274

<210> 314
<211> 564
<212> DNA
<213> Enterococcus faecalis

<400> 314
ggcttagttg tcagttgtgg ggcctttttt gcccaaccta ctgtgactca cgcagaagaa 60

gatattaccg cgattgctaa aaaaatgggg acgactttga aagcggatgg cattcccaaa 120

gcagccatcg ttgttgatgc tgattctgga gaaattctct ggtcgcagca accagattta 180

gcgtggaatc ctgccagtat tgccaaagtg atgaccatgt acttggcctt tgaagcaatg 240

gagcaaggaa aatttacaat ggatacgact gtgactgcta cgcaaaaaga tgtcgatatt 300

tctaaaatat atgccattag taataacaaa attacgttag gtgttgctta tccagtccgt 360

gaactgttaa aaatgattgc tgtcccctct tctaatgtgg cgactctcat gttggcaaac 420

ttaatttcag ggaaccagcc tactgacttt gttcatttaa tgaatcaaaa agcggctgaa 480

ctagggatga caaatactac ctattacaac tgcagtggag cgcaagcaag tgcctttaac 540

ggcctgtatc aaatgcaagg aatt 564

<210> 315
<211> 478
<212> DNA
<213> Enterococcus faecalis

<400> 315
gtttgattgt tgcgaggtca aagaataatg ttataggcaa gaatggtaat ataccatgga 60

aaataaaggg agaacaaaag caatttagag agttaacaac gggtaatgtg gttattatgg 120

ggcgaaagtc ttatgaagaa atcggtcatc cgttgcctaa tagaatgaat attgttgttt 180

ccaccacaac agagtatcaa ggagataatt tagtttcagt taaatcatta gaagatgcat 240

tattattggc taaaggacga gatgtataca tatctggtgg atatggacta tttaaggaag 300

ctttgcaaat agtagataaa atgtatatca cagaagtaga tttaaatatt gaagatggag 360

atacattctt tccagaattt gatatcaatg attttgaagt tttgataggg gaaacacttg 420

gtgaggaagt gaaatatacg agaacatttt atgtaaggaa aaatgaattg agtagatt 478

<210> 316
<211> 380
<212> DNA
<213> Enterococcus faecalis

<400> 316
ttttactaaa ccattaggtg taaaattacc cccatttttt gatattgcac attttgacgc 60

aatggctgaa attttaaata aattcccttt agtttacgtg aatagtatta atagcatcgg 120

taatggttta tatattgaca gtgacaagga agaagtggtc attaaaccaa aaggaggctt 180

cggtggactg ggcggcgaat atgtcaaacc aacagcgtta gccaatgttc gtgcgtttgc 240

gcaacgtttg aaaccagaaa tcaaaattat tggaacgggc ggtattacat gtggaaaaga 300

tgtttttgag catcttttat gtggtgcgac attagtacaa gttggcacac aattgcatca 360

agaaggtcca caagtttttg 380

<210> 317
<211> 537
<212> DNA
<213> Enterococcus faecalis

<400> 317
catgtattgg ttgttagata gggagtatga aaacttatat aatagtactt ataaagaaag 60

tgcgcattta agagtgcaaa ttgcagacga tttgtcaaat ttaccattat cctatttttc 120

aaaacataat ttatcagatt tatctcaaac tatcatgtct gacgttgaag gtattgagca 180

tgcgatgagt catgcaatac ctaaatccgg tggtatggct ctgtttttcc cttttatttc 240

agtgatgctt ttggttggta atgtcaaaat gggattagct gttattttgc caacgttatt 300

tagttttgtc ttaatcttgt tatcaaagaa atcccaaacg aaagccaata ctaaatatta 360

cgatactttg agagaaaact cggaagaatt tcaagaaact attgaattgc agcaagagat 420

taatagcttt aatctatcta aaaaagttca agacagactt ttcaaaaaaa tggaagagag 480

tgaaaggatt catttaaagg tagaattaag tactttttca gtcatggcct tatcctc 537

<210> 318
<211> 606
<212> DNA
<213> Enterococcus faecalis

<400> 318
gatcaggaag atcaatcagg aaaaacacaa tggacaaagt attatctaac cgtttatttt 60

tctggcttat ttaattttct gatgattctg attttatcag ttttatttgg gacgttaagc 120

gaaaccttta ttgtatacgt cgtactgatt tttttacggc ctgtcgcagg tggctggcat 180

gcaaaaacta aatggctctg tcgtctagaa agcattgtta tctatgtcgc cataccattt 240

gtattgaaaa attcttctgt gagcttaccg tttatttata aaattctatt gatttgcctc 300

ttagtcgtat tattttattg gtatgcgcca caaggaacag caattgaacc tgttcagcca 360

tctgatttaa acgtgctcaa aaagcaaagc cttataaggg tgtgtttact tattttatgt 420

agtctgtttg tcaaagaaaa gattgcttca gtaatactct acggtctcgt catccaaggt 480

ctgatgatac tccctgtaac aaaaaattta attgaaggaa gtgtttttat gaaatttggt 540

aaaaaaataa ttaaaaatgt tattgaaaaa agagttgcaa aagtcagtga tggtgtggga 600

actaag 606

<210> 319
<211> 507
<212> DNA
<213> Enterococcus faecalis

<400> 319
gttgggctac tcttggttga tttatgcgct gacagtcgtt gtttttacag gatttttact 60

cattcacaaa aagaggttct caatttttaa agcgatattt ttatccgttt ttacattgct 120

tatggtttcg tttatcaatt acacggagca aacgatttta agtgtttttt ttcaacagat 180

ttatcaaaat aaattattat ggattgcctc aaatgttctt ctgttgctta taaatatctg 240

gattgcttta aaaattccca atagtgtttt tttaagatta aatcgtgtgt tagaaaatag 300

ccgaattttt tttggttgtt tacttttatt gttgattctg ttgttacttt ttgtgttttt 360

gatttcgcca gagatttcac ctgactttat gcgaggattt gtcacggtaa atagttctaa 420

attggagtta ttaataagtg taggtttatt tttaattctg attggcttag tcattgaagc 480

ttatttggaa gaacaacgta tcaacac 507

<210> 320
<211> 500
<212> DNA
<213> Enterococcus faecalis

<400> 320
ttacgttaga agaagcatac caagagtcaa aacggatgca agaattggtc aatttttcac 60

caaataatca attgctctat aaaacagctg ttcagctaga aggattgcct cgccatgttt 120

ctacgcacgc agcaggtgtg gtaattagtg atgaaaatct tttgaatttg gttccgttac 180

aaccaggatc gaatgaaatt ttattgaccc aatttactat gaatgatgtt gaaaaaattg 240

gtcttctgaa aatggatttc ttgggcttaa gaaatttatc catcattgat gataccctca 300

cagctgttaa acgcgtctat aatcgaacca ttcgtttaaa tcagattcca ttagatgacg 360

aaacaacgct ggctttattt agaaaagggg aaacaagtgg cgttttccag tttgaatctg 420

ctggaattcg gaatgtatta agaaaattag ggccaactag cattgaagat attgctgctg 480

tcaatgccct gtatcgtcct 500

<210> 321
<211> 407
<212> DNA
<213> Enterococcus faecalis

<400> 321
tttatgaagg cccaaagaat gatcttctgc taccttcaat tcaggctttt ttatcttaag 60

tgcctcaaga aataacgaat cggttttctt tttcattaaa aagacttttg agccttcctt 120

aaattctgag gaaaaaattt cgatgctttt ttcattgtag gttacttttt ctatctgaga 180

aagcggaagt gcttttttgc gaaaccacaa aacatctctg acaattaaag atgtttctgt 240

catattaaaa gaccgcgcaa ttcctagata agcaaacaca aaaaatggaa ccatcaccag 300

attactaatc aagtaaggac cattattttc caatgctaaa attaaactaa taaataatat 360

acaaaatgtg caagaccagt aaataattgt tgatgctaat tctggct 407

<210> 322
<211> 607
<212> DNA
<213> Enterococcus faecalis

<400> 322
tttacctcac cgaccaatcg cggcaacaat tgcatgtgcc tttaggcatt gttagtaatc 60

acgaagccga atttaaagtg ctgattgaag ctttaaaaca agcgattgcc aatgaagaca 120

atcaacaaac cgttcttctc cactcagata gtaaaattgt tgtccaaaca attgaaaaaa 180

actatgctaa aaatgaaaag taccagcctt atttagcaga atatcaacaa ctagaaaaga 240

attttccttt gctcttaatc aaatggctac ctgaaagtca aaacaaagcg gccgatatgc 300

ttgcacggca agcattacaa aaattttatc ccaataaaaa gtagcactgt ttacttaatg 360

cttttccttt attaatttga taattaaaca cgtggagcaa aaattccaag tgatttttgc 420

tccacgttta aaaacagata aacggttctg tctcgacttc ttcttatagc cacttattct 480

tttgtcgtta tttccgcaaa ttgcccattg gttagcgaaa ggattgcttc aggcgctaat 540

tcaatttgca tgccacgttt gcctgcagaa acaataattg cagaatattg ttgagcttct 600

tcagcca 607

<210> 323
<211> 521
<212> DNA
<213> Enterococcus faecalis

<400> 323
tctgtttacg ttagcggtct ttctacaagg aggcgttact gatttaaaca cgaatcaaat 60

tggacaagtg attcctaatg gcccagccgc agaagctggg ttgaaagaaa acgataaagt 120

cttatcgatt aataatcaaa aaatcaaaaa atacgaagat tttacaacca ttgtgcagaa 180

gaaccccgaa aagccgttaa cgttcgtagt tgagcgtaac ggcaaagaag agcaactaac 240

agtgacacca gaaaaacaaa aagtggaaaa acaaacaatt ggtaaagtcg gcgtttatcc 300

ttatatgaaa accgatttac cgtcaaaatt gatgggcggt attcaggata ctttaaatag 360

tacgacacag atttttaaag cactcggctc actattcaca ggctttagtt taaacaaact 420

aggtgggcca gtcatgatgt ttaaattatc ggaagaagca tccaatgctg gagtaagtac 480

agttgtattc ttaatggcca tgttgtcaat gaacttaggg a 521

<210> 324
<211> 531
<212> DNA
<213> Enterococcus faecalis

<400> 324
ggcgacgaag ttaaagtgaa taataaacaa attgtttctg gactcgatgt ttcggcagct 60

tcggttagtg agatgatttc aaagttagta aaagaagatt tggttgagca ttctccttat 120

caaggggtac aattaactga aaaaggctta aaaaaagcga gtacgttaat tcgcaaacac 180

cgaatctggg aagtcttttt agtagagcac ttaaattaca cttggaatga tgtgcacgaa 240

gaggcagaag ttttagaaca tgttacttca cagacgcttg tgaaccgttt agcggattat 300

ttaaatcatc cagaattttg tccacacggt ggtgttattc ccgaagataa tcaacccatt 360

catgaggaga aacgccaaac gttaacagac taccctgttg gcacaaaaat tcggattgca 420

cgtgtcttag acgaaaaaga attactggat tatttagttt ccattgattt aaatattcaa 480

gaagaatata cgattaaaga aattgctgca tatgaaggac cgatcaccat t 531

<210> 325
<211> 342
<212> DNA
<213> Enterococcus faecalis

<400> 325
gatacgaaga agatagcgaa acggttcaag ataaagtcac agcgctgcca agtaccggtg 60

aatttgcttc tgacaacaga aaagcaaaaa gctgtgataa ggaacagaac aagtcaaaga 120

aaatatggaa ccacgtgtag aataaacagt taaagggagg aaacaatcat gggctttatt 180

tgggcattaa ttgtcggcgg ggtcattggg gcaatcgctg gagcaattac taaaaaagga 240

tcatcaatgg cattattgca atatcattgc agggttagtt ggttcaacaa ttggtcaagc 300

catttaggca catgggacaa gcttagctgg gatggctatt gt 342

<210> 326
<211> 512

<212>  DNA
<213>  Enterococcus faecalis

<400>  326
aagatggtac gtgtattcgt tttgacactc tttggcaagc aggtttgcaa gcttgttttg      60

aaacactaag tatgttagcc cctcatcatt cagcagaaat aaaaaagata ttagctattc     120

aggagcaacg ttttttgcaa aaacatttac ttgatgaagt cctttatcag gaactttatc     180

aggaattggc gcaatttgag gaattagtcg aacagggaat cagcagtcga tggctggagc     240

aattttttta tgattattta cgaaaaaatc tgaaaaagat cgaaccaatt ggtgatttaa     300

aacagttatt tcttgagcta aaacggaaga actataaaat tggattagca acttcagata     360

ctttgccagc gactatgttg attatggaat atcttggttt aacagaaatg tttgatttta     420

ttgcgacagg agatcgttac ttaccgaaac cagatgcgga catgctccaa gccttttgtc     480

agtcatgtca attgaaggcg acagaagtaa tt                                   512


<210>  327
<211>  643
<212>  DNA
<213>  Enterococcus faecalis

<400>  327
ttatttctgt tgagggcaaa gcggaagcag gtaaatactt gttcttcaca accttaaaag      60

gaaccgtcaa acggacagcc gtaacagcct tttctaatat ccgtagtaat ggattaatcg     120

ccattagctt aaaagaagat gatgagttag ttaacgtagt aacgactaat ggcaatcaga     180

agatgattat cggaacacat gcaggatact ctgtcacatt tgatgaaaat actgtacgtg     240

atatgggccg gacagcatca ggtgttcgtg gaatccgtct ccgcgaaaat gattatgtgg     300

tcggcgcagc gattctggat gaaaataaag aagtcctagt cattactgaa aatggttatg     360

gtaagcgtac aaaagcctct gaatatccag ttaaaggacg tggcggtaaa gggattaaga     420

cagcaaatat cactgagaaa aatggtccat tagctggttt aaccacggtc aatggtgatg     480

aagatatctt attgattacg aacaaaggcg tcattatccg ctttaacgtt gattctgttt     540

ctcaaacagg acgcgcaaca ttaggggttc gtttaatgag aatggaagat ggtgccaaag     600

tggtaacaat ggctgttgta gaaccagaag aagtggaaga aga                       643


<210>  328
<211>  402
<212>  DNA
<213>  Enterococcus faecalis

<400>  328
ttgatcgttt tgacgtaatg ataaaaaaag cgaagaaaac ctaccaacgc ctagacttag      60

aagaaaaggt cactctttta gaaggacaag cagctgagat tctaccaacg ttggaaggac     120

cttatgactt tatttttatg gatagtgcca aatcaaaata cattgaattt ttacctgaat     180

gtttacggtt gctgccagtt ggcggcgttt tgatggtgga tgatgtattt caagctggga 240

caattttaga ccctgctgag gaagtaccga aaaaaaatcg agcaattcat cgtaaattaa 300

accaattttt agatgtagtc atggctcacc ctgatttaac ttctacttta gttcctcttg 360

gtgatggagt tattttaatt accaaagaga aagaaacgat ta 402

<210> 329
<211> 608
<212> DNA
<213> Enterococcus faecalis

<400> 329
agcgactaga gagcatacaa gtaaacgaac gggcgttgcc ttgtgggtgg tgacggagtt 60

agccataatg gctacagata tcgctgaggt aattggtggt gccgttgctt tgcaattatt 120

atttggtttt ccattattaa ttggtgtgtt gataacaacg tttgatgttt tattactgtt 180

gctactgaca aagttaggct ttcgcaaaat cgaagcaatt gtttcttgtt taattgcagt 240

catctttttt gtttttgctt atgaagtggc attagcagat ccaaatgttg gtgaagtatt 300

acgaggtttt attccagaca caaaaatagc gacagataaa tccatgttat ttttagcctt 360

ggggatcgtt ggagcgacag tcatgcccca taacttatat ttgcattctt ccattgcgca 420

agcacggaaa tttgatcgta acgatgatgt tgagaaagcc aaagcaattc gtttcactac 480

ttgggattca aatattcaat taactgttgc tttcgtcgta aattgtttgt tgttaatttt 540

aggaggagca ttattttatg gaaccaacag tgaattaggt aaatttgttg atttatttga 600

tgctctga 608

<210> 330
<211> 450
<212> DNA
<213> Enterococcus faecalis

<400> 330
aaattgttgc acgtatggaa aaaatgaaag acggaaattt aagtggtatc caacgacata 60

atcaacgaga aaccaataat cattccaatc ctgatattga tattgagaaa tctcacttga 120

attatgactt agtcaatcct ggttcaatca attatcggga gaaaatcaaa caaatcattg 180

agagccaacg aatcagtaaa cgagcggtta gaaaagacgc agtccttgtg aacgaatgga 240

taatcactag tgataccgcc ttttttcaag agaatacaga cacacaagca ttttttaccg 300

atgttgtcgc atatttctct gatcgctgcg gtcgacaaaa tgtcgcctat gccacggtac 360

atttagacga aaccacgccc catatgcact taggaattgt gcctatgtac gaagggcgat 420

tgagcagtaa acaggtgttt agtcggcaaa 450

<210> 331

<211>  360
<212>  DNA
<213>  Enterococcus faecalis

<400>  331
caatggaaca aaggccactc tgatgaaacg tcgtttgctg aaaatattcc agctaataat      60

tgggaaaacg aattggccat gctctttatc ttaattaatg atggcgaaaa agatgtttcc     120

agccgtgatg gaatgaaacg aacagtagaa acttctagct tttatcaagg ttggttggac     180

aatgtggaaa aagatttatc ccaagttcat gaagcaatta aaacaaaaga cttccctcgt     240

ttaggagaaa tcattgaagc caatgggtta aggatgcatg gaaccacctt aggcgctgtc     300

cctccattta cttactggtc cccaggcagc ttacaagcga tggctttagt tcgccaagca     360


<210>  332
<211>  526
<212>  DNA
<213>  Enterococcus faecalis

<400>  332
ctgcggttaa agtcgttgca ttttctaaaa gggaaatgag tcccagataa agtgaacccg      60

tatacaagtt tcctacgcga cgactataga tgatgctttc ttcataacgg gctaaaattc     120

gttcctgttc tgcttcagtt tggtcggaga tttttgctaa taaggctttt ttgcccattt     180

ttgtgtaagg aatatggaac gctaaagcat cataatctgc aaaatcaaga ccggttcttt     240

ttttatgttc atcccagact tgggcaaaag attggatgta ggtttcgttt gacaaaggac     300

catcgaccat aggatacgga tggcctgttg gacgccaaaa gtcatagata tcttgcgtca     360

gcatcacatt atcctctttt aaagccaaga tgcgcggttc actagcaact aacattgcaa     420

ccgccccagc tccttgtgta ggctcaccgc cagaatttaa tccatatttt gcaatatctg     480

ctgctacaac caagactttt ttatctggat gtaaggctac gtgatt                    526


<210>  333
<211>  512
<212>  DNA
<213>  Enterococcus faecalis

<400>  333
atccgactat gcgtttactg aagaacaagc tgaagcaatc gttactttac agctataccg      60

tttaaccaat acggatatta ctgatttaca agaagaagcg aaaactttag aacaacaaat     120

tgctgagtta ttgaacattt taaacaatga aaaagaacta ttctcagtca tgaaaaaaga     180

acttcgcgaa gttaaaaagc aatatggcaa tccgcgctta actcaaattg aagaggaaat     240

ccaagaaatc aagattgaaa cagccgtgtt agttgcgcag gaagacgtgg tcgtaaccgt     300

gacgcacgaa ggctatatca agcggagtag tattcgttct tatacagcat caaaaccaga     360

agaaatcggc atgaaagaag gcgacttttt attatatgct ggcgaagtca atacattaga     420

tcatctttta ctagtaacaa ataaagggaa tatgatctat cgccccgtcc atgagttgcc 480

agatttacgc tggaaagaaa ttggcgaaca ta 512

<210> 334
<211> 604
<212> DNA
<213> Enterococcus faecalis

<400> 334
aggatcaatc gtaaatggtg tatacaaaac attttgttat tcatacattt gataaattaa 60

acaatgcctg ctcgtatatt gagaatgcag aaaaaactga agtcacgaat gataatccgt 120

ctgaacactt ggaacattta tttcaatata ttgtgaatga cgataagaca tacatgaaaa 180

aattagtttc tggtcatggc attgtggatc caacaaatcc ttatgaagaa tttaaattaa 240

caaaattaca agcagcaatt caacgaaaaa tcgggtacac attcgatcca aaatcagaac 300

gattgcttcc gccaacgtta acagaattag aaaaaggcaa cgccgtttta gcacaccatt 360

taatccaatc attttctcca gaagatgatt taacgccaga aaaaatacat gaaatagggt 420

acaacacggt gatggaattg acaggtggaa agtatgaatt tgtgatcgcc acacatgtcg 480

acaaagaaca tttacacaat catattattt ttagttcaac caacttaaaa acaggtaaag 540

cctttcgctg gcaaaaagga accaaaagag tctttgaaca aatttcggat aagattgcag 600

cgaa 604

<210> 335
<211> 451
<212> DNA
<213> Enterococcus faecalis

<400> 335
aagatggtga aacattggtg gttacaactg cagatcattc aacaggtggc ttgtctttag 60

gcaaaggaga tcaatacaac tggttgacgg agcctttaca tgcggcaaaa cgcacgcctg 120

atttcatggc agaagaaatt attaaaaatg gtaatgtgga aaaaacagtg actgagtata 180

ttgattttca attaagtgag gctgaattga aagcagtgaa aacagcggcg gagtcaaaag 240

atgttgaaaa aatcgctcag gcattaagaa agatttttga tgaacgttcg aatactggtt 300

ggactactgg cggacacaca ggagaagatg taaatgtcta tgcttatggc ccacaagcag 360

aagctttttc aggacaaatt gataatacag accaagcgaa gattattttt ggcttagtag 420

atggcaccgg gcaaaaagct gagattaaag a 451

<210> 336
<211> 543
<212> DNA
<213> Enterococcus faecalis

<400> 336

gtttccgttc aaataaccac aaatcagaca acatttacag aggaacaatt aacggattat 60

tggcagttgg ccttgttaaa tagtcagtgc aatacaccgt tagttcagaa agtcctaaaa 120

acacagacac cacaatttga agatcggaaa attatcttac ctgttgataa tgaagcagtt 180

attccttatc tgaagcaaca atatttacca attattgagg aactttattt ctcttatggg 240

tttcctaaat ttcatattga accaaaaatg gatcaacagc aagctgcaga agtgttgaaa 300

aagtttgaag agcaaaaatt agaacaagcc gcagcctttc aacaacaagc tgctgaatcg 360

cttgttaaac atgaacaaat gaaaaaagaa aaacaacaac aagcgcctgc gtttgatggt 420

ccaattcgtt taggtcggaa tattcccaat gatgaaccca ttatgcccat gggaaatata 480

ctggaagaag aacgtcgtat aacgattgaa ggctttattt ttgataaaga agtgcgtgaa 540

ttg 543

<210> 337
<211> 578
<212> DNA
<213> Enterococcus faecalis

<400> 337
aattgcagga ggttcacaac cagagatttt acagctagtt aaaaaagcac taaaagaagc 60

cgagcaaccg ttgcagttta ttgtatttga tacaaatgaa aatcttgata ctgaaaatct 120

ctggaaatat gttcattgct cagatgaggc cgcggtagca caggaagctg tcagtttagt 180

tgcaaccggt caagcacaaa ttttattgaa aggaattatt cagacccaca cattactaaa 240

agaaatgttg aaaagtgagc atcaattaaa aaataaaccg attctttccc atgtagcaat 300

ggtggagctg cctgcgggaa aaaccttctt gttaaccgat tgtgcgatga atatcgcccc 360

cactcaagcg accctcattg aaattgttga aaatgctaaa gaagtcgccc aaaaattggg 420

actgcaccac ccgaaaattg ctttgttaag cgcagcggaa aatttcaatc ctaaaatgcc 480

ttcgtctgtt ttagcaaaag aagtcacggc acattttaat aatcaacaag aggctacggt 540

tttgggcccc ctttcgcttg atttagcgac ctctgaag 578

<210> 338
<211> 320
<212> DNA
<213> Enterococcus faecalis

<400> 338
aatgcgtgat cagggtgtat gataaaactc ttggaaagag gcagaatttt gaaagttgca 60

tatgcaagag tttcatccat tggcaaaact tggaacggca aattcaagag ttaaaaaaat 120

taggagcgaa aaaaatattt gtagagaaaa aatctggcgc aagtattgaa caacgactaa 180

tttttacaga agctatctat tttgtgagag aatccgatat ttttatggta gaagccattg 240

accgattagg cagaaattac gatgaaatta ttcagacggt taatttattg aaaaataaaa 300

atgttcgact cataattaca                                                320

<210>  339
<211>  693
<212>  DNA
<213>  Enterococcus faecalis

<400>  339
ctcaacagct tcaacaatcc attcaaattt tacaatttaa tacggaagaa ctggctgcct      60

ttgttgaagc gaaagcacta gagaatccat taattgattt acaagtagac acgcagtaca     120

ccacagattt tccgataact agtcgttctt acaccaacca agacgaagaa aataattata     180

tgaatcaaat tccagactat catttatcat tatttgagtc tttaattgat caaattcatt     240

tgaattaccg cgatacatac ttgcgaacat tggtattgtt tttagtagaa tatatagacg     300

tgaatggtta tttaaagatt tcgttagaag aagcggcaga gaaaaccgaa gcaagcgcca     360

ttcaaatgct agatgcatta actttgttac aacagctaga tccagcaggt gtgggggcac     420

gcaatttaca agaatgtttg atgctacaaa cagaacgaga cgataccgcg cctaacttag     480

cgtatatttt attggaggaa gagtttgatg ccttagtgag tcgtaaatgg ggcccgttag     540

ctaaaaaatt cgggattgaa ttagcagaaa ttcaattgat ttttgattat atacaaacgt     600

tatcgccagc gccagggaat atttttgatg cgaccgagga attgtatatt cgaccagatt     660

taactgtccg aatcaaggaa gatcgaatag tgg                                  693

<210>  340
<211>  210
<212>  DNA
<213>  Enterococcus faecalis

<400>  340
aggtttagaa gtgggggagt ttgtacacac gctaggagat gcccacttat atcaaaatca      60

tgtggaacaa atgcaagaac aattatcacg agaagttcgt tctttcccaa cgctcgtttt     120

gaatccagac aaggcttctg tttttgattt tgatatggaa gatattaaag tagaaggcta     180

tgacccacat ccaacgatta aagcgccgat                                      210

<210>  341
<211>  504
<212>  DNA
<213>  Enterococcus faecalis

<400>  341
aacgcacatc tgaaagctac gaaaaaactg tcaaccatat gaaagatgta ttgaatgaaa      60

tctcttctcg catgcgtaca cattcagttc catggcatac agcaggtaga tattggggac     120

atatgaactc agaaacatta atgccttctc tattagctta caactttgca atgctatgga     180

acgggaacaa cgttgcctat gaatcttctc cagcaacttc tcaaatggaa gaagaagtag     240

gacatgaatt tgctcacttg atgagctaca aaaatggttg gggacacatc gttgctgatg 300

gttctttagc taacttagaa ggcttatggt atgcccgtaa cattaaatca ttaccatttg 360

ctatgaaaga agtaaaacca gaattagttg ctggcaaatc agattgggaa ctattgaaca 420

tgccaacaaa agaaattatg gacttattag aatcagctga agatgaaatt gatgaaatca 480

aagctcattc agctcgttca ggta 504

<210> 342
<211> 400
<212> DNA
<213> Enterococcus faecalis

<400> 342
atggagggtg ataacatgaa tatcattgac gagctagcat ggcgtgatgc aatcaatcaa 60

caaacaaacg aagaaggact aagagaactt acagaaaata cgagcatttc gctatattgc 120

ggtgtcgatc caactggaga tagcatgcat attggacatt taattccttt tatgatgatg 180

aaacgattcc aattagcagg tcatcaccca tacattttaa ttggtggcgg aactggaaca 240

attggtgacc caagtggacg aacaactgaa cgtgttttac aaacgatgga agctgtgcaa 300

cataatgtgg acagtctttc aaaccaaatg aaaaaattat ttggtaaaga tgctgaggta 360

acaatggtga acaactacga ttggttatca gaactatctt 400

<210> 343
<211> 585
<212> DNA
<213> Enterococcus faecalis

<400> 343
caggaggaac attggttgtt cttcacaaaa atcaaccagt aactattacc tatggcaatt 60

tgaatgctag ttatttgggt aaaaaaattg ctagtgctga attccaatat acagtgaagg 120

ccacacctga ttcaaaaggt cgattgaatg ctttcttaca tgatgatcca gtggccacaa 180

ttgtctatgg aattaacatt gaccctcgta caaagaaggc tggtgctgag attgaaatgc 240

tcgttcgctt ctttggagaa gatggcaaag aaatcttgcc aacgaaagag aatccatttg 300

tattttcagg tgcttcatta aattcacgtg gtgaaaacat tacgtatgag ttcgtaaaag 360

taggaaacac ggatactgtt catgaaatta atggatcaaa agtagctcgt catggaaata 420

aagtttattc taaaacggat attgatgtag ggacgaatgg gatttcaata agtgactggg 480

aagcagttca aggcaaagaa tatattggcg caactgttat ttcaacacca aatagaatta 540

aattcacttt cgggaatgaa attgttaaca atccagggta tgacg 585

<210> 344
<211> 544
<212> DNA

<213>  Enterococcus faecalis

<400>  344
cgacagaact tgctaaagta gatccaaaaa cggtaacaaa acaagggatt cgagatacct      60

ttgatgcaga aaaagtgacg attgatttat ccaaagtgaa agtttatcaa gcagacgcaa     120

gtctaaacga gaaagactta aaagctgttg ctgcagcgat taattcagga aaagccaaag     180

acgtgaccgc ttcttatgat cttaatttag accaaaacac cgtcacagca atgatgaaaa     240

ccaacgcaga cggctccgtt gttttagcaa tggggtataa atatttactt gtcttgccgt     300

ttgtagtgaa aaatgtagaa ggcgattttg aaaatacagc tgttcagctg acaaacgatg     360

gtgaaacggt aacaaataca gtgattaacc atgtgccagg tagtaatcct tccaaagatg     420

taaaagcaga taaaaacggt acagttggca gtgtttctct acatgataaa gatattccgt     480

tacaaacaaa aatttattat gaagtgaaat cttccgaacg tccagccaac tatggcggaa     540

tcac                                                                  544


<210>  345
<211>  341
<212>  DNA
<213>  Enterococcus faecalis

<400>  345
cttctttcgt gctttcaacc acaatagatt gctctttatc agccaacagc caatggagag      60

gggataacgg aagttcatca ctaaaattaa tatttactaa attaagattc ttcaataatt     120

tttttgcttc atctacagta gagcattggc ccaataccca aggaataaac tcaaatggag     180

aaacattttc ttttccttct tcaatttttt tataatctgc atagcctgaa aagtttaatc     240

cagccattcc taatcctttt tcatttattg catcataata aagcggataa tcagcaatcc     300

cagcagcaat tccaattatt gcaaaatgat gatctaaatt t                         341


<210>  346
<211>  594
<212>  DNA
<213>  Enterococcus faecalis

<400>  346
aaacctggat gatagtgata ggaagtttat aggtaaatat tttaatgttt cggaagggaa      60

aaaattacca gattttaaac ctgaagaagt taatagttct attttaaaca ttaatatttt     120

aaacaaagat tttaagtctt ttaattggcc atataaaaaa attttatctc atattgatcc     180

agtgaaagaa caactaggga aagatataac catagctcta attgactcgg ggattgatag     240

gcttcatcct aatcttcaag acaataacct aagattaaaa aaccatgtta atgatattga     300

gttagatgaa tatggtcatg gtacacaagt tgctggagta atagacacga ttgctccaag     360

agtaaattta aattcttata aggtgatgga tgggacagat ggaaactcta taaatatgct     420

taaagctata gttgatgcta caaatgatca agtagatata ataaatgtga gtcttggatc 480

atataaaaat atggaaatag acgacgaaag atttactgta gaagcattca gaaaagctgt 540

taactatgca agaaaaaata acattctaat tgttgcatca gcaggaaatg agtc 594

<210> 347
<211> 504
<212> DNA
<213> Enterococcus faecalis

<400> 347
caaggagagc atagtgaatg tgctttggca tgtatcacta tgctacttaa ttattatggt 60

aatcaaagta cactagtaga actaagggaa aaatatgggg tgcccaaagg aggactaact 120

atcaagaata ttcgtactgt ctttgacgaa tatggatttg atgtatcgac atttaaatca 180

agtttttcaa attatttaga tcttccgact cctgtaataa gttattggaa taatcaacat 240

tttgtggtca tagagaaaat aaaaaagaag aaagtattaa tcttagatcc tgcaagtaat 300

aaacgctgga ttgatatttc agaattcaaa aaaaattttt caaatatatt aatatacgca 360

cataagaaaa agactaaaaa agaaggcaaa aggaaacagt tttttttaaa gtcatttatt 420

tttacaaaat tcaaaagata tttctttagt ttaataatat tatcatttgt ttcacaactt 480

ttattactct taattcctat tgca 504

<210> 348
<211> 562
<212> DNA
<213> Enterococcus faecalis

<400> 348
gttagggcac ttagcttttg gattatttaa taaagttaga ccagagtcat taatatttgg 60

gtttataaaa ttttcgtggg aaaatcagtt taagatcaga ttaaatacac agtggggatt 120

ttttggtgga ttatttagat ataaaccaac tacgtttaat aataagaaaa ttctgaggtt 180

gttaaccggg ggaccaatat ttagtctttt ttttacatta accttttttg taaaaattga 240

cttttttcaa tatttttctt tatttaattt ttcgatattt ttaattactg cagttccttt 300

taattttaac gggtttatga atgatggata caatatatat aaattagtta ctaaggatta 360

tatttttgaa atgtattata ttgtatcaaa tagcttactt aataaatata atcagtcaac 420

tttcttaaat acaactgagg tatgcaaaat aataaaaaaa aataaagaat taccattata 480

tgtgctaaat acattcttat tgtatgttat atatgagtat ttaatagaca aaaataatag 540

gaaattaaaa ctaatatacc ca 562

<210> 349
<211> 402
<212> DNA
<213> Enterococcus faecalis

<400>  349
tggaaaattt aagtgtagtt cctagttttg aagaactaag tgttgaggaa atggaagcga      60

ttcaaggtag tggagatgtt caggctgaga caacaccggt gtgtgctgtt gcggcgacag     120

ctgcagcaag tagtgctgct tgtggctggg ttggtggcgg tatttttact ggagtaactg     180

tagttgtgtc tttaaaacat tgttaaaata tactataaaa cttagtgagg tgaagcacag     240

tgctaaataa ggaaaatcaa gaaaactatt actctaataa attagaactt gttggtcctt     300

cttttgaaga gttaagttta gaagaaatgg aagcgattca aggtagtgga gatgttcagg     360

ctgagacaac tccagcatgt tttaccatag gcttaggagt ag                        402


<210>  350
<211>  562
<212>  DNA
<213>  Enterococcus faecalis

<400>  350
agcaaagtgg taacgagaag tacgacatta aaaatttaca agcttggaaa gaaagaaaaa      60

gtgttcttaa acaagatgat ttagactact tgattaaata taaatatgaa tcactggata     120

attttggatt aggaataaca cctattgaaa actttcctga taaagaagtt gcaattcaat     180

acattaaaga tcaatcatgg tatatttttt ttgaatccat tttagattct tataatgata     240

gtgaagagca attattagaa gtagatgcta gttatccttt tagatatttc ttacagtatg     300

ctcgtttatt tttacttgat ttaaactcag agttaaatat ttgtacaaaa gaattcatta     360

ttaatttatt agaaattcta acacaagagc ttattcactt aacaagtaaa acattagtgc     420

tagatttgca tacttttaaa aaaaatgaac ctctaaaggg aaatgatagt agcaagcgat     480

ttatctatta tctaaaaaaa agatttaact ctaaaaaaga tataatagct ttttatacat      540

gctatcctga gttgatgcgt at                                              562


<210>  351
<211>  590
<212>  DNA
<213>  Enterococcus faecalis

<400>  351
tagttggaat gaccgagaac gatggctcac cacgaaaaat caatttaaat ggtttagggg      60

aagtttttat ctataaagat catgttgtag caacatttaa tgaaaaagtt gaatctttac     120

ataatgtgaa tgggcatttt tctttcggga ttaaaacgct tatcaccaat agttcgcaac     180

cgaatgtgat agaaacggat ttcggaacag caacggcgac tcaacgtttg acgattgaag     240

gagtgaccaa cacagagact ggccaaattg agcgagacta tccgtttttt tataaagtag     300

gcgatttggc tggagagtca aatcaagtac gttggttttt aaatgtgaac ctcaataaat     360

ccgatgtcac agaagatatt tcaattgcgg atcgacaagg aagtggtcaa caattaaata     420

aagagagttt tacatttgat attgtgaatg acaaagaaac taaatatatt tcacttgccg 480

agtttgagca acaaggttat ggcaaaattg acttcgtaac agataatgac tttaatttac 540

gtttttatcg ggataaagca cgctttactt cctttatcgt ccgttacact 590

<210> 352
<211> 648
<212> DNA
<213> Enterococcus faecalis

<400> 352
tcaacgtcac aaacaagaac ctgatatctg tgaaaatgca aatcaattga atgaagctgt 60

aaagcccaaa accggaaacg aaaacaaaca accaaaaata ccgaagaaaa aatctaatta 120

tagcaagtat atattcgcat tgtttaccgc acttattcta gtaattgtcg ctactggcgg 180

ctatattgtt tatacattaa aacagcaaga agtagaagct caagccaaat atgaaactgc 240

tgtaaaaaat ctcatggctt caatccaaga agagcaagac caaagtggaa tttcaacgaa 300

aatagatact ataaatgacg gagaaaataa gtgccttatt taccgtccag tttatgaaag 360

tactgttcct tttaaaaatg caaaccagct cttagacgag cttgctcaaa agcaacaaaa 420

gaagcatcgt gaaaaagaag tgcttacagt tgccagaata aaagcaacag caatatcttc 480

taaaattggt cagtatagaa ttgaagcaga tagttttatc tgggatcgca gtaaggaaaa 540

ttttaaaaag ccagacagta tttctgagaa agccatttat gtttccgaaa aaactggtaa 600

agaaatcaca aataaggatt tgattccgga tgaaggaagt ctcttagg 648

<210> 353
<211> 520
<212> DNA
<213> Enterococcus faecalis

<400> 353
tcggaagtat tgcgtttggt gggacaacat tagcttacgc tgatgaagtg cataatagta 60

taaatcagga tatacaagat tctggtagta caattattgg agaaaatgat tcttctacca 120

aatcagctga gtataaaatg attcatgaaa ttgatggaac taaaattagt aacggtgaaa 180

atagtaaaga aacaactaca agttcaggaa ctatactggc tgaagaagca atagaaagtt 240

caaatcaaaa aaattcaaag acaagtgaag tcgaacagga tcttcataaa gatgtatcag 300

gatctgaatc agtaaaacaa gtagaaactt ctgattctat aaaaaaatct gaagaatcag 360

ctgttaaaac attaaatctg gatgattcac aagagaatac taattcaata actaccaagg 420

cagaaaatga tgcgctatct acagttaatg atgaaaaagt attaaatgaa agtgatagta 480

ttatcaaatc aattccttcg gaaacagaga atgtcgataa 520

<210> 354

<211>  668
<212>  DNA
<213>  Enterococcus faecalis

<400>  354
ttgtctttgg gcttctctct tttttcttgc ctatattttt agtctttgga ggcttacttt      60

tttttctttt attattaacg agtacgtcag atacttcaaa aaatgattgt attcagccaa     120

gtataaataa tccaactgat gcgacagata cacctaaatc gatcgagcag tttgtaaaaa     180

gccataaaga tgcttacctt ttatcatgga aagcaggtgg ctttttaccg tctgctagta     240

tttctcaaac gatggtagaa aatgggttta attttactaa tccatcgggg acgtcatttt     300

ggcaggcaca caatatgggc ggtgttaaaa cgtcaaaaaa agaagatttt cctgtaactt     360

tagcaacatt cggccaagat tctgttgata tttctggtac aaagccaggg tcaaacgtcg     420

gtgatggcac tggtggggca tatacctggt ttaaagacta caatgctgga attgttggaa     480

aagcagaatt tatggcacac cagacactgt atacaggtgc tatcaataat actgacggat     540

taagtacttt atcagctatt tattcaggag gatgggctac agaccctact tacctcatga     600

agttacaggc cacatataat agcttaggca agcagtttca atggttggac caagaagcaa     660

tacagaaa                                                              668

<210>  355
<211>  517
<212>  DNA
<213>  Enterococcus faecalis

<400>  355
ctatagattc cctatcttgt tggtacaaag ataaataaag aaatattttt tttcgataga      60

atacgttaaa atatgaatag atatagatag taattatatt atctataaat agtagagtat     120

aacgatcttt tatttttgga ttttctataa attttaagta gtaagaaaat ctttttcgtt     180

caaacttttc tataatctct aaatttttaa tttgaacaga attagttgaa ataagcatat     240

aaaaatttaa tagtaattgc tccttatcag attttagacg tactctttca attatattca     300

tgatatattc atcgatggta gagcttttat cagcaatttt ttctaattca gagtttatta     360

tatccaaatt atacacaatc actgcctcat ataaatcatg ttttgtttta aaaaagctat     420

atacggtagt agtgctggtt ttagcttcat tagcaatatc taaaagtttt gttttttcat     480

aaccaaattt agaaaagtgt ttcattgcgg taaatat                              517

<210>  356
<211>  380
<212>  DNA
<213>  Enterococcus faecalis

<400>  356
atgtatgtat cttttgttct aagttgtttg cttggatttt ctgcctacag tctattaaat      60

aggctaaatt cgttggaatt tgtggatgtt tggttagaca aagaaacaca aaaaatcaca     120

ctaaaacgct gtttttatga tacgtctttc aagaaacaaa cactaaaaga gttagaacga     180

gtatatttcc aattaaaaga aataatcaac gtgcaaataa acaagcggtc tttaaatacg     240

aatgacatac gtaatgtacg agaactagag gaaaaacaac aagaaataaa acgattcatg     300

ttagacgttt tagaagatgc ttattggaaa gaattagcaa atatgccaga agaccaacga     360

cacttagacg attgggattt                                                 380


<210>  357
<211>  320
<212>  DNA
<213>  Enterococcus faecalis

<400>  357
aaagtactac cttttattgc cttagtcggc ttgttattgt tgtcaggttg tggaacagat      60

atgaaaaaga tattgactgc cgatggtggt aaatggaaag tggaagaaac acgtgcaact     120

tacacttttt ttgatgacgg taaattttca gctaatgact cagaggatag tgttagtggg     180

acatacactt atgatgaaaa aaataaaaaa ataacctttg acattactag cagaaactct     240

ttcattatgg aaaaagtaga atacaaagat aacaagatta caggggaaat tggcgaaaaa     300

caaagaacac ttataaaaca                                                 320


<210>  358
<211>  503
<212>  DNA
<213>  Enterococcus faecalis

<400>  358
tgaacaaaaa gcacaggata gtgtaaaaga agttactgaa aatgttactc aaactatttc      60

aaacgatcaa cgtataccag ctgattttgt taggcacgtg gatggcgata ccacagtatt     120

aaaaattgac ggaaaagaac aaaaagttcg gtttttatta attgacacac ccgagactgt     180

gaaaccgaaa acaaaagttc agccgttcgg attggaagct agcaaacgca caaaagagct     240

tttgtctact gcttcagaaa ttacgtttga atatgataag ggcgataaaa cagatcgtta     300

cggacgagcg ttgggctaca tattcgtaga tggaacatta ctacaaaaaa cgcttgtaag     360

tgaaggatta gctcgtgttg cctatgtaaa agagcctaca actaagtatt tggcagaact     420

agagcaagcc caagaacagg ctaaaaatga gtcactcgga atctggagca taccaggtta     480

tgtgacacaa cgggggttta gta                                             503


<210>  359
<211>  220
<212>  DNA
<213>  Enterococcus faecalis

<400>  359

tgatgaaaat ttaaaagaag aagcagaaca attatttgat gatttagggt taaatatgac 60

aagtgcaatt acgattttct taaaacagtc tattaatgag caagcaattc cttttatgat 120

taataaggga aacaaagaga ctctacaagc attaaaagac attaaagaag gaaatgttca 180

tggtggattt tcttccgtgg aggatttaat ggaggattta 220

<210> 360
<211> 380
<212> DNA
<213> Enterococcus faecalis

<400> 360
tcaaatacac gtaagccttc tttcgtgctt tcaaccacaa tagattgctc tttatcagcc 60

aacagccaat ggagagggga taacggaagt tcatcactaa aattaatatt tactaaatta 120

agattcttca ataatttttt tgcttcatct acagtagagc attggcccaa tacccaagga 180

ataaactcaa atggagaaac attttctttt ccttcttcaa tttttttata atctgcatag 240

cctgaaaagt ttaatccagc cattcctaat cctttttcat ttattgcatc ataataaagc 300

ggataatcag caatcccagc agcaattcca attattgcaa aatgatgatc taaatttcca 360

acttctcgaa atgaaaactt 380

<210> 361
<211> 511
<212> DNA
<213> Enterococcus faecalis

<400> 361
cattattttc attaggggat attagagata ttcttatcct tataaattat ttttttacgg 60

gaaagattga agacttattt cataagccgt tacatgatta tgagaaaaaa ttttcagaag 120

atatccaaat agaacggata gatatgttat tatctcaaaa ttatgatcca gaaatttatt 180

tatttttata tgaaaataaa attttagaat atgttgtaaa tggtaatgta caagaattaa 240

gtaatatgat atttaaacta agtaatggtg ttgttcctgt ggttagtggg gataacgtac 300

gttctgaaaa gaattattca atagttgtat ttgagaagtt agcacaagca gctataaata 360

tgggaatgga cttaataaat gcatatcaga gtcgagatag ttttataagg aaaaatgaac 420

tatgtataaa tttaaaagaa gtattaaaag ttagagatac tgctatagta ttttatacct 480

ctgaaatagg aaaagctaaa gtaaggaatc t 511

<210> 362
<211> 526
<212> DNA
<213> Enterococcus faecalis

<400> 362
ttgcgatttc tgttgtagga accattattt ttgtaatagg actttatgtt agtaaaataa 60

aaaaataaat cacaattaag gttctggttg ttattaatct atctcatgaa gcattagatg 120

aattagttct agaagtacct gttgtactag ttaaaaatac tgttaaatca aattttttgt 180

ttaaaagaat cattaagttg gtgcctaact ataaaatcaa attgactaaa atccaataac 240

attgggggat actctgtaaa tcgtgtgtcg cagtacgtta gtcttgtaat aaatagatct 300

taattaggag gggtttctat gaaaaatatt ttactttcta ttctaggggt attatctatc 360

gttgtttctt tggcgttttc ttcttattct gtcaacgcag cttctaatga gtggtcgtgg 420

ccactgggca aaccatatgc gggaagatat gaagaaggac aacaattcgg gaacactgca 480

tttaaccgag gaggtactta tttccatgat gggtttgact ttggtt 526

<210> 363
<211> 505
<212> DNA
<213> Enterococcus faecalis

<400> 363
aatcaagccg ctgaaaagaa agaaaaatta gcaattgtga caacgaactc gatcttatcc 60

gatttagtga aaaatgttgg gcaagacaaa attgagctgc atagtattgt gccaattggg 120

acagaccctc acgaatatga accgttacca gaagacattg cgaaagcttc tgaagcggac 180

attttattct ttaacggctt gaacttagaa acaggcggaa atggctggtt taacaaatta 240

atgaaaacgg ccaaaaaagt tgagaataaa gattactttt ctacaagcaa aaatgttacg 300

ccacaatatt taacaagtgc cggtcaagaa caaacagaag atccgcatgc ttggttagac 360

attgaaaatg gcatcaaata tgtagaaaac attcgtgacg tgttagtaga aaaagatcca 420

aaaaataaag atttctatac agaaaacgcg aaaaattata ccgaaaaact tagcaaacta 480

catgaggaag ccaaagctaa atttg 505

<210> 364
<211> 557
<212> DNA
<213> Enterococcus faecalis

<400> 364
aaatgggtga aggaagatta gcaaattatt ctgcttcagg aaatacgttt caagaaaatc 60

cgggatatac gaagaattat aatttctcgg atttacaatt caaccctaaa gcaataactg 120

gtgatgtgtt acagggaaat acaattgatt ttgaggttta tgggaaacat aatattgcag 180

cttcaactgc aaactgggaa attcgtcttc aattagatga acgattggcc cagtatgttg 240

aaaaaattca agttgatccg aagaagggcg taggaaatag tagacgaact tttgtaagaa 300

ttaatgattc gcttggcaga cctacaaaca tttggaaggt taattacatt cgagcaaatg 360

atggactatt tgctggggca gaaacaactg atacacaaac tgctcctaac ggtgtgatta 420

catttgaaaa aaatttagat gaaattttta aagaaattgg tgcagataat cttaaaagcg 480

accgtttaat gtatcgtatc tatttggtaa gtcatcaaga tgacgataaa attgtacctg 540

gaatagaaag cactggt 557

<210> 365
<211> 523
<212> DNA
<213> Enterococcus faecalis

<400> 365
aggtacaggc atctttgttg gaagttcatg tctattttct tcactttttg tagccgcaga 60

agaacaagtt tattcagaaa gtgaagtttc aacagtttta tcgaagttgg aaaaggaggc 120

aatttctgag gcagctgctg aacaatatac ggttgtagat cgaaaagaag acgcgtgggg 180

gatgaagcat cttaagttag aaaagcaaac ggaaggcgtt actgttgatt cagataatgt 240

gattattcat ttagataaaa acggtgcagt aacaagtgtt acaggaaatc cagttgatca 300

agttgtgaaa attcaatcgg ttgatgcaat cggtgaagaa ggagttaaaa aaattattgc 360

ttctgataat ccggaaaata aagatcttgt ctttttagct attgacaaac gtgtaaataa 420

tgaagggcaa ttattttata aagtcagagt aacttcttca ccaactggtg accccgtatc 480

attggtttat aaagtgaacg ctacagatgg aacaattatg gaa 523

<210> 366
<211> 400
<212> DNA
<213> Enterococcus faecalis

<400> 366
ctggttcaaa agaagccatt gatgcccgcg ttcatttaat taaaaaccaa atcggcgaaa 60

caacgtctga ttttgatcgt gaaaaattac aagaacgttt agctaaatta gctggcgggg 120

ttgctgtcgt taaagtcggt gctgcaactg aaacagaatt aaaagaatta aaattacgaa 180

ttgaagatgc attaaacgca acacgtgccg ctgtagaaga aggcatggtt tctggtggtg 240

gtaccgcact tgtcaatgta attggtaaag tcgctgcgct agaagctgaa ggcgatgtgg 300

caacagggat caagattgtc gttcgtgcat tagaagaacc aatccgtcaa atcgctgaaa 360

atgctggtta tgaaggatca gtgattgttg acaaactaaa 400

<210> 367
<211> 264
<212> DNA
<213> Enterococcus faecalis

<400> 367
gatcgcgtcg taattagagt cgcgaaagaa gaagaaaaaa ctgttggagg aattgttctt 60

gcatccgttg cacaagaaaa accacaaaca ggtgaagtta tcgcagtagg tgaaggtcgt 120

gtgcttgaaa atggcacaaa agttccgatg gaagtaaaaa ttggtgacac agtaatgttt 180

gaaaaatatt caggaacaga agtgaaatac gaaggcgtag aatacttaat tgtatcagcc 240

aaagacatta ttgccactgt tgaa 264

<210> 368
<211> 505
<212> DNA
<213> Enterococcus faecalis

<400> 368
atctcgcgga acaattagat agtattcttt tacaagtcag tgaagaagat gaactaatta 60

tttcagatga tggttctact gatcatacgt tggaaatttt gagaacgtat gcagcgaatt 120

atccccaaat tcaattgtta caaggtccag ggcaaggagt gattgctaat tttgcatttg 180

cgcttacgca tacgaaaggc gaagtgatat ttttagcaga tcaagatgac gtttggttgc 240

caaataaagt aacaacagtg acagaatatt ttgaaacgca ccctgacatc caagtggtta 300

ttagtgactt gaaaattgtt gatgcggatt tacaagttac caatccctct tattttaagt 360

ttcgaaaagt caaaccaggg ttttggcgaa atgcgataaa aagtggctat attggggcag 420

gtatggcctt tcgtcaagag atgaaaaacg tcattttacc cattccgcca gaagttccta 480

tgcatgatat gtggattggc ttatt 505

<210> 369
<211> 688
<212> DNA
<213> Enterococcus faecalis

<400> 369
tcggctctaa tggtatgttc cattacatta acaagcgtag cgttgccatc cgcagcattt 60

gcagatgaat acgatacaaa gattcaacaa caagatcaaa aaattaatgc gttaactagc 120

caaatgtcag atgcagaagc aaaagttgcc gcgattgaaa atgatatggt tgaaacggcc 180

aaacaaatcg atacattaac agctaaaaag aacaagctat catcagaagt atctaaatta 240

tatagtgaaa tttctgattt gaatgtccgt attcaaaaac gtgaagtaca aatgacaaaa 300

caagcacgcg atgtccaagt gaatggtcaa agtgattcaa ttattgatgc tgtcttagat 360

gcagattcag tagcagatgc aattggtcgc gttcaagcgg tctcaacaat gatgagcgcc 420

aataatgaat tactagaaca acaaaaagaa gacaaagcga ctgttgaaaa gaaaacaaag 480

aatgttgaaa aacaaattgc tgaattagaa gcagcaacaa aagaattaaa tgataaaaca 540

gaatcattaa aaacattgaa gattcaacaa gaagtggcta aaaatgattt agaagcacaa 600

cgttctgaag aacaagggaa aaaagacggc ttcattaaac agaaaaaaga agcggaaaaa 660

cgtttagcag aagaacaagc acgtcaac 688

<210> 370

<211>  500
<212>  DNA
<213>  Enterococcus faecalis

<400>  370
gcttcattag cattagaaca atcatcagct gaaagttcta aagctggctt agaaaaacaa      60

aaagcagctg ctgaagcaga gcaagcacgc ttagctgctg aacaaaaagc tgcagctgaa     120

aaagccaaac aagctgctgc aaaaccagct aaagctgaag tgaaagcaga agcaccagtt     180

gcctcttcat caacaacaga agcacaagca ccagcaagct caagctcagc aactgaatca     240

agcacgcaac aaacaactga aacaactaca ccaagtacag ataatagtgc aacagaaaat     300

actggctctt cttcatcaga acaaccagta caacctacaa caccaagcga taatggaaat     360

aatggtggcc aaactggtgg tggaacagtt acaccaacac cagaaccaac accagcgcct     420

tctgctgatc caacaatcaa tgcattgaac gttctacgtc aatcattagg tttacgtcca     480

gtagtatggg atgcaggttt                                                 500


<210>  371
<211>  529
<212>  DNA
<213>  Enterococcus faecalis

<400>  371
ttaactgaac aagaaaagca agcaatggaa aaagaagcat tagcattaaa taaagttttt      60

cctgaaaatc aagcagatgc ggcaaaagta acggaaatga tcaatgtcaa aaatcctacc     120

gaaaaacaaa agcaacaaat gagcgattac gttgtaggac ttatcaatga tgttcgcgaa     180

aagcttgggt tacaaaagtt gaagatttct aaccaagcta tgaaatttgc ttgggatgta     240

gcaaaatatg ataatcccaa agaatttgat catgacgtaa atgcgatcaa tcgtgcagca     300

aaagaaaatg gttttaaaga attccctgga caaaactttt atgaaaacct aagtatggga     360

agatttacga cacaagaagg taaagtttct atgtatgact ttgaaaaagc tgctcgaaat     420

gcacttgtaa gcatgttgat gaacgatgga cattctggct attcccattt agattcttta     480

ttagatgcaa atgaaacaaa catggcagtt tctatttcag gagatttaa                 529


<210>  372
<211>  558
<212>  DNA
<213>  Enterococcus faecalis

<400>  372
acaaccaaca gtgaaagcta cacaaacaac ggagcaagcc attactgaaa aacagcaaca      60

agtaatagag aaacaagcaa ttgtcgatca aaaacaacaa gttgctgaca ctgcgaaaaa     120

agaaaaagac accattgatc aatctgttaa agaccaacaa gcagtggtcg atcaaaacaa     180

agacgcattg gttcaaagtc aacaagcagt gactgaccaa caagcagttg tagacgaagc     240

taaaaaagtc gtggatgaag caacaccttc agccattgaa aaagccaaag agcaagtggc 300

tactgataca caggctgttg atgaccaaca aaaagtagta gagcaagctc aaacagacgt 360

taaccaacaa caagctgttg ttgatgaaaa agcaaaagaa acgactgctg ctaaagtgca 420

aaatgataaa gatcaacaag cagtaacagc tgcaaaacaa gaacaagtca agcttgaaga 480

attagcgaaa aatgcggaag cggaaaaagt aaaggcagaa aaagaacaag cagcaaaaga 540

agcagaattg gctaacaa 558

<210> 373
<211> 687
<212> DNA
<213> Enterococcus faecalis

<400> 373
cattggtggc tatttcattc gtgaattgga agccactaca atttccgatt ttaaaaaaaa 60

tatggattcc caagttgtcc aattgtcaaa cacgttaagt acgcagatga gcaacaaaga 120

tctcgaacgt agtgacgttg atgcaaattt aaaaaaagcg ttatctgatt tttcaaatgc 180

agatatttct gaagcgagaa ttgtcgatga taaagggatt attcgggcaa ccaatgattt 240

aaatcaacaa aatattattg ggaaaaagaa tgattatcgt gatttaaatg actttacgag 300

taaaaaatat caagctttag ataatgataa acgcgtgtat gtgaatgtcc agccgattca 360

atcgcctact ggagaaacag tgattggcgt cctttatgtg aaaagtaatt tagaaaataa 420

ataccaagaa attaccaaca cagcaagtat ctttttcact gcttctatta ttgccgcagc 480

aatctcgatt attgtgactt tactgattgc acgatcaatc acgaagccga ttggtgaaat 540

gcgcgagcaa gccattcgaa tcgctcgtgg tgattacgct ggaaaagtag aagtccatgg 600

aaaagatgaa ttaggccaat tagcagaaac atttaatcaa ttatcagaac ggattgaaga 660

agcacaagaa acaatggaag cagaaag 687

<210> 374
<211> 534
<212> DNA
<213> Enterococcus faecalis

<400> 374
tatcttagct tcgcaaccag ttactcgttt taggaatgct tttttcaatg aaacggaaga 60

tatccaaacc aatgaagaca gtcaagactt aacctacacg agtaaagaag aacgattgtt 120

tgcagaagaa aaactgggaa aaattgattt taaagggacc ttgccagaag agaataaacg 180

ggactcaatc tataatcaaa gcttttctta tgtaaaacgt ttaggaacca atatggggaa 240

tttgcgttac tttgatcgaa cgaaagatag tgtcaattat cggacttttg tggaaggttt 300

cccagtgttc agtaatgatt taaaaggcca agtggatatt cgcatcacga acaacgatgg 360

tgctgcacca agcgtaacca ttaacacaag tgtgaatacg atccaagtgc cgattccttc 420

agaagaagaa gtgacgctgg aaagcacgga aaaattgatt aagcgtttag aaacggctgg 480

tgctaaaaag gaaaaaattc aatcggctgt tatcggttat acgtggcaga caat 534

<210> 375
<211> 547
<212> DNA
<213> Enterococcus faecalis

<400> 375
gagcaacgtc tcttcttcca gccaaacaga atcgattgaa agtcggttgg aaaaagataa 60

catctcgtat aaagggacac tttcttcaga acgattggaa ggttattatt taagtggcga 120

acaaaccaat ttttctgctg ctttaaaaat ccaacgtgaa aagaataaaa attttttgag 180

aaatgggctg caaattgcgg ataatacttt aacgagtgta cctagtaaaa actattttat 240

tgatcctaag aaaattgata aagatttaag taccttttta aatgaaaaaa atgctttatt 300

attcggagac gaatatcaat acttaccaga attttctcat ttaaaagagc cgacggcaga 360

aattgtggct gcacaatcgt ataaaggaat tccttttaga gacgacacgg caaaattaag 420

tattttagca gattcgtcag gtgaattatg gcaaattagt aaatattcgc aaacgcacat 480

tgaaaatatt gaagagttac gagacaaaac ggatttatat tccaatcgtg atgcgataga 540

cacgctc 547

<210> 376
<211> 224
<212> DNA
<213> Enterococcus faecalis

<400> 376
ttcatcgcaa taatcgttcc tttgttgttc taacggatac aggttattgt agcgatcata 60

ttcgtggtac gattgaaaat gcagatgctt atttagtcga aagcaatcat gaaattgaaa 120

ttttgcgagc aggaccttat ccatggagtc ttaaacaacg gattttagga gataaaggcc 180

atttatccaa tgatgatggt gctcttgtga tggcggatgt gtta 224

<210> 377
<211> 500
<212> DNA
<213> Enterococcus faecium

<400> 377
tcttcatttg ttgaatatgc tgttttaagt attcgatgcg atattcatca tgtatgtttt 60

tatcatctgt caaaacatct atggcaccta atccattttc tgtaattatg atagggagtc 120

catattttct ataagtgtaa ttcagcagat accgtaaacc cgtcggatca atggtccatc 180

cccatttact agtcacaaga tacgggtttt gaagaccgcc aaataaggca ctcttttctt 240

cagctgctcc ttcgtacttc gcaacagatg atgcataata gttcatacca ataaaatcaa 300

gtgttccttt agagaacata tatttatcat tctctgttat ggtcaacttt attccttggt     360

ctgcatattc attgatttta tagtctggaa actttcctgt gcacatagca tctatttgat     420

agaaatctcg atccatttgt ttaaaagcat tcatcacatt tgttggattg caatctactg     480

gataaacagg ttcgattcca                                                 500

<210>  378
<211>  665
<212>  DNA
<213>  Enterococcus faecium

<400>  378
attattgtcg cctctttccg ctacgcgatt aacatgaatc atcaaataag tgtattcatc      60

ttgagataaa taggtgttga actttccttt tacatatatt tctatctttt ctacagctgt     120

atatgcttta gggtatagtt tttttacttg ttcaaataac tgagattcat tttcaacgta     180

tgcttgtttt tttcttaatc gttcaataaa atactgtaaa tgtgtcacta gcctcatgta     240

gttgatgctc tcttcgtcaa tagataaact aaaatgatat ttgatgatat tcaacatgtc     300

tcttagtgtc tccatatctt ctatctgttc atcaaaattg acctgatttt cttgaagatt     360

aacaaagtgt aaggcaattg aaacagcttc atctgtggga aaggaatgat taaaatattt     420

cttcatcatt tttaaagctt ccaaaccgat tttgtaataa actggataaa actttttaac     480

ttcccaaaag agcggacttc taagatattg tcctttttct gagcgtttca atgcaaagga     540

gagatgatct aataaagcta aataaagata atcatttgct tttttaccga tttccttttc     600

tccataacta acgagctcgt tgatcataga gatcagtcta tcatcagaat gcgataacaa     660

atagc                                                                 665

<210>  379
<211>  504
<212>  DNA
<213>  Enterococcus faecium

<400>  379
ctcctgatcc tcttcttgtg cagggacgcc taagagataa gcagctacag ctgatccagc      60

aaaactaatc acgactgccg ctaacataaa ccagaagttc atgaaatctc cttcacctat     120

atacgctggg aggccaaata aaccccaagc aacagaataa gctttaacac tagtcaaacc     180

agcaaataat ccaccaagtc ctcctccaat cattactgca acaaatggtc gacgatattt     240

aacaaagaca ccatagatag caggttcagt cacaccaagt actgcagaaa gtgttactgt     300

cccaaataat tgtttttgtt ttaaattccg tgttcttaag aaatacccaa gcattgctcc     360

cccaacagca atatctgaga ttgtacatga agatataaat gcagggtcat acccatttgc     420

tgcaattaga gaagctacaa ccggcatgat aaagtttcct gcgcccaaca ttataataaa     480

tggttgaaga gcagagtata acat 504

<210> 380
<211> 555
<212> DNA
<213> Enterococcus faecium

<400> 380
cggatgaagg aagtaaagaa aagttgtcag tcgtggctac caattcgatc ttggcggaca 60

tggcaaaaga agtaggtaca atagatatcc acagtatccc gttcggaaca gatccgcatg 120

aatatgaacc attaccagaa gacatcaaaa aggcaagtgg tgcagatgtt attttataca 180

acggtttgaa tcttgaaaca ggtaacagct ggttcgataa cttgatggaa acggctaaaa 240

aagaagggaa agattatttt gcagttagca aaaatgtaga acctctatat ttaactagcg 300

gtgaagaaca tacaaaagca gatccccacg catggctaga cctatctaac ggaataaaat 360

atgtggagga aatcgcacgt atattctctg aaaaagatgc agaaaatgcg acactctata 420

aaaaaaatgc agaagcatat gtggaaaaac taaaagaatt agatacccca gccaagggaa 480

cttttgcttc tatcgaagag aacaaaaaat tattagtaac aagtgaaact gctttcaagt 540

atttacgagc atatg 555

<210> 381
<211> 401
<212> DNA
<213> Enterococcus faecium

<400> 381
aaagcgattt gttgctgaca gcactcgtta gtggaatcgt cttgatattt gtctttttct 60

tttataaaga attgaagatc acatctttgg atccgacaat ggcaaaggct ttttggctga 120

acacttggtt gatccattat cttttgatgt tctttttgac attagtggct gtagtcagtt 180

tacagacagt aggaacaatc ttggtgattg ccatgttgat cacaccagcc gccacggctt 240

acttgctaac gaaccattta ctgaaaatga tcattacagc tgcaggaatc ggtatgctaa 300

gtgcagttgt cggtgtgttt ttcagtatag ttacattggc catcagagct acgatcgtgt 360

tagcatgtac cgcatttttt atccttgcta atttaatttt c 401

<210> 382
<211> 507
<212> DNA
<213> Enterococcus faecium

<400> 382
agccggtaaa ctcagtccgt aaaaaaatag cctacgtgga acaacgaagt gaattggatc 60

tttcctttcc agtgatggta ataggcgttg tacttttagg aacatatcca tctttacgaa 120

ttggacaaag acctgggaaa cctggaaaag aacgtgcaag acaagctttg aaaaaagtag 180

ggttggaaga atatgccaaa agacagatca gcgaactatc gggtggacag ctccagagag 240

tttttattgc aagagctcta gcccaaggag cagaatggat ctttttagat gaaccattcg 300

tagggattga tgcgttaagt gaacgaaaga tctttgacat cttgcaggaa ttgaagaatt 360

caggaaaaac gattttgatc gtccatcatt ttcttcataa agtagacgaa tatttcgatg 420

aggttattct tgtaaataaa cagctgatcg cttccggtcc agtacaagag tcttttacat 480

cagaagacct tcaattgcct tatggtg 507

<210> 383
<211> 456
<212> DNA
<213> Enterococcus faecium

<400> 383
attactcgtt tcccctgaca gttggcagga catgctgatc gtagacaagg tttctaaaga 60

cggtatcgaa gcaaatatgg cagtcatgtc gcaaaaagga ttgattggcc gagtgatcga 120

ggtcaatacg gcttcgtcta aaatcgaatt actgtcatcc tctaatgaaa gctccaatca 180

ttttccagta cgggtatctt cggctaatgg cgaagcgttt ggtttgctta aaaactatga 240

tgaaaagctc catgccttag tggtgaccca attaactggt gatacggata tcaaagaagg 300

ggatgttgtc cagacatccg gtcttggagg gaattctcca gctaacttgc cgatcggtac 360

ggttattaaa acgaaaccag atagttatgg gctggatcgg gaagtttatg tgaaacctta 420

tgcagaaatg tatgacgtgt cagttgtgac gattgt 456

<210> 384
<211> 500
<212> DNA
<213> Enterococcus faecium

<400> 384
atgttgaaga aagaaacaat gaagtactat ctgccaatcg ttttgttctt tttgatgttg 60

atagatggtc atttaacaag aatgctaggg gagtggtcga aaggcaccta tatgtcaaat 120

gcccactttc tgatattggc attattatgt tgcagtatgg cgtttgaaaa acgttattta 180

ctgattacca cgattgttct cggggctatc tatgacgctt actatattgg cgttatcggt 240

atctatgcag tagctctccc tttaattgta tggttgatgt atgtaatgaa agacgttatc 300

catgtcaaca tctttactga atttttcagt atgatcatct ttgtcacggg ttatgaattg 360

tttacgatgg tggtccagtt gatttttaaa ttagcagtag taaataacac gtattttatt 420

acaaggtttt taggacctac actgctgttg aacatgatta tatttgtatt attcattttt 480

ccctttaaga aattattcag 500

<210> 385
<211> 507

<212>  DNA
<213>  Enterococcus faecium

<400>  385
tcagtcagtt tcttgacctt tttcgtaaag aagccggcct ctcactaaaa gcttcagtaa      60

tcagtcagaa tttcgttcct accgcagctg gattagcctc ctctgccagc gggctagctg     120

ctttagcagg agcttgcaat actgctctta agcttggatt agacgatctc tctctttcaa     180

gatttgctcg acgcgggtct ggttcagctt gccgaagtat tttcggtggt ttcgtcgaat     240

gggaaaaagg ccatgacgac ttaagttctt acgctaagcc agtcccttcc gattctttcg     300

aagacgattt agcaatggtt ttcgttttga tcaacgacca gaaaaaagaa gtgtccagca     360

gaaatgggat gcgtcggaca gtcgaaacat ccaattttta tcaaggctgg ttagattccg     420

ttgaagggga tctatatcaa ttgaaacaag caatcaaaac aaaagatttc caacttctcg     480

gagaaacgat ggaaagaaac ggactaa                                         507


<210>  386
<211>  508
<212>  DNA
<213>  Enterococcus faecium

<400>  386
ccaattaggt gaagcagaac ttgtgatagc cggcggaaca gagagtatgt ctcaagcacc      60

tatgctgaaa ccgtatcagt cagaaacaaa tgaatatggt gaaccaattt ccagtatggt     120

caacgacgga ttgactgacg cattttcaaa tgcacatatg ggattaaccg cagagaaggt     180

tgcaacacaa ttttctgtga gcagagaaga acaggatcgc tatgccttgt cgtcccagtt     240

gaaagcagca catgctgtcg aagccggtgt attttctgag gagatcatcc cagtcaagat     300

ttctgatgaa gacgtgttat ctgaggatga agcagttcgt ggaaatagta cattggaaaa     360

actgggcacg ttacgtacag tattctcaga agaaggaact gtaacagcag gaaatgcttc     420

cccgttgaat gacggtgcct ctgtggtgat ccttgcatcc aaagaatacg cagaaaataa     480

taatctgcct tatttagcaa ccatcaaa                                        508


<210>  387
<211>  501
<212>  DNA
<213>  Enterococcus faecium

<400>  387
gattgccttt cctttctatg caacaaaagt caccgcattc cttgaagagc tggatgcaat      60

ggacgatcaa ctggtttctt cctactattc aggaaattta gccgaagctc ctcatgcatt     120

aaaaaatatc aaaaaattat tcattcactt aaaaaaacag catgacatcc aaaaaaactt     180

gcaactgacc attgaaagca cgattcctgc tgaacgtgga atgggatcaa gcgctgcagt     240

cgccacagca gtcactcgtg ctttttatga ttacttagca tttcctttgt ctcgtgaaat     300

actattagaa aatgtccagc tttcggaaaa aatcgcccac ggtaatccta gtggaatcga 360

tgcagccgct actagcagct tgcagccgat ttattttaca aaagggcatc ctttcgacta 420

cttttctttg aacatcgatg cttttttgat tgtcgctgat acaggaatca aaggacaaac 480

aagagaagcc gtcaaagatg t 501

<210> 388
<211> 505
<212> DNA
<213> Enterococcus faecium

<400> 388
caagaacaag aaactcagca ttctatcagt gagttacttg ccctggattg gccaggtcta 60

tccattgagc cattgattgc tcctgaagat ttacgtttat tgattggttg gacgggtagc 120

cctgcctcta cttctgattt ggtcgatcaa gttcaccgtt cgagagaaga taaaatggtg 180

gcttatcagc ttttcttaaa aaacagtaca gaatgtgtca atgaaatgat caaaggtttt 240

aaagaaaata atgtaacgtt gattcaacag atgattcgaa aaaaccgaca attactgcat 300

gatttatctg caatcactgg ggtcgtcatc gaaacgcctg ctttgaacaa attgtgtaat 360

ttagctgaac agtatgaagg agccgcaaaa tcttctggtg caggtggggg cgattgcgga 420

atcgtaattg ttgaccagaa atctggcatt cttcctttaa tgagtgcatg ggaaaaagca 480

gaaatcactc cactgccgtt acatg 505

<210> 389
<211> 585
<212> DNA
<213> Enterococcus faecium

<400> 389
aaattcactt actgcaccag agccgtagct gaatagaccg atgcgatctc ctggctgtag 60

tgatttcgaa ttttccagta gagaagttag ccccaggtat aatgaaccag tgtaaagatt 120

accgattcgt cggctgtaac ggatgctttc ttcatagcga gccataagac gttcctgatt 180

atcttcgtct gtttggtcta atacgctttg caatgccttt tttcccatct tagtatacgg 240

aatatggaaa gcaatcgcct gataatcttc gagtcctcga cccgacaatt ctttatgtcg 300

attccaaact ttttggaatg attcgatata cgtagaatta gataaaggac catcaacaac 360

aggaaattcg ctataatctg gacgccagaa atcatagata tcttctgtca gaaatacgct 420

gtcgtcttca atcgataaaa tacgcgggtt ttgagtgatc atcatcgcaa cagcaccgac 480

accttgcgtc acttcaccac cgcttgccaa gccgtaacga gcaatatcac ttgctatgac 540

tagtactttt cgttctggat gatttttgac atattctttc gccat 585

<210> 390

<211>  300
<212>  DNA
<213>  Enterococcus faecium

<400>  390
gcatatttcg cttgatatat aggttcatac gtggtggaac aacgtatgat gttttaggaa      60

atagttgtga taaatcacgt ggtctactca catttgtaat atcataccgc ttttttgctt     120

caggagaaga agctctaata tcaatcctaa accagtattg tcagcgcgac tcataacaac     180

aagttctgtt gttaatggat caaaatttct ttctatacac tcgatactcg cataaaaagg     240

cttcatgtcg attagaaaat aatcatttac tgattctttc gaataatcca gcatgaataa     300


<210>  391
<211>  273
<212>  DNA
<213>  Enterococcus faecium

<400>  391
atatttcatc cccagctctt tttttactaa tataccaact acatttaata acaaaataac      60

tagtaaactt aatattttta gtggcataga atattcaaaa ataaataaag gcaccataca     120

tgtagctatc aatataaata cagaacttac gtattttatt attttacgga acattataac     180

ctattacaac tccgcaaata gccatagccc ataccataga taagattttt accagcacca     240

ccaccacatg tttgttttat ctctttcata ctt                                  273


<210>  392
<211>  626
<212>  DNA
<213>  Enterococcus faecium

<400>  392
agcagttccg gtatctcttt ttttctcaga atattatttc tatgtgcttt gttacaatcc      60

attttctttc aaaaaatagc atcatttata atatggttct ccgtatcgcg agcgaatgtt     120

attggctaat ctcttggcaa acaagtgttc accacaaaat tcctaactaa acaaaaaata     180

gcataaatta atgctcttag tcacagatca tactgtaaca gtatgatctt attttctgac     240

aaaataagaa taccaatcat ttatggtacg acattctaag cgtaaatgat tgatattctt     300

ttgcagaaac attcttaatt tgtacctaaa gattgctgac taaaaaatag atagaaaatt     360

ttcttcactc tatttaatac gttgcttgaa gttttatagt tatctattaa cattctcgtc     420

ccctattgtc ggggataggt ttcgattaga tgaactcgaa aacgttgcta tatcaattat     480

ggaaacatta ttctctgtcc agtgatggga caatccatac tcttccaatt agttatttgg     540

tcgattcacg ggaaaaattt tatatgcagt tcattattac tactcatctt cagactgtac     600

cgattcaaaa cattaccctt tcttca                                          626


<210>  393

<211>  508
<212>  DNA
<213>  Enterococcus faecium

<400>  393
tgaagtcctt tgtctttgtt gcttagtacg ctcgggattt cttctttttg tcaaggatga      60

aaatgatttt tcaaaggatt ttggattttc attgtatcta ttatccaaaa tgttttgaat     120

gtttaacact aatgtcataa ctaataatgg cttattgcta gcgtctatcg aagtattttt     180

tatttccttc aatatcaatg tcatagagat agacatttaa aatctgcgac attttcaccg     240

ggatttagcc catctttttc gtcaattttt ggattctttt ttagtttcta ttggaaagaa     300

tcttcaactg acataattca ttttgtattt ttatctgtcc tcttaacatt ttagtgtcaa     360

ttttaatagt gcttcacacg agaaaggtat aaacatacca ataaatttgg tatgactaat     420

gaaccttgca ctgcatagta tagccatacg cggatatact atatctctta tgttccttag     480

agtaaaacct ctaaatcggt gtgtattg                                       508


<210>  394
<211>  321
<212>  DNA
<213>  Enterococcus faecium

<400>  394
tctattaaac agacacaact tatctatggg ggtaccactc atagtggaaa atattatgga      60

aatggagtgt attgcactaa aaataaatgt acggtcgatt gggccaaggc aactacttgt     120

attgcaggaa tgtctatagg tggtttttta ggtggagcaa ttccagggaa gtgctaaaat     180

gaaaaaaaat gctaagcaaa ttgttcatga attatataat gatatatcta taagtaaaga     240

tcctaaatat tctgatattc ttgaggtttt acaaaaggta tatttaaaat tagaaaaaca     300

aaaatatgaa ttagatcccg g                                               321


<210>  395
<211>  613
<212>  DNA
<213>  Enterococcus faecium

<400>  395
ttcataagga cgatgtgttg gttagattgg attgttcttt aatagagaat gaaaaggctc      60

agatagaaca agaaaaccaa cgtattactc aacaaataaa gatggctcag ctatttattg     120

aaagtataag taaaggaaaa aatttgtttt caacggatga cagttttggc tacagtaatc     180

aattaaagag catgttgtca gaaaaagaat cactccgcta cgctttgaag caaagtgaat     240

taaatgatca aaagcaatta gaagtatacg aaaagacaaa aagacaacta gaaaaacaaa     300

ttgagagttc agatagtaaa ttacaagaat ggcaacaagt acaggtagct tggagtaata     360

atcaatcatt aaaagatttt tcaaaagaaa tgatggcaaa ctatgagaat tggcaagaac     420

aactaaataa tgtttctgat gatcaaaaaa atcaagtgaa actgacaatt tcagcaagca 480

taaatgaaca aattgagcaa ctaaaaaaag aagtagaaca gtatcagtca gaaaaagcta 540

aattagttaa accaactact tctgagaatg acagaattag tcaaacggaa aaaggaaagc 600

aagagctaga aca 613

<210> 396
<211> 400
<212> DNA
<213> Enterococcus faecium

<400> 396
attatgtgaa gatcaaatta tacaattaaa tcagttagaa cgaattattg ataatttcat 60

tctttttcac gataaagtat ttaagatagt attgaaaaca caaagtccgt tagaagttaa 120

aaaatacctc aaacaattcc gaccaaagca aggaatatat ttcttagata ttgatttaaa 180

tcatgaagtt aacggtatag aattagcaga agtaatcaga aaatatgatg ttcaagcaaa 240

aatcattttt acaactactc atgatgagat gttacccgta acaataaaaa gaagagttga 300

aacgttagga tttgtaacaa aagatcaaac actagatgag tatcgaaacg agattgttga 360

gttattgtta ttagcgcaag aaaggataga tgcaacaaaa 400

<210> 397
<211> 533
<212> DNA
<213> Enterococcus faecium

<400> 397
atcttgatct tgccattcca tttttctttt accgaaaaga ttagcttttc tagtcaagta 60

attaacaagg ggttgtttgt ttttctggat tgtatcccac atgacagaca atgtttcttt 120

cttcaaccga ttgtactcta atggtttttg tagaaaatct gtgacaccat gaagttcata 180

gtcagaaagt ctaaaaccat ctaaatgatt caaagtatcc gtgaagaggg gtgccttttc 240

tttccaggct tcttcccatg ctgcgaaaag tgtttctctg acttttggat ctggatcgcc 300

catcatctta ttgaaggctt gtccagcaga taattcgact acttgtccat cttgttcgaa 360

gggaatcgaa atgctggcta caatcgtatc ataatgactg ctccaagcat ttagaccatc 420

taaagaaagc gtatttataa tgttttcttc agcttctgat aataattgtg agccatcacg 480

acgaatctcg tttaaacgaa aagcaattgt ttcaaacgaa gattgagaaa gca 533

<210> 398
<211> 171
<212> DNA
<213> Enterococcus faecium

<400> 398
tgaatcttca gcaacagaag aatcaacaac agtgcctgaa tcttcaacaa cagaagaatc 60

aacaacacct gcgcctacaa caccatcaac agatcaaagt gttgatacag gaaacggcac 120

aggaagtagt actccggctc caacgccaac accaacacct gaacaaccaa a 171

<210> 399
<211> 519
<212> DNA
<213> Klebsiella pneumoniae

<400> 399
aggatcattt gtctcctacg gcccgcactg ggccaacgtc agcaacgccc cctacgccaa 60

ttatcacaaa accaccagcg cccagggcgg catcaatacc gactttatga tctccggtcc 120

cgggatcacc cgccacggta aaatcgacgc ctcgacgatg gcggtgtatg acgtggcgcc 180

gacgctatat gaattcgccg gcatcgatcc gaacaagtcg ctggcgaaaa agccggtgtt 240

gccgatgatc ggcgtcagct ttaagcgcta tctcaccggc gaagtacagg agccgccgcg 300

cggcaactac ggggttgaac tgcatcatca ggcggcctgg gtcgatggcg aatggaagct 360

gcgacggctg gtgccgcgcg gcctcaccgc cggcgacgcg ccgtggcagc tgtttaatct 420

gcacgacgac ccgctggaga cgcatgatgt cgcggccgaa catcccgatc gggtcaaagc 480

catgagcgag gcctacgagg catttgctaa gcgcaccat 519

<210> 400
<211> 320
<212> DNA
<213> Klebsiella pneumoniae

<400> 400
ctgatcaacg acgcatggtg ccgactgttc cgcgaacatg gctttattat cgggttgagc 60

ctcgaaggca acgaagcgct gcaggactac catcgtccgg ataaacgcgg ccggtcgacc 120

tggtcggcag cgctgcgcgg cattgacctg ctccatcagc atcaagtggg ctttaatctg 180

ctggtggtgg tgcataacga gatggcggcc cacgcggcgg cgatttatga ccggctggtc 240

agcctcggcg cgcgctatct gcagtttcag ccgctgatga gcgaaggcgc ggccctgcgc 300

gaaggatacc agctcagcgc 320

<210> 401
<211> 201
<212> DNA
<213> Klebsiella pneumoniae

<400> 401
ccgatcgagt ccattacccc ggagattgtc gacaaagtct acaacatcaa cgtcaaaggg 60

gtgatctggg gcatccaggc ggcggtcgag gcctttaaga aagagggtca cggcgggaaa 120

atcatcaacg cctgttccca ggccggccac gtcggtaacc cggagctggc ggtgtatagc 180

tcgagtaaat tcgccgtacg c 201

<210> 402
<211> 305
<212> DNA
<213> Klebsiella pneumoniae

<400> 402
gcctgcttcg ttgatagatt acctaccgcc ctttcgcaca acgaatggat ctcgatcgtg 60

gggaatctac ttgataacgc ctacaatgcc agcctgcgtc aaccgcaggg ttcaaaacag 120

atcgaatgcc tgatcaacag tgatggccag gaggtgatca ttgagatcgc cgaccaggga 180

tgcggcattg acgaggcgct gcgcgatcgg atcttcgagc gcggcgtcac cagcagcgcc 240

agcaaagatc atggtatcgg actctggcta gtacgcagct acgtggaaca agcaggcggc 300

agtat 305

<210> 403
<211> 608
<212> DNA
<213> Klebsiella pneumoniae

<400> 403
gccaccttta ttccttccgc gctggtccac tatggtctgc tgcctgacgt ggttattgaa 60

tccacgacca aattctataa atccactaac atcctctatc tctatatctg ctgcatcatt 120

gtcggcagca tcatgagtat gaaccgcacc acgctgattc agggctttct gaagatcttc 180

ttcccgatgc tgtgcggcga agtggtcggc atgctggtgg gcatcggcgt cggcacgctg 240

ctgggcatgg agccgttcca ggtgttcttc tttatcgtgc tgccgattat ggccggcggc 300

gtgggagagg gggcgatccc gctgtcaatg ggttatgccg cgctgatgca tatggagcag 360

ggcgtggccc tgggccgggt attgccgatg gtgatgcttg gcagcctgac ggcgatcgtc 420

atctccggct gcctcaacca gctcggcaag cgcttcccgc atctgaccgg cgaagggcaa 480

ctgatgccga accgcagcca tgaaacccgc agcctcagcg agagcgaagg cgtgagcggt 540

aagaccgacg ttgggaccct cgcctccggc gcgctgctgg cggtactgct gtatatgatg 600

gggatgct 608

<210> 404
<211> 490
<212> DNA
<213> Klebsiella pneumoniae

<400> 404
gtcagcatcg aggcattgct ggcggcgaaa gagcagcgtg cagcccgcca ggccgactgg 60

ttggcccatt atcagcagcc tgttatttcc ctgaccctgg tgaccccggg ggcggtgaag 120

gacagcattc gctatcgtaa tatgatgggc gttgccctcc aggcctgcga tcagctgctg 180

tggaagcacc gctggcaaac gctggatcgt caggtgctat ggctgccgac cgggccagaa 240

gcgctgtggt gcgtagcgca tccggccagc gaaatcaaag cgatgtgcag tacgctggag     300

cagatccatc cgctgggacg cctgtgggat atcgatgtaa tctgtccgca gaacgggctg     360

gtgggacgcc agtcgctggg cgaatcgcag cgccgctgcc tgctgtgcga tgagccggcg     420

cacgcctgtg cgcgcagccg tcgtcacgac accgatctcg tcgtcgcccg cgttgagcag     480

atgattgacg                                                           490

<210>  405
<211>  509
<212>  DNA
<213>  Klebsiella pneumoniae

<400>  405
gttgttctcc actacccact ggataaaggt ctcccccatc accggcgctt tgtcatcaat      60

tccggcttgc gctttgatgc gggccggcag atcggccgcc ggacgcgggg tgatgcggtc     120

caccatggtg ttcggacagg tggtattggc cgccatccag tcaatcaccg cctgtttgcc     180

ggtgagctgc aggaactcga ccataccgtc gtggaaacgc tcgccgttat ggcgcacgtt     240

atcgcagttg agcagggtca gcggcccggc gttgtcggcc atgcgctttt ccaggatccg     300

cgcgagggtg ccgtaaatgg ttttgcactc gccttgcagg tcggcctgca gatcggggtt     360

gctggtttcc agccgatggc gagtgttcag gtagtacccc ccttccgtca cggtaaaggc     420

gataactttg gtctgcgggt ttgccccttc gttaatcagc ggctgtagcc cggcctgcca     480

cggtagcagt ttctggattg aggtgatct                                      509

<210>  406
<211>  533
<212>  DNA
<213>  Klebsiella pneumoniae

<400>  406
gacttccggt ttttcacaca ccgcggcaat gggtttgccc gccgccaggc aggccagccg      60

cgccgcgccc agcgcgccgc cggtctctcc gcctttgtgg gtcaccaccg gcatagcgag     120

aatatcggcc agcagctggg cccagaacgg gctgcgggcg cccccgccca ccagcgagca     180

ctgcgcgatc ggcgtcccgc tctctttcaa tgcctgcagg ccgtcgttga tcccaaagct     240

caccccctcc agcaccgcgt agccgagctg cgcgcgcagg ctggcgtggg tcatgcccca     300

gaagatgccg cgcgcgtcag gatcgttatg cggggttcgt tccccggaga gatagggcag     360

gaagaacggc gcgttggctt tatcctcctc gcttagctcg gcaatctccg ccagcagcgc     420

cacctccgtg gtgccggtca agcggcagaa ccactgcaaa cagctggcgg cgctcagcat     480

gacgctcatc tggtgccaca ggttcggcag cacgtgacaa aacgcatgta ccg           533

<210>  407
<211>  260

<212>  DNA
<213>  Klebsiella pneumoniae

<400>  407
ccagctcgga aaacttctca cgggtggtga gattctgcat atgctgcggc gtttgaatat      60

ggcgcaggga aaccagggca atcacgcccc cggtaaggca gaaggccagc gccagccaca     120

gggtgcccat ttcgccaatg tgaggaatgg taaagctcgg aatatagctg ccgaagaccc     180

cgatgccgat ggaatacacc gcccagaacc agccgatggc cgagctggcg ttgtcgcttt     240

tgacgttatg gacaatcgcc                                                 260


<210>  408
<211>  501
<212>  DNA
<213>  Klebsiella pneumoniae

<400>  408
taacggcaaa gacgctaaaa accggcaacg tcggtgtctc ttttacgggc gatggtggtt      60

ctaatcaggg cctggtcttt gaagccatca atatggccgt cgtgctccag cttccagccg     120

tctttatttt cgagaataac ggttacggcg aaggaaccgg ccatgactac gccgtgggtg     180

ggcgtgatat cgcccggcgc gccgctggct tcggcctgcc ggcagtgacc gtcgatggca     240

ccgatttctt tgccgtttat gaggcaacct cagaggcggt caagcgtgcg cgagaaggcg     300

gtggcccaag cgtcattgag gccaaagcct tccgctggca tggtcatttt gagggcgatc     360

ccgcgctata tcgtgcggaa ggtgaagtgc aacgcctgcg tgaacaacat gatccgctga     420

agattttcac cgctaaggtc aagcaacata tcacccagga agaactggcg gcgattgacg     480

aggaagtaga agccctggtc a                                               501


<210>  409
<211>  535
<212>  DNA
<213>  Klebsiella pneumoniae

<400>  409
cctataatat ctttgccacc acgcctggac tgaaggtggt ggtgccctcg acgccttatg      60

acgtcaaggg tctgttaatc cagtccattc gcgacgacga cccggtggta ttctgcgagc     120

ataaaatgct gtacgacctc aagggcgagg taccggacga gatctatacc atcccgctag     180

gtgtagccaa ctacactcgc gaaggggagg acgtcaccat cattgcgttg tcggcaatgg     240

tacataaagc aaaccaggtg gcggacaaac tggccagaga ggggatctcg gtcgaggtgg     300

tcgacccgcg aaccatttcg ccgctggatg aggaaggtat tctggaatcg gtggcgtcca     360

cggggcgggt cgtgattgtc gacgaatccg ctgcacgctt cggttttgct catgatgtcg     420

cggcgctgat tgcgtcccag gcattccatt tcctcaaagc gcccgttctg ctggtgacgc     480

cgccacacac gccggtcccg ttctcccctg ctctcgaaaa actctggatc cctgg          535

<210> 410
<211> 543
<212> DNA
<213> Klebsiella pneumoniae

<400> 410
gcttgaaatg ccaaagtggg ggctttccat ggaggaaggc ttgctcgctc gatgggcaat 60

ccaggagggt gacgatttca ccagagggca ggaaatatgt gagattgaaa ccagtaaaat 120

cgtcaatgtg ctggaggccc cctttgccgg tacgttacgt cggatactcg cccgcgaggg 180

tgagacgctt caggtaggcg ccgtgctggc cctggcggct gacgcgtcgg tcagcgatgc 240

tgaactggac gaatttgttg cccgcctggc gacggcgaaa cccgcagccc caggcccgga 300

ggctgccgcg ccggacgtag cggcacaggc aggcgctaag ccagcttccg ttgtttcgcc 360

gccatccaac agccccgagc cccctgttgg gcagaccgtc atccccgtca gtctgcaagg 420

tgtgaccgat gtgactcagg ttaatgccac gccccatgcg ttacgactgt ctgcccgctg 480

gggtgtcgac ctgaaaaaag tcgcggcagc gggcgcgggg atcgtatctc tgtttctgat 540

ctg 543

<210> 411
<211> 596
<212> DNA
<213> Klebsiella pneumoniae

<400> 411
cagtcaggaa cacagcattg tcgatatcag catatccgga tgaatcaggg cgtgggagag 60

cattatgaga tccctgtccc ctggtttctg ctgacccatc cggatgggtt tacgctgatt 120

gacggcggtc tggctgtcga aggattgaaa gatcccagcg gttattgggg aagtactgta 180

gagcagttta aaccggtgat gtcagaagaa cagggttgcg tggaacaact taagaggatt 240

ggcattgctc ctgaggatat ccgctatgtg gtcctgtccc atttgcactc tgatcatacg 300

ggagcaattg gtcgcttccc ccatgctacg catgttgtcc agaggcaaga gtatgaatat 360

gcctttgccc ctgactggtt tacttcggga gcctattgcc gacgcgattt cgatcgtccc 420

caacttaact ggctatttct gaacgggttg tccgatgatc actatgacct ttacggtgat 480

ggcacgttac aatgtatttt caccccaggg cattcaccgg gccatcaatc ttttcttatc 540

cgcttaccgg gtggtacaaa ttttacgcta gcgattgatg cggcttatac cttaga 596

<210> 412
<211> 693
<212> DNA
<213> Klebsiella pneumoniae

<400> 412
ccgttaccga tgttgattct gagccgcagc ccggccatct ggctgtggca aacgctgctc 60

tatcaggtga gtcatccgga tcgtctgcgc aacgtccata ctgcccccgc cgatctgtcc    120

tgcgcggagc tggcccatcg gctggagaat gcgccgcggc ttgagcggct tgccggcgaa    180

gccgccctga tccacggaaa acgggtcgtc gggttgaccc acgccgagct caaggtgatc    240

ctcgccctgc tgcaagggca gacgataggc gagcaggccc aacgtctcgg attgagccag    300

aaaacgctct acacccagcg gctggctggg gtgaaaaagc tggtggaatg tcatccgcat    360

ctggcccccc gctttccgcg cacgctgctg ccgcgctcac ccgcaaacgc actgacggcg    420

tttgaacagg aatgggtaca agcgattcac gatcgccagg tcttcccggt ttttcaacct    480

atcgtcgata gtcgctcaca gctacagggg gtggagatcc tgatccgctg gcgccaccgc    540

ggccaggtac ttcaccccca gacctttctg ccgcacttcc gcgccgacta cacctggctg    600

ctgcttacgg cctttgttct gcaggaggcc gtgcagaata ttaatgagta tccaggcacc    660

ttctattttt cggtcaacat accctcctca ctc                                693

<210>  413
<211>  514
<212>  DNA
<213>  Klebsiella pneumoniae

<400>  413
ccgatcatga gaacatcagt attgaactgc agcgtgagtt ccttcctgag gaacgtgaag     60

attacgctca tgtcttctat agcggccctc ttgacgcctt ctattcgtac cagtacggtc    120

ggttaggcta ccgcactctg gatttcgaaa aatttaccta tcaaggtgac tatcaggggt    180

gcgctgtgat gaattattgc tccatcgatg tgccatatac acgcatcact gagcataagt    240

atttttctcc atgggaaagc catgaaggtt cggtctgcta taaagaatac agtcgcgctt    300

gcggcgagaa tgatattcct tattacccca ttcgacagat gggggagatg gctttactgg    360

aaaaatatct ttctcttgcc gaaagtgaaa aaaatattac cttcgtcggt cggttaggta    420

cctatcggta tcttgatatg gatgtaacca ttgcggaagc gctgaaaaca gccgatgagt    480

ttttatcttc ggtggctaac caggaagaga tgcc                                514

<210>  414
<211>  584
<212>  DNA
<213>  Klebsiella pneumoniae

<400>  414
agagatgggc tgcaaactgc tgcagcgtac cacccgcaag ctgcgtctta gcgatgccgg     60

ggaaacgata tatcagcatg cccagcagat gctggaagcg gcgcgacagg caatggattc    120

cgcaggcagt cgccaaacgg tcgcccaggg aaagctgacg ctaagcgtcc cgaaagccgt    180

cggccgcttt gtgatccacc cgctgatgat ggcgtttttc caccgctacc cgcaggtgga    240

cgtctgcctg cggctggaag atcgccctct cgattttatc gatgacggta ttgatctggc 300

gctacgcatc accgataccc cctcccccgg cctgcatggc aaaccgctga tgccaatcag 360

gcacgttatc tgcgccactg aggcctattt acagcagcac ggtacgccgt acacgccgca 420

ggatctgcgc gcgcatagct gcattagcct tggcgaaacg cccgccgatg cgcgctggaa 480

gttccgtcgg gaaggcaaaa cagaaacggt gcaaacctac gggcggtacg ccgccaacca 540

taccgccgta cgcctcgacg cggtcagaca gcatttaggg atcg 584

<210> 415
<211> 281
<212> DNA
<213> Klebsiella pneumoniae

<400> 415
acagattaca ttgtcatttc ctgccagccg cgccctgagc ggccgagcgc tggcaggagt 60

cgtgggttca ggcgatatgg aagtacttta taccgccgca cagagcgcca cgctcaacgt 120

acagatcacc acctcagtgg ataacagcca ggcgcgctgg caggcgctgt tcgacaggtt 180

gaacctgatc aacggcctgc ccgccgggca gttgattatc cacgacttcg gcgccacgcc 240

gggcgtcgcc cgtattcgta ttgaacaggt ttttgaggag g 281

<210> 416
<211> 656
<212> DNA
<213> Klebsiella pneumoniae

<400> 416
atggattttg ccttacccgc cacgctgttt agcgcgacgg tacaaacgcc gtgggccggg 60

atcgtcgccc agtcgccgct ggtgctggtg ttgaccggcg cgatgtggat cacctatgcc 120

gcgatctact tcctcgccac cagcgtgttc aaacgcacgc cgcaggatgc cgcggtgctg 180

accctcaccg tcgccctgcc aaactatgcc gcgttaggtc tgccgatcct cggcagcgtg 240

ctgggtgaag gcgcgtcaac ctcactgtcg gtagcggtct ctatcgcctg cggctcggta 300

ctgatgaccc cgttctgcct gctgattctg gagcgtgaaa aagcccgcgc cgcgggtgaa 360

aacagcggtt ctacgctggc aatgctgccg gtgctgatgt ggcgttcggt gaaaaaaccg 420

atcgtctggg gcccgctgct tggggtggtg ctttccgcga tcggcattaa aatgccggac 480

ctgctgctgg cgtcgatcaa accgctgggc ctggccgcca ccgccgccgc gctgttcctc 540

accggggtga tcctgtcggc gcgtaaactg cagctcaatg cgctgatcgc tacatcaacc 600

atcgtgaaac tgctggtgca gccgtttatt gcctggggtc tggtgatgtt acttgg 656

<210> 417
<211> 456
<212> DNA
<213> Klebsiella pneumoniae

<400>  417
tatttacctt tcccggtcag ggcggccagc gtcccggcat gctggcgatg atccccgatc      60

gcgaggcgat cctcacccag gcgcgcgccg tgctggggga tgaagtcgat accctcgata     120

gcgccgatgc gctacaacac acccgtgcgg tccagctctg tctgctgatc gccggtgtcg     180

cctgggcgcg cgagctacag cgtcagggcg tggatccgca gatggtcagc ggcctctcta     240

tcggcgcgtt tccggccgcg gtgattgccg gcgcgctcga tttcgccagc gcgctgcggc     300

tggtagccct gcgcggggac ttaatggaac aggcgtatcc tgaaggttac ggactgacgg     360

cgattatggg cctgacccgc ccgcgggttg aggcgctgat gcagggcaac gaggtttatc     420

tcgccaatct gaacgccgaa acgcagttcg tgattg                               456


<210>  418
<211>  537
<212>  DNA
<213>  Klebsiella pneumoniae

<400>  418
tgctgctgat accaatgtag gcggcggtca ggttaatttc ttcggtaaag ttaccgacgt      60

atcttgtact gtttccgtaa acggccaggg cagcgatgcg aacgtttatc tgtcaccagt     120

gactttaacg gaagttaaag ctgccgcggc ggatacctat ctgaaaccga aatctttcac     180

catcgatgtt tctgactgcc aggcggctga tggcaccaaa caggatgatg tgagcaaact     240

gggtgtgaac tggaccggcg gtaacctgct ggcgggcgca accgctaaac agcagggcta     300

cctggctaac accgaagccg ccggcgcgca gaatatccag ctggttctct ccaccgataa     360

cgccaccgcg ctgaccaaca aaatcatccc gggcgacagc acccagccta aagcggccgg     420

tgatgcctct gccgttcagg atggcgcgcg cttcacttac tacgtcggct atgcgaccag     480

caccccgacc acggttacca ccggtgtggt taacagctac gcgacttacg aaattac        537


<210>  419
<211>  554
<212>  DNA
<213>  Klebsiella pneumoniae

<400>  419
cgcaatacca taccttcacc gcccacgatg ccgtggctta cgcgcaacag ttcgccggca      60

tcgacaaccc atctgagctg gtcagcgcgc aggaagtggg cgatggcaac ctcaatctgg     120

tgtttaaagt gttcgatcgt cagggcgtca gccgggcgat cgtcaaacag gccctgccct     180

acgtgcgctg cgtcggcgaa tcctggccgc tgaccctcga ccgcgcccgt ctcgaagcgc     240

agaccctggt cgcccactat cagcacagcc cgcagcacac ggtaaaaatc catcactttg     300

atcccgagct ggcggtgatg gtgatggaag atctttccga ccaccgcatc tggcgcggag     360

agcttatcgc taacgtctac tatccccagg cggcccgcca gcttggcgac tatctggcgc     420

aggtgttgtt ccacaccagc gatttctacc tccatcccca cgagaaaaag gcgcaggtgg 480

cgcagtttat taacccggcg atgtgcgaga tcaccgagga tctgttcttt aacgacccgt 540

atcagatcca cgag 554

<210> 420
<211> 220
<212> DNA
<213> Klebsiella pneumoniae

<400> 420
gtgcgtttaa tctcctcaag ccagctcgcc agacgcgctt cggtctggtc gaactggtta 60

tcctgatcca gcaccagccc aacaaagcgg tcgccttcca gcgccgagga cgcgctgaat 120

tcataaccct catttggcca gctgccaatc atctgcgcgc cgcgcgcgct cagggcgtcg 180

aacagcgggc gcatcccgct gacgaagttg tccggatagc 220

<210> 421
<211> 341
<212> DNA
<213> Klebsiella pneumoniae

<400> 421
aaattgccga agctcaatct ggtgaccggc tttgaaacct atctcggcaa cttccgcgta 60

ttaaagcgga tgatggaaca gatggcggtg ccgtgcagcc tgctctccga tccgtcggaa 120

gttctcgaca cgcccgccga cggtcactat cggatgtatt ccggcggcac cacgcagcag 180

gagatgaaag aggcccctga cgccatcgat acgctgctcc tgcagccgtg gcagctgctg 240

aagagcaaaa aagtggtgca ggagatgtgg aaccagcccg ccaccgaggt cgccattccg 300

ctggggctgg ccgccaccga tgaactgctg atgaccgtca g 341

<210> 422
<211> 400
<212> DNA
<213> Klebsiella pneumoniae

<400> 422
agagagcgtc attgagcagt gggtgccgcc ggcgccgcgc ccggctcagc gcaatcgccg 60

ggtcaatctg ctggtcagcc atctctgttc gccgggcgat atcgagtggc tgcgccgatg 120

cgtcgaagcc tttggtctgc agccgataat cctgccggac ctggcgcaat cgatggacgg 180

ccacctggcg cagggcgatt tctcgccgct gacccagggc gggacgccgc tgcgccagat 240

agagcagatg gggcaaagcc tgtgcagctt cgccattggc gtctcccttc atcgcgcctc 300

atcgctgctg gccccgcgct gccgcggcga ggttatcgcc ctgccgcacc tgatgaccct 360

cgaacgctgc gacgccttta ttcatcaact ggcgaaaatt 400

<210> 423
<211> 536
<212> DNA
<213> Klebsiella pneumoniae

<400> 423
acagggttga tcctcgtcga cattacgatg cgctttcctc ctggcgccag ttttcatacc 60

ttcacccatg ccctgctcgg acccgctgcc agcgtaatca tcggcgtggc tttctgtttc 120

gtactttatt tactgaccta cgcgtacata tcaggcgcag catcgatagt gtgggatctc 180

cttcctcccg atattgctgg ccgcagctgg ctgccgatca ttttgctgtc gctgacgacc 240

tcgctgattc tgtgggccgg cggcaaattg cccggttttc tcctctccgg ccttatcgcc 300

gccaaattca ccctttttct cctgctgttc gccggtgccg caggaggcgt aaaagtactc 360

agattactcg acttcgccgg cagcacgccg ctccagtatt acctgccgat cgtaccggtc 420

tgcgttatcg cttttggatt tcatggcagc gtcccctctc tgacaagaat gtaccggggg 480

gataatcatc gtgcggtcct ccgctctctc tattacggtt tcgccgtttc attaac 536

<210> 424
<211> 282
<212> DNA
<213> Klebsiella pneumoniae

<400> 424
aaaagacaag ctgttgctgt ttaccgccgc gctggtggcg gagcgtcgcc tggcccgcgg 60

cctgaagctc aactatccgg agtccgtggc cctgatcagc gcctttatta tggaaggcgc 120

tcgggacggc aaaagcgtgg cctcgctgat ggaggaaggc cgacatgtcc tgacccgcaa 180

gcaggtgatg gagggcgtcc cggaaatgat cccggatatc caggtcgaag ccaccttccc 240

ggacggctcg aagctggtca ccgttcacaa cccgattatc tg 282

<210> 425
<211> 587
<212> DNA
<213> Klebsiella pneumoniae

<400> 425
atttcataaa ctcggattgg tattttgatt tgcattggac cgaccgagca atagccgctc 60

gtgatgctgg ttatgagatt cacatcatta gtcattttgt tgatagtaaa ataaccaata 120

aattcaaatc gttagggttt atctgtcata acgttccgct tgctgcccag tcattcaacg 180

tatttacttt tattcgagca ttctttgatt ctcggaaaat aattaaagaa atagacccgg 240

atctgctgca ctgcatcact ataaaacctt gtctaattgg cgggttcttt gcgaaaaaaa 300

cgcagcgtcc agttattttg agctttgttg gccttggtcg ggtgttttcg gaaaattccg 360

ggcttattaa actactacgg cattttacaa ttaaagcata caaacatatt gcgagtaata 420

aacgcagtat gtatatgttt gagcatgata aagatagaag gaaaattgtt gattttctcg 480

gtattgatat ccagaaaacc attgtcattg atggtgccgg tatcaacccg gaaatatata    540

aatattcgtt ggaacaaaag cgagatatcc ctgtagtgct gtttgcc                  587


<210>  426
<211>  320
<212>  DNA
<213>  Klebsiella pneumoniae

<400>  426
aggttcaggt agctggaaaa acagtaagtc aagtacgaca agatattaca agccgattaa     60

ccacatatat tgaaagccct caagttgatg tcagcatagc tgcattccgg tcacaaaagg    120

tttatgtaac tggtgaagtt gcaaactctg gaaaacaggc tattacaaat attcccctaa    180

ctgtgatgga tgctatcaat gcggcaggag ggcttgcggc tgatgctgac tggagaaacg    240

ttgttcttac tcataacggt aaagatacaa agatttcatt atatgcacta atgcagaaag    300

gagatctaac ccagaatcat                                                320


<210>  427
<211>  280
<212>  DNA
<213>  Klebsiella pneumoniae

<400>  427
tgattcaatt ttagtgatct gcacaggaaa tatctgccgt tctccaattg gtgagcgttt     60

attaagacgg ctattaccaa gcaaaaagat taattccgct ggggttgggg cattggttga    120

tcatgcagca gatgaatccg caattcgcgt cgctgaaaaa aatggtcttt gtctcaaagg    180

ccaccgtggg acaaaattta cctctgcatt agctcgacag tatgatcttt tactcgtgat    240

ggaatattct catctagaac aaattagccg gatagcacct                          280


<210>  428
<211>  200
<212>  DNA
<213>  Klebsiella pneumoniae

<400>  428
acatgatccc ggagaaattt agctggatta ttacttataa ccctctggcg agtatgatac     60

ttagctggcg tgagctattc atgaatgggg ttttaaacta tgaatatatc tccatactct    120

atattacagg ctttatcctg accatcgtcg gcttggccat ctttaataaa ttaaaatatc    180

gatttgcaga gattttgtaa                                                200


<210>  429
<211>  387
<212>  DNA
<213>  Klebsiella pneumoniae

<400>  429

tggaaccagt gatcaatttc agtaacgtta cgaaagaata tcctctttac catcatattg 60

gttcaggtat taaagactta gtctttcatc ccaagcgagc ttttcagctg cttaaaggga 120

ggaagtatct cgcgatcgag gatatctcat ttaccgtcgc caaaggtgag gcagttgcgc 180

tgattgggcg aaacggcgca ggtaaaagca cttcgttagg actagtcgct ggcgtaataa 240

agccaacaaa aggctcggtg actactcatg gccgagttgc ttcgatgctg gaactcggcg 300

gtggttttca tccagagtta acgggtcgtg aaaatattta tcttaatgcc acccttctcg 360

ggctgcggcg gaaggaagtt cagcagc 387

<210> 430
<211> 225
<212> DNA
<213> Klebsiella pneumoniae

<400> 430
ggtgtcatcc caaacctgct gggtcagcgc cacgttaccg ttcagcttct ggccgtagct 60

cggcacaata gcgtgaatgc ggctctgcca ctccggcgag ttaaactgct gcgggaacat 120

ctgcttgagc acgttcaggg tgattggcgc ggcggtggaa gcccccggcg aagcgccgag 180

cagcgcggaa atggttttct gctgatcgac caccacttcg gtacc 225

<210> 431
<211> 690
<212> DNA
<213> Klebsiella pneumoniae

<400> 431
cctgctgcta ttgctgtcgc tggtacgcca ggaaaaccgc caggcgctgg ccggggtgtt 60

acgcgagcag tggcagacct ggacgctgct ggcggctttc tttatctatt acgccctcag 120

taatgtgtgg ggccatacgc cgcagcatat tgactcgccg atcacccacg gcgtgtatct 180

gaccgggtat ctgttgctga tgacgatgct gctcagggac ggacgaaccc gccgactggc 240

gatgctggcg gtggtcggcg ggatcaccgt gctctccctg tggacgctga ttatcgacca 300

tacgctggtt ctcaccgaac gagcggtctc ccccgagaac cccggaccaa cgaacgttat 360

cgaccttgcc ggttactgcg gcatcggcat tttaatctgc ggcatgctac tgaaagaaaa 420

agccagccac tggctctatc tgccggtggt catcatgctg gtgatgctgc tgctcaccca 480

aagccgcggg ccgatcatcg ccctggtgct ggcggtcggc tgtacgctgc acctgcacgt 540

cttcacccgc cgcaacctgc tgatcgccgc ggcgctggcc gtgctggtag cgctgctttt 600

ggtcatgacg ccggtgggcg acatgctgct cgcccgtttc gaggagctgg gcacccaaag 660

cgggctgcgc ctgagcatct ggcaccatac 690

<210> 432
<211> 211

<212>  DNA
<213>  Klebsiella pneumoniae

<400>  432
aatttaacct ggtttgataa gaaaactgaa gagtttaaag gggaagagta ttctaaagac      60

tttggtgatg atggttctgt cattgaaagt cttgggatgc ctttaaagga taatattaac     120

aatggttgtt ttgatgtgaa aaatgagtgg gtttcattat tgcaacccta ctttaaacat     180

aaaatcaatc tttctgatag ttcatatttt g                                    211


<210>  433
<211>  326
<212>  DNA
<213>  Klebsiella pneumoniae

<400>  433
ggggagaata tccttgtctt taaacgcgcg ctgggggtga ccaccgggat cctgccgtgg      60

aacttcccgt tctttcttat cgcccgcaag ctggcgccgg ccctgatcac tggaaatacc     120

atcgtcatta agcccagcga atttacgccc aataatgcca tcgcctttgc cgagattgtc     180

catcaggttg ggttgccgaa aggggtcttt aaccttgtgc ttggccgcgg agaaaccgtt     240

ggccaggagc tagccggcaa tccgaaggtg gcgatggtca gcatgaccgg cagcgtggcg     300

gcgggagaaa aaattatggc cgctgc                                          326


<210>  434
<211>  465
<212>  DNA
<213>  Klebsiella pneumoniae

<400>  434
gactcgcggg tgattaacac cgggcaggtg tgtaactgcg tcgagcgggt ctatgttcag      60

cagggaatat acgaccgctt cgtcaaccgc ctcggtgagg cgatgaaggc cgtccagttt     120

ggcgacccgg cgacgcgaga tgacatcgcg atggggccgc tgatcaacgc ggcggcgcgg     180

gaccaggtgg cgggcaaagt gcgaagcggt ggcgcagggg gcgcgggtgg cgctggcggt     240

cagccgctgg agggcaaagg ctatttttat ccgccgaccc tgctgctgga tgtacgtcag     300

gagatggaca ttatccatga ggaaaccttc ggtccggtgc tgccggtggt ggccttttcg     360

accctcgatg aggcgctggc gacggccaat gacagcgatt atggcctgac ctcctcaatc     420

tatacccgcg atctgaacgt ggcgatgaaa gcgattaagg gactg                     465


<210>  435
<211>  465
<212>  DNA
<213>  Klebsiella pneumoniae

<400>  435
atgaaacgac ctgattgcat tcgccactgg cgcgaactgg aagggcccga cgatgccact      60

tatcccgaca gcccggagcg tttttcgatt ggcgcgccgc tggggcgcgg tttacgtctc    120

aaccggttgg ggatccacca cgagcgactg ccgcccgggc ggcgcacctc gtacccgcac    180

gcggagagcg atgaggaaga gttcatctac gtgctggagg gctatccgga agtgtggata    240

aacggctatc tctggaagct ggagccgggg gacagcgtgg gttttcccgc gggtaccggt    300

atctgccaca cttttctcaa taacaccgag caggaggttc gtctgctggt ggtgggcgag    360

gccaacaaga aatacaaccg catctattat ccgctcaatc caggctatgc cgcgacgcgc    420

caggatcgtt gggttgacca tccgccgcaa ttcttcggtc cacac                    465


<210>  436
<211>  270
<212>  DNA
<213>  Klebsiella pneumoniae

<400>  436
ttgcgtatat agaagtcata ccatcgttcg taagcggcaa cattgataga ttcagatgct     60

tccagaagcc gggggatata ataaaccagt tcttcaaagg caatactgcc ttgagggata    120

tcagaacggc tcaggcgaca aagaaggtta atcgtggctc gaaggtgatg ccactgctgt    180

gccggggagg atgggagggc gttcatgctt atcggaagtt catgaggaat taaagcaagg    240

atctgatttc cactggtaga cagctcacgc                                     270


<210>  437
<211>  406
<212>  DNA
<213>  Klebsiella pneumoniae

<400>  437
gattcctgct ctgacaacca ttgttttaac catgaacgta gagtaacttc aggcacaggt     60

agcctggcat attgagatag catgtaggat agcataaaaa atattttgcc cttggatgta    120

aaaacgtttt ttaaacaaat cagaatagtt ctactctcgt tttattacca attatagctg    180

gcacgtcagc tccttgctca atgcggacct ttcgctcgat agcttgtccg ctccgcgcca    240

gaagcgaaca gtgttatgag tggccagtga taaaacgtca gcccgttgac cttgccttac    300

agcacctcaa ccacttcaaa ttcttctgcg atcaactcca tatcttcaga aaaatgacct    360

tcagagctga aaaatctctg atgctctttc ttccagtatt caaggc                   406


<210>  438
<211>  401
<212>  DNA
<213>  Klebsiella pneumoniae

<400>  438
attgacggga tatctgacca gtcggggaat taaaaaacag gaaatcgttg aggtcaacaa     60

tgctgcggat ctgcagaaac actgtacgtc gtgttgcccg gcggtggtgt ttctgaatga    120

agactgtttc gtgcatgatg atgaaagtaa tggcattatt cgccagatca ttacgcaaaa 180

cccggcgacg ctgtttgtta tctttatgtc gctggcgaac atccattttg accgctattt 240

gcgggtacgg aagaatctgc taatcagttc aaaatcgata accccaaaag accttgatgt 300

tattctggtt aattatctta aatacaaaaa caccagtgta gggcagttaa ctttaccgac 360

attgtcactg agtaaaacag aatcaaatat gctgcaaatg t 401

<210> 439
<211> 450
<212> DNA
<213> Klebsiella pneumoniae

<400> 439
cagcagcaag gtgtttaatg aggcggtggg ccgtcaggtg gaattcgtcc aggacaacca 60

ttcccagtcc cagaaacgcg tattacgcgg gttgcactat cagctggatc cgcacgctca 120

gggcaagctg gtccgctgtg tggaaggtga ggtgtttgac gtggcagtgg atatccgtcg 180

ttcatcgcct acctttggta aatgggttgg agcggtgctc agcgcagaga ataaacgtca 240

gctgtggatc ccggaagggt tcgcccacgg gtttatggcg ctgagcgaca cggtgcagtt 300

tgtctataag ggcacgaact actacgcgcc gcagtcagaa cggagtatca tttggaacga 360

tccggagata aggattgact ggccggcact gagcgactgc gtgctgtctc tgtcggagaa 420

agacctgcgg gcacatactc tggccactgc 450

<210> 440
<211> 380
<212> DNA
<213> Klebsiella pneumoniae

<400> 440
ggggagaaag agaccctcac catcattgac gaccttcttt gggcgcccac cggcgctgag 60

ctgctggcag actgcacggc gacggcaatc cgtgaaacgc tgcgtaatcc ggcgctggcc 120

ggcacgtatc acctggtggc cagcggcgaa acagctggtg cgactatgcc cgctatgtgt 180

ttgaagtggc gagagcgcac ggtgccgagc ctggcggtgc aggaagtgaa gggcattccg 240

aacgacggcc tatccgacgc cggcgaagcg tccgctcaac tcgcgcctgt cgaattaaaa 300

atccagcagg cattcggggt gactctcccg gactggcgtc agggtgtggc tcgcgtggta 360

acagaagtcc tgggcaaata 380

<210> 441
<211> 180
<212> DNA
<213> Klebsiella pneumoniae

<400> 441
agtaaattca ggctggctct ggtgcggcag aagtaccgcc cggacggcgg cgcagaacgg 60

tttgtctccc gcgcgctgga agccctcgac agcagtcatt tgcaactgaa cgtcatcacc 120

cgcgaatggc aggggccggt gaaaccggac tggcagatcc atatctgtaa cccacgtaaa 180

<210> 442
<211> 689
<212> DNA
<213> Klebsiella pneumoniae

<400> 442
tcatttgaag aacgacacag aggttcggtt gaagatatca agaaccgcct gagtttttat 60

ttacctttct tgtctcgtct gaaggatctt tatcccgaag gcgtgattgc ggatattggc 120

tgtggacgtg gtgaatggct ggaaatcctg actgaaaatg gtattgcgaa catcggcgtc 180

gatctcgatg atggcatgct ggcacgtgcc aaggaagccg ggctgaacgt gcagaaaatg 240

gattgtctgc agtttctgca aaatcaagca gaccagagtc tgatagcgtt gactggtttc 300

catattgctg agcatttgcc ctttgaggta ttgcagcagc tcgtcatgca taccttacgg 360

gtgctgaaac ctggcggttt gctaatcctc gaaacgccga acccggagaa tgtaagcgtc 420

gggacctgtt cattttatat ggatccaacg cataatcacc ctttgccgcc gccattgctt 480

gagtttttac ctattcatta tggttttaac cgggcaatta ccgttcgtct acaggaaaaa 540

gaggctctca aatccccgga cgcagcggtt aatctggtcg atgtgcttaa aggtgttagc 600

cccgattaca gcatcattgc tcagaaagca gcgcctgcag atgttcttga acgctttgaa 660

accctgttta cccaacaata tggcctgac 689

<210> 443
<211> 581
<212> DNA
<213> Klebsiella pneumoniae

<400> 443
tgcctctatt atccaacctc tgcatgtcgg taaagcaaac tcttataatg atattggctg 60

tgcaggtgat gatactggag ataatatctc gtttaaaaat ccattctact gtgagctgac 120

ggcccattac tgggtatgga aaaatgaatc tctttccgat tatgtcggct tcatgcatta 180

tcgtcgacat ttaaatttct ccacgcagca ggatcatgcg gaagataact ggggggtggt 240

gaattatccg ctaataaacc cggactacga ggcacagttt ggattaaccg atgacgctat 300

tcgtacatgc gttgagggga gtgatctttt actacctaaa aaatggtcgg taacatcggc 360

tggcagtaaa aataatctcg accactacag caagggtgag tttttacata ttaaagacta 420

caaggctgcg ctagaggttg ttgaagaact ttatccagaa tataagacag caatacagca 480

gtttaataat gccactgatg gttattatac aaacatgttt gttatgcgca aagatatgtt 540

cattgattac tcagagtggt tgtttagcat tctggatcgt c 581

<210> 444
<211> 649
<212> DNA
<213> Klebsiella pneumoniae

<400> 444
ggtttaaggc aggtagtcag catatttgtg atgcgattga tacggactgg gttttctttt 60

acgatgatga tgcttttcct gccagcgata tactggaaaa gttttttgct cttgaaaaaa 120

aggaatgtca ggtctttact ggtttagtca aagatcttca cggccaccct tgtgcaatga 180

atcttccttt caggaaagta ccttcatctt ttgctgatac tttacgttat attcgcaccc 240

cccaacgctt tgttcctacc attgacgaga gtgtcatggt tgagacagtt tcgtttgttg 300

gcatgattat tagcagcaaa gtattgcaag agcatattga tcacatccat gatgaactgt 360

ttatctattt tgatgatctt tattttggct atgcgttgac attggacggt caaaaaatcc 420

tctattcacc agaactgatt tttcatcatg atgtcagtat ccaggggaaa atcatctctc 480

cggaatggaa ggtatattat ctgtgccgaa atttaatttt ggccaggaaa ctattccagg 540

aagtaaaagt atttagcaat ttctctatcc ttatacgcct atgtaaatat ttatccatat 600

tgccatggca gcgcagaaaa tcatcatatc tgtgtttcat gtatcgtgg 649

<210> 445
<211> 606
<212> DNA
<213> Klebsiella pneumoniae

<400> 445
gtggcattgg tcgttatagt attgctatcg ccagagcgat tattagaaat aacaatcgac 60

atgaggtttt catcgcgcta tccgctatgc tgggtgagtc gattactgat gttaaggcgc 120

aatttgctga tctccagcca gcagacaaca tagtcgtctg gcatgctgca ggaccagtac 180

gtgcaatgga taaaggtaat gaatggcgtc gggagagcgc agaactgatt cgggaagcgt 240

ttcttgaatc attgcgtccg gatgtcgttt tcattacaag cttgtttgaa ggtcatgtcg 300

acgatgcggc cacttcggta cacaaattta gtcgtcagta caaagtagcc gtactgcatc 360

acgatcttat tcccctggtg caggctgaga cctatctgct ggatgatgta ttcaaatcct 420

attatttaca gaaagtggaa tggttaaaaa acgctgacct tctgctaact aactccgctt 480

atacggcaca ggaagcgatt gagcatctgc atttgcaggg cgaccatgtg cagaatattg 540

cagctgcagc cgatcctcag ttttgtatgg cggaagtgac agcgagcgag aaagagtccg 600

tccttg 606

<210> 446
<211> 450
<212> DNA
<213> Klebsiella pneumoniae

<400>  446
tgacctatca ctcggatatt gtgaaacaaa aacggttaat gaagttgtac cagccgctgc      60

aggagcgatt cctcgccagc gtagactgca tcgtcgcctc gtcgcccaac tacgtggcct     120

ccagccagac cctgaaaaaa tatcaggata aaaccgtggt gatcccgttt ggtctggagc     180

agcatgacgt gcagcacgat ccgcagcggg tggcgcactg gcgggaaacc gtcggcgata     240

acttcttcct cttcgtcggc gctttccgct actacaaagg gctgcacatt ctgctggatg     300

ccgccgaacg taaccggctg ccggtggtga tcgtcggggg cgggccgctg gatgcggaag     360

tgcggcgtga ggcgccacag cgcgggctga gcaatgtggt gtttaccggc atgctcaact     420

acgaagataa atacattctc ttccagctct                                      450


<210>  447
<211>  507
<212>  DNA
<213>  Klebsiella pneumoniae

<400>  447
ttcaggcgaa atgctatgct cgcatcgcgg acttcaaaaa gcaggggacc acgctgctgc      60

tggtttccca cagcgccggg gatatcgtca agcactgcga ccgcgccatt ttcctcaaaa     120

atggtgacat ctgcatggac ggcaccgccc gcgacgtaac caaccgttac ctggatgagc     180

tgtttggcaa accggataaa gacagcgcga caaaaagcgc aacggctatc tcgtcagcca     240

gtggcgaaag ccagatgtct ctcgatgaga ttgaagatgt gtaccacacg cgcccaggct     300

accgtccgga agaatatcgc tgggggcagg gtggcgcgaa aatcatcgat tatcatatcc     360

agagcgccgg ggttgatttt cctccctcac tgacgggcaa tcagcagacc gattttctga     420

tgaaggtcgt gtttgaatac gattttgatt gcgtggtgcc tggcatcctg attaagaccc     480

tcgatggctt attcctctac ggaacca                                         507


<210>  448
<211>  678
<212>  DNA
<213>  Klebsiella pneumoniae

<400>  448
gctatgaact gatcctggtg aacgatggtt cgacagacaa cagcctggcg gtgatcgccg      60

aatggcagga gcggctgcag aacgtccagg tgctggagca ggaaaaccag ggcgtctcgg     120

tcgcgcgcaa taccggcctc gccgccgcca gcggcaaata tctcgcgttt ccggatatcg     180

acgacaaact ctatccgggc atgtatcgca cgctgctgga gatggccgag aaagaacatc     240

tcgatatcgc cacctgcaac ggcacctatg tgtacgaaaa gcgccgcgag agccacccga     300

tcttcccact ggatcgcctg ccctcgacgg gtgtgctgcc gggccatgtc tggcttaagc     360

aggccctgga ctcgcggaag tttctgcacg tcacctggct taatatttat cgtcacgact     420

ttatccgcca gcatcacttc catttcgagc ctggcctgcg ccatcaggat atcccatgga 480

ccacagaagc cctgctggcc gcggagcgcg tgcagtacac cagtcagcag ttctatgatt 540

actacattca ctctgagtcg gtgtcgcata agccggacaa cgacgacacg ctgatgcgtt 600

cggcgcgcca ctatatgaag attctggaga tgctggaggc gattaaccag cgctacccgg 660

ataaagtacg ccatatcg 678

<210> 449
<211> 585
<212> DNA
<213> Klebsiella oxytoca

<400> 449
ctctgcctct attgctcttg ctctcacagc gcccgtacat tcatttgcag ccagcgatca 60

gcgtgggtac aaacctgaag acgtcgcttt tgatgaaagt tttttttcgt ttggtggcca 120

tgtagggact tctgttgaat atgaagataa ggtaactcgt ggtttcaata acacggataa 180

aaaggagaag acgattacca atgaggtttt caactttttt tataacaatc cacaatggaa 240

ttttatgggt ttttactctt ttaaaataga aaatagagag caaaaggagc ctggttatta 300

tgagaatgaa gatggtatta agcagctttt ttcattgaat aaaggtcatg atcttggtaa 360

cggttgggct actggtttaa tttatgagct agaatataca agaagtaaag tttattctcc 420

ggatgttagt ggtctacgta aaaaccttgc cgagcacagc attagaccat atttaaccta 480

ctggaataat gattataata tgggattcta ttctaatctt gaataccttt tgagtaaaga 540

agatcgcaat gcatggggga aaaggcaaga gcagggatat agtgc 585

<210> 450
<211> 340
<212> DNA
<213> Klebsiella oxytoca

<400> 450
tatcgatgcg gatgaaaatt gcccaactac atcgttctct gaaagaggag gggcatcctg 60

ctacaatgat ttatgttact cacgatcaga ctgaagcgtt aactctagga gatcgcattt 120

gtgttcttaa ccatgggaat atcatgcagg ttgatacacc tactgatctt tataattatc 180

ctaataataa gttcgttgcc agttttatcg gttcaccatc aattaatttg atagatactg 240

ctatccgtaa gaataatgag aggttgtatg ttgaaattgc tcctggcgtt gaaatattaa 300

ttccacatag taagcaagtg ttgcttgaag gttatattaa 340

<210> 451
<211> 608
<212> DNA
<213> Klebsiella oxytoca

<400> 451

atccaatgac cagaaatgag ctgcgtagcg cccataataa gaaaagatgc cggaaatata 60

cgcatgcttt ttccctcaga caataacata gttactcctg aaatttgatt tgctcatcaa 120

tgatattacg agcacggtca agtgctgctt ttggcgtttg gtcattgatc cacatatcgg 180

taatcgcatt tgccagtggg gaccataaat aacccatttc cggaatagat ggcatggcat 240

cagagtgaag cccttgttta ataattgcgc tcgtcgcttc atttgcagtt ggtaggattt 300

tgttcatcag attcggtacc ggaggtatag attctgtcat ctcatagcgt ttcattaaca 360

tttcatcaga tgagagatag tcagcgaaaa gttgtgccgc cttaggcgat ttactataag 420

aagagacgac cgccaggcga accgtagaaa acgaacgtgg ctgttttcct tcaagagtag 480

gtatgggaac aacgccaaaa ttaattttac tgttgttata tccctggatt gcccatggac 540

cgtcgatgat ggcagctact ttgccttcag aaaataagcc tcgacgcacc tgtggattac 600

gcatatct 608

<210> 452
<211> 589
<212> DNA
<213> Klebsiella oxytoca

<400> 452
cgtaaatatg ggacaaaggt ataaaccgtt aacgccaaga tcttgcaaat aatcaagttt 60

gttaataatg ccctgcaaat caccgcccat aaagtttttt gaatctggag gcgttcccca 120

cggttgtacg ttttctggcg atatcgatgg atcgccattg caaaatcgtt caggaaagat 180

ctgataccat attgtttttt taacccattc tggcgtagaa agtacatcac ctggattgat 240

ataagggaag caaaaaaagt tggacaagtt actcagttct gtctctgcta caggtggttt 300

acttatatca acacagcgtc gttcaccaaa taataatttt tccccgttat ttccgtataa 360

tataaaacca tagcggctac gtcgtttgca cggagtaaat gcggcaaacc agtggtcata 420

gctctcgctt tgtccctctt tttccatgtg aacttcgttg ccgccgctcc atccatgcgc 480

gtcgctgccg ccaaggtttc caccatctag gccaccttcc tcccattgat agggatcgcc 540

gatccacaga gagactttcg cgacctcgcc tttgactgtg cgaaatcta 589

<210> 453
<211> 528
<212> DNA
<213> Klebsiella oxytoca

<400> 453
gcaagggtag aggtgtattg cgccttttcc ttattagcca tcgccgcatc ataggcaaaa 60

cgatattctt cataatttaa gcgaattatt tctggtagat aattatttgg acagtgtcgg 120

cttaatacac tttttagact taacggaaag tctgagtgta ttgttgctaa tccactgagc 180

actaacaatc taggtttaaa aaccattatt ggatcaagta aggctctggc tagttgatcc 240

atccacattc ggtagacttt ttgcgcccat gcatcactgc catcaactcc ctgaattatt 300

tctaaggcac tcttccgctg aagagagaac tgaaaatact gtctttcgat cccggatgtc 360

gaaatgaatt gatgaacgca tcctatcccg cagcattcgc aaaccggaga tataccatca 420

atgagtggct gataattttt caatgggaga tgagcccagg aaacatttat tgcatggtca 480

aatacgctat catcgttgta tttatcgact acacaaagtt cacagcca 528

<210> 454
<211> 510
<212> DNA
<213> Klebsiella oxytoca

<400> 454
ataagccatg tgtttcttcc cgatgggaaa gcattagagc atttttcata tcaatcacta 60

gcatgaagcg atgtgatgga taaatctttt catctatttc aaatcgagag tacaattcga 120

tagattcatg tggtagcgcg agtcggttaa atgagaacac gataatccga accccgcgct 180

caacggcatt aatgagttct tgagcaatga gttcaagatg gaagtcagtg ttcaggtaaa 240

cttcgatttg agccagttcg agcatttctc tggctttttg tagtgaatta tcaaaaccag 300

agacgttata tatgaactct ttctcttcct gtagcatcat acgtgagagt tcttttttta 360

atacattgat gttttcaatg gtttgctttt ctatgttgct gaaaataagc tcgggagatt 420

ttgcttgata ctctttagta ttgccatcgg ccataaaaat gaagccattt ttatatagac 480

tatcaattga tgagtagacg ctagaacgtg 510

<210> 455
<211> 383
<212> DNA
<213> Klebsiella oxytoca

<400> 455
gccggtaatc ttgagctgct ggcccagggc cgtagcgtgc gcgtggatgt ggccgccggc 60

gccgaagcca tcatgaaagc ggtcgacggc tgcggcaggc tcgataacgt caccggcgaa 120

tccggcacca atatcggcgg catgctggaa cacgtgcgcc agaccatggc cgagctgacc 180

aacaagccga gcagcgaaat atttattcag gacctgctgg ccgttgatac ctcggtaccg 240

gtgagcgtta ccggcggtct ggccggggag ttctcgctgg agcaggccgt gggcatcgcc 300

tcgatggtga aatcggatcg cctgcagatg gcaatgatcg cccgcgaaat cgagcagaag 360

ctcaatatcg acgtgcagat cgg 383

<210> 456
<211> 400
<212> DNA
<213> Klebsiella oxytoca

<400>  456
cctgctctat tccgtcagga gttttgccgc cgcgatgctc gcctattacg ttgccctggc      60

gattggcctt gaacgccctc atgggcactc atcaccgtct acatcgtgtc gcaaacctcg     120

gtgggcgctc cctgtgcaga agcctttatc gcctggccgg taccgtggcc ggcgcggggg     180

ccacggtatt gattgtgccg acgtttgtga atacgccaat tctatgtagc gtgattctgg     240

ctggctggat caccttctgc ctctatttat ccctgcttga acgcacgccc cgcgcctatg     300

cctttgtgct ggccggttat accgcaagcc tgattggttt tcccgccgtc gccgatcccg     360

gcacgtgttt aacatcgccc tcatccgggt acaggaaatc                           400


<210>  457
<211>  535
<212>  DNA
<213>  Klebsiella oxytoca

<400>  457
ggctgtctgc tatggattta ctctgcctgg cccgatggcg gcacggcggt gtcgattctc      60

ggggtttgct gcacgctgtt tggcagtttc gacacgccgg ccccgcatat tgtgaaatat     120

attatcggct ctgtctgggg cgtagtgata agccttatct atagcttcgc cctgcttcct     180

ccgctcagcg atttccccgt gctggtggcg gtgcttgccc cggtctatct gcttgccgga     240

tcgctgcagg cgcggccccc cacgaccttt atggccatgg ggatcaccct gacgctgccg     300

gtactgtgcg agctgggcgc gcgctacagc ggcgacttcg ccgacgcggc caacaccgcg     360

atcgccctgt ttttcgcgac cggctttgcg gttatcggca tgagtctgct gcaaaccgta     420

caggcggacg cggcgataaa gcgtctgctg aaactgtgcc aacgcgatat tcgccgcagc     480

gtgagcggcg tatttaaagg cgatgaaacg cactggacca atctgatgat cgacc          535


<210>  458
<211>  400
<212>  DNA
<213>  Klebsiella oxytoca

<400>  458
tggcgtttat tttctggaaa cagtatgcgc agacgccctg gacgcgcgat ggccgggttc      60

gggcagatgt ggtgcagatt gcgccggatg tttccgggcc ggtgagcagc gtggcggtgc     120

gggataatca gtgggttaac cgcggcgatg tgctttatgc catcgacccg cgctggctga     180

agctggcggt gctcagcgcg caggccgacg tcgaagcaaa acgtcatgaa atgctgatgc     240

gccaggatgc cgccccgcca cgcgcgctca tcaaaggggt catttccggc gaggatatcc     300

agcaaacagg cagcgcagct gctgttcgcg gcggccaatt atcagggggc gctggctgcg     360

ctggaactgg cgcagtgaaa cttatcccat gcaacgctac                           400


<210>  459

<211>  260
<212>  DNA
<213>  Klebsiella oxytoca

<400>  459
cgttctcccc tgattcttgc cggcaccccg ggaacttaca gctatgcagg aaccggtaac      60

gtagtagcga tcgctcgcga tctggctaag atctgggatc ttcctttagc agtccacctc     120

gatcaccatg aagatctggc cgatatcacg cgcaaagtac aggccggtat ccgctcggtc     180

atgatcgacg gatcgcattc gccttttgaa gaaaacgtcg cgttagtcaa gagtgtggtt     240

gaactgagcc accgctatga                                                 260


<210>  460
<211>  456
<212>  DNA
<213>  Klebsiella oxytoca

<400>  460
cggcgcattt aaaatatcaa tcggttgatt taaatgaagt gatcacgcat tcgcttcaac      60

tggttagcca ggatgccgcc agccgggcaa tatctctgac gtttaccgcg cagcccgcgc     120

tatgccgcat ccaggccgat ccggatcgtt tgaaacaggt gctgcttaac ctttatctca     180

atgctgtcca tgccattggc cgcgagggcg tgattacggt ggcggtgagg gagtgcggcg     240

atgggcgagt caaggtgagc gttgctgaca gcggcaaggg aatgacggcg gaacagctac     300

aggccatttt cacaccgtac tttagtacca aggccgacgg caccgggctg ggcctggcgg     360

tggtgcagaa catcgttgag cagcacggcg ggacaattga cgccgagagc gcccccggca     420

agggcgcgct atttacgttc tatttgccgg ttaatg                               456


<210>  461
<211>  536
<212>  DNA
<213>  Klebsiella oxytoca

<400>  461
tattgaaggc accaccagcg acattcgctt cgtccacaac gttctgttcc cgtacgcccg      60

cgaacgcctg gccggtttcg ttaccgctca gcagtttgtc gagccggtga agaccattct     120

cgataacctg cgcgaagaga tcgcccagcc ggcggctggc gccgaagaac ttattgctac     180

cctcttcgcc tttatggatg aagaccgcaa atcgaccgcc ctcaaggcgc tgcagggcat     240

tatctggcgc gatggctacg ttcatggcga ctttaccggc cacctgtatc cggatgttct     300

gccggcgctg gaaaaatgga agtcacaggg tattgattta tatgtatatt cctcaggctc     360

cgttgctgcg cagaaattgt tatttggcta cagcgatgaa ggtgatatta ctcatctgtt     420

caacggctat ttcgataccc tggtaggtgc caagcgtgaa gcgcagtcct accgcaacat     480

tgctgagcaa ctgggacagc ctcctgccgc catcctgttc ctgtccgata ttcatc         536

<210>  462
<211>  557
<212>  DNA
<213>  Klebsiella oxytoca

<400>  462
cctggagtgt gcataagggc tggcatcgcg acggtaaact gcggatggtg ccggtcgcgc      60

cgcaacctac ccgggcgacc accgatgcgt tctatccgct gatcctcaac agcgggcgga     120

tccgcgatca atggcacacc atgacccgca ccggcgcggt gccgcgtctg atgcagcata     180

ttaacgagcc ggtggtggag gtcgcgccgg cggacgcgca gcgttatcac ctgctggaag     240

gtgaactggc gcgggtccgc tcaccgaagg gggtgatggt cgcaaaagtg acgatcggcg     300

acgggcaacg gcccgggtcg ctgtttgtgc cgatgcactg gaataatcag tttgctcgtc     360

agggacgggt gaacaacctg ctggctgcgg tcaccgaccc gcactccggg cagccggaaa     420

gtaaacagac ggcggtggcg atagccacct ggcttcctgc gtggaaaggc gagctttttt     480

cgcgccagcc ggttccgctg cccgcttcgc tgcactggcg gcggcgggcg gcgcagggca     540

ttatccatct ttcgctg                                                    557


<210>  463
<211>  231
<212>  DNA
<213>  Klebsiella oxytoca

<400>  463
acacgcatat aaaccgcaac cgccggccag cgccgataaa gcgcccggcg aaattattac      60

cctgccgccg ctgcaggtgc gcaaaaccac gcctccgctc agccgctggc tgcgcgatgt     120

tacccaacgt cttctgccgc cgctgctcgg gctgggattg ctgctgctgg gctggcagct     180

ggcggcgatg aacagcaaag gtttcccgac gccgctctcc acgctggatt c              231


<210>  464
<211>  459
<212>  DNA
<213>  Klebsiella oxytoca

<400>  464
gcgataagtt ttcgatttca cggcgacgtt tattacagac gggggcggcg ctgggcggcg      60

cgatgctgct ccccggcata atgcaggcgg cgtgggcggc tgggtcggat aaaccggaac     120

agaccaccgt gcgggtgggg tttattccgc taaccgactg cgctccctta gccattgcct     180

ccctgaaggg gttcgataaa aagtacggta tcaccctcgt gccgagcaaa gaggccagct     240

gggccgcggt gcgcgacaag ctggttgccg gagagctcga cgccgcgcac attttgtacg     300

gcatgctcta cggcctggag ctggggatcg ccagtaaacc gcaggcgatg gccaacctga     360

tgacccttaa ccgcaacggc caggcgatta cgctctccag cgagctgcag gaacagggcg     420

tcaccgacct gagcgggctg aaaaaacgga tcggtcagc 459

<210> 465
<211> 594
<212> DNA
<213> Klebsiella oxytoca

<400> 465
atgtcatggt tccgatactg tctgccgatg aaaacagcct ggtgctggtc tgggaaaaac 60

cggagtctga gaccgagcag gtggtggact acgccgtcta tcgtcaaggc gagcggctgg 120

gcctggcgcg tgaaaatcaa aaccattttt ccccggcaaa gccctatatt gataacttct 180

atcagcggat cgccagcgac ggctggcagc agaaaatcga tctgcgcagc ttcacggcca 240

ccaacctgca gccggatacg gagtatgcct ttacggtgcg cgcggtctac gccaatggcc 300

aggaatctcc ggacagcgcg gtggttaaag cgcaaacccg caaaacgccg cacgtcatcg 360

aagccagcac attcggcgcg aagggtgacg gcaccacgct gaatacccag gcgctgcagc 420

gggccattga tagctgtacc gtcacgcact atcctcaggg ctgcaaggtg ctgatttccg 480

gcggcgaatt caaaactggc gcgttgttcc tgcacagcga tatgaccctg gatattgcgg 540

ctggcgccac cctgctgggt tcggacgatc cggcccagta tccgcttgat aaag 594

<210> 466
<211> 625
<212> DNA
<213> Klebsiella oxytoca

<400> 466
aagctggaac gtactaacga cggatttatt acctcatggg cggcaacggg cagtaatgaa 60

tgggtaagcc agcgggttcc tcacgccgat ctgattgctc agcaggataa agaacattac 120

tacgtcggtt tcttcgcctc acgtaacgcc aaaatcaccg tcagcaatgc ttccctgacg 180

acctccgcgg caaatacggt tccctccgcc ccgtatgttg ccaaaagctg gccgccggtc 240

atgcaaattg cctcggggac aaaaagccag agcaaagagt atctcctgca ggcgcgcacg 300

aatagtgacg gacgcatcac cgtgcgtcag gatgaagtgg tgatcggcca ggataaagcc 360

gtgaaggccg gagagatgta tacccagcct gccgttctga aagataaaag cacattcgaa 420

attagcttca ctccagccac cggcgcaaac acgctgaccc aaacgctgac ggttgaacag 480

agcgccaatg tgacaggcaa tacgctgtac gccgcgccgg atgggctgtc gcaggctaaa 540

gggacgacgg actcgccgct ggatttagcc accgctgtcg acctcgttcc ccctggcggg 600

caaattgtat tagccgcagt gatta 625

<210> 467
<211> 503
<212> DNA
<213> Klebsiella oxytoca

<400> 467
acaggatagc gaacacctcg atattctacg ccagcttacc catgccatga gcgacgagcg 60

cgtacctgaa gcgtatcagc gcacccccag agctccgcag gcggtgctgg agattctggc 120

cgggatatct ctcctgccga gggggaagat atggaccgcc tccggtgata cgatgaagag 180

gcgacgttta ccctacgcga atccccacgg actgcacgcg cggccaagcg cggtcctggt 240

gaaagcggtg aagcagtggc gatcgcaaat tcgggtggaa aatctcgaca cccgttccgc 300

tattgttgac gccaaaaatc tgatgcgggt cgtttctctc ggcgcaaagc aggggcatcg 360

gctgcatttt atggccagcg gggaagatgc ccatcaggcg ctggaggcta tcggtacggc 420

ctttaatgcc ggattaggcg aaattgccgc acagccgcag caggtcgttc agccagcaga 480

aaagcctaaa cggagctggc ttt 503


<210> 468
<211> 534
<212> DNA
<213> Klebsiella oxytoca

<400> 468
atccataccc tgacactcaa tacggcaatc gatatgaata tgttttgcga tccgctgaag 60

ccgtcggcag tgaaccgaac ccgacacacg gaatattgcc caaatggtaa aggagtgaac 120

gtatcgctga tattaaatca ttatcagcag cccactcaca ttataggtat tttcggtggt 180

ttcactggcc gttatattgt ggaagagtta cgtcagaaaa aaattaaagt gacgccggca 240

tgggtctctg agccacccag aattaatatt tttattaatg acggcgctga ggaatataag 300

ctcgttaatc ctggagcaaa aattgatgat gagtgtaaac agcaggttat tcatcatctg 360

caatgcgtcg cctctggtga ttatttagcg atcagcggca gcctgccccc ggggattgaa 420

agccgatttt atgctgaaat tattgaatta tgccagcaga aaaggtgtga agttatcctc 480

gatatcagcc atccggtcct gcgccagctg cttgaattac ggcctttgtt gatc 534


<210> 469
<211> 599
<212> DNA
<213> Klebsiella oxytoca

<400> 469
gcttcaggtg ttgaaaatgc gattacgccc gcggatttaa aagatattta tggcgttatt 60

attgccgctg ataaagacgt taacgccgag cgatttaatg gtctgccggt cattgaagtt 120

ccggttaaag aagccattca ccatccggcc gacttaatta ataaatttat cagcggccag 180

gcggcgcgtc gtcagggtat ttctgcctcc gccgattcaa cggagaaatc cgagcgggag 240

tttttcgggc ccaaggtata taagcacctg atgagcggcg tctctaacat gctgccgttt 300

gttgtcgccg gagggatttt gattgccatc tccttcctgt ggggcatcta ctccgccgat 360

ccaaactcgc cgcaatataa cgttatcgcc gccacgctaa tgaaggtggg gtcaacaggg 420

ctttctcaat tcatggtgcg gattttcacg gcttatatgg cctggtctaa ttccgggcgt 480

cccggtaatg gtgcgcgggc tttgtcggtg ggctataagc caaacgcaac cgcgcgacag 540

gcttttctcg gcgggattat cgccgggtct cgccgccggg gttattttat gctgctgct 599

<210> 470
<211> 675
<212> DNA
<213> Pseudomonas aeruginosa

<400> 470
caagcacaac aagaaatacg tcgtcgccct ggaccagggc accaccagct cccgcgccat 60

cgtcttcgac cgcgatgcca acgtggtcag ccaggcccag cgcgagttcg cccagttcta 120

tccgcaggcc ggctgggtcg agcacgaccc gatggaaatc tgggccacgc agagttcgac 180

cctggtcgag gccctcgccc aggccagcat cgagcgcgac caggtggccg ccatcggtat 240

caccaaccag cgcgagacca cggtggtctg ggaccgtcac agcggtcggc cgatccacaa 300

cgtcatcgtc tggcagcgcc ggcgcagcgc ggcgatctgc gcgcagctca agcgcgacgg 360

gctggaagac tacatccgcg aaaccaccgg gctggtcacc gatccgtact tctccgggac 420

caagctgaag tggatcctcg acaacgtcga aggcgcccgc gaacgcgcgc gcaacggcga 480

cctgttgttc ggcaccatcg acacctggct gatctggaag ctcaccgaag gcaaggtcca 540

cgtcaccgac tacaccaatg cctcgcggac catgctgttc aatatccaca gccgcgactg 600

ggacgcacgg atgctcgagg tgctcgacat tccccgctcg atgctacccg aggtgcgcaa 660

ctcttcggag gtcta 675

<210> 471
<211> 630
<212> DNA
<213> Pseudomonas aeruginosa

<400> 471
gagcgacctt ggattctcga agatcctgtt cggcctgttg cctaaggaca gccaggacta 60

cgagaacgcc ttcatcgtcg gcaactaccc ggccgcctgg cgcgagcatt acgaccgggc 120

tggctacgcg cgggtcgacc cgacggtcag tcactgtacc cagagcgtac tgccgatttt 180

ctgggaaccg tccatctacc agacgcgaaa gcagcacgag ttcttcgagg aagcctcggc 240

cgccggcctg gtgtatgggc tgaccatgcc gctgcatggt gctcgcggcg aactcggcgc 300

gctgagcctc agcgtggaag cggaaaaccg ggccgaggcc aaccgtttca tggagtcggt 360

cctgccgacc ctgtggatgc tcaaggacta cgcactgcag agcggtgccg gactggcctt 420

cgaacatccg gtcagcaaac cggtggttct gaccagccgg gagaaggaag tgttgcagtg 480

gtgcgccatc ggcaagacca gttgggagat atcggttatc tgcaactgct cggaagccaa 540

tgtgaacttc catatgggaa atattcggcg gaagttcggt gtgacctccc gccgcgtagc 600

ggccattatg gccgttaatt tgggtcttat 630

<210> 472
<211> 324
<212> DNA
<213> Pseudomonas aeruginosa

<400> 472
atggatgctc gggtactgcc gcgcgctttc atcgatgaag gtacgcaggc gcttcttctg 60

ccccagcggg ctgatcccgc ccaccaggta gccggtggcc cgctgcgccg cctgcggatc 120

ggccatgtcg gccttcttcg cccccgccgc atgggccagg gccttcaggt cgagactgcc 180

gatcaccggc accaccgcca ccagcaactc gcccttctcc gtggcggcga gcagcgtctt 240

gaacacccgc tgcgggtcca ggccgagctt ttccgcggcc tccaggccat aggaaggtgc 300

cttggggtcg tggctgtagc tgag 324

<210> 473
<211> 669
<212> DNA
<213> Pseudomonas aeruginosa

<400> 473
gatcgtctct gcccagtcca tcaccttgcc caagggcggc gacgtgcacc tggtgccgcc 60

tccgcccaag ccttgcgtga ccatcgtggt gcatggcgtc aacgatctcg cgggttgcta 120

cgaacggatc gagcgagggc tctgccaggg gctcaatgaa cgcctggaca tgccgccgac 180

cttgcccggc gggcaggcca atcccggcta cctgacgccg gcgggctaca gcctgccggc 240

ggacgacgaa ggcaaggcag agaaccccga cgtcgtctac taccggcgca agttcgccag 300

tggcgccggc ggggccgccg tacgcagcgt agtcgtacct ttctactggg gcttccgcga 360

ggaagagcaa tacatcaaca agaccgcggc ccacggcgaa tggctggacc gcaacggcaa 420

ccggctggac aagtccggca ccaaggaagg cgggcagttc gtcaatgcca ccaccaacct 480

gccggacatg tggggccagg gtttcaacgg caagctgttc ggtttcatct cgctggactg 540

gttcggcggc accatgaccc atccgctgtt ttcggcggca gggcgcaagt acatggtcct 600

tgcggccatg cgcctggcca tgttgatcaa gatcatccgc aagcgttacc cggacgacac 660

catcaatgt 669

<210> 474
<211> 810
<212> DNA
<213> Pseudomonas aeruginosa

<400> 474

aggagagaac atgagtcgct cacccatccc tcgccaccga gcgttgctgg ccggtttctg 60

cctggctggc gcgctgtccg cccaggctgc cacccaggaa gaaatcctcg atgcggcact 120

ggtcagcggg gattcctcgc aactgaccga cagccacctg gtcgccctgc gcctgcagca 180

gcaggtcgag cgcatccgcc agacccgcac ccagttgctc gacggtctct accagaacct 240

cagccaagcc tatgatcctg gcgccgccag catgtgggtc ctgccggcca acccggacaa 300

taccctgccc ttcctcatcg gcgacaaggg gcgcgtgctc gccagcctga gcctggaggc 360

cggcggccgc gggctggcct atggcaccaa cgtgctcacc cagttgagcg ggaccaatgc 420

cgcccacgcg ccgttgctga agcgggcggt gcagtggctg gtgaacggcg acccgggcgc 480

ggccactgcg aaggacttca aggtcagcgt ggtcggggtg gacaagaccg ccgccctcaa 540

cggcctgaag agcgccggcc tgcaaccggc ggacgccgcc tgcaacgcgc tgaccgacgc 600

cagttgcgcc agcaccagca aattgctggt actgggcaac ggcgccagcg ccgctagcct 660

gagcgccacg gtgcgcgcac ggctacaggc cgggctgccg atcctcttcg tgcacaccaa 720

tggctggaac cagagcagca ccggccagca gatcctcgcc ggcctgggcc tgcaggaagg 780

cccctacggc ggtaactact gggacaagga 810

<210> 475
<211> 524
<212> DNA
<213> Pseudomonas aeruginosa

<400> 475
aggagcaact gaagcgactc ggcatcgagg ccagggccgc gggcgatgcc tacgctcgac 60

tgggcgagat gcagcgtggc ctggatatgc aggtccgcgg cctgcaacgg ctggagcagg 120

ccagccaggc aatgccattg gctagcgcat tttccggact ggtcgtggaa gccagcaaga 180

cggctgccgg ttatcaagcg cggttgcgcg acctgtcgat ccgcaacggc ctggacgtcg 240

gccgggagcc agccttggca tccctgatcc aggacagcgc caaccagagc ggcctgggac 300

gcacggtgac gctggacatg ctggagcact tgaacgccac cggcatgggg ttcgccgccg 360

cgcaaatgaa tctgggactg gcgggccgct tcggctttgg ccaagggatt gcttcagccg 420

aggttgcggg gctggttcga gcgttgcaac tggcccaggg ttcggactcg ccagagcaat 480

tgtccgccac cctcgaccgc ctggtcgtcc tgggtaaagg caga 524

<210> 476
<211> 704
<212> DNA
<213> Pseudomonas aeruginosa

<400> 476
aaggttggca ggatcaacga tcagaaaatg cgcgccatgg aggcgcgcgc ggaaaaggct 60

ggcaaggcta tcggcaaaag cctggacagt tcggcactga ttgccagcag cgtgctggac 120

caggcgctgg acatgctggg caggaccagt cgccaggcgg gtcaggccaa gaagcctgtg 180

cagagcgccc aggacaaggt actggccgag tggaagaccc ggcagaagga gctgggcgaa 240

gcctggaaga gctatcgcga accactccag gatctgtcca agctcaacga agcactactg 300

aagaactctt ccgacaagct cgacaaggcg ctgctcaatc tcagcgagac cggcaagctg 360

tcgcttgcca acgtgggcaa ggccgcctac gccgatgctg cgcgcctcgc ctcgcggcag 420

atgaccctga tgctgctgga cgggctgttt ggctgggtcg ccagcgtcgg taccgagaag 480

cccaaggtcg acgacaaggc gggcaaggga caggcgaagg ctggcgacga cgagaaggaa 540

cagccgtcgc tccagtcgca ggtcttcaag cagtggctgt tgcagatgaa cagtgtctgg 600

ggcgcctacc gcgcgcccct gcaggatatc tccgggatga ccgacgagct gttcaggaat 660

gcgtcggaga agctcgagaa gtcgctgttc aatttcgcca ctag 704

<210> 477
<211> 234
<212> DNA
<213> Pseudomonas aeruginosa

<400> 477
aggcatccat cgagctaccg gcaggcccgc cgcagaccct gctggtgccg ttgcgggcgg 60

tttcgccaga ggctctgggc atgcgtgcgg ggccgccgat gccacagatg gtcgaaggcc 120

agcgggtgct gctggcgcca cgcgtggagg gctcgctgga ccgcgccagg gtcggagcgc 180

tgagcctgtc cctgcgctcg ccgcaagctc cccagagtat cctgctcgga cgtt 234

<210> 478
<211> 349
<212> DNA
<213> Pseudomonas aeruginosa

<400> 478
gcgaggaggt attcgacagc ctcctggaga tgcgcgtggc gatcctgctg aacctggggc 60

gcgcggaaca ggcgctggcc ctgatcggcg agatggagga gaaggtcgag ggcgcggagt 120

ggaacaacat cagccagcca cggcgtctgt acaaggccca cggcctggcg ttgctggggc 180

gcgacgagga ggccctggag gcgctgctgc cgttctccga gattgccccg cgctaccgta 240

cgatctggct gcgcgccgtc tacctgctgc tgcaacggac ccctgagcgc aacacctggg 300

acttcggcgg gcgcctgcag cagatgctcg aacactactc gcagaaggg 349

<210> 479
<211> 402
<212> DNA
<213> Pseudomonas aeruginosa

<400> 479
aaggacttct ggtcggtgct cgaaccgcag gacggccagg ccgcactgat ggcgcggatg 60

ctcgagcttg gccacagcca gccgttgcag ccgaatgcga agatccccga aggcctggac 120

atttcgatca accgcgccaa ccagtgcccg acgccggcca gcatcgatgc gttcatccgc 180

aagaacccag gttccggcat gcctttcgcg gtggccgggc tgagcgacga cgaatacgcc 240

actttgcaga agtggctggc cgcgggcgcc ccggtcgacc agcagccgtt gcggccgacc 300

gccgccgagg cgcgccaggt ggccagctgg gagcgtttcc tcaaccagcc tggggccaag 360

cagagcctgg tctcgcgctg gctctacgag cacctgttcc tg 402

<210> 480
<211> 514
<212> DNA
<213> Pseudomonas aeruginosa

<400> 480
ttccctaacg aatgctgtca atcgcctggt tcagattgct ggtagtggac ccggcgcata 60

cgttgccacc ttcgcggtgc tgtcgctgta ttccgaccag gctggtaaag acagtgacaa 120

ggttcctgcc ggcgttcgca atgcattggc actggaggcg tccgctctgg ggcttcctgg 180

cacggctgat ttgcaaagcg tcgccaaggc aggtggcacg gttgatatgc cggtacgact 240

cacgagtgct gcacaagaga gcccgagtgg taaatcgcag attgccgcga tgttgaccaa 300

cggtgcaact gtccccaagg gcgtgcctgt tcgcgccgcg accctcaatg ctgcgacggg 360

ccggtatgag gtgacggttc ccgcaaagtc caccgtgccg aatacaccac cgctgatctt 420

gacctggacc cctgccaccc ctccaggaag ccagaacccc tcaagcacca ctccggtcgt 480

accgcagccg gttccggtgt atgagggagc aacg 514

<210> 481
<211> 604
<212> DNA
<213> Pseudomonas aeruginosa

<400> 481
cgagcaccaa tatcgaactg gtttcgacca agggcgacct ggacctcgac ggctcggtga 60

actgggcatc gggcaaccgg ctggggctgg gctccgcggc cgacctgacg ctgaatggca 120

ggctgaatgc cagtggcgcc aaggctgggc tggagctgaa ggccgaaggc gctatcgata 180

tcaatgacaa gatcgttctc ggcggggctg gcagcgcgct ggccatggat gccggcgaag 240

gccaccgggt gaacggcacg gcgtcggtct ccctggccgg ggccaacgcg acctacgtct 300

ccggtggcta ttactacacg gtggtgcaga acctggcgca gttgcaggcg atcaacaaga 360

acctggacgg cctgtacgtg ctcggcggca atatcctggg cggcagctat tactgcacgg 420

cgctgcaatc catcggcggg cccgccggcg tcttcagcgg caccctggac ggtctcggca 480

acagcatcgg caatctctcg atcagcaaca ccgggccgaa tgtcggcctg ttcgcccgct 540

cctcgggcac cctgagcaac ctgaagctga acaacctgcg ggtatccgat aacacctacg 600

gctc 604

<210> 482
<211> 412
<212> DNA
<213> Pseudomonas aeruginosa

<400> 482
gctttacctt gatcgaactg atgatcgtgg ttgcgatcat cggtattctt gctgccgtcg 60

ctttgccggc atatcaggat tacaccattc gtgctcgcgt gacagagggg gttggcctgg 120

ctgccagcgc caagacgctt attggcgata gctctgccac tgccggtgag ctagccgctt 180

cggcaagggt ctggaatgct caagccggta acgccggtgc taccagtaag tatgtgacct 240

ctgtacaaat tgcagaggcg actggtgaaa tcactgttac tttcaatgcc gcaaacgtgg 300

gtaatattcc ggctaactct accctggtat ttactcccta tgtgcagaat gctgccggtg 360

ccccgactca attgggtgcc agttatgctt ccggtgtgac tggctctatt ga 412

<210> 483
<211> 320
<212> DNA
<213> Pseudomonas aeruginosa

<400> 483
tgccgtgagt gaaatcagcg cgttgaagac cgctgcggag tcggcgattc tggaaggcaa 60

gaagcttgtt tccaaggata atcccgcgga tggggaatat gatcttggtt ttaccaagtc 120

tactttgctt gctggcaacg acggtaaggc acagatcacc atcactggcg aaagcagtgc 180

aaccccgacc attgcgggga ctctgggtaa ctctgctggt aaggccatca gcggtgccgt 240

tatcaccatc aagcgtagtg ctgagggagt ctggacctgc gctaccagtg ggtctccggc 300

caactggaaa gccaactacg 320

<210> 484
<211> 738
<212> DNA
<213> Pseudomonas aeruginosa

<400> 484
ggtatcaacc cactaaaggt ccgcaagaaa ggtatcaccc tgtgggcagg gaagaagatt 60

aagcccatgg acatcgcctt gtttcactcg gcagatgtct accatgatgg gtgccggcga 120

ccggtactgc aatcttttga catcatcggc gaaggattcg aaaatccaaa catgcgcaag 180

ctagtcgatg agatcaagca ggatgttgcc gccggtaaca gcttagccag ttcacttcga 240

aagaaaccca tttacttcga tgatctctac tgcaacctgg tcgatgctgg cgaacagtcc 300

ggtgctttgg agacattatt ggatcgggta gcaacttata aagaaaagac agaatccctg 360

aaagccaaaa ttaaaaaagc catgacttat cccattgcag taattgtagt ggcccttgta 420

gtatcggcga tccttctgat aaaagtggtc ccacagttcc agtccgtatt tgcaaatttt 480

ggtgccgagt tgccggcctt tactcaaatg gtcatcaatc tttccgagat gcttcaagag 540

tggtggctca tagtgcttat tggtcttttt gccgcagctt ttgcatttag ggaagctcat 600

catttgggat cagtagatcg gggcctgctg aaactaccta tcatcggcgg gatactttac 660

aaatcagcta tcgcccgcta cgcccgaacg ctatccacta cctttgcggc tggagtgcct 720

ctggtagaag ctctggac 738

<210> 485
<211> 740
<212> DNA
<213> Pseudomonas aeruginosa

<400> 485
gaagtgaact ccgccaagga tctgaaggcg gcgctgggca tcatcgtgca gcgggtcaag 60

gaagccatgg gtacccaggt ctgctcggtg tacctgctcg acaccgagac ccagcgtttc 120

gtcctgatgg ccaccgaagg cctcaacaag cgttccatcg gcaaggtcag catggccccc 180

agcgaaggcc tggtcggcct ggtcggcacc cgcgaggagc cgctcaacct ggagaacgcc 240

gccgcccacc cgcgctaccg ctatttcgcc gagaccggcg aggagcgcta cgcgtcgttc 300

ctcggcgcgc cgatcatcca ccataggcgg gtgatggggg tgctggtggt gcagcagaag 360

gagcgccgcc agttcgacga aggcgaggag gccttcctcg tcaccatgag cgcccagctc 420

gccggggtca tcgcgcatgc cgaggcgacc ggttcgatcc gcggcctggg caagctcggc 480

aagggcatcc aggaagccaa gttcgtcggc gtgcccggcg cccccggggt cggggtgggc 540

aaggcggtgg tggtgttgcc tccggccgac ctggaagtgg tgccggacaa gcaggtcgac 600

gacatcgacg ccgagatcgc cctgttcaag caggccctgg agggcgttcg cgccgacatg 660

cgcgcgctgt cgagcaagct cgccagccag ttgcgcaagg aagaacgcgc gctgttcgac 720

gtctacctga tgatgctcga 740

<210> 486
<211> 680
<212> DNA
<213> Pseudomonas aeruginosa

<400> 486
tcgagaagtc gatgttcaag gacctcggca ttcccactcc ggattttgcg gacgtccagt 60

cccaggccga cgttgatgcc gctgcagcag ccataggcgt gccggcggtg ctcaagaccc 120

gcacactggg gtacgacggc aagggccaga aggtcctgcg ccaaccggcc gacgtgcagg 180

gcgcgtttgc cgaactgggc agcgtgccgt gcatcctcga gggcttcgtg ccgttcaccg 240

gggaagtttc gctggtggcg gtgcgcgctc gagatgggga gacgcgttta taccctctgg 300

tgcacaacac ccacgacagc ggcatcctca agctctccgt ggccagcagc gcgcatccgt 360

tgcaggcgct ggccgaggac tacgtcggcc gtgtgctggc ccggctcgac tacgtcggcg 420

tgctggcctt cgagttcttc gaggtggacg gcggcctgaa ggccaacgag atcgccccgc 480

gcgtgcacaa ctccgggcac tggaccatcg aaggcgccga gtgcagccag ttcgagaacc 540

acctgcgcgc cgtcgccggc ctgccgctgg gctcgaccgc caaggtcggc gagagcgcga 600

tgctcaattt catcggcgcg gtgcccccgg tggctcaggt ggtcgccgtc gccgactgcc 660

acctgcatca ctacggcaag 680

<210> 487
<211> 210
<212> DNA
<213> Pseudomonas aeruginosa

<400> 487
agacctacaa caaggtttcg cgcttcatcc gcgagatccc gccggcgctg atccaggaag 60

tgcgcctgtc caataccgtc agccgcccct acggcggcac ctcgcgcagt gccggcggca 120

acctcttcag cggcgccggg gtgccggaga cgcccttctc cctcggccag cgggtgcgcc 180

acgcgctgtt cggcgaaggg actatcctca 210

<210> 488
<211> 351
<212> DNA
<213> Pseudomonas aeruginosa

<400> 488
attcctctct gaatcgctgg aagggctttc cgccgccatg atcgccgagc tgggacgcta 60

ccggcatcag gtcttcatcg agaagctggg ctgggacgtg gtctccacct ccagggtccg 120

cgaccaggag ttcgaccagt tcgaccatcc gcaaacccgc tacatcgtcg ccatgggccg 180

ccagggcatc tgcggttgtg cccgcctgct gccgacgacc gacgcctacc tgctcaagga 240

agtcttcgcc tacctgtgca gcgaaacccc gccgagcgat ccgtcggtct gggagctttc 300

gcgctacgcc gccagcgcgg cggacgatcc gcaactggcg atgaagatat t 351

<210> 489
<211> 530
<212> DNA
<213> Pseudomonas aeruginosa

<400> 489
aggaatgacg gaggcttttt gctgtggtgg cacggtttgc gttgcgagat gcagccgatc 60

cacgacagcc agggcgtgtt cgccgtcctg gaaaaggaag tgcggcgcct gggcttcgat 120

tactacgcct atggcgtgcg ccacacgatt cccttcaccc ggccgaagac cgaggtccat 180

ggcacctatc ccaaggcctg gctggagcga taccagatgc agaactacgg ggccgtggat 240

ccggcgatcc tcaacggcct gcgctcctcg gaaatggtgg tctggagcga cagactgttc 300

gaccagagcc ggatgctctg gaacgaggct cgcgattggg gcctctgtgt cggcgcgacc 360

ttgccgatcc gcgcgccgaa caatttgctc agcgtgcttt ccgtggcgcg cgaccagcag 420

aacatctcca gcttcgagcg cgaggaaatc cgcctgcggc tgcgttgcat gatcgagttg 480

ctgacccaga agctgaccga cctggagcat ccgatgctga tgtccaaccc 530

<210> 490
<211> 569
<212> DNA
<213> Pseudomonas aeruginosa

<400> 490
ttcaacctca acggactggt gcgcaagcgc aaggtcaagc cggactcggt gaagcagttc 60

cgtcgcctgc tggccaccct ggggatgaag gaagagatcg tccagggctt gccggaccgg 120

ctggccgact ggctcgacgc cgaccagaat ccgcagggcg agcaaggcgc cgaggacaac 180

cagtacctgc tggaggcgcc ggcctaccgc gccgccaacc gcagtttcaa ggacgtgtcc 240

gagctgcgcc tgctgaaatt gtcggaagcc gactatcgac gcctgctgcc gttcgtcagc 300

gccttgcccg aagatgcgcc gctgaacgtg aacactgcca gcgtgccggt gctggccgcc 360

atgttcgaga tcgatccggg acaggcggaa aacatcgtgg acgcccgcgg tcgggaaggt 420

ttccagagca aggacgattt caccaagcat ctgacccagt tgggttcgaa gaccggaaac 480

gtcagttatg ccgtcggcac ccgctacttc caggtgatca gcgaggtcag cctgggcgac 540

cgccggcagg tgctggtgag taccttgca 569

<210> 491
<211> 345
<212> DNA
<213> Pseudomonas aeruginosa

<400> 491
cattgaaagg tcgtagcgat gcgtataccc aggtagacaa cttcctgcat gcctatgcgc 60

ggggcgggga cgaattggtc aatggccatc cgtcctatac cgtcgaccag gcggcggagc 120

agatcctccg cgaacaggcg tcttggcaga aagcgccggg cgactcggtg ctgaccctgt 180

cctattcgtt cctgaccaaa ccgaacgact tcttcaatac gccgtggaag tatgtcagcg 240

atatctactc gctgggcaag ttcagcgcct tttccgcgca gcagcaggcc caggccaagt 300

tgtcgctgca atcctggtcg gacgtcacca atatccactt cgtcg 345

<210> 492
<211> 576
<212> DNA
<213> Pseudomonas aeruginosa

<400>  492
ggtcaagcac atcctagtgc gcgacggcca gcatgtggag gcgggcgagc cgctgatccg      60

catggaaccg acccaggccc gggccaacgt cgattcgctg ctcaaccggt acgccaacgc     120

gcggctcaac caggcgcgcc tgcaggccga atacgacggc cggcggaccc tggagatgcc     180

cgcggggctg gccgagcagg ccccgctgcc gaccctcggc gagcgcctgg agttgcagcg     240

gcagttgctg cacagccgcc agaccgcgct ggccaacgaa ctctccgcat tgcgggcgaa     300

catcgagggg ctgcgcgccc agctcgaagg gttgcgccag accgagggca accagcgcct     360

gcaacaacgc ctgttgaaca gccagttgag cggtgcgcgc gacctcgccg aggaaggcta     420

catgccgcgc aaccagttgc tcgaacagga gcgccaactg gccgaggtga acgcccggct     480

atcggagagc agcggtcgct tcgggcagat ccgccagagc atcgccgagg cgcagatgcg     540

catcgcccaa cgcgaggagg agtaccgcaa ggaagt                               576


<210>  493
<211>  581
<212>  DNA
<213>  Pseudomonas aeruginosa

<400>  493
ccgaaggact tggtttactc caactttgtg cagcaggacg gcggtagcac cctgttgggg      60

cagtacgaca tgatcaacga aggcagccaa gtgattgaac ttgccgtcaa cttgcaacaa     120

gggttagtgg acaccttcac ctggagcgtc actgagcagt tgaaggtcgg tgtggaagtc     180

aaggtgaagg cgaacattcc cctagtgggc ggcgctgaga tcaccagtac ggtggaattg     240

tcactgtcct ctacccaagg ggcgagtacc agcaagtctt ccaactatgg cgcctctacc     300

aaggtgctta tttccccaca tagccacggc tggggagagg ttgccttgag ctttactgag     360

ctgcgcactc agtgggtcgg taatgtcggg cttcaaggat atgtggcaat ttggttcaac     420

aacaaagtcg cattgaacaa cgatggcgat taccactacc tgtggttcat tcccgtggag     480

caggtatttt gggagtgcgt ccagcacaac atagtcaata cctcgggcta tgtcgtacaa     540

ggcaatggag tgttggcgca agccacaggc accttccata g                         581


<210>  494
<211>  457
<212>  DNA
<213>  Pseudomonas aeruginosa

<400>  494
cactttccgt tattgcctcg aagacgaagg ctacagcgtg gccaccgcca gcagcgcgcc      60

gcaggcggag gccctgttgc agcgccaggt attcgacctg tgcttcctcg acctgcgcct     120

gggcgaagac aacgggctcg acgttctcgc ccagatgcgc gtccaggcgc catggatgcg     180

cgtggtgatc gtcaccgcgc attcggcggt ggataccgcg gtcgatgcca tgcaggccgg     240

cgcggtggat tacctggtca agccctgcag cccggaccaa ctgcgcctgg ccgccgccaa 300

gcaactggag gtgcgccaac tgaccgcgcg cctggaggcc ctggaggacg aagtgcgccg 360

ccagggcgac ggcctggaat cgcacagccc ggccatggcc gcggtactgg agaccgcgcg 420

ccaggtagcg gcgaccgacg ccaacatcct catcctc 457

<210> 495
<211> 289
<212> DNA
<213> Pseudomonas aeruginosa

<400> 495
gactggctga atcgtctcgc cgaggccgat cgccagaaca gtttccaagg caccttcgtc 60

tacgagcgca atggcagctt ctccacccat gagatctggc atcgcgtgga gagcgatggt 120

gcggttcgcg agcgcctgct ccagctcgac ggcgcgcgcc aggaagtggt ccgggtcgac 180

gggcgcaccc agtgcatcag cggcggcctt gccgaccaac tggccgatgc ccagctgtgg 240

ccggtgcgca agttcgatcc ctcccagctg gcttcctggt acgacctgc 289

<210> 496
<211> 659
<212> DNA
<213> Pseudomonas aeruginosa

<400> 496
attgtcgatg acgaacctct ggcgcgagag cgcctggccc gattggtagg gcaactggac 60

ggctatcgcg tcctcgagcc ctcggccagc aatggcgaag aagcgctgac gctgatcgac 120

agcctcaagc ccgatatcgt cctgctggat atccgcatgc ccggtctgga cggcctccag 180

gtcgcggcca gactctgcga gcgggaagcg ccgccggctg tgatcttctg cacggcccat 240

gacgaattcg ccctggaagc cttccaggtc agcgccgtgg gctacctggt caagccggtg 300

cgcagcgaag acctggccga ggcgttgaag aaagcctcgc gaccgaaccg cgtgcaactg 360

gccgcgctga ccaagccccc ggcctccggc ggcagcggtc cgcgcagcca catcagtgca 420

cggacccgca aggggatcga gctgatcccg ctggaagagg tgatcttctt cattgccgac 480

cacaagtacg tgaccttgcg ccatgcgcag ggcgaggtgc tgctggacga gccgttgaag 540

gcgctggaag acgagttcgg cgagcgcttc gtgcgcatcc accgcaacgc gctggtcgcc 600

cgcgaacgga tcgaacgcct gcagcgtacg ccgctggggc atttccagct ctacctgaa 659

<210> 497
<211> 629
<212> DNA
<213> Pseudomonas aeruginosa

<400> 497
cgtttgggac agattgaggc ccgccaggtc gccaccccca gtgaagcgca gcagttggcc 60

cagcgccagg acgcgccgaa gggtgagggg ctgctcgctc gcctgggcgc ggcgctcgtg 120

cgtccgttcg tggcgatcat ggactggctg ggcaaactgt tgggctccca cgcccgcacc 180

ggcccgcagc ccagtcagga cgcgcagcct gcggtcatgt cctcggccgt cgtgttcaag 240

cagatggtgc tgcagcaggc attgcccatg accttgaagg gactcgacaa ggcgagcgag 300

ctggcgaccc tgacaccgga aggactggcc cgggagcact cccgcctggc cagcggagat 360

ggggcgctgc gttcgctgag caccgccttg gccggcattc gtgccggcag ccaggtcgag 420

gagtcccgta tccaggctgg ccgcctgctc gaacggagca tcggcgggat cgcgctgcag 480

cagtggggca ccaccggcgg tgccgcgagt caactggtgc tcgacgcaag cccggaactg 540

cggcgcgaaa tcaccgacca gttgcatcag gtaatgagcg aggtcgcact gttgcgccaa 600

gcggtagaga gcgaggtcag cagagtatc 629

<210> 498
<211> 332
<212> DNA
<213> Pseudomonas aeruginosa

<400> 498
aatgcgataa ccatcagcgt cgccgaggcg gcggacagca gcgtcgatct cggcgccacc 60

atgatcacct ccaaccagtt gggcaccatc accgaggaca gcggctccta tacgccaggc 120

actatcgcca cggcgacccg cctggtcctg actccgcgcg agacgcccca gtcgatcacc 180

gtggtcaccc gccagaacat ggacgacttc ggcctcaaca acatcgacga cgtcatgcgc 240

catacgccgg gcatcaccgt ctcggcctac gacactgacc gcaacaacta ctatgcccgc 300

ggcttctcga tcaacaactt ccagtacgac gg 332

<210> 499
<211> 456
<212> DNA
<213> Pseudomonas aeruginosa

<400> 499
ctgggacgtt agtgtcatcg acgagatgga aatcgatggt tatgacgcac tcagtcctta 60

ttacatgttg atccaggaag atactcctga agcccaggtt ttcggttgct ggcgaattct 120

cgataccact ggcccctaca tgctgaagaa caccttcccg gagcttctgc acggcaagga 180

agcgccttgc tcgccgcaca tctgggaact cagccgtttc gccatcaact ctggacagaa 240

aggctcgctg ggcttttccg actgtacgct ggaggcgatg cgcgcgctgg cccgctacag 300

cctgcagaac gacatccaga cgctggtgac ggtaaccacc gtaggcgtgg agaagatgat 360

gatccgtgcc ggcctggacg tatcgcgctt cggtccgcac ctgaagatcg gcatcgagcg 420

cgcggtggcc ttgcgcatcg aactcaatgc caagac 456

<210>  500
<211>  275
<212>  DNA
<213>  Pseudomonas aeruginosa

<400>  500
aagaagtctc tgctccccct cggcctggcc atcggtctcg cctctctcgc tgccagccct      60

ctgatccagg ccagcaccta cacccagacc aaatacccca tcgtgctggc ccacggcatg     120

ctcggcttcg acaacatcct cggggtcgac tactggttcg gcattcccag cgccttgcgc     180

cgtgacggtg cccaggtcta cgtcaccgaa gtcagccagt tggacacctc ggaagtccgc     240

ggcgagcagt tgctgcaaca ggtggaggaa atcgt                                275


<210>  501
<211>  648
<212>  DNA
<213>  Pseudomonas aeruginosa

<400>  501
atggcagttt cagtgtcgac gccagcggca acctgctgat cacccgcgac atccgcaacc      60

tgttcgacta cttcctcagc gccgtcggcg aagagcccct gcagcaaagc ctggaccgcc     120

tgcgcgccta catcgccgcc gaactccagg agccggcgcg cggccaggcg ttggcgctga     180

tgcagcaata catcgactac aagaaggaac tggtgctgct cgaacgcgac ctgccgcgcc     240

tggccgacct cgacgccctg cgccagcggg aagccgcggt gaaagccctg cgcgcgcgga     300

tcttcagcaa cgaagcgcac gtggcgttct tcgccgacga ggaaacctac aaccagttca     360

ccctggagcg cctggcgatc cgccaggacg gcaagctcag cgccgaggaa aaggccgccg     420

ccatcgaccg cctgcgcgcc agcctgccgg aagaccagca ggaaagcgtg ctgccgcaac     480

tgcaaagcga actgcagcag cagaccgccg ccctccaggc cgctggcgcc ggcccggaag     540

ccatccgcca gatgcgtcag caactggtgg gcgccgaagc caccacccgc ctggagcaac     600

tcgatcggca acgctcggcc tggaagggcc ggctggacga ctatttcg                  648


<210>  502
<211>  405
<212>  DNA
<213>  Pseudomonas aeruginosa

<400>  502
aatgtcggca tcattctcgc caacgaggcg gggcaggtgc tgtgggcgcg gcgtatcaat      60

caggaagcct ggcagttccc gcagggaggc atcaatgatc gcgaaacgcc ggaagaggcg     120

ctgtatcgcg aattgaacga agaagtcggg ctggaggccg gggacgtgcg catcctggcc     180

tgcacccgcg gctggctgcg ctaccgtttg ccgcagcgcc tggtgcggac ccacagccag     240

ccgctgtgca tcggccagaa gcagaaatgg ttcctgctgc ggctgatgtc cgacgaggcg     300

cgcgtgcgca tggatatcac cagcaagccc gagttcgacg gctggcgctg ggtgagttac     360

tggtaccccc tgggacaggt ggtgaccttc aagcgcgagg tctac 405

<210> 503
<211> 542
<212> DNA
<213> Pseudomonas aeruginosa

<400> 503
gacctgctgt tccagttgct cggctatctg gccaagagcg gcgggcgggt ggaggagatg 60

catatccgcc aggcgcgcga ggagatggcg ttgcgcaagc tcgataggcg agcccagcgg 120

cgtgccatcg cgtccttcgg caagggcaag gccggcatcg cccatctgca ggcggaggtc 180

gcgcgtctga agggcgaacg tgcggaggca gtattgctcg cctgctggcg gatggcctgg 240

gctggcggcg tgctcagcca gtcggcgcga caactggtgt tgcaatgggg gcgctggctg 300

ggttggtcgg cggagcgaac ggaacgcttg tcggcgcggg tcatgccgaa gcggacgcgc 360

gctgtcgccc gggatagcta ccgtgaggcc ctgctgctgc tcggcgtgga ggccggaagc 420

gagccggcgc tgatcaaacg cgcctatcgc aagctgatca gccagcatca tccggacaaa 480

ctggcgggag ccggcgccag cgtcgagcgc gtgcgtgcgg ctaccgagaa aacccgtgaa 540

tt 542

<210> 504
<211> 427
<212> DNA
<213> Pseudomonas aeruginosa

<400> 504
   cctgctcaac accttctatc cgcagttgcc ggcggtggcg cgtttcatcg aactgggccg 60
   ccagttgcac caccggcgcg gcatccgcca cctggacgcg gcctgcgggg tgcaggtcgg 120
   tttcgccacc ctggacatcc tcgccgcgtt gctggagggc gtcggcccct ggtcgctgga 180
   gtcgccctcg aacgacctgt cggcgatgcg cgggctgtcc ctggtgttgg cggaagtgcc 240
   gttgagcctg cacgtgctca acgaactggc ggccgccgac gatgggcgca tgaccttgtt 300
   gcagcgcgtc agcctgacca ccgatcgcgg cacgctgagc ctgctcagcc cccatggccc 360
   gttgttgtgg acgcctgcgg tggcggtacc ggcagaggat gacgacggcc tgttcgcgtt 420
   gttcgac 427

<210> 505
<211> 417
<212> DNA
<213> Pseudomonas aeruginosa

<400> 505
gttgaaaggg tttaccgaca acctggaatt gcggcggcgc aaccgtgcca cggtcgagca 60

ctacatgcgc atgaaggggg ccgaacggtt gcagcggcac agcctgttcg tcgaggacgg 120

ctgcgccggc aactggacca cggaaagcgg cgaacccctg gttttccggg gccatgagag 180

cctcaggcgg ctcgccgagt ggctcgagcg ctgcttcccc gactgggagt ggcacaacgt 240

gcggatcttc gagaccgagg atccgaacca cctctgggtc gagtgcgacg ggcgcggcaa 300

ggcgctggtc ccggggtatc cgcagggcta ttgcgagaac cactacatcc attccttcga 360

actcgagaac ggccggataa aacgcaatcg cgagttcacg aacccgatgc agaaatt 417

<210> 506
<211> 356
<212> DNA
<213> Pseudomonas aeruginosa

<400> 506
atgctcgata atgctattcc ccaaggtttc gaagacgccg tggagttgcg caggaagaat 60

cgcgagacgg tggtcaagta tatgaacacc aaaggccagg atcgcctgcg ccgccatgaa 120

cttttcgtcg aggacggctg tggcggttta tggaccaccg ataccggctc gcccatcgtc 180

attcgtggca aggacaagct ggccgagcac gcggtgtggt cgctgaaatg cttcccggat 240

tgggagtggt acaacatcaa ggtcttcgag accgacgatc ccaaccactt ctgggtcgag 300

tgcgacggcc acggcaagat cctcttcccc gggtatcccg agggttacta cgagaa 356

<210> 507
<211> 671
<212> DNA
<213> Pseudomonas aeruginosa

<400> 507
gacttcgctg ttcgacttcc agggcgcagg cgatacccag gtcgcccccg acctgaccaa 60

cgtcccgcag agcgacgcgc gcaaggagga cgcctactgg cagcagttct accggcccag 120

tcccaaatac tggtcctacg agcccaagag cctgcccggc caggaaaagg gccagcgccc 180

taccctcgcg gtgccctacc agttgcacgc cacgctggcc ctcgacatcg ccgccggcaa 240

gctgcgcctg accctgggca acgacggcat gagcctgccg ggcaatccgc aggacactgc 300

cgctgcggta ttccaggtgc agccgcggga agtcggcaat ccgcgcttct ataccgtgac 360

cagctatccg gtggtccagg aaagcggaga ggaactgggc cggaccctca acgacgaact 420

cgacgacctg ctcgacgcca acggccgcta cgccttcgag gtgcacggcc ccaacggctt 480

cttccgcgag ttccacggca acctgcatct cgccgcgcag atggcgcggc ccgaggtatc 540

ggtcacctat caacgcaacg gcaacctgca gttgaacatc cgcaatctcg gccgcctgcc 600

gtgcaggcgt gacggtgacg ccgaacccgg cctatacccg ggaggcagcc gtcgctatga 660

actcgaaccg a 671

<210> 508

<211>  304
<212>  DNA
<213>  Pseudomonas aeruginosa

<400>  508
gtgttccagg tgttcgacct gctcgacagc gagaacccgc cgaaacgcta caccgtcggc      60

gcgcgcaagc gcctgcacga cagcttccag ggcgacgcca gcggcgacta ccacctggaa     120

gtgcacggtc cgaacggttt cctccgggtc tttcgcggca acctgcggcg cgacctggcg     180

gacggcaagg cgccgctgcc ggaagtgcgg atcgactacg agccgctgtt cggcaacctg     240

cgcgtgcaac tgatcaaccg tggccgccat ccggtcaagc tgacggtcaa ggacaacgtc     300

tatc                                                                  304


<210>  509
<211>  302
<212>  DNA
<213>  Pseudomonas aeruginosa

<400>  509
acaacctgga acagcaactc ggcgagttcg gccgcaacgc cgggcagatg tccgagatcg      60

aacgcaagca ggccgccgaa ggtctgatcg aacagctcaa gcgcgaggtg gcggtcggcg     120

ccgatccgcg ccagaccttc gaggagatcc agcgtctgac gccctatgtg gaggccgatg     180

ccaggcgccg cgaggcgctc gacttcgaga tctggatggc gctcaaggac aacgcctccg     240

tccagcagca agcgccgacg cctggcgagg aagagcaact gcgcgaatac gcgcaagagt     300

cg                                                                    302


<210>  510
<211>  722
<212>  DNA
<213>  Pseudomonas aeruginosa

<400>  510
gtcaagggtg ttgtctgccg tgccctgctg ggcgctggtc gcgaggtgcc ggagtgggac      60

gatatggttg cggaatacgc cgagcaattg ctgcaggagc accccgaagg ggttttcaac     120

ctggcgggat ggtcgctcgg cggcaacctg gcgatggatg tcgcggcccg gctggagcag     180

cgtgggcggc aggtggcttt cgtcggctgg atcgatgcac cggcaccggt cagggtcgaa     240

gcgttctgga acgagatcgg gccgacgccg gaggcagtcc cgaacctatc cgtgggcgag     300

atgcgggtgg aactgctcgg tgtcatgttt ccggagcggg ccgagcatat cgaacgggcc     360

tggtcatcga tctgctccgc cacgacggac gatgagcagc gctggacgag gatgagcgac     420

tgggcggaag cggagatcgg cgccgagttc gcgacactgc gcagcgaaat cgcacagagc     480

aacgaactgg aagtgtcctg ggagttgaaa cagatcctcg acgagcgcct gaaagcgatg     540

gattacccgc gtctgacggc gaaggtcagc ctctggtggg ccgcgcgcag caccaatgcc     600

atccagcgga gcgcggtgga gcgctcgatg gccgaggcga tcggggctga gcgtgtcgaa 660

ccggtgcggg tgctggatac ccggcacgac aagatcatcg accaccctga gtttgtgcag 720

ag 722

<210> 511
<211> 616
<212> DNA
<213> Pseudomonas aeruginosa

<400> 511
aagtcggtac tggagtcctt ggtggaggcg ctgaaccaga ctgcgctggg cgatgcctac 60

gagctcgtgg gcgtgatcta cgacgacgac gcggagctgc ctcgcgacca gggaaaaatc 120

aaggactacg gtttcgccta tcgtcccggg cagcaatggt tctatccggc agacctgcag 180

gtgcaaggca agaccctgaa cgacctcttg ctcagcgtgc cgtccaccta ccgtcggtac 240

ccgcggggta cccccgagca tgtggccggc aagagcgatt tcgagcgacg cctgcatgac 300

accctggtgg agctgggcgc cgatgtggtg gtattggacg ggctcctggt catcctcgat 360

gagctggtac gcccgggcgc tccgttcgca cggcggatca tgaatatcca tcctggcgtg 420

acgcgcgagg actcgcctta cgagcgtcgt ggcgcctatg cgaccctgga cgcgttgtat 480

ggagcgcggg gcgagaaggt ggtggattgg gcgaccatgg aaaaggtcgc ggtcgagccg 540

ctgtactgga ccggagcact cgttcactat gtggacaatg gcatcgattc cggcgaagtg 600

ttccatgatg tgctga 616

<210> 512
<211> 741
<212> DNA
<213> Pseudomonas aeruginosa

<400> 512
cttcagttcc gagatgccga gaaaaaactt gaggcgtcgg tacaagccga gctggataag 60

gctgatgccg ctcttggtcc ggcaaagaat cttgcaccat tggacgtcat caaccgcagt 120

ctgaccatcg ttggaaacgc cctccagcaa aagaatcaaa aactactgct gaatcagaag 180

aagattacca gcctgggtgc aaagaatttc cttacccgta cggcggaaga gatcggtgaa 240

caagcggtgc gagaaggcaa tattaacggg cctgaagcct atatgcgctt cctcgacagg 300

gaaatggaag gtctcacggc agcttataac gtaaaactct tcaccgaagc gatcagtagt 360

ctccagatcc gcatgaatac gttgaccgcc gccaaagcaa gtattgaggc ggccgcagca 420

aacaaggcgc gtgaacaagc agcggctgag gccaaacgca aagccgaaga gcaggcccgc 480

cagcaagcgg cgataagagc tgccaatacc tatgccatgc cggccaatgg cagcgttgtc 540

gccaccgccg caggccgggg tctgatccag gtcgcacaag gcgccgcatc ccttgctcaa 600

gcgatctccg atgcgattgc cgtcctgggc cgggtcctgg cttcagcacc ctcggtgatg 660

gccgtgggct ttgccagtct gacctactcc tcccggactg ccgagcaatg gcaggaccaa     720

acgcccgata gcgttcgtta c                                              741


<210>  513
<211>  211
<212>  DNA
<213>  Pseudomonas aeruginosa

<400>  513
atatacggaa aaagagtttc ttgagtttgt tgaagacata tacacaaaca ataagaaaaa      60

gttccctacc gaggagtctc atattcaagc cgtgcttgaa tttaaaaaac taacggaaca     120

cccaagcggc tcagaccttc tttactaccc caacgaaaat agagaagata gcccagctgg     180

agttgtaaag gaagttaaag aatggcgtgc t                                   211


<210>  514
<211>  589
<212>  DNA
<213>  Pseudomonas aeruginosa

<400>  514
tatacggctt cagactttcc tcagaagtca gagtcgatgt accagagtca gttgctggcc      60

agccgaaaat tctatggaga gttcctggat cgccatatga gtgagctggc caaagcgtac     120

agcgccgata tctataaggc gcaaatcgct atcttgaaac aaacgtctca agagctggag     180

aataaagccc ggtcattgga agcagaagcc cagcgagccg ctgctgaggt ggaggcggac     240

tacaaggcca ggaaggcaaa tgtcgagaaa aaagtgcagt ccgagcttga ccaggctggg     300

aatgctttgc ctcaactgac caatccaacg ccagagcagt ggcttgaacg cgctactcaa     360

ctggttacgc aggcgatcgc caataagaag aaattgcaga ctgcaaacaa tgccttgatt     420

gccaaggcac ccaatgcact ggagaaacaa aaggcaacct acaacgccga tctcctagtg     480

gatgaaatcg ccagcctgca agcacggctg gacaagctga acgccgaaac ggcaaggcgc     540

aaggaaatcg ctcgtcaagc ggcgatcagg gctgccaata cttatgcca                589


<210>  515
<211>  710
<212>  DNA
<213>  Pseudomonas aeruginosa

<400>  515
atccagtata ttcctgctcg atcaagctac ggtactccac catttgtccc accaggacca      60

agtccgtatg tcggtactgg aatgcaggag tacaggaagc taagaagtac gcttgataag     120

tcccattcag aactcaagaa aaacctgaaa aatgaaaccc tgaaggaggt tgatgaactc     180

aagagtgaag cggggttgcc aggtaaagcg gtcagtgcca atgacatccg cgatgaaaag     240

agtatcgttg atgcactcat ggatgccaaa gcaaaatcgc taaaggccat tgaggatcgc     300

ccggccaatc tttatacggc ttcagacttt cctcagaagt cagagtcgat gtaccagagt 360

cagttgctgg ccagccgaaa attctatgga gagttcctgg atcgccatat gagtgagctg 420

gccaaagcgt acagcgccga tatctataag gcgcaaatcg ctatcttgaa acaaacgtct 480

caagagctgg agaataaagc ccggtcattg gaagcagaag cccagcgagc cgctgctgag 540

gtggaggcgg actacaaggc caggaaggca aatgtcgaga aaaaagtgca gtccgagctt 600

gaccaggctg ggaatgcttt gcctcaactg accaatccaa cgccagagca gtggcttgaa 660

cgcgctactc aactggttac gcaggcgatc gccaataaga agaaattgca 710

<210> 516
<211> 752
<212> DNA
<213> Pseudomonas aeruginosa

<400> 516
tcgccaataa gaagaaattg cagactgcaa acaatgcctt gattgccaag gcacccaatg 60

cactggagaa acaaaaggca acctacaacg ccgatctcct agtggatgaa atcgccagcc 120

tgcaagcacg gctggacaag ctgaacgccg aaacggcaag gcgcaaggaa atcgctcgtc 180

aagcggcgat cagggctgcc aatacttatg ccatgccagc caatggcagc gttgtcgcca 240

ccgccgcagg ccggggtctg atccaggtcg cacaaggcgc cgcatccctt gctcaagcga 300

tctccgatgc gattgccgtc ctgggccggg tcctggcttc agcaccctcg gtgatggccg 360

tgggctttgc cagtctgacc tactcctccc ggactgccga gcaatggcag gaccaaacgc 420

ccgatagcgt tcgttacgcc ctgggcatgg atgccgctaa attggggctt cccccaagcg 480

taaacctgaa cgcggttgca aaagccagcg gtaccgtcga tctgccgatg cgcctgacca 540

acgaggcacg aggcaacacg acgacccttt cggtggtcag caccgatggt gtgagcgttc 600

cgaaagccgt tccggtccgg atggcggcct acaatgccac gacaggcctg tacgaggtta 660

cggttccctc tacgaccgca gaagcgccgc cactgatcct gacctggacg ccggcgagtc 720

ctccaggaaa ccagaaccct tcgagtacca ct 752

<210> 517
<211> 739
<212> DNA
<213> Pseudomonas aeruginosa

<400> 517
atcgttctgg tcttccttgc agcgttggtg tggatgctga gtgcaggcag tatctccggc 60

ggctgggggg gggcgatgct gggtgcaggt tctggcgtgg cactgttagg gttcctggat 120

gaccatgggc acattgctgc gcgttggcgg ctgctcggcc atttctcagc agcgatatgg 180

atcttgctgt ggacgggtgg tttcccgccg ctggatgtgg ttgggcatgc tgtcgactta 240

ggatggctgg gccacgtatt ggcagttttc tatttggtat gggtgctgaa cctttataac 300

ttcatggatg gcattgatgg tattgccagt gtcgaggcca ttggtgtctg tgtaggaggg 360

gccctgatct actggcttac agggcatgtc gcgatggttg gtatccctct gttgctggcg 420

tgcgcggtcg ccggcttcct gatctggaac ttccctccag ctcgaatctt catgggtgat 480

gcggggagtg gttttcttgg tatggttatt ggtgcactag ctattcaggc tgcatggacc 540

gccccctcgc tgttctggtg ctggttgata ttgctgggag tgttcatcgt tgatgcaacc 600

tatactctga tccgccggat cgccagaggg gagaaattct atgaggcgca tcgcagccac 660

gcttatcagt ttgcctcgcg tcgttatgct agccatctgc gggttacctt gggtgttctg 720

gctatcaaca ctctttggt 739

<210> 518
<211> 756
<212> DNA
<213> Pseudomonas aeruginosa

<400> 518
agtctgttgg tatcggtttg caagggcctg cgggtacatg tcgagcgcgt tgggcaggat 60

cccgggcgca gcacggtgat gctggtcaac ggcgcgatgg cgaccaccgc ctcgttcgcc 120

cggacctgca agtgcctggc cgaacatttc aacgtggtgc tgttcgacct gcccttcgcc 180

gggcagtcgc gtcagcacaa cccgcagcgg gggttgatca ccaaggacga cgaggtggaa 240

atcctcctgg cgctgatcga gcgcttcgag gtcaatcacc tggtctccgc gtcctggggc 300

ggtatctcca cgctgctggc gctgtcgcgc aatccgcgcg gcatccgcag ctcggtggtg 360

atggcattcg cccctggact gaaccaggcg atgctcgact acgtcgggcg ggcgcaggcg 420

ctgatcgagc tggacgacaa gtcggcgatc ggccatctgc tcaacgagac cgtcggcaaa 480

tacctgccgc cgcgcctgaa agccagcaac catcagcaca tggcttcgct ggccaccggc 540

gaatacgagc aggcgcgctt tcacatcgac caggtgctgg cgctcaacga tcggggctac 600

ctggcttgcc tggagcggat ccagagccac gtgcatttca tcaacggcag ctgggacgaa 660

tacaccaccg ccgaggacgc ccgccagttc cgcgactacc tgccgcactg cagtttctcg 720

cgggtggagg gcaccgggca tttcctcgac ctggag 756

<210> 519
<211> 473
<212> DNA
<213> Pseudomonas aeruginosa

<400> 519
aacgctttct cgatcagggc agccggccgc tggtgttcac ccagggctcg accgaacacc 60

tgcagggcga cttctacgcc atggccctgc gcgcgctgga acgcctcggc gcgcgtggga 120

tcttcctcac cggcgccggc caggaaccgc tgcgcggctt gccgaaccac gtgctgcagc 180

gcgcctacgc gccactggga gccttgctgc catcgtgcgc cgggctggtc catccgggcg 240

gtatcggcgc catgagcctg gccttggcgg cgggggtgcc gcaggtgctg ctgccctgcg 300

cccacgacca gttcgacaat gccgaacggc tggtccggct cggctgcggg atgcgcctgg 360

gcgtgccatt gcgcgagcag gagttgcgcg gggcgctgtg gcgcttgctc gaggacccgg 420

ccatggcggc ggcctgtcgg cgtttcatgg aattgtcaca accgcacagt atc 473

<210> 520
<211> 459
<212> DNA
<213> Pseudomonas aeruginosa

<400> 520
ttcgattact acgcctatgg cgtgcgccac acgattccct tcacccggcc gaagaccgag 60

gtccatggca cctatcccaa ggcctggctg gagcgatacc agatgcagaa ctacggggcc 120

gtggatccgg cgatcctcaa cggcctgcgc tcctcggaaa tggtggtctg gagcgacagc 180

ctgttcgacc agagccggat gctctggaac gaggctcgcg attggggcct ctgtgtcggc 240

gcgaccttgc cgatccgcgc gccgaacaat ttgctcagcg tgctttccgt ggcgcgcgac 300

cagcagaaca tctccagctt cgagcgcgag gaaatccgcc tgcggctgcg ttgcatgatc 360

gagttgctga cccagaagct gaccgacctg gagcatccga tgctgatgtc caacccggtc 420

tgcctgagcc atcgcgaacg cgagatcctg caatggacc 459

<210> 521
<211> 519
<212> DNA
<213> Pseudomonas aeruginosa

<400> 521
cccctattcc ataccttacc tatatcgccc gtcccggcat caataaattt gatcttcctg 60

aaggggcgaa aatcaaggat ctgattaagc gctatcagta tattggttcg caaatcccgg 120

cagcaatcat gattcgtggt gtgcaggaag agatcaaaaa atccacgaac actgccttgg 180

ccaatgtggg ggcaattgtc gatggcgaac tggcgtatct tgctagccag aaaaaggaaa 240

aattaaatcc tgccgaggcg acacccttgc agatggcctc tgctgaaaag gccgcggcgg 300

tggaactgct tgcgtccaaa cagaaggaac tggctgacgc acgaaccatt gcaaatgcat 360

tctttggcta tgaccctctc acggtcaatt atgttaatgt aatgaatgaa atctacggcc 420

gccgcgaaga taaagatttc agtttcgaca actggtcgaa gtcttattca gccgcacaaa 480

agatccgctt gatcgaagcg aaaatcagcg tcctcaata 519

<210> 522
<211> 417
<212> DNA

<213>  Pseudomonas aeruginosa

<400>  522
gtgcgctaca gctacacgcg ccaggcgcgc ggcagttggt cgctgaactg gctggtgccg      60

atcggccacg agaagccttc gaacatcaag gtgttcatcc acgaactgaa cgccggtaac     120

cagctcagcc acatgtcgcc gatctacacc atcgagatgg gcgacgagtt gctggcgaag     180

ctggcgcgcg atgccacctt cttcgtcagg gcgcacgaga gcaacgagat gcagccgacg     240

ctcgccatca gccatgccgg ggtcagcgtg gtcatggccc aggcccagcc gcgccgggaa     300

aagcgctgga gcgaatgggc cagcggcaag gtgttgtgcc tgctcgaccc gctggacggg     360

gtctacaact acctcgccca gcagcgctgc aacctcgacg atacctggga aggcaag        417


<210>  523
<211>  573
<212>  DNA
<213>  Streptococcus pneumoniae

<400>  523
actcttggct tgattttggg tgactctaag ctgatacctg ggtcaggagt gaacattgaa      60

aagtatcctg tgcaacagta tccaaatggt ggaaatggaa ttcaaggaga aacaatcgtt     120

tttaatttta ttggtaggat attaaaagaa aaaggtatag atacttatct ggctgctgcc     180

caaattatta agagtcgata tcccaaaaca gagtttaata ttattggctt tatagaaccg     240

acagagagta attatgaact taaaatttgt gacttagaaa aaaaaggaat cgtttattat     300

ttgggacaac aaaaagatgc gatacctcat attacccgtt cccatgcaat tatccatccc     360

agtgtgtatg gtgaaggaat gagcaatgta ttactagaaa acgctagttc aggacgtgtt     420

ttaattacga cagataatcc aggttgcaaa gaaattgtta aagatagaga gacaggctat     480

atatttcaag ggggaaatgt tgaggaacta gtctctatat tggaagtttt tttaggtcta     540

gaaaatgaaa aacgaaaaga gatgggactt caa                                  573


<210>  524
<211>  535
<212>  DNA
<213>  Streptococcus pneumoniae

<400>  524
aaatattgtt ggtttacttc gtgcaaatat ttgcttgata ttattaaaag tgtgtcgaaa      60

caaggatctt acttttcatt attttttaaa atttggaaag aatgtaactg tcgaacagga     120

atctaatgct aaattaatct taggaaaaaa gattagagta aacgccgggg gagtattgaa     180

agttagaaaa ggagcaaaac tcaagatttc tgatgatgta tttttgagta ataattgtat     240

gatagcttgt cgtaaataca tagatattaa atctggagta aaatgtggtc ctggagtact     300

tatatatgat catgactatg atgttagtgt tccaggtgga ttgaaagcaa aaaaatttaa     360

gacggcccca gttatgattg gagaaaatgt ttggattgga gctaacagca ttgtcttgaa 420

gggagtgagt attggtgaga atagtgtggt tgcagcagga agtgttgtaa caaaggatat 480

tccagctgat actatattta ttcagaaacg tttatcaagg gagatgaaat tatga 535

<210> 525
<211> 691
<212> DNA
<213> Streptococcus pneumoniae

<400> 525
ctaaagcatt tggagagatt gtactatgta gtcgttatga gaaggtggaa tcataccctg 60

aaggatatca taaagcgaac tttattaata aatttatccc tatagaaggt ctacatcagg 120

ttctatttgg tcaaaataaa aataagatta ttgaaggaat gattgatagc gacttaatag 180

ttgttcgtat tccgtctata attggatcaa aaactgcaga ctacgcattg aagataggta 240

agccgtatct gacagaaata atgggggatg cttgggattc ttactggtat catagtttaa 300

agggaaaatt attagctcca tatatatacg ccaaaactaa atcaattgta aaaaacgcta 360

attattgcat atacgtgaca gaaaaatatt tacaagatag atatcctaat attaaatcta 420

atatcgttgc ttcaaatgtt aatattacct ctgtagagaa tagatctttg aagagccgtc 480

tttataagtt gaaaaaattt aatcctcaaa aaatttcaat aatgacaaca gcatctgtga 540

atgtacgagc caagggccat agatttgtat tggaagcaat gaagagatta gaaatacaag 600

gtattttgtt ggattattat ttagcaggtg atggtgatca aagtttctta aaaaagaaag 660

cagaggaatt gggagtagcg aatagaatcc a 691

<210> 526
<211> 509
<212> DNA
<213> Streptococcus pneumoniae

<400> 526
tgtaggaact gatttggatg attctgagtt aacaaaaaga gcatggcagt ttgcagatct 60

acttaaaggt ggagctatta aggaagaggt tccgatactg gttgttgctt ttaatgaagc 120

agaggttgca aaattgttta gtaacactta cttggcaact cgcgtacgtt attttaatga 180

gatagataca tatagcgagg taaaagggct taatcccaag acaattattg atattgtttg 240

ttatgatcct agaattggat catactataa taaccctagc tttggttacg gagggtattg 300

cttaccaaaa gacacaaagc aattgaaagc aagttttagg gatgttcctg aaaatctgat 360

tacagctgtg gtgcaatcta ataaaacaag aaaagattat atagctggag ctattctagc 420

taaacaacct agtgttgtag gtatttatag attaattatg aaatctgatt ctgataattt 480

tcgttctagt gctgttaagg gagttatgg 509

<210>  527
<211>  695
<212>  DNA
<213>  Streptococcus pneumoniae

<400>  527
tttccagaca taaaccatcc gaaattattg tggttattaa cggcccaaaa aacgagagac      60

ttgtaaaact ttgtcatgat tttaatgaaa aattagaaaa taatatgact ccaattcaat     120

gttattacac tcctgttcct ggcaagagaa atgctatcct ctttgggctg gagcatgtgg     180

attcgcagag tgatattaca gttctagtag atagtgatac agtatggacg cctagaacct     240

tgagtgagtt gctgaagcct tttgtttgcg ataaaaaaat aggtggggta acgacaagac     300

aaaaaattct tgaccctgag cgtaatctcg tgacaatgtt tgctaacttg ttagaggaaa     360

ttagggcaga aggaactatg aaagcaatga gtgtgactgg taaagtaggg tgcttacctg     420

gtcgaacaat tgcttttaga acagagattc tcagagagtg tatacatgag tttatgaatg     480

agactttcat gggatttcat aaggaagttt ctgatgatag aagtcttaca aatttgactt     540

taaaaaaagg ctataaaact gttatgcagg atacttctgt tgtgtataca gatgctccta     600

caagttggaa aaagttcatt agacagcaac taaggtgggc agaaggttct cagtataaca     660

atctaaagat gactccttgg atgattagaa atgcc                                695

<210>  528
<211>  542
<212>  DNA
<213>  Streptococcus pneumoniae

<400>  528
tcgtcatctg tactggtctg ggcttgcttg taggaggatt tttcctgcta aaaccagctc      60

cacaaacacc tgtcaaagag acgaatttgc aggctgaagt cgcagctgtt tccaaggatt     120

tggtatccga aaaggaagtg aacaaggaag aaaaggaaga accccttgaa caagatctaa     180

tcacagtaga tgtcaaaggt gctgtcaaat cgccagggat ttatgacttg cctgtaggta     240

gtcgaatcaa tgatgctgtt cagaaggctg gtggcttgac agagcaagca gacagcaagt     300

cgctcaatct agctcagaaa gttagtgatg aggctctggt ttacgttcct actaagggag     360

aagaagcagt tagccaacag actggtttgg ggacagcttc ttcaataagc aaggaaaaga     420

aggtcaatct caacaaggcc agtctggaag aactcaagca ggtcaaggga ctgggaggaa     480

aacgagctca ggacattatc gaccatcgtg aggcaaatgg caagttcaag tcagtagacg     540

ag                                                                    542

<210>  529
<211>  545
<212>  DNA
<213>  Streptococcus pneumoniae

<400>  529
gtggaaatct gctggtaaag ttctaataat ttgcggaatc tttggatttt ggtttgtttt      60

tcaaaattgg caacagagtc aagcgagtca aaatctggcg gattctgttg aaagggtacg     120

gattctgcct gacactgtta aggtcaatgg tgatagtctg tcctttcgcg gcaaggctga     180

tggacgcatt tttcaagtct attataaact ccagtccgag gaggagaaag aagcctttca     240

agctttaacc gacctgcatg agataggact agaagggaag ctttcggagc cagaagggca     300

gagaaatttt ggtggcttta attaccaagc ctatctgaag actcagggaa tttaccagac     360

tctcaatatc aaaaaaatcc agtcacttca aaagattggc agttgggata taggagaaaa     420

cttgtccagt ttacgtcgaa aggctgtggt ttggattaag acgcactttc cagaccctat     480

gcgcaattac atgacaggac tcttgctggg acatctggac accgactttg aggagatgaa     540

tgagc                                                                 545

<210>  530
<211>  402
<212>  DNA
<213>  Streptococcus pneumoniae

<400>  530
gattatcgga gaaattcgtg acagcgagac ggcgcgtgca gtggtcagag ctagtttgac      60

aggtgcgaca gtcttttcaa ccattcacgc caagagtatc cgaggtgttt atgagcgtct     120

gctggagttg ggtgtgagtg aagaagaatt ggcagttgtt ctgcaaggag tctgctacca     180

gagattaatc gggggaggag gaatcgttga ctttgcaagc agagattatc aagaacacca     240

agcagccaag tggaatgagc aaattgacca gcttcttaaa gatggacata tcacaagtct     300

tcaggctgag acggaaaaaa ttagctacag gctaagcaaa aaaatatcat caccctattt     360

aacaatctct tttctagcgg ttttcatctg gtggagacta tc                        402

<210>  531
<211>  463
<212>  DNA
<213>  Streptococcus pneumoniae

<400>  531
tggacaagca gtgtgtgacc cagatgcgtg tgggcttgtc tcaggggaaa tcattctcag      60

aaatgatgga aagtttggga tgttcaagtg ctattgtcac tcagttatcc ctagctgaag     120

ttcatggcaa tctccacctg agtttgggaa agatagaaga atatctggac aatctggcta     180

aggtcaagaa aaaattgatt gaagtagcga cctatccctt gattttgctg ggttttcttc     240

tcttaattat gctggggcta cggaattacc tgctcccaca actggatagt agcaatattg     300

ccacccaaat catcggtaat ctgccccaaa tttttctagg catggtaggg cttgtttccg     360

tgcttgccct tttagcactc actttttata aaagaagttc taagatgagt gtcttttcta     420

tcttagcacg ccttcccttt attggaatct ttgtgcagac cta 463

<210> 532
<211> 322
<212> DNA
<213> Streptococcus pneumoniae

<400> 532
aaaaatgatg acattcttga aaaaagctaa ggttaaagct tttacattgg tggagatgtt 60

ggtggtcttg ctgattatca gcgtgctttt cttgctcttt gtacctaatc tgaccaagca 120

aaaagaagca gtcaatgaca aaggaaaagc agctgttgtt aaggtggtgg aaagccaggc 180

agaactttat agcttagaaa agaatgaaga tgctagccta agaaagttac aagcagatgg 240

acgcatcacg gaagaacagg ctaaagctta taaagaatac catgataaaa atggaggagc 300

aaatcgtaaa gtcaatgatt aa 322

<210> 533
<211> 380
<212> DNA
<213> Streptococcus pneumoniae

<400> 533
atgctggaaa gtctcttggt tttgggactt gtgagtatcc ttgccttggg cttgtccggc 60

tctgtccagt ccactttttc agcggtagag gaacagattt tctttatgga gtttgaagaa 120

ctctatcggg aaacccaaaa acgcagtgta gctagtcaac aaaagactag tttgaacttg 180

gatgggcaga tgattagcaa tggcagtcaa aagttgacag ttcctaaagg aattcaggca 240

ccatcaggcc aaagtattac atttgaccga gctgggggca attcgtccct ggctaaggtt 300

gaatttcaga ccagtaaagg agcgattcgc tatcaattat atctaggaaa tggaaaaatt 360

aaacgcatta aggaaacaaa 380

<210> 534
<211> 547
<212> DNA
<213> Streptococcus pneumoniae

<400> 534
ggctgtagga gacaatgaag ttcgtctctt gtctttgctt gagattgcca gtcaacgtag 60

cagtctggtg attttgacag gcggtttggg ggcaactgag gacgacctaa ccaaacaaac 120

cctagctaaa tttttaggga aagcattagt ctttgatcct caggctcagg agaagttgga 180

tatctttttt gccctgcgac cagactatgc ccgaacaccg aataacgaaa gacaagctca 240

aattgtagaa ggagcgattc cactgccaaa cgaaacagga ctggctgttg gaggaaaatt 300

agaagtagac ggagtgacct atgtcgtcct tccaggtccg ccaagtgaat tgaaacccat 360

ggtcttaaac caacttctac ccaagttgat gacagggagc aagctgtatt cccgagttct 420

tcgtttcttt gggattggcg agagccagtt ggttacgatt ttggctgatt taattgataa 480

tcagatcgat cctaccttgg ccccttatgc caagacagga gaagtcactc tacgtctgtc 540

aacaaag 547

<210> 535
<211> 520
<212> DNA
<213> Streptococcus pneumoniae

<400> 535
ttgttagaga gacagaactt gaacgttctt cgatggttat actatacctt ctccactttt 60

ttgtattcta ttttagttcc tatggtaaca aattttttaa aagagggtac ctagttgagt 120

ttaatagtac tataagatat atttttttct ttgcaatagc tataagtgta ttaaactttt 180

ttatagcgga acggtttagt atctctagaa gaggaatggt atacttctta actttagaag 240

gaatatcctt atacttgtta aatttcttag taaagaaata ttggaagcat gtgtttttta 300

atccaaaaaa tagcaagaaa attttactgt taacagtaac ggaaaatata gaaaaagttc 360

ttgataaatt gctagaatct gatgaacttt catggaaact ggtagcagta agtgttttgg 420

ataaatctga ttttcaacat gataaaatac ctgtaattga aaaggaaaaa attattgaat 480

ttgcaacgca tgaagttgtg gatgaggtgt ttgtcgatct 520

<210> 536
<211> 210
<212> DNA
<213> Streptococcus pneumoniae

<400> 536
aatattttat ccatgttatt atcctactaa tcgtaatcta aaaaatctta ttaaaaatac 60

gattcttgct ttcaaaattt tgagaaagga acgccctgat attatcgtct catcaggggc 120

agctgtagca gttcctttct tttatctagg gaaaatattt ggtgctaaga cagtctatat 180

agaagtattt gatagaattg atgctccgac 210

<210> 537
<211> 405
<212> DNA
<213> Streptococcus pneumoniae

<400> 537
tgagggattt attcaggatg atgtttttat tcaaacagga tactctaatt atgttccaaa 60

attttgtaaa tgggaaaaat taatatctta tgaaaaaatg aatcaattga ttaaggaatc 120

agatattatc attacccatg gcggtccagc tacgtttatg gcagttattg ctaaaggtaa 180

aaatccaata attgttccgc ggctaaaaaa atttggtgag catgtaaatg atcaccagat 240

gcaatttgta aaaataacga aagaaatata caatttaata gttatagatg atatttcaga 300

cttacattta attcttcata attttaagga caaacatttt gaaacttatt tgaataacga 360

gagatttaat gtacgtttca atgtggaaat cagtaacctt tttaa 405

<210> 538
<211> 622
<212> DNA
<213> Streptococcus pneumoniae

<400> 538
tgctttaact cttttaccaa cctatataaa agaaaaacaa gtttttaaaa tagatacacc 60

gtctttttgt atggtgctat ggactattat atattctata tctataatat ttaattctct 120

gattgatgga ttggctgttc aagtgttatt ttcagatttg agtaaagcat ttaattggct 180

aatagcagta tttttttata attattattt gaaaatgcca atcaatattg acaggataaa 240

gagatatatg tattataatt ttactatctt agttgttttt gtcggtttat tctatataca 300

aagaggctcc aatgtaattt tgtttggaag aagtttgtta gactgggacg gatttacatt 360

agctactagt tatggtgtaa gatatacagg ttttttagaa tacgcaactt taaatggtca 420

gttaattctt tttttattac cgttaattag attgtttaga tttagatttt ttacacaaac 480

tatcattttt gcttttcttc tagaggtttt ggtactaagc aaatctagaa tagcgattgt 540

tgcaatgctt atatatatag catttgcagt agtcaatgag attaattcaa acaataaatg 600

gcttattgga attttctgtc ca 622

<210> 539
<211> 687
<212> DNA
<213> Streptococcus pneumoniae

<400> 539
aaggcaattc caatacaaag cagatcgtta ttattgataa tttttctaat aacggtacgg 60

gtgaaaaact acaagagctg tatgagtcag attcagagat tgatgtcttg attaaccatg 120

aaaatgctgg tttcgctcga ggtaataatg tagcatatca gtttgctaag gaaaagtaca 180

aacctgattt tatggttatc atgaataatg atattgagat agaaacagaa gagtttgaaa 240

aaatcgtgac agatatctat cggaaggaaa aattccattt gttaggacca gatatctttt 300

cgacgacgta tcagcttcac caaaacccaa aacggttgac gcattatact tatgaagagg 360

ttaaggctct caatgaaaaa tttaagaaag ggagccaagt tagtctagca ttaaaaatta 420

aatgttggtt gaagtctagt aaagttcttc ggacagcaat ctatcaaaat aggcgtaaaa 480

agaaatcagt agactataga aaacaggtag aaaacccaat tcttcatggt tcgtttattg 540

tatattctag agatttaatt gagaaagagg agtatgcttt taatcccaat accttcttct 600

attatgaaac agagatatta gattatgaag ctgagttaaa aggatataag agaatttata 660

caccgaagat taaggtcttg caccatc 687

<210>  540
<211>  534
<212>  DNA
<213>  Streptococcus pneumoniae

<400>  540
tttcaatgcc tctcttggct cttaattcgc ctagtctaaa taccaagatt aaagtgacgg      60

atcggctcat tgatatccaa ttctggaaaa tagctcttac tattatagtt gacctcatta     120

ttctatatct ttataggaga gagattcata atcttgcact tagccatggt tatacgggtt     180

caaattttca gtggttcttt agaaatgcta ccagttatga aggtgagcta acagtgcgaa     240

cttcgattcg ggtcctcatt cgtatcattg acgtatctgc ttatattttt ggatatactt     300

ttattaataa tttcttcatt tatagtcata aacgctctaa agatttactg ctcttagttc     360

cattcttgat ttttatttct aaaaccttat tatctggggg tagattggat attataaaaa     420

ttttaattgc gtatgttgta atggcctata ttcagcaaaa acgaaaagtt ggctgggata     480

aggtcatctc ccataaatat atgagacttg gttttgtagg cttgatagct ggga           534


<210>  541
<211>  450
<212>  DNA
<213>  Streptococcus pneumoniae

<400>  541
tccattagtc aatgagttga aaaaacacga agatatggaa acaattgtgt gtgttactgg      60

acaacacaaa gagatggtta gtcctgtttt agatttattt ggtgttgtac cagattatga     120

tttagaaatt atgaaggcta accaaacctt gttctctatc acaactagta tcttggaaaa     180

gataaaacca gttttagaga aggaacaacc agatattgtc ctagttcacg gtgacactac     240

gacaacttat gcagcagcct tggcagcatt ctatttggga attaaagtag gacatgttga     300

agctggtttg cgaacgtaca atttacaaag tccatttcct gaagaattta acaggcaatc     360

gacatcaatc attgcaactt accattttgc tccaactgag ttggctaaag aaaatctctt     420

aaaagaaggt agagagaatg tttatgtgac                                      450


<210>  542
<211>  565
<212>  DNA
<213>  Streptococcus pneumoniae

<400>  542
gaagcatacg acaaacttcc aagtgttttc aaagatagaa ttatcgctgg gaaatatcag      60

gttcttactt atcaatactg tgatacgttg cattgctact ttcctcgact attcctttta     120

gcagatgaaa gaaaacgttt gggcttgcca cgaaatacca atctaggatt gcatttgatt     180

gatatcattc ctttagatgg agcaccaaat cattcggttt taagaaagat ttacttttgt     240

aaagtatact ggtatcgttt tttagcaagc ttaggaacaa cttatgttgg cgaccatgtg 300

gatatgcatt ccactaagca aaaactaatt attggtttct ttaaaaaact aggatttgca 360

aaactatttc ctcaaaattc tgtatacaga cgcttggata atctctatag aaagtatgat 420

tggaaaaagc agaagtatgc ggggactatc aatgcttctt tatttgctaa agaagttatg 480

ccagtagaga tttggggaga aggagtagag aagccttttg aggatacctt ctttaaagtt 540

ccaacggagt atgatcgcta cctga 565

<210> 543
<211> 662
<212> DNA
<213> Streptococcus pneumoniae

<400> 543
gtgatagtga acttgggatt gtctagtatt attcagtaca tttcttattt tatgttgatg 60

ttgtgtgtat ttttaacatt aattaagaat actctcaacg tgtttgcaaa tagaatcata 120

tattttttga ttatttcatt tttgtttatt attgggatta atttacaaaa tcttccatta 180

tcaagaaaga tttatttatc attctctatg ttaattattt ctagcttatc caccttaccg 240

ataaagctaa taaataatct cagtgattta agaaggatat catattactt attgcacagc 300

atatttttat ctgtattttt aggtttggtt tttaaaatat ctttagtaac agttgctgta 360

gagggaattg gcttttcata tggttttaat ggaggtttga ctcataaaaa tttttatgca 420

attacaattt tagtttccta tattctacta tatgtcagca gaaaatatga cgctaaacat 480

cagattgata gttttgtatt atggttagat ctttttttac ttttaatatc taatacgcga 540

acagtttata taatactagt tgttttttgg attattatta atagaaattt tataaataat 600

attaaaaaag agcatagact ggtagtgaca gcaacgacaa tagtcatctc tttactggcg 660

tt 662

<210> 544
<211> 380
<212> DNA
<213> Streptococcus pneumoniae

<400> 544
agagcaaaaa cgctggtttc tcaacaggtc aaccttggtt ggcggttaat ccaaattacg 60

agatgatcaa cgtacaagaa gcgctggcaa atccagattc tattttctat acctatcaga 120

aactggtcca aattcgcaag gagaatagct ggctaattcg agctgacttt gaattgcttg 180

atacggctga taaggtcttt gcttatatac gtaaggatgg cgaccgtcgc ttcctagttg 240

tggctaactt gtccaatgaa gagcaagact tgacagtaga aggaaaagtc aaatctgtct 300

tgattgaaaa caccctagct caagaagtct ttgaaaaaca aatcttagtt ccatgggatg 360

ctttctgtgt ggaattacta 380

<210> 545
<211> 610
<212> DNA
<213> Streptococcus pneumoniae

<400> 545
acgaacagtg gacctgatac atggtccgat tcttccctcg ctcttaagct tcacctttcc 60

aatcttgcta tcaaatattt ttcaacagct ctataacact gctgatgtct tgattgttgg 120

acgatttctt ggtcaagaat ccttggctgc agtaggagcg acgacagcga tttttgacct 180

gattgtaggc tttacacttg gtgttggcaa tggcatgggg attgtcattg ctcgttatta 240

tggggctcgg aatttcacta aaatcaagga agcagtagca gccacctgga ttttaggtgc 300

tcttttgagc attctagtta tgttgctggg ctttcttggc ttgtatcctc tcttgcaata 360

cttagatact cctgcagaaa ttcttcctca atcttatcaa tatatttcta tgattgtgac 420

ctgtgtaggt gtcagctttg cttataatct ttttgcaggc ttgttgcggt ctattggtga 480

cagtctagca gccctgggat ttctgatttt ctctgccttg gttaatgtgg ttctggatct 540

ctattttatt acgcaattgc atctgggagt tcaatccgca ggacttgcta ccattatttc 600

gcaaggttta 610

<210> 546
<211> 546
<212> DNA
<213> Streptococcus pneumoniae

<400> 546
ttgtcttgac tggttgtgtc aatgtcgata aaaccacagg tcagccaaca ggatttattt 60

ggaataccat cggagcccct atggctgaag ctatcaagta cttcgctact gataaaggtc 120

taggctttgg tgtcgctatc atcatcgtaa ccattatcgt gcgcttgatt atcttgccac 180

ttggtatcta ccaatcatgg aaggcaacgc ttcactctga aaagatgaac gccctcaagc 240

acgtccttga gccacaccaa acgcgtctca aagaagcgac tactcaagaa gaaaaactcg 300

aagcccaaca agctctcttt gctgctcaaa aagagcacgg tatcagcatg tttggcggtg 360

taggatgttt ccctatcctc cttcaaatgc ctttcttctc tgctatctac tttgctgccc 420

aacatactga aggggttgct caagcaagct acctaggcat tcctctaggt tctccaagta 480

tgattttggt tgcctgtgct ggtgtccttt actatcttca atcgctcctt tcacttcacg 540

gagtag 546

<210> 547
<211> 262
<212> DNA
<213> Streptococcus pneumoniae

<400> 547
tgcaaaaggt tagaatgatt gcccaaggta gggtacaggg agtcggcttt cgttggggtg 60

tttacagctt ggcacttgaa attggtggca tcacaggtcg agtatggaat aacgacgatg 120

gcacagtgga aatcttagcc caagcagact catctgctat catggcaaaa tttatccaag 180

aaatccgaaa aggaccgaca cctttttcaa aagtaagcta cttagatgtc aaactaagca 240

actttcctcc ctactctgac tt 262

<210> 548
<211> 629
<212> DNA
<213> Streptococcus pneumoniae

<400> 548
gttcggtaat ccagttgaag tcccttttga acattgggaa ctagaacatt gaattccaat 60

tctccatccg aagacttggc ttcaatctgt gaagctgagg gaactaaatc ctcgtttgaa 120

gcgtagtaaa gggttacacg gtaacggaca cgcttgtttt ggtccaaggc tttacgcacc 180

ttgctttcat agtagttttg accagtcgaa tactcggctt gtgcctgatt tgcccaggct 240

gtctgaacag caatgttttt aggattgctt gttgaggcat caaaaccatc caaaccaccg 300

attaaggcat agcctaacaa atgacctcta tcgactgcat gggtataaga gccctttaga 360

ttcttgacct gatgccaacc tggaggagtc caagaagttg aaccattccc agtttcttta 420

cgattcttgt actgacgagt ggccttagac aagagggcat tagctacggt tggaacagtt 480

tccttgccca ctgtctttgt tttattgtca gcgtagggct tacttgaaac cttggcatct 540

agatttgttt tattaccatt gacgataaaa gcacctgagc cattccactc cagactcccc 600

tttatttgac tcttgactgc gtctgttaa 629

<210> 549
<211> 323
<212> DNA
<213> Streptococcus pneumoniae

<400> 549
cgtggaaatt ttagaagaac tcttcccagg ctacgaaaac acgtggcgtt cttcccaaga 60

gcctgcccgt aaaggctatg ctggaaccat gttcctttat aagaaagaac ttacacctac 120

tatcagcttc ccagaaatcg gtgccccttc taccatggac ttggaaggtc gtatcatcac 180

tctagaattt gatgcatttt tcgtaaccca agtttacact ccaaacgctg gtgacggtct 240

caaacgcttg gaagaacgcc aagtctggga tgccaaatat gctgagtatt tggctgaact 300

agacaaagaa aaaccagtcc ttg 323

<210> 550
<211> 206

<212>  DNA
<213>  Streptococcus pneumoniae

<400>  550
aaaatttggg ggattcagtt agctgcaatt aaaaaaattg gtgttttgag ggaagaacgt      60

ataagcccca atcagctttg gcatgcactg gaaacagatt atgccggaga agaaggtaag     120

gtcattcaag aaatgttgat tcatgatgca cctaagtatg gtaatgatga tgattatgct     180

gacaaattgg ttactgctgc ttatga                                          206


<210>  551
<211>  510
<212>  DNA
<213>  Streptococcus pneumoniae

<400>  551
cctctaaggc tatgatggaa aagattgctg ttgctaagtc aaggacggta gaagaagatc      60

agacaaaagt ctgtgtaact cgctacggca atgttctatg tagtcgtggt tctgtgattc     120

ccctatggat tgatcaaata aagcaaggga atcctataac gattacggaa cctagtatga     180

ctcgttttat tatgtcctta gaagaagcgg tagacctagt tctgtttgct tttgaaaaag     240

gaaaaacagg agatatccta gtacagaaag caccagcatg taccattgaa gtgttggcgc     300

aagctgttac ggaacttttt gcacctaatc aagatattaa agtaatcggg attcgccacg     360

gtgaaaagat gtatgaaacg ttgttgacta ctgaagaatg tacgaatgcc attgatttag     420

gcggctttta tcgtgtgcct agcgataatc gagatcttaa ctatgataag tatttcaacg     480

aaggggatgc caaacgcaat cccttaatag                                      510


<210>  552
<211>  589
<212>  DNA
<213>  Streptococcus pneumoniae

<400>  552
tgaaagatgg acgagatagg actcgtccta atttagagat tggagagatt tttcagtatg      60

atcgtgatac agatccgatt ttattagatg aatattgtaa gaaggccgat ttcgtattcc     120

atttagctgg tgtcaatcgt ccacagaatc ctgatgaatt catggaggga aattacggtt     180

tttcaagtag attattggag attttagaaa agtatgaaaa cacttgtcct gttctactct     240

caagttctac tcaagctagt ttagaaggcc gattttcaaa ctctatatat ggacaatcta     300

agctagtagg ggaagaactc ttctttgaat atggaaagaa aacgggagca cctgtcttag     360

tttaccgttt cccgaatctt tatgggaagt ggtgccgtcc taactacaat tctgctgtag     420

caactttctg tcataatcta gctcacgatt tacctattca agtaaatgat ccaagtgtag     480

aattggagtt gctgtatatt gatgatttga tacaagagtg tctaactgca ttggaaggaa     540

atcctcatcg ttgtaatcta gatggattac aaatcttacc tagcccatc                 589

<210>  553
<211>  545
<212>  DNA
<213>  Streptococcus pneumoniae

<400>  553
tacatggatg ctgttggaga tgatcttggt gctactgtag ggaatattat taatacttca      60

tacaaattga tgaatcaaat taaaccagat gctttattga ttttagggga tacaaattct     120

tgtttatcag ctattactgc caagcgttta catattccaa tttttcatat ggaggctggc     180

aatcgctgta aggatgagtg cctgccggaa gagactaatc gtcggattgt tgatattatt     240

tcagatgtta acttagcata ctctgaacat gcacgtaagt atttacatga gtgtggttta     300

cctaaagagc gcacatatgt aacaggttct cctatggcag aagtgttaca taaaaattta     360

tctgccattg agtcttcaga tatccatgaa cgtttgggat tgaaaaaagg aggttatatc     420

ttactttcag ctcaccgtga ggaaaatatt gatacagata aaaattttat ttctctcttt     480

acagcaatta atcaattagc tgaaaagtat aatatgccaa tcttatattc ttgccatcct     540

agatc                                                                 545

<210>  554
<211>  250
<212>  DNA
<213>  Streptococcus pneumoniae

<400>  554
catatggtac atttgattta ttgcattatg gtcatatcaa tcttttgaaa cgtgctaaac      60

agctaggtga ttatttgatt gtagttgttt caagtgatga gtttaattta aaagaaaaga     120

ataaagtatg ttactttaac tacgaacaca gaaaaaattt agtagaagct attcgatatg     180

tcgatttagt aatccctgaa actagttggg aacagaaaaa gtcagatgtt aaagactacc     240

atattgacac                                                            250

<210>  555
<211>  283
<212>  DNA
<213>  Streptococcus pneumoniae

<400>  555
ctcctagtgc cctatatctt tgaatttcct gcggatgatg ccctgcgtct caaggaaaga      60

atgcctctct tagaggaagt gggcgtcttt ctagcagagt acggagaaaa tcaatttatt     120

ctacgtgaac atcctatttg gatggcagaa gaagagattg aatcaggcat ctatgagatg     180

tgcgacatgc tccttttgac caaggaagtt tctatcaaga aataccgagc agagctggct     240

atcatgatgt cttgcaagcg atctatcaag gccaatcatc gta                       283

<210>  556
<211>  284
<212>  DNA
<213>  Streptococcus pneumoniae

<400>  556
cttggtgcac agagtcctca aaaatcaatt tcagaacaaa cagcttatga aattgatgaa      60

gaggttcgtt cattattaaa tgaggcacga aataaagctg ctgaaattat tcagtcaaat     120

cgtgaaactc acaagttaat tgcagaagca ttattgaaat acgaaacatt ggatagtaca     180

caaattaaag ctctttacga aacaggaaag atgcctgaag cagtagaaga ggaatctcat     240

gcactatcct atgatgaagt aaagtcaaaa atgaatgacg aaaa                      284


<210>  557
<211>  627
<212>  DNA
<213>  Streptococcus pneumoniae

<400>  557
aagtaggcga tggttatgtc tttgaggaga atggagtttc tcgttatatc ccagccaagg      60

atctttcagc agaaacagca gcaggcattg atagcaaact ggccaagcag gaaagtttat     120

ctcataagct aggaactaag aaaactgacc tcccatctag tgatcgagaa ttttacaata     180

aggcttatga cttactagca agaattcacc aagatttact tgataataaa ggtcgacaag     240

ttgattttga ggctttggat aacctgttgg aacgactcaa ggatgtctca agtgataaag     300

tcaagttagt ggaagatatt cttgccttct tagctccgat tcgtcatcca gaacgtttag     360

gaaaaccaaa tgcgcaaatt acctacactg atgatgagat tcaagtagcc aagttggcag     420

gcaagtacac agcagaagac ggttatatct ttgatcctcg tgatataacc agtgatgagg     480

gggatgccta tgtaactcca catatgaccc atagccactg gattaaaaaa gatagtttgt     540

ctgaagctga gagagcggca gcccaggctt atgctaaaga gaaaggtttg acccctcctt     600

cgacagacca tcaggattca ggaaata                                         627


<210>  558
<211>  784
<212>  DNA
<213>  Streptococcus pneumoniae

<400>  558
gcatctctcg ttatgtcttt gcgaaagatt taccatctga aactgttaaa aatcttgaaa      60

gcaagttatc aaaacaagag agtgtttcac acactttaac tgctaaaaaa gaaaatgttg     120

ctcctcgtga ccaagaattt tatgataaag catataatct gttaactgag gctcataaag     180

ccttgtttga aaataagggt cgtaattctg atttccaagc cttagacaaa ttattagaac     240

gcttgaatga tgaatcgact aataaagaaa aattggtaga tgatttattg gcattcctag     300

caccaattac ccatccagag cgacttggca aaccaaattc tcaaattgag tatactgaag     360

acgaagttcg tattgctcaa ttagctgata agtatacaac gtcagatggt tacatttttg 420

atgaacatga tataatcagt gatgaaggag atgcatatgt aacgcctcat atgggccata 480

gtcactggat tggaaaagat agcctttctg ataaggaaaa agttgcagct caagcctata 540

ctaaagaaaa aggtatccta cctccatctc cagacgcaga tgttaaagca aatccaactg 600

gagatagtgc agcagctatt tacaatcgtg tgaaagggga aaaacgaatt ccactcgttc 660

gacttccata tatggttgag catacagttg aggttaaaaa cggtaatttg attattcctc 720

ataaggatca ttaccataat attaaatttg cttggtttga tgatcacaca tacaaagctc 780

caaa 784

<210> 559
<211> 502
<212> DNA
<213> Streptococcus pneumoniae

<400> 559
gaccattacc actttattcc ttacagcaag ctttctgcct tagaagaaaa gattgccaga 60

atggtgccta tcagtggaac tggttctaca gtttctacaa atgcaaaacc taatgaagta 120

gtgtctagtc taggcagtct ttcaagcaat ccttcttctt taacgacaag taaggagctc 180

tcttcagcat ctgatggtta tatttttaat ccaaaagata tcgttgaaga aacggctaca 240

gcttatattg taagacatgg tgatcatttc cattacattc caaaatcaaa tcaaattggg 300

caaccgactc ttccaaacaa tagtctagca acaccttctc catctcttcc aatcaatcca 360

ggaacttcac atgagaaaca tgaagaagat ggatacggat ttgatgctaa tcgtattatc 420

gctgaagatg aatcaggttt tgtcatgagt cacggagacc acaatcatta tttcttcaag 480

aaggacttga cagaagagca aa 502

<210> 560
<211> 462
<212> DNA
<213> Streptococcus pneumoniae

<400> 560
tttcatttct tatttcctcc aaaatactct cgggaacata gcaggcaata tcctgaccaa 60

acttcaaaag ctctctaacg cctgatgaac tgatatagag atgttcaggt cgactatgta 120

aataaatagt ctctatatca gaagacagct gatgattgta gtaatcaaaa ctggcttcat 180

attgcaaatc cgacgcattc ctcaaaccac gcactagaca agtagcaccc aatctttttg 240

caacatcgac caccaattca tcatgagaag ccacgacttc aacattttcc agatgtccca 300

aagccttttc tagcccccgt ttacgatttt cgataggaag aaatccttgt ttgtggggat 360

taaaaaaaat acccacataa agcttatcaa aaagtctgct cgcccgttca atgatatcca 420

gatgcccatt tgtcatcgga tcaaatgagc ctgtgaataa gc 462

<210> 561
<211> 508
<212> DNA
<213> Streptococcus pneumoniae

<400> 561
gatttctgta tgaggcagtt cgcgattgac tgcaaattta tcattgtttt cacagagtga 60

accaaccaca tctaccgctt cagctggtcc atctggatgg gtcacgttgc taatatgatg 120

gtaagctccg tacatagctg gacgcatgag gttgactgct gaggcatcca cacctagata 180

ggtacggtag gtttccttct tatgagtgac tcttgtgact agagcaccgt gaggtgccag 240

cataaaacga cccaattcgg tgaaaatctt gacctgacca agacctgctg acgtaagaac 300

ttcttcatac accttacgaa ctccctcacc aatcaaggcg atatcgttcg gctcctggtc 360

tggacgataa ttaacaccaa taccgccaga aagattgata aagtctagcc aaatgcccaa 420

cttttccttg atttcaacag ccagttcaaa gagctgacga gccaactctg gataatagag 480

atgggtcacg gtattggacg ctaggaag 508

<210> 562
<211> 652
<212> DNA
<213> Streptococcus pneumoniae

<400> 562
ggctgttagt ccaagtcaag aactatttgg aaagatcaaa gtaaaggaag ttcgttattt 60

gaaggaattt agaaatttaa attctaagga tgcaagggaa tatgacttgg ctttattaat 120

tctagaaaag cccattggtg caaaattagg gactttgggt cttcctacta gtcaaaaaaa 180

tttgacagga ataactgtga ctatcacagg ctatccatca tataatttta aaattcatca 240

aatgtataca gataaaaaac aagttttaag tgatgatggc atgttcttgg attaccaagt 300

tgatacttta gaggggtcta gtggatctac agtttatagt gctagtcacc gtgtagtagg 360

agtgcatact ttaggagatg gagctaatca aattaacagt gcagttaaat taaatgaagc 420

aaattgccat ttacttattt attcggttct taaaggttac tctcttgaag gatggaagaa 480

aataaatggt agttggtact attatagaca acatgataaa caaacgggtt ggcaggagat 540

aaatgatact tggtattatt tagacagttc cggtaagatg cttacagatt ggcaaaaagt 600

aaatggaaac tggtattatc tcaattcaaa tggagcaatg gttacaggta gc 652

<210> 563
<211> 250
<212> DNA
<213> Streptococcus pneumoniae

<400> 563

cttgtgctgt tcttcgttga tttccttgat atccaaaaga accaagtcag tgacagccat 60

gagtttgtca aacttctcaa ggtaacgcgg tttattacgg aaaggaagag cacaggtgtc 120

caaggtacag tggattcctt gttccttagc cttggtgaag agagcaatca ggaaatcaat 180

ctgcaagaga gcttctcctc cactgactgt aatcccaccc ttatttcccc agaaaccacg 240

gtagcgcaag 250

<210> 564
<211> 500
<212> DNA
<213> Streptococcus pneumoniae

<400> 564
ttgatatcca acaactacaa aaagacgaag taaacaatat tacatatttt gctgaaaatg 60

ctgctggcga agactgggat ttatcagata atgtcggttg gggtccagac tttgccgatc 120

catcaaccta ccttgatatc atcaaaccat ctgtaggaga aagtactaaa acatatttag 180

ggtttgactc aggggaagat aatgtagctg ctaaaaaagt aggtctatat gactacgaaa 240

aattggttac tgaggctggt gatgaggcta cagatgttgc taaacgctat gataaatacg 300

ctgcagccca agcttggttg acagatagtg ctttgattat tccaactaca tctcgtacag 360

ggcgtccaat cttgtctaag atggtaccat ttacaatacc atttgcattg tcaggaaata 420

aaggtacaag tgaaccaatc ttatataaat acttggaact tcaagacaag gcagtcactg 480

tagatgaata ccaaaaagct 500

<210> 565
<211> 525
<212> DNA
<213> Streptococcus pneumoniae

<400> 565
aggaaacaga aaataaagag aaacataaag atattcataa tgctatagaa acttcaaagg 60

atactgaaga aaagaaaaca acaattattg aggaaaaaga agttgttagt aaaaatcctg 120

taatagacac taaaactagc aatgaagaag caaaaatcaa agaagaaaat tccaatcaat 180

cccaaggaga tcatacggac tcatttgtga ataaaaacac agaaaatccc aaaaaagaag 240

ataaagttgt ctatattgct gaatttaaag ataaagaatc tggagaaaaa gcaatcaagg 300

gactatcaaa tcttaagaat acaaaagttt tatatactta tgatagaatt tttaacggta 360

gtgccataga aacaactccg gataacttgg acaaaattaa acaaatagaa ggtatttcat 420

cgattgaaag ggcacaaaaa gtccaaccca tgatgaatca tgccagaaag gaaattggag 480

ttgaggaagc tattgattac ctaaagtcta tcaatgctcc gtttg 525

<210> 566
<211> 250

<212>  DNA
<213>  Streptococcus pneumoniae

<400>  566
cattgaaacc aggagaaaag gtagcagaag ctaagaagaa ggttgaagaa gctaagaaaa      60

aagccgagga tcaaaaagaa gaagatcgtc gtaactaccc aaccaatact tacaaaacgc     120

ttgaacttga aattgctgag ttcgatgtga aagttaaaga agcggagctt gaactagtaa     180

aagaggaagc taaagaatct cgaaacgagg gcacaattaa gcaagcaaaa gagaaagttg     240

agagtaaaaa                                                            250


<210>  567
<211>  280
<212>  DNA
<213>  Streptococcus pneumoniae

<400>  567
aaaagcgaaa gttaagagtg aacaagctga ggctacaagg ttaaaaaaaa tcaagacaga      60

tcgtgaacaa gctgaggcta caaggttaga aaacatcaag acagatcgtg aaaaagcaga     120

agaagctaaa cgaaaagcag aagcagaaga agttaaagat aaactaaaga ggcggacaaa     180

acgagcagtt cctggagagc cagcaacacc tgataaaaaa gaaaatgatg cgaagtcttc     240

agattctagc gtaggtgaag aaactcttcc aagcccatcc                           280


<210>  568
<211>  414
<212>  DNA
<213>  Streptococcus pneumoniae

<400>  568
aagatattgc caacctcaac atcgatggct tggttcttga catcaatatt agttaccttc      60

aagagtgact tgtagtcaaa ctgcttttca cgttcttctt gggcattgtc cgcaaagacc     120

gctacacctg ccagttctga gtcgaactcg cgcaagagac taatcatacc gttgaccgtt     180

ccgccacctt tcaagaagtc atccacaatc aagacacggc tgcctgcctt aagactacgt     240

tttgaaagga acattttctc gatacggtca ccacttgaac ctgaaacata gttgacgcta     300

acagttgaac cttcggtaat tttcaggtca cggcgcacaa tgacaaaaga gacattgagg     360

acattggcaa ctgcatttgc aagtggcaca cccttagttg ctacggtcat aacc           414


<210>  569
<211>  312
<212>  DNA
<213>  Streptococcus pneumoniae

<400>  569
gcttgtatga cgatagagga gttagaagag gtcaaaaact cagcggcagg aacctttgtt      60

actaagacag cgaccttgga cttccgtcag gggaatcctg agccacgcta ccaagatgtt     120

ccacttggtt ccatcaactc tatgggcttg ccaaataatg gcttagacta ttatttggat 180

tatcttttgg atttgcagga aaaagagtcg aaccgaactt tcttcttatc tctggtcggc 240

atgtctccag aggaaaccca tactattttg aaaaaagtcc aagagagtga ttttcgtggt 300

ctgactgagc ta 312

<210> 570
<211> 599
<212> DNA
<213> Streptococcus pneumoniae

<400> 570
ttgtagggct aggaacaggt tctactgcct attattttgt cgaagaaatc ggtcgtcgaa 60

tcaaggaaga aggcttgcag attacagctg tgacgacttc tagtgtgacc agtaaacagg 120

ctgaagggct caatatcccg ctcaagtcta ttgaccaagt agactttgtc gatgtgacag 180

tcgacggggc ggatgaagtg gatagtcagt ttaatggaat caaaggcggt ggtggtgccc 240

ttctcatgga aaaggtggtc gcaacaccat caaaagaata catttgggtg gtggatgaaa 300

gcaagctggt cgaaaaacta ggtgctttta aattgccagt agaagtggtt cagtatggtg 360

cagagcaggt ctttcgtcat tttgaacgag ctggctacaa accaagtttc cgtgaaaaag 420

acggccaacg ttttgtgacc gatatgcaga attttatcat tgacctcgcc ttggatgtca 480

ttgaaaatcc aattgctttt ggacaagaat tggaccatgt cgttggtgtt gtggagcatg 540

gtttattcaa ccaaatggtg gataaggtaa tcgttgctgg acgagatgga gttcagatt 599

<210> 571
<211> 450
<212> DNA
<213> Streptococcus pneumoniae

<400> 571
atgacgcgct atgctttgct ggtgagaggt atcaatgttg gtggtaagaa taaggtcgtc 60

atggcggagc ttcgtcaaga attgacaaac ttgggactgg aaaaggttga gagctacatc 120

aatagtggca atattttctt tacttcgata gattccaaag cccaattggt tgaaaagcta 180

gagactttct ttgcagtcca ttatccattt attcagagct tttctttact gagtctagag 240

gactttgagg cggaacttga aaatctacca gcttggtgga gcagagactt ggcacgaaaa 300

gattttctct tttacactga gggtttggat gtggaccaag tcatcgcgac agttgaaagt 360

ttagagctga aagatgaagt gctttatttt ggaaaacttg ggattttctg ggggaaattt 420

tctgaagaat cctattctaa gactgcctat 450

<210> 572
<211> 527
<212> DNA
<213> Streptococcus pneumoniae

<400>  572
aaccatgtat tgaggaaagt gacaactttc agtgctccta aagtgattac ttccagaact      60

gtttggaatg ctaagaatgg tttctgggat gttggtttgg aaagtcgtaa attagctgtt     120

agtggaaaaa ttaagcatta tgtggttgat aatgacaatg ttgtgactcc cttgattcat     180

aataatcgtg atattgttac atttacaggt aattcacgct ttaaacaccg ttctcgtggc     240

tattttgaaa gtccaatgaa tgatattcct aactttaata ttggtaaaca agctaccttg     300

gataaacatg gttatcgtga tccgaaattg gataaagtgc gattctttaa gaaacaggct     360

ctgcctcgat cttctagtca accaagcgct gaaccaatgg aaaatattgc ctcaggaaaa     420

caggttactc aaagttcgac agctttcgga ggagatgcta gaagagctgt ggatggcaaa     480

gtcgatggta actatggtca caattctgtc actcatacaa acttcca                   527


<210>  573
<211>  561
<212>  DNA
<213>  Streptococcus pneumoniae

<400>  573
ggacttcctt ttcttatcca gaaattgatc tagctctctc tgatttcgaa gaatagtgac      60

tttatgtgaa tattcttggc aaagtttttg gtaattttct ttttgagttt tgctacgccc     120

atcccaaaga atccatctga taaactccca ctcaaagcgt tcagggcaat ctaccgccat     180

actttctctg acttttccac ggtatttaag ataacgctta aaggctctaa agagacaggt     240

caatggcgaa aaattgagaa agatgatttg gtcagcttct tgcattcgtt cttggtagta     300

gcaccaagaa taattaccat cgatgaccca agctttatgc ttggtgagaa agttttttat     360

ctcggttaac atccattcgc agtcactgtc ttgccaacca ggttgaaatt ggagtgtgtc     420

catgtgcagt tttggaatgg agtagtagtt agataacttt tctgctatag ttgacttacc     480

agaaccagaa tatccgataa ttgcgatttt catttctacc cttttcctat ttggagacaa     540

aaaaacagcc tctatggact g                                               561


<210>  574
<211>  503
<212>  DNA
<213>  Streptococcus pneumoniae

<400>  574
tttgcagatt tgtgagattg atactgatcc agacctgatg ggcaaaggac tccacttccc      60

caatcggcat ctgagtaaaa cgataatagt agctatcagg gtcgttttgg gctagactca     120

gcattttcaa gaaacggtcc agataccaac tttcaatatc atccactcca gcatctacat     180

agatggaaaa gtcaaagaag tcagtgatat agagacgatc gttttgtgga ttttgaaaga     240

cattgattcc ctcaacaatt acaaaatcag cagctttgac actttgtttc tcttcgggta     300

cgatgtcgta aacttcatga gaatagacag gaatatctac atcttgtcca tttttgatgc 360

ggtccaagaa gttgagaaga gcttccatat catagctttc aggaaatcct ttacgattta 420

aaatcccctg ctcaatcaag gtttgattgg gatagagaaa accatcagtt gtaaccaact 480

caaccgtagc atctgtaaac gta 503

<210> 575
<211> 501
<212> DNA
<213> Streptococcus pneumoniae

<400> 575
aatagcagta gcagggacag gttatgtggg tttatctatt gcaattctat tagcgcaata 60

tcataaggtt atagcggtag atgttattcc tgaaaaagta gagcttatca atcgtcgcca 120

atctcccatt aaggatgatg atattgaaac ttatttagtg gaaaaggaat tagacttagt 180

tgcaacatta gatggtaatg aagcttatcg agatgctgac tttgtcataa ttgctgtccc 240

aactaactat gacagtaaaa aaaattattt tgatacatct gttgtggaag cagttattga 300

gcagattatt gcggttaatt tgaaggcaac aattgtcata aaatccacaa ttcctgtggg 360

atatacagaa agtctccgaa cacgttttgg gcaatttaag attctcttta gtcctgaatt 420

tttacgggag tctaaagcac tttatgataa tctctatcct agtcgaatca tcgttggagc 480

agatttgaga gatacggagc a 501

<210> 576
<211> 200
<212> DNA
<213> Streptococcus pneumoniae

<400> 576
atgaatttaa catttttagg cttatgtatt gcctgtatgg gcgtatctgt cggtgaaggt 60

ttattgatga atggactgtt taaatcagta gcacgccaac cagatatgct ttctgagttt 120

cgtagtttga tgtttttagg tgttaccttt attgaaggaa ctttctttgt aactcttgtc 180

ttctcattta ttatcaaata 200

<210> 577
<211> 300
<212> DNA
<213> Streptococcus pneumoniae

<400> 577
atgagtgaaa taggctttaa atacagtatt ttagcgtcgg gttccagtgg aaattctttt 60

tatctggaaa cctcaaaaaa gaagctttta gtagatgcag gcttgtctgg caagaaaatt 120

accagtctgc tagctgaaat taaccgtaag ccagaagacc tggatgccat cttgattacc 180

catgagcatt cagatcatat ccatggagta ggcgttttgg ctcgcaagta tggtatggat 240

ctttatgcca atgaaaagac ctggcaagct atggaaaata gtaaatatct tggcaaggtg 300

<210> 578
<211> 550
<212> DNA
<213> Streptococcus pneumoniae

<400> 578
ttgcacttta tatcctccat tattttgtct tttatatcag tgattatgga caggatttct 60

ttaaaagggg atatttgatt gaacttgtcc agacattgaa atatatccta ttctttgcac 120

tagcgattag tatttctaat tttttcttag aggatcgatt tagtatttcc agacgaggca 180

tgatttactt cctcacatta catgctctct tagtctatgt gctaaaccta tttatcaagt 240

ggtattggaa gcgggcttat cccaacttta aaggaagtaa gaagattctc ctacttacag 300

caacttctcg tgtcgaaaag gtactggata gattaataga atcaaatgag gttgttgggg 360

agttggtagc cgtcagtgtc ttagataaac cagattttca gcatgattgt ttaaaggtag 420

tagcagaggg ggagatagta aactttgcga ctcatgaggt ggtcgatgaa gtctttatca 480

atcttccaag taaaaaatac aatattggag agcttgtctc tcagtttgaa acgatgggaa 540

ttgatgtaac 550

<210> 579
<211> 345
<212> DNA
<213> Streptococcus pneumoniae

<400> 579
aagtaggggc tttcttgcga aaaacatctt tggatgaact accacaattg tttaatattc 60

ttgttggtaa tatgagtatt gtaggtccta gaccagcggg tataaatgaa ctagatttga 120

ttgcagagag agataagtat ggagcaaatg atatcttgcc agggttaact ggatgggcac 180

aaattaacgg gcgtgatact ttgtctgttg agatgaagac ggagttagat ggctactatg 240

ttaaacatct gtctttgata atggatatta gatgtatagt taagacaata ccttacgtac 300

tgaaacgaaa aggtattgta gagggtagtg gtaagaaaga aagtt 345

<210> 580
<211> 600
<212> DNA
<213> Streptococcus pneumoniae

<400> 580
taacgagatt attacaaaac aaaactacta tcgtatttct ttttctggta aaggaaaatt 60

aagtaagata ttaggttatg taaaattcag aaaagaaatt aaaaagaagc taaaagaaaa 120

tgattatgat atgatattgc cgttacatag tattgtgtct ttcattttag tagattttct 180

tctcttttca tttaaaaata gatatattta tgatattcgt gattacagtt atgaaaaatt 240

tttggtttat cgtttggttc agaaacaatt ggtgaaaaat tctttaatga atatcgtttc 300

ttcagacggc tataaatttt ttttaccaat gggagagtat tttactaccc ataacctacc 360

caatatgata gaattaaacg aggtaaagca gttaaaaaat aatagtacgt ttccaattca 420

actttcctac attggtttaa ttcgttttca agaacaaaat aaaaaaataa tcgatttttt 480

tgcaaatgac agtcgatttc agttgaattt tataggtact aatgcaggag aattaaggga 540

attttgtcaa gaaaaaaata tcagcaatgt taacttggtg gacacattcc agcctaaaga 600

<210> 581
<211> 561
<212> DNA
<213> Streptococcus pneumoniae

<400> 581
gaaagaattg ggtgcaaagg tttatcatgt gcctctatta aggaaaaagc ctctacatca 60

gtttctctct cttgctagaa taataaagaa aggagattat gatatagttc attgccatgg 120

ctataaatct gcaattggtc tgatcttatc taaaataatt ggttgtaaaa ttagaattat 180

tcatagtcat atggcttatg taacagaaaa cagttttcaa aaagtattgc gtaaattagt 240

aacaattttg gtaaaaatct tagcaactca ttggtttgca tgtggggaag attcggctaa 300

gtggttatat ggagagaaag cgtataaaga cggaaaaatt gaaattattt ttaatgcaat 360

tgatttgaaa aagtatcaat ttttgtcaga tgttagagaa aaatgtcgta gagaattaga 420

tgtgtcaaat aagttcgtat taggaaatat agctcgccta tcagatcaaa aaaaccaaag 480

ttatttattt aacgttttaa aagaactcat tttaatcaaa ccaaatgtta ttttactcct 540

agttggtaat ggtgaggatg a 561

<210> 582
<211> 736
<212> DNA
<213> Streptococcus pneumoniae

<400> 582
gcttccatca aatcacttta cactactaat tcagatttgg atttaaattt atggattatt 60

gctgataaag tttcggatag aaataaagaa aagataaata gattatcaaa acaatttgcg 120

cagagagaaa ttaattggat agagaacgtt gagatcccat ttaaattaca tttagatagg 180

ggatcaatta gttcatttag cagattattt ctgggaagtg ttcttccatc ttcaatgagt 240

aaagttcttt atcttgacag tgatattatt gttatggatt ctttacgaag tatttttgat 300

attgatttta agggtaaaat tctctatggg gtgaatgata cttttaataa agaatacaag 360

caggtgttgg gtataccaat tgacaagcca atgtttaatg ctggagttat gcttattaat 420

ttagagttat ggagaaataa taacgtcgaa gaaagatttt tgcaagtaat tcaaaagttt 480

aatggtacta tattacaagg agatttaggg gttttaaatg cagttttata taactcattt     540

ggtgtacttc ctccagaata taattatatg accatatttg aagatttgac ttatgaagaa     600

atgatagttt ttaaaaaacc aattaattat tattcaaaag aggaaattaa aaatgccaga     660

gaacgtatag tcttacgaca tttcacaact agttttttat caaaaagacc ttggcaagaa     720

ggcagtaatg ttgcac                                                     736


<210>  583
<211>  525
<212>  DNA
<213>  Streptococcus pneumoniae

<400>  583
tggaagacct ttatcctgtc ttttaacagt tcctttcgtg aaaacaaata ttactcccaa      60

tcaaatatct tatttatcta taattccttt gattgttgga tttataataa tgatatttac     120

aactgatttc gttgtattat tactggcatg gtttctattt tttttatgga acttactaga     180

tggagtagat gggaacttag ccagatatcg ggagcaatac tcgaaggatg gaagtgtagt     240

agatgcaatg gctggctatg tagccatggt gttgacgtat ttcggtgcag gaatagtagc     300

tgctcattta aacgactcag atatctatat aattttgggt gcattatctg ggatttcatt     360

gatttttcca aggttagtga tgcataagta tatcaataca gtagctcaag atgagtctgt     420

gagtagcatt aaagataaat ctgattttaa tactataaaa atactggctc taaacatgac     480

atcaattaca ggaattccgc aggttttact gctattaact atttt                     525


<210>  584
<211>  596
<212>  DNA
<213>  Streptococcus pneumoniae

<400>  584
ctataatggt gagcgatatt tgtcacaaca gattgatagt attaggtctc aaacattcac      60

taattggacg ctttttatta gggatgatgg atcaaaagat aaaacaatag aagtaataca     120

gaggtattct aagatagatg atagaattag attcgttgaa aatccctcaa agtttcatgg     180

agcttattac aattttttta atctaattga atacgttaaa aacaattatc aatttgatta     240

ttactttttt tgtgatcaag atgatatttg gaaagagcac aagttagaaa tacagctgtt     300

aagattttct aaagatgaca tgccagagat ggtttactct gatatgtcaa cgattgatgc     360

cagtaataat ttgatagata ttagtataaa taaaataatg gggattgaat taccgaacat     420

aaataatttg tattttattc atgcctatat ctgggggtgt actgcaggtt ttaatcatgc     480

attgctagag atggttcctt cagttgatat tgataaagat tatttatata tagaaaaact     540

gtctcatgat aattattttg caaagtttgc actagagtat gggaaggtgt tgttct         596

<210>  585
<211>  530
<212>  DNA
<213>  Streptococcus pneumoniae

<400>  585
cgtatcaagt cggcattttc aaactttggg tatgcaaaat aatttttggc tggcagagaa      60

tgtggaattt ctggaatttg gattacctcg aaatgatgat ttttttaaaa gtgaaaaaat     120

caaaaccaca aatataaaat ttagaacatt atttgatatc gatttagacg aactggtagt     180

tttgtatatg ccgacgttca gagatgatgg atcgttgaat gcctataatt tagattactc     240

gaaactaata catgtttttc aaaataaatt tagaaaaaat gtaaaaatat tagttcgttt     300

tcatccaaat gttgattcta gttttataaa tttacaggat acagactgta taaatgtgtc     360

gacctattca aatcctcagg atctgatgat gagtgcagat gtgatgatta cggattattc     420

atcggcttct attgatttta tgttattaaa tcgtccagta tttctgtatt taccagatta     480

tcaaagttat gtgaatgata gaccattgga tgataacttt gataaattgc                530


<210>  586
<211>  380
<212>  DNA
<213>  Streptococcus pneumoniae

<400>  586
ggatatgcca gcaaaaacgt tagccagcaa agttcaagtg gctgtaccag ctgacactcg      60

tatcgtctca atctctgtca aggataaaca gccagaggaa gccagtcgta tcgctaattc     120

tctacgagaa gttgctgcag aaaagatcgt cgctgtaacg cgagtatctg atgtaacgac     180

acttgaagaa gcgcgaccag ctacgactcc ctcttctcca aatgttcgac gcaattcctt     240

gtttggtttt cttggaggag cagtcgtaac agtaattgct gttcttttga ttgagttgct     300

cgacacccgt gtgaaacgtc ctgaagatat tgaagatgta ctgaaaattc cacttttagg     360

gctcgttcca gattttgaca                                                 380


<210>  587
<211>  290
<212>  DNA
<213>  Streptococcus pneumoniae

<400>  587
atcaacgact tccaccaata tcgcttgggc ttttgcgcgt gcaggttaca aaacgttgct      60

gattgatgga gatattcgca attctgttat gttaggtgtc tttaaagcaa gggataagat     120

tacaggcctg acagaatttt tatcaggaac tacagaccta tcacaagggc tttgtgatac     180

caatatcgaa aatctctttg taattcaggc tggctctgtg tcaccgaatc cgacagctct     240

tcttcaaagt aagaatttca gtacaatgct tgaaaccttg cgtaaatatt                290

<210>  588
<211>  507
<212>  DNA
<213>  Streptococcus pneumoniae

<400>  588
agattacact tttacagcta tctccctcag ctacttaacc agtattattg ttgcctttag      60

gcagggagga cttagtcaat ttatcttgat actaacagat gatagtttca atggttcggt     120

actagaaatg catgaagttg cacctattac agctctcttt attctgtact atttgtacaa     180

atattttata aaagaaaata gtttttcttc agtattttat aatatcttaa tagctctcat     240

tattcttttt ttaagcctta aacgaatcgt tcttttgagt gtattaatta tcataccagt     300

atttttggta atttattggt atgataaaaa agtaagtaaa ctagggaaag aacgaaaaat     360

tttaagttta ttaaatatct tttccttaat atttataaca ggaatattcc tttatgttta     420

tagtgtaaaa tctgatttta tatatacatt tattcaagaa cataatatta attcgatggc     480

tagaacagat ttatggaagg gagttga                                         507


<210>  589
<211>  558
<212>  DNA
<213>  Streptococcus pneumoniae

<400>  589
tctggactct cgataattgg aataatggtt ttcttatatc taattatgtt ccgtctatat      60

ttatatgggt ttgctttcta attatttttc aaattactgg ttttatttta caaaaagtta     120

gtatatatga tttttctgta tggtatctga ttttatctta tttttttatg tttggattaa     180

ttttcaatga gtatatgggg tttcaaacaa ctctgctgtg gagccctagt aacttctata     240

ataatgaaga attatttcat tcatatattt ttataatttg gattttgttt tgttattctg     300

taggctattt atttttttat agtgatggaa aggtacatta tcattcagaa gtacaaaatt     360

atcaggaaaa tgaagagaaa attttgtaca atgcgggtag gattttaaca ggagtgggct     420

ttatttctag ggtaataact gattctaaaa cagtactagc agttagagcg gcgaatagct     480

attcagcata ttcagaggca gctagttcag gaataataga tgatttagga gtacttatgc     540

ttcctggtgt gttctcct                                                   558


<210>  590
<211>  516
<212>  DNA
<213>  Streptococcus pneumoniae

<400>  590
acatttgtta tagtttcctt gttgacaaaa ttgtcgtaca ggcctaaagt ggagggaatt      60

tcgcatgaag aattgaaaga aataaatcct tcaaagataa tctatgtcat tcttctgact     120

ctaaatcttg ttatgttatt tctttatatc cgtgaaattc agaaagtagt attgttttca     180

ggtagaagtt tttctaatat tacagatttg ataagtaact ataggtacct atcttattat 240

tcaaatgaag tagaaaatcg tgtaagtgga atgattaatc aactatctaa aattattcca 300

gcgactacac ttatttcttt atatatattt atgaataatt attttataac taaacaaata 360

aagaaaaatt tcatttattt gattccaata gctatattct ttgtctatgc aatcattagt 420

ggtggtagat tgccccttat aaggttagtt gttggagctc tgttgatatt gtatatatac 480

tctgtgtacg ggagtcctaa atctcaactt accaaa 516

<210> 591
<211> 383
<212> DNA
<213> Streptococcus pneumoniae

<400> 591
ttttaaccca ccaagttgac tttagcttga tgcgagagat tggtaaggtt tttgcggaaa 60

aatttgctgc tactggcatt accaaggtcg taaccattga agcgtcgggt attgccccag 120

ccgtttttac agctgaagcc ttaaacgttc ccatgatttt cgccaaaaaa gctaagaaca 180

tcaccatgaa cgaagacatc ttaactgctc aagtctactc ctttaccaag caggtgacca 240

gcaccgtttc tatcgctgga aaattcctct caccagagga caaggttttg attatcgacg 300

atttccttgc taatggccaa gctgctaaag gcttgattca aatcatcgaa caggccggtg 360

ccacagtcca agctatcggt atc 383

<210> 592
<211> 723
<212> DNA
<213> Streptococcus pneumoniae

<400> 592
gtggatgctc aagaaactgc gggagttcac tataaatatg tggcagattc agagctatca 60

tcagaagaaa agaagcagct tgtctatgat attccgacat acgtggagaa tgatgatgaa 120

acttattatc ttgtttataa gttaaattct caaaatcaac tggcggaatt accaaatact 180

ggaagcaaga atgagaggca agccctagtt gctggtgcta gcttagctgc tctgggaatt 240

ttaatttttg ctgtttccaa gaaaaaggtt aagaataaaa cggtattaca tttagtattg 300

gttgcgggaa taggaaatgg tgtcttagtt tcagtccatg ctttagaaaa tcatcttttg 360

ctaaattaca atacggacta tgaattgacc tctggagaaa aattacctct tcctaaagag 420

atttcaggtt acacttatat tggatatatc aaagagggaa aaacgacttc tgattttgaa 480

gtaagtaatc aagaaaaatc agcagccact cctacaaaac aacaaaaggt ggattataat 540

gttacaccaa attttgtaga ccatccatca acagtacaag ctattcagga acaaacacct 600

gtttcttcaa ctaagccgac agaagttcaa gtagttgaaa aacctttctc tactgaatta 660

atcaatccaa gaaaagaaga gaaacaatct tcagattctc aagaacaatt agccgaacat    720

aag                                                                 723


<210>  593
<211>  465
<212>  DNA
<213>  Streptococcus pneumoniae

<400>  593
attatcactg gcggaaagac ccataattag gttttttctc gcacattgtt gggaacggtt     60

gcatcatgca ggtaggacct gttgataatg gtgcctggga cgttgggggc ggttggaatg    120

ctgagaccaa tgcagcggtt gaactgattg aaagccattc aactaaagaa gagttcatga    180

cggactaccg cctttatatc gaactcttac gcaatctagc agatgaagca ggtttgccga    240

aaacgcttga tacagggagt ttagctggaa ttaaaacgca cgagtattgc acgaataacc    300

aaccaaacaa ccactcagac catgtggatc cataccctta cttggcaaaa tggggcatta    360

gccgtgagca gtttaagcat gatattgaga acggcttgac gattgaaaca ggctggcaga    420

agaatgacac tggctactgg tacgtacatt cagacggctc ttatc                    465


<210>  594
<211>  452
<212>  DNA
<213>  Streptococcus pneumoniae

<400>  594
aatggaatga acggaagtga agctgctgtt catgaagtgc cagaatacac aggcccatta     60

gggacatccg gcgaagagcc agctccaaca gtcgagaagc cagaatacac aggcccacta    120

gggacatccg gcgaagagcc agccccgaca gtcgagaagc cagaatacac aggcccacta    180

gggacagctg gtgaagaagc agctccaaca gtcgagaagc cagaatttac agggggagtt    240

aatggtacag agccagctgt tcatgaaatc gcagagtata agggatctga ttcgcttgta    300

actcttacta caaaagaaga ttatacttac aaagctcctc ttgctcagca ggcacttcct    360

gaaacaggaa acaaggagag tgacctccta gcttcactag gactaacagc tttcttcctt    420

ggtctgttta cgctagggaa aaagagagaa ca                                  452


<210>  595
<211>  526
<212>  DNA
<213>  Streptococcus pneumoniae

<400>  595
ggtcaactgt ccatatctcc tatttttcaa ggaggttcat atcaactgaa caataagagt     60

atagatatca gctctttgtt attagataaa ttgtctggag agagtcagac agtagtaatg    120

aaatttaaag cagataaacc aaactctctt caagctttgt ttggcctatc taatagtaaa    180

gcaggcttta aaaataatta cttttcaatt ttcatgagag attctggtga gataggtgta 240

gaaataagag acgcccaaga gggaataaat tatttatttt ctagaccagc ttcattatgg 300

ggaaagcata aaggacaggc agttgaaaat acactagtat ttgtatctga ttctaaagat 360

aaaacataca caatgtatgt taatggaata gaagtgttct ctgaaacagt tgatacattt 420

ttgccaattt caaatataaa tggtatagat aaggcaacac taggagctgt taatcgtgaa 480

ggtaaggaac attacctcgc aaaaggaagt attggtgaaa tcagtc 526

<210> 596
<211> 506
<212> DNA
<213> Streptococcus pneumoniae

<400> 596
agtcgcacta gccacatttt tcttcggttt gctagggacc agtacagtat ttgcagatga 60

ttctgaagga tggcagtttg tccaagaaaa tggtagaacc tactacaaaa agggggctct 120

aaaagaaacc tactggagag tgatagatgg gaagtactat tattttgatc ctttatccgg 180

agagatggtt gtcggctggc aatatatacc tgctccacac aagggggtta cgatcggtcc 240

ctctccaaga atagagattg ctcttagacc agattggttt tattttggtc aagatggtgt 300

cttacaagaa tttgttggca agcaagtttt agaagcaaaa actgctacga ataccaacaa 360

acatcatggg gaagaatatg atagccaagc agagaaacga gtctattatt ttgaagatca 420

gcgtagttat catactttaa aaactggttg gatttatgaa gagggttatt ggtattattt 480

acagaaggat ggtggctttg attctc 506

<210> 597
<211> 518
<212> DNA
<213> Streptococcus pneumoniae

<400> 597
atttcgagtg ttgcttatgg gcgccaagtc tatctcaagt tggaaaccac gagtaagagt 60

gatgaagtag aggctgcttt tgaagctttg ataaaaggag tcaaggtagc tcctcagaca 120

gagtggaagc agattttgga caatacagaa gtgaaggcgg ttattttagg gggcgaccca 180

agttcgggtg cccgagttgt aacaggcaag gtggatatgg tagaggactt gattcaagaa 240

ggcagtcgct ttacagcaga tcatccaggc ttgccgattt cctatacaac ttctttttta 300

cgtgacaatg tagttgcgac ctttcaaaac agtacagact atgttgagac taaggttaca 360

gcttacagaa acggagattt actgctggat catagtggtg cctatgttgc ccaatattat 420

attacttggg atgaattatc ctatgatcat caaggtaagg aagtcttgac tcctaaggct 480

tgggacagaa atgggcagga tttgacggct cactttac 518

<210> 598
<211> 534
<212> DNA
<213> Streptococcus pneumoniae

<400> 598
gggtaactat gcgacttctg cttcaagttc ttcatgggat ttagtagcaa ataatcatct 60

gaaaatgacc gacactggaa atgtaacacg aactgcagca catgaagatg cgatagcggt 120

cgcttctgct aaaaatcaaa cagttgagtt tgataaagtt aacataggtg gagaaagttt 180

taaatacaga aatatagggg cctttttcga taagagtaaa atcacaacaa atgaagatgg 240

aacaaaagct cctagtaaat taaaatttgt atatataggc aaggggcaag accaagattt 300

gataggtttg gatcttaggg gcaaaattgc agtaatggat agaatttata caaaggattt 360

aaaaaatgct tttaaaaaag ctatggataa gggtgcacgc gccattatgg ttgtaaatac 420

tgtaaattac tacaatagag ataattggac agagcttcca gctatgggat atgaagcgga 480

tgaaggtact aaaagtcaag tgttttcaat ttcaggagat gatggtgtaa agct 534

<210> 599
<211> 604
<212> DNA
<213> Streptococcus pneumoniae

<400> 599
gatcaacaag ctgaagaaga ctatgctcgt agatcagaag aagaatataa tcgcttgact 60

caacagcaac cgccaaaagc tgaaaaacca gctcctgcac caaaaacagg ctggaaacaa 120

gaaaacggta tgtggtactt ctacaatact gatggttcaa tggcgacagg atggctccaa 180

aacaacggtt catggtacta cctcaacagc aatggtgcta tggctacagg ttggctccaa 240

tacaatggtt catggtatta cctcaacgct aacggcgcta tggcaacagg ttgggctaaa 300

gtcaacggtt catggtacta cctcaacgct aatggtgcta tggctacagg ttggctccaa 360

tacaacggtt catggtatta cctcaacgct aacggcgcta tggcaacagg ttgggctaaa 420

gtcaacggtt catggtacta cctcaacgct aatggtgcta tggctacagg ttggctccaa 480

tacaacggtt catggtacta cctcaacgct aacggtgcta tggctacagg ttgggctaaa 540

gtcaacggtt catggtacta cctcaacgct aatggtgcta tggcaacagg ttgggtgaaa 600

gatg 604

<210> 600
<211> 500
<212> DNA
<213> Streptococcus pneumoniae

<400> 600
gtgtcagcac aaattacgat taaccataaa aaagcgcgct atgttcggat tgagctagaa 60

ggctataatg ccctcagtct tgcagaagtt gaagttttct gctttatagc tacgaatgct 120

gaaacggcga cacaagtttc taagccagtt caaccaatca gtcagactcc tgtgaaggat    180

aaaacattga caattcaaca cagtggagct tacattgccc gctactccat aacttgggaa    240

gaagttccag tagataaaga tggaaaccaa gttgttcgta gtcattcttg ggaaggaagc    300

ggtcgcaacc agactgcagg ttttgtcctc aacctcccaa tcaaagaaaa tatgagaaat    360

ctgcgagtta agattgagaa aaagacgggc ctactatgga atagatggca aacaatctat    420

gaaaacagac caattttagc tcaaccccac cgtaaaatta cccattgggg tacgacattg    480

aattccaagg tgagtgacga                                                500

<210>  601
<211>  419
<212>  DNA
<213>  Streptococcus pneumoniae

<400>  601
tgttcggatt gagctagaag gctataatgc cctcagtctt gcagaagttg aagttttctg     60

ctttatagct acgaatgctg aaacggcgac acaagtttct aagccagttc aaccaatcag    120

tcagactcct gtgaaggata aaacattgac aattcaacac agtggagctt acattgcccg    180

ctactccata acttgggaag aagttccagt agataaagat ggaaaccaag ttgttcgtag    240

tcattcttgg gaaggaagcg gtcgcaacca gactgcaggt tttgtcctca acctcccaat    300

caaagaaaat atgagaaatc tgcgagttaa gattgagaaa aagacgggcc tactatggaa    360

tagatggcaa acaatctatg aaaacagacc aattttagct caaccccacc gtaaaatta     419

<210>  602
<211>  401
<212>  DNA
<213>  Streptococcus pneumoniae

<400>  602
atctgtagaa ggtcttggtt tcgcaattcc tgcaaatgat gctatcaata ttattgaaca     60

gttagaaaaa aacggaaaag tgacgcgtcc agctttggga atccagatgg ttaatttatc    120

taatgtgagt acaagcgaca tcagaagact caatattcca agtaatgtta catctggtgt    180

aattgttcgt tcggtacaaa gtaatatgcc tgccaatggt caccttgaaa aatacgatgt    240

aattacaaaa gtagatgaca aagagattgc ttcatcaaca gacttacaaa gtgctcttta    300

caaccattct atcggagaca ccattaagat aacctactat cgtaacggga aagaagaaac    360

tacctctatc aaacttaaca agagttcagg tgatttagaa t                        401

<210>  603
<211>  690
<212>  DNA
<213>  Streptococcus pneumoniae

<400>  603
atttgtgagg atcttgttga gagaggtcat gaagttactg ttttgacagg aattcctaat      60

tatcctgaag gtaaaacata tgcggattat cggaataaca aaaatagacg agagactata     120

gaaggagtta ctgtttttcg ttcctataca attccaaggg gaaaaagtac tttacatagg     180

atattaaatt attttagttt tgctatcagt tcctcgatag gggttctact gggacagtat     240

aaagcaaaag atggatcaga atttgattgt atttttgtaa atcaatcgtc tccagttatg     300

atggcatggg ctgctatggc ttataaaaat aaatataaga aacctatgtt tctgtattgt     360

atggatgttt ggccagatag tttaactgta ggtggagtga aacaagatgg cttgattttc     420

aagttgttta aatttatatc gaaaaaagtt tatcgagcta gtgattatat atttgttact     480

agtccatcat ttaagaatta ttttgtgaac caatttgaca taacagaaca aaagattact     540

tatttgccac aatatgcaga agatcttttt atccctgatg aatctagagt taataaagaa     600

agtgttgacc taacttttgc tggtaatatt ggcaaagcac aaaatttgga aactattttg     660

aaagctgcca gtttgataga gaagaatacc                                      690


<210>  604
<211>  588
<212>  DNA
<213>  Streptococcus pneumoniae

<400>  604
caacttctga ttatgccttt gtggatcctg ttgggagtag tgtctatttt ttctagaatt      60

gacatggaac gatcattcct atttttttta ttaacaatag gttgtttaat tagcactatt     120

gctttgttag atatagttac gggagtatct tatgtcttta atggtttgtc tcagcaactc     180

tatttggctg tgggaattct agttttaggc tactggaatg ctgatgtgat tgttcattat     240

tggaaaatca tcaccatgac ttttttggga gcatgtttgc tgatttcagt ggatatttat     300

tttcactact ttcaaggaca tactttttca aatattgatt atgtttatcg agctaagaat     360

tcagcggcat ctatcttttt atcggcagtt attctcaact tgtctctata taatcgcaag     420

tgggcactgt ggagaaaagt attgttatta gctagtagcg gattgctgat ttacatgtgc     480

atccttatgc ggtcacgagc agttctgtta gcagctgcag tacttccgct agtttatata     540

tggtttcagg agacgtcttt ggggcataag attggacgga cattagga                  588


<210>  605
<211>  739
<212>  DNA
<213>  Streptococcus pneumoniae

<400>  605
agtggaacta tgtgtttaat cttgccaata aactattttc tctaactatt ccacttattg      60

ttactcccta tgtcactcga gtcttttctt cagatcattt tgggatttat acttatacca     120

atacagttgc ttcttacttt gttaccttta cattgatggg gataagtatg tatggaagta 180

agaaaatttc tcttaaaaga catgatgaga tagcagtcaa tgatgaatat gcttccttac 240

tgactgtcca gctgcttaat gtaggtctag ccacgttaac ttactttctc tatgtgacct 300

tttttgtcaa taataatcaa gttatttatt ggatccagat gttgtatgtg atttctgctg 360

ttttgatatg acttggtttt tatcaggatt ggaacgtttt cgtgaaattg ctgttcgaaa 420

tatcattgta aatgtcttat cagccctcat gatttttttc tttgtgcata cggaggctga 480

tttggctatc tataccttaa taaaggtagg gacgattttt atcagtcaga ttgttatttt 540

tttaccagtt gttcggatgc aacggtttta tcttgcagga gctgaacata ttcgacgtac 600

ctatcgaggc ttgcttttgt tgtttatccc tgttttggca gacacccttt ttcaaactat 660

ggataagatc atgctaggta tctatgcatc ctatactgct gtgggtttgt attactcaag 720

taggatggtt gctgacatc 739

<210> 606
<211> 533
<212> DNA
<213> Streptococcus agalactiae

<400> 606
aaagaaacac tcacatacac ctctacgggt gattaattta tttcttttgg tgatttttat 60

tttgttaagt gtagtctcat tatttcttat gtatcgtcac cattttttgg catttagaca 120

cttgaacgtc atttatggag ttgtaattat tttaatcatt ttagcaagtt tatttctttg 180

tattaagaat aaagctagaa tttttacaac tataatttta gtactatcct ctattttcgt 240

tgctactact ttatatggat ttaagtcaac cattgatttg acaaataatc taaataaaac 300

tgcttcatac tctgaaattg agatgagtgt agttgtacca aaagattcta aaataaccaa 360

tatagaagct gtcagcaaat tagccgcacc agttaaaaac gatacttcaa atattactga 420

tttgatagaa catataaaat cagaaaaagg aatctctatt acaccacaaa aaacagattc 480

ttaccaggat gcatacaata gaattaaaaa tggtgatagt caagctatgg ttt 533

<210> 607
<211> 510
<212> DNA
<213> Streptococcus agalactiae

<400> 607
taaacacgaa gcggaaaaaa gatttgaaca tcttcaaatc ttatatggtg gagaattgta 60

ctatacaagt gatatgttag aaaaactgaa gttaaagcaa attccaactt taaacaatac 120

gaaatttgct ttaattgaat tttctatgca aacttcttgg aaagatattc atacagcttt 180

gtcaaatgtt ttaatgcttg gtattacacc agtcgttgcg catatagaga ggtataacgc 240

tttagagaat caaaaagaac gggtgaagga aattattaat atggggtgtt acacacaaat 300

aaatagttcc catattttga aacaaaaact ttttaatgat aagcataaac gctttaagaa 360

aagagcccgt tattttttag aggaaaattt agtgcatttt gtagcgagtg atatgcataa 420

ccttgatgtt agaccgccat ttttagcaga agcttataag attatctgta gagatttcgg 480

taaagaacgt gctaaccaac tttttattga 510

<210> 608
<211> 534
<212> DNA
<213> Streptococcus agalactiae

<400> 608
taatcacatt tgttgccatt gcatttgcaa cagctggact tttctatagt ctttttattg 60

ttacgccaca atatacttct tcaacaagga tatatgtcat taaccctaat acccctaata 120

atagtattac tgcacaagat ctacaagcgg ggagttttct tgcaaatgac tataaggaga 180

ttattacgtc tactgacgtt ctagaaaaag ttatttcttc tgaaaaattg aattatcctt 240

cgtctcagtt gctacaaaaa ataacagttt ctattttaaa agatacacgt gttatttcaa 300

tatcggtcga agatgctaat ccaaaaatgt ctcaaaaatt agcaaattca gttagagaag 360

cagcagtttc aaaaatcaag gcagttactc aagtagaaga tatcactact cttgagaagg 420

gaaatttacc taaagcacca tcttctccta atattaaaaa gaatgtacta atcgggttta 480

ttgttggtgc aggattatcg actattgttt tagttattat gggtattttg gatg 534

<210> 609
<211> 585
<212> DNA
<213> Streptococcus agalactiae

<400> 609
cgtacaaaca tacagtttag tggaaaggaa aataaaattc ttgcaattac ctctgttagg 60

gaaggggaag gaaaatccac tacttcaaca agtttagctt tatctttagc tcaagcagga 120

tttaaaacat tattaattga tgcggatact aggaactctg ttatgtctgg aacctttaaa 180

gcaactggaa ctattaaagg cttgacgaat tatttatcag gtaatgcaga tcttggagat 240

attatctgtg aaaccaatgt tcctagactg atggtcgttc cttcagggaa agtaccacca 300

aatccaacag cattacttca gaacgcttat tttaataaga tgattgaagc tattaaaaat 360

atatttgatt atattatcat cgatactcca cctattggtt tagttgttga tgccgcaata 420

atcgctagtg cttgtgatgg ctttgtttta gtaacccaag caggtagaat aaaacgtaat 480

tatgttgaaa aagcaaaaga acagatggaa caaagtggtt caaagttctt aggtattatt 540

cttaataaag ttaatgaatc tgttgctact tacggcgatt atgga 585

<210> 610

<211>  527
<212>  DNA
<213>  Streptococcus agalactiae

<400>  610
tttggagtcg tggctatctt gaagagttta aaatggtatt gaaatacagc ttttactata      60

ttttcatatc aagttcatta ttttttattt ttaaaaactc ttttacaacg acacgacttt     120

ccttttttac ttttattgct atgaattcga ttttattata tctattgaat tcatttttaa     180

aatattatcg aaaatattct tacgctaagt tttcacgaga taccaaagtt gttttgataa     240

cgaataagga ttctttatca aaaatgacct ttaggaacaa atacgaccat aattatatcg     300

ctgtctgtat cttggactcc tctgaaaagg attgttatga tttgaaacat aactcgttaa     360

ggataataaa caaagatgct cttacttcag agttaacctg cttaactgtt gatcaagctt     420

ttattaacat acccattgaa ttatttggta aataccaaat acaagatatt attattgaca     480

ttgaagcaat gggagtgatt gtcaatgtta atgtagaggc acttagc                  527


<210>  611
<211>  360
<212>  DNA
<213>  Streptococcus agalactiae

<400>  611
tgatcaagaa gtgttcattc aaacgggtta ctcagacttt gaacctcaga attgtcagtg      60

gtcaaaattt ctctcatatg atgatatgaa ctcttacatg aaagaagctg agattgttat     120

cacacatggc ggcccagcga cgtttatgtc agttatttct ttagggaaat taccagttgt     180

tgttcccagg agaaagcagt ttggtgaaca tatcaatgat catcaaatac aatttttaaa     240

ttcgattgcc cacctgtatc ccttggcttg gattgaagat gtagatggac ttgcggaagc     300

gttgaaaagg aatatagcta cagaaaaata tcagggaaat aatgatatgt tttgtcataa     360


<210>  612
<211>  384
<212>  DNA
<213>  Streptococcus agalactiae

<400>  612
tgaggtttga tgagaactta aaaattggtg aggatttact ttttaattgc aaactcttat      60

gtcaagagca ccgtatagtc gtagatacga cttcttcctt atatacttat cgaattgtaa     120

aaacttctgt aatgaatcag aaattcaacg aaaactcatt agattttata acaattttta     180

atgaaataag tagtttggtt cctgccagat tagctaatta tgttgaagcg aaatttttaa     240

gagaaaagat aaagtgtctc cgaaaaatgt ttgaattagg tagtaatatt gacaataaaa     300

tcaaagtaca acgagagatt tttttcaaag acattaaatc atacccgttc tataaagcgg     360

tcaaatactt atcattaaag ggat                                            384

<210> 613
<211> 514
<212> DNA
<213> Streptococcus agalactiae

<400> 613
gggttgtcag aagctagaaa ctatggaatt tatcattcaa agggaaaata cttaactttt 60

gttgattcag atgataaagt ttcctctgat tacatagcga atttgtataa tgctattcaa 120

aaacatgatt cgtctatagc tatcggtggc tatttagaat tttatgaaag acataatagc 180

ataagaaatt atgaatattt agacaaagtg atatcagttg aagaagcact actaaacatg 240

tatgacatta aaacttatgg ttcaattttt attactgcat ggggaaaatt attccataaa 300

tctatattca atgatttaga atttgcatta aataagtatc atgaggatga gttctttaac 360

tataaagcat acttaaaagc taattctata acatacatag acaagcctct ctatcattat 420

cgtatacgag taggtagtat catgaataat agtgataatg ttataattgc tagaaagaaa 480

cttgatgttt tatcagcatt agacgagcga ataa 514

<210> 614
<211> 524
<212> DNA
<213> Streptococcus agalactiae

<400> 614
tattcttggg tggttctgtc gtaaaacgag taattaaaac ttacttattt aaaaagtatg 60

ttccgtatgg tttttcaaaa tattgtctct caattgaggt taattcttta gtggggttac 120

ctcatgacat aaggagtaag aaatataaag aactaccgag aaaaaaatta tttgatagtc 180

ttaacaaaga acaaaaatca ctgattttca aaatatttaa aacaaaacca ttaactataa 240

ctccaaagtc agtattattg ttgacacagc cacttgcaca agataaatgt tataaaacac 300

ctacagagag gtttcaaagt attcaagagc aatacgatta ttttgacgat attgtccagg 360

aatatagaac gttagggtac aatgtttatt taaaagttca tcctagagat gtagtagatt 420

attccaaatt gccggtagag ctattaccat caaatgttcc tatggaaatt atagagttga 480

tgtcaacagg tcggttcgaa tgtgggataa cacattcgtc cact 524

<210> 615
<211> 613
<212> DNA
<213> Streptococcus agalactiae

<400> 615
tacactcggg aatttattgg tgcaaggatt agcctttatc acacttccca tctatactcg 60

ggttatctca acagaagttt atggccaata tagtttatat gtcgcttgga tgaatattat 120

tatgcttttt attggccttc agacgagcgg ttctttaagt tcagcccggg tcaaatacgg 180

agaagaattt aaaagctatt cagggagtgc cttttctgta ggtaatatat ggtttcttat 240

tatacttttg atagcttttc tatttagaag ttttcttgca ccattagttg gtttttctga 300

atctattttt ttattaatgg tgtgtcaaag ttacgctagc tatgtggtga ctttctttgg 360

tcagtatttt atacaacaac agaggagttt ggctaattta atattatcct tagccaatgc 420

agtttcatct gttgcactat ctctattttt aatttttcat tggtccgatg actttttatc 480

tagggttttt ggagcttttg ttcctactat aataactgga atagttgcct ttgcttatat 540

ttattatcat agcaaatctt tttacaatcc taagtatttt cggttcattg tcactgtgtc 600

tgttcccttg att 613

<210> 616
<211> 451
<212> DNA
<213> Streptococcus agalactiae

<400> 616
tcaggactgt ttatttttat gattaaacga aacttcctct aataaatagt caataaattt 60

ttgtcccatt tttgaaaggt ttgctttgtc gtgtcggata tagacaatat caataacatc 120

atccacgtca agagggattg cgacaatttc gtcaccgttc aattttgagt tgaggatacc 180

agttgccact gtataaccat ctaatccaat cattaaatta aacagagttg ctctatcact 240

gacaacaatg gattttgggt gaggaatttg agacatcatt tcctcagaaa agtagaaaga 300

attatgcagg ccttgatcat aactcagata aggataatct tctaaatctt tcatacttaa 360

tttttttcta ttagctaagg gattggattt gctcacgaaa atatgaggtg tagtagtaaa 420

aagagttgtg gcaattaagg aattatcgtc g 451

<210> 617
<211> 361
<212> DNA
<213> Streptococcus agalactiae

<400> 617
aaagttcgta ttgggtcatg tctgtgccat taaagagagc tacaaaagcg ttaaccacaa 60

atgcatagtg ttgtgaagaa acgctgaaca gctcacgact tgtattatca cccttataac 120

gctcttcaag aagtgctgtt tgttctagaa tttgtcgcgc atatgacaga aattccatac 180

catcttttgt taacgttatt ccttttggat ttcgaataaa aatttggata cccatctcag 240

tctcaaggtt cctaacagcg tttgaaagtg aaggttgggt aatgtagagt tgtttagcgg 300

cttcattcat gctaccagtt tctacaatct taataacata ttgtaattgt tgaattctca 360

t 361

<210> 618
<211> 383

<212>  DNA
<213>  Streptococcus agalactiae

<400>  618
cgtttcctta gggactgttt tatctgcggc gatgatttta cctattttag cctttattca      60

tactagttca gcttttacga ctcttagcac aattgtaggg acttttattg gttttatttc     120

aggagtatat ctctcaattg gctcggtagg aaaagcatta caacaggtta tgacatggtt     180

tccattgact caaatcaatt ctcttttgaa acaggtctta atgaagggtt ctattgcgaa     240

ggtatttgac aaagccaacg aagccactgt ctctaactat aaagaatcat atggtgttgt     300

tttgcgtaat gctgatggag aaaggctgag taatcacttt atgttgattt atatcattgc     360

cctcattctt attttattgg caa                                             383


<210>  619
<211>  535
<212>  DNA
<213>  Streptococcus agalactiae

<400>  619
ttacctatgg ttgtttagat tggaatagta gctttagtgg ttttctttta attgattcga      60

aaagtcgtag tggacaggca atcacttatc tatataaaac tatcttaaag aagataacaa     120

aactgaagtt ttctaatatc tataccgaaa tcagtaacta caataaaccc tccttagctt     180

tatcaaaatt gaatggcttc aaagagtatg ataagacata tgaagacata ctgcattgtc     240

gatcattgcg tagtcacctc cctaagatat tgaatacgtt tcgtatttcg aattattatg     300

gtaaaacata cgacatatca acttttcaaa ttatggagga aattgaaaat cccttggaag     360

aggagacaga aattaggacc aaagtctctg atgaagaaat attatttaaa gcagaagata     420

gtgcttcact tccttattat ttaaaaatga gcttgtttca aatggaaatt gctaagttag     480

ataaccgtta tgttttacaa gttgactttt tatcggaaca agtaaagagg gttcg          535


<210>  620
<211>  519
<212>  DNA
<213>  Streptococcus agalactiae

<400>  620
ctggtgaaga atgtgaagaa gccttagacc ttgtgattcc taagaatatt gtatttgcag      60

atacagatac ttgtggctac acttttttac tcaatgaaga tggaacagtt tatgatgatg     120

tgactttcta caaatttgat gataaatatt ggttggctag tcataaagct ttggattctt     180

atttagacaa catcaatttt gactataccg taacagatat ttctgacgag tataaaatgc     240

tgcaaattga aggaagatat tcgggagaaa ttgctcagtc attttatgaa tatgatattt     300

caacacttaa ttttcgtact ttgatagaga tgacttataa aggtgagaaa ggttatcttg     360

ctagatttgg tttttctgga gaatttggct atcaattttt cctaccatct tctatttttg     420

ctacttttgt ttcggatgtc tgtgaaggta tagcagagtg tggggatgaa cttgatagat 480

atttaaggtt tgaagtggga caacccatta ctgatattt 519

<210> 621
<211> 573
<212> DNA
<213> Streptococcus agalactiae

<400> 621
cgagcataaa cagcatctct tcgacttaaa agaaggaatt tctaaacatt tatataaaaa 60

tcacgactct attttagaat cttatacagg aagcataact agtgacccag aggttcctga 120

gcaatacaaa gatgagacac gtaattttaa atttgctttt accgcttttg aagaggctct 180

tgcttcttca ggtgttaatt taaaagctta tcataatatt gctgtgtgtt tagggacctc 240

acttggggga aagagtgctg gtcaaaatgc cttgtatcaa tttgaagaag gagagcgtca 300

agtagatgct agtttattag aaaaagcatc tgtttaccat attgctgatg aattgatggc 360

ttatcatgat attgtgggag cttcgtatgt tatttcaacc gcctgttctg caagtaataa 420

tgccgtaata ttaggaacac aattacttca agatggcgat tgtgatttag ctatttgtgg 480

tggctgtgat gagttaagtg atatttcttt agcaggcttc acatcactag gagctattaa 540

tacagaaatg gcatgtcagc cctattcttc tgg 573

<210> 622
<211> 610
<212> DNA
<213> Streptococcus agalactiae

<400> 622
tcttatcatc tgctgcttcc atttctaaga atgaatcact ttctataacc tatgaaaaag 60

ttgctagtaa tttcaacgac tttgaagcat tacgctttaa aggggctaga ccacccaaaa 120

ctgtcaaccc agcacaattt aggaaaatgg atgatttttc caaaatggtt gccgtaacaa 180

cagctcaagc actaatagaa agcaatatta atctaaaaaa acaagatact tcaaaagtag 240

gaattgtatt tacaacactt tctggaccag ttgaggttgt tgaaggtatt gaaaagcaaa 300

tcacaacaga aggatatgca catgtttctg cttcacgatt cccgtttaca gtaatgaatg 360

cagcagctgg tatgctttct atcattttta aaataacagg tcctttatct gtcatttcga 420

caaatagtgg agcgcttgat ggtatacaat atgccaagga aatgatgcgt aacgataatc 480

tagactatgt gattcttgtt tctgctaatc agtggacaga catgagtttt atgtggtggc 540

aacaattaaa ctatgatagt caaatgtttg tcggttctga ttattgttca gcacaagtcc 600

tctctcgtca 610

<210> 623

<211>  606
<212>  DNA
<213>  Streptococcus agalactiae

<400>  623
agcgatatga tgctttcttt tctctttcaa cagactttaa ttttttatca gaagaagaaa      60

ttgaacgcat ctgttctatt gaaagagact tgttacgcca agaaaaattt gatgctgtat     120

tgacaggtta ccgtctatct attgttacaa gttgtcgttt agaaagcata ccattaattt     180

ggattatttc aggggcaacg catatcagtg aaattgttga aaactcagaa ggaatattgc     240

ctaatgggaa gataagtaaa gctagtaagc ctcaaacaaa ggattttatc aaaagagtaa     300

tcactactta ttcaacaaat gttaaaacgt ggaataacta tattaaaaaa tatggtggca     360

aaccttttaa taatgcttta gaattattta ctggtgatct aaacttggta acagattact     420

ctttgtttta tgaatttgat aaagattcgt cctataaaac gataggacct attttgatag     480

ataatgtagg tttttcaaaa tgcagccaaa ttaatcaaga caataagact gtactgctca     540

gctttggtac ttcatttaaa cgagattggg tggaatcttt tttaaagaca ttaccaaggc     600

attatc                                                                606


<210>  624
<211>  511
<212>  DNA
<213>  Streptococcus agalactiae

<400>  624
aaaaataggc agtctacatt ggagaataat ttatctggcg atcggttgat gagatatcaa      60

caagcaaata gacaggtaag caaagataaa ctgttaacag ggagttattt tgtgtaccaa     120

tgcttaaaaa agttaggttt taccaataag atttgtcagg aatctttttc tagtgttagt     180

agttatttaa ttggtttgcc aaaggggaaa attagttact ctaattctgg tgactatcat     240

attctaacct atgctcccag tggttcaact ggggttgata ttgagaaata caaagataga     300

tcagaacaaa cctaccaaaa ctacctagga gaatcagtta gtagtgatat ggattctaaa     360

ttattatttt ataaggcatg gttacaaaaa gaaatttcct ataaatgtgg gaaatctata     420

gatattacct atcaaacaat gatagatggc tatatttatg gttatgcttt tgataatact     480

tttaaagtag aagcggttta cttaaaagaa t                                    511


<210>  625
<211>  231
<212>  DNA
<213>  Streptococcus agalactiae

<400>  625
agtaatggcc aattgttgcg gaataaccta attcttcaat taactcacga gttaaagctt      60

ctaggtgatt ttcattttct tcaatttccc cacctggaag aaaccaagca ccattaggtg     120

cctgtactaa aataatttta tcatgagttg gatttggaat aatagcatag acaccaaatc 180

gtgacctata gtttacatta tctatttttt caccgaaagt aggattagtc a 231

<210> 626
<211> 240
<212> DNA
<213> Streptococcus agalactiae

<400> 626
aatccctttg gcttgagaag gagttgcagt ttggttgcga ttattatttc gtccattacc 60

gtttcctcca cctcggtttc ctcctgattg tgtaggagga gtgtttggta aatcttggcc 120

attaaaggct ctgaaattat ttaaatctcc aaagaaatca tcaaagaaag gattgatgcc 180

accaggcgca tgtgaaacat gatttaaacc tgaaaaaagt ggattattag ggtcagtttt 240

<210> 627
<211> 400
<212> DNA
<213> Streptococcus agalactiae

<400> 627
ctattataaa gaacgagggc aaacactttt agacgttttg caaaccattt acgataaatt 60

tggctattac aacgagcgcc aattttctct tgagttagag ggtgctgagg ggcaagaacg 120

tattagtcgt attatggagg attttagaca ggacccaata ttacaagtag gtgagatgac 180

attggagaat tctattgatt tcaaggatgg ttataaggat tttccaaagc aaaattgttt 240

aaaatattat tttaatgagg gttcatggta tgctttaagg ccgtcaggga cggaacctaa 300

gataaaatgt tacctttata cgattggttg tacagaagca gatagtttat cgaaacttaa 360

tgcaattgag tcggcttgtc gtgctaaaat gaatagtact 400

<210> 628
<211> 628
<212> DNA
<213> Streptococcus agalactiae

<400> 628
ccctaaatca agccataaag gtgattatgg tagtgttctt ctgataggag gtttttatcc 60

ctatggaggt gctattataa tggcagcttt ggcctgtgtc aaaactggtg caggattagt 120

tactgtagca acccaaagtt gcaatatccc ctctttgcat agtcaactac cagaggtaat 180

ggcgtttgat agtgatgatt acaaatggtt ggaaaaatca attgttcaaa gtgatgttat 240

tgtaattggt cctggattag gagtatcaga atcatctcga aaaattttga accagaccat 300

ggagaagatt caatcacatc aaagtgtcat ccttgacgga tcagccttga ctctgttatc 360

agaaggtgcg tttccgcaaa caaaggctaa aaatttagtg ttgacacctc atcaaaaaga 420

atgggagcga ttgtcaggta tcgctgtatc gcaacagaca aaagaaaata cccaaaccgc 480

tcttaaatct tttcccaaag ggacgatttt agtcgctaag agttcgcata cgcgtatttt 540

tcaagattta gacgaaaaag aaattatagt aggaggtcct taccaggcga ctggagggat 600

gggggatact ttgtgtggta tgattgca 628

<210> 629
<211> 388
<212> DNA
<213> Streptococcus agalactiae

<400> 629
agttttatct attgacgatt taagcttgat tcatattaat aaaacgggac gcctgttagc 60

ttatcccttt gttgcagcag gtattttagc tgagaagtcg gaagaagtaa aaggaaaact 120

gcatcaagct ggccttttaa tcggtcatgc ttttcaagta cgtgatgata ttttagatgt 180

gactgctagt tttgaagaat tggggaagac accaaataaa gacattgtag cagaaaagac 240

aacttatcca aatttattgg gtttggataa gtcacaggaa atacttgatg atactttgaa 300

aaaagctcag gcaatttttc aaaatctaga gaaaaaagct aactttaatg ctagaaaaat 360

aatagatata atagagggat tacggttg 388

<210> 630
<211> 410
<212> DNA
<213> Streptococcus agalactiae

<400> 630
tgttcttaac catgctgttg aaacacaaca tgagttgtta cgcagattgg aagcttatgg 60

ggtaactcta actcaagcaa ctatttcacg tgatatgaat gaaattggca ttataaaagt 120

gccatcagca aaaggtcgct atatttacgg tttgtcaaat gaaaacgacc ctatctttac 180

aactgctgtg gcaaagccta ttaaaacaag tattttatca atatcagata agctactagg 240

tttagagcaa tttatcaata ttaatgtcat accaggtaac agtcaattaa ttaaaacctt 300

cataatgtca cattgtcaag aacatatttt tagtttgaca gctgacgata atagtctcct 360

tttgattgca aaatcagaag cagatgctga tcacattcgt caatcaatga 410

<210> 631
<211> 240
<212> DNA
<213> Streptococcus agalactiae

<400> 631
taatgaatga aggtgtggaa catatcattg caattcattt aacgcataca ctatcaggaa 60

ctattgaagc atcacgccag ggagctaata ttgctggtgc agatgttaca gttattgatt 120

ctacttttac agaccagtgt caaaaattcc aggttgtaga agctgcgaaa ttagctaaag 180

agggagctga tttagatacc atcttggctc gtgtggaaga agtacgccag aagtcagaat 240

<210> 632
<211> 240
<212> DNA
<213> Streptococcus agalactiae

<400> 632
aatgggcatt tctcttattg ttggcaatta acctttcctt cacagcagtg attgcaagtc 60

gcttaattca agtacgtgag cctaatacag gaaaaatttc gactggggta caagataaag 120

taaaagtagg tacttttacg accaataagt cgcaactgaa taagacaatt gcactttatt 180

taaaacaata tcaaactaag aagatgaatt ataagattta tgctgcttca tcttctatac 240


<210> 633
<211> 200
<212> DNA
<213> Streptococcus agalactiae

<400> 633
tcgaaacctt tctgaggtaa caaaggaggc tgatatcctt attgttgcga ttggtcaggg 60

gcactttgtt acaaaagact tcgttaaaga aggtgctgtg gtgattgatg ttggtatgaa 120

tcgcgatgaa aatggtaaat tgattggaga cgttgtattt gaacaagtgg cagaagttgc 180

tagtatgata acacctgttc 200


<210> 634
<211> 545
<212> DNA
<213> Streptococcus agalactiae

<400> 634
tgaatctgga atgtttgata agaaagatat ttttgttagt acagattcag aattgtacag 60

agagatttgt ttagaacgcg gtatttcagt ggtgatgaga aaaccggaac tttcaactga 120

tcaggcaact tcgtatgata tgttaaaaga ttttttatct gactatgaag ataatcagga 180

gtttgtgtta cttcaagtaa cttctccact aagaaaatca tggcatataa aggaagcaat 240

ggagtattat tcttcacatg atgttgacaa tgttgtaagt ttttctgaag ttgagaaaca 300

ccctagtctg tttacgacat tgtctgataa aggctatgct atagatatgg tgggagcaga 360

taaaggttat cgtcgccaag atttacaacc tttatactat ccgaacggcg ctatttttat 420

ttctaataaa gaaacttact taagggaaaa aagctttttc acctctagga catatgctta 480

tcaaatggca aaggaatttt cattagatgt tgatacgaga gatgatttta tccacgtcat 540

cggtc 545


<210> 635
<211> 557
<212> DNA
<213> Streptococcus agalactiae

<400>  635
ttattctttc gacgggtatg gcggtaatgg aagagatcca tcaagcggtg aatattttac      60

gtcagaatgg tacaaccgac atttctattt tacattgtac aacagagtac ccaacacctt     120

acccctctct aaatttaaac gttattcata ctttgaaaga tgaatttaaa gatttaacga     180

taggttattc ggatcattca attggatcag aagtacctat cgcagcagca gcaataggtg     240

cagaagttat tgaaaaacac tttactttag atactaatat ggaaggtccg gatcataaag     300

ccagtgcaac acctgatatt ttagctgctt tagttaaagg ggttcgcatt gttgaacaag     360

ccttaggtag atttgaaaaa atcccagatc cagtagaaga aaaaaataag attgttgctc     420

gtaaatcagt agttgcttta aaaccaatta aaaaaggcga tatttattca atagaaaata     480

ttacggtgaa gcgcccaggt aatggtattt ctcctatgaa ctggtatgat atcttgggac     540

aagaagcgca agatgat                                                    557


<210>  636
<211>  532
<212>  DNA
<213>  Streptococcus agalactiae

<400>  636
gctgaatacg gaataatgaa gccattgatt caaagattat caaaagataa agaagtcaac      60

ttacaaatta ttgcaacagc aatgcatctg gaagaaaagt acggctatac ttatcgtcaa     120

attgaagaag acggttttga tattgcttat aaagttccct tacatcttta tgatactgac     180

agaagaactg tatctactgc aatggcgcat ttacaactag gattgaccaa aatttttgac     240

aaggaagact atgatctagt catcatttta ggggatcgtt atgaaatgtt accagttgtg     300

aatgtagcgt tgatttataa tgtcccagta tgccaccttc atggagggga gacatcatta     360

ggcaattttg atgagtatat tcgccatgca attactaaga tgagtcacct acacttagtc     420

tctacagagg attttcgtca acgtgtgatt cagatgggag aacaacctca atttgtaatt     480

aacacaggag ctctcggagt ggaaaatgct ctatcaattc ctcctctaac ca             532


<210>  637
<211>  507
<212>  DNA
<213>  Streptococcus agalactiae

<400>  637
agtcatgctg atgcgattgc tccggttatt gatcctcttg tgtatgattt cgtaggtttt      60

tttgatgata aagatattac ggagcatgat ggttatcctg ttcttggaaa actttatgat     120

gtgctacctt accttgaaga tggctcaata gatgcagtat ttattacaat aggtgataat     180

gctaaaagga aagaactatt tgaatatgta gcaaaggatt attatgactt tattattaac     240

atcattagtc ccaatgcttt agtattgaca ccagatagta tttgtggacg tggtatcttt     300

attggttttg gggcttttat aggttctaaa gtgaagctgt ttgataacaa tgttgttaat 360

acaggagcgc tcattgaaca tcatactgtt gtagaatcac actgtaatat agcacctaac 420

gctaccataa atggtctttg ttatattaga gaagaagttt atgtaggtag tgccagtgtt 480

attattcaaa ccttggatat ttcatcg 507

<210> 638
<211> 510
<212> DNA
<213> Streptococcus agalactiae

<400> 638
gcatgaccaa gaggaactaa tgaaacctaa catgcacatt ctgatgttag atgaatttgg 60

taatacagaa tttaatgtca taaaagaacg ttatcaaagt ctttttgatg cttatcgtca 120

gcttcgtaaa cgcgtattgg ataagcaaaa aaatgaacaa gagaataaat cacgtattga 180

aatgctagaa tttcaaatag cagaaattga gtctgtagcc cttaaatcag atgaagacca 240

aacgctactc aagcaacgtg ataaattaat gaatcataag aatattgcag atactttgac 300

aaatgcatat cttatgttag ataacgaaga gttttcaagt ttatcgaatg ttcgttctgc 360

aatgaatgac cttatggctt tagaagaatt tgatcgagaa tataaagatc tttccaccaa 420

tctttcagaa gcttactacg ttattgaaga agttactaaa cgtttaggtg acgttatcga 480

tgatttagat tttgacgctg gtttactaca 510

<210> 639
<211> 627
<212> DNA
<213> Streptococcus agalactiae

<400> 639
aataccttga aatgtgtcgt gattatgctc tcagccaagt tgacaaacaa cgtgatgatt 60

ttaaacgtct gggcgtttct gccgattggg aaaatcctta tattacacta acaccagatt 120

atgaagcaga tcaagtacgt gttttcggtg ctatggcaga taaaggatat atctatcgtg 180

gtgctaaacc agtgtattgg tcatggtcat cagagtctgc ccttgctgag gctgaaatcg 240

aatatcatga tattgattcg acatcactct actatgccaa taaagttaaa gatggtaagg 300

gaattcttga tacagatacc tatatcgtcg tttggacgac aacaccattt actgtaacag 360

cttcacgcgg tttaacagta ggaccagata tggagtatgt tgtagttgta ccagtaggta 420

gtgagcgtaa ataccttctt gcagaggttc ttgtagatag tctcgctgct aagtttggct 480

gggaaaactt tgaaattgtg actcatcaca ctggtaaaga acttaatcac attgttacag 540

aacatccatg ggatacagaa gtagaagagt tggttatcct tggagaccat gttacaacag 600

attctggtac aggtattgtc cacacgg 627

<210>  640
<211>  326
<212>  DNA
<213>  Streptococcus agalactiae

<400>  640
acatatgatg tatctatctg gaactctagt ggctggtgca ttgttatttt caccagctgt      60

attagaagta catgctgatc aagtgacaac tccacaagtg gtaaatcatg taaatagtaa     120

taatcaagcc cagcaaatgg ctcaaaagct tgatcaagat agcattcagt tgagaaatat     180

caaagataat gttcagggaa cagattatga aaaaccggtt aatgaggcta ttactagcgt     240

ggaaaaatta aagacttcat tgcgtgccaa ccctgagaca gtttatgatt tgaattctat     300

tggtagtcgt gtagaagcct taacag                                          326


<210>  641
<211>  210
<212>  DNA
<213>  Streptococcus agalactiae

<400>  641
tatacaaaat caaaacttga taaggaaatc tggaatacac gctttactag agataaaaaa      60

gtacttaacg tcaaagaatt taaagtttac aatactttaa ataaagcaat cacacatgct     120

gttggagttc agttgaatcc aaatgttacg gtacaacaag ttgatcaaga gattgtaaca     180

ttacaagcag cacttcaaac agcattaaaa                                      210


<210>  642
<211>  230
<212>  DNA
<213>  Streptococcus agalactiae

<400>  642
ggagcgcgtt tagtttacgc agtagatgta ggaacaaatc aattagtttg gaagttacgt      60

caggatcatc gtgttcgttc tatggaacaa tataatttta ggtatgccca aaaagaagat     120

ttcaaggagg gactgcctga atttgcatcg atagatgtct catttatctc tcttaatttg     180

attttaccag ctctaaaaga aattttagtg gatggtggac aagtagtggc                230


<210>  643
<211>  522
<212>  DNA
<213>  Streptococcus agalactiae

<400>  643
ctagggaatg gtctgcttgg attgataaag aaaatactgc tgataaatca cctattatcc      60

aacgtaccga acaaggccaa gtaagtctat ccagcgacaa aggctttaga ggtgctgtaa     120

cacaaaaagt gaacattgat cccactaaaa aatatgaggt caagtttgat attgaaacaa     180

gtaacaaggc tggacaagct ttccttcgta ttatggagaa aaaagataac aatacgcgac     240

tttggctttc tgagatgacc agcggtacta ctaacaaaca taccttaaca aagatatata 300

acccaaagtt agatgtctcc gaggtgacac ttgaacttta ttatgaaaaa ggaacaggtt 360

ctgttacttt tgataatata tcaatgaaag caaaaggccc taaagactca gagcatccac 420

aacccgtcac aacacaaatt gaaaaaagcg ttaatacggc tttaaacaaa aattacgttt 480

ttaataaagc tgactaccaa tacactctaa ccaatccgtc tc 522

<210> 644
<211> 586
<212> DNA
<213> Streptococcus agalactiae

<400> 644
tcccacttaa ctatgttgct cttggagatt ctctgaccga aggtgtgggc gatacaacct 60

ctcaaggtgg ttttgttcca ctgctatcag aatcactcca taatcgatac tcttaccaag 120

tgacttctgt taattatggt gtgtctggga atactagtca acaaatttta aaacgtatga 180

cgacagatcc tcaaatcgaa aaagatttag agaaagctga tttattgacg ctaactgttg 240

gtggtaatga tgtcttggct gttattcgta aagagctcag tcatttatca ctaaattcct 300

ttgagaaacc agcagaagca tataaggaac gtttgaaaga aatccttgca aaagcaagac 360

aagataatcc taaattgcct atttatgttt taggcattta taatcctttt tacctaaact 420

ttccacaatt aactaaaatg caaaccgtta ttgataattg gaataaagct acaaaagaag 480

tagttgatgc ttcagaaaat gtttattttg tcccaattaa tgaccgcctt tataagggaa 540

taaatggtaa agagggtatt acagagtcat caaatagtca ggcaag 586

<210> 645
<211> 511
<212> DNA
<213> Streptococcus pyogenes

<400> 645
tagctcatat tgtcgcaaag acaagtgttg ctattgcttt ggctggagca atgggtagca 60

gtttattagc taatagcaca acgtacgctg ttagtggcaa agaaaataaa aaaagcgatg 120

tcaaatatga aacgaccaaa gttatggaag ctaacgcaac ttcctctaaa gaagacaatc 180

atgtcatgca cacattagac ggctcaatga gtactgtctg ggaggaaaat tcacctggtg 240

gtggtgttgg tgaggtactt tcctacaagt ttgcgtcccc gatgcatatt gggagaattt 300

taattgttaa tggagacaca tctagcaagg agaattacta caagaaaaat agaattgcaa 360

aggctgatgt taaatactat aacgggaata aattggtcct ctttcaaaaa attgaattag 420

gcgacaccta cactaaaaaa ccgcatcaca ttgagattga taaaaaatta gatgttgatc 480

gtattgatat tgaggtaaca gaggtccatc a 511

<210> 646
<211> 300
<212> DNA
<213> Streptococcus pyogenes

<400> 646
ttaaatacgc taaagccctc ttgagggctt ttttagatac aattgacatc ttaaaatgga 60

taccagtttc tgtgaaacaa tctttgattg tgaacaaaca aagcataatt tagagtatta 120

aaatctggac cctgctatct atggtaagtc tttttgttat tgttaaagag gttaagcgga 180

atgttaatat gttttagcta aaaaatgtag tgaaaaatga gtacgtagac tattgtaata 240

gtaattccgt aaaattgttg aaaaagaaaa tgggaaatac cttgtcaaat taagcaccct 300

<210> 647
<211> 579
<212> DNA
<213> Streptococcus pyogenes

<400> 647
ccggttatgt taatggaaag agaaaatata ttaggcgaga aggtttcaaa actaagcagg 60

ctgcaaggga aaccttaatt agtttacaag ctgaacttga taaacctaaa tcaagtatga 120

catttggagc attgacagat caatggctaa aggaatatga aaaaaccgtt cagggcagta 180

cctacttaaa aacagaaaga aatattaata aacatatttt gccaaaactt gataaagtga 240

agattggaga catcaatcca ctacttatcc agcggcttac tgaagaatgg tgcaacgatt 300

taaaatatgg aggaaaaatt cttgggcttg ttaggaatat cttaaatcta gctgttagat 360

acggatatat caataacaat ccagctttgc caattacacc tccaaaaata aaaaggaaaa 420

gaaaaatgaa taataatttt tatacacttg atcaacttaa acaattcctt gaactagttg 480

aaaaaactga caacattgaa aaaatagcct tgtttagatt attagcattt actggaatac 540

gaaaagggga gcttctggca ctaacttggg atgatttga 579

<210> 648
<211> 507
<212> DNA
<213> Streptococcus pyogenes

<400> 648
gctatttggc cctgtgtagc aaaaaagtgc ctcatcatgt gcggagtaaa atgaacgtcg 60

caatttttat taattttttt aaataagcag tagatgtaat ggctaccgat tggttttcct 120

acaaaagatg gctttacttt ttcgtcgtct cctaaaaata ggaaatcagc cttatttaag 180

atgcgatcgt tgcacacggc tattttgata gaagtatcaa tagctttttt tagcagttgt 240

gatgtctcaa aatctaaata cacatatcgt tccgaataag ctgtctttag gcctccgcca 300

tcttttctac ctcttgtcct tgattcgtct atctttacga ccgcacaatc tttatcatta 360

aaagttatat tccctaattt tatgccagct acctcgcttc tacgcaatcc aagatatgtt 420

atcctaacca ttgcgtagtc ataatcatca agcatttttc tggcaacttt atcccaagct 480

ttaaattctt gcattgatct acgtttg 507

<210> 649
<211> 501
<212> DNA
<213> Streptococcus pyogenes

<400> 649
gctcacggtc actaataatc tctactgggc gtgcgatatg gctggcaatt tcttttgaaa 60

tatcttctgg atcctcgaaa ttagggtcat cagcagtgag aatgacggtg aggtttggat 120

gctgatgaat caccctacca aaatcagctc gtctgctctc gcctttattt cctggtgcac 180

ctaaaatcaa cattaatttg cctgtctgat gttcttccac aacgctgagt aatttttcta 240

agctgtcacc attatgggca taatcaacaa aaactttagc atgatttgtc atagtgagga 300

cttccatacg gcctgggacg cgagtcttag cgataccttt ttgaatatca gctaggctag 360

cacccaaacg aaggcaggca agtccagctg ccatagcatt ttcttggtta aaatggccaa 420

ttaattgaat gtcataatgg ccagctaatt gtcctttagc ttcaaaggag aaggcttggc 480

tagtggtgat ctggttgtca g 501

<210> 650
<211> 632
<212> DNA
<213> Streptococcus pyogenes

<400> 650
cccagttcaa ttagattacc ctgttgacca agcaaacgca gcaactgttc aggaagccca 60

gtctttcaaa caatctgttg aagcatctct tggtaaagaa aatgtcattg tcaatgttct 120

tgaaacagaa acatcaactc acgaagccca aggcttctat gctgagaccc cagaacaaca 180

agactacgat atcatttcat catggtgggg accagactac caagatccac ggacctacct 240

tgacatcatg agtccagtag gtggtggatc tgttatccca aaacttggaa tccaagcagg 300

tccaaataag gatgttgtgg cagctgcagg ccttgatact taccaaactc ttcttgatga 360

agcagcagca attacagacg acaacgatgc gcgctataaa gcttacgcaa aagcacaagc 420

ctaccttaca gataatgccg tagatattcc agttgtggca ttgggtggca ctccacgagt 480

tactaaagcc gttccattta gcgggggctt ctcttgggca gggtctaaag gtcctctagc 540

atataaagga atgaaacttc aagacaaacc tgtcacagca aaacaatacg aaaaagcaaa 600

agaaaaatgg atgaaagcaa aggctaagtc aa 632

<210> 651
<211> 534
<212> DNA

<213>  Streptococcus pyogenes

<400>  651
tttgatggtg ttgggtatgg ggcacgtaat tctattttaa tctcagttat agcgacccta      60

attaatatca ccattggggt agtgttagga gccatatggg gagtttctaa agcatttgat     120

aaagttatga ttgaaattta taacattatc tcaaatatcc cttctatgct tattatcatt     180

gttttgacct attcattagg tgcaggattt tggaatttga ttctagcttt ctgtatcact     240

ggatggattg gtgtcgccta ctccatccgt gttcaaatct tgcgttaccg tgatttagaa     300

tacaaccttg ctagtcaaac tttgggaaca ccaatgtaca agattgctgt taagaacctc     360

ctgcctcaat tggtttcagt tatcatgact atgttgtcac aaatgctacc agtttatgta     420

tcttctgaga ccttcttatc cttctttggg attggtttac caaccaccac tccaagttta     480

ggacgtttga ttgctaatta ttcaagcaac ttaacaacaa atgcctacct cttt           534


<210>  652
<211>  340
<212>  DNA
<213>  Streptococcus pyogenes

<400>  652
tcgaagagat tttctatgat ccaagacacc cctatacatg gagtttgctg tctagcttac      60

cgcagttggc agatgaatct ggtgaacttt acgctattcc aggaacgcct ccatcacttt     120

attcaccaat tatcggagat gcctttgcac ttcgctcaga atatgctatg gttttagact     180

ttgaaaaagc acctccggcg attaacgtat ctgagactca ttgggccaaa acatggcttt     240

tacacccaga ggctccaaaa gttcaaaaac cagaagtcat tcaaggtttg catcaaaaaa     300

tcttaaggaa aatgtcacaa caggaggaag gaaatgtctg                           340


<210>  653
<211>  542
<212>  DNA
<213>  Streptococcus pyogenes

<400>  653
caccagacaa cctttctttc aagaccttat caattatctc gaccagcatg atcacgttat      60

attacgagag atcaaaaaag cctttcctaa tgtgacaggt attgacaagg ccatcgaaag     120

ctatgttcaa gctggctata ttcgccgtga aaataagcgt tatggcatca atcttccttt     180

ggtgagttct gatcagcagc tggccttaga cactatgctt tttgtggaca cctgttcagc     240

tatgtatgaa aatattttag cggttgtttt tgagactcag ctaacaaacc aaaccaatcg     300

cgtgatgatc aaggaaaaga ccaacatcac gagagacgat ttgaccctgg ctaattattt     360

ttaccgtctc aaaagaggtg agaagccatc agctgagcag atggacttgt atgacctctt     420

gggggatgtg aatcaggaat atgcccttaa atatatgaca acttttttgc ttaaattcac     480

gcgcaaagac tttgtgatgc aaaaacgtcc tgatatattt gtggaagctc tggttacact 540

tg 542

<210> 654
<211> 616
<212> DNA
<213> Streptococcus pyogenes

<400> 654
ttaattgcag tagcagcatc tattggtggt gtaggtattg ccttgttaac tgaaaattat 60

gtcaaaaaag acatgaaagc agctgctcgt ttaatcatta acaacattga aatgttagtg 120

atgtttttgt tacctgctct tactggggca attattttag caagacctct atattctgtt 180

ttttacggag ctagcgagga gcgtgccatt cacctctttg tggcggttct ctttcaaacc 240

ttgctactgg cgctttacac cctcttttca ccgatgcttc aagctctttt tgaaaatcga 300

aaagcgattt actactttgc ctatggtatc ttgattaagt tagttttaca gataccgctt 360

atttatttgc tacatgctta tggtccttta ctagcgacga cgattgcttt agtggtgccg 420

atttatttga tgtatcgacg cctatatcag gttactcatt ttaaccgcaa actgttgcaa 480

aaacgtttat tattaacctt aattgaaacc ttattaatgg gactggtcgt gtttgtggcc 540

aactggctat tgggctatgc ctttaaaccg acaggccgct tgaccagcct tctttacctc 600

ctcattattg gtggct 616

<210> 655
<211> 208
<212> DNA
<213> Streptococcus pyogenes

<400> 655
agcagtaatc tttggtactg ttttgggtca tgttctatgt gtcccaattc atgcaagatt 60

acttttttcc tgtctttttc agacaggttt ttattaacat agattattcg atgatcagga 120

taataaaatc ctgacctatc ccacattgtg tctgggaact caaatagttt tatgttgtac 180

tgattaagta tctcatctat cttcacca 208

<210> 656
<211> 230
<212> DNA
<213> Streptococcus pyogenes

<400> 656
ttatctgatt taggacattt atcaaatgaa gatggagcgg gagccatgat tagaagcctg 60

gggtacaata ccaaaaaaat atacctaggt catctgagta aagaaaataa catcaaagag 120

ttagcgcata tgacgatggt caatcaactg gctatggcag atttagcagt aggtacagac 180

tttacggtcc atgatacctc tccagatact gcttgtccat taactgatat 230

<210> 657
<211> 411
<212> DNA
<213> Streptococcus pyogenes

<400> 657
cacgagaagt tcatttcaat ctttatttaa ttgaactgat gagccttttt tagaaagtta 60

tcatgaaacg attacgtcca tatgtgaaag ggtacctaaa agaaagtatc ttaggtcctc 120

tttttaaatt attagaagct ttatttgaat tattagtccc tttgttaatt gctaacatga 180

ttgatatatc gattagtcaa cacaacagcc agggaatttt gagggttgtt ttaacattat 240

ttggtttagc aaccattggc ttattgcttt ccgttacagc ccagtatttt tcttcgaaag 300

cagctgttgg ttttacaaga caaatgacag atgatttgtt taaaaaaatc atgtttttga 360

gcaaggagga ccaagaccat cttggttatg ctagtctgtt atcacgattg a 411


<210> 658
<211> 660
<212> DNA
<213> Streptococcus pyogenes

<400> 658
aagaaatgga gcaaacaaac aaggagcttt tgaaatcaag aaaaataaaa gtcaagaaga 60

atataattat gaagtttatg ataacagaaa catacttcag gatggggaac ataaacttga 120

aataaaaaga gttgatggga caggtaaaac ttatcaaggt ttttgctttc agttaacgaa 180

aaattttccc actgctcaag gtgtaagtaa aaagctgtat aaaaaattga gtagtagtga 240

tgaagaaaca ctaaagcaat atgcctctaa atatacaagt aataggagag gagatactag 300

tggtaatctt aaaaagcaaa ttgctaaggt tctgacagaa ggttacccaa ctaacaaaag 360

tgattggtta aatggattga ctgaaaacga aaaaatagaa gtaacccagg atgcaatttg 420

gtattttaca gaaacgacag ttccggctga tagaagttat acgaatcgca acgtaaatag 480

tcaaaaaatg aaagaagtgt atcaaaagct aattgataca acagatatag ataaatatga 540

agatgtacaa tttgatttat ttgtgccaca agatacaaac ttacaggcag taattagtgt 600

agagcctgtt atcgaaagcc ttccttggac atcgttgaag ccaatagccc agaaggatat 660


<210> 659
<211> 410
<212> DNA
<213> Streptococcus pyogenes

<400> 659
aacaggttga tcagcatagg ccatgacatt ggtggcttgc atagcccaat gaatggcttt 60

tttgtcagct ggactaagat ttccaaagta agattgatgg ggaaaacgtc cgtaagaaac 120

caattcgtaa acagttatgc cgttagttgc ttcttgaact tgaggtaaaa gagctagttt 180

tttagcaacc tctttagttt ctaatgtggc aatgttttgc ccatttaaat atacaactcc 240

ctgttttggt ggtaataatc ttgtcagtgc ttttaataaa ctagacttcc cacagccatt 300

ggcgccaata atggtcgtaa ttttaccttc aggaatataa aacgataatt tatcgatgat 360

ggtacgctgt tcataggcaa ttgtgaggtc ttcagcacta attgttgtca 410

<210> 660
<211> 718
<212> DNA
<213> Streptococcus pyogenes

<400> 660
tcaacccata ccattgaaac tagcgccaga aactgcacaa agcggcaaaa ggtatcggta 60

atgatgaggt gtaaaatgtt tcataagatg aggaatgact aagccgataa aagaaatgct 120

tccagcaatg gctacagctg ctgatgataa aataagaacc aaaatcataa agactgcact 180

gatcaagttg gttttttgcc caagagcttt tgcctgagac tcacttagac taaggacggt 240

caggtgataa gataatagct gggctaaaca aagactaaga ataataagag gagcaatata 300

gccgatcatt tgccaattga ccccgacaag acctcctgct tgccagccga taacagcatt 360

tgccagatga tagtaattgg taataccttg acctaatgct gatagcagta tggaaaccat 420

agctcctgct aagacaaggc ggagctgatg gtagcctttg ccagattgat aagaaaggcc 480

aaagactaag gttgctgcca ggctagaccc taacaacgaa agcagaataa tgagggaata 540

gtgcaagtga ggtacaaacg cataagccag taccaaggct agtcctgctc cagcattaat 600

gcctaaaagg ccaggctcag cgataggatt acgtgtgatt gcttgcatga tagttccaga 660

aacagctaac gcagagccag ttaaggtagc accaaaaagt ctcggtaatc gtatagcg 718

<210> 661
<211> 574
<212> DNA
<213> Streptococcus pyogenes

<400> 661
gcaacgacga cataatcacc aatatagtct ggcaagactt cctgcgacaa tgacaggtaa 60

ccttgtttaa aaacttcagt cttcactttt tcaggagcat cataatggaa agcttgatgg 120

ataatctctc cgccacgtcc ccagtcttta ccaaaaaggt aaacgtcttt ttcataaagc 180

cccataatag taaaggttgc tttgtcacct gtaacggcct tgacttcttt ttcataagct 240

gctgttttag ttttccaatc ttttaaccac ttcttggctt tgtcttcttt gttaaagatg 300

cggccgaagt cagataacac ttgtaaataa tcacgtttgc ggtattcaat cgagataaca 360

ggcgcaattt ctgccaattg tttaatattt tcttctgtag agccaacaac aatgaggtca 420

ggcttaagcg ttgtaacggc ttctaaatca gttgcagcaa cttgctttgc tttttttaca 480

gtctttgcta agatggggtt tttcttgtca taagaagtaa ccccgactag attcatatct 540

aattttttga ggtaaccagt gtaagttgaa gcca 574

<210> 662
<211> 545
<212> DNA
<213> Streptococcus pyogenes

<400> 662
tctggatatt actgccaacc agatagactc gtcctactgt atctgcaaca agaagtagga 60

tcattccgat taaaatacta atgggtattg ttattttatg attactccct accaagtatg 120

tactaaagtg tccagctatt aagccgataa aactaatatt accaaccaaa agaacactca 180

aagcacctaa gccagctgct aagacaagta tcaaacgacg cttacggttt aaagggagtc 240

ctagcccaat agcagtatta tcagctaatc ccataatatt aaggaaatgg gcttgactat 300

aagtcaatag ccaaaaacac aataacaaag gagcgatgac actcagagta ggccagtcgt 360

ctcctattaa ttggccgcta agccaattga tgaccaaatc gactttgtag cgattaatat 420

gaccaacaag agcaaccatt agacttgaaa gcatggtagt aacagctaca ccagtaagaa 480

ttaaccgtgt aggatcaatt tgtccttgtt ttgtcaaaga tagccaataa acgctaaagg 540

ttgtt 545

<210> 663
<211> 647
<212> DNA
<213> Streptococcus pyogenes

<400> 663
cggtaatgtg cttatagatg gcttatcgca attaattcct attattcaaa aaacaaaatc 60

tcctatagaa gcggataaaa tggctactat ttatcattgg attaaccatt cttttttccc 120

tatcatcgtt cgtggagaaa tgatggatat gactcgaggg cgttctatca gtcgttttaa 180

tgctcaatct catgttgctg gcattgaagc acttcgtgct attttacgta ttgctgacat 240

gtctgaagag cctcaccgtt tggcacttaa aacacgtata aaaacactcg tcacacaagg 300

gaatgttttt tacaatgtct atgataattt gaaaacctat cacgatatca aacttatgaa 360

agagctacta agtgatactt ctgttccagt ccaaaaactt gatagttacg tagctagttt 420

caatagtatg gataaattgg cactatataa taataaacac gattttgctt ttggcctatc 480

catgttttcg aatcgaactc aaaattatga agctatgaat aatgaaaatc ttcatggctg 540

gtttacttct gatggaatgt tttacctata caataacgat ttaggacact acagtgaaaa 600

ctattgggca acggtaaatc cctaccgctt acctggaacc acagaaa 647

<210> 664
<211> 585
<212> DNA

<213>  Streptococcus pyogenes

<400>  664
cttactggtc ccaaaggtcc taaaggagac actggtctcc aaggtaaaac tggaggaact      60

ggtcctcggg gccctgctgg caagcctgga acgacagatt atgatcaact ccaaaataaa     120

ccagatctag gtgcgtttgc acaaaaagaa gaaactaata gtaaaatcac caaattagaa     180

tcaagcaaag cagataaaag cgctgtttac tcaaaagcag agtcaaaaat agagctagac     240

aaaaaattga gcttaacagg cggcatagtg acaggacaac tacagtttaa acctaataaa     300

agtggtatta aaccctcatc ttccgtagga ggagcgatta acattgatat gtctaaatcg     360

gaaggtgctg ctatggtgat gtatacaaat aaagatacta ctgatggacc attgatgatt     420

ttacgttctg acaaagatac gtttgatcag tcagctcaat ttgtggatta cagcggtaag     480

actaatgctg taaatattgt aatgcgccag ccaagcgcac ctaatttttc ctcggcactt     540

aatataacca gtgccaacga aggcggtagt gcgatgcaaa ttaga                     585


<210>  665
<211>  537
<212>  DNA
<213>  Streptococcus pyogenes

<400>  665
aatctactga ctaacaagcc aaatattgat ggattagcga caaaagtcga gaccgctcag      60

aaactacaac aaaaagcaga taaagagacc gtctatacaa aagctgaatc gaagcaagag     120

cttgacaaga aattaaatct caaaggtggc gttatgacag gtcaactaaa atttaagcca     180

gccgccactg ttgcttattc ctcgtcaacg ggtggagcgg tcaatattga cttgtcgtct     240

accagaggtg ctggtgttgt tgtctattct gacaatgata ccagtgatgg gccgttaatg     300

agcttgcgga cgggtaaaga gacctttaat caatcggcgc tttttgtcga ctataagggg     360

acaacaaatg ccgttaatat tgcgatgcgt cacgcaacca cccccaattt ttcatcggcg     420

cttaatatta ctagcggcaa tgaaaatggt agtgcaatgc agctacgagg gtcagaaaaa     480

gcgctaggaa cgctaaaaat tactcatgag aacccaagta ttggagcgga ttatgat        537


<210>  666
<211>  516
<212>  DNA
<213>  Streptococcus pyogenes

<400>  666
tttcaacgta tgggttatgt caattatttt tcaagcaaag aattaaaaga taatgcttct      60

aaagtagata gtagtgtaac gacagaagca actagtgcta acaaagctgt ctatgagaag     120

tatattgatt ctcttggcaa tggctggcag gtaaaacgct tccccactag caaacaggtt     180

tatgcaattc gcaatattcc tatttacgaa cgtgtttgga actttttctc aaatctagtt     240

ggtattgatc acccttggaa gattcaggat aaagataatc caaaattagc taggtatatt 300

cgcctagaaa aagataaatc aggtggctgg tcacttgttg ggtcggggac aaaacataaa 360

tatctcctct atactaacgg aaaatttcct tatcttcacc aaaactttgt taccctaaac 420

ttaggaacat cttatccaac atacagcaat attcctgttc ttcaggttat ttcacaaggg 480

caaggacgaa cagctcttca agatgtgacc ttccca 516

<210> 667
<211> 604
<212> DNA
<213> Streptococcus pyogenes

<400> 667
tctcactagg caaacctatt atcgttttat taaaaataat cttgacattt cttcgaaaaa 60

gttactttat atcttagaca acttgaatgt caatgttgat gagtttctct ttattagtaa 120

taactttaaa caatataaag aatttattga tatggatacg gcaaaacatt attttgaatg 180

ccgaaacata gaaggtttaa atcatatcct tgattcttat aaagatagta agtcaacaaa 240

ggaaaagaac ctttttgcct tggtcaaggt gttattagca actcttactg aggaagactg 300

tctgacagag cggacttatt tgtcaaacta tcttattaat attgagactt ggagtcacta 360

tgagactgtg ctttttaata attgtatgtt tattttagag tcttgcttta ttgagatggt 420

gttttcaaaa gttattgtga acctcgataa atacaatacc ctaaggtatt atgggaatga 480

atcgattcgg atgtttgtca atatgttgat tttgtttatt cagcgacaag agtatgataa 540

agcttctgag attttggcaa aaattgaaga ttatcagcta aatgatgatt gcttatatga 600

acgg 604

<210> 668
<211> 522
<212> DNA
<213> Streptococcus pyogenes

<400> 668
cagacgatca gcaagataaa gaaatgcctg ttctttcaac aaaccgtttt gagccaaaca 60

aggcttacga ctatgcttat gctaatcgtg ggatgaaaga ggatgatttt aaggatgtca 120

aaggcaagat tgcccttatt gaacgtggcg atattgattt caaagataag gttgcaaacg 180

ctaaaaaagc tggtgctgta ggggtcttga tctatgacaa tcaggacaag ggcttcccga 240

ttgaattgcc aaatgttgat cagatgcctg cggcctttat cagtcgaaaa gacggtctct 300

tattaaaaga caatccccaa aaaaccatca ccttcaatgc gacacctaag gtattgccaa 360

cagcaagtgg caccaaacta agccgcttct caagctgggg tctgacagct gacggcaata 420

ttaagccaga tattgcagca cccggccaag atattttgtc atcagtggct aacaacaagt 480

atgccaaact ttctggaact agtatgtctg cgccattagt ag 522

<210>  669
<211>  554
<212>  DNA
<213>  Streptococcus pyogenes

<400>  669
cagaaaccac aacgacaagt gagcaaccaa aaccagaaag tagtgagcta actatcgaaa      60

aagcaggtca gaaaatggat gatatgctta actctaacga tatgattaag cttgctccca     120

aagaaatgcc actagaatct gcagaaaaag aagaaaaaaa gtcagaagac aaaaaaaaga     180

gcgaagaaga tcacactgaa gaaatcaatg acaagattta ttcactaaat tataatgagc     240

ttgaagtact tgctaaaaat ggtgaaacca ttgaaaattt tgttcctaaa gaaggcgtta     300

agaaagctga taaatttatt gtcattgaaa gaaagaaaaa aaatatcaac actacaccag     360

tcgatatttc cattattgac tctgtcactg ataggaccta tccagcagcc cttcagctgg     420

ctaataaagg ttttaccgaa aacaaaccag acgcggtagt caccaagcga aacccacaaa     480

aaatccatat tgatttacca ggtatgggag acaaagcaac ggttgaggtc aatgacccta     540

cctatgccaa tgtt                                                       554


<210>  670
<211>  518
<212>  DNA
<213>  Streptococcus pyogenes

<400>  670
agtgactaag aaacttgatg ttagagatgc tagagatttt tttattaact ccgaaatgga      60

cgaatatgca gccaatgatt ttaaagatgg agataaaata gctgtgttct ccgtcccatt     120

tgattggaac tacttgtcag aaggaaaagt cacagcatat acgtacggcg gaataacacc     180

ctaccaaaaa acttcaatac ctaaaaatat ccctgttaat ttatggatta atggaaagca     240

gatctctgtt ccttacaacg aaatatcaac taacaaaaca acagttacag ctcaagaaat     300

tgatctaaag gttagaaaat ttttaatagc acaacatcaa ttatattctt ctggttctag     360

ctacaaaagt ggtaaattag tttttcatac aaatgataat tcagataaat attctttcga     420

tcttttctat acaggatata gagataaaga aagtattttt aaagtataca aagacaataa     480

atctttcaat atagataaaa ttgggcattt agatatag                             518


<210>  671
<211>  612
<212>  DNA
<213>  Streptococcus pyogenes

<400>  671
ttatctgtag ggctcgtctc cgtcggaacg atgctgatag ccccgacagt tttaggacag      60

gaggttagtg ctagtagtag tacggagagc agtaccacta cagctaatac tggtaccggt     120

acggcaagtg ggatgactgc cactactcct agtgctacga cagatactgg tgaagcagct 180

gggagcggag ctaggagtga agctaatggt gcatcgtccg tagtatctag cgaagaaagt 240

cagagttcag gcactactcc agcctcaccc caagcacaga cagctccagc agcaacgtca 300

acatcatcgg tttcttctag taatgagaaa actcccaaga cagcaactac aactacatca 360

tcgactccag tagcaagtac cagtaataat agcaacaaag taactagtac tgaagctgaa 420

acacagacgg tggacgtgga acggtataca gttgataagg aaaattcaaa gctaaatatt 480

aaagacggta agactccaaa aactaggagt agtgttaata aagacacaaa acttattaga 540

aaccgcgatg acaaacagcg tgatatcgtt gatgttactc ggacagttga aactaacgaa 600

gatggcctat tg 612

<210> 672
<211> 500
<212> DNA
<213> Streptococcus pyogenes

<400> 672
gtactagaca tggccgaaaa agtgggaata agtttaccta gtagtctgaa gtcggcagtg 60

aaagtccttg gcttaactaa tagtgcaata ggttctattt tagggaaagg tttgacagag 120

taccttggtt tgacagaata tagttcagat aacttagatg gaggagggtt tgattatagt 180

aaacgtgtag ggaaggttta ctactaccac agtttatcag ataggaaata tgaaaataca 240

atgccccttg aagaagctat caggacggcc ttagcatcta attttcccaa actcacagat 300

aattggtttt tcgatatctt aaatagtttt gtcaataaag atacagttga gaaagctaaa 360

ttagacgtaa ttatgaaggt acttaatagt attttttaca aaaaagaata tcgctattac 420

aaccataacc tgtcagcaat agccgaagct aaaatggctc aacaagaggg cattaccttc 480

tattccgttg atgttactga 500

<210> 673
<211> 568
<212> DNA
<213> Streptococcus pyogenes

<400> 673
tccaagccaa cttcacagat ctagtttagt taaaaacctt caaaatatat attttcttta 60

tgagggtgac cctgttactc acgagaatgt gaaatctgtt gatcaacttt tatctcacga 120

tttaatatat aatgtttcag ggccaaatta tgataaatta aaaactgaac ttaagaacca 180

agagatggca actttattta aggataaaaa cgttgatatt tatggtgtag aatattacca 240

tctctgttat ttatgtgaaa atgcagaaag gagtgcatgt atctacggag gggtaacaaa 300

tcatgaaggg aatcatttag aaattcctaa aaagatagtc gttaaagtat caatcgatgg 360

tatccaaagc ctatcatttg atattgaaac aaataaaaaa atggtaactg ctcaagaatt 420

agactataaa gttagaaaat atcttacaga taataagcaa ctatatacta atggaccttc 480

taaatatgaa actggatata taaagttcat acctaagaat aaagaaagtt tttggtttga 540

ttttttccct gaaccagaat ttactcaa 568

<210> 674
<211> 597
<212> DNA
<213> Streptococcus pyogenes

<400> 674
agcattaggt ggatttgttc ttgctaaccc agtatttgcc gatcaaaact ttgctcgtaa 60

cgaaaaagaa gcaaaagata gcgctatcac atttatccaa aaatcagcag ctatcaaagc 120

aggtgcacga agcgcagaag atattaagct tgacaaagtt aacttaggtg gagaactttc 180

tggctctaat atgtatgttt acaatatttc tactggagga tttgttatcg tttcaggaga 240

taaacgttct ccagaaattc taggatactc taccagcgga tcatttgacg ctaacggtaa 300

agaaaacatt gcttccttca tggaaagtta tgtcgaacaa atcaaagaaa acaaaaaatt 360

agacactact tatgctggta ccgctgagat taaacaacca gttgttaaat ctctccttga 420

ttcaaaaggc attcattaca atcaaggtaa cccttacaac ctattgacac ctgttattga 480

aaaagtaaaa ccaggtgaac aatcttttgt aggtcaacat gcagctacag gatgtgttgc 540

tactgcaact gctcaaatta tgaaatatca taattaccct aacaaagggt tgaaaga 597

<210> 675
<211> 553
<212> DNA
<213> Streptococcus pyogenes

<400> 675
tcatactgat ttctacttat ttcacctatc atcaaagtga ctctaagaaa gacatttcga 60

atgttaaaag tgatttactt tatgcataca ctataactcc ttatgattat aaagattgca 120

gggtaaattt ttcaacgaca cacacattaa acattgatac tcaaaaatat agagggaaag 180

actattatat tagttccgaa atgtcttatg aggcctctca aaaatttaaa cgagatgatc 240

atgtagatgt ttttggatta ttttatattc ttaattctca caccggtgag tacatctatg 300

gaggaattac gcctgctcaa aataataaag taaatcataa attattggga aatctattta 360

tttcgggaga atctcaacag aacttaaata acaagattat tctagaaaag gatatcgtaa 420

ctttccagga aattgacttt aaaatcagaa aataccttat ggataattat aaaatttatg 480

acgctacttc tccttatgta agcggcagaa tcgaaattgg cacaaaagat gggaaacatg 540

agcaaataga ctt 553

<210>  676
<211>  504
<212>  DNA
<213>  Streptococcus pyogenes

<400>  676
ataatctttc atgggtacgg aagtgtaaaa tcagatagtg aaaatattaa agacgttaag      60

ctacaattaa attacgcata cgaaatcata ccagtagatt atacgaattg taatattgat     120

tacttgacta ctcatgattt ttatattgat atttccagtt ataaaaagaa aaatttttca     180

gttgattctg aggtcgagag ctatattaca acaaagttta cgaaaaatca aaaagtaaat     240

atttttggtc ttccgtacat atttactcgt tatgatgttt attatatata tggtggggtt     300

acaccatcag taaacagtaa ttcggaaaat agtaaaattg taggtaattt actaatagat     360

ggagtccagc aaaaaacact aataaatccc ataaaaatag ataaacctat ttttacgatt     420

caagaatttg acttcaaaat cagacaatat cttatgcaaa catacaaaat ttatgatcct     480

aattctccat acataaaagg gcaa                                            504


<210>  677
<211>  645
<212>  DNA
<213>  Streptococcus pyogenes

<400>  677
ttgctagtcc cagtctttat gatagtgtaa tagattttga taaatgtaat gaaaagaaaa      60

agcaaaatgt aaaagtctca ttaaatagtt attctaaaag agcagcgttt agaactactc     120

catttggtat ctttactgct attaatacag ttgatttgac aaagggaacc acttctaatg     180

tacaaaaggt tagttttatt aaaaaagcag tcccagatta ttcatggata tattctctag     240

taaaatcgta tgaaataagt aaccttgaaa aactttcatt aaaaataaat acagcggcgt     300

ttactcaagg tgacagatat gtactaccat ttacagtgaa tgaatcagag gaagatagaa     360

acataagttt ttcaaaacca attaagttac ttgtagaaaa atgtaaaact cgatatataa     420

aatatgaaga gttaatagat gtatttaaaa ataattatcc agatatagtg tcagacatgc     480

tagagtcata tatacacgac ttagttacaa atgatttttt aatttcagat ttaagaccac     540

caatttgtaa tatcaattcc ttagattatc tactatctaa attagaagaa ggaacactat     600

gtactgactt aactacttta aagaagatga ttgaagatta caacg                     645


<210>  678
<211>  703
<212>  DNA
<213>  Streptococcus pyogenes

<400>  678
gatagtagct gaaagtgaga ataaagtact aattaatatt gaatctaaat atagaaatac      60

cttttttgat aaatctctga gtcatggtgt gccatcatta atcttaatgt atagttcact     120

atataaagtg acaaaagatg agagatatct aacattctcc aacatatacg ttgagaaatt 180

agtagacata atctctagag atggtataga atctccgtct ctctatgctg gcactgcagg 240

tattgcatta gctattaaag aagcgagtat atctggaaag tattatccga aattattatc 300

atccttaaat tgtcttctaa aagaacaaat taaagacaaa ttggtagtaa gtttttctaa 360

tattgagaaa ggaattattg agccatatga ttatgatatg gttaacggat ttagtggaat 420

aactaattac ttactccttg aacaagaatt tttttctgta gagttaaatc aaattggaaa 480

ttatcttttg aaatatattg agacaatttt aaataaggta actaattgtt ctgaagatag 540

agaagctgaa tttgatctag gaattgcaca tggaattact ggtcctatgc taatcctagc 600

aaaattaaaa tcagaaaaaa ttttgagtgt agaagtagga gatatactga ataaagctat 660

aaatttaatg tttttattta gaagggatga caaactatgg cct 703

<210> 679
<211> 593
<212> DNA
<213> Streptococcus pyogenes

<400> 679
ttaactgcgg aaacaacagt aaatcagtgg ggagttattt ggattaatgt tattattgct 60

gtaaattctg gtttgttaaa taaattagaa ttagattcta ataagtattc tgttttttta 120

tcaagggatg ttgatttaaa aaaggtagaa tatgccagag ttctattgat agctcttata 180

aatctaataa ttagtatgat tttaagctta atgcttattg taattagttt tgtcctacct 240

actccaagtc ttattagtat aggtaggata ctattaacta tcttgttaat ttggttgact 300

acactatggc aaatcccgtt tattttatgg ctatcaagaa aaattaatgt gtattttgct 360

atgattatta atattatatc tccactaatt attggtacaa gtttttctct cttaaataaa 420

tggtatttgt tcccttatga ttggtcgttg aagttgcttg agccaatgac aagaatgaga 480

ataaatagta taccttttgg agcggagttt gttccagact actcacagat ttttatatca 540

ttgttcctag gaattgcttt tttcatctta ctgaccaatc tatttgctat ctc 593

<210> 680
<211> 544
<212> DNA
<213> Streptococcus pyogenes

<400> 680
taatcggtcc aaatggtgct ggaaaatcaa ctattatgaa aattttgaca ggcttagtta 60

gtaagacaag tggttctatt atttttgaag gtagagaatg gtcacgtcgg gatctgcgaa 120

aaatcgggag tattattgaa gaaccaccac tttataaaaa tttgagtgct tacgataata 180

tgaaggtagt tacaacaatg cttggtgttt cagaaagcac tatacttcca ttattaaata 240

aagttggtct aggaaatatt gacaagagac cagtaaaaca attttctctt ggaatgaagc 300

aacggttagg tatagctatc tctttaataa attcacctaa actacttata ttagacgaac 360

ctactaatgg cttggaccca attggaattc aagaattaag ggaaattata gagtcattta 420

aatcagaagg aatgacaatt atgatttcaa gccatatact gtcagaagtt gaacatctag 480

ctgattttat tggatttatc tatgaaggaa agattattct ggaaaaagaa tatgacggct 540

ctga 544

<210> 681
<211> 548
<212> DNA
<213> Streptococcus pyogenes

<400> 681
ggaggtttac aaagcaggac tgtattattc tcctactttc aaatagtagg tgtacttctt 60

ccttttgttg caagtattgt gtgcattcaa ctaaaaaatt tagaagaatc atctggaaaa 120

tataaatatt tattgggtta ctcacagtcg aattataaac catttattgt agaattagta 180

tttctatggt tatgctattg tatagtatta attatttcaa ttactatatt tattctttta 240

ttgaaaacta ttggtataaa tgtatctctc agactactta ttttgaatag tttaatttat 300

atcatttttg cctatgtaac ctatctgatc aatcatatta ttagctatat atttagtaca 360

ggtgtggcat taggtatttc aatggtaggt gttattgctg cagcattttg tgaaacgagc 420

cttggtgata aggtatggtt tcttattcca tgggcatggc ttttaagaat atcagatact 480

ctatataacc aacagaaaat ggcaattgtt ccacttattg ttatattttt tgtttcatgc 540

acagtagc 548

<210> 682
<211> 311
<212> DNA
<213> Streptococcus pyogenes

<400> 682
aattagattt gaccattaca gagttgcagg accatattgc tcatctcaat aaggttgcgg 60

aggtcttgct taatctgaac aacaacgata tagagaaccg ccgtttggcc agatatgact 120

atgccaagat gaacttgact gcagctataa aaatagaaga agttgagaaa gagattgaaa 180

cttctcaaaa tgaacttaat atatccatag atgagtatga atatctagta agaaggttgg 240

aaaagtttgg agagatcttg agtgatagca aaattatcga tacttctcga aatgaaatac 300

aatgggagta a 311

<210> 683
<211> 521
<212> DNA
<213> Streptococcus pyogenes

<400>  683
gtcgttgaaa ttgtctttgg agtttgtatc actgttttaa tgattgcgat aatatcgctt      60

tctttttcaa agttaaatat agtgacatct cataatgtgg gagaagaatt ttatattaaa     120

gataaacagt caatcaaaca gttgaacaat tatatgaaga cattgggatt agattatgtt     180

gtttttgata gaaaaacaga taaagctatg gaaggaaaat atttatctaa agaattttct     240

ttatttaacg aagttgcaga agaaaaaaat aatctgactt ttaattctgt acattatgat     300

ttatatacta atatcaatta taatattgtc ataagatata atgagatacc ggagttttct     360

aaccattacc ttagaaatgt ttcatataac atgttgacat tttatattct gggaatagga     420

acgagtatca gcattgtagt tgctttaaca cggtttgtaa aagaaatttc tttgaatttc     480

aaggaaatca agaagttagc aaataaaatg gggatagaag t                         521


<210>  684
<211>  548
<212>  DNA
<213>  Streptococcus pyogenes

<400>  684
cttatgaaga ctgctttgga gattgaaaat tatcatgtta taacctgtca agagatagaa      60

ttaccaatag tttttgatga ttttaaagga tatgatttga tcttattgga tatcatgatg     120

cctaatataa gtggaactga gttttgttat aaaattcggg aagaagttca ttctccaatt     180

atttttgtta gcgctttaga tggcgataat gaaattgtcc aagctttaaa tataggggga     240

gacgatttta ttgtgaaacc atttagctta aaacaattcg tagctaaagt taactctcat     300

ttgaagagag aagagagagc aaagataaag aatgaggctg aggagagagt gaagcgcagt     360

tttccaccta tagaaatcta tctagaggaa cgtatgttat atattgataa acagccgtta     420

ttcctgactt atagagagta cgaaatttta gaattactgt cacgtcatcc gtataaagtt     480

ttcacaaaag aagagatata tgaacaagta tatagcgatg aagcttcagc attgtttcat     540

tctatttc                                                              548


<210>  685
<211>  543
<212>  DNA
<213>  Streptococcus pyogenes

<400>  685
ttctccctac tggtgttctc aattgctatt agcttatttc aagggatttt acccattttt      60

agtatgctac tagttcaaaa aatgttaaac attataacga ctgatataaa aaattttcac     120

acattgatga ttgcttttat ctcatatatt gctttaacat tattgacaat tataatagga     180

gaagttgata gttatattga tactaaatta cagatacttc ttcactataa gatgaaccat     240

ttagttatgc agaaaactgt aaagttaaca ttggctgaat ttgagacacc agaaatctat     300

gatgatatca ctcgcataca aaatcaaata tcttataaac cttttcaaat atataagtca    360

attatttctg tattatcttc gttagtatca ttaatttcct catttgttat tttattaaat    420

tggaagatat caatttttcc acttttactt atccttccta tcgtttctat atatatctac    480

ttgaaaattg gtaaaaatga atttgagata ctatataaaa ggagtagcga tgagagagca    540

aat                                                                  543

<210>  686
<211>  512
<212>  DNA
<213>  Streptococcus pyogenes

<400>  686
agcgatcctt atcttacttg tttttgttgc tttttacttt atccatcttg cggtgcgtga     60

ttaccgaaat gcacgtatta ttcggatgat gagccataaa atccgagact tgattaatgg    120

tcgctatact gatataatcg acgaaaaagc agacattgag ttaatggagc tttcagacca    180

gttaaatgac ctgtcagatg tttttcgctt gacgcatgaa aatcttgccc aagaaaaaaa    240

tcgcttggca agtattttgg cttatatgtc agatggtgta cttgctacag accggtctgg    300

taaaatcatc atgattaacg agacagctcg caagcaatta aatttaagta aagaagaggc    360

actaaagaaa aacattacag atttgttaga aggtgatact tcatatacct accgtgattt    420

ggtatccaaa acaccagtgg taactgttaa tagccgaaat gatatgggtg agtttgtctc    480

attacgcttg cgctttgcgt tgaataggag ag                                  512

<210>  687
<211>  520
<212>  DNA
<213>  Streptococcus viridans

<400>  687
acgtcctctt aacagtcaca aggggacttt tggccgtgtc cttttgattg gcggcaacta     60

cccttacggt ggtgctatca tcatggctgc ccttgcttgt gtcaatagcg gagctggttt    120

ggtgacggtt gcgacccata aggacaatat cacagctctg cacagccatt tacccgaagc    180

tatggctttt gatatggttg aaaaagatcg tttgtcagag caaataacag cagcagatgt    240

ggttcttatg ggaccgggct tggcagaaga tgacttggct caaacaacct ttgatgtggt    300

ttggcaggct atcgaaccaa aacagacttt gattattgat ggctctgcta taaatctatt    360

agccaaaaga aaaccagcta tttggccaac caagcaaata atcctaacac cccatcaaaa    420

agaatgggaa agattgtctg gactgactat tccagaacag atagaagcag caacacaaac    480

agcactagct cattttccaa aagaaaccat cctagtcgca                          520

<210>  688

<211>  463
<212>  DNA
<213>  Streptococcus viridans

<400>  688
atcaggctgt tatgcgtctg attagacaaa aggatgaaca agttaagaaa ctgcaaagat      60

cagttatttt cagacagcct gaaagactct atgatgctta tgttcaaaaa ttggatcatt     120

taagaacaca tttgttgacc aaggtgcggc aggtttatga tgtttatgat agcaaggaac     180

atttgctgag acaaagattg ttgtccttta atttatcagg gtgtattcag cgctatcaag     240

cacaattaaa acaagatcag cgtttattgt taagccacat gagcagtcaa tatgatagta     300

aattagcccg ttttgaaaaa gcacaagatg cgcttttgtc actggatacg actcggattg     360

tggcgcgtgg ctatgctatt gttcaaaaag ataatcacat tattcaatca acccaacaga     420

tcaaaaaagg agatcgcttg catcttgaaa tgaaagatgg gca                       463


<210>  689
<211>  360
<212>  DNA
<213>  Streptococcus viridans

<400>  689
aattgtgaac cagttagaaa ccggtgaaat tcctcttgaa gaagctatta ctcaattcca      60

aaaaggaatg gcgctttcta aagatttgca gaaaaccttg gagtctgctg aaaagacctt     120

ggtcaaagtc atgcaggctg atggcagtga agcagaaatg gacgagttgt gaaagataag     180

attaaatcca ttaatcaggc tattaagcat tattatgcgc aaactcatgt gtctcaggat     240

ttgattgaag cggtcttgta ttctgttgat gcaggcggta aacggattcg tcctctctta     300

ctattggaaa tcttgcaagg ctttggtttg gtacttacag aagctcacta tcaggtggca     360


<210>  690
<211>  463
<212>  DNA
<213>  Streptococcus viridans

<400>  690
gaagaaacgt gaacgattag aattaattaa aaaaattgtt ttagaaaatg aaattgaaac      60

acaaaaagaa ttggtcaaac ttttagagaa cgaaggctta caagcaacgc aagcgacaat     120

ttcccgtgat attaatgagg tcggtatcat taaagtacca gcttcaaata gtcgctatat     180

ttatggcctt tctaaggaaa taagcaaaaa agaagagtca acaccaaaac cagctgaaaa     240

agcagttaag tttatttcag atcaggtggc aggtttagag catctcattc atattgatgt     300

tgttcctgga aatagctatt tattgaaacg ttttttacta gagagatttg aaggacttat     360

ttttagcttg ctggcagatg atgacagttt gcttttgatt gttaaaaatg ctaaagatag     420

tgatagaatt cgtcaagaaa tcaaatcttg gatggccaat taa                       463

<210>  691
<211>  412
<212>  DNA
<213>  Streptococcus viridans

<400>  691
agatatgatt gcaacaattg aaaattttgc tcaagaacag gcagaatttc cggtttataa      60

tattttagga gaaatccata cctatggaga attaaaagct gattctgatt cgcttgcagc     120

tcatcttgat cagttagatt taacagcaaa atcaccagta gttgtctttg gaggacagga     180

atatgccatg ctggctagtt ttgttgctct gacaaaatca gggcatgcct atattcctat     240

tgatcatcat tcagccttag aacgtattga ggctatttta gaggtagcag agccaagttt     300

agttattgct gttgatgatt tcccaattga caatcttcaa gtcccagtaa ttcagtatag     360

tcaattagaa gagattttta aacaaaagct atcttatcaa atcaatcatg cg             412


<210>  692
<211>  560
<212>  DNA
<213>  Streptococcus viridans

<400>  692
gtgaaaagtc gcatcaaatc tttgccttgt ttttctatat catttggcaa attttctgtg      60

tctatagtta taaattttat agaaaatcac gggataataa gtggattttt tatcttcatg     120

tcttcatgtc tatcttacct ttatctttgg taaagattac tcctgcgatt tggacaaatc     180

aacaatcttt atttggtttt ttgggtatat cctatcttac ctttcgttca gtaggtatga     240

ttatggaaat gcgagacggt gttctcacgt catttacatt ttgggaattt atccgtttta     300

tgctgtttat gcccactttt tcaagtgggc ccattgatcg tttcagaaga tttaatgatg     360

attatgagaa gattcctgat aaagatgaat tgctagatat gttggaacaa tctgttcact     420

atatcatgct tggttttttc tataagtttg ttttagcgca aatattggga acaatgattt     480

taccgggttt gaaagaaatg gccttgcaaa aaggtggttg gttcaattgg ccgactttag     540

gagtcatgta tgtttatggc                                                 560


<210>  693
<211>  250
<212>  DNA
<213>  Streptococcus viridans

<400>  693
cagctgttgc agaagattta gccaaaattg caggtgttga cttgcaggaa tatggtttgg      60

ctatgcttaa ggctggtacc aatttagcaa gtaaaacggc tgcacaactt gttgatattg     120

atgctaaaac atttgaactt aatggtagtc aagtacgtgt agctcaagtc aatacggttg     180

atatcaatga agttttggaa cgtcaaaatg aaattgaaga agccattaaa gcatcacaag     240

cagctaatgg                                                          250

<210>  694
<211>  508
<212>  DNA
<213>  Streptococcus viridans

<400>  694
ttctcttaaa tcaaacagga agtatagcgg atcgttatgc agctaaacgt ttattagaca      60

ttaaaccgag ttcgaatttg caaggtatga taaaaaaaat tgcggctggt aaaaccttaa     120

atagctttga tagggcaagc ctgcgcctta ttaagagttt cttgaaaaaa gaagacgctt     180

tatttggaag tctgaccttt agtgataatt atgaacgtcg tgtattgccg catgtcaaaa     240

aattgcccaa gcacttttct tatggaacct taagtcaaat tgctagcaaa aatggtcaaa     300

ggttaacaaa aacaaatcaa tttgaaatta atgatcattt ttataataaa cgtattaaag     360

gacaattgaa aagactcaaa ggcttccaaa agcaactgtc ttatttacag tctccagaat     420

acaatgattt acagctggcg ttaactcaat tagcaaagtc aaagaccttt gtcatatttg     480

ttattccgcc ggttaatgcc aaatgggt                                        508

<210>  695
<211>  300
<212>  DNA
<213>  Streptococcus viridans

<400>  695
aattgttctc ttattggctt tctttggttt tcaacaaggt gttgatgcgc aaagcaaata      60

ccactatagt caagaactaa attactataa tggtaatgcc atggaacttc gtaatggttc     120

taatggtggt atgtttaact gtaattttgt ccctggaaat gtcggcttta ataacggctt     180

gatgagtctt aaaattgaca gtgatggtcg tggcggctac actgggggcg aatggcgtag     240

taaagaacgc tttggctatg gtcttttcca agtaaacatg aaacctatta aaaatccagg     300

<210>  696
<211>  266
<212>  DNA
<213>  Streptococcus viridans

<400>  696
gtcttggcgc gagttttaaa ccaattcaat gttttcttga tggtgtagct cgtgtttttg      60

gtactgacgg tcagttctcc atcttcaatg ctagcggcca caatgtctgg catcttaagg     120

acaatgcttc tatctgctaa agtcagtgtt aacagttcca tacctttctt ttctgtcgta     180

gctgtttcat ctattgtaga taagtgttgt attttcttaa ctttagccaa agctttatcc     240

acacgcccct gctcaaaagg cttcaa                                          266

<210>  697

<211>  400
<212>  DNA
<213>  Streptococcus viridans

<400>  697
cattggttcg atcagtcaga ccaacggcat caaaattagt atgctgctta atgatattac      60

acactttagt agctgattgc tgactcagtc cctgtcttaa ataaggaagg gtttgctgcg     120

tcaaatctaa aacatcccga gtctgaacag ctctcagctg tctttcattg gataagtagg     180

tcttgagaat agctagaaat aaggtcgaac ctagactgtt aagcaacatc ataggaatga     240

aaatcagttt aaccaaatcc cagccagtaa agaaaccgac aaagagcatt tgaatacttt     300

cagcaataat gctgataaca atgacctgcg aagtagatgg ataaaggtta ttagttttta     360

attgatcacc cagtcgacca ctaatatatc ctaccaaagc                           400


<210>  698
<211>  381
<212>  DNA
<213>  Streptococcus viridans

<400>  698
tgattaaagg agttaagttg gtgaattgac ctgaaaaaat tgtttcaagt cccttaaatt      60

tcgttacatt taagttgtca aattccaagg caattttaga ataattcgtt ttaggcaata     120

aggtcttaat tttttccaaa ggattttcaa aatcaagata gccagtcacc gaaatatcta     180

atatactttt tgctcttaaa gcatcaagtt caggtaaaaa aagcagagtt tcttgatctg     240

caaagaggaa gaggaacata aggcgttcat gcggatcact ataaaaacct gttaaataat     300

taatggagac aggatctgaa aggacagcag cttcaatacc ttgctttttg agtttttgaa     360

caatctgtgc taattttgac a                                               381


<210>  699
<211>  505
<212>  DNA
<213>  Streptococcus viridans

<400>  699
agaaaaagtt gactacgaaa aagtaacagg acttgttaat tctacagaat cttttgggtc      60

tgtagacgga cctggtatac gctttgttgt ttttatgcaa gggtgccaaa tgcgttgtca     120

atattgccac aatcctgata cttgggcaat gaagaatgat agagcaacag aaaggactgc     180

aggagatgtc tttaaagaag ctttacgttt taaagatttt tggggagata caggaggtat     240

tactgtttct ggtggtgaag caacgctcca gatggatttt ttaattgccc tcttttcttt     300

agcaaaagaa aagggaattc atacgacctt ggatacctgt gctctgactt ttagaaacac     360

accaaaatat cttgaaaaat atgaaaagtt aatggctgtc actgatttag tattgttaga     420

tattaaagag attaatcctg accaacataa aattgtcact ggtcatagca ataaaactat     480

tttagcttgt gcgcgttatt tatct 505

<210> 700
<211> 407
<212> DNA
<213> Streptococcus viridans

<400> 700
tgatgctgag tacaaggatt tatccaataa tctcagcgaa tcttactata ttttagaaga 60

tgttagcaaa cgtctagaga ctatactgga tcatatggat tttgatgcca atactttggt 120

taaacttgaa gcacgtcttg atgttatcaa caccatcacg cgtaagtatg gtggttcagt 180

tgatgatgtt ttggcttatt ttgacaatat cagtaaggaa tacaatcatt tgacggtaaa 240

tgacctcgct tttgatgata tggaaagaga actaaaagtt ttggagcgct cactattaga 300

agcagcagct caattgagtc aaaaacgcca tgccattgcg gaaaccttgt ctcaggagat 360

taagcaggaa ctaaaagatc tctacatgga taaggctgat tttaaag 407

<210> 701
<211> 250
<212> DNA
<213> Streptococcus viridans

<400> 701
cggcagacaa gtcagtcatt actcagcctg ctacaaccct gacagctatt aaaaagattt 60

tagagagatt agaaattggc ggtcgtttgg caattatggt atattatggt catgagggtg 120

gcgataagga aaaatatgcg gttctgaact ttgttaaaga gctagatcaa cagcatttta 180

cagtcatgct ttatcaaccc ttaaatcaaa taaatacccc accctttttg gtgatgatag 240

agaaattata 250

<210> 702
<211> 213
<212> DNA
<213> Streptococcus viridans

<400> 702
gtgatattat ccaaaccatt ctcaatgaaa gattttcgcg gattcctgtt tacgatgatg 60

ataaagataa gattattgga atcattcata ctaaaaattt attgaatgct ggtttcaagg 120

aaggttttga tcacatcaat cttcgccgta ttttgcaaga gccgcttttt gtaccagaaa 180

ctattgttgt aaatgacctt ttgaccgctt taa 213

<210> 703
<211> 615
<212> DNA
<213> Streptococcus viridans

<400> 703
attggacttg tgtcagcttc gatttcaagc ctattttttg tgtccattgc gagcagtggg 60

atcgtatttg ctcaagaaaa tgcagctgtt cactacaaat atgtgacgga tacagagcta 120

agtagtcaag agaaggactt gattgtaaag ggcattccta aaattacgga agatagtgag 180

agcacctatt atctagtcta ccgtatggat gagaaagctc agctgggtca gttgcccaat 240

acaggtgggc agaatagcct tactagtgtt ttaactggtg gagtcctagc ttcgattggc 300

cttcttattt ttgtcgtatc gaaaaagaaa ggcaaaaaga aagcactgtt gaaagttgtc 360

ttgataacag gaatgggcag tggtttggct tcttcggttc atgctatcga aaatcaactt 420

ttgctccaat acaatcagga ataccaatta tctcaaggag atagtctgcc tttgccacgc 480

gccctgtcag gatataccta cctaggctat attaagcaag acaaagagat taatcagcaa 540

gaaactgctg ctagggatca gaaatttgac tacacggttc agcctcattt tcagaccaat 600

gaaggtagac aaagg 615

<210> 704
<211> 541
<212> DNA
<213> Streptococcus viridans

<400> 704
gaaggcaatg aggagactta ctatcttgtc tacaggttaa actcaaatgc tggatcaaaa 60

accttaccga atacaggcga cagtaacaat tccaatacta tgatgacggc tggtttgtta 120

acgacgatag gattggttgt ttttgttgtg tcgaaaagaa aggttaaaag caagttccta 180

ctgactgttt tggtgggggc tagtgtcggt ggaggtttga tactatccgt caatgcgctg 240

gaaaatggga tcttgctaca gtataatgcg gaatatcaag tgtcggctgg ggaaagtctg 300

ccgtcaccaa gtgaaatttc aggctatacc tatgttggct acattaaaga agaatcgatt 360

cagaaattat tagacaataa gattcttaac aatcagcaaa atgctaatct agataaagaa 420

actttaaacc aaaataagaa gctagattat tctctttctt ttgataagaa tgggctgaaa 480

aatcaaacgc ttggcgtcaa tacaattgag cctcaagatg aagtcttgtc tggccgagta 540

g 541

<210> 705
<211> 563
<212> DNA
<213> Streptococcus viridans

<400> 705
ttttattggg aggttttctt tattatatta ctaatcctat tgtcactttt ttagaaaacc 60

gttttaaaat taagcgtatt tgggggatca ctcttatttt tgctgtattg ctttccttgc 120

tggttttttc tattaccagt ctgattccca atttgattaa tcagctaaca gatcttattt 180

cagccagcca aaatatttat gtgggtttgc aggatttatt caatgaatgg aaaagcaatc 240

ctgcctttaa aaatattgat atccctgttc ttttaaaaca gttcaattta tcttatgttg 300

atattttgac aaatgttttg gatagcgtga cagttagtgt ctcaagtatt gtttatatga 360

ttacaaatac ggtgatgatt ctggttctta cacccgttat tcttttttat ctcctcaagg 420

acaaagatgg tttaatgccc atgttagatc gtactatatt gaaaaatgat aggcataata 480

tcagtcaatt actgaatcaa atgaacaaaa ccatttctcg ttatattagt ggtgtagcta 540

ttgatgctgc cttcatattt gtt 563

<210> 706
<211> 500
<212> DNA
<213> Proteus mirabilis

<400> 706
tgaaaaagtt attattatct gcaattatta cttcagcaat ggccataatt gctacacctg 60

ccctagcaga agatactggt acaccagcac caacagaagt tacagttaat ggtggtacta 120

ttaactttga aggttctgtc gttaatgctg cttgtggtgt tgatagtagt tcaagtaacc 180

aaactgttcg tttgggtcaa ttccgtgtcg ctgaattcac taaaaaaggt gatgaaacag 240

gacgtattcc ttttagcatt aaattaaata actgcgatat tactgtttca tcattagcag 300

caattacctt taacggtaca gcttctgatg gtgatgcaac tgcattcgca ttacaaggca 360

gtggcgcagc aaccaatgta gcgttaaaaa ttaccgattc aagcagcaaa aatgttgttc 420

caggacaacc ttcttcaact caaaaattaa tcgaaggtga aaaccaatta aattataacg 480

cttctcttat ttccactgat 500

<210> 707
<211> 346
<212> DNA
<213> Proteus mirabilis

<400> 707
agatgaaaag cttgctaatg aaaatacact acaaattgcc atccagagtc ggataaagct 60

tttctaccgt cctagtggat tgtccgctta tactgaaaaa tatgccaatg aagtgacttt 120

ttcctataaa aatggggagt taattgccca taatccaaca ccttatcata ttactatggt 180

caatttagct gcagccgaca gtcaacttcc ttcaagtatt atgattaacc cattttcaca 240

attaacatta ggaaaagtta atcagaatgc taataccatt tcattccaaa ccattaatga 300

ttatggcgca cagactcctg ttttaaaaaa agaaatcgtt cattaa 346

<210> 708
<211> 563
<212> DNA
<213> Proteus mirabilis

<400> 708

tggctgactc tcctgatgcc gtcaccgatt taagttattt tgaagcaggc aaccgcatta 60

aacccggtga ttatcttctt gatattgttt ttaatcatga gtatctgcgt agtgaaaata 120

ttcattttat tagtcaagat aaccatgtta ttccttgttt aaatcgagat tattatcaat 180

cactcgggat caatattaaa ctatttgctg attttgagaa attctcggca aatgaatgta 240

ttgatattga aaaaatcatt ccagattctg ttgttaatta tgatattgag aaacaagctt 300

taaatattca agtccctcaa gccgcgttag atttgaaagc acgcggttat attccaccag 360

aaaaatggga taacggtata acagcaggta ttttaaacta cacctttagt ggcgctaata 420

gctggggaaa ttctcataat aatagctact acttaaatct acgtagtggg atcaatattg 480

gtgcttggcg attacgcgat tattccactt ggaattcgtc aaacgggaaa aaccaatgga 540

accatatcaa tacctatctg caa 563

<210> 709
<211> 527
<212> DNA
<213> Proteus mirabilis

<400> 709
atggataata agcgaacaca gcgggatatt atatttagca taatatggat tatttggtca 60

tgtgcattaa tggctttttg gcgatatcaa atagctggtg agcttttgat cacccttatc 120

ctaattttta gcattattat ttactctata actttgaggt taaaaaaaac ggcaatgttt 180

agtcgaaaaa cagaaacacc aaaagccgcg gagcctattg ctccagttat ggcagaagag 240

aagaagccta tgccggagca aaaattgtat accattattg ctaaaggcac tgtatttcaa 300

ggtgatatta acgttgatgg tgatattcaa atttggggta aaatttcagg gaatatcaat 360

gtaaaagatg gcgtgatccg tgttatgcat gcaggccaag ttgaagggga attgacggcg 420

ccagacatca ttattgatgg ttttgttaaa ggtatttgtg ccgcaaacaa tcttgatatt 480

ctagagcatg gtgaactacg tggcactagt cgttgtggca gtatgtc 527

<210> 710
<211> 431
<212> DNA
<213> Proteus mirabilis

<400> 710
ttatattgaa acactgaagc aacggctgga tgcgattaat caactcaggc tggaacgtgc 60

atttgcatcg atgagtgatg tgtttaaaca ggtgtatggt ttaattcctg ttttactgca 120

ttaccaccac cctcagttgc caggctatat tcaaggaaat gtccctcatg gtacatgttt 180

ctttgaacct gatgacgtgc aacgtcaatg ggttaataag ctgactaatg catcatgtga 240

tgagccaatg aatggataca ccagcggaga gttacctatt acgggtatct attcgatggg 300

aagtacttcc tcgattgggc aaagtcactg ctccgatatt gatatttggg tctgtcacca 360

atcttggctg gaccaagatg agcgtgcgcg tttacaacgt aaatgtttac tgatagaaca 420

atgggcagga g 431

<210> 711
<211> 528
<212> DNA
<213> Proteus mirabilis

<400> 711
cgctattaac cttgctgaac gtggtatgag tgtcactatc ttagaaaagg gtcagattgc 60

cggtgagcaa tcaggccgtg catacagcca aattattagt taccaaacat cgccagaaat 120

cttcccatta caccattatg ggaaaatatt atggcgtggc atgaatgaga aaattggtgc 180

ggataccagt tatcgtactc aaggtcgtgt agaagcgctg gcagatgaaa aagcattaga 240

taaagctcaa gcgtggatca aaacagctaa agaagcggca ggttttgata caccattaaa 300

tactcgcatc attaaaggtg aagagctatc aaatcgctta gtcggtgctc aaacgccatg 360

gactgttgct gcatttgaag aagattcagg ctctgttgat cctgaaacag gcacacctgc 420

actcgctcgt tatgccaaac aaatcggtgt gaaaatttat accaactgtg cagtaagagg 480

tattgaaact gcgggtggta aaatctctga tgtggtgagt gagaaagg 528

<210> 712
<211> 409
<212> DNA
<213> Proteus mirabilis

<400> 712
ttgttgcaat acagccatca tgcttttaac ttgagagtta ataaatgtca catctgccag 60

atatttaacg ctattatcgg gtacgtacaa attactccgt tccatatcca tatcgaccgt 120

gttaccatcc atcgatgttt gatgaggaac ccgataaagc aaatcagcct ctaagcgata 180

acccggctta ataggaatat gccgttctga tgtcattgtt agttgcaatc catgactacc 240

agtacgcccg ttttccatgg tttttttcaa ctcagctgcg aaatcaatat cccgagcttg 300

gaagcctggc gtatcagcat tagcaatatt tgccgccaaa atttcttggc gtttattacg 360

tagtgaaagc gcttcttgtt gaaaatgaaa cgtattttgt aatttatcg 409

<210> 713
<211> 513
<212> DNA
<213> Proteus mirabilis

<400> 713
aactttagcc ccactactat cagtaatatt gactgtaagc gtatctattg gactaaagga 60

ttcaaaacca aaggcgctgg cgaagatatt ttcattatca ggatctggcg tatcagcaga 120

tcttaataac ggatctaatt tagtatcagg cgttttatct gtatcgatat ctgtatctgc 180

atcagatcca gcgttttcac cattattact cttatcaacg tagatatcat taccggcaat    240

catcaccata tgattaacca atgaggtcgc acgtaatgcc tgactttggt caatttggcc    300

aacaatagta ttgactgttt tatttagcgt ctctatccct tcaaccgtag aaatctgtgc    360

taactgtgac gttaactcat tattctgcat tggattggtg ggatcttggt tttgcatctg    420

cgtgataagc aacgtcagga aattaccttt aatatcatca ctcccccctt ttttagtgtg    480

gtaggacgaa ggcgcttccc cgataatggt att                                 513


<210>  714
<211>  404
<212>  DNA
<213>  Proteus mirabilis

<400>  714
actccgccag accttagatc ttcagttatc acagctcaat accattgcgg gtattttacg     60

tgctgagcaa cagttattat gtgcaggaag tattgatatc aataagctgc atgaaataac    120

tgaacagaag aattttgtat taacagctct aggtcatacc gatcaaaaac gtcaaatact    180

cagtaaacaa gttggtattg atagacccta tcaaggacag ccttttttag ctgatttatg    240

ggggcaactg gttgatctaa cggaagagtt aaaacatctt aatcaacata atggcttatt    300

gttagagcaa catattactc gcaatagtga aacgctgcat tttctacaga aaaatcatag    360

cccaacactt tatggtgcag atggacaagc acagcgttca atat                     404


<210>  715
<211>  236
<212>  DNA
<213>  Proteus mirabilis

<400>  715
gcgattctat ggctgatgca ctaaaagagc taacattgcc tcaattggtt aagttggctg     60

aaacaaacca actaatctgt aatttccggt ttgaagacag cgaaacaata gaacaactca    120

ctaaagaatc cagagtggat gatttgcaac aaattcatac tggtatcctt ctttcttcta    180

acttgttccg tcaactatcg gaacatgata cctctgctac aaagaaacgg gcataa        236


<210>  716
<211>  633
<212>  DNA
<213>  Proteus mirabilis

<400>  716
tctaatctta attcctctgt tcctattaac aagtttttac cttcaatatc aactttatca     60

tcatttaatt gtaatataac atgaccatgc ccctggattt taccaccaag agatatacta    120

tcatcagcga caatatgaaa aacactatta tacgacaatg atatattgtt tatttctagg    180

gttcctactc ttccatataa ataactatca tcaatattaa atgttctatc taaaatcaac    240

gtatccgctt tcacaaatag aatactatct ttaacattaa atgatgattc attaatataa 300

gaagaatatt cataaaccat tacttttgat tgtttcgtcc catcatgagc tgttctatat 360

aaattatcat cttgatttat atctatgtgt cctgtgttaa ttaagccata agactcatga 420

tttagtgaca aagtatttat attaccatca ttataaacgt taccctcttt tgcttttatt 480

attaaatatc gactatttat tcctgcctcg gcatttaatg aaaatagagc atttctatca 540

taatcatttt tagctccaag attaaactca cttaacccca tatcaaaacg aatatattca 600

gcatttaaat cacctttaat caagtctata tca 633

<210> 717
<211> 628
<212> DNA
<213> Proteus mirabilis

<400> 717
tctttactga gcttccatcc agccgatccg agttggtcac aaaccacgtg gaatgaacct 60

attcaaaact taggtggcaa tattggtgca tggcttgctg atatcctttt ttcagcattt 120

ggcttgctcg cttatgcaat ccctattgtg gtggtatttg gttgctggaa tgcattacgt 180

catcaaaaaa atcgtgaata cacagatttt ttctcccttg cattacgtac aattggtgcc 240

ttggctctgg ttcttacatc ttgtgcgtta gctgatctta attttgatga tatctacaat 300

tttagctctg gtggggtgat tggtagctta tttagcaagg cattgctacc ttggtttaat 360

atgctaggtg caacactggc gctactatcg gtatgggcga taggctttac gctatttact 420

ggctggtcat ggctgacgat taccgaaaag attggtgcgg ttatcttagg tgcggttgct 480

tttattacta atcgtggtca aaaagatatt gattatgatg attatgaaga acccgccgat 540

cctgctcagg cagatcccga ggcgttggtc gataacaaca cccagccaga acatcaactt 600

tctgcgcaaa tagagccaga tagtgatg 628

<210> 718
<211> 501
<212> DNA
<213> Proteus mirabilis

<400> 718
tattgagcgc attgatttac gcaccaaaaa aacagagtca gggaaagatt ttcttgccat 60

caaccctaaa gggcaagttc cggttcttca attagataat ggtgatattt taacggaggg 120

tgttgctatt gtgcagtatc ttgccgatct gaagccagat agaaatctta ttgccccacc 180

aaaagcatta gaacgttatc atcaaattga gtggctaaac tttcttgcca gtgaagttca 240

taaaggctac agccctctat tttcatctga tacgcctgaa agttatctcc ctgtggtaaa 300

aaacaaacta aaaagtaaat ttgtttatat taatgatgta ctaagcaaac aaaaatgtgt 360

ttgtggtgat cactttactg tggcggatgc gtatctgttt acgttaagtc aatgggcacc 420

tcatgtggcg ctagatttaa ccgacttaag ccatttacaa gactatctag cacgtattgc 480

acaacgtcct aatgtgcata g 501

<210> 719
<211> 301
<212> DNA
<213> Proteus mirabilis

<400> 719
tcatatcgct gtattacctt gatatacagc ggttaaaatc tctctagctc ctttatctag 60

tagctcttcg gcaagagaaa taccagcttg ctctgcatct tcaggtgata ctaagcgttc 120

gccacgtaaa atagttttac catcaggggc gccaactaat gctcgtagcc aaattttgtc 180

gttttgccaa attgcataac taccaatagg tacttggcac cccccctcaa ggcgagtgtt 240

catcgcacgt tctgctttta cacaagttgc agtatctgcg tggttaaggg cggctaataa 300

g 301

<210> 720
<211> 507
<212> DNA
<213> Proteus mirabilis

<400> 720
agcgcaaact cttcagatac ataatcaata aatagacgct gataacattc acattcatca 60

acaattgcac cgcgttgacg taatgttgtg gcaagtagtt cgcgaccacc attgcctctt 120

agtagtagta ctcgcttgtt ttctatttgt tgaagtgaag acaaggccag taggtcttca 180

ctggtttctc caaattctgg ataacgtata gaatgtgctg ttaattgctg aaactcttcg 240

gcggtgcttt gacctattcc ataatagaat agcgtatctg gccacgattg ttgtaattga 300

tttagttgcc agtttgcgta ccacaccgca tttttagaaa gtaaaaaaag gtaatcaccc 360

gtacttagct tatttaattt gttttctaat atggaaagct ctttacccgc ggctatttct 420

attaaaggtg catgaaaagc atgctttccc gcatcaatta agcgttgcgt aagttgttct 480

cctgctgggc taggacgagt gattaaa 507

<210> 721
<211> 402
<212> DNA
<213> Proteus mirabilis

<400> 721
aatattggtc aagctgcaaa aaatccggta ttttcaagta aaatgatccg atattatgag 60

caaattggtt taatacctaa ggcaattcgt actgacggag gttatcgtga ttacaatgat 120

agcgatgtag attgttttcg ctttatcagc cattcacgtg ctcttggttt ttcaacagag 180

caaatatcaa cattattagt tttatggaat aacagagaac ggacaagtgc tgatgtaaaa 240

gctattgctc tttctcatat cgatgaatta aaccgtaaaa taacgcaatt gcaacgaatg 300

acgcaaacat tatcgcattt agcacaagag tgccaaggtg ataataatcc tgattgccca 360

attattgcta agctagtcga accccaaaca gggacagaac at 402

<210> 722
<211> 300
<212> DNA
<213> Proteus mirabilis

<400> 722
aaatagtggt ggtgtgttcc aagagcaacc tgactttaaa gagccaccac tttctattga 60

aggtgcagcc gatcactgga accatcgtga agatgaagat tatttcagcc aacctcgtgc 120

actgtatgag ctattaagcg atgacgagca tcaacgtatg tttgcgcgta ttgcgggcga 180

attatcacaa gcaagtaaag aaacacaaca acgccaaatc gacttattta ccaaagttca 240

tcctgaatat ggcgctggtg ttgaaaaagc gattaaagtg ttagaaggga aagacgctaa 300

<210> 723
<211> 220
<212> DNA
<213> Proteus mirabilis

<400> 723
atgaaagcaa aaattgtact aggtgcggta attctggctt caggcctatt agcaggttgt 60

tcttctagca acaacgcaca attagaccaa atctcttctg atgtaaaccg tttaaatacg 120

caagttcaac aactaagtag tgatgttcaa tcagctaacg ctcaagcaaa agccgcttat 180

gaagcagctc gtgctaatca gcgtctagat aaccaagtaa 220

<210> 724
<211> 521
<212> DNA
<213> Proteus mirabilis

<400> 724
tgcacttgtt tacgagagat tttgatccct ccattcttta gctgttctgg taatagtaaa 60

caagcgatgg gacgttgaaa gcctgctaaa cgcgtatgat accattgtgg taattgatta 120

attgtctcaa tatcagtatc aacaacagca acgggacgtt gaccaaattc aatatcgtca 180

atcgggacaa taaatgtttg gcgaacatta gggtgactat ttagcacctt ttcaatatct 240

tctggctgta taccttcacc cgcactaaaa aacagattat ctaatcgccc taaaatgcac 300

cattcctctt ctttaaaaca acctctatcc cgtgttgcat accaagcatc ctgagtctgt 360

gataacggct ttaattgacc atcaaaccaa tatcctaatg caacactatc agatttgatc 420

tggatttcat tatccactaa tctgaccgct ttacccttta atggcgtacc aactcccgct 480

ttaccatctg cgcgttttgc acaaacagta gaggccattt c 521

<210> 725
<211> 273
<212> DNA
<213> Proteus mirabilis

<400> 725
cagacaatgc gtttattttg tgttcactaa aagcggtgag catatcgtcg ataaaagtga 60

gtcgtttttc tcgatcggtt agggtgatca taaatttcaa tttggtaggg ccatctaaac 120

ggaagatgct cggttgctct tgcaataaac cgattaaata agtcgggtcg acattatttt 180

tttcaccaaa ctcaataaag ccacctttgt catgggcttc aatgcgagat atacctaaac 240

tcatggcgct taatcgaatc gccgcattac gta 273

<210> 726
<211> 769
<212> DNA
<213> Proteus mirabilis

<400> 726
aaacaataga tactttgcca cttaactttc gtattttaat aaaattagcc ccactccctc 60

tgattagtgg cattattatg gcaataatct caacaatgct aagtttagca ccattatgga 120

tcatctataa aatcagccag atttgttttt caacatcacc taatattcaa caaataaata 180

atctagttta tatcactgtg attattttaa ttttacgttg gggattaatg gcaataagtc 240

atattgccgc acatcggggt gctttttata ttcagcatca attacaactc gcaatagcta 300

aaaaaatcag taaagtacca ttatcatttt ttgctcaata tggcagtgga aatctgcgac 360

gtattatcaa tgatgatata aaaagcttag aaggtttttt cgcacatatg ctacctgatc 420

tcgtctcagc catagtgact ccctttatcg ctattatatt acttttttat gtaaattggc 480

ctcttgcctt attatctcta accccattac ccattgcttt tatggctcaa cttctcatgt 540

tgcgtcgagc caataaacaa accaatgagt ggatgaatat tcagaaaaaa attgctaatg 600

aaataggaga atatattaaa ggaatacgag aaataaaagc gtttaactta acctcccata 660

cttttggtaa attatctcaa tctatcaatt cctctgttaa atggataaaa aataatgtca 720

aagctagtac aggctcttgg atggtattta gtgggatatt aacagcgaa 769

<210> 727
<211> 516
<212> DNA
<213> Proteus mirabilis

<400> 727
cttgccgtag ttgaaggtct ttttgctact attccatata ttctactcta ttttttatta 60

attgatcttt ttgccaataa aatcacacta gctcagttat tttattactt tttatcaata 120

ctcttatcta ttgttctgcg tattgtcatt ggtacttata gtatgccaat gatttttatt 180

ggtgcttata aaatgatggg acaagcccgt ctaagaatag ccgatcattt acgaaaaatc 240

ccgattggct ggttttcttc tcaacgcagt ggtgatcttg catcacggct tactgctgat 300

ttagagatca ttcaaaatat ctggtcacat ttcttaggaa tgtttatcag tactttagcg 360

atgcctgttt ttctctcact attcttagta tgggtagatt ggcaactcac tttaattata 420

ttattttcta ttccaatcgc tctgttcgca ttatccataa gccataaaat aatgttaaaa 480

gctgcacaac aggcagctga tgctaatgcc aatgta 516

<210> 728
<211> 673
<212> DNA
<213> Proteus mirabilis

<400> 728
ttaagtaaag ctaaatccag aatgcacatc ttaaataacg atgaaatttc atcgttattc 60

gactaccctt ttataaaaca acgaaatatt tttttgctat caagagtcat gttacgcgat 120

attttatcat tttatttaaa aatttcacca gaagatgtaa gattttcaaa aaatgagtac 180

gggaaaccat ttattttaaa cgaatcaaag gaaagcattt acttcaattt atcacattca 240

aataattgtg ttgctctcgc tatttcaaat acatcatccg ttggtattga tattgaatat 300

ttcaaccgtg atatagaaat aaatagcatt atagattatt atttctcaaa aaaagaaaaa 360

aaatacctat cttattttga cgagactcaa aaaaaacata atttctataa gatgtggaca 420

ttaaaagaag cctatattaa atcaagaggc attggattat cagaagaaat cattaagaat 480

ttagattttt atataaagag agatcaattt gataaattat attttataga acagcattac 540

tctgctcatc tttcatatat taccaaatca atattagata gctataaaat atccataatc 600

acatatcacc atccatttaa ctataaattc ttaacatggg gtgatataaa aagcaacctg 660

ccacatcatt tat 673

<210> 729
<211> 682
<212> DNA
<213> Proteus mirabilis

<400> 729
attattctcg ccagtgtttc gacagaaaac gatcactttg atcaagagtt acctagtgac 60

accaagatac aacaaagttc acactaccaa tggcaattaa caccttgtgt ggaacaagat 120

cttatcaagc caacattaat atttaaccat caacaaacat tgccttctga aatcacagct 180

attttatccg ctattggatg tctttctgaa cagggcgaaa atcaattaat cgttgccaac 240

cttgcagatc ctttaattat cgccgagcag atccgccaaa tattactgtc atcgtctgat 300

gggtttgttg ttatcacgca acaagcctgg gcattaactg ccatagaaac ggttaatcca 360

gcacaacgta gtattcgaag tttattaaaa actattcaaa aagaatatag ctcaaggtta 420

attgccattg ttgatttagg tataaatgcc tcatggtctg aattagttcc tgcttttata 480

caaatagaac aaggtaataa tgaaattatt gttcgtaacc attgctgcta cttaccacaa 540

ttaaccccac tgccctcatc ttctacgatc atagcgcaga acatcatggt atccccgcgc 600

tggcatatta ttactggtgg ttttggaggc ttaggtcgaa ttacagcaag ttggttagta 660

aggcaaggtg ctaaacgtat cg 682

<210> 730
<211> 609
<212> DNA
<213> Proteus mirabilis

<400> 730
aatgtaaata aagacaatgc taacctattg gtttgcccat ttgctggtgc cagtaatagt 60

gcatttaact cgtggagatc taccgatatt tcagggttaa attgtcaatt agttaattac 120

tctggccatg gttgcagatt taaagaacca gcctttaatg atattgggtt attagccaat 180

gaattaataa caataataaa gaaattttat ccaccacggc ataattcatt attactttgc 240

ggtcacagta tgggggccca agttgccttt gaaactgcta ttcaattaga aaaaaatggc 300

tgggaattat ctggactaat attatcaggc tgccaagctc ctcatattca agcaaggaga 360

ttactgagtg atttaaatga tgatgacttt attcaacaat taattgccat tggtggatgt 420

gatgctgaat taatcaagca gccacagttg ttaaaacagt ttatgccatt attacgtgct 480

gatttccttg ctaccgagcg ttattttttt caaaaaagca ctaaacggct ttttcatacc 540

cctgttttat taatgtatgg tagtcatgat agtgaagctg ataaaaacga agttgaagca 600

tggcaagat 609

<210> 731
<211> 609
<212> DNA
<213> Proteus mirabilis

<400> 731
taaagcattc ggtattccac tttttactcg atttgatcaa ctacctgaaa aaatagatct 60

agcttgtatt gttgtgcgat cagccattgt tggtggcgaa ggtagccaac ttgcgcaagc 120

atttttacaa cgggggatct cggttgtaca ggaacatcct gtacatcctg atgaaattac 180

cagactacaa tcattagcag aaaaaatgca ttgccactat atcgttaaca gcctctatcc 240

acataataaa gcaggacgtt tatggataga aaacacacag aagatatatc agcaaataca 300

acaacgacca gtgtggggac aaattatcac aagcaggcaa ttaatttatt ccgccttaga 360

tatatattgc caagcaatga aattacaccc taatgatatt acagtcacat tagaaaaaga 420

taataccccg ctacaatttc tacgactatc caaccctact ggtgatttgc ttttatgcct 480

acaaaaacat ttgtcatcta acgatcctga tcagcatagc ctcgtgatgc atcatatgat 540

attaggctgg ccagctggtt atttaactct cgctggaagt tatgggccag tagaatggaa 600

taatgcgct 609

<210> 732
<211> 502
<212> DNA
<213> Proteus mirabilis

<400> 732
aattggctac tcctcgatta cgtttacgag aatggcgtga aagtgataaa gcgccatttt 60

ttttacatat taacgcctcc gcatcggtga tgcgttattt cccttcacca cttactcgtg 120

cagaaagcga tgcaatggtt gatacattgc gtgataaatt tattcagcaa aatggttggg 180

gattttgggc cgttgaatta aaagagactc aagagcttat tggctttgtt ggattaaata 240

ttcctaatgc ccctttgcct tttaatccgt gtgttgaaat aggttggcgt attgcacaat 300

ctcattggcg caaagggtac acttatgagg cggctttaac agtattaaaa tatgcttttg 360

aacagttgaa actggaagaa gtcgtggcat ttaccgcagt gactaattta ccctcagaag 420

gggtgatgaa aaaactcggt atgaagaagt ctgaatattt tatgcatcca tctctagata 480

aaacacaccc tttagcacaa ca 502

<210> 733
<211> 511
<212> DNA
<213> Proteus mirabilis

<400> 733
tgcggcttta gtatttggta ttaattctgt tgctacagct gaaaatgaaa cgcctgcacc 60

aaaagtaagt tcaactaaag gcgaaattca attaaaaggt gaaattgtta attcagcatg 120

tggattagca gcatcttcaa gccctgtaat tgttgatttc agtgaaattc caacttctgc 180

attagcaaat ctgcaaaaag caggaaatat caaaaaagat attgaattac aagactgtga 240

tacaactgta gcgaaaactg ccacagttag ctatacacca agtgttgtta acgctgtaaa 300

taaagattta gcctcttttg tttctggtaa cgcatctggc gcaggtattg gcttaatgga 360

tgcaggtagt aaagcagtta aatggaatac tgcaactaca ccagtacaat taattaacgg 420

tgtatctaaa atcccattcg ttgcttatgt tcaagctgaa tcagctgacg ctaaagtaac 480

gccaggtgaa ttccaagccg ttatcaactt c 511

<210> 734
<211> 726
<212> DNA
<213> Proteus mirabilis

<400> 734
tttctcacaa gcagagcagg acgattctgt ggaatttaac attcatatgc tagacgcgga 60

agatcgcgat aatgtcgacc tttcacgttt ttctacctca aattatatca ttccgggtat 120

gtactattta gatattcgtc taaatggtcg cgactttcct cgccaaaata ttaattatat 180

tgaagtagca gataatcatt ccgtggcttg tatcgaccct actcttttaa aaaagttaac 240

aatcaaccaa gaaaaccaaa aatatatcaa acaaatatca ccagattgtt ttgatattag 300

ccaattaccc ggtatctcga ttaaaaatga tggtggtgta cttgatatca cgttaccgcg 360

ctcattaatg aaatatgaag aatctgattg gacacctccg gagctttggg atagcggggt 420

ctctgggctt atttttgatt atacactaac aggaacgtca actcgcccta ataaaggcaa 480

taataacaat acgttaactg gttatggtca agcgggatta aacttgggtg aatggcgttt 540

acgagctgaa tatcaaggca attattcttc tgaatattca tctaacaatc gttttgattg 600

gaaccaaatt tatgcctata agccattacc tgatctcgca gctaaattaa cggttgggga 660

aacttattta aactctcaaa tttttgatag tttccgtttt acaggagcca atttacaaag 720

cgatga 726


<210> 735
<211> 568
<212> DNA
<213> Proteus mirabilis

<400> 735
atgccgtatt agatcacacc acctttccta ataacaaagc gggagaatta gcaacagtaa 60

acttttcgtt gcctgatcgc tatgatggca cggtatattg tcctaactca cgtatttatg 120

atcgtgcatt aacctatttt aaagcaacca ctgatttacc tcctgttggt aataactttt 180

atcaattaaa tgagtatgtt gatatcaaaa ttaattttga aatttggggg cctaatcctt 240

tacccacagt gcccttttct gacataccta ataatagaaa taaccaacaa ggttgcagag 300

taccctcttc acctaaaccg catatttcct caggaagtag cggtcaactc actttccgtt 360

taagaaaacc cattattaat ggtgtcagtc ttaatgggca atctcttgca caaatgtatg 420

ccatggtaag tcacagcggt gcgccaaaaa cctatggttc agagcccatt tctaaattag 480

tgatcacctc ggggatcatt accactaaag ataaatgtat ttttaataat ggttcaccaa 540

ttacctttga ctttggtaat gtgggaaa 568


<210> 736
<211> 544
<212> DNA
<213> Proteus mirabilis

<400> 736
aacaggcaca ttaacagagg gtaaacctca agtcactgat gtcatagcta atgtaggctt 60

taatgagaaa gagctactga tgttggcttc ttctgtagaa gttggctctc atcaccctct 120

tgcaaaagcc attattaata aagcacaaga gcaacaaatt gatgttgtgg aagccgataa 180

tcgcaaggct ttagcgggta aaggtattga aggttattta aataatcagc atattctggt 240

cagtgcccca acacaattat cagaaaccat accattatct gcacaatggc aacaacaagt 300

cgctcgtctt gaagatgaag gcaaaaccgt tgtggtggta ttaaaagaag atcagttcat 360

tggtgtgatt gcgatgcaag atacattgcg caacgatgct atcgaatcaa tgaaagagaa 420

gaaagtgttg aaatcgatga atatcaatgc cgtgatgtta accggtgata acccaagagc 480

agcggctgcg attgcacaaa aactgggtat ggatttccgt gcaggattgc tccctgaaga 540

taaa 544

<210> 737
<211> 641
<212> DNA
<213> Proteus mirabilis

<400> 737
gcacactgac ccaattaaag ccaaatacat taactcgctt atatgctctg tttcttctgt 60

ttatggccat atcccttttt ctatatgctt atagctattt tgatacttgg ctagaaagta 120

aaaaaaatgc cattaacaac acgactaata agtttgcatc tcaagttgaa gattaccgct 180

atcacgctaa ccaactattc cagttatcaa acaaaattaa tgatccaacc ctctttctgc 240

ctttaaaaat caatccggtc aaactacgct ctgatgttta ttggcttgaa ggacgcgatc 300

agaccgttga tgctattgtt tttggtaaat cgaatgaaca aacctttcag ttagccggtt 360

attttgcaaa cgcgttagaa attatttggg gggtacgtaa taactatagc tctctctatt 420

atcttaatgg taaaggcaat gatcttatcc ttattactac ccactcaata ctaaaaccag 480

aattgcgtta taaagaaagc tatttaacac taacggctga aaacaaacgt tctgagctat 540

taatgcaatc aacggcatta gatgaaaaag agagcctttc tcccattagg aaaatgccga 600

cagaaaacat ttattactat acctatcgca ccatgtttaa t 641

<210> 738
<211> 699
<212> DNA
<213> Proteus mirabilis

<400> 738
tggcttggaa acacaatcat tcattcccgc atcaatacaa cgttgttttt cttcggcaat 60

cgcatttgct gtaacgccaa taataggaat agtgctactc aattcacgca ctgttgtcgc 120

taattgatag ccattcatat ttggcatatt gacatcggtt aaaataatat cgacatgatt 180

ttcttgcata aaggctaaag catcacagcc atcttctgcc gttgcggtat taaaaccaat 240

ttttttcagt tgatcggtca acaataaacg attgataggg tgatcatcaa caatgagcac 300

cgtcaataat tgtagatcgt gatctgtcat taacgcgtta ttttctgagt catcagactc 360

taattttgtt tgtggtaatt gtaggataat cttaattaat tcatttaatt tatatgtact 420

gcataaccaa ttattttcag agattttctt tgctggctca aaatagtgtt cataaatacg 480

gataaattgg caagagttat ctaaacattc atcatgatcg gtgataataa aatcattctc 540

agatacctga gtcacttccg tgaataattg acaatgtaag cccacataac ttagatatcg 600

ttcaacaaag ctttctagat agagattttt aatactgata aagcagcgaa tagtactctc 660

tttatagaga ttatatttgg tttgtccatc actcgaatt 699

<210> 739
<211> 341
<212> DNA
<213> Proteus mirabilis

<400> 739
cgagcatgac actaataatg gctttactgt cttggatgct gcacaagtta atgatcgtgg 60

tgttgatgat ttagtcgcgc aaattaaaga gattgtgggt tcacttcctg tttatttgac 120

ttttgatatt gattgccttg atcccgcatt tgcaccgggt acaggaacac cggttgtggg 180

gggattaacc acggataagg cgctgaaaat gctgcgtgct ttacagccgt taaatattgt 240

aggcatggac ttagttgaag tatcgccagc gtatgatcaa tcagatatta ccgcccttgc 300

cggagcaacc attgcacttg atatgctata tctgcaagcg g 341

<210> 740
<211> 323
<212> DNA
<213> Proteus mirabilis

<400> 740
tacgtacatc gccaccagcg aaaactgcat attgccatta atacttttgc tcatcctgat 60

ggatttgaac gctggcaaaa agccattgat atggcggctc atttaggtgc cgatgcatta 120

attttggccg atattgctat gctagagtac gcagctgaac gctatccaca gatagagcgt 180

catgtatcgg tacaggcgtc ggccactaat actcaggcaa tcgcatttta tcaacgcaat 240

tttgatgttg cacgtattgt actaccacgc gttctttcta ttcatcaagt caaacaattg 300

gctcaaagta gtcctgttcc ttt 323

<210> 741
<211> 360
<212> DNA
<213> Proteus mirabilis

<400> 741
gaaatacgca ttaggctctg tactttatta ttggcaaaaa gaaacacttg agacatttta 60

tcggcaagca aaacagagcg atgctgatat tatctactta ggcgaaacag tctgtagtaa 120

gcgccgtgag actaaaccac aagattggat taatctggcc aaagaagtgg ctaaaagtgg 180

taaacaagtg atcctttcta ccttagcact actacaagcg ccttctgaac taaaagagat 240

agcaaagctg gtggataacg gtgaattttt agttgaggct catgattttg gtgtgatcaa 300

tatgctttat gagcgtcatt taccttttgt agtaggccat ggattaaact gctataacgc 360

<210> 742
<211> 516
<212> DNA
<213> Proteus mirabilis

<400> 742
caaggtttcg ctaactaaag agaaaccggc aattagctta actaaaaagg atgatttcgg 60

caaaatccgc attaacctcg attggcatcg agaaagtaaa agcggtggtt ccgggttatt 120

aggtggatta tttggtggta acaaaggtat tgatttagat attggcgcct ttgttgaact 180

acaagatggt tataagtcag tgatccaagc cttaggaaat ggattcggtg attttaatcg 240

catgccttat gttgagttac aaggtgatga tcgcactggt gatgtagcgg gtggcgagtg 300

gatttttatc aatggacgtg aatggaaaaa tatcaagcaa gtgcttattt ttacttttat 360

ttatgaaggg gttcctaact ggagtaaaac agatggtgtg gtcactattc atgttcccga 420

gcaaccacct atcgaaacac gtttaacgga tggtaataat ggtcgagcta tgtgtgccat 480

tgcacgactt attaatgaaa acggatcaat caaagt 516

<210> 743
<211> 516
<212> DNA
<213> Proteus mirabilis

<400> 743
ttctaaaggt ggtaatgttt ctttaagcaa agcagcccca acgatgaaaa acgtcctagt 60

cggacttggt tgggatgccc gttctacaga tggtcaagat tttgacttag atgcatctgc 120

atttctgtta gccgctaatg gaaaagtacg tagcgatgcc gatttcattt tttataacaa 180

cttaagatct tccgacggct ctgttgttca cactggtgat aaccgaacag gtgaaggtga 240

tggtgatgat gaagcactaa aaatcaaact agataccatc cccagttatg tcgaaaaaat 300

tatctttgta gtgactatcc atgaagcgca accgcgtcgt caaagctttg gtcaggtatc 360

tggtgcgttt attcgtttag ttaatgatga caaccaaatt gaagttgctc gttatgattt 420

aacggaagat gcatcaacgg aaacggcgat gttatttggt gagttatatc gtcataacgg 480

tgagtggaaa ttccgtgctg taggccaagg atatgc 516

<210> 744
<211> 500

<212>  DNA
<213>  Proteus mirabilis

<400>  744
gagccttgtt tatccctctt tcattccctt tactcgcctt atcttgctac tgttgggcag      60

gaaatggtct ggtggataga acatggacag cttcaggaat ggatagctat agttatcgcc     120

aagcgctaag acagccaacg gttggctcac gttatactct ttttaatatt acacccgata     180

tgccaacgcc aggtggcacg agtcctgttg gtactaaagg aattcgctat attgcgatga     240

aatatggccc ttatggacaa cctgaacact ataaaaccta ccaagtgatg ttctctcact     300

attccaccac cactacacgt aaagttcgtt atttaggtga gttatatacc gttgtcggtg     360

atatttattt aatcgatcct gctgctacca ccaatgaatg gcaacgcggt cgtagccaaa     420

tagttgaaga gtattatgag attttagata cacatggaaa taggacaggc aaaggattgc     480

gttttaaccg ctgggataga                                                 500


<210>  745
<211>  550
<212>  DNA
<213>  Proteus mirabilis

<400>  745
gctcatatca tcttccatcc ctcgccaaac cgacttaccg taaactaaca caagatgatt      60

atggggtaat tagtgactat ttgtcctatt ttggcacctc taagttttct gctgggtatt     120

cgttacaaaa ctttcctgaa atacccacta aaggtgaagt cgttacgaca ctgcgtaata     180

ttgttaatcg gtttgcggga tcatcagagg ggatcaatca ttggcgctat tacattgatg     240

cggtagagat ccatattcct ccattactgg tgccttatct gcaacaagaa aatgtcctcg     300

atgtcgtttg tactccttct atccccattg tcattggtgt gaatggccat tttcttaaag     360

atgaaaaatc acatttttct gcgttaagtt taaaacaact ttctgaaccc atactgtcaa     420

atggcacttc cactatccag aaaaatgaag gtgatgcggc gcatttatta catattcgcc     480

aagaaaccaa cgaagagtat cggttacacc attcttcagg tttttggaat ggttcgttaa     540

tttgcttagg                                                            550


<210>  746
<211>  401
<212>  DNA
<213>  Proteus mirabilis

<400>  746
aagataggta cgcttttaat ttcttacagt ttactcacaa tgagtttgat ctctttttcc      60

tcctttgctc aagtaaatca cgatcccctg accaaatgtt atgagttgtc aacagatgca     120

agccaaacaa ccattaaatc ttgtctatta gatgaactga gattatctga agagcagttg     180

aatgttatct ataataaaag caaaggcgac cttgaagata gtgactctat cgcggctaaa     240

agtgctattg atgcattagt cagttcacaa gagcagttta ttctttttag aagtagtgaa 300

tgccaacgtc aatctgcttt aatgatgggg ggcaatggtg ctgatgaagt actgctggct 360

tgtgaaataa aattaaatca atggcgagct aaattattac t 401

<210> 747
<211> 513
<212> DNA
<213> Proteus mirabilis

<400> 747
tcacagtcac cactaatctc acgttgatta ttcctaaata tagtcaagtt tcttgtgatg 60

tgacaaattt tttcccgacc aaaccgattg aattacatac cttagtactg tctgaaactg 120

aattacaatc tgtgttctct ttactcaaac cattaataaa atcaggggcg ccgattactc 180

gtcatcttcc agattatcat ctatcaacac ctgaggtggt taaaactaat tttacgttac 240

ttcagcaatg tctaccgctt gaacatggca ccccctctca agagaccctg tttatgcaac 300

agagcctctt ttttattttg ctggcggttt atcacgaagg ggtcgatatt cttaatattt 360

ttcgttttaa ttatgatgag ccaaaaaatc aggcgatcac tcatctaata acacaagatc 420

cgcaacgtaa atggcattta gaggatgtag caaaaacgct ctatactaca ccatcaacat 480

tacgtcgcca tttaagtaaa gagggcgttt cgt 513

<210> 748
<211> 583
<212> DNA
<213> Proteus mirabilis

<400> 748
acgtccctga aacactctca ttagccattg atagcttctt aagttatatc gaagttgaac 60

ggcgattaag tccggtaacg gtagaaaatt accagcgaca attaatgacc attgcacaaa 120

tgatggttgc aataaaaatc aaccaatggt cgttactgga aagccaacat gtgcgcatgt 180

tattggctaa aagccatcgc agtggattac aacctgcaag tttagcattg cgcttttcag 240

cattgcgtag cttccttgat tggcaagttt ctcaaggaat gttagcagta aaccccgcca 300

aaggggttcg aacacccaaa tcaggtcgtc acttgcctaa aaatatggat gttgatgaag 360

tcagccagtt gatgaatatc gacttaaaag atcccctctc tgtaagggat agaacgatgc 420

tggaagtgat gtatggcgct ggattacgtt tatctgaact gactaactta aatatcaatg 480

atattgatct ccaagaaggc gaagtccgag tattaggtaa aggcagtaaa gagcgtaaag 540

ttcccttggg aagaaaagct gtagagtggt tacagcattg gtt 583

<210> 749
<211> 193
<212> DNA

<213>  Proteus mirabilis

<400>  749
caggaacggg tttcttatca tcaataagat gtctaattgc taaataggga tgataaatta      60

gcattcgagg cccagcccaa cgcatgactt gtttcattgc tgctcttttt actggctgat     120

agcagtgaat tgggcattgt ttgcaggcgg gtttttcctc accatagcga catttatcta     180

gtcttttata agc                                                        193


<210>  750
<211>  520
<212>  DNA
<213>  Proteus mirabilis

<400>  750
atcacttcta tccaaaacga agtgaagaac gttttagacg aaatcaaccg tatttctgaa      60

caaactcagt ttaacggcgt taaagtactg agcggtgaga aatcagaaat ggttatccaa     120

gttggtacta acgataatga aactatcaaa tttaacttag ataaagttga taacgataca     180

ttaggtgttg ctagcgataa actgtttgat accaaaacag agaaaaaagg tgttacagca     240

gcaggtgcgg gtgttactga tgctaaaaaa atcaatgcag ctgcgacact ggatatgatg     300

gtatcactgg taaaagaatt taatcttgat ggtaaaccag taactgataa atttattgtt     360

actaaaggtg gtaaagacta tgtagcaact aaaagtgatt ttgaattaga tgctacaggt     420

actaaacttg gattaaaagc atctgccact acagaattta aagttgatgc aggtaaagac     480

gttaaaactt taaacgttaa agatgacgct ttagcaactt                           520


<210>  751
<211>  515
<212>  DNA
<213>  Proteus mirabilis

<400>  751
caacagtgat ttccatttga gaatctgtac cttctttacg agaagtcaga actaaatggt      60

taacgccatc ttgggctttt acaatagtgg cagaaacgtt gccttctttt ttattgatag     120

catcacgtaa ttcaataata gaagtttggc tgtctgttaa ctctattttt aaaggctctt     180

tttcaccttt ttgggtgatc actaaagtcc gtgttttgcc ttcacctaat gtttcaccaa     240

taggatcttt gatatcactg acggcttttg atttcagtgt ttgagcatgg gcaagctctg     300

ttacagagac cgtataatta ccaatgcttg ctttaccatc agtggttact ttaaaggcat     360

caaactcatc atcaactttg gtggcgacga ttttatcgaa tttttttaat tcttcagatg     420

ctttctgtaa tttatctaat tggctacgaa tttttccata tgcagtaatt tgtgcatcgt     480

agcttttcat ctgtttgtct aaaggttcaa ggcgt                                515


<210>  752

<211>  274
<212>  DNA
<213>  Proteus mirabilis

<400>  752
acacaatcca ttcatcagtt agagcaggat ctaggacgac caccatcaga acaggaagtt      60

gctgatcatt tgcagattga gttagcagaa taccggcaga tcctattgga tacaaataac     120

agccagttgt tctcttatga cgaatggcat gaaatttacg gtgaaagctg tgaaccgtct     180

caagacgaag atcacgatga caatccttta caaatgttat tggaaagtga tatacgccaa     240

agagtcatag acgcgataga attgcttccc gaaa                                 274


<210>  753
<211>  657
<212>  DNA
<213>  Proteus mirabilis

<400>  753
gacttaattg ctcctcgtat tgatagtaga gggaaaatca ctgctgctga aatttcagcc      60

tttactggac aaaacacctt ctcacaacat tttgatattc tctcttcaca aaaacccgtt     120

tcagcattag atagctattt ctttggtagt atgcaatcgg gtcgtatccg cattattaat     180

acggctgaag gtagtggagt taaattagca ggtaaattta ccgcagataa cgacctaagt     240

gttaaagccg ataatattca aacagatagt caagtccgtt atgacagtta cgataaagat     300

ggcagtgaaa attaccaaaa ctatcgtggc gggatcacgg ttaataatag tggctctagt     360

caaacactca ctaaaaccga attaaaaggt aaaaacatca cattagtagc gagtagccat     420

aatcaaatca aagcctctga tttaatgggg gatgacatca cgttacaagg tgctgattta     480

actatcgatg gtaaacagct acagcaaaaa gagaccgata ttgataatcg ctggttctac     540

tcgtggaaat acgatgtgac taaagagaaa gaacaaatac agcaaattgg tagccaaatt     600

gatgctaaaa ataatgcgac attaaccgca actaaaggag atgttacctt agacgcg        657


<210>  754
<211>  622
<212>  DNA
<213>  Proteus mirabilis

<400>  754
attaagcgca aatgaaacag gaaatttagg ctcaatcagt gaatcaaggc gtgcattgca      60

agatagccaa cgtgaaatta atcaattaat agaacaaaat cgctatcagc aactgcaaga     120

aaaagcggta aatatttcac ctaccccaac tttaattact gagtcagaac actgtttgcc     180

tataaaaggc gtttatattc aaggtattac tttacttact gagaaggatc tcaattcatt     240

atctccgtta cctgatcaat gtattaagag tgctgatatt aatcgcctcg taaaagaact     300

cacacagcgt tatcttcaac atggttatat taccgcacgt atccaatttt tacgtcctaa     360

ccaacatggc gaattaggtc tgtatgctat tgaagggttt gttgaacgta ttgaaggggg 420

tgatcgaggt gttaacacca cactactatt tcctcgaatc aaagggcaac cattaaaact 480

cgctacactc gatcaaggct tagatcaagc taaccgtttg caatcaaata aagtcacagt 540

ggatattctt cccggtaccg aattgggggg ctctgtcatt aagttgtcta atcaacgaaa 600

atcaccttgg catctcaata tc 622

<210> 755
<211> 450
<212> DNA
<213> Proteus mirabilis

<400> 755
aaaaatgtag tgtttcagat tttagcatta ttaagagata gtattttagt taaaagtgat 60

cgctgttcaa tgcttaattc cattgaagcc agagctccaa ttctggatta taggataatt 120

gaatttgcat ttaatgaggt tcctgataat tttaaaatta gaaatggaat gaaaaaattc 180

ctattgaaag atatatcaaa aaaaatatta cctaatgagt ttgattttca gaggaaatta 240

ggatttaatc taccactagg tatgatgatc agagagggaa aatggaagga atttttcggt 300

gatatattaa attcaaaatc tgatataatt aattattatt tttatactaa aatgtttgat 360

gagcatttaa gtggtaaaga gcgtgcagat cgtctatttg gcgtagtttt atttctaatc 420

tgggcaaaac ataataaagt atcgctatga 450

<210> 756
<211> 400
<212> DNA
<213> Proteus mirabilis

<400> 756
taaattagct ttagttcttg gtttaggttt atctgttgtt gcgggttctg ctttagctgc 60

agatcaaggt catggtactg ttaaatttgt tggttcaatc attgatgctc cttgctcaat 120

tactcctgat actgaaaatc aaacagttcc actaggtcaa atttctactg ctgcattaaa 180

agatggtggc cgtagtaatt ctcgtgactt taaaatctct ttagaaaatt gtactacaga 240

gacttacaaa actgttcaaa caactttcac tggctctgaa gcaactgaag ttttagaagg 300

ttctttaggc attgaaggta tcgctaaaaa tgcagctgtt gttatcaccg atgcgggtgg 360

taaacaaatc aaattaggca ccccaagtgc tgctcaaaac 400

<210> 757
<211> 500
<212> DNA
<213> Proteus mirabilis

<400> 757
tggcaccact attgctatgc cttgttgcca gtttagtgac tgcgccaacg atagccagtg 60

atgtaaaaca agataaaaac atgcatcagc gatttgggtg gctcaatcta caaggaacca 120

tattagagcc gtcatgtgca atatcagcgg gaagtagtga tcaagtgatc ccgctaacga 180

cggtatctat cccaacgtta gtcactgaag gtcaaggacc gattgaatat ttttctatca 240

gattaacgga ctgtacgcta attagccaga aagggcaaga agcggataat ccacgtttta 300

tcgcaacgtt cgatggtcct tctaatggaa atggcaactt tgagttatcc ggtgaggcca 360

aaggtgcttc attagcgata gcggatcgtt atggtcgaca agctattcca ggacaacccc 420

taccgcccgt tggcattgat tcgcagtcaa tggcattgct gtaccaagct cgaatagtca 480

aaaataacga tacgccaaaa 500

<210> 758
<211> 546
<212> DNA
<213> Proteus mirabilis

<400> 758
gatggtaatg ctgataataa caaagaactt tataccatta tgtttagtaa gcaatttcct 60

gactggggat tgagtactta cttaaactat agtcaccaaa catattggaa taagccaact 120

aatgataatt acaacttatc gttagcgaaa agcgcggata ttggtcgttt taaaaatatc 180

aattttagtc tctccgcttt ccgtaataaa tttaatggca ccaatgataa tggcgtttat 240

atgaatgtca gtatgccttg gggtgatcgt gcgaccatca gttacaacac tgtcattaat 300

aagagcggta actctcataa tgtcagctat tacgatcgaa ttgatgacaa tagcagttat 360

cgtgttggcg ctggggtaag tagcaatggt aaaccttcag ccgatgctta ttttatgcat 420

tatgctgatg cggccttagt caccgccagt gcaagtcata tcaatggtga atatacctct 480

gccactttat cgctacaagg tggtgccaca cttacgccga aaggtggagc attacaccgc 540

gttagt 546

<210> 759
<211> 320
<212> DNA
<213> Proteus mirabilis

<400> 759
caatctatgc ctcgcaaacg gatttaacca atccttggca agagcagatc actttaacta 60

aaaaaggtga tcgcttcgaa gtgaataatc caacgcctta ctatgtgaca ttagtcgatg 120

gattaaccag tttgaaagga aaaagcttgg atggctttga accattaatg atcgcaccta 180

aaagtagcgg cacgataaat ctgagtgctt ccatgtttgg tgcttcaccg gtattgagct 240

acatcaatga ttatggcggt cgccctcaga tgaaattcac ctgtagtggc aatcaatgca 300

aagtaacaga aacggcagct 320

<210>  760
<211>  507
<212>  DNA
<213>  Proteus mirabilis

<400>  760
aacagtggtt tagcccaagc aaggcagtgt tggtttttat tctggcaaca tttactggag      60

gcctgagttc tactgctgtt gctaacttac ctgcaggagc agtaataaga gcgacccccg     120

ggattgttta tatcaatatt actggaaacg tcatcgctcc acctccttgc ttaatcaatg     180

acggcaagat gatcgaggtg aattttggcg aagtaatgag tacgcgtatt aatgatagca     240

attataagca acctatcgaa tataccgcga cttgccaaaa aagaccgact aacgccatga     300

aagtctatat aacaggtaat gcaacagggt tcgatagtaa tgccctacaa actaatatta     360

cgggattagg ggtacgcatt ctttatcaag gtaaattatt aggattaggc tcagcggtta     420

aatttaccta tcccaatttg cctaaactag aagcgatccc tgtgcgtgat aatagagaaa     480

cactagttgg tggagatttt gttgcca                                         507


<210>  761
<211>  451
<212>  DNA
<213>  Proteus mirabilis

<400>  761
ttactaaatt ctacagcagt aatggcggcc gactcgccta atttaaaatt attcggaaca      60

ttattagtgc cacctccttg tgttatcagc aatgacgaac gaattgaggt tttttttggt     120

aagaacgtcg gtattaataa agttgatggt attaactata ccgaatcggt gaattatacc     180

ttggtatgcg acgctaattt aaaaggttgg gatttgggat tatcaattat cggacctaaa     240

acccagtttg atgaggcggc attgcaaacc aatattccag atttaggtat tcatttaact     300

caagatggta agccttttaa gctaaatgag cgtattggga tttcaccaga ttcgcctccg     360

gttattcaag ctgttccagt aaaaagaccg ggaagtacat tgcctgaagg ggcatttgaa     420

gtctcagcca ccttattagc agaataccaa t                                    451


<210>  762
<211>  526
<212>  DNA
<213>  Proteus mirabilis

<400>  762
ccggttcata ttgggtctat ctgtatccat agggttaact tcagcggctt ttgcaatacc      60

ggacaacctc tattttcacg gcatattagt tgatgagcct tgtaccataa aaccgggtga     120

tgaaaccgtg gtactcgatt ttggcaatat tcctgataaa aacctttatg cctataaaag     180

aacgccaagc aagttatttc aattacgtct gtcagaatgc gatctctcaa tcggtaaaag     240

cgtcaaaata acctttaaag gagaggaaaa ccaagcaatg gcaggagaag gatttttggc      300

aataagtccg ggcagccaag cttctggtat tgcggtggga ttagagtctg aaaatggtaa 360

tgctctacct ataaataaag aaacagacaa gatgtcatta actgcgggtg acactatttt 420

gaatttttat gcctttattc aaggtgagcc ggatgcgatt gcgaataagt cgattaaacg 480

tggtcctttt agtgcaatag ccaccttcta tttgaattat gactga 526

<210> 763
<211> 505
<212> DNA
<213> Proteus mirabilis

<400> 763
ccttctaacg ccacttacac ttatgttatt gagcgttggg atccagaaac ctcaggaata 60

ttaaatcctt gttatggttg gcctgtgtgt tatgtcacag tgaaccataa acatacagta 120

aatggtactg ggggaaatcc tgcatttcag attgctcgaa ttgaaaaact acgtacttta 180

gctgaagttc gtgatgtagt acttaaaaat agatcattcc ctattgaagg gcaaaccacc 240

cacagagggc cttcattaaa ctctaatcaa gagtgtgtgg gattatttta tcaaccgaat 300

tcaagtggta tatcacctcg aggaaaactc ttaccaggtt cactatgcgg tatcgcacca 360

ccaccagtgg gtgcttgtaa aatatctgaa ggtgcggtga accttaacta tggtgatatt 420

gatgaagcta gtttaagtgg tgctaagcgc tctgaaacaa tcaatgtaac ctgtaattta 480

gcaatgaaag tgttggttat cgcat 505

<210> 764
<211> 408
<212> DNA
<213> Proteus mirabilis

<400> 764
aacatatgag ggtgtggact aatagaaaca ttggcttttt tacctaaacg gcgtaataac 60

ccataaactt gttgacggga aattggtccc gttttttgtg ataaaaatac ccattcagaa 120

tctgattctc tccagttttc tctacttttc aaccagttgc ataaggcttc atattcctca 180

tcaataatag gttgtgttgt tgaaagccca ccttttaaac gcctgacata gagtattcta 240

ctttctagat caatatcgct taatgttaaa ttacatagtt cgctaacacg aaaaccatgt 300

aaaaaacaca ttaaaaacat acagtaatct ctttcgggat acctaccttc cttagcttgc 360

tttaaaatag cattcacttc aaaacgtgta agaaatttac gttgcttc 408

<210> 765
<211> 310
<212> DNA
<213> Proteus mirabilis

<400> 765
ttgattttgc gaatatagat gtaaatgctt ctgtaggtaa aaagatccaa aaaaaacgta 60

aagagctggg ttataccggt atgcagctgg ctaaaaaaat tggtgtcagc cagcaacagt 120

tttctcgcta tgaacgaggt atgaacaaaa tagatctcag acatttagtg ttgttagctc 180

tctatttaaa tacacccatt tattggtttt ttgaggattg ctacgtaaaa aagccttcat 240

taaataataa aggaatagat aagcgcaatt atgttattgc tcaagcaaca cctgatgctt 300

ttcattattg 310

<210> 766
<211> 510
<212> DNA
<213> Proteus mirabilis

<400> 766
tggagtatca gagctatttt gtttaagcct ttttttggaa atattggact ttattcctat 60

cttggaaaac ctatattctt attgggtatc aaaaaagtat ttttaggacg tagagttaga 120

attttccctc actctagaat tgaagtgcat ggaaataaat tgtatgagga taatatctct 180

ataggacagt catttcatat aatatgttca agtaatatta ttatatctga aggtacatta 240

atctctgcta atgtatttat tactgatact gatcatacat ataaaaatat ttctctaccc 300

attcatgttc aaaaaactaa tatttctacc acttatattg gtaaaaattg ttttatagga 360

tatggcgttg ttattcaagc tggaacaaaa ttaggaaata attgtatcgt tggtgcaaac 420

tcaacaataa aaggctcttt tttcgataat tcaataattg taggttcacc tggacggatt 480

attaaaaaac tagataaatt atggctgact 510

<210> 767
<211> 934
<212> DNA
<213> Proteus mirabilis

<400> 767
ctatcagcca cctcttcttg aatacagaga atacgcaaag cagaacaacg ttgacccgca 60

ctatcataag cagaggccat aacgtcagtg accacttgtt ccgttaaggc agaagagtcg 120

acaatcatgg cgtttaagcc acctgtttca gcaattaaag gaacagggcg accttcgcta 180

tcgagtctac ccgctaaggt tttttgcaaa atgtgggcaa cttcggtaga gcctgtaaac 240

atcacaccac gcacgcgttc atctgccact aattgtgcac caatggtctc cccttgaccc 300

ggtaagagtt gtaatgcact gcgaggtact cctgcttgat aaaatagttc tacggcttta 360

aaagcaatca gaggggtttg ctccgcaggt ttggccagta cggtattacc tgccgctaac 420

gccgctgcaa tttgtccact aaagatggct aatgggaagt tccaaggact aatacagaca 480

acagggccta aaggacgatg ggtattatta tcaaaatcat ttgccacttg tgctgagtaa 540

taataaagaa aatcaattgc ctcacgcact tctgcaatag cattactata ggttttgccc 600

gcttctctta ctaagacccc cattaatggg cccatttgct gttccatcag ctcagccgtg 660

cggactaaaa atgccgctct ttccgcaggt ggggttgcaa accaaatttc accattttct 720

tgggcaatat ctaaagcaaa attagcttcg gcggccgtcg tttcacgtac tgtaccgacg 780

acatcggtgt gatttgccgg attgagtata gattgtggtg caatcacttc ttctgcacta 840

ttacactcac caccaatgag cggatggctg tgaatttttt ccatcgcaga ggtcagtaat 900

gcactggata atgaggctaa acgatgctca ttag 934

<210> 768
<211> 501
<212> DNA
<213> Proteus mirabilis

<400> 768
gcactagcta ctattctttc tgctgcattt gctggctcat ctatggcgta tgacggaaca 60

attacattta caggtaaagt tgttgcgcaa acctgctctg tcaatacaaa tgataagaat 120

ttagcggtaa cattacctac agtatccacc actacattaa atgaaaatgc ggctactgca 180

ggtcttactc catttactat tcatttaact ggttgcgctg ttggtatgga tggtgcacaa 240

agtgtcaaaa catattttga accttcaagt gacattgatg taaccacaca caacttaaaa 300

aatactgcac aaactaaagc tgataatgtt caagttcaat tacttaactc agatgcagca 360

acaacaatcc agttaggtac tgattctgca acacaagatg tccatccagt acaaatcgac 420

aatgctaatg taaacctccc atattttgct caatattatg caaccggaca atctaccgct 480

ggggatgtaa aagcaaccgt t 501

<210> 769
<211> 383
<212> DNA
<213> Proteus mirabilis

<400> 769
gaggtactgc atcgcaaacg cagacattga cggttgcaca agagggcttt ttagagtggt 60

taccccaaga gaatatcttt tttcctgatg ctcaagtgtg tttaaccaca catattcatt 120

tagcctcatc agcgaaattt atcggctggg aaatgcagtg ttttggacgc ccagttttaa 180

atgagtggtt tgaaactggc aaggtaaaag ggcgcttaaa tttttatgtt gatgagagat 240

taattttaac agagtcaatg cgggttgaag gcttacaaaa acaagctgcc gcaatgcgtg 300

aatttcctat gtttggctcg ctttatattt atcctgcaac cgatgcatta aaagagatta 360

ttcaacacca tttagagaag gta 383

<210> 770
<211> 414
<212> DNA
<213> Proteus mirabilis

<400>  770
gcgcttgaac taacctctac agaaaagcca aagttaaccc tttgtcttac catggatgag      60

cgcaccaaaa gtcgcttaaa agtggcttta agtgacgggc aagaagccgg gctatttttg     120

cctcgaggca ccgtacttaa agagggggat attctgctgt cagaagaggg cgatgttgtc     180

accattgaag cggctaaaga gcaagtatca acggtttata gtgacgatcc attattgctt     240

gctcgtgttt gttatcactt aggtaaccga catgtaccat tgcaaataga agcgggttgg     300

tgtcgttatt ttcacgatca tgtattagat gatatggctc gcggcttagg ggctacggtg     360

gtggttggct tagaaaaata ccaacctgag ccgggggctt atggtgggtc atcc           414


<210>  771
<211>  500
<212>  DNA
<213>  Proteus mirabilis

<400>  771
gctcagcaga aaccttgtca gattggttaa gcgcacaaat gaccggaaca ttagccacac      60

tcgagcttcc tatattgcgg caattacaaa cgagtttggc aaagggtgat agcgatacag     120

tgaaatattg gtgtgacttt atggtcgcaa gtcgcgaaac caaagagtta aggcaggaag     180

agcgtcaacc ggggatcgct tttccccgtt tacttcctca attaggcatt gaattagacg     240

atacgttaca acagcgggtt aaacagacgc aattaatggc gtttgcgtta gctgccgtgc     300

attggcatat cgatagtgaa aagctctgtt gtgcctatgt ttggggctgg ttagaaaata     360

cggtgatgtc tggggtaaaa ctggtgccat tagggcaaag cgcagggcaa aaaatgttgt     420

ttgctctagc tgagcagatc cccgctattg ttgagttatc ggcacattgg ccacaagagg     480

atattggcag tttacgccag                                                 500


<210>  772
<211>  560
<212>  DNA
<213>  Proteus mirabilis

<400>  772
gggatcttct ataactattc aaccaaagta ttaccttctt ttgattatga taccgcagga      60

aaacatatag cccgtgaaga ttccacttgg aatggcaaat atgttattgg gcaacccgct     120

gaggtgactt attcattccc aaaatgggaa ggcaaattta atcaatttgg taataagaat     180

ccttatgaat ttaatgaatt acaaaaagag catgcaagaa aatctttaga tgcatggtct     240

gatattgcaa atattaaatt tactgaagtt gctgttggga atgttgatgg aatgaaggct     300

tctgacgtaa aaacagatat tacttttggt aatatctatg atcccaatgg cacatttcag     360

gcttatgcaa cattgcctaa tacctatgct tatggaaaag atctttctgg ccaagcatgg     420

tttagtgatt atcattatgc aggtaatact acaccagaat tgggtaatta tggtcgttta     480

actattatcc atgaaattgg tcatacactg ggtcttatgc atcctggtga ttataacgca 540

ggtcagaatg ttccaggtta 560

<210> 773
<211> 509
<212> DNA
<213> Proteus mirabilis

<400> 773
tttctttgat ctaccttggt tccctattta ccttttagtt attactttat ttaatccttg 60

gttaggatta tttgcacttt gtggtgccct tatcttattt gctttggcta tccttaatga 120

atatctatct aaaaatcatt taaaaaaagc gaatagtttt gccaatcaag cacaattaat 180

acaaagtcat catttagaac atccacagac tatcgaagcg atgggaatgc ttagtcaatt 240

acgtaaacaa tggcaaacct ctcatttcaa atacttacaa gcacaaacac aagccagtga 300

taatgcagcc ggtatcaacg ctatcacaaa agtaacacgt atggcattac aatctttaat 360

gctaggttta gggggatggc ttgctattga taatactatt agtcctggaa tgatgattgc 420

aggttcaata cttttaggtc gagcattagc ccctattgag caagtgatca atgtatggaa 480

aagttgggat agtagtaaag cagcctaca 509

<210> 774
<211> 576
<212> DNA
<213> Proteus mirabilis

<400> 774
aagaacaagt agcaggtaaa gagtatgaaa atatcgggtt atcacaattg ctacccaata 60

tttctgtcaa ttacaaaaat aatcctcgca actggcaacg taaggcttat ccaataaata 120

tatttcagga taaaataaca acagttgagt atcaaaacta tcaaagctat tctgtcaacg 180

cgattattag tcaaccacta tttgactaca ccgcatttag tgaatacaaa gcttctatca 240

ttaaaacatt attagcagac agtcattatc aaaataaatt ttcagaatta attattcgac 300

ttatcgataa ttatattcaa gttgcttata cacaagataa attattacta aatcaagcac 360

agcaagaaat ctatcaaaaa caactagctt caagtcaacg cctatttgag ttaggagaag 420

gaaccaaaac agatattgct gaaatagaga ctcgtttata tttaacccag tcacaatata 480

ccgatcttca attagaaatt gaaaaggcta aaaacaaact cagtgctatg atcggttcac 540

aattgcctac tcatgagcac atcgcaaagc taactg 576

<210> 775
<211> 626
<212> DNA
<213> Proteus mirabilis

<400>  775
ccaacttact tctatctacc tgatggtaaa attgttataa attatatgta tggttggcct      60

aaacaaccac atagtaatat caccaaaata aattatatat tccctgatta tgataaaaaa     120

agaaattact caaataaaaa atattcagta acagaaaaag atagaataga atcaataaaa     180

cataccgcta aagtatatga attgacctat cttaaggaaa aaaaagaaaa agaaatcgct     240

tcattaaaat attatagaaa taaatattca ataagtagaa tagctgaatt agaaaaagat     300

atagaggata tagaaaatag tattattttt cacaagaata gtatacatcc gtatttttac     360

aatacacaaa caaccatata tcctcatcaa caagaagtta tttccgatat tcttagtgaa     420

attgcccata taacacaagc aaagtttgtt gcatctaatc cagaattcga tgccgatata     480

aaatttggtt tttacgatga ttttcatatt agtcatggtt caatagaatt ttcatatacc     540

accagaggtt ttgcaaccta ccctagcaga tattcaaccc ctataaaaaa gataaatatt     600

gatgaacaat accaatactc tggaac                                          626


<210>  776
<211>  583
<212>  DNA
<213>  Proteus vulgaris

<400>  776
catcttattg tgggtccaag cctacagcaa tttaataaag tattagctta tgagatacga      60

actttcatcc ccgaggagct cattttagtt gatggcactc cgcttaaaat ttccccagct     120

ctgcgtaata aaatctacaa tgaattaggt atttcctttt ttgataaaaa aacagcatta     180

aaagaagggc ttcattgggc gaaagaagat gatgagctta gccaacagat gtctgaatac     240

cttaatggtg aaaccgtaat ttggattgag agcacactgg aatatcctgt tttatggatt     300

aacacctata tttcaccttc tttatggatc cgggttccac tcactgaatt aggcgaaaat     360

ttcttactgc cagtttatcg ccaagcaatt atttttatta ttattgttat tgcctttttc     420

tggttatata accgttttca aaatcgccca ttaaacgaag tggaatatgc agctcgtcgt     480

attggtaaag gcgttattcc tccccctatc ccagaatcag gttcatcaga gatgcgttcg     540

atcattcgag catttaatca aatgtcatca ggtattcgct ctt                       583


<210>  777
<211>  383
<212>  DNA
<213>  Proteus vulgaris

<400>  777
cgtaagcctt atgttcgtgg tatgcagcca aactggtgga cgaaactcgg tttctatcgt      60

ttctacatca cccgtgaagg aacttgtcta ccacaacttt ggttcagtct ggttgtactg     120

ttcggtgtat ttgcactgaa aaatggacca gaaagttggg cgggattcgt tggattccta     180

agtaacccaa tactgatgct gattaatatt gtgaccctta tcgcaacggt attccatacg 240

gccacttggt ttaagcttgc accgaaagcc gttaatatcg tcgttaaaga tgaaaaatta 300

ccacaagagc ctatcgttcg tggtttatgg ggtctaacca tcgtcgtgac tgtcgttatt 360

ctggcagtgg cgctaattgt tta 383

<210> 778
<211> 345
<212> DNA
<213> Proteus vulgaris

<400> 778
aatcagaatc aacttcctaa gcgctctgat gaacctattt tctggggatt atttggtgca 60

ggtggtatgt ggagtgcgat tgtctctcca gcaattatta tcctgctcgg tattctaatc 120

ccgatgggta ttgcgccaga agcatttact tacgatcgta tcatggcatt tagccaaggt 180

tttattggtc gtattttctt actgctaatg attattctgc cagtttggtg tgcattacac 240

cgtattcacc atacgttgca ccattttaaa gtgcatgtac ctgctagtaa ttgggtattt 300

tatggtgctg cagcaattat tagcgttatc gcaattattg gtgtt 345

<210> 779
<211> 534
<212> DNA
<213> Proteus vulgaris

<400> 779
gcgaagtaga agagaaagca cagcgcgaag cacaagaaaa agcacagcgc gcagctgaag 60

aaaaagcaaa acgtgaagca caagaggcca agaaacaggc cgaagaaaaa gcgaaacgtg 120

aagctgaaga agcaaaacgt gaagcagcgg aattagctaa gcgcgaagca gcggaaaaaa 180

ataaagtgaa acaaaacgat aaaccaaaag ctgatgtagc agatcaggat aaagcacgtc 240

gcaatgctga actggctgaa ctgaaacgta aaacagaaga agcacagcgc cttaaagttg 300

aagaagagac gcgcgctgca gcagaaaaag cacgccgctt agctgaagaa aacgctgaaa 360

aatggactgc tgaacctaag gctcctgaaa cagaaagcgc ggactatcat gtaactacat 420

ctcgttatgc tcgtgatgca gaagatgaaa gcgatgcaga agtagaaggt gatcgccgcc 480

gcggtcgtac tgctaaagca cctcgtgcta agaaaaataa ccgccactct gaaa 534

<210> 780
<211> 582
<212> DNA
<213> Proteus vulgaris

<400> 780
agctgatgtt gttgttgttg gtgctggtat ccttggtatt atgacagcaa ttaaccttgt 60

agaacgtggt ttatctgttg taattgttga gaaaggtaat atcgcgggtg agcaatcttc 120

gagattctat ggtcaggcaa ttagctataa aatgccagat gaaacgttct tattacacca 180

tttgggcaaa catcgctggc gtgaaatgaa tgcgaaagta ggtattgata ctacttatcg 240

tacacaaggc cgcgttgaag ttcctcttga tgaagaagat ttagttaacg taagaaaatg 300

gattgatgaa agaagtaaaa atgttggctc agatattcca tttaaaacca gaattattga 360

aggtgctgaa ttaaatcaac gtcttcgtgg cgcgacaaca gattggaaaa ttgctggctt 420

tgaagaagat tctggtagct tcgatccaga agttgcaacc ttcgttatgg ctgaatacgc 480

taaaaaaatg ggtgttagaa tttacactca atgcgcggct cgtggcttag aaacacaagc 540

tggtgtaatt tctgacgttg taacagagaa aggtgcaatc aa 582

<210> 781
<211> 553
<212> DNA
<213> Proteus vulgaris

<400> 781
ctaaatatgg cgcaggaaca aattactttg atatatccaa agagttatta ccgaagtggg 60

cttgttatat tgccaatgct tcattgatct ttgtattata tatattgatc tatgcttata 120

tctctgcggc gggttctatt atctatgaag catcactgtt atatggtatt aattttaatc 180

tgagagctat attttttatt tttacgatag cccttggtgc tacaatatgg tggggtggcg 240

cttgtgctag ccgtttaacc tcaattttct tattcattaa gatagtatta tttatattag 300

cgttttcggg tttgtttttt aaagcaaaag gtgatttatt atttagtgca acttttgcag 360

gaaaaagcca attatatctt tatcctttta tttttattat cattccttat gccattacct 420

catttggata tcatggtaat gtttgtagtc tttataagct ttataatcaa aacgaaagaa 480

aagtagttaa gagttgtatc attggttgct tgttagcatt agtcatctat ttactttgga 540

tgattggcac tat 553

<210> 782
<211> 260
<212> DNA
<213> Proteus vulgaris

<400> 782
gttcataggc ttcacgtagt tcagcacagt ttttaacaga gtttaaatgg ctaacagggg 60

accaaacacg agaaagctta tcgcctgctt ctgctaatgg ttggcaaagg ttatcctgag 120

taaattgagt attatcagcc agtaattttt caactgtttc acgatatgtg gttaaaactt 180

cgtttagtgc aggaacgaca tgttctggtt tgataaggga aaatgcaggt aatcccgttg 240

tgctaagtaa tggatttgac 260

<210> 783
<211> 199

<212>  DNA
<213>  Proteus vulgaris

<400>  783
tggctgaaaa tgtcgttaat gatattctaa aatggttaga aacccagtta caacgtaacg      60

aaggtataaa aatcgatact attgcgaaca aaagtggtta ttcaaaatgg cacttacaac     120

gcatatttaa agattttaaa ggctgcacat taggcgaata tgtccgcaaa cgtcgcttat     180

tagaagcggc taaatcatt                                                  199


<210>  784
<211>  220
<212>  DNA
<213>  Proteus vulgaris

<400>  784
gaaaggactt aaacttaact atccagagtc tgtcgcatta attagttgcg cgattatgga      60

aggtgcaaga gaaggtaaaa cagtggctca attaatgagt gaagggcgtg ctgtattaac     120

agcagaacaa gttatggaag gcattcctga gatgatcaaa gacattcagg tggaatgcac     180

attccctgat ggtacaaaac ttgtttctat tcacgaccct                           220


<210>  785
<211>  503
<212>  DNA
<213>  Pseudomonas aeruginosa

<400>  785
actgacgctt atttgattga cactccattt acagctaaag atactgaaaa gttagttact      60

tggtttgtag agcgcggcta taaaataaaa ggcagtatct cctctcattt tcatagcgac     120

agcacgggcg gaatagagtg gcttaattct caatctattc caacatatgc atctgaatta     180

acaaatgaac ttcttaaaaa agacggtaag gtacaagcta aaaattcatt tagcggagcc     240

agctattggt tagttaagaa aaagattgaa attttttatc ctggcccagg gcacactcca      300

gataacgtag tggtttggct acctgaacat agagttttgt ttggtggttg ttttgttaaa     360

ccgtatggtc taggtaattt gggtgacgca aatttagaag cttggccaaa gtctgccaaa     420

ttattagtgt ccaaatatgg taaggcaaaa ctggttgttc caagtcacag tgaagttgga     480

gatgcatcac tcttgaaacg tac                                             503


<210>  786
<211>  348
<212>  DNA
<213>  Staphylococcus epidermidis

<400>  786
atggataata aaacgtatga aatatcatct gcagaatggg aagttatgaa tatcatttgg      60

atgaaaaaat atgcaagtgc gaataatata atagaagaaa tacaaatgca aaaggactgg     120

agtccaaaaa ccattcgtac acttataacg agattgtata aaaagggatt tatagatcgt 180

aaaaaagaca ataaaatttt tcaatattac tctcttgtag aagaaagtga tataaaatat 240

aaaacatcta aaaactttat caataaagta tacaaaggcg gtttcaattc acttgtctta 300

aactttgtag aaaaagaaga tctatcacaa gatgaaatag aagaattg 348

<210> 787
<211> 530
<212> DNA
<213> Pseudomonas aeruginosa

<400> 787
tagctcgtgc atcaaaggaa tatcttccag catcaacatt taagatcccc aacgcaatta 60

tcggcctaga aactggtgtc ataaagaatg agcatcaggt tttcaaatgg gacggaaagc 120

caagagccat gaagcaatgg gaaagagact tgaccttaag aggggcaata caagtttcag 180

ctgttcccgt atttcaacaa atcgccagag aagttggcga agtaagaatg cagaaatacc 240

ttaaaaaatt ttcctatggc aaccagaata tcagtggtgg cattgacaaa ttctggttgg 300

aaggccagct tagaatttcc gcagttaatc aagtggagtt tctagagtct ctatatttaa 360

ataaattgtc agcatctaaa gaaaaccagc taatagtaaa agaggctttg gtaacggagg 420

cggcacctga atatctagtg cattcaaaaa ctggtttttc tggtgtggga actgagtcaa 480

atcctggtgt cgcatggtgg gttgggtggg ttgagaagga gacagaggtt 530

<210> 788
<211> 322
<212> DNA
<213> Proteus vulgaris

<400> 788
acactggctg aattaagtgc tgctacattg caatatagcg ataatacagc aatgaataag 60

atattagatt atttaggcgg tccagccaaa gtcactcaat ttgcacgttc aattaatgat 120

gtcacttatc gccttgatcg taaagagcct gaattaaata cagcaattca tggtgatcct 180

cgtgatacta cttctccaat tgcgatggct aaaagtcttc aagcactgac attaggcgat 240

gcactaggtc aatctcagcg tcaacaactt gtgacttggt taaaaggtaa tacaacgggt 300

gataacagta ttaaagcggg tt 322

<210> 789
<211> 625
<212> DNA
<213> Klebsiella oxytoca

<400> 789
ttatctgcaa cactgatttc cgctctgctg gcgttttccg ccccggggtt ttctgccgct 60

gataatgtcg cggcggtggt ggacagcacc attaaaccgc tgatggcaca gcaggatatt 120

cccgggatgg cggttgccgt ctccgtaaag ggtaagccct attatttcaa ttatggtttt 180

gccgatattc aggcaaaaca gccggtcact gaaaatacac tatttgagct cggatctgta 240

agtaaaactt tcacaggtgt gctgggtgcg gtttctgtgg cgaaaaaaga gatggcgctg 300

aatgatccgg cggcaaaata ccagccggag ctggctctgc cgcagtggaa ggggatcaca 360

ttgctggatc tggctaccta taccgcaggc ggactgccgt tacaggtgcc ggatgcggta 420

aaaagccgtg cggatctgct gaatttctat cagcagtggc agccgtcccg gaaaccgggc 480

gatatgcgtc tgtatgcaaa cagcagtatc ggcctgtttg gtgctctgac cgcaaacgcg 540

gcggggatgc cgtatgagca gttgctgact gcacggatcc tggcaccgct ggggttatct 600

cacaccttta ttactgtgcc ggaaa 625

<210> 790
<211> 482
<212> DNA
<213> Staphylococcus aureus

<400> 790
gaaaattcac gtatgtcatg gaatcataag cattaccctt ttgatgcttg gaataaggaa 60

caagatttaa atacagcaat gcaaaattca gttaattggt acttcgaacg tattagcgat 120

caaataccaa agaactatac tgcgactcaa ctcaagcaat taaattatgg taataaaaat 180

ttgggaagtt ataaaagcta ttggatggaa gatagtttga aaatatctaa tcttgaacaa 240

gtaatagttt ttaaaaatat gatggaacaa aataaccatt ttagtaaaaa agcaaagaat 300

caattatctt cttcattatt gattaagaaa aatgaaaagt atgaactgta tgggaaaaca 360

ggtacaggta tagtaaacgg gaagtataat aatgggtggt ttgtaggtta cgtaattaca 420

aatcatgata agtattattt tgctacacat ttatcagatg gaaagccatc tgggaaaaat 480

gc 482

<210> 791
<211> 703
<212> DNA
<213> Pseudomonas aeruginosa

<400> 791
acgttctgac tggaggaagt ttttcagcga atttcaagcc aaaggcacga tagttgtggc 60

agacgaacgc caagcggatc gtgccatgtt ggtttttgat cctgtgcgat cgaagaaacg 120

ctactcgcct gcatcgacat tcaagatacc tcatacactt tttgcacttg atgcaggcgc 180

tgttcgtgat gagttccaga tttttcgatg ggacggcgtt aacaggggct ttgcaggcca 240

caatcaagac caagatttgc gatcagcaat gcggaattct actgtttggg tgtatgagct 300

atttgcaaag gaaattggtg atgacaaagc tcggcgctat ttgaagaaaa tcgactatgg 360

caacgccgat ccttcgacaa gtaatggcga ttactggata gaaggcagca ttgcaatctc 420

ggcgcaggag caaattgcat ttctcaggaa gctctatcgt aacgagctgc cctttcgggt 480

agaacatcag cgcttggtca aggatctcat gattgtggaa gccggtcgca actggatact 540

gcgtgcaaag acgggctggg aaggccgtat gggttggtgg gtaggatggg ttgagtggcc 600

gactggctcc gtattcttcg cactgaatat tgatacgcca aacagaatgg atgatctttt 660

caagagggag gcaatcgtgc gggcaatcct tcgctctatt gaa 703

<210> 792
<211> 758
<212> DNA
<213> Klebsiella pneumoniae

<400> 792
tcacgctgtt gttaggaagt gtgccgctgt atgcgcaaac ggcggacgta cagcaaaaac 60

ttgccgaatt agagcggcag tcgggaggca gactgggtgt ggcattgatt aacacagcag 120

ataattcgca aatactttat cgtgctgatg agcgctttgc gatgtgcagc accagtaaag 180

tgatggccgc ggccgcggtg ctgaagaaaa gtgaaagcga accgaatctg ttaaatcagc 240

gagttgagat caaaaaatct gaccttgtta actataatcc gattgcggaa aagcacgtca 300

atgggacgat gtcactggct gagcttagcg cggccgcgct acagtacagc gataacgtgg 360

cgatgaataa gctgattgct cacgttggcg gcccggctag cgtcaccgcg ttcgcccgac 420

agctgggaga cgaaacgttc cgtctcgacc gtaccgagcc gacgttaaac accgccattc 480

cgggcgatcc gcgtgatacc acttcacctc gggcaatggc gcaaactctg cggaatctga 540

cgctgggtaa agcattgggc gacagccaac gggcgcagct ggtgacatgg atgaaaggca 600

ataccaccgg tgcagcgagc attcaggctg gactgcctgc ttcctgggtt gtgggggata 660

aaaccggcag cggtgactat ggcaccacca acgatatcgc ggtgatctgg ccaaaagatc 720

gtgcgccgct gattctggtc acttacttca cccagcct 758

<210> 793
<211> 680
<212> DNA
<213> Streptococcus pneumoniae

<400> 793
cggaactgta taatcccttg aattccgtag aagattctac taatcggcgc gatactgtct 60

tgcagaatat ggttgcagca ggatatattg ataaaaacca agaaaccgaa gctgctgaag 120

ttgatatgac ttcgcaattg cacgataagt atgaaggaaa aatctcagat taccgttacc 180

cctcttattt tgatgcggtg gttaatgaag ctgtttccaa gtataatcta acagaggaag 240

agattgtcaa taatggctac cgcatttaca cagagctgga ccaaaactac caagcaaata 300

tgcagattgt ttatgaaaac acatcgctat ttccgagggc agaggatgga acgtttgctc 360

aatcaggaag tgtagctctc gaaccgaaaa cagggggagt tcgtggagtt gtcggtcaag 420

ttgctgacaa tgataaaact ggattccgga atttcaacta tgcaacccaa tcaaagcgta 480

gtcctggttc tacaattaag cctttagttg tttatacacc agcagttgaa gcaggctggg 540

ctttgaataa gcagttggat aaccatacca tgcagtatga tagctataag gttgataact 600

atgcagggat caaaacaagt cgagaagttc ctatgtatca atccttggca gaatcgctta 660

atctacctgc tgttgccact 680

<210> 794
<211> 669
<212> DNA
<213> Klebsiella pneumoniae

<400> 794
cgtaggcatg atagaaatgg atctggccag cggccgcacg ctgaccgcct ggcgcgccga 60

tgaacgcttt cccatgatga gcacctttaa agtagtgctc tgcggcgcag tgctggcgcg 120

ggtggatgcc ggtgacgaac agctggagcg aaagatccac tatcgccagc aggatctggt 180

ggactactcg ccggtcagcg aaaaacacct tgccgacggc atgacggtcg gcgaactctg 240

cgccgccgcc attaccatga gcgataacag cgccgccaat ctgctactgg ccaccgtcgg 300

cggccccgca ggattgactg cctttttgcg ccagatcggc gacaacgtca cccgccttga 360

ccgctgggaa acggaactga atgaggcgct tcccggcgac gcccgcgaca ccactacccc 420

ggccagcatg gccgcgaccc tgcgcaagct gctgaccagc cagcgtctga gcgcccgttc 480

gcaacggcag ctgctgcagt ggatggtgga cgatcgggtc gccggaccgt tgatccgctc 540

cgtgctgccg gcgggctggt ttatcgccga taagaccgga gctggcgagc ggggtgcgcg 600

cgggattgtc gccctgcttg gcccgaataa caaagcagag cgcattgtgg tgatttatct 660

gcgggatac 669

<210> 795
<211> 551
<212> DNA
<213> Salmonella typhimurium

<400> 795
cacgatagtt gtggcagacg aacgccaagc ggatcgtgcc atgttggttt ttgatcctgt 60

gcgatcgaag aaacgctact cgcctgcatc gacattcaag atacctcata cactttttgc 120

acttgatgca ggcgctgttc gtgatgagtt ccagattttt cgatgggacg gcgttaacag 180

gggctttgca ggccacaatc aagaccaaga tttgcgatca gcaatgcgga attctactgt 240

ttgggtgtat gagctatttg caaaggaaat tggtgatgac aaagctcggc gctatttgaa 300

gaaaatcgac tatggcaacg ccgatccttc gacaagtaat ggcgattact ggatagaagg 360

cagccttgca atctcggcgc aggagcaaat tgcatttctc aggaagctct atcgtaacga 420

gctgcccttt cgggtagaac atcagcgctt ggtcaaggat ctcatgattg tggaagccgg    480

tcgcaactgg atactgcgtg caaagacggg ctgggaaggc cgtatgggtt ggtgggtagg    540

atgggttgag t                                                          551


<210>  796
<211>  557
<212>  DNA
<213>  Staphylococcus haemolyticus

<400>  796
agcttttgtt ttatatttct attggtatta ttttttaggt acatattaaa acgctatttt     60

aattatatgt taaattataa agtttggtat ctaactcttc ttgcaggatt aattcctttc    120

attcctatta aattctctct ttttaaattt aataatgtga ataatcaagc gcccacagtt    180

gaaagtaagt cacacgactt gaaccataac ataaatacca ccaaacctat tcaagagttc    240

gcaacagata tccataagtt taattgggat tcaattgata atatctgcac agttatttgg    300

atagttttag ttattatttt aagttttaaa tttttgaaag ccttattata tcttaaatat    360

ttaaagaaac agtcacttta tctaaacgaa aatgaaaaaa ataaaataga tacgatactt    420

ttcaaccatc aatataaaaa aaatattgtg attcgaaaag cagagactat tcaatctcca    480

ataacttttt ggtatgggaa atatattatt ttgattccta gttcatattt taaaagtgta    540

attgacaaaa gactaaa                                                    557


<210>  797
<211>  558
<212>  DNA
<213>  Pseudomonas aeruginosa

<400>  797
ttgacgaagg cgtttatgtt catacttcgt ttgaggaagt taacggctgg ggcgtggttc     60

ctaaacacgg cttggtggtt cttgtaaata ctgacgctta tttgattgac actccattta    120

cagctaaaga tactgaaaag ttagttactt ggtttgtaga gcgcggctat aaaataaaag    180

gcagtatctc ctctcatttt catagcgaca gcacgggcgg aatagagtgg cttaattctc    240

aatctattcc aacatatgca tctgaattaa caaatgaact tcttaaaaaa gacggtaagg    300

tacaagctaa aaattcattt agcggagcca gctattggtt agttaagaaa aagattgaaa    360

ttttttatcc tggcccaggg cacactccag ataacgtagt ggtttggcta cctgaacata    420

gagttttgtt tggtggttgt tttgttaaac cgtatggtct aggtaatttg ggtgacgcaa    480

atttagaagc ttggccaaag tctgccaaat tattagtgtc caaatatggt aaggcaaaac    540

tggttgttcc aagtcaca                                                   558


<210>  798

<211>  421
<212>  DNA
<213>  Staphylococcus aureus

<400>  798
ttaaagaatg gaaccaagat caaaatttaa attcttcaat gaaatattca gtaaattggt      60

attacgaaaa tttaaacaaa catttaagac aagatgaggt taaatcttat ttagatctaa     120

ttgaatatgg taatgaagaa atatcaggga atgaaaatta ttggaatgaa tcttcattaa     180

aaatttctgc aatagaacag gttaatttgt tgaaaaatat gaaacaacat aacatgcatt     240

ttgataataa ggctattgaa aaagttgaaa atagtatgac tttgaaacaa aaagatactt     300

ataaatatgt aggtaaaact ggaacaggaa tcgtgaatca caaagaagca aatggatggt     360

tcgtaggtta tgttgaaacg aaagataata cgtattattt tgctacacat ttaaaaggcg     420

a                                                                     421


<210>  799
<211>  260
<212>  DNA
<213>  Klebsiella oxytoca

<400>  799
gacaataccg cgatgaataa gatgattagc taccttggcg gaccggaaaa ggtgaccgca      60

ttcgcccaga gtatcgggga tgtcactttt cgtctcgatc gtacggagcc ggcgctgaac     120

agcgcgattc ccggcgataa gcgcgatacc accaccccgt tggcgatggc cgaaagcctg     180

cgcaagctga cgctgggcaa tgcgctgggc gaacagcagc gcgcccagtt agtgacgtgg     240

ctaaaaggca ataccaccgg                                                 260


<210>  800
<211>  605
<212>  DNA
<213>  Streptococcus pyogenes

<400>  800
aatcatcctc gtggctttga tcagcattta aaactactgt aataaccctc atttgatttt      60

cgacactagt agctacaaaa gaagcaccgg cttttttaga ataaccaaca aaaagaccat     120

ccacgccttc tcgataacaa ggcatgcctt taagcatgta attataactg taaatggttt     180

gtccagcaaa aatagtggag gatttgctag ataatttcag tacttctgga aattctaata     240

agagatgcct ggcaataaca gctaaatcag tggcgcaaaa acaattttca tcatctggtt     300

ctgtattagg ataagtatta gctcctaaaa aatggttagt taagccagtt gaattgacga     360

cctttgcatc ggaaatgccc cattgtctta attgtttttt cattttgtca acaaatttgg     420

gttcggttcc gcctattttt tcagctaaag caatagcggg gctattggcg ttattaacaa     480

ctaacgcact taaaagttct ttaacggtat attttctctt atcaagagga acattactaa     540

tagtatagtt tgtagtgagt tcataagggt agttagaaat agttacagga ctatcccaat 600

ttagc 605

<210> 801
<211> 713
<212> DNA
<213> Staphylococcus aureus

<400> 801
tacagtcatt tcacgcaaac tgttggccac tatgagttaa agcttgctga aggttatgaa 60

acacatttag tgggaataaa gaacaataat aacgaggtca ttgcagcttg cttacttact 120

gctgtacctg ttatgaaagt gttcaagtat ttttattcaa atcgcggtcc agtgatcgat 180

tatgaaaatc aagaactcgt acactttttc tttaatgaat tatcaaaata tgttaaaaaa 240

catcgttgtc tatacctaca tatcgatcca tatttaccat atcaatactt gaatcatgat 300

ggcgagatta caggtaatgc tggtaatggt tggttctttg ataaaatgag taacttagga 360

tttgaacata ctggattcca taaaggattt gatcctgtgc tacaaattcg ttatcactca 420

gtgttagatt taaaagataa aacagcagat gacatcatta aaaatatgga tggacttaga 480

aaaagaaaca cgaaaaaagt taaaaagaat ggtgttaaag taagatattt atctgaagaa 540

gaactaccaa tttttagatc attcatggaa gatacgtcag aatcaaaagc ttttgctgat 600

cgtgatgaca agttttatta caatcgctta aaatattaca aagaccgtgt gttagtgcct 660

ttagcgtata tcaattttga tgaatatatt aaagaactaa atgaagagcg tga 713

<210> 802
<211> 715
<212> DNA
<213> Staphylococcus aureus

<400> 802
agttgtagtt gtcgggtttg gtatatattt ttatgcttcc aaagataaag aaattaataa 60

tactattgat gcaattgaag ataaaaattt caaacaagtt tataaagata gcagttatat 120

ttctaaaagc gataatggtg aagtagaaat gactgaacgt ccgataaaaa tatataatag 180

tttaggcgtt aaagatataa acattcagga tcgtaaaata aaaaaagtat ctaaaaataa 240

aaaacgagta gatgctcaat ataaaattaa aacaaactac ggtaacattg atcgcaacgt 300

tcaatttaat tttgttaaag aagatggtat gtggaagtta gattgggatc atagcgtcat 360

tattccagga atgcagaaag accaaagcat acatattgaa aatttaaaat cagaacgtgg 420

taaaatttta gaccgaaaca atgtggaatt ggccaataca ggaacagcat atgagatagg 480

catcgttcca aagaatgtat ctaaaaaaga ttataaagca atcgctaaag aactaagtat 540

ttctgaagac tatatcaaac aacaaatgga tcaaaattgg gtacaagatg ataccttcgt 600

tccacttaaa accgttaaaa aaatggatga atatttaagt gatttcgcaa aaaaatttca 660

tcttacaact aatgaaacag aaagtcgtaa ctatcctcta ggaaaagcga cttca 715

<210> 803
<211> 360
<212> DNA
<213> Staphylococcus haemolyticus

<400> 803
gccaataagc aagttgaaat atctatggct gaatgggatg ttatgaatat aatatgggat 60

aaaaaatcag tatcagctaa tgaaattgta gttgaaattc aaaaatataa agaagttagc 120

gataaaacga ttagaacatt aatcacaaga ctatataaaa aagagattat aaaacgatac 180

aaatcagaga atatttattt ttactcatca aatattaaag aagacgatat taaaatgaaa 240

actgctaaaa cctttcttaa taaactgtat ggaggggaca tgaaaagttt agtgctgaat 300

tttgcgaaaa atgaagaatt aaataacaaa gaaattgaag aattgcgaga cattttaaat 360

<210> 804
<211> 300
<212> DNA
<213> Pseudomonas aeruginosa

<400> 804
catgcgtgta aatcatcgtc gtagagacgt cggaatggcc gagcagatcc tgcacggttc 60

gaatgtcgta accgctgcgg agcaaggccg tcgcgaacga gtggcggagg gtgtgcggtg 120

tggcgggctt cgtgatgcct gcttgttcta cggcacgttt gaaggcgcgc tgaaaggtct 180

ggtcatacat gtgatggcga cgcacgacac cgctccgtgg atcggtcgaa tgcgtgtgct 240

gcgcaaaaac ccagaaccac ggccaggaat gcccggcgcg cggatacttc cgctcaaggg 300

<210> 805
<211> 500
<212> DNA
<213> Streptococcus pneumoniae

<400> 805
tgaggaaggt agtaagggaa acaatatcaa actgaccatt gatttggcct tccaagatag 60

cgtggatgct ttactgaaaa gttatttcaa ttctgagcta gaaaatggtg gagccaagta 120

ttctgaaggt gtctatgcag tcgcccttaa cccaaaaaca ggtgcggttt tgtctatgtc 180

agggattaaa catgacttga aaacaggaga gttgacgcct gattccttgg gaacggtaac 240

caatgtcttt gttccaggtt cggttgtcaa ggcggcgacc atcagctccg gttgggaaaa 300

tggagtctta tcagggaacc agaccttgac agaccaaccg attgtcttcc aaggttcagc 360

tccgattaat tcttggtata ctcaagccta cgattcattc ccgattacag ctgtggaggc 420

cttggagtat tcttctaatg cctatatggt tcaaacggct ttgggcatta tgggtcagac 480

ctatcaaccc aatatgtttg 500

<210> 806
<211> 565
<212> DNA
<213> Staphylococcus epidermidis

<400> 806
tagcaataca atcgcacata cattaataga gaaaaagaaa aaagatggca aagatattca 60

actaactatt gatgctaaag ttcaaaagag tatttataac aacatgaaaa atgattatgg 120

ctcaggtact gctatccacc ctcaaacagg tgaattatta gcacttgtaa gcacaccttc 180

atatgacgtc tatccattta tgtatggcat gagtaacgaa gaatataata aattaaccga 240

agataaaaaa gaacctctgc tcaacaagtt ccagattaca acttcaccag gttcaactca 300

aaaaatatta acagcaatga ttgggttaaa taacaaaaca ttagacgata aaacaagtta 360

taaaatcgat ggtaaaggtt ggcaaaaaga taaatcttgg ggtggttaca acgttacaag 420

atatgaagtg gtaaatggta atatcgactt aaaacaagca atagaatcat cagataacat 480

tttctttgct agagtagcac tcgaattagg cagtaagaaa tttgaaaaag gcatgaaaaa 540

actaggtgtt ggtgaagata tacca 565

<210> 807
<211> 524
<212> DNA
<213> Streptococcus pneumoniae

<400> 807
tgaagatggc agcaagagct tgctgggaac ttctggaatg gagagttcct tgaacagtat 60

tcttgcaggg acagacggca ttattaccta tgaaaaggat cgtctgggca atattgtacc 120

cggaacagaa ctggtatcgc aacaaactgt ggatggcaag gatgtttata caacattgtc 180

tagtccgcta caatctttca tggaaactca gatggatgcc tttctagaaa aagtaaaagg 240

taagtatatg accgcgacct tggtcagtgc aaagaccggt gaaattctcg ctaccaccca 300

acgacctacc tttaatgcag atactaaaga aggaatcact gaggactttg tttggcgtga 360

tattctttat caaagtaact atgaaccagg atcagccttt aaggtcatga tgttagcttc 420

ttctattgat aataatacct tcccaagtgg agaatacttc aatagcagtg aattcaaaat 480

agcggatgcg acgactcgag attgggatgt taatgagggt ttga 524

<210> 808
<211> 715
<212> DNA
<213> Staphylococcus aureus

<400> 808
agagatgaat gcaggaacag ttttagatcc acaaatgata aaaaatgaag atgtcagtga 60

aaaagagtat gcagcagttt ctcagcaact ttccaaatta ccaggtgtta acacgtctat 120

ggattgggat agaaaatatc catatggcga tactttaaga ggtatattcg gagatgtatc 180

gacacctgct gaaggtattc caaaagaatt gacagaacat tacttatcca aaggatattc 240

acgcaatgat cgtgttggaa aatcttacct agaatatcaa tatgaagatg tattgcgtgg 300

taagaagaaa gaaatgaaat acacaacgga caaatctggt aaagttacat cttcagaagt 360

gttaaatcct ggcgctcgcg gtcaagattt gaaattaacg atcgatatag atcttcaaaa 420

agaagtagaa gcattattag ataaacaaat taagaagctt cgcagtcaag gtgccaaaga 480

tatggataat gcaatgatgg ttgtacaaaa tcctaaaaat ggagacattc ttgcgcttgc 540

cggaaagcag attaataaga gtggtaaaat gactgattat gacattggta cgtttacttc 600

tcaatttgcg gttggatctt ctgtaaaagg tggaacatta ttagccggtt atcagaataa 660

agctatcaaa gttggagaaa caatggtcga tgaaccatta catttccaag gtggt 715

<210> 809
<211> 623
<212> DNA
<213> Enterococcus faecalis

<400> 809
caaacaagaa ttagccgaag cgaagaaaac agctactaca tttttaaacg tattgtcaaa 60

acaggaattt gataagttac cgtccgttgt tcaagaagct agcttaaaga aaaatggcta 120

tgatactaaa tctgttgttg aaaaatacca agcaatttat tcagggattc aagcagaagg 180

agtcaaagct agtgatgttc aagtcaaaaa ggcgaaagac aatcaataca catttaccta 240

taaattatcg atgagcactc ctttaggcga aatgaaagat ttgtcttatc aatcaagtat 300

cgccaagaaa ggcgatacct accaaatcgc ttggaagccg tctttaattt ttccagatat 360

gtcaggaaat gataaaattt cgattcaagt agataatgcc aaacgtggag aaattgtcga 420

tcgtaatggt agtgggctag caattaacaa agtgtttgac gaagtgggcg tagtgcctgg 480

caaactcggt tctggcgcag aaaaaacagc caatatcaaa gcttttagtg ataagttcgg 540

cgtttctgtt gatgaaatca atcaaaagtt aagccaagga tgggtccaag cagactcctt 600

tgtaccaatc acagttgctt ctg 623

<210> 810
<211> 660
<212> DNA
<213> Enterococcus faecium

<400> 810
tacagatgca gacggtgtag agaaaaaagt tctgatcgaa catgaagttc aaaatggcaa 60

agatatcaaa ttgacaatcg atgcgaaggc acaaaaaaca gcttttgaca gtctaggagg 120

aaaagctgga tcaactgttg cgacaacgcc aaaaaccggt gatcttcttg cgcttgctag 180

ctctccaagc tatgatccaa acaaaatgac aaacgggatc tcacaagaag attacaaagc 240

ttatgaagaa aatcctgaac agccattcat cagccgattt gcgacaggtt atgctcctgg 300

atctacgttt aaaatgatta cagcagcaat cggtctcgac aacggcacta tcgatccaaa 360

tgaagtgttg acgatcaacg ggcttaaatg gcaaaaagac agttcttggg gatcttatca 420

agtaacgcgt gtcagtgatg tatcacaagt agacttaaaa actgctttga tctattccga 480

taatatatat acggcccaag aaacgttgaa aatgggtgag aaaaaatttc gtacaggctt 540

agataaattc atttttggtg aagaccttga tttgccaatt agtatgaatc cagcacaaat 600

ttctaatgaa gatagcttta actcagatat cttgctagct gatactggat atggacaggg 660

<210> 811
<211> 522
<212> DNA
<213> Enterococcus faecalis

<400> 811
gccggtgtat cactaaagga aaaaacagct tctctatatg aaggaagcca agtggtaaaa 60

gctaagcgag gatcaatttt agatcgatat ggtaatccaa ttgcagaaga tgctacttcc 120

tattcgttat atgtcgtatt atcaaaaaaa tatacgggac aaaataatga aaagctatac 180

gcggagaaaa aagacttcga tgatattgct gaaattttag cgaaatatac caaactagac 240

aaaaaaacag cattgaaata cttgaataat gggatccatg aagatgggtc aacacaatat 300

caagtggaat ttggtacggg tggtcaaaac atcaccttgg aaacacgcca aaaaattgaa 360

gcagatttga aaaagaaaaa aatttcaggt gtttatttca atgaacatcc agccagatta 420

tatcccaatg gtcagtttgc ttctcacttt attggctata caaaagcagc caatccagat 480

gatgataaag aaggcttagt aggagcaatg ggactagaac ag 522

<210> 812
<211> 332
<212> DNA
<213> Staphylococcus aureus

<400> 812
taataaaacg tatgaaatat catctgcaga atgggaagtt atgaatatca tttggatgaa 60

aaaatatgca agtgcgaata atataataga agaaatacaa atgcaaaagg actggagtcc 120

aaaaaccatt cgtacactta taacgagatt gtataaaaag ggatttatag atcgtaaaaa 180

agacaataaa attttttaat attactctct tgtagaagaa agtgatataa aatataaaac 240

atctaaaaac tttatcaata aagtctacaa aggcggtttc aattcacttg tcttaaactt 300

tgtagaaaaa gaagatctat cacaagatga aa 332

<210> 813
<211> 530

<212>  DNA
<213>  Streptococcus pneumoniae

<400>  813
cttggttagc gattcagtta gaacaaaaag caaccaagca agaaatcttg acctactata      60

taaataaggt ctacatgtct aatggcaact atggaatgca gacagcagct caaaactact     120

atggtaaaga cctcaataat ttaagtttac ctcagttagc cttgctggct ggaatgcctc     180

aggcaccaaa ccaatatgac ccctattcac atccagaagc agcccaagac cgccgaaact     240

tggtcttatc tgaaatgaaa aatcaaggct acatctctgc tgaacagtat gagaaagcag     300

tcaatacacc aattactgat ggactacaaa gtctcaaatc agcaagtaat taccctgctt     360

acatggataa ttacctcaag gaagtcatca atcaagttga agaagaaaca ggatataacc     420

tgctcacaac tgggatggat gtctacacaa atgtagacca agaagctcaa aaacatctgt     480

gggatattta caatacagac gaatacgttg cctatccaga cgatgaattg                530


<210>  814
<211>  355
<212>  DNA
<213>  Staphylococcus aureus

<400>  814
agcaagttga aatatctatg gctgaatggg atgttatgaa tataatatgg gataaaaaat      60

cagtatcagc taatgaaatt gtagttgaaa ttcaaaaata taaagaagtt agcgataaaa     120

cgattagaac attaatcaca agactatata aaaaagagat tataaaacga tacaaatcag     180

agaatattta tttttactca tcaaatatta aagaagacga tattaaaatg aaaactgcta     240

aaacctttct taataaactg tatggagggg acatgaaaag tttagtgctg aattttgcga     300

aaaatgaaga attaaataac aaagaaattg aagaattgcg agacatttta aatga          355


<210>  815
<211>  702
<212>  DNA
<213>  Escherichia coli

<400>  815
acatcgaact ggatctcaac agcggtaaga tccttgagag ttttcgcccc gaagaacgtt      60

ttccaatgat gagcactttt aaagttctgc tatgtggtgc ggtattatcc cgtgttgacg     120

ccgggcaaga gcaactcggt cgccgcatac actattctca gaatgacttg gttaagtact     180

caccagtcac agaaaagcat cttacggatg gcatgacagt aagagaatta tgcagtgctg     240

ccataaccat gagtgataac actgctgcca acttacttct gacaacgatc ggaggaccga     300

aggagctaac cgcttttttg cacaacatgg gggatcatgt aactcgcctt gatcgttggg     360

aaccggagct gaatgaagcc ataccaaacg acgagcgtga caccacgacg cctgcagcaa     420

tggcaacaac gttgcgcaaa ctattaactg gcgaactact tactctagct tcccggcaac     480

aattaataga ctggatggag gcggataaag ttgcaggacc acttctgcgc tcggcccttc 540

cggctggctg gtttattgct gataaatctg gagccggtga gcgtgggtct cgcggtatca 600

ttgcagcact ggggccagat ggtaagccct cccgtatcgt agttatctac acgacgggga 660

gtcaggcaac tatggatgaa cgaaatagac agatcgctga ga 702

<210> 816
<211> 596
<212> DNA
<213> Klebsiella oxytoca

<400> 816
tgtgcagcac cagtaaagtg atggccgccg ccgcggtatt aaaacagagc gaaagcaata 60

aagaggtggt aaataaaagg ctggagatta acgcagccga tttggtggtc tggagtccga 120

ttaccgaaaa acatctccag agcggaatga cgctggctga gctaagcgcg gcgacgctgc 180

aatatagcga caatacggcg atgaatctga tcatcggcta ccttggcggg ccggaaaaag 240

tcaccgcctt cgcccgcagt atcggcgatg ccacctttcg tctcgatcgt acggagccca 300

cgctgaatac cgccatcccg ggcgatgagc gtgataccag cacgccgctg gcgatggctg 360

aaagcctacg caagctgacg cttggcgatg cgctgggcga acagcaacgc gcccagttag 420

tcacctggct gaaaggcaat accaccggcg ggcaaagcat tcgcgcgggc ctgcctgaaa 480

gctgggtggt cggcgataaa accggcgccg gagattacgg caccaccaat gatattgcgg 540

ttatctggcc ggaagatcac gctccgctgg tattagtcac ctactttacc cagccg 596

<210> 817
<211> 558
<212> DNA
<213> Enterococcus faecium

<400> 817
acagtgccag ttcttatcgt ttattgcaag ccgatgaaaa tacaaaaagt ctattattgc 60

gtcaactaat tttcatattt ttgagttgga gcgtgatctt cttagctcgt tcagtcaaac 120

tacactattt acttcaccct aaaatagcag gatacggttt agccttatcg attttctttt 180

tagtattagt aagaataggg atattcggtg tcactgtcaa cggcgcacaa cgttggatct 240

ctctgtttgg cattcaattc cagccttctg aactggcaaa tctttttttg attttttatt 300

taagctggtt ttttcgtgac ggaaatagta gcccaaaaga tctaaaaaaa ccattcctga 360

ttacagtagg tataactttt ctgattttat ttcagccaaa gattgctgga gcattgatga 420

tcctttcgat tgcgtgggtc atattttggg cagcggcggt tccatttaaa aaagggatct 480

atctaatcgt tactttttct gcattgctga ttggagcagc aggcggggta ttatatttag 540

gaaataaagg ttggcttc 558

<210> 818
<211> 750
<212> DNA
<213> Staphylococcus aureus

<400> 818
ctcacccaaa tggagattta ttacaattaa cgaaatgggc agaaacaaag aaattaactg 60

gatggtacgc gcgaagaatc gctgtaggtc gtgacggtga agttcagggt gttgcgcaat 120

tactttttaa aaaagtacct aaattaccgt atacgctatg ttatatttca cgtggttttg 180

ttgttgatta tagtaataaa gaagcgttaa atgcattgtt agacagtgca aaagaaattg 240

ctaaagctga gaaagcgtat gcaattaaaa tcgatcctga tgttgaagtt gataaaggta 300

cagatgcttt gcaaaatttg aaagcgcttg gttttaaaca taaaggattt aaagaaggtt 360

tatcaaaaga ctacatccaa ccacgtatga ctatgattac accaattgat aaaaatgatg 420

atgagttatt aaatagtttt gaacgccgaa atcgttcaaa agtgcgcttg gctttaaagc 480

gaggtacgac agtagaacga tctgatagag aaggtttaaa aacatttgct gaattaatga 540

aaatcactgg ggaacgcgat ggcttcttaa cgcgtgatat tagttacttt gaaaatattt 600

atgatgcgtt gcatgaagat ggagatgctg aactattttt agtaaagttg gacccaaaag 660

aaaatatagc gaaagtaaat caagaattga atgaacttca tgccgaaata gctaaatggc 720

agcagaagat ggaaacatct gaaaagcaag 750

<210> 819
<211> 363
<212> DNA
<213> Proteus vulgaris

<400> 819
acaacatttc gccaaacagc gacgattgca gtttcattaa tatctctatt ggtatctcca 60

atgctatggg ctaacaccaa taatacgatt gaagagcaat taagtacgct tgaaaaatat 120

agccaaggtc gtttaggtgt tgctttaatc aacacggaag ataattcaca aataacatat 180

cgtggtgaag aacgttttgc gatggcaagt acaagtaagg ttatggctgt tgcggcagtt 240

ttaaaagaga gtgaaaaaca agcgggatta ttagataaga atattacaat taaaaaatcc 300

gacttagttg cttacagccc tattacagaa aaacatttag taacaggaat gtctttagct 360

caa 363

<210> 820
<211> 545
<212> DNA
<213> Staphylococcus haemolyticus

<400> 820
aatgatggct ttgaagtagt gttactaggt gtaaaagatg aaagcaataa agtattagct 60

gctagtcttt tctctaaaat accgaccatg ggaagttatg tatattactc aaatcgaggc 120

ccagtaatgg actattctga tttaggctta gttgattttt acttacgcga attagaaaag 180

tatttacatc aacaccaatg tttatacgtt aaaattgatc catactggat ttatcaaatt 240

tatgataaag atattaatcc acttgaagat agagagaaaa atgatgctat agttaatttg 300

tttaaatcac atggatatga acaccatgga tttactactg aatatgacac atcaagtcaa 360

gcaagatgga tgggtgttag ctatctaaaa ggggaaacac ctgcttcatt aagaaaacaa 420

tttgatagcc aacgtaaaag aaatattaat aaagcgataa actatggggt gaaagtaaga 480

ttccttggta gagatgagtt tcatatattc ttagacttat accgtgaaac agaagcaaga 540

acagg 545

<210> 821
<211> 633
<212> DNA
<213> Pseudomonas aeruginosa

<400> 821
ccatcaggca acagaatgat acctaaatca ttagtggccg cagtttttcc ggctttgata 60

cccgaagtac cagttttatg tgcgaccaca gtaccagctg gtaacaaacc ttttaaccgc 120

tctggtcctg tggtggtttc gaccatccac ttccataaca aagcctgcga ggtttcagac 180

agctgtgttt tttgctcaaa ctttttcagg atctctgcag cacctttcat cgaggtccag 240

ttttgatact gcacctgatc atcggcgtgc atctgcgctt catttgcgac cacagcggtc 300

tcctttatac ccatagactg gatatagtca tgcaaagcag ctggtccacc aaccagttca 360

aataacaaat cacaggccac gttatcgctg tgcgagaccg agtattgcag cagttgctgc 420

actggaacac taaactcgtc tccctgatac gctttcatta tcggagccca ggtattctgt 480

aaaaccttag ccctgtttac gataacggtc tgatttaaat ccaactttcc ctgatcaacc 540

tgatgcagta ccaacatagc taaatgcaat ttaaatacac tttgcattgg gaatttttca 600

aaaggattaa tcagtaaagg ttccagatcg tca 633

<210> 822
<211> 340
<212> DNA
<213> Klebsiella oxytoca

<400> 822
cttactatcg gagctggtca ccggtttatc cggcagggac tcatcgccag tattccaacc 60

ccagcatagg cctgtttggt cacctggccg caaatagtct gggccagcca tttgagcaac 120

tgatgagcca gaccctgctg cccaagctgg gtttgcacca cacctatatc caggtgccgg 180

agtcggccat ggcgaactat gcctacggct attcgaagga agataagccc atccgggtca 240

ctccgggcgt gctggcggcc gaggcttacg ggatcaagac cggctcggcg gatctgctga 300

agtttgccga ggcaaacatg gggtatcagg gagatgccct 340

<210> 823
<211> 768
<212> DNA
<213> Proteus vulgaris

<400> 823
tcactcatta accattgctg aaatatttcc attgatgctg tcattggctt tgattttaaa 60

tatgttagcc aatattttcc catttcaact tctattttaa agggttgcac taactgacca 120

ttttcaattt ctcttgaaaa catttttgct ggtgctaatg caactcctcc ttcataaata 180

gcgctttcaa tcattaagcg tgaagagtca aaaatagagc ccgttatttt tataggcgac 240

atatttgctt tttcaaacca ttgcaaccac tcatcttctc gataagagcg atacaagttt 300

tcatttatta gatcagttgg atgttgtaaa cgtttcgccg tacccgatga acacaatacc 360

gttaatggtg cagaaaataa tgctttgtta tgagttaatg gccataaacc ttcaccaaat 420

cgaatagcaa aatctaatcc ttcagtagcc aaattgacca cattattatt tgttcttaaa 480

ttcacttcta ttcttggata taactgccta aattcggcca acctaggtaa taaccaccca 540

accgcaaatg taccgacagc tgcaattgaa acaacatcgc gatattcacc gcgttcaaat 600

tgtttaaata cacgctcaat atcactaaaa gccgttgtta atacagaaaa taagatttga 660

gcatcatccg tcatttctaa acctcgaggt aagcgcttaa aaagaataac gccaagccgc 720

tcttctaaca ttctcacttg ttggctaaca gcaccttgag tgacatac 768

<210> 824
<211> 568
<212> DNA
<213> Enterococcus faecium

<400> 824
ttatctgttt tgttactgct tacactagta gtcggctttt tttcgattga atttgtccat 60

ggattttcgt ctgcaaaaca gacctcaacc gtaaaaaagg tagatccgaa aagtgtccct 120

accacactaa atgtggcttt gattggttcg gatgcccggt cgaaagaaga aaatggtcgc 180

tcagattcac ttatggttgc acaatacgac cagaaaacac aacaagcaaa actaatctct 240

atcatgagag actcatatgt cgatatacca ggttacggaa tggataaaat caatgcagcg 300

tactcttacg gaggaattga tttattgaac caaacattaa aggaaaattt caaatttgaa 360

gccccgtatt atgcaagtat cacatttcaa gattttatcg attgcgtcaa tgaactgttt 420

cctgatggag taaagattga tgcagaaaaa tctttagatt tagatggcgt atatataaaa 480

aaagggaagc aagtaatgga tggcaatacg ttactgcagt atgctcgatt ccgtgaagac 540

gaagaagggg actttgggag gattagaa 568

<210> 825
<211> 763
<212> DNA
<213> Staphylococcus aureus

<400> 825
tgacttcgga tgagttcaat gcgtttacaa caaagcattt ttcacattac acacaatcag 60

ctattcatta caatcataga gttgatttaa aaggcgatgt gcatcttgta ggggttaaag 120

atgacaatgg tcaagtgatt gcaggatgct tattgacaga agcacgcaca cttaaatttt 180

tcaaatattt ttatacacat cgcgggccag tgatggatta tacaaatcaa tcattagtag 240

catttttctt taaagcatta acgtcatatt taaagaaaca caattgttta tatgtccttg 300

tagatccata tttaattgaa aatttacgca atgcagacgg tgaaattgtt aaatcttatg 360

ataaccgagc atttgttaga acaatggata aattaggtta taaacaccaa ggtttccctg 420

taggttatga ttcaatgagc caaatccgtt ggctgtcagt gttagattta aaagataaga 480

ctgaagacca acttttaaaa gaaatggatt atcaaacgag acgtaatatt aaaaaaacat 540

atgatattgg tgtcaaaact aaaacgttaa cgattgatga aacgcaaact tttttcgact 600

tattccatat ggctgaggaa aagcacggtt tcaaattccg tgagttacca tactttgaag 660

aaatgcaaaa gttatacgat gaccacgcca tgttaaagtt ggcgtatatt gatttaaacg 720

agtatttaaa aacgttacaa ttaaagcaac aacaattaac agc 763

<210> 826
<211> 552
<212> DNA
<213> Staphylococcus epidermidis

<400> 826
aagtataatc agttcattgc tcacgatatg tgtaattttt ttagtgagaa tgctctatat 60

aaaatatact caaaatatta tgtcacataa gatttggtta ttagtgctcg tctccacgtt 120

aattccatta ataccatttt acaaaatatc gaattttaca ttttcaaaag atatgatgaa 180

tcgaaatgta tctgacacga cttcttcggt tagtcatatg ttagatggtc aacaatcatc 240

tgttacgaaa gacttagcaa ttaatgttaa tcagtttgag acctcaaata taacgtatat 300

gattcttttg atatgggtat ttggtagttt gttgtgctta ttttatatga ttaaggcatt 360

ccgacaaatt gatgttatta aaagttcgtc attggaatcg tcatatctta atgaacgact 420

taaagtatgt caaagtaaga tgcagttcta caaaaagcat ataacaatta gttatagttc 480

aaacattgat aatccgatgg tatttggttt agtgaaatcc caaattgtac taccaactgt 540

cgtagtcgaa ac 552

<210> 827
<211> 810

<212>  DNA
<213>  Staphylococcus aureus

<400>  827
tgctttagtt ttaagtgcat gtaattcaaa cagttcacat gccaaagagt taaatgattt      60

agaaaaaaaa tataatgctc atattggtgt ttatgcttta gatactaaaa gtggtaagga     120

agtaaaattt aattcagata agagatttgc ctatgcttca acttcaaaag cgataaatag     180

tgctattttg ttagaacaag taccttataa taagttaaat aaaaaagtac atattaacaa     240

agatgatata gttgcttatt ctcctatttt agaaaaatat gtaggaaaag atatcacttt     300

aaaagcactt attgaggctt caatgacata tagtgataat acagcaaaca ataaaattat     360

aaaagaaatc ggtggaatca aaaaagttaa acaacgtcta aaagaactag gagataaagt     420

aacaaatcca gttagatatg agatagaatt aaattactat tcaccaaaga gcaaaaaaga     480

tacttcaaca cctgctgctt tcggtaagac tttaaataaa cttatcgcaa atggaaaatt     540

aagcaaagaa aacaaaaaat tcttacttga tttaatgtta aataataaaa gcggagatac     600

tttaattaaa gacggtgttc caaaagacta taaggttgct gataaaagtg gtcaagcaat     660

aacatatgct tctagaaatg atgttgcttt tgtttatcct aagggccaat ctgaacctat     720

tgttttagtc atttttacga ataaagacaa taaaagtgat aagccaaatg ataagttgat     780

aagtgaaacc gccaagagtg taatgaagga                                      810


<210>  828
<211>  565
<212>  DNA
<213>  Plasmid RGN238

<400>  828
tttgaaggaa ctgaaggttg ttttttactt tacgatgcat ccacaaacgc tgaaattgct      60

caattcaata aagcaaagtg tgcaacgcaa atggcaccag attcaacttt caagatcgca     120

ttatcactta tggcatttga tgcggaaata atagatcaga aaaccatatt caaatgggat     180

aaaaccccca aaggaatgga gatctggaac agcaatcata caccaaagac gtggatgcaa     240

ttttctgttg tttgggtttc gcaagaaata acccaaaaaa ttagattaaa taaaatcaag     300

aattatctca aagattttga ttatggaaat caagacttct ctggagataa agaaagaaac     360

aacggattaa cagaagcatg gctcgaaagt agcttaaaaa tttcaccaga agaacaaatt     420

caattcctgc gtaaaattat taatcacaat ctcccagtta aaaactcagc catagaaaac     480

accatagaga acatgtatct acaagatctg gataatagta caaaactgta tgggaaaact     540

ggtgcaggat tcacagcaaa tagaa                                           565


<210>  829
<211>  226
<212>  DNA

<213>  Klebsiella pneumoniae

<400>  829
ggcttacggg atcaagaccg gctcggcgga tctgctgaag tttgccgagg caaacatggg      60

gtatcaggga gatgccgcgg taaaaagcgc gatcgcgctc acccacaccg gtttctactc     120

ggtgggagac atgacccagg gactgggctg ggagagttac gcctatccgg tgaccgagca     180

gacattgctg gcgggtaacg caccggcggt gagcttccag gccaat                    226


<210>  830
<211>  502
<212>  DNA
<213>  Proteus mirabilis

<400>  830
gcggtaagat ccttgagagt tttcgccccg aagaacgttt tccaatgatg agcactttta      60

aagttctgct atgtggtgcg gtattatccc gtgttgacgc cgggcaagag caactcggtc     120

gccgcataca ctattctcag aatgacttgg ttaagtactc accagtcaca gaaaagcatc     180

ttacggatgg catgacagta agagaattat gcagtgctgc cataaccatg agtgataaca     240

ctgcggccaa cttacttctg acaacgatcg gaggaccgaa ggagctaacc gcttttttgc     300

acaacatggg ggatcatgta acccgccttg atcgttggga accggagctg aatgaagcca     360

taccaaacga cgagcgtgac accacgacgc ctgcagcaat ggcaacaacg ttgcgcaaac     420

tattaactgg cgaactactt actctagctt cccggcaaca attaatagac tggatggagg     480

cggataaagt tgcaggacca ct                                              502


<210>  831
<211>  391
<212>  DNA
<213>  Staphylococcus warneri

<400>  831
agttgaaaat gaaatatgta taagaacttt aatagatgat gattttcctt tgatgttaaa      60

atggttaact gatgaaagag tattagaatt ttatggtggt agagataaaa aatatacatt     120

agaatcatta aaaaaacatt atacagagcc ttgggaagat gaagttttta gagtaattat     180

tgaatataac aatgttccta ttggatatgg acaaatatat aaaatgtatg atgagttata     240

tactgattat cattatccaa aaactgatga gatagtctat ggtatggatc aatttatagg     300

agagccaaat tattggagta aaggaattgg tacaagatat attaaattga tttttgaatt     360

tttgaaaaaa gaaagaaatg ctaatgcagt t                                    391


<210>  832
<211>  380
<212>  DNA
<213>  Pseudomonas aeruginosa

<400>  832
tcattcgcac atgtaggctc ggccctgacc aagtccaatc catgcgggct gctcttgatc      60

ttttcggtcg tgagttcgga gacgtagcca cctactccca acatcagccg gactccgatt     120

acctcgggaa cttgctccgt agtaggacat tcatcgcgct tgctgccttc gagcaagaag     180

cggttgttgg cgctctcgcg gcttacgttc tgcccaagtt tgagcaggcg cgtagtgaga     240

tctatatcta tgatctcgca gtctccggcg agcaccgccg gcagggcatt gccaccgcgc     300

tcatcaatct cctcaagcat gaggccaacg cgcttggtgc ttacgtgatc tacgtgcaag     360

cggattacgg tgacgatccc                                                 380


<210>  833
<211>  616
<212>  DNA
<213>  Escherichia coli

<400>  833
gaccgatcac cctacgagga gactcgtaat ggcgctcggt tggatgacaa aacccgccgt      60

acctggccgc cgttcgatcc cgcaacggcc gggacttacc gtgggttcgg cctgctgaat     120

cagtttctgg ttcaagcccc cggcgcgcgg cgcagcgcgc accccgatgc atcgatggtc     180

gcggttggtc cactggctga aacgctgacg gagcctcaca agctcggtca cgccttgggg     240

gaagggtcgc ccgtcgagcg gttcgttcgc cttggcggga aggccctgct gttgggtgcg     300

ccgctaaact ccgttaccgc attgcactac gccgaggcgg ttgccgatat ccccaacaaa     360

cggcgggtga cgtatgagat gccgatgctt ggaagcaacg gcgaagtcgc ctggaaaacg     420

gcatcggatt acgattcaaa cggcattctc gattgctttg ctatcgaagg aaagccggat     480

gcggtcgaaa ctatagcaaa tgcttacgtg aagctcggtc gccatcgaga aggtgtcgtg     540

ggctttgctc agtgctacct gttcgacgcg caggacatcg tgacgttcgg cgtcacctat     600

cttgagaagc atttcg                                                     616


<210>  834
<211>  707
<212>  DNA
<213>  Escherichia coli

<400>  834
aagtttcatt gccagacggg acttctgcaa tcgtcaaggg attgaaacct atagaagaca      60

ttgctgatga actgcgcggg gccgactatc tggtatggcg caatgggagg ggagcagtcc     120

ggttgctcgg tcgtgagaac aatctgatgt tgctcgaata tgccggggag cgaatgctct     180

ctcacatcgt tgccgagcac ggcgactacc aggcgaccga aattgcagcg gaactaatgg     240

cgaactgtat gcccgcatct gaggacccct gccttctgcc cttctcccga tccgggatcg     300

ctttgcagct ttgtttcagc gggcgcgcga atgatcaaaa cgcaggttgt caaactgact     360

acgtccacgc ggcgattata gccgatcaaa tgatgagcaa tgcctcggaa ctgcgtgggc 420

tacatggcga tctgcatcat gaaaacatca tgttctccag tcgcggctgg ctggtgaaag 480

atcccgtcgg tctggtcggt gaagtgggct ttggcgccgc aaatatgttc tacgatccgg 540

ctgacagaga cgacctttgt ctcgatccta gacgcattgc acagatggcg gacgcattct 600

ctcgtgcgct ggacgtcgat ccgcgtcgcc tgctcgaaca ggcgtacgct tatgggtgcc 660

tttccgcagc ttggaacgcg gatggagaag aggagcaacg cagtcta 707

<210> 835
<211> 545
<212> DNA
<213> Enterococcus faecalis

<400> 835
gccgaagtat cgactcaact atcagaggta gttggcgtca tcgagcgcca tctcgaaccg 60

acgttgctgg ccgtacattt gtacggctcc gcagtggatg gcggcctgaa gccacacagt 120

gatattgatt tgctggttac ggtgaccgta aggcttgatg aaacaacgcg gcgagctttg 180

atcaacgacc ttttggaaac ttcggcttcc cctggagaga gcgagattct ccgcgctgta 240

gaagtcacca ttgttgtgca cgacgacatc attccgtggc gttatccagc taagcgcgaa 300

ctgcaatttg gagaatggca gcgcaatgac attcttgcag gtatcttcga gccagccacg 360

atcgacattg atctggctat cttgctgaca aaagcaagag aacatagcgt tgccttggta 420

ggtccagcgg cggaggaact ctttgatccg gttcctgaac aggatctatt tgaggcgcta 480

aatgaaacct taacgctatg gaactcgccg cccgactggg ctggcgatga gcgaaatgta 540

gtgct 545

<210> 836
<211> 515
<212> DNA
<213> Escherichia coli

<400> 836
gcaggtcaca ttgatacaca aaattctagc tgcggcagat gagcgaaatc tgccgctctg 60

gatcggtggg ggctgggcga tcgatgcacg gctagggcgt gtaacacgca agcacgatga 120

tattgatctg acgtttcccg gcgagaggcg cggcgagctc gaggcaatag ttgaaatgct 180

cggcgggcgc gtcatggagg agttggacta tggattctta gcggagatcg gggatgagtt 240

acttgactgc gaacctgctt ggtgggcaga cgaagcgtat gaaatcgcgg aggctccgca 300

gggctcgtgc ccagaggcgg ctgagggcgt catcgccggg cggccagtcc gttgtaacag 360

ctgggaggcg atcatctggg attactttta ctatgccgat gaagtaccac cagtggactg 420

gcctacaaag cacatagagt cctacaggct cgcatgcacc tcactcgggg cggaaaaggt 480

tgaggtcttg cgtgccgctt tcaggtcgcg atatg 515

<210> 837
<211> 502
<212> DNA
<213> Staphylococcus aureus

<400> 837
gctattggtg tttatggctc tcttggtcgt cagactgatg ggccctattc ggatattgag 60

atgatgtgtg tcatgtcaac agaggaagca gagttcagcc atgaatggac aaccggtgag 120

tggaaggtgg aagtgaattt tgatagcgaa gagattctac tagattatgc atctcaggtg 180

gaatcagatt ggccgcttac acatggtcaa tttttctcta ttttgccgat ttatgattca 240

ggtggatact tagagaaagt gtatcaaact gctaaatcgg tagaagccca aacgttccac 300

gatgcgattt gtgcccttat cgtagaagag ctgtttgaat atgcaggcaa atggcgtaat 360

attcgtgtgc aaggaccgac aacatttcta ccatccttga ctgtacaggt agcaatggca 420

ggtgccatgt tgattggtct gcatcatcgc atctgttata cgacgagcgc ttcggtctta 480

actgaagcag ttaagcaatc ag 502

<210> 838
<211> 452
<212> DNA
<213> Pseudomonas aeruginosa

<400> 838
gctaaatcga tctcatatcg tcgagtggtg gggcggagaa gaagcacgcc cgacacttgc 60

tgacgtacag gaacagtact tgccaagcgt tttagcgcaa gagtccgtca ctccatacat 120

tgcaatgctg aatggagagc cgattgggta tgcccagtcg tacgttgctc ttggaagcgg 180

ggacggatgg tgggaagaag aaaccgatcc aggagtacgc ggaatagacc agtcactggc 240

gaatgcatca caactgggca aaggcttggg aaccaagctg gttcgagcac tggttgagtt 300

gctgttcaat gatcccgagg tcaccaagat ccaaacggac ccgtcgccga gcaacttgcg 360

agcgatccga tgctacgaga aagcggggtt tgagaggcaa ggtaccgtaa ccaccccaga 420

tggtccagcc gtgtacatgg ttcaaacacg cc 452

<210> 839
<211> 565
<212> DNA
<213> Escherichia coli

<400> 839
ctcatttggc tcaaaggtcg aggtgtggct tgccccgagg tgatcaactg gcaggaggaa 60

caggagggtg catgcttggt gataacggca attccgggag taccggcggc tgatctgtct 120

ggagcggatt tgctcaaagc gtggccgtca atggggcagc aacttggcgc tgttcacagc 180

ctatcggttg atcaatgtcc gtttgagcgc aggctgtcgc gaatgttcgg acgcgccgtt 240

gatgtggtgt cccgcaatgc cgtcaatccc gacttcttac cggacgagga caagagtacg 300

ccgcagctcg atcttttggc tcgtgtcgaa cgagagctac cggtgcggct cgaccaagag 360

cgcaccgata tggttgtttg ccatggtgat ccctgcatgc cgaacttcat ggtggaccct 420

aaaactcttc aatgcacggg tctgatcgac cttgggcggc tcggaacagc agatcgctat 480

gccgatttgg cactcatgat tgctaacgcc gaagagaact gggcagcgcc agatgaagca 540

gagcgcgcct tcgctgtcct attca 565

<210> 840
<211> 707
<212> DNA
<213> Staphylococcus aureus

<400> 840
gagaatatca ccggaattga aaaaactgat cgaaaaatac cgctgcgtaa aagatacgga 60

aggaatgtct cctgctaagg tatataagct ggtgggagaa aatgaaaacc tatatttaaa 120

aatgacggac agccggtata aagggaccac ctatgatgtg gaacgggaaa aggacatgat 180

gctatggctg gaaggaaagc tgcctgttcc aaaggtcctg cactttgaac ggcatgatgg 240

ctggagcaat ctgctcatga gtgaggccga tggcgtcctt tgctcggaag agtatgaaga 300

tgaacaaagc cctgaaaaga ttatcgagct gtatgcggag tgcatcaggc tctttcactc 360

catcgacata tcggattgtc cctatacgaa tagcttagac agccgcttag ccgaattgga 420

ttacttactg aataacgatc tggccgatgt ggattgcgaa aactgggaag aagacactcc 480

atttaaagat ccgcgcgagc tgtatgattt tttaaagacg gaaaagcccg aagaggaact 540

tgtcttttcc cacggcgacc tgggagacag caacatcttt gtgaaagatg gcaaagtaag 600

tggctttatt gatcttggga gaagcggcag ggcggacaag tggtatgaca ttgccttctg 660

cgtccggtcg atcagggagg atatcgggga agaacagtat gtcgagc 707

<210> 841
<211> 329
<212> DNA
<213> Pseudomonas aeruginosa

<400> 841
cctgaccaag tccaatccat gcgggctgct cttgatcttt tcggtcgtga gttcggagac 60

gtagccacct actcccaaca tcagccggac tccgattacc tcgggaactt gctccgtagt 120

aggacattca tcgcgcttgc tgccttcgag caagaagcgg ttgttggcgc tctcgcggct 180

tacgttctgc ccaagtttga gcaggcgcgt agtgagatct atatctatga tctcgcagtc 240

tccggcgagc accgccggca gggcattgcc accgcgctca tcaatctcct caagcatgag 300

gccaacgcgc ttggtgctta cgtgatcta 329

<210> 842
<211> 423
<212> DNA
<213> Pseudomonas aeruginosa

<400> 842
tgcgatgctc tatgagtggc taaatcgatc tcatatcgtc gagtggtggg gcggagaaga 60

agcacgcccg acacttgctg acgtacagga acagtacttg ccaagcgttt tagcgcaaga 120

gtccgtcact ccatacattg caatgctgaa tggagagccg attgggtatg cccagtcgta 180

cgttgctctt ggaagcgggg acggatggtg ggaagaagaa accgatccag gagtacgcgg 240

aatagaccag tcactggcga atgcatcaca actgggcaaa ggcttgggaa ccaagctggt 300

tcgagcactg gttgagttgc tgttcaatga tcccgaggtc accaagatcc aaacggaccc 360

gtcgccgagc aacttgcgag cgatccgatg ctacgagaaa gcggggtttg agaggcaagg 420

tac 423

<210> 843
<211> 613
<212> DNA
<213> Staphylococcus aureus

<400> 843
agatttgcca gaacatgaat tacacgaggg caaaaaagaa gattgttatt taatggaata 60

tagatatgat gataatgcca caaatgttaa ggcaatgaaa tatttaattg agcattactt 120

tgataatttc aaagtagata gtattgaaat aatcggtagt ggttatgata gtgtggcata 180

tttagttaat aatgaataca tttttaaaac aaaatttagt actaataaga aaaaaggtta 240

tgcaaaagaa aaagcaatat ataatttttt aaatacaaat ttagaaacta atgtaaaaat 300

tcctaatatt gaatattcgt atattagtga tgaattatct atactaggtt ataaagaaat 360

taaaggaact tttttaacac cagaaattta ttctactatg tcagaagaag aacaaaattt 420

gttaaaacga gatattgcca gttttttaag acaaatgcac ggtttagatt atacagatat 480

tagtgaatgt actattgata ataaacaaaa tgtattagaa gagtatatat tgttgcgtga 540

aactatttat aatgatttaa ctgatataga aaaagattat atagaaagtt ttatggaaag 600

actaaatgca aca 613

<210> 844
<211> 424
<212> DNA
<213> Staphylococcus aureus

<400> 844
atatcaggaa agattggaaa tacggattct gttagaccac ttgaagttac ggttataaat 60

aggagtgaag ttgtcccttg gcaatatcct ccaaaaagag aatttatata cggtgagtgg 120

ctcaggggtg aatttgagaa tggacaaatt caggaaccaa gctatgatcc tgatttggct 180

attgttttag cacaagcaag aaagaatagt atttctctat ttggtcctga ttcttcaagt 240

atacttgtct ccgtaccttt gacagatatt cgaagagcaa ttaaggattc tttgccagaa 300

ctaattgagg ggataaaagg tgatgagcgt aatgtaattt taaccctagc tcgaatgtgg 360

caaacagtga ctactggtga aattacctcg aaagatgtcg ctgcagaatg ggctatacct 420

cttt 424

<210> 845
<211> 532
<212> DNA
<213> synthetic construct

<400> 845
aagatacgga aggaatgtct cctgctaagg tatataagct ggtgggagaa aatgaaaacc 60

tatatttaaa aatgacggac agccggtata aagggaccac ctatgatgtg gaacgggaaa 120

aggacatgat gctatggctg gaaggaaagc tgcctgttcc aaaggtcctg cactttgaac 180

ggcatgatgg ctggagcaat ctgctcatga gtgaggccga tggcgtcctt tgctcggaag 240

agtatgaaga tgaacaaagc cctgaaaaga ttatcgagct gtatgcggag tgcatcaggc 300

tctttcactc catcgacata tcggattgtc cctatacgaa tagcttagac agccgcttag 360

ccgaattgga ttacttactg aataacgatc tggccgatgt ggattgcgaa aactgggaag 420

aagacactcc atttaaagat ccgcgcgagc tgtatgattt tttaaagacg gaaaagcccg 480

aagaggaact tgtcttttcc cacggcgacc tgggagacag caacatcttt gt 532

<210> 846
<211> 200
<212> DNA
<213> Staphylococcus aureus

<400> 846
acacagtcaa aactttatta cttcaaaaca taatatagat aaaataatga caaatataag 60

attaaatgaa catgataata tctttgaaat cggctcagga aaagggcatt ttacccttga 120

attagtacag aggtgtaatt tcgtaactgc cattgaaata gaccataaat tatgcaaaac 180

tacagaaaat aaacttgttg 200

<210> 847
<211> 510
<212> DNA
<213> Enterococcus faecium

<400> 847
cgtttaccaa aggagaaggt gaccaatact ctgatataga gttctatata tttttgaaac 60

atagtataac ctcgaacttt gattcatcca actggttgtt tgacgtagct ccgtacttga 120

tgctttataa aaatgagtac ggaacagagg tagttatttt tgataatctt atacgtgggg 180

aatttcattt cctttctgaa aaagatatga acataatccc ctcgtttaaa gattcaggtt 240

atattcctga tacgaaggct atgcttattt acgatgaaac agggcaatta gaaaattatt 300

tatcagagat aagtggtgca agaccaaata gacttactga agaaaatgct aattttttgt 360

tgtgtaattt ctctaatcta tggttgatgg gaatcaacgt tctaaaaaga ggagaatatg 420

ctcgttcatt agaactctta tcacaacttc aaaaaaatac actacaactt atacgtatgg 480

cagaaaaaaa tgctgataat tggctaaaca 510

<210> 848
<211> 227
<212> DNA
<213> Staphylococcus aureus

<400> 848
gtgattacag aaatgaaagc agggcacctg aaagatatcg ataaacccag cgaaccattt 60

gaggtgatag gtaagattat accgaggtat gaaaacgaga attggacctt tacagaatta 120

ctctatgaag cgccatattt aaaaagctac caagacgaag aggatgaaga ggatgaggag 180

gcagattgcc ttgaatatat tgacaatact gataaggtaa tatatct 227

<210> 849
<211> 708
<212> DNA
<213> Staphylococcus aureus

<400> 849
gacagatttt cgatccctta atattgaaaa tctttatgct tatcaatttg aaaaaatagc 60

acttattgga ggtaatggta ctggcaaaac cacattactg aatatgattg ctcaaaaaac 120

aaaaccagaa tctggaacgg ttgaaacgga tggcgaaatt caatattttg aacagcttaa 180

catggatgtg gaaaatgatt ttaacacgtt agacggtagt ttaatgagtg aactccatat 240

acctatgcat acaaccgaca gtatgagtgg tggtgaaaaa gcaaaatata aattagctaa 300

tgtcatatca aattatagtc cgatattact tttagatgaa cctacaaatc acttggataa 360

aattggtaaa gattatctga ataatatttt aaaatattac tatggtactt taattatagt 420

aagtcatgat agagcactta tagaccaaat tgctgacaca atttgggata tacaagaaga 480

tggcacaata agagtgttta aaggtaatta cacacagtat caaaatcaat atgaacaaga 540

acagttagaa caacaacgtc aatatgaaca gtatataagt gaaaaacaaa gattgtccca 600

agccagtaaa gctaaacgaa atcaagcgca acaaatggca caagcatcat caaaacaaaa 660

aaataaaagt atagcaccag atcgtttaag tgcatcaaaa caaaaagg 708

<210> 850
<211> 259

<212>  DNA
<213>  Staphylococcus aureus

<400>  850
gatataggat acaaaataga agttgattgg atgccttcac gtatggaact taaacataaa      60

gaatatggat atttagatat tcatcccata aatctaaatg atgatggttc aattactcaa     120

gcaaaccccg aaggtggcaa ttacgttttt caaaatgaat ggttctcaga aactaattat     180

aaaggccgaa aaataccatg tatttcaaaa gaagctcaac ttctttttca ttctggttat     240

gacttaacag aaaaagacc                                                  259


<210>  851
<211>  544
<212>  DNA
<213>  Staphylococcus aureus

<400>  851
catttaacga cgaaactggc taaaataagt aaacaggtaa cgtctattga attagacagt      60

catctattca acttatcgtc agaaaaatta aaactgaaca ttcgtgtcac tttaattcac     120

caagatattc tacagtttca attccctaac aaacagaggt ataaaattgt tgggaatatt     180

ccttaccatt taagcacaca aattattaaa aaagtggttt ttgaaagcca tgcgtctgac     240

atctatctga ttgttgaaga aggattctac aagcgtacct tggatattca ccgaacacta     300

gggttgctct tgcacactca agtctcgatt cagcaattgc ttaagctgcc agcggaatgc     360

tttcatccta aaccaaaagt aaacagtgtc ttaataaaac ttacccgcca taccacagat     420

gttccagata aatattggaa gctatatacg tactttgttt caaaatgggt caatcgagaa     480

tatcgtcaac tgtttactaa aaatcagttt catcaagcaa tgaaacacgc caaagtaaac     540

aatt                                                                  544


<210>  852
<211>  614
<212>  DNA
<213>  Staphylococcus aureus

<400>  852
ccagaaaaac cctaaagaca cgcaaaattt tattacttct aaaaagcatg taaaagaaat      60

attgaatcac acgaatatca gtaaacaaga caacgtaata gaaatcggat caggaaaagg     120

acattttacc aaagagctag tcaaaatgag tcgatcagtt actgctatag aaattgatgg     180

aggcttatgt caagtgacta aagaagcggt aaacccctct gagaatataa aagtgattca     240

aacggatatt ctaaaatttt ccttcccaaa acatataaac tataagatat atggtaatat     300

tccttataac atcagtacgg atattgtcaa aagaattacc tttgaaagtc aggctaaata     360

tagctatctt atcgttgaga agggatttgc gaaaagattg caaaatctgc aacgagcttt     420

gggtttacta ttaatggtgg agatggatat aaaaatgctc aaaaaagtac caccactata     480

ttttcatcct aagccaagtg tagactctgt attgattgtt cttgaacgac atcaaccatt 540

gatttcaaag aaggactaca aaaagtatcg atcttttgtt tataagtggg taaaccgtga 600

atatcgtgtt cttt 614

<210> 853
<211> 525
<212> DNA
<213> Enterococcus faecium

<400> 853
gtccgaatcc tatgaaaatg tatcctatag aaggaaacaa atcagtacaa tttatcaaac 60

ctattttaga aaaattagaa aatgttgagg ttggagaata ctcatattat gattctaaga 120

atggagaaac ttttgataag caaattttat atcattatcc aatcttaaac gataagttaa 180

aaataggtaa attttgctca ataggaccag gtgtaactat tattatgaat ggagcaaatc 240

atagaatgga tggctcaaca tatccattta atttatttgg taatggatgg gagaaacata 300

tgccaaaatt agatcaacta cctattaagg gggatacaat aataggtaat gatgtatgga 360

taggaaaaga tgttgtaatt atgccaggag taaaaatcgg ggatggtgca atagtagctg 420

ctaattctgt tgttgtaaaa gatatagcgc catacatgtt agctggagga aatcctgcta 480

acgaaataaa acaaagattt gatcaagata caataaatca gctgc 525

<210> 854
<211> 467
<212> DNA
<213> Staphylococcus aureus

<400> 854
cattagcagg aggatgtttc tggtgcatgg ttaaaccatt tacatcatat ccaggcatca 60

agtcagtcgt atctggttat agtggcggtc atgttgacaa cccaacttat gaacaggtat 120

gtacgaatca aaccggccat gtcgaagcag tacaaattac gtttgatcca gaggttactt 180

cctttgaaaa tatattagac atatatttca aaacatttga cccaactgat gatcaagggc 240

aatttttcga tagaggcgaa agctatcaac cagtcatttt ctatcatgat gaacatcaga 300

aaaaggctgc tgagtttaaa aagcaacaat taaatgaaca aggtattttc aagaaaccag 360

tgattacacc tattaaacca tataaaaatt tctatccagc tgaagactac catcaagatt 420

attacaaaaa gaacccggta cattattacc aatatcaacg tggttca 467

<210> 855
<211> 451
<212> DNA
<213> Staphylococcus aureus

<400> 855
gcatataaat atcaaaacca tacaagaaat aaaaaatgac tttcaaagaa gaatgaataa 60

agttaaagaa acttatggtg tatcagatga attatggaac agatggaaac aatggttaga 120

aaacgacgaa ctatggcctc gacatgcgac catgatacat ggggacttac atccaggaca 180

tataatggta gataaccaag caaacgtcac aggtctcata gactggactg aagcaaccca 240

ctccgaccca tcaatggact ttatgggaca ccatcgtgta ttcgacgacg aaggattaga 300

gcaactcata acagcatatg gtaaagctgg aggtgaaata tggccacgaa tgaaagagca 360

tataatagaa ctcaatgcag tattcccaat gtttatcgct gagtttgcta tggaatcagg 420

agaatcggcg tatgaaacga tggcattgaa a 451

<210> 856
<211> 505
<212> DNA
<213> Streptococcus pyogenes

<400> 856
ggtcttgtct atggcttcac tattaggttt tttaccctat gcggtctttg gacctgcaat 60

tggtgtgcta gtggatcgtc atgataggaa gaagataatg attggtgctg atttaattat 120

cgcagcagct ggttcggtgc ttactattgt tgcattctat atggagctac ctgtctggat 180

ggttatgata gtattgttta tccgtagcat tggaacagct tttcacaccc cggctctcaa 240

tgcggttacg ccacttttag taccagaaga acagcttacg aaatgtgcag gctatagtca 300

gtctttgcag tctataagct atattgttag tccggcggtt gcagcactct tatactccgt 360

ttgggaacta aatgctatta ttgccatcga tgtattgggt gctgtgattg catctattac 420

ggtagcaatt gtacgtattc ctaagctggg tgatcgcgtg caaagtttgg acccaaattt 480

cataagagaa atgcaagaag gaatg 505

<210> 857
<211> 540
<212> DNA
<213> Escherichia coli

<400> 857
gttgagaatg ggagagactg agccggtcag cagtcccacg agcgcgccca acaacatcag 60

caccggcacg cctggcaact gtgaaagcag aagcgagccc accgcagagc cacaaaatgc 120

caccgccagc cagttctgcg ctgatatccg ggcgccgacc gacgcatgaa tggcaatgcc 180

aaggagacca ccagccccca tcattgagga gaacagcccg agctctgcta cttggcgtcc 240

tgcatctaca aacagcgcag gcatgatgac gctgccgttg gcgccaacga tgcccacgaa 300

gatcatcact ataccaaaga gagggcgcag caggggttcg ctccagagaa aagcgacgcc 360

ggcgcgcatg gagagagtcg ccgtcgtggt catcgtccga gcggcacgcg cgggaagcac 420

ccacgcgccg agcagacctg caaggacgga gcagaacgcc gtcagcccga gcgttggcgc 480

agcgccaagc aggccgattg cggccccccc aagggccggg ccacctagaa tcgcgacgtt     540

<210>  858
<211>  500
<212>  DNA
<213>  Streptococcus pneumoniae

<400>  858
actaagaaaa tcgtagctat ttgggcccag gatgaagagg gtgtgattgg gaaagacaat      60

cgtctacctt ggcatttacc agcagaactg caacacttca aagaaacaac tctgaatcat     120

gctatcttga tggggcgggt aacctttgat ggaatagggc gtcgcttgct tccacaacgg     180

gagactttga ttttgacacg taacctagaa gaaacgatag atggggttgc tacttttcat     240

gatgtccagt ctgtcttgga ctggtatcag gctcaagaaa agaatctcta tattcttggt     300

ggaaagcaga tttttcaggc ttttgaaccc tatcttgacg aagtgattgt gactcacatt     360

catgctcggg tggagggaga tacctatttc cctgaagagt ttgatttgtc tctttttgag     420

acagtttcaa gtaaatttta cgccaaagat gagaagaatc cttatgattt taccatccaa     480

tatcgcaaga gaaaggaagt                                                 500

<210>  859
<211>  423
<212>  DNA
<213>  Staphylococcus aureus

<400>  859
caattacctt ggcacttacc aaatgattta aagcatatta aacaactgac cactgggaat      60

acacttgtaa tggcacggaa aacttttaat tctataggga agccattgcc aaatagacgt     120

aacgtcgtac tcactaacca agcttcattt caccatgaag gggtagatgt tataaactct     180

cttgatgaaa ttaaagagtt atctggtcat gtttttatat ttggaggaca aacgttatac     240

gaagcaatga ttgaccaggt agatgatatg tatatcacag taatagatgg aaagtttcaa     300

ggagacacat tctttccacc atacacattc gaaaactggg aagtcgaatc ttcagtagaa     360

ggtcaactag atgaaaaaaa tactataccg catacattct tacatttagt gcgtagaaaa     420

ggg                                                                   423

<210>  860
<211>  506
<212>  DNA
<213>  Escherichia coli

<400>  860
tggaatgggt agcttcttcg tctttttctc cattgcgccc ggactaatga tgggcaggca      60

aggtgtgtct cagcttggct tcagcctgct gttcgccaca gtggcaattg ccatggtgtt     120

tacggctcgt tttatggggc gtgtgatacc caagtggggc agcccaagtg tcttgcgaat     180

gggaatggga tgcctgatag ctggagcagt attgcttgcc atcaccgaaa tatgggcttc 240

gcagtccgtg ttaggcttta ttgctccaat gtggctagtg ggtattggtg tcgccacagc 300

ggtatctgtg tcgcccaatg gcgctcttcg aggattcgac catgttgctg gaacggtcac 360

ggcagtctac ttctgcttgg gcggtgtact gctaggaagc atcggaacgt tgatcatttc 420

gctgttgccg cgcaacacgg cttggccggt tgtcgtgtac tgtttgaccc ttgcaacagt 480

cgtgctcggt ctgtcttgtg tttccc 506

<210> 861
<211> 530
<212> DNA
<213> Enterococcus faecium

<400> 861
gataaccatc acaaacagaa tgatgtacct gtaaagatag cggtaaatat attgaattac 60

ctttattaat gaattttcct gctgtaataa tgggtagaag gtaattacta ttattattga 120

tatttaagtt aaacccagta aatgaagtcc atggaataat agaaagagaa aaagcatttt 180

caggtatagg tgttttggga aacaatttcc ccgaaccatt atatttctct acatcagaaa 240

ggtataaatc ataaaactct ttgaagtcat tctttacagg agtccaaata ccagagaatg 300

ttttagatac accatcaaaa attgtataaa gtggctctaa cttatcccaa taacctaact 360

ctccgtcgct attgtaacca gttctaaaag ctgtatttga gtttatcacc cttgtcacta 420

agaaaataaa tgcagggtaa aatttatatc cttcttgttt tatgtttcgg tataaaacac 480

taatatcaat ttctgtggtt atactaaaag tcgtttgttg gttcaaataa 530

<210> 862
<211> 535
<212> DNA
<213> Staphylococcus aureus

<400> 862
agaaaattgg gatagaaaag aatattttga acactatttt aaccagcaaa ctacgtatag 60

cattactaaa gaaattgata ttactttgtt taaagatatg agtaaaaaga aaggatatga 120

aatttatcct tctttgattt atgcaattat ggaagttgta aataaaaata aagtgtttag 180

aacaggaatt aatagtgaga ataaattagg ttattgggat aagttaaatc ctttgtatac 240

agtttttaat aagcaaactg aaaaatttac taacatttgg actgaatctg ataacaactt 300

cacttctttt tataataatt ataaaaatga cttgcttgaa tataaagata aagaagaaat 360

gtttcctaaa aaaccgatac ctgaaaacac cctaccgatt tcaatgattc cttggattga 420

ttttagttca tttaatttaa acattggtaa caatagcaac tttttattgc ctattattac 480

gataggtaaa ttttatagtg agaataataa aatttatata ccagttgcct tgcag 535

<210>  863
<211>  632
<212>  DNA
<213>  Proteus mirabilis

<400>  863
ttagcactct atgcgacgat gcaggtgatc ttcgcaccta ttttaggaaa attatcagat      60

aaatatggca gaaaacctat tttattaatt tcgctattgg gtgccgcatt agattaccta     120

ttaatggctt gccccacctc attatggatg ctctacattg gacgaataat tgcgggtata     180

acaggagcca ctggtgcagt atgcgcatca gcaatgactg atgtaactca tcctcatgaa     240

agaacacgct atttcggttt tttgggtggt gcatttggtg tgggtttaat tattggcccc     300

atgttagggg gattactcgg tgagatcagc gcccatacgc catttatctt tgcggctatt     360

tctcattcgt tattatttat attttcatta ctttgtttcc aagaaactca aaccacaaaa     420

atttcgactg aaatatccgc attaaatcag gatacagcgc ctcactctac cactggtttt     480

attaaaaaga gtctcttttt ttggcttatt gcctatttta ttattcaact aatagggcaa     540

attccggcca ctatttgggt gctattcaca caagttcgtt tcgcttggca cactactgaa     600

gtaggtttat ctcttgcatt tcttggtgta tt                                   632


<210>  864
<211>  656
<212>  DNA
<213>  Enterococcus faecalis

<400>  864
cacctgcgag tacaaactgg gtgaacacag cctttatgtt aaccttttcc attggaacag      60

ctgtatatgg aaagctatct gatcaattag gcatcaaaag gttactccta tttggaatta     120

taataaattg tttcgggtcg gtaattgggt ttgttggcca ttctttcttt tccttactta     180

ttatggctcg ttttattcaa ggggctggtg cagctgcatt tccagcactc gtaatggttg     240

tagttgcgcg ctatattcca aaggaaaata ggggtaaagc atttggtctt attggatcga     300

tagtagccat gggagaagga gtcggtccag cgattggtgg aatgatagcc cattatattc     360

attggtccta tcttctactc attcctatga taacaattat cactgttccg tttcttatga     420

aattattaaa gaaagaagta aggataaaag gtcattttga tatcaaagga attatactaa     480

tgtctgtagg cattgtattt tttatgttgt ttacaacatc atatagcatt tcttttctta     540

tcgttagcgt gctgtcattc ctgatatttg taaaacatat caggaaagta acagatcctt     600

ttgttgatcc cggattaggg aaaaatatac cttttatgat tggagttctt tgtggg         656


<210>  865
<211>  554
<212>  DNA
<213>  Enterococcus faecalis

<400>  865
gacaaaggta caacgaggac ggataatacg cttttagaac gtcagagagg aattacaatt      60

cagacaggaa taacctcttt tcagtgggaa aatacgaagg tgaacatcat agacacgcca     120

ggacatatgg atttcttagc agaagtatat cgttcattat cagttttaga tggggcaatt     180

ctactgattt ctgcaaaaga tggcgtacaa gcacgaactc gtatattatt tcatgcactt     240

aggaaaatgg ggattcccac aatctttttt atcaataaga ttgaccaaaa tggaattgat     300

ttatcaacgg tttatcagga tattaaagag aaactttctg ccgaaattgt aatcaaacag     360

aaggtagaac tgtatcctaa tgtgtgtgtg acgaacttta ccgaatctga acaatgggat     420

acggtaatag agggaaacga tgacctttta gagaaatata tgtccggtaa atcattagaa     480

gcattggaac tcgaacaaga ggaaagcata agatttcaga attgttctct gttccctctt     540

tatcatggaa gtgc                                                       554


<210>  866
<211>  404
<212>  DNA
<213>  Enterococcus faecium

<400>  866
tcttatggca gtacgcaacg taaaatcgat tgtgcgctct gtggaaaaac atgatttcag      60

gttggacagc gaccgtggca aggtactcag cgacatgaca gttggtgtgg tgggaacggg     120

ccagataggc aaagcggtta ttgagcggct gcgaggattt ggatgtaaag tgttggctta     180

tagtcgcagc cgaagtatag aggtaaacta tgtaccgttt gatgagttgc tgcaaaatag     240

cgatatcgtt acgcttcatg tgccgctcaa tacggatacg cactatatta tcagccacga     300

acaaatacag agaatgaagc aaggagcatt tcttatcaat actgggcgcg gtccacttgt     360

agatacctat gagttggtta aagcattaga aaacgggaaa ctgg                      404


<210>  867
<211>  250
<212>  DNA
<213>  Enterococcus faecium

<400>  867
gtgcggtatt gggaaacagt gccgcgttag ttgttggcga ggtggaccaa atcaggctgc      60

agtacggaat ctttcgtatt catcaggaag tcgagccgga aaaaggctct gaaaacgcag     120

ttataaccgt tcccgcagac ctttcagcag aggagcgagg acggatacag gaaacggcaa     180

aaaaaatata taaagcgctc ggctgtagag gtctagcccg tgtggatatg tttttacaag     240

ataacggccg                                                            250


<210>  868
<211>  663
<212>  DNA

<213>  Enterococcus faecium

<400>  868
aagtgtgggc attactgttt ttggatgcga acaggatgag gcaaatgctt tccgcgcttt      60

atcgccggat tttcatatta tccctacgct gattagcgat gcgatatcgg cagacaacgc     120

aaaattggcc gctggcaatc aatgcgttag cgtaggccat aagtccgagg tttccgaggc     180

gacaattctt gcgctgagaa aggtcggggt aaaatacatt tctacccgca gcatcggctg     240

cgatcacatt gatacgactg ccgccgagag aatgggaatc tcggttggca cggttgcgta     300

ttcgccggac agcgttgcgg attatgcttt gatgctgatg ctgatggcca tacggggtgc     360

aaaacccacc atgcacgccg tggcgcaaca agatttcaga ttggatcgta tccgggggaa     420

agaactgggg gatatgactg tgggagttat tggaaccggc catatcgggc aagcggtcgt     480

caaaaggctg cggggatttg gatgccatgt gctggcctat gataacagcc gaaaaatgga     540

tgcagattat gtccagcttg atgagcttct aaaaaacagc gatattgtta cgctccatgt     600

gccgctttgt gcggataccc gccatctgat cggtcagaag caaattggag agatgaagca     660

agg                                                                   663


<210>  869
<211>  572
<212>  DNA
<213>  Enterococcus faecium

<400>  869
acgagaatta tacggttttc aaatactata ccgccaaaga agcattggaa tgtatagaca      60

agtctgagat tgaccttgcc atattggaca tcatgcttcc cggcacaagc ggccttacta     120

tctgtcaaaa aataagggac aagcacacct atccgattat catgctgacc gggaaagata     180

cagaggtaga taaaattaca gggttaacaa tcggcgcgga tgattatata acgaagccct     240

ttcgcccact ggagttaatt gctcgggtaa aggcccagtt gcgccgatac aaaaaattca     300

gtggagtaaa ggagcagaac gaaaatgtta tcgtccactc cggccttgtc attaatgtta     360

acacccatga gtgttatctg aacgagaagc agttatccct tactcccacc gagttttcaa     420

tactgcgaat cctctgtgaa aacaagggga atgtggttag ctccgagctg ctatttcatg     480

agatatgggg cgacgaatat ttcagcaaga gcaacaacac catcaccgtg catatccggc     540

atttgcgcga aaaaatgaac gacaccattg at                                   572


<210>  870
<211>  280
<212>  DNA
<213>  Enterococcus faecium

<400>  870
gaattctact tgtcgaggat gatgatcata tctgtaatac agtaagggcg tttctggctg      60

aggcaggata tcaggtggat gcctgcacag atggaaatga ggcatacacc aagttttacg 120

aaaacactta tcaactggtt attcttgata ttatgctgcc cggtatgaac gggcatgaac 180

ttttgcgtga atttcgtgcg aaaaatgata ctcccattct gatgatgaca gccctgtcgg 240

atgacgaaaa ccaaatccgg gcgtttgatg cagaggcaga 280

<210> 871
<211> 564
<212> DNA
<213> Enterococcus faecium

<400> 871
aatgatccga gggaaacttg gggattggat cttaagtatt ttggaaaaca aatatgactt 60

aaatcacctg gacgcgatga aattatatca atattccata cggaacaata tagatatctt 120

tatttatgtg gcgattgtca ttagtattct tattctatgt cgcgtcatgc tttcaaaatt 180

cgcaaaatac tttgacgaga taaataccgg cattgatgta cttattcaga acgaagataa 240

acaaattgag ctttctgcgg aaatggatgt tatggaacaa aagctcaaca cattaaaacg 300

gactctggaa aagcgagagc aggatgcaaa gctggccgaa caaagaaaaa atgacgttgt 360

tatgtacttg gcgcacgata ttaaaacgcc ccttacatcc attatcggtt atttgagcct 420

gcttgacgag gctccagaca tgccggtaga tcaaaaggca aagtatgtgc atatcacgtt 480

ggacaaagcg tatcgactcg aacagctaat cgacgagttt tttgagatta cacggtataa 540

cctacaaacg ataacgctaa caaa 564

<210> 872
<211> 595
<212> DNA
<213> Enterococcus faecium

<400> 872
acatgagttg gaggaaacac agcgatattt ctttgcggca gcttctcatg agctaaaaac 60

gcccatcgcg gctacaagcg ttctgttgga gggaatgctt gaaaatatcg gtgactacaa 120

agatcattct aagtatctgc gcgaatgcat caaaatgatg gataggcagg gcaaaatcat 180

ttccgaaata ctggagcttg tcagcctgaa tgatgggaga atcgtaccca tagctgaacc 240

gttggacata gggcgcacgg ttgccgagtt gctgcccgat tttcaaacct tggcagaggc 300

aaacaaccag cggttcgtca cagatattcc agccgggcaa attgtcctgt ccgatccgag 360

gctgctccaa aaggcactat ccaatgtcat attgaatgca gttcagaaca cgccgcaggg 420

aggcgaggta cggatatgga gtgagcctgg tgctgaaaaa tgccgccttt ttgttttgaa 480

catgggcgtt cacattgatg atactgcgct tccaaggctg ttcaccccat tctatcgcat 540

tgatcaggcg cgaagcagaa aaagtgggcg aagcggttta ggacttgcca tcgta 595

<210>  873
<211>  598
<212>  DNA
<213>  Enterococcus faecium

<400>  873
ggcagcaaag accttaaacg gcttattgat aagaccgggg gaaacctttt ctttctggtg      60

gctggtacgc catgcggaca aagatacccc ctataaagac ggccttacgg tgaccaatgg     120

taaactcacc accatgtcgg gcggcggtat gtgccagatg agcaatttac tattttggat     180

gttcctgcat acgccattga caattatcca gcgcagaggt cacgaagtaa aggagtttcc     240

agagccaaac agtgacgaga ttaaaggggt ggatgcaacc atttcagagg gctggattga     300

tttaaaagtg cgaaacgata ccgactgcac ctaccaaata tgggtgacct tagatgatga     360

gaaaatcatc ggtcaggtgt ccgccgacaa agagccccaa gcattataca aaattacaaa     420

tggcagtatc cagtatgtcc gtgaaagtgg cgggatttat gaatatgccc aggttaaacg     480

gatgcaagtt gccttaggta ccggggaaat aatagattgc aagctgcttt atacaaacaa     540

atgcaaaatc tgttatcccc tcccggaaag tgtggatatt caggaggaga accaatga       598


<210>  874
<211>  673
<212>  DNA
<213>  Enterococcus faecalis

<400>  874
gaagatggaa caattcaagg attcatggaa accattaata tgccttatgt aggcgcgggt      60

gtcttagcta gcgttaacgc aatggacaaa atcatgacga aatatctttt acaaactgtt     120

ggcattccac aagtaccatt cgtgccagtt ttaagaagtg actggaaagg aaatccaaaa     180

gaagtctttg aaaaatgtga aggttcttta atttatccgg tctttgttaa acctgccaat     240

atgggttcta gtgtcggaat tagcaaagtg gaaaatcgtg aagaattgca agaagcattg     300

gaagaagctt tccgttatga tgcccgagca attgttgaac aagggatcga agcacgtgaa     360

attgaagtag ccattttagg aaatgaagat gtccgtacga ctttacctgg tgaagtggtg     420

aaagatgtcg ctttctatga ttatgatgca aaatacatca ataacacgat tgaaatgcaa     480

atcccagcgc atgttccaga agaagtagct catcaagcgc aagaatacgc taaaaaagcg     540

tatattatgt tagatggaag tggcttaagt cgctgtgatt tcttcttaac aagcaaaaac     600

gaattattcc tgaatgaatt gaacaccatg cctggtttta ctgactttag tatgtatcct     660

ttactgtggg aaa                                                        673


<210>  875
<211>  360
<212>  DNA
<213>  Staphylococcus aureus

<400> 875
tacagtctat ccgggcattg ccagtcgggg atattaaaaa gagtataggt ttttattgcg 60

ataaactagg tttcactttg gttcaccatg aagatggatt cgcagttcta atgtgtaatg 120

aggttcggat tcatctatgg gaggcaagtg atgaaggctg gcgctctcgt agtaatgatt 180

caccggtttg tacaggtgcg gagtcgttta ttgctggtac tgctagttgc cgcattgaag 240

tagagggaat tgatgaatta tatcaacata ttaagccttt gggcattttg caccccaata 300

catcattaaa agatcagtgg tgggatgaac gagactttgc agtaattgat cccgacaaca 360


<210> 876
<211> 508
<212> DNA
<213> Enterococcus faecium

<400> 876
tgggataact tcacaggaaa accggtggat gggtatgagg tgaatcgcat catcggcaca 60

aaggccgtgg cgtttgctct gcgcgaggca caaatccatg cggctgcgct tggctatggc 120

ttgcttttat gggatggata tcggccaaga actgcggtgg actgcttcct gcgttgggca 180

gcgcaaccgg aggacaatct cacaaaagaa aaattttacc ccaatataga gcgagccgag 240

ttgattacaa agggttatgt ggcctcacaa tccagccata gccgtggaag cgcaattgat 300

cttacgctct accacctgga tacaggggaa cttgtttcaa tgggaagtaa cttcgatttt 360

atggacgaac ggtcgcatca tacagcaaaa gggatagggg atgcagaggc acaaaatcga 420

agatgcttgc gtaaaatcat ggaaagcagc ggatttcagt cttatcgctt tgaatggtgg 480

cactataagt tgattgatga gccatacc 508


<210> 877
<211> 551
<212> DNA
<213> Enterococcus faecium

<400> 877
atacttaggt tatgactacg ttaatgaagc actgttttct caggaaaaag tcgaatttca 60

aaattatgat caaaatccca aagaacattt agaaaatagt gggacttctg aaaataccca 120

agagaaaaca attacagaag aacaggttta tcaaggaaat ctgctattaa tcaatagtaa 180

atatcctgtt cgccaagaaa gtgtgaagtc agatatcgtg aatttatcta aacatgacga 240

attaataaat ggatacgggt tgcttgatag taatatttat atgtcaaaag aaatagcaca 300

aaaattttca gagatggtca atgatgctgt aaagggtggc gttagtcatt ttattattaa 360

tagtggctat cgagactttg atgagcaaag tgtgctttac caagaaatgg gggctgagta 420

tgccttacca gcaggttata gtgagcataa ttcaggttta tcactagatg taggatcaag 480

cttgacgaaa atggaacgag cccctgaagg aaagtggata gaagaaaatg cttggaaata 540

cgggttcatt t                                                          551

<210>  878
<211>  552
<212>  DNA
<213>  Enterococcus faecium

<400>  878
gtgcgttcat tatttcgttc acagtctgca cgctgttttt ggggtggaga ctggcttccg      60

tattggaggc aacacagata ccgcccatcc ctgcaactca tacaggcagc agcactgacg     120

tagtggagaa tttggaggaa aacgctcttg ccaccgccaa agaacaggga gatgaacagg     180

aatggagcct gattttagtg aacaggcaga accccatccc cgcacagtac gatgtggagc     240

ttgagcaact atcaaatggt gagcggatag atattcggat ttctccctat cttcaagatt     300

tgtttgatgc cgcaagaact gatggagttt acccgattgt cgcatccgga taccgaacaa     360

cagaaaaaca gcaagaaatt atggatgaaa aaattgccga atataaggcg aaaggctaca     420

cctctgcaca ggctaaagcg gaagcagaaa cttgggtggc cgtgccggga acgagcgagc     480

atcagcttgg tcttgctgtg gatatcaatg cggacggaat tcattcaaca ggcaacgagg     540

tttatagatg gc                                                         552

<210>  879
<211>  542
<212>  DNA
<213>  Enterococcus faecalis

<400>  879
ttgtctggta tcccctatgt aggctgcgat attcaaagct ccgcagcttg catggacaaa      60

tcactggcct acattcttac aaaaaatgcg ggcatcgccg tccccgaatt tcaaatgatt     120

gaaaaaggtg acaaaccgga ggcgaggacg cttacctacc ctgtctttgt gaagccggca     180

cggtcaggtt cgtcctttgg cgtaaccaaa gtaaacagta cggaagaact aaacgctgcg     240

atagaagcag caggacaata tgatggaaaa atcttaattg agcaagcgat ttcgggctgt     300

gaggtcggct gcgcggtcat gggaaacgag gatgatttga ttgtcggcga agtggatcaa     360

atccggttga gccacggtat cttccgcatc catcaggaaa acgagccgga aaaaggctca     420

gagaatgcga tgattatcgt tccagcagac attccggtcg aggaacgaaa tcgggtgcaa     480

gaaacggcaa agaaagtata tcgggtgctt ggatgcagag ggcttgctcg tgttgatctt     540

tt                                                                    542

<210>  880
<211>  457
<212>  DNA
<213>  Enterococcus faecium

<400>  880

aggattgcta gctttatatt tagtgacact aatctggtta gtgttattca aattacaata     60

caatatttta tcagtattta attatcatca aagaagtctt aacttgactc catttactgc    120

tactgggaat ttcagagaga tgatagataa tgttataatc tttattccat ttggcttgct    180

tttgaatgtc aattttaaag aaatcggatt tttacctaag tttgcttttg tactggtttt    240

aagtcttact tttgaaataa ttcaatttat cttcgctatt ggagcgacag acataacaga    300

tgtaattaca aatactgttg gaggctttct tggactgaaa ttatatggtt taagcaataa    360

gcatatgaat caaaaaaaat tagacagagt tattattttt gtaggtatac ttttgctcgt    420

attattgctc gtttaccgta cccatttaag aataaat                             457


<210>  881
<211>  360
<212>  DNA
<213>  Enterococcus flavescens

<400>  881
aagctgcctt atgtaggttg cggggtggcc ggttctgcct tatgtatgaa caaatggctg     60

ctgcatcaag ctgcagcagc cattggcgta caaagtgctc ctacgattct cttgacaaat    120

caagccaacc agcaagaaca aatcgaagct tttatccaga cccatggctt tccagttttc    180

tttaagccta atgaagcggg ctcctcaaaa gggatcacta aagtcacctg cgttgaagaa    240

atcgcttctg ccttaaaaga agcctttact tattgttccg cagtgctcct acaaaaaaat    300

attgccggtg ttgagatcgg ttgcggtatt ttgggcaacg actctttgac tgtcggtgct    360


<210>  882
<211>  459
<212>  DNA
<213>  Enterococcus faecium

<400>  882
gacatacgag ttggctgaat cgcttttgaa ggcaaaagaa ctggctgcta cccaagggta     60

cggattgctt ctatgggacg gttaccgtcc taagcgtgct gtaaactgtt ttatgcaatg    120

ggctgcacag ccggaaaata acctgacaaa ggaaagttat tatcccaata ttgaccgaac    180

tgagatgatt tcaaaaggat acgtggcttc aaaatcaagc catagccgcg gcagtgccat    240

tgatcttacg ctttatcgat tagacacggg tgagcttgta ccaatgggga gccgatttga    300

ttttatggat gaacgctctc atcatgcggc aaatggaata tcatgcaatg aagcgcaaaa    360

tcgcagacgt ttgcgctcca tcatggaaaa cagtgggttt gaagcatata gcctcgaatg    420

gtggcactat gtattaagag acgaaccata ccccaatag                           459


<210>  883
<211>  500
<212>  DNA
<213>  Proteus mirabilis

<400>  883
cctttgaagc tggtactgac cctgatattg cgcaagtcca agtgcaaaat aaattgcaat      60

tggcaatgcc tcttttacct caggaagtac aacaacaagg gattagtgtc gataaatctt     120

ctagttcatt cttaatggtt gcaggtttta tctctggtga tggctcgatg tcacaagatg     180

acatcgccga ctatgtaggt gcaacaatta aagatccatt aagccgtgtc acaggggtgg     240

gtgaaacgca gttatttggt acacaatacg caatgcgtat ttggttagat ccagataaac     300

tggtgaaata taacatgacc acacttgatg ttattaatgc gattaaatcg caaaataacc     360

aagtggcggc aggccaatta ggtggtacgc caccagtgcc tggtcagcgt ttaaatgtat     420

ctatcattgc gcaaactcga cttaatacac ctgagcaatt tgctgatatt ctgatgaaag     480

tcaatcaaga cggttcacag                                                 500


<210>  884
<211>  280
<212>  DNA
<213>  Pseudomonas aeruginosa

<400>  884
tgtcgaagtt tttcattgat aggcccattt tcgcgtgggt gatcgccttg gtgatcatgc      60

tcgcgggcgg cctgtcgatc ctcaatctgc cggtcaacca gtacccggcc atcgccccgc     120

cggccatcgc cgtgcaggtg agctacccgg gcgcctcggc cgagacggtg caggacaccg     180

tggtccaggt gatcgagcag cagatgaacg ggatcgacaa tctgcgctac atctcctcgg     240

agagtaactc cgacggcagc atgaccatca ccgtgacctt                           280


<210>  885
<211>  477
<212>  DNA
<213>  Staphylococcus aureus

<400>  885
caatggttac aggttgtgga agaactttct ccttttaaag ctggcttata cctattacct      60

atggcaatag gagctatggt gtttgcacca attgcacccg gattagcggc gcgatttgga     120

ccgaaaatag tgttaccttc cggaattgga attgcagcca ttggcatgtt tattatgtat     180

ttctttggtc atccattatc atattctaca atggctttag cattaatttt agttgaagct     240

ggtacggctt cactagcagt tgcatctgct ctaataatgt tagaaacacc tacatcaaaa     300

gcaggtaatg cagctgctgt tgaagagtct atgtatgacc ttggaaatgt ttttggtgta     360

gcagtacttg gtagcctatc ttctatgctt tatcgtgtat ttttagatat ttcatctttt      420

tcatcaaaag gtatagttgg agatttagct catgtagctg aagaatctgt agtgggc        477


<210>  886
<211>  584

<212>  DNA
<213>  Escherichia coli

<400>  886
ctcttagacg ccctgtccga tcagatgcac cgtgtttcaa tcgacagctt ccaaccggaa      60

acccagcgct atgcgctcaa gcgcggcgtg ggctacctga acgatatcca aggatttcct     120

gaccctgcgc tctatcccga tattgctgag gcggactgca ggctggtggt tatgcactca     180

gcgcagcggg atggcatcgc cacccgcacc ggtcaccttc gacccgaaga cgcgctcgac     240

gagattgtgc ggttcttcga ggcgcgggtt tccgccttgc gacggagcgg ggtcgctgcc     300

gaccggctca tcctcgatcc ggggatggga tttttcttga gccccgcacc ggaaacatcg     360

ctgcacgtgc tgtcgaacct tcaaaagctg aagtcggcgt tggggcttcc gctattggtc     420

tcggtgtcgc ggaaatcctt cttgggcgcc accgttggcc ttcctgtaaa ggatctgggt     480

ccagcgagcc ttgcggcgga acttcacgcg atcggcaatg gcgctgacta cgtccgcacc     540

cacgcgcctg gagatctgcg aagcgcaatc accttctcgg aaac                      584


<210>  887
<211>  784
<212>  DNA
<213>  Escherichia coli

<400>  887
catcgtcaac ataacctcgg acagtttctc cgatggaggc cggtatctgg cgccagacgc      60

agccattgcg caggcgcgta agctgatggc cgagggggca gatgtgatcg acctcggtcc     120

ggcatccagc aatcccgacg ccgcgcctgt ttcgtccgac acagaaatcg cgcgtatcgc     180

gccggtgctg gacgcgctca aggcagatgg cattcccgtc tcgctcgaca gttatcaccc     240

cgcgacgcaa gcctatgcct tgtcgcgtgg tgtggcctat ctcaatgata ttcgcggttt     300

tccagacgct gcgttctatc cgcaattggc gaaatcatct gccaaactcg tcgttatgca     360

ttcggtgcaa gacgggcagg cagatcggcg cgaggcaccc gctggcgaca tcatggatca     420

cattgcggcg ttctttgacg cgcgcatcgc ggcgctgacg ggtgccggta tcaaacgcaa     480

ccgccttgtc cttgatcccg gcatggggtt ttttctgggg gctgctcccg aaacctcgct     540

ctcggtgctg gcgcggttcg atgaattgcg gctgcgcttc gatttgccgg tgcttctgtc     600

tgtttcgcgc aaatcctttc tgcgcgcgct cacaggccgt ggtccggggg atgtcggggc     660

cgcgacactc gctgcagagc ttgccgccgc cgcaggtgga gctgacttca tccgcacaca     720

cgagccgcgc cccttgcgcg acgggctggc ggtattggcg gcgctgaaag aaaccgcaag     780

aatt                                                                  784


<210>  888
<211>  344
<212>  DNA

<213>  Staphylococcus lugdunensis

<400>  888
gaggtgtaat tatgattcag actattgtaa ctgctgctat ctttatattg cgcaagcatt      60

agacttatta gtgattttat taatgttctt tgctagagca aagactagga aagaatatcg     120

agatatttat attggtcaat atgtaggatc tgtggcatta attgtcataa gtttattctt     180

tgcctttgtc ttaaattatg ttcctgaaaa atggatatta ggattattag ggttaatacc     240

gatttattta ggaattaaag tggctattta tggtgatagt gacggagaag agagagctaa     300

aaaagaattg aatgaaaagg gattgtctaa attagttggt acga                      344


<210>  889
<211>  503
<212>  DNA
<213>  Pseudomonas aeruginosa

<400>  889
ctcgacccga tctacgtcga cgtcacccag ccgtccaccg ccctgttgcg catgcgccgc      60

gaactggcca gcggccagtt ggagcgcgcc ggcgacaacg ctgcgaaggt ctccctgaag     120

ctggaggacg gtagccaata cccgctggaa ggccgcctcg aattctccga ggtttccgtc     180

gacgaaggca ccggctcggt caccatccgc gccgtgttcc ccaacccgaa caacgagctg     240

ctgcccggca tgttcgttca cgcgcagttg caggaaggcg tcaagcagaa ggccatcctc     300

gctccgcagc aaggcgtgac ccgcgacctc aagggccagg ctaccgcgct ggtggtgaac     360

gcgcagaaca aggtcgagct gcgggtgatc aaggccgacc gggtgatcgg cgacaagtgg     420

ctggtcaccg aaggcctgaa cgccggcgac aagatcatta ccgaaggcct gcagttcgtg     480

cagccgggtg tcgaggtgaa gac                                             503


<210>  890
<211>  503
<212>  DNA
<213>  Proteus mirabilis

<400>  890
tgtcatcata gctcttaaca taatcgcggc tcttcttaaa tcaaggttgg caggaagttg      60

ttttttttcg atacagcgag ataaagattg ctctattcta gagtaatcgg ctgcacataa     120

ttctcggcgg atttcaacaa ttggtgtcat ttcaccaaca aattcgcact tatggaaata     180

tatttctaga agtgcattat gtttcggatc ttcgacaatt gatgtcaata tgtaaataag     240

caattctctt aatacaaata gtggatcatc tggatatttt gattgatact ctaattctaa     300

tgattctatt tttaagtcgg tgagttcaca cgcttcagta aataaatcca ctttattctt     360

aaagtgccaa tatattgcac ctcgagttac tccggcctcg gttgcaatat ctgaaagtga     420

tgtggcagaa acaccttgca cagtaaatag cctaagtgca gcatcaataa tctgctgtct     480

tgtctcttgt gcttggcgtt tag                                              503

<210>  891
<211>  343
<212>  DNA
<213>  Enterococcus faecalis

<400>  891
gaccaggagt tggtggtttt attgcttatt taggaattcg cgctccattt tttgcggccg      60

catttttagc gtttattggt tttattttga cattaactgt tttgaaggag ccagagaaac     120

gaattttagc cgctgttgaa gcgaaaaaag gttcatttat ggatatttta agaaatccaa     180

tgtttacctc attatttgtg attatcttaa tttcctcttt tggcctgcaa gcgttcgaat     240

ctatttatag tattatggcg accattaatt ttggctttac cacaagtgaa atagcaatcg     300

tgattacggt tagtggtatt ttagcgttga tttgtcagct gtt                       343

<210>  892
<211>  544
<212>  DNA
<213>  Proteus mirabilis

<400>  892
ctggctctgt tagtgctttc aggcagcttg gttgttgctg gatgtggcga caaaaatcag      60

tctgctggag gtccacctcc tgctcctgct gtaggtgttg ttacattaga tgcgaaacca     120

ctgactatca caacagactt acctggtcgt acatctgctt atcgtatcgc agaggttcgc     180

cctcaggttg gcggcatcat cttaaaacgc aattacaccg aaggtagtta tgtagaagca     240

ggaacatctt tataccaaat cgatcctgct atttttcaag ctacattaaa cagtgctcaa     300

gctgatttag caaaagcgaa agcgaatgct gaaattgctc gtctgactgt agagcgctat     360

aaacctctac tcggcaccaa ttatgtcagt aaacaagatt ttgataccgc aacatctcag     420

tacgctcaag ctgttgctgc agtaaaagca gctgaagcta cagtgactaa tgcaaaaatt     480

aatcttgaat ataccaaagt caccgcacca atttctggcc gttcaggtaa atcaacggta     540

acag                                                                  544

<210>  893
<211>  573
<212>  DNA
<213>  Proteus vulgaris

<400>  893
cctgaaatcc actactgacc ggctccagcc gctgacctta gatacctgcc agcaagctaa      60

ccccgaactg accgcccgcg cagcgtttag catgaatgtc cgaacgtttg tgctggtgaa     120

agataaaaaa acattctgtt catctgcgac cggtgagatg gacattccac tcaatgaatt     180

gattccggcg ctcgacatta ataaaaacgt cgatatggcg atcttacccg gcacgccgat     240

ggtgccgaac aaacccgcaa tcgtcatctg gtatcgcaac cctttgctga aaaatagcgg 300

cgtctttgcc gctctgaatc tcaacctgac gccttcactc ttttatagtt cacggcagga 360

agattacgat ggcgtcgccc tcattattgg caatactgcg ctatctacct tttcttcacg 420

tttgatgaac gttaacgaat taaccgacat gccagtccgt gaaactaaaa ttgcgggcat 480

tcctctgacc gttcggcttt atgcagatga ctggacatgg aacgatgtgt ggtacgcatt 540

tttactgggc ggcatgagtg gaactgtcgt tgg 573

<210> 894
<211> 581
<212> DNA
<213> Streptococcus mutans

<400> 894
gaaatgatat tgacgggact ttcataaaaa ttttcaagga cttgaggtgt aataatatct 60

tttttaggac cttgagccac tattttacct ctacgaagga ggaggatatg actcatttta 120

tcagtgattt cttcagcatg gtgggtaaca taaaggatag ttggagcatg tggtaactca 180

gtaatctttt caacttgtgt tagcaatttt tcacgggcaa aaagatccag tccgctggtt 240

gcttcatcca aaataatgat ttcaggatct tccataaggc tgcgcgcaat aaggaggagt 300

tgtttttcac cttgtgagag gctgctatag atgcgaccaa gcaagtgttt tccgccgatg 360

acagtaagca tttggcgtgc ttcattaagt tctgtttcgt cgtattcctt gtagagaatg 420

cttgatttgt atttaccagt tagcacgatc ttttcagcca acatatttgc agggagtcgc 480

tcagcaataa aagagcccac gacaccgatt ttagtccgca tattgggaat atcaccctga 540

ccaaacctag tattgagaat ttcaacctgt ccttgtgttg a 581

<210> 895
<211> 281
<212> DNA
<213> Escherichia coli

<400> 895
aaggctggct ttttcttgtt atcgcaatag ttggcgaagt aatcgcaaca tccgcattaa 60

aatctagcga gggctttact aagcttgccc cttccgccgt tgtcataatc ggttatggca 120

tcgcatttta ttttctttct ctggttctga aatccatccc tgtcggtgtt gcttatgcag 180

tctggtcggg actcggcgtc gtcataatta cagccattgc ctggttgctt catgggcaaa 240

agcttgatgc gtggggcttt gtaggtatgg ggctcataat t 281

<210> 896
<211> 609
<212> DNA
<213> Staphylococcus aureus

<400> 896

attagaaatt gcgactggtg caatcactgc aggtacatta attgcaatga tattttatgt 60

tattcagtta tctatgcctt taatcaatct ttccacgtta gttacagatt ataaaaaggc 120

agtcggtgca agtagtagaa tatacgaaat catgcaagaa cctattgaac cgacagaagc 180

tcttgaagat tctgaaaatg tattaattga tgacggtgta ttgtcatttg aacatgtaga 240

ctttaaatat gatgtgaaga aaatattaga tgatgtgtcg ttccaaatcc cacaaggtca 300

agtgagtgct tttgtaggcc cttctgggtc tggtaaaagt acgatattta atctgataga 360

acgtatgtat gaaattgagt caggtgatat taaatatggc cttgaaagtg tctatgatat 420

cccgttatct aagtggcgac gcaaaattgg atatgttatg caatcaaatt cgatgatgag 480

tggtacaatt agagacaata ttttatacgg aattaatcgt catgtttcag atgaagaact 540

tattaattat gctaaattag cgaactgtca tgattttatc atgcaatttg atgaaggata 600

tgacacgct 609

<210> 897
<211> 274
<212> DNA
<213> Staphylococcus aureus

<400> 897
ttggatagtt caacaaaaac attaacagaa gataaacagg tttaccgtgt ggagggtttc 60

tcgtgtgcga attgtgctgg gaagtttgaa aaaaatgtaa aagaactatc aggggtgcat 120

gatgctaaag tcaatttcgg agcttccaaa attgatgtct ttggcagtgc aaccgttgaa 180

gatctggaaa aggctggtgc tttcgagaat cttaaagtgg caccagagaa ggctagaaga 240

agggtcgaac cagtggtaac agaagataaa aatg 274

<210> 898
<211> 532
<212> DNA
<213> Klebsiella oxytoca

<400> 898
tgagcagcgt aaccagacat ggctggagtt ggtgggggaa gcgcagcagc tcatgggcga 60

acgctgcccg gcagatgagc cgcgggcgat tgcgctggca acccgctgga tggagcagct 120

ggagcaggat accgccggca ggccggagtt tctgactcgc ctgaatgaga tgcacgccgc 180

cgaaccgcag atgcgtgaac aaactggggt gacgccggag atgattgatt tcattacccg 240

tgcttttgcc gaaagcaagc tggccatctg ggcgcgctat ctgaacgccg aagagctggc 300

ctttacccgg cagcactatt tcgatcgcct gatggagtgg ccggcgctgg tggccgacct 360

gcatcgggcc tgtcgtgaga agcgagaccc ggcctccccg gaaggtcagc agctggcgca 420

gcgctggctg gcgctgttcc agtcttacgc gggtaaagat gcgcagacgc agcagaagtt 480

tcgctatgcc atggagcagg agccgcattt gatgaaagga acgtggatga ct 532

<210>  899
<211>  500
<212>  DNA
<213>  Klebsiella pneumoniae

<400>  899
atgaaaaacc ctcatcaaaa actttcatca tcaccattag cagtggtgcg cagtattgcc      60

actcactgga gtattattct gcagatggct aaacgtgatg ttgttggaag atataaaggt     120

tctgtgatgg gcctgctttg gtcttttttg aaccctttat ttatgttaac agtatatact     180

tttgtcttct ccgtggtatt caaagccaga tggtcaactg gtggggacga aagtaggaca     240

cagtttgcta taattttatt tgtcggaatg atagttcatg gttttttaag tgaagtggta     300

aataaagcgc cgttgattat tttgggaaat acaaactatg tgaagaaagt tatatttcca     360

ttggaaacgc tgcctgttat ctctttattt gcggcattat ttcatacttg tatcagcctt     420

tgtgtgttac tgatggcgtt tttcattttt aatggatatt tacattggac catagtgttt     480

ttacctttgg tctttttccc                                                 500


<210>  900
<211>  370
<212>  DNA
<213>  Enterococcus faecium

<400>  900
agaacatatc cgcaaacaag gagaaggaat ccttctctct ccgaaagtaa gctttcaagt      60

atatcagcaa aagggttatc aaatgacatc tgaagaatcc atcattcgtt ttgtcatgag     120

acaaacagag ttttcagaat cgcttgtccg tagtttgctg aatcacttag gggttgctca     180

ggaaactctg acgaaaccgt tatgtacatt aagtggggga gaagcgaccc gtctgacgat     240

tgctttgctt tttactaagc caagtaatgt gttgctgtta gatgaaccga ctaattttat     300

tgatatggca acgatcgaag ctttagagaa gctaatgcaa atatatccgg gaacgatttt     360

gtttacatca                                                            370


<210>  901
<211>  400
<212>  DNA
<213>  Escherichia coli

<400>  901
aaccgtttat acgttggtga gttggttggc cattctggga tactggttgc tcattgcagg      60

cgtaacttta cgcattctaa tgaaacgacg cgcagttccc tccgcgatgg cctggctgtt     120

gattatttac attctgccgt tagtcggaat tattgcctat cttgccgttg gcgagctcca     180

tttaggcaaa cgccgcgctg agcgcgccag agcgatgtgg ccttccaccg caaaatggct     240

taacgacctt aaagcctgta agcatatctt cgccgaagaa aatagcagtg tcgctgcgcc     300

attattcaag ctttgcgagc gtcgtcaggg gatcgctggg gtcaaaggga atcagctaca 360

actgatgacc gagtcagatg atgtgatgca ggcgttaatc 400

<210> 902
<211> 540
<212> DNA
<213> Klebsiella pneumoniae

<400> 902
atgttctcgc tgcagttctg gcctatgaga aagggatgat cctggccaac gataaaccag 60

agcctacaaa acttgcagag aaccgctctt ctgaaacttg cagtttggaa gacctcaaaa 120

gcattcagtt acatactgct aatgaagaaa ttggggaaaa acgttttggt actgcgcgtg 180

ctattattaa aaatcttacc atctacaaat cagatggtac gactttgaca gagaaaccac 240

tcatcaaatc aggtgaagaa gttacatttg atttcaccat attagctacc gaagagatta 300

aggatgttgc tcttggcctt tccatatcca aagctcaggg aggggatatt tggggagata 360

gtaatattgg cgcaggttca ccaattacac ttcgtccagg tagtcagcgt atcgtttata 420

aagcaacgct gcctataaat tcgggcgatt acctaataca ctgcggcctc gctatggttg 480

gcaacggtgc tcgagaggag cttgatcaac gtcgcccgat gatgaaaata aagttttggt 540

<210> 903
<211> 770
<212> DNA
<213> Klebsiella pneumoniae

<400> 903
ataaagcaat gaagcctaaa gttatcgctt ctattgtatt atttaatcat tcctatgatg 60

atattaaaga tacgttcctc tcattatgcc atgaagagag cgttgaaaaa ataatcttcg 120

ttgataatgg tggttgtcag tgggcggcat cattgaatga acctaaggtg agctacatca 180

agtctcctta caactgtggt tttggtgctg gacataatct tgcaataaaa gcaagtgcag 240

actttgacgg ttattttctt atatgtaatc cggatataag ctttgataag cagtcacttg 300

ataaattagt ttcgtttgcg tgggaaaatg agtatagttt tttgttttcc ccgcaaataa 360

tatatagaaa tggtgagaga caatatagtt gccgtgtact acctactccc ggtaatcttt 420

taagacgttt ctttccagtg actgcaataa agtacgatgt taaatatgaa ctgaaagatg 480

cagcctatga tgagatattt tccccaccaa cggtatgtgg ctgtttcatg ttattaagta 540

atgtattatt gcaaaaactt aacggttttg atgaacgata ctttatgtat ctggaagatg 600

tagatttatg tcgccgagca ttacagctaa ccaaaatata ctattatcct ggaacaacta 660

ttgtccatgc ctttaataaa ggttcgtata aaagcaaatt attactttgg taccatattc 720

gctccgcagt ttcctatttt aataaatggg gatggttcct tgatcgtaaa 770

<210>  904
<211>  614
<212>  DNA
<213>  Staphylococcus aureus

<400>  904
ggttacttgt tgctgctttt gcgttatctc aaatgattat atcgccgttt ggtggtacgc      60

tagctgacaa attagggaag aaattaatta tatgtatagg gttaatttta ttctcagtgt     120

cagaatttat gtttgcagtt ggccacaatt tttcggtatt gatgttatcg agagtgattg     180

gtggtatgag tgctggtatg gtaatgccgg gtgtgacagg tttgattgca gatgtttcac     240

caagccatca aaaagcaaaa aactttggct acatgtcagc gattatcaat tctggattca     300

ttttaggacc agggattggt ggatttatgg cagaagtttc acatcgtatg ccattttatt     360

ttgcaggtgc attaggcatt ttagctttca taatgtcagt tgtattgatt catgatccga     420

aaaagtctac gacaagcggc ttccaaaaac ttgagcccca attattaaca aaaattaatt     480

ggaaagtctt tattacgcca gcaattttaa cgctcgtctt agcgttcggt ttatcggcat     540

ttgaaacact gtattcttta tatacatcgt ataaagtaaa ttattcacct aaagatattt     600

cgattgcgat tacg                                                       614


<210>  905
<211>  411
<212>  DNA
<213>  Pseudomonas aeruginosa

<400>  905
gaactacccc gtgaatcccg acctgatgcc cgcgctgatg gcggtcttcc agcatgtgcg      60

gacgcgcatc cagagcgagc tcgattgcca gcgactcgac ctgaccccgc ccgacgtcca     120

tgtattgaag cttatcgacg aacaacgcgg gctgaacctg caggacctgg gacgccagat     180

gtgccgcgac aaggcactga tcacccggaa gatccgcgag ctggagggaa gaaacctggt     240

ccgccgcgag cgcaacccca gcgaccagcg cagcttccag ctcttcctca ccgacgaggg     300

gctggccatc caccagcatg cggaggccat catgtcacgc gtgcatgacg agttgtttgc     360

cccgctcacc ccggtggaac aggccaccct ggtgcatctc ctcgaccagt g              411


<210>  906
<211>  401
<212>  DNA
<213>  Escherichia coli

<400>  906
gcaaggaccg ttctatcatg gaaccaaagc caatttggcg attggtgact tgctaaccac      60

agggttcatc tctcatttcg aggacggtcg tattcttaag cacatctact tttcagcctt     120

gatggagcca gcagtttggg gagctgaact tgctatgtca ctgtctggcc tcgagggtcg     180

cggctacata tacatagttg agccaacagg accgttcgaa gacgatccga atcttacgaa 240

caaaaaattt cccggtaatc caacacagtc ctatagaacc tgcgaaccct tgagaattgt 300

tggcgttgtt gaagactggg aggggcatcc tgttgaatta ataaggggaa tgttggattc 360

gttagaggac ttaaagcgcc gtggtttaca cgtcattgaa g 401

<210> 907
<211> 742
<212> DNA
<213> Staphylococcus aureus

<400> 907
tacgatgaca ccagtctttg aattgaaaaa tgtcaattac tactatgatc ataaaaaagt 60

gttagaaaat ataaacatta aaataaataa aggtgaattt ttagcaattg ttggaccaaa 120

tggtgctggt aaatcaacat tattgaagtt gattctaggg ttattacctt tacaaagtgg 180

tgagattttt gttggaggta ttgattttaa aaataagaaa acatccatta aattaagcta 240

tgtatcacaa aaagcaaatg cctttaattc aggtttccca gcaagtgtta aagaagttgt 300

tttaagcgga ttaacaaaga caaaacgtct tttccaaaca tttaatagca aagataatga 360

aaaagtgatt aaagtactag aaagactgaa tataagtgat ttaattcata aaaatatagc 420

agaattatca ggtggtcaac aacaacgtgt aatgattgct cgagcattga tttcagaacc 480

tgcagtatta gtacttgatg aaccaacgaa tggtattgat gcaaaacatg taagtgaatt 540

ttataatact ttagatcaat taaaacaaga aggtatcacc attatcttag ttactcatga 600

tatcggtgtt gtagcagata ctgctactga agtagcatgt ttaaataagc atttgcattt 660

ccatggtaca actgatgagt ttaaatcact tgatgaagtt gaaatttcaa aaatttatgg 720

acatcctgta cgttttgtcg at 742

<210> 908
<211> 352
<212> DNA
<213> Staphylococcus aureus

<400> 908
tagcaacctc cctttgatac aagaaagctt tttctacaag ctgtttaaca tgctcatcat 60

ctagtgaata atagactact tttccttctt tacggtattt tgctatacct aaatttttca 120

ataatcttaa atgatgggat gccgtagccg ttgaagattc aatgatatta gctacatcac 180

aaacacataa ctctccctct aaagacaaaa cataagcaat tttaactctt gtatcatctg 240

atagagcctt aaaaactttc gctacatcca taggattctg tttagcaagg tcttttttag 300

ccctgtttac cttatcttca tgaatatagg taacttcaca catatctttt gt 352

<210> 909
<211> 583

<212>  DNA
<213>  Enterococcus faecalis

<400>  909
gcgtagaacg tggacttgat ccattgaaga caatgcttgt ggtcatgagc aattctgaaa      60

attcaggtgg cctggtactc gctgcttccc ctatggcaaa aaaagtatta ggtatttcca     120

atgttacaag gaaaaatgaa gttccggacc acccaaactt aattattgta cctccacgca     180

tgaaattata catgaagaaa aatcaagaaa ttaacaattt atataaccgc tttgtttcta     240

atgaagatca ttctgtattc agtgtcgatg aatcgtttct tgatgtgact gcttcgctga     300

cctattttaa gtgtgacacc gcctataaac tggccaagat tattcaacgt gtgatttata     360

accatatggg attgtatgta acaatcggaa ttggggaaaa tccgttgctg gccaagttag     420

cattggataa tgaagcaaag aatgcaccag gctttgtggc tgaatggcgc tatgaagatg     480

tgccagaaaa agtttggcca atctcccctc ttacagaatt ttgtgggata ggaaatcgca     540

tggctgctcg cttaaaaaag ctaggtattc ggtccattta tga                       583


<210>  910
<211>  231
<212>  DNA
<213>  Candida albicans

<400>  910
atggcttgtt ctgctgctca atgtgtctgt gctcaaaaat ccacttgttc atgtggtaaa      60

caaccagctt taaaatgtaa ttgttctaaa gcttcagtag aaaatgttgt tccatcatca     120

aatgatgctt gtgcttgtgg aaaaagaaat aaatcaagtt gtacttgtgg tgctaatgct     180

atttgtgatg gtactagaga tggtgaaact gatttcacta acttgaaata a              231


<210>  911
<211>  240
<212>  DNA
<213>  Candida albicans

<400>  911
ctaagatgtc gtcgcaagat gaatctaaat tagaaaaggc aattagtcaa gactcttcct      60

cagaaaacca ttccattaat gaataccacg ggtttgatgc ccatacaagt gaaaacattc     120

agaatttagc cagaactttc actcatgatt ctttcaaaga tgactcgtca gcaggtttat     180

tgaaatactt aacccatatg tcagaagtgc ccggggtcaa tccatatgaa catgaagaaa     240


<210>  912
<211>  513
<212>  DNA
<213>  Candida albicans

<400>  912
gctgaattat ctaaattacc aagagataat gatccagaag cacttttgaa atatgctgca      60

ccactttgga aacaatactt attggtcagt tggagaacta ttgttcaaga ttggagatca 120

ccaggatata tttattctaa aatctttttg gttgtttcag cagcattatt taatggattt 180

tcatttttca aagctaaaaa caacatgcaa ggtttacaaa atcaaatgtt ttcggtgttt 240

atgtttttca ttccatttaa tactttggtg caacaaatgt taccatactt tgtgaagcaa 300

cgtgatgttt atgaagtgag agaagctcca tcaagaacat tcagttggtt tgcatttatt 360

gccggtcaaa ttacatcaga aattccttat caagttgccg ttggtaccat agcatttttc 420

tgttggtatt atccattagg attgtataat aatgctacac caactgattc tgtcaatcct 480

cgaggtgttt taatgtggat gcttgttact gca 513

<210> 913
<211> 609
<212> DNA
<213> Candida albicans

<400> 913
ggtggtaaat taagagattt ggtcatccgt gatgctccac tcaaacaaca attattgcaa 60

gaagctaaaa ctttacctgc tttgacgtta actgctagac aattatgtga tttagaatta 120

attttaaacg gagggttttc tccattaact ggattcttaa atcaagaaga ttataatagt 180

gttgttaacg atttaagatt aagtagtgtt aagaatgaat caaatggtaa aggtttatta 240

tggccaatcc caatcacctt agatgttgat gagaccactt ctaaaaaaca ttctgttggt 300

gatagaattg tattaataga tttgagagat gaaactccat tggccatttt aactattgaa 360

tctatttata aacctgataa aaaattagaa gcaaaaaaag tgttccgtgg tgatccagaa 420

catcctgcta ataaatattt attagaaacc gctggcgatt attatatcgg tggtgaatta 480

caagggatca attatcctaa acattatgat tatgttgatg ctagaaaaac accaactgaa 540

ttgagacaag aatttgaaaa attgggttgg gctcaagaaa atattgttgc ctttcaaacc 600

agaaatcct 609

<210> 914
<211> 528
<212> DNA
<213> Candida albicans

<400> 914
tcatggatta tttgttcgtg gtgctaacca aatggatggg ccagagatgg ttacccagtg 60

tcctatccca cctggtgaaa catacttgta caacttcact gttactgatc aagtgggaac 120

ttattggtat catagccata cagggggtca gtatggagac ggtatgagag gtgtctttat 180

tattgaagat gatgatttcc cgtatcacta cgatgaagaa gttgttttaa ctttaagtga 240

ccattaccac aaatattcag gtgacatagg gcctgccttt ttaaccagat ttaatccgac 300

aggagcagaa ccgatccctc agaacttttt gttcaatgaa acaagaaatg ccacttggaa 360

ggtcgaacct ggaaaaactt actttgttag gattcttaat gttggtggtt ttgtatcaca 420

gtacttgtgg atggaagatc atgaatttac tattgttgag atcgatggcg tttacgttga 480

aaaaaacacc actgatttga tttatatcac agttgctcaa agatatgg 528

<210> 915
<211> 585
<212> DNA
<213> Candida albicans

<400> 915
aaacggtcca gagttgaaga aaaagttgta tcgtcagatt tggctagggg cggggctagg 60

tgttcttatt tgtataatca ttggtggcgc ttttattggt accttttacg ggttgggtaa 120

agatatctgg ggaaaatcag aagacttgtg ggaagggata ttttgtatca ttgccacagt 180

cttgatcact gctatgggta ttccaatgtt gagaatcaac aagatgaaag aaaaatggag 240

agttaaatta gcacaagctt taatcaaatc tccagaaaat aagaagaacc gattcaaatt 300

gggatatctt gggaaaaagt acgcactttt tattttgcca ttcatcactt gcttgcgtga 360

aggtttagaa gctgttgttt tcgttggtgg ggtcggtatt actagtcctg cttcatcttt 420

cccaatccca gttattgttg gtataatttg tggtcttgca gtgggtgcct tgttgtacta 480

ctttggttcc aatatgtcga tgcaaatctt cttgatcatc tccacttgta tcttgtactt 540

gatcgctgct ggtttgttct ccagaggtgt ctggttcttt gagag 585

<210> 916
<211> 560
<212> DNA
<213> Candida albicans

<400> 916
tgttattggt tatggtgttg gcccattggt tttcagtccg atgtcagaaa atgctatatt 60

tggtcgtaca tccatatata tcataacatt atttttattt gtcatactac aaatccccac 120

tgctttggta aataatattg ccggtttatg tatattgaga ttcttgggtg gattctttgc 180

tagtccttgt ttggccactg gtggtgctag tgttgctgat gtggttaaat tttggaattt 240

accagttggg ttagccgctt ggagtttggg tgctgtttgt ggtcctagtt ttggtccatt 300

ctttggttca attttaactg tcaaagccag ttggagatgg actttttggt tcatgtgtat 360

tatttctggg ttttcatttg ttatgttgtg tttcacttta cctgaaactt ttggcaaaac 420

attattgtat cgcaaggcta aaagattgag agccatcacc ggtaacgaca gaatcacaag 480

tgaaggagaa attgaaaata gcaaaatgac aagtcatgaa ttgatcattg atacattatg 540

gagaccatta gaaatcaccg 560

<210> 917

<211>  574
<212>  DNA
<213>  Candida albicans

<400>  917
attccttggt ttggttctgc agcttcatat ggtcaacaac cttatgaatt tttcgaatca      60

tgtcgtcaaa agtatggtga tgtattttca tttatgttat tagggaaaat tatgacggtt     120

tatttaggtc caaaaggtca tgaatttgtt ttcaatgcta aattatctga tgtttctgct     180

gaagatgctt ataaacattt aactactcca gttttcggta aaggggttat ttatgattgt     240

ccaaattcta gattaatgga acaaaaaaaa tttgctaaat ttgctttgac tactgattca     300

tttaaaagat atgttcctaa gattagagaa gaaattttga attattttgt tactgatgaa     360

agtttcaaat tgaaagaaaa aactcacggg gttgccaatg ttatgaaaac tcaaccagaa     420

attactattt tcactgcttc aagatcttta tttggtgatg aaatgagaag aatttttgac     480

cgttcatttg ctcaattata ttctgattta gataaaggtt ttacccctat taattttgtt     540

ttccctaatt tacctttacc tcattattgg agac                                 574


<210>  918
<211>  647
<212>  DNA
<213>  Candida albicans

<400>  918
gctctttgct tcaattatcc gaagctgaag atgaatctgt ctacaaggcc agctttgatg      60

acaccgtgca agaaattgat ctgttattga ttgctttcaa agacctcctt agacttttac     120

gacccaaaga taaatccaac aaattcgata catacgaatt gaaatttcat tctttgaagc     180

acaaattgcg tgagttgcaa gtatttatta atgatcaaca acaagacaag ttgcatgaat     240

ataggataaa gcatttccat ctacaagatc tgcctgtgga taccatcaat aacgaatttg     300

ctcgagacca attatttgct gatcgttcca ctaagaagac taagaaagaa atggaagcat     360

ctataaatca acaaattgtc agccaaaata aacaaataac aaaatccttg caagcatcga     420

gacaattgtt atcagcaggt atattgcaga gtgaattgaa cattgacaac attgatcagc     480

aaaccaagga tttatacaag ttaaatgaag gatttatcca attcaacgat ttgttaaata     540

gatctaagaa aattgtcaag tttattgaaa agcaagataa agctgaccgt caacgtatat     600

atttgagtat ggggttcttc atactttgtt gttcttgggt ggtttat                   647


<210>  919
<211>  552
<212>  DNA
<213>  Glycine max

<400>  919
atccaagttg aaagagataa attgaacaag tatggtcgtc ccctattagg atgtactatt      60

aaacctaaat tggggttatc cgctaagaat tatggtagag ctgtttatga atgtcttcgt 120

ggtggacttg attttaccaa agatgatgaa aatgtgaatt cccaaccatt tatgcgttgg 180

agagaccgtt tcttattttg tgccgaagcc atttttaaat cacaggctga aacaggtgaa 240

atcaaagggc attacttgaa tgcaactgcg ggtacatgcg aagaaatgat gaaaagagct 300

gtatttgcca gagaattagg cgttcctatc gtaatgcatg attatttaac agggggattc 360

actgcaaata ctagcttagc tcattattgc cgagataatg gtctacttct tcatatacac 420

cgtgcaatgc atgcagttat cgacagacaa aagaatcatg gtatgcactt tcgtgtacta 480

gctaaagcat tacgtttgtc tggtggagat catgttcacg ccggtaccgt agtaggtaaa 540

cttgaagggg aa 552

<210> 920
<211> 358
<212> DNA
<213> Homo sapiens

<400> 920
gctcaagggg caaatgcagc atgtacagca ttggcagtgg tgcctcagag gtggcagaac 60

tatttcacac aaaccagttt aggactacac aaattagtac catccagcat caggatatag 120

ctgtggattt tacaaaccat tcctatttct aacttcagga attgatgttt ttcccagtcc 180

atcttaaaat attactgctt taatcacaga tcagataaaa aggatatcag gcacaacctc 240

caactaaagt cctgttgtag catagacagt gaaatgctat gacatcagaa gactttaaaa 300

ttgcagctct tttcggatcc cccaaagtgt gtctgcacgc ttcttcaaac gggcctct 358

<210> 921
<211> 271
<212> DNA
<213> Homo sapiens

<400> 921
cggagtcaac ggatttggtc gtattggatg cctggtcacc agggttgctt ttaactctgg 60

aaaagtggat attgtcgcca tcaatgaccg cttcattgac ctcaactaca tggtctacat 120

gttccagtag gattttacct atggcaaatt ccatgcaccg taaaggctga gaatgggaag 180

cttgtcatca gtggaaatcc cattaccatc ttccaggagc gagatccctc caaaatcaaa 240

tggggcaatg ctgacgctga gtacgttgtg g 271

<210> 922
<211> 239
<212> DNA
<213> Homo sapiens

<400> 922
atggataatg atatcgccac gctcgtcatg gacaatggct ctcccatgtg caaggccagc 60

ttagcaggcg acgatgcccc tccatcgtga ggcacccatg gcaccagggc atgatcgtgg 120

gcatgggtca gaagaagtcc tacgtggaca atgaggccca gggcaagaga agcatcctga 180

ccctgaaata ccctatcgag catggcattg tcaccaatgg agaagatctg gcaccacac 239

<210> 923
<211> 365
<212> DNA
<213> Homo sapiens

<400> 923
gccaaattgc caaaactcaa gtcacctcag taccatccag gaggctgggt attgtcctgc 60

ctctgccttt tctgtctcag cgggcagtgc ccagagccca caccccccca agagccctcg 120

atggacagcc tcacccaccc cacctgggcc cagccaggag ccccgcctgg ccatcagtat 180

ttattgcctc cgtccgtgcc gtccctgggc cactggcctg gcgcctgttc ccccaggctc 240

tcagtgccac cacccccggc aggccttccc tgacccagcc aggaacaaac aagggaccaa 300

gtgcacacat tgctgagagc cgtctcctgt gcctcccccg ccccatcccc ggtcttcgtg 360

ttgtg 365

<210> 924
<211> 342
<212> DNA
<213> Homo sapiens

<400> 924
caccctggat ttgcatacat tcttcaagat cccatttgaa ttttttagtg actaaaccat 60

tgtgcattct agagtgcata tatttatatt ttgcctgtta aaaagaaagt gagcagtgtt 120

agcttagttc tcttttgatg taggttatta tgattagctt tgtcactgtt tcactactca 180

gcatggaaac aagatgaaat tccatttgta ggtagtgaga caaaattgat gatccattaa 240

gtaaacaata aaagtgtcca ttgaaaccgt gatttttttt tttttcctgt catactttgt 300

taggaagggt gagaatagaa tcttgaggaa cggatcagat gt 342

<210> 925
<211> 552
<212> DNA
<213> Glycine max

<400> 925
atccaagttg aaagagataa attgaacaag tatggtcgtc ccctattagg atgtactatt 60

aaacctaaat tggggttatc cgctaagaat tatggtagag ctgtttatga atgtcttcgt 120

ggtggacttg attttaccaa agatgatgaa aatgtgaatt cccaaccatt tatgcgttgg 180

agagaccgtt tcttattttg tgccgaagcc atttttaaat cacaggctga aacaggtgaa 240

atcaaagggc attacttgaa tgcaactgcg ggtacatgcg aagaaatgat gaaaagagct 300

gtatttgcca gagaattagg cgttcctatc gtaatgcatg attatttaac agggggattc    360

actgcaaata ctagcttagc tcattattgc cgagataatg gtctacttct tcatatacac    420

cgtgcaatgc atgcagttat cgacagacaa aagaatcatg gtatgcactt tcgtgtacta    480

gctaaagcat tacgtttgtc tggtggagat catgttcacg ccggtaccgt agtaggtaaa    540

cttgaagggg aa                                                        552


<210>  926
<211>  286
<212>  DNA
<213>  Pseudomonas aeruginosa

<400>  926
caggcctaac acatgcaagt cgagcggatg aagggagctt gctcctggat tcagcggcgg     60

acgggtgagt aatgcctagg aatctgcctg gtagtggggg ataacgtccg gaaacgggcg    120

ctaataccgc atacgtcctg agggagaaag tgggggatct tcggacctca cgctatcaga    180

tgagcctagg tcggattagc tagttggtgg ggtaaaggcc taccaaggcg acgatccgta    240

actggtctga gaggatgatc agtcacactg gaactgagac acggtc                   286


<210>  927
<211>  643
<212>  DNA
<213>  Enterococcus faecium

<400>  927
aggataggta ggagccgtag aaatcggaac gctagtttcg atggaggcgc tggtgggata     60

ctacccctgc gttatggcca ctctaacccg caccactaat cgtggtggga gacagtgtca    120

gatgggcagt ttgactgggg cggtcgcctc ctaaaaggta acggaggcgc ccaaaggttc    180

cctcagaatg gttggaaatc attcgaagag tgtaaaggca gaagggagct tgactgcgag    240

accaacaagt cgagcaggga cgaaagtcgg gcttagtgat ccggtggttc cgcatggaag    300

ggccatcgct caacggataa aagctaccct ggggataaca ggcttatctc ccccaagagt    360

ccacatcgac ggggaggttt ggcacctcga tgtcggctcg tcgcatcctg gggctgtagt    420

cggtcccaag ggttgggctg ttcgcccatt aaagcggcac gcgagctggg ttcagaacgt    480

cgtgagacag ttcggtccct atccgtcgcg ggcgttggaa atttgagagg agctgtcctt    540

agtacgagag gaccgggatg gacttaccgc tggtgtacca gttgttctgc caaggttttt    600

gctgggtagc tatgtaggga agggataaac gctgaaagca tct                      643


<210>  928
<211>  245
<212>  DNA
<213>  Streptococcus pyogenes

<400>  928

gcgtgagtga aagaaggttt tcggatcgta aagctctgtt gttagagaag aatgatggtg      60

ggagtggaaa atccaccaag tgacggtaac taaccagaaa gggacggcta actacgtgcc     120

agcagccgcg gtaatacgta ggtcccgagc gttgtccgga tttattgggc gtaaagcgag     180

cgcaggcggt tttttaagtc tgaagttaaa ggcattggct caaccaatgt acgctttgga     240

aactg                                                                 245


<210>  929
<211>  240
<212>  DNA
<213>  Streptococcus pneumoniae

<400>  929
ccacactggg actgagacac ggcccagact cctacgggag gcagcagtag ggaatcttcg      60

gcaatggacg gaagtctgac cgagcaacgc cgcgtgagtg aagaaggttt tcggatcgta     120

aagctctgtt gtaagagaag aacgagtgtg agagtggaaa gttcacactg tgacggtatc     180

ttaccagaaa gggacggcta actacgtgcc agcagccgcg gtaatacgta ggtcccgagc     240


<210>  930
<211>  242
<212>  DNA
<213>  Streptococcus agalactiae

<400>  930
cacggcccag actcctacgg gaggcagcag tagggaatct tcggcaatgg acggaagtct      60

gaccgagcaa cgccgcgtga gtgaagaagg ttttcggatc gtaaagctct gttgttagag     120

aagaacgttg gtaggagtgg aaaatctacc aagtgacggt aactaaccag aaagggacgg     180

ctaactacgt gccagcagcc gcggtaatac gtaggtcccg agcgttgtcc ggatttattg     240

gg                                                                    242


<210>  931
<211>  250
<212>  DNA
<213>  Enterococcus faecium

<400>  931
gtgcattagc tagttggtga ggtaacggct caccaaggcc acgatgcata gccgcacctg      60

agagggtgat cggccacatt gggactgaga cacggcccaa actctacggg aggcagcagt     120

agggaatctt cggcaatgga cgaaagtctg accgagcaac gccgcgtgag tgaagaaggt     180

tttcggatcg taaaactctg ttgttagaga agaacaagga tgagagtaac tgttcatccc     240

ttgacggtat                                                            250


<210>  932
<211>  263
<212>  DNA

<213>  Enterococcus faecium

<400>  932
tgcctataca tgcaagtcga acgcttcttt ttccaccgga gcttgctcca ccggaaaaag      60

aggagtggcg aacgggtgag taacacgtgg gtaacctgcc catcagaaag ggataacact     120

tggaaacagg tgctaatacc gtataacaaa tcaaaaccgc atggttttga tttgaaaggc     180

gctttcgggt gtcgctgatg gatggacccg cggtgcatta gctagttggt gaggtaacgg     240

ctcaccaagg ccacgatgca tag                                             263


<210>  933
<211>  267
<212>  DNA
<213>  Enterococcus faecalis

<400>  933
ggcgtgccta atacatgcaa gtcgaacgct tctttcctcc cgagtgcttg cactcaattg      60

gaaagaggag tggcggacgg gtgagtaaca cgtgggtaac ctacccatca gagggggata     120

acacttggaa acaggtgcta ataccgcata acagtttatg ccgcatggca taagagtgaa     180

aggcgctttc gggtgtcgct gatggatgga cccgcggtgc attagctagt tggtgaggta     240

acggctcacc aaggcgacga tgcatag                                         267


<210>  934
<211>  200
<212>  DNA
<213>  Klebsiella pneumoniae

<400>  934
caggcctaac acatgcaagt cgagcggtag cacagagagc ttgctctcgg gtgacgagcg      60

gcggacgggt gagtaatgtc tgggaaactg cctgatggag ggggataact actggaaacg     120

gtagctaata ccgcataatg tcgcaagacc aaagtggggg accttcgggc ctcatgccat     180

cagatgtgcc cagatgggat                                                 200


<210>  935
<211>  635
<212>  DNA
<213>  Staphylococcus aureus

<400>  935
acacggtcca gactcctacg ggaggcagca gtagggaatc ttccgcaatg ggcgaaagcc      60

tgacggagca acgccgcgtg agtgatgaag gtcttcggat cgtaaaactc tgttattagg     120

gaagaacata tgtgtaagta actgtgcaca tcttgacggt acctaatcag aaagccacgg     180

ctaactacgt gccagcagcc gcggtaatac gtaggtggca agcgttatcc ggaattattg     240

ggcgtaaagc gcgcgtaggc ggttttttaa gtctgatgtg aaagcccacg gctcaaccgt     300

ggagggtcat tggaaactgg aaaacttgag tgcagaagag gaaagtggaa ttccatgtgt     360

agcggtgaaa tgcgcagaga tatggaggaa caccagtggc gaaggcgact ttctggtctg 420

taactgacgc tgatgtgcga aagcgtgggg atcaaacagg attagatacc ctggtagtcc 480

acgccgtaaa cgatgagtgc taagtgttag ggggtttccg ccccttagtg ctgcagctaa 540

cgcattaagc actccgcctg gggagtacga ccgcaaggtt gaaactcaaa ggaattgacg 600

gggacccgca caagcgtgga gcatgtggtt taatt 635

<210> 936
<211> 243
<212> DNA
<213> Enterococcus faecalis

<400> 936
gcattagcta gttggtgagg taacggctca ccaaggcgac gatgcatagc cgacctgaga 60

gggtgatcgg ccacactggg actgagacac ggcccagact cctacgggag gcagcagtag 120

ggaatcttcg gcaatggacg aaagtctgac cgagcaacgc cgcgtgagtg aagaaggttt 180

tcggatcgta aaactctgtt gttagagaag aacaaggacg ttagtaactg aacgtcccct 240

gac 243

<210> 937
<211> 274
<212> DNA
<213> Staphylococcus hominis

<400> 937
cgtgcctaat acatgcaagt cgagcgaaca gacgaggagc ttgctccttt gacgttagcg 60

gcggacgggt gagtaacacg taggtaacct acctataaga ctgggataac ttcgggaaac 120

cggagctaat accggataat atttcgaacc gcatggttcg atagtgaaag atggctttgc 180

tatcacttat agatggacct gcgccgtatt agctagttgg taaggtaacg gcttaccaag 240

gcaacgatac gtagccgacc tgagagggtg atcg 274

<210> 938
<211> 200
<212> DNA
<213> Staphylococcus haemolyticus

<400> 938
acacgtgggt aacctaccta taagactggg ataacttcgg gaaaccggag ctaataccgg 60

ataatatttc gaaccgcatg gttcgatagt gaaagatggt tttgctatca cttatagatg 120

gacccgcgcc gtattagcta gttggtaagg taacggctta ccaaggcgac gatacgtagc 180

cgacctgaga gggtgatcgg 200

<210> 939
<211> 287

<212>  DNA
<213>  Enterococcus faecium

<400>  939
cctttaggtg tattggtagg agagcgttct aagggcgtcg aaggcagatc gtgaggactg      60

ctggagcgct tagaagtgag aatgccggta tgagtagcga aagacaggtg agaatcctgt     120

ccaccgaatg actaaggttt cctggggaag gctcgtccgc ccagggttag tcgggaccta     180

agccgaggcc gacaggcgta ggcgatggat aacaggttga tattcctgta cccgttgttt     240

ttgtttgagc aatggaggga cgcaggaggc taaggaatgc agacgat                   287


<210>  940
<211>  281
<212>  DNA
<213>  Proteus mirabilis

<400>  940
caggcctaac acatgcaagt cgagcggtaa caggagaaag cttgctttct tgctgacgag      60

cggcggacgg gtgagtaatg tatggggatc tgcccgatag agggggataa ctactggaaa     120

cggtggctaa taccgcataa tgtctacgga ccaaagcagg ggctcttcgg accttgcact     180

atcggatgaa cccatatggg attagctagt aggtggggta aaggctcacc taggcgacga     240

tctctagctg gtctgagagg atgatcagcc acactgggac t                         281


<210>  941
<211>  200
<212>  DNA
<213>  Proteus vulgaris

<400>  941
tggttgatca tggctcagat tgaacgctgg cggcaggcct aacacatgca agtcgagcgg      60

taacaggaga aagcttgctt tcttgctgac gagcggcgga cgggtgagta atgtatgggg     120

atctgcccga tagaggggga taactactgg aaacggtggc taataccgca tgacgtctac     180

ggaccaaagc aggggctctt                                                 200


<210>  942
<211>  309
<212>  DNA
<213>  Staphylococcus aureus

<400>  942
tctctgatgt tagcggcgga cgggtgagta acacgtggat aacctaccta taagactggg      60

ataacttcgg gaaaccggag ctaataccgg ataatatttt gaaccgcatg gttcaaaagt     120

gaaagacggt cttgctgtca cttatagatg gatccgcgct gcattagcta gttggtaagg     180

taacggctta ccaaggcaac gatgcatagc cgacctgaga gggtgatcgg ccacactgga     240

actgagacac ggtccagact cctacgggag gcagcagtag ggaatcttcc gcaatgggcg     300

aaagcctga                                                          309

<210>  943
<211>  183
<212>  DNA
<213>  Klebsiella oxytoca

<400>  943
ctaacacatg caagtcgaac ggtagcacag agagcttgct ctcgggtgac gagtggcgga      60

cgggtgagta atgtctggga aactgcccga tggaggggga taactactgg aaacggtagc     120

taataccgca taacgtcgca agaccaaaga gggggacctt cgggcctctt gccatcggat     180

gtg                                                                183

<210>  944
<211>  548
<212>  DNA
<213>  Mus musculus

<400>  944
ataggtcggc ggttcatgcc ccccatgcag gagctattac acatgtactt gtagtggatg      60

gtggtatact cagagccggc ctgggggaag acacaggatc caggtgaagt cgctccctac     120

ctcactacag gtgacctgca gcagccggga atggctggct atagcctcta ataagtttca     180

gttttagttg tagagtaggg atattccacc tgttcggcac acctgctgga gctgtggggc     240

ccaacacttg cttagcatgg gagggaaccg gactcagcgt ctctatttcc cgcctggatg     300

gggaagcccc ttctcccaga gactgctgtt aaagtagacc ctgggctggg cacggcagct     360

tgcacctcta agcctagcta gcactcagga gggtgaggca aacgggttgc tagaaagtca     420

acatcagtct aggctggagt caactgtctc taagacgcac aaaccaaaac aaaattacag     480

acctcgggtg gctcataagg taccaccacg ctgtggcgaa aagtctgcct gtcttccaga     540

tactcggg                                                           548

<210>  945
<211>  577
<212>  DNA
<213>  Dictyostelium discoideum

<400>  945
attggagaag gtgcagcagg agaagtattt gtagcaacat catcaaagaa caataagaga      60

gttgcaatta aaaagattga aattaacaat gataatgcca aactcttggt aacagagatt     120

gcaattatga agacatcaca tcatgataac attgtaaatt acattgatag ttatatagtg     180

aacgatagag aactttgggt tgcaatggag tttatgggtg gtggttgttt aacagacatt     240

ttagaggcat ttgataatat caaaatgagt gagattcaaa ttgcttatgt ggttaaagag     300

accttgaagg cattgcaata cattcatagt cttcatcgta ttcatagaga tataaagagt     360

gataatattt tattgggctc agagggtagt gtaaagattg ctgattttgg ttatgccgct     420

caattaactc aaaaacaaca aaaacgtaat accgtcgttg gtacccctta ttggatggcc     480

ccagaactca ttagaggtca cgattatggt gtcaaggttg atatttggtc tttgggtatc     540

atgatgatgg aaatggctga aggtgaacca ccttata                              577


<210>  946
<211>  963
<212>  DNA
<213>  Mus musculus

<400>  946
ggaagccaca ctgctacaca gggttgtcaa acgactttga ccttccccaa gatgtgaggg      60

catagtaact ataacatcag gttcctggaa aatttagctg aaagacagaa gcaagcactg     120

aacatggccc cgagatctac gtaccgggta cccattgaga ggctggaaat acaggctggt     180

gtctgtaatg cacacatgcc cagggttcgt cactagaggt gttaccatct ctgctttgca     240

ctccatgtgc agcttttcag aaacactttg gaacctgcaa agatgtttcc agcaaaagta     300

agaaaagtta ggaaccaatc cactgcctcg gcttaacctc agtgtgagtg agcttgctta     360

cctgccgaaa cactacagtc atcaagtggc tgcttaaata aggttgacat gctttcaaat     420

ggcacaatgg aggcctggct tgtttattaa ttgaagagct tacatatcac agaaacaaat     480

gtctcctaac tgatcagtcc tgaagctctt tctccatcat gtgacttcct acttttatat     540

tgttaagacc gacttttaga gcctacataa agagcagcgg tgctacgttt tatgccagca     600

ctaggaggaa gctgagacag gagcttgggc cacagagaaa gaccctaccc ttagcacact     660

tcctttatca gggttagaac acataattac aattgctttt aggtcagttt catttctcca     720

tataaaacca ctcaaagatg ccttttctac tcctaaaatg cttaactaaa aaataactcc     780

atttctgatt tgtgaattta aaaagtagtg tggaaacaac taaattatca atattcttgg     840

atgattactt tgttaaataa ctggattaac agtaaatctc agggtctaga agtgcagctc     900

ggtgctagag cagcgctgat catgctggag cctggctcag tcctggcacc gagataacta     960

agg                                                                   963


<210>  947
<211>  538
<212>  DNA
<213>  Dictyostelium discoideum

<400>  947
ccaccggttt aacttggaat ccaaaaggtg gagatgctaa atctgctaca ccagcaccag      60

cttcatcagc accagccgcc ccagttgcac cagctgtttc atccactcca gttgaatcaa     120

agaaaggtcc aggtttaggt gcagttttcg gtgaacttag caaaggtgat ggtgttacca     180

gtggtttaaa aaaagttacc aacgatatga aatccaaaaa tttcaccgac aaatcatcag     240

ttgttaaagc tgctgatact aaagtcgcca aagttgatgc tccatctaga ccagccgttt 300

ttgctctcca aggtaacaaa tggtccattg aatatcaagt taacaacaaa gaaattgtca 360

ttgccgagcc agatagtcgt caaactgttt acattttcca atgtgtaaac tctttagttc 420

aaatcaaagg taaagttaat gcaattactc ttgatggttg taaaaagact tcaatcgttt 480

tcgaaaatgc catttcctct tgtgaagttg tcaattgtaa tggtgttgaa atccaagt 538

<210> 948
<211> 26
<212> DNA
<213> synthetic construct

<400> 948
taaattgttt agattacaat cagagg 26

<210> 949
<211> 22
<212> DNA
<213> synthetic construct

<400> 949
ttcaaagttt tcgtatgttt ca 22

<210> 950
<211> 19
<212> DNA
<213> synthetic construct

<400> 950
cgtgtttggg ttaaattcc 19

<210> 951
<211> 20
<212> DNA
<213> synthetic construct

<400> 951
ataatggtgt gttcctccac 20

<210> 952
<211> 20
<212> DNA
<213> synthetic construct

<400> 952
aaaagaaaaa cacgcaattc 20

<210> 953
<211> 20
<212> DNA
<213> synthetic construct

<400> 953

cattcgtcaa ctgattcgta                                                    20

<210>  954
<211>  21
<212>  DNA
<213>  synthetic construct

<400>  954
tagcatagca acaaacagtg a                                                  21

<210>  955
<211>  21
<212>  DNA
<213>  synthetic construct

<400>  955
gttttgacct gaagctgtat c                                                  21

<210>  956
<211>  20
<212>  DNA
<213>  synthetic construct

<400>  956
aacaaagcag atgcgatagt                                                    20

<210>  957
<211>  20
<212>  DNA
<213>  synthetic construct

<400>  957
gaaatacgat gcggtttatc                                                    20

<210>  958
<211>  18
<212>  DNA
<213>  synthetic construct

<400>  958
ctaggcgcat tagcagtt                                                      18

<210>  959
<211>  20
<212>  DNA
<213>  synthetic construct

<400>  959
tagccttctc ttccatttca                                                    20

<210>  960
<211>  21
<212>  DNA
<213>  synthetic construct

<400>  960

aatgctgcta acctgcgtga t                                                21

<210>  961
<211>  24
<212>  DNA
<213>  synthetic construct

<400>  961
cacgtctaac cgctttactg attg                                             24

<210>  962
<211>  30
<212>  DNA
<213>  synthetic construct

<400>  962
aaagtaaaga gtagactaag ctgtctgctc                                       30

<210>  963
<211>  25
<212>  DNA
<213>  synthetic construct

<400>  963
acctaataaa attcaagcat tggga                                            25

<210>  964
<211>  27
<212>  DNA
<213>  synthetic construct

<400>  964
aagaatttaa aatggttagg tgtcgta                                          27

<210>  965
<211>  24
<212>  DNA
<213>  synthetic construct

<400>  965
acgtaatcgt tttgttgcca aata                                             24

<210>  966
<211>  22
<212>  DNA
<213>  synthetic construct

<400>  966
aacgtcccat gccattaatt tt                                               22

<210>  967
<211>  24
<212>  DNA
<213>  synthetic construct

<400>  967

ttgcgtttct atttagctca gaca                                          24

<210>  968
<211>  23
<212>  DNA
<213>  synthetic construct

<400>  968
acagagcagc aaaagcgtta gtg                                           23

<210>  969
<211>  24
<212>  DNA
<213>  synthetic construct

<400>  969
gaccttgaat gaaccattga ccat                                          24

<210>  970
<211>  30
<212>  DNA
<213>  synthetic construct

<400>  970
catatggtga ttttacattc ttcttaattg                                    30

<210>  971
<211>  28
<212>  DNA
<213>  synthetic construct

<400>  971
cctaaccatg tactttgtaa cactttca                                      28

<210>  972
<211>  29
<212>  DNA
<213>  synthetic construct

<400>  972
aaatttatta gcagaagtag cagaaaatg                                     29

<210>  973
<211>  27
<212>  DNA
<213>  synthetic construct

<400>  973
ctgaactctt ctaatgcttc aacgatt                                       27

<210>  974
<211>  24
<212>  DNA
<213>  synthetic construct

<400>  974

tttaggcgaa aatattggtg aaga                                              24

<210>  975
<211>  24
<212>  DNA
<213>  synthetic construct

<400>  975
tttgtcgtcg tcttttactt cgtt                                              24

<210>  976
<211>  26
<212>  DNA
<213>  synthetic construct

<400>  976
ggtcttatcg ttgcagctat cactat                                            26

<210>  977
<211>  26
<212>  DNA
<213>  synthetic construct

<400>  977
gagcgtatcg cataaataat cttttc                                            26

<210>  978
<211>  24
<212>  DNA
<213>  synthetic construct

<400>  978
tcaggtgaaa tgttagaatc agca                                              24

<210>  979
<211>  23
<212>  DNA
<213>  synthetic construct

<400>  979
taagtcacca aataagaatg gcg                                               23

<210>  980
<211>  21
<212>  DNA
<213>  synthetic construct

<400>  980
tagtcaccat gaagttgccc c                                                 21

<210>  981
<211>  23
<212>  DNA
<213>  synthetic construct

<400>  981

cctcttgaag atggtacacg gat                                              23

<210>  982
<211>  22
<212>  DNA
<213>  synthetic construct

<400>  982
cgaatgatgc aatcagacga aa                                               22

<210>  983
<211>  23
<212>  DNA
<213>  synthetic construct

<400>  983
caccacgatt tattggcaaa gtt                                              23

<210>  984
<211>  23
<212>  DNA
<213>  synthetic construct

<400>  984
ttgaatcacc aaattgaggt tgt                                              23

<210>  985
<211>  25
<212>  DNA
<213>  synthetic construct

<400>  985
cagtcgttca gatttgaatt tcttt                                            25

<210>  986
<211>  28
<212>  DNA
<213>  synthetic construct

<400>  986
aaatccatcg agatggtaat atatatca                                         28

<210>  987
<211>  31
<212>  DNA
<213>  synthetic construct

<400>  987
aaacttaaaa tactttctga atattgatca t                                     31

<210>  988
<211>  23
<212>  DNA
<213>  synthetic construct

<400>  988

gtatgcaatt tgatcgtggt tat                                              23

<210>  989
<211>  21
<212>  DNA
<213>  synthetic construct

<400>  989
tgttaatgca tcgccttcaa c                                                21

<210>  990
<211>  30
<212>  DNA
<213>  synthetic construct

<400>  990
atgtatgtta gcactcttta atgttaagtg                                       30

<210>  991
<211>  24
<212>  DNA
<213>  synthetic construct

<400>  991
gtttagttgt gtttcatttt cgtt                                             24

<210>  992
<211>  29
<212>  DNA
<213>  synthetic construct

<400>  992
tgtcatatta tcaacatgta atcgaactg                                        29

<210>  993
<211>  29
<212>  DNA
<213>  synthetic construct

<400>  993
aatatcagta attccagaac caagaagat                                        29

<210>  994
<211>  26
<212>  DNA
<213>  synthetic construct

<400>  994
aaaatgatca aaggtgaaga aacatc                                           26

<210>  995
<211>  25
<212>  DNA
<213>  synthetic construct

<400>  995

aatcctcgac atttaatgca cctac 25

<210> 996
<211> 27
<212> DNA
<213> synthetic construct

<400> 996
aatgggatta ttagttatgg cttatgg 27

<210> 997
<211> 26
<212> DNA
<213> synthetic construct

<400> 997
gtggatatgg atcattattc ttttcg 26

<210> 998
<211> 27
<212> DNA
<213> synthetic construct

<400> 998
atgagatata cgaaatcaga agaagca 27

<210> 999
<211> 32
<212> DNA
<213> synthetic construct

<400> 999
ctaatcttaa agtatccaat gtagcttctg ta 32

<210> 1000
<211> 29
<212> DNA
<213> synthetic construct

<400> 1000
aaacagcaag atcctaatat tgatgtaac 29

<210> 1001
<211> 32
<212> DNA
<213> synthetic construct

<400> 1001
ctctacgtac aatcgatact aattcattat ct 32

<210> 1002
<211> 26
<212> DNA
<213> synthetic construct

<400> 1002

attaacaaaa ttgatttacc tgctgc                                              26

<210>  1003
<211>  27
<212>  DNA
<213>  synthetic construct

<400>  1003
ctataaccaa aacctaatgc ttgtgac                                             27

<210>  1004
<211>  21
<212>  DNA
<213>  synthetic construct

<400>  1004
aaagacgcat caaaaccagc a                                                   21

<210>  1005
<211>  29
<212>  DNA
<213>  synthetic construct

<400>  1005
ggctaatgac acctaaagag ttaacaact                                           29

<210>  1006
<211>  28
<212>  DNA
<213>  synthetic construct

<400>  1006
gattaaccac ttagcactaa acacacct                                            28

<210>  1007
<211>  24
<212>  DNA
<213>  synthetic construct

<400>  1007
aatgtttaac aagcacttca cgct                                                24

<210>  1008
<211>  20
<212>  DNA
<213>  synthetic construct

<400>  1008
cgacaaacac ccaacaagca                                                     20

<210>  1009
<211>  22
<212>  DNA
<213>  synthetic construct

<400>  1009

tggctgttat acgcttggtt gt                                                22

<210>  1010
<211>  28
<212>  DNA
<213>  synthetic construct

<400>  1010
gttatcgtat taactggtga aggtgatt                                          28

<210>  1011
<211>  26
<212>  DNA
<213>  synthetic construct

<400>  1011
acatttagta cattaccgcc acctac                                            26

<210>  1012
<211>  25
<212>  DNA
<213>  synthetic construct

<400>  1012
cgtaagggaa gtagttatca gtccg                                             25

<210>  1013
<211>  26
<212>  DNA
<213>  synthetic construct

<400>  1013
ttagctgtat actcgaaatc caatcc                                            26

<210>  1014
<211>  28
<212>  DNA
<213>  synthetic construct

<400>  1014
atggactttt ggttatataa acaagcac                                          28

<210>  1015
<211>  25
<212>  DNA
<213>  synthetic construct

<400>  1015
tatttcagca atgtcacccg tatta                                             25

<210>  1016
<211>  26
<212>  DNA
<213>  synthetic construct

<400>  1016

attgataatt tacatccaac acctgc                                          26

<210>  1017
<211>  29
<212>  DNA
<213>  synthetic construct

<400>  1017
tcactatctg gatcagaatc tttaacaat                                       29

<210>  1018
<211>  31
<212>  DNA
<213>  synthetic construct

<400>  1018
atgaggtact ctttaattag tggtatcttg a                                    31

<210>  1019
<211>  25
<212>  DNA
<213>  synthetic construct

<400>  1019
atcagctaat gaaatgaaga ttgca                                           25

<210>  1020
<211>  27
<212>  DNA
<213>  synthetic construct

<400>  1020
gaaaatacag aacttgatgg tgaaatg                                         27

<210>  1021
<211>  27
<212>  DNA
<213>  synthetic construct

<400>  1021
gcaagactca catacaccat aaacttc                                         27

<210>  1022
<211>  27
<212>  DNA
<213>  synthetic construct

<400>  1022
tcgtttacat cataataatc atcagac                                         27

<210>  1023
<211>  24
<212>  DNA
<213>  synthetic construct

<400>  1023

acacagagaa taaccaggag aaga                                              24

<210>  1024
<211>  26
<212>  DNA
<213>  synthetic construct

<400>  1024
ttgtaattca cttaacttca ccaatg                                            26

<210>  1025
<211>  21
<212>  DNA
<213>  synthetic construct

<400>  1025
tcaagttgcg aaattagctg a                                                 21

<210>  1026
<211>  25
<212>  DNA
<213>  synthetic construct

<400>  1026
actcaaacag ttagcaagat tgctc                                             25

<210>  1027
<211>  20
<212>  DNA
<213>  synthetic construct

<400>  1027
tgcatttacc caaccagtgc                                                   20

<210>  1028
<211>  27
<212>  DNA
<213>  synthetic construct

<400>  1028
ggtgttccaa actcaaaaga tgatata                                           27

<210>  1029
<211>  30
<212>  DNA
<213>  synthetic construct

<400>  1029
ttgacaccat aactcattat aggaatattg                                        30

<210>  1030
<211>  24
<212>  DNA
<213>  synthetic construct

<400>  1030

aacacaatcg gaaatgttgg atac                                              24

<210>  1031
<211>  25
<212>  DNA
<213>  synthetic construct

<400>  1031
ctatcccaat ccatagacgt gttaa                                             25

<210>  1032
<211>  24
<212>  DNA
<213>  synthetic construct

<400>  1032
tgacatttca aatcaatcac atcg                                              24

<210>  1033
<211>  24
<212>  DNA
<213>  synthetic construct

<400>  1033
ttggtaacca aacattttca gctt                                              24

<210>  1034
<211>  27
<212>  DNA
<213>  synthetic construct

<400>  1034
ctggagatac tattgaagaa gacgatg                                           27

<210>  1035
<211>  28
<212>  DNA
<213>  synthetic construct

<400>  1035
ttgcttttac agttctgttt tcatctac                                          28

<210>  1036
<211>  30
<212>  DNA
<213>  synthetic construct

<400>  1036
tagttatcga gattatcaaa gattggtaga                                        30

<210>  1037
<211>  27
<212>  DNA
<213>  synthetic construct

<400>  1037

gtaattgtga gtgtccataa gaatcca                                          27

<210>  1038
<211>  30
<212>  DNA
<213>  synthetic construct

<400>  1038
tgaatcttaa tatagaaaca accactcaag                                       30

<210>  1039
<211>  24
<212>  DNA
<213>  synthetic construct

<400>  1039
acgatctgac acacctaaaa tgta                                             24

<210>  1040
<211>  24
<212>  DNA
<213>  synthetic construct

<400>  1040
tctaaagaag attttatcga aatg                                             24

<210>  1041
<211>  24
<212>  DNA
<213>  synthetic construct

<400>  1041
cccacattgt tattttcttt gtat                                             24

<210>  1042
<211>  26
<212>  DNA
<213>  synthetic construct

<400>  1042
tgagatagat gcaatcatgt ttatgg                                           26

<210>  1043
<211>  32
<212>  DNA
<213>  synthetic construct

<400>  1043
gaaataggta caatctctgt aaagtccata ta                                    32

<210>  1044
<211>  30
<212>  DNA
<213>  synthetic construct

<400>  1044

aatataattg ggaagaagta catcaagaag                                    30

<210>  1045
<211>  27
<212>  DNA
<213>  synthetic construct

<400>  1045
atattagcaa atcggtctta tctctca                                       27

<210>  1046
<211>  24
<212>  DNA
<213>  synthetic construct

<400>  1046
ttgaattacc aaaattacca tacg                                          24

<210>  1047
<211>  26
<212>  DNA
<213>  synthetic construct

<400>  1047
ctcccagaat aatgaatggt ttaaat                                        26

<210>  1048
<211>  18
<212>  DNA
<213>  synthetic construct

<400>  1048
gcgcattttg aaaaggca                                                 18

<210>  1049
<211>  22
<212>  DNA
<213>  synthetic construct

<400>  1049
gggatagcac gtaaaagtgg aa                                            22

<210>  1050
<211>  22
<212>  DNA
<213>  synthetic construct

<400>  1050
ttcgttgttc ataggtgcga gt                                            22

<210>  1051
<211>  20
<212>  DNA
<213>  synthetic construct

<400>  1051

ctttgaacag cactcgtgcg                                                    20

<210>  1052
<211>  28
<212>  DNA
<213>  synthetic construct

<400>  1052
tattgcctta tttagatgta ttgctttt                                          28

<210>  1053
<211>  24
<212>  DNA
<213>  synthetic construct

<400>  1053
tcgtagcttc aaacacattt tcaa                                              24

<210>  1054
<211>  28
<212>  DNA
<213>  synthetic construct

<400>  1054
aatcaaatga tattggaaga tattagca                                          28

<210>  1055
<211>  28
<212>  DNA
<213>  synthetic construct

<400>  1055
tattcagtat cttgtgctat tgtcattg                                          28

<210>  1056
<211>  25
<212>  DNA
<213>  synthetic construct

<400>  1056
catgcggtaa caattctgat aaaga                                             25

<210>  1057
<211>  22
<212>  DNA
<213>  synthetic construct

<400>  1057
aattttcgct ttaggtgcag ct                                                22

<210>  1058
<211>  25
<212>  DNA
<213>  synthetic construct

<400>  1058

gctgactatg aaggtaaagc tgaca                                          25

<210>  1059
<211>  24
<212>  DNA
<213>  synthetic construct

<400>  1059
cagctaagtt ttcttttggt tgga                                           24

<210>  1060
<211>  25
<212>  DNA
<213>  synthetic construct

<400>  1060
caattggctt tcgattattg ttgta                                          25

<210>  1061
<211>  30
<212>  DNA
<213>  synthetic construct

<400>  1061
aaccaatgat ctagtgtaaa tgttaaacct                                     30

<210>  1062
<211>  20
<212>  DNA
<213>  synthetic construct

<400>  1062
cttagatgtc ccatgctgat                                                20

<210>  1063
<211>  20
<212>  DNA
<213>  synthetic construct

<400>  1063
gtcaaacgag tgctaatggt                                                20

<210>  1064
<211>  18
<212>  DNA
<213>  synthetic construct

<400>  1064
ttcaataggc gtggtgtc                                                  18

<210>  1065
<211>  20
<212>  DNA
<213>  synthetic construct

<400>  1065

ttatctgtcg gtttctctgg 20

<210> 1066
<211> 20
<212> DNA
<213> synthetic construct

<400> 1066
aggctcgtat gattgaaaaa 20

<210> 1067
<211> 21
<212> DNA
<213> synthetic construct

<400> 1067
ggttttgagc acgatatgta g 21

<210> 1068
<211> 20
<212> DNA
<213> synthetic construct

<400> 1068
ttggcacaac tgataagaca 20

<210> 1069
<211> 20
<212> DNA
<213> synthetic construct

<400> 1069
aaaaatcgtt caaagtgctc 20

<210> 1070
<211> 20
<212> DNA
<213> synthetic construct

<400> 1070
atcatcagcg acaatgagag 20

<210> 1071
<211> 26
<212> DNA
<213> synthetic construct

<400> 1071
tttttaacat ctcgaactat atctaa 26

<210> 1072
<211> 19
<212> DNA
<213> synthetic construct

<400> 1072

gtgccaattg caggatatg                                                    19

<210>  1073
<211>  24
<212>  DNA
<213>  synthetic construct

<400>  1073
taatgttgtt catttaagcg tttt                                             24

<210>  1074
<211>  22
<212>  DNA
<213>  synthetic construct

<400>  1074
ttgataacat tgctgtgata gg                                               22

<210>  1075
<211>  22
<212>  DNA
<213>  synthetic construct

<400>  1075
ttcaattagt gattcgagtg tt                                               22

<210>  1076
<211>  21
<212>  DNA
<213>  synthetic construct

<400>  1076
tcttccattc tctcagtcaa a                                                21

<210>  1077
<211>  20
<212>  DNA
<213>  synthetic construct

<400>  1077
agaccatgta tgtaggtggc                                                  20

<210>  1078
<211>  18
<212>  DNA
<213>  synthetic construct

<400>  1078
agggcaaatg ctttcagt                                                    18

<210>  1079
<211>  23
<212>  DNA
<213>  synthetic construct

<400>  1079

aaaaggattg tttctctttt tct                                              23


<210>  1080
<211>  19
<212>  DNA
<213>  synthetic construct

<400>  1080
gtgccagagt tgagattcc                                                   19


<210>  1081
<211>  18
<212>  DNA
<213>  synthetic construct

<400>  1081
atttgtcggt cgagtttg                                                    18


<210>  1082
<211>  21
<212>  DNA
<213>  synthetic construct

<400>  1082
tgcatcttcc attttaatag c                                                21


<210>  1083
<211>  20
<212>  DNA
<213>  synthetic construct

<400>  1083
gtcattgtcc tttgttggtt                                                  20


<210>  1084
<211>  20
<212>  DNA
<213>  synthetic construct

<400>  1084
ttgacagctt tgcattttta                                                  20


<210>  1085
<211>  20
<212>  DNA
<213>  synthetic construct

<400>  1085
ggctttgttg cttttaatga                                                  20


<210>  1086
<211>  20
<212>  DNA
<213>  synthetic construct

<400>  1086

gtattattgc ttggggtgat                                                    20

<210>  1087
<211>  20
<212>  DNA
<213>  synthetic construct

<400>  1087
ggatatttct ttcgtgctgt                                                    20

<210>  1088
<211>  20
<212>  DNA
<213>  synthetic construct

<400>  1088
tggctatcag taatgtttcg                                                    20

<210>  1089
<211>  18
<212>  DNA
<213>  synthetic construct

<400>  1089
gaatcagcgt tgtcttcg                                                      18

<210>  1090
<211>  19
<212>  DNA
<213>  synthetic construct

<400>  1090
atggtcaagc tgctgaagt                                                     19

<210>  1091
<211>  27
<212>  DNA
<213>  synthetic construct

<400>  1091
atcatcttct ttaagtgtaa taagttt                                            27

<210>  1092
<211>  20
<212>  DNA
<213>  synthetic construct

<400>  1092
tggaagacat cgtaaacgta                                                    20

<210>  1093
<211>  20
<212>  DNA
<213>  synthetic construct

<400>  1093

tggatcaaag aaacgtgaat                                                    20

<210>  1094
<211>  18
<212>  DNA
<213>  synthetic construct

<400>  1094
ggcagttgta ctcccaca                                                      18

<210>  1095
<211>  19
<212>  DNA
<213>  synthetic construct

<400>  1095
ctttcaacac cccaagttc                                                     19

<210>  1096
<211>  24
<212>  DNA
<213>  synthetic construct

<400>  1096
tgaatagaaa tactaggacc acaa                                               24

<210>  1097
<211>  24
<212>  DNA
<213>  synthetic construct

<400>  1097
ttaagtttaa tccatctgaa aaat                                               24

<210>  1098
<211>  25
<212>  DNA
<213>  synthetic construct

<400>  1098
ggtgtattag ataatgaagg tatgg                                              25

<210>  1099
<211>  20
<212>  DNA
<213>  synthetic construct

<400>  1099
tctttatttt tggcaggttg                                                    20

<210>  1100
<211>  18
<212>  DNA
<213>  synthetic construct

<400>  1100

aaccaaaagg cgagagtt                                                    18

<210>  1101
<211>  23
<212>  DNA
<213>  synthetic construct

<400>  1101
gctgtcaaca taataaatgc ttc                                               23

<210>  1102
<211>  19
<212>  DNA
<213>  synthetic construct

<400>  1102
gcgaaagagt cgaaatcag                                                    19

<210>  1103
<211>  20
<212>  DNA
<213>  synthetic construct

<400>  1103
aaggtgaacg ctctaattca                                                   20

<210>  1104
<211>  20
<212>  DNA
<213>  synthetic construct

<400>  1104
ccgttacgtt gttcttcagt                                                   20

<210>  1105
<211>  20
<212>  DNA
<213>  synthetic construct

<400>  1105
gcaatggttt cattcaaagt                                                   20

<210>  1106
<211>  25
<212>  DNA
<213>  synthetic construct

<400>  1106
tgatattgga agatattagc ataga                                             25

<210>  1107
<211>  21
<212>  DNA
<213>  synthetic construct

<400>  1107

tgacaatcgc tttattcatt t                                                21

<210>  1108
<211>  21
<212>  DNA
<213>  synthetic construct

<400>  1108
ttttgattta tcttctgacg g                                                21

<210>  1109
<211>  20
<212>  DNA
<213>  synthetic construct

<400>  1109
cattcatttt attcccacct                                                  20

<210>  1110
<211>  24
<212>  DNA
<213>  synthetic construct

<400>  1110
tggatataac aatcaaaatc actc                                             24

<210>  1111
<211>  21
<212>  DNA
<213>  synthetic construct

<400>  1111
ttttagaaat gattcgacag c                                                21

<210>  1112
<211>  27
<212>  DNA
<213>  synthetic construct

<400>  1112
atgagatacc taacatcagg agaatca                                          27

<210>  1113
<211>  29
<212>  DNA
<213>  synthetic construct

<400>  1113
gctattcttc catctaattt acgatcata                                        29

<210>  1114
<211>  19
<212>  DNA
<213>  synthetic construct

<400>  1114
   accttcaata ttcgcatcc                                                    19

<210>  1115
<211>  21
<212>  DNA
<213>  synthetic construct

<400>  1115
tattccgatt attaggcgta g                                                21

<210>  1116
<211>  20
<212>  DNA
<213>  synthetic construct

<400>  1116
tcgaggaatt aacaaaggtc                                                  20

<210>  1117
<211>  20
<212>  DNA
<213>  synthetic construct

<400>  1117
atcaggttta gttggtggtg                                                  20

<210>  1118
<211>  20
<212>  DNA
<213>  synthetic construct

<400>  1118
gaacctagcc atcaagacag                                                  20

<210>  1119
<211>  20
<212>  DNA
<213>  synthetic construct

<400>  1119
gcattattag aggcatgtgg                                                  20

<210>  1120
<211>  20
<212>  DNA
<213>  synthetic construct

<400>  1120
tcgtgcatta gtaccatcag                                                  20

<210>  1121
<211>  19
<212>  DNA
<213>  synthetic construct

<400>  1121

aaccaccttc gtcacctac 19

<210> 1122
<211> 18
<212> DNA
<213> synthetic construct

<400> 1122
cgccaaaata gtgcttca 18

<210> 1123
<211> 20
<212> DNA
<213> synthetic construct

<400> 1123
ggtttgtcct catcacctaa 20

<210> 1124
<211> 25
<212> DNA
<213> synthetic construct

<400> 1124
tgaaaaataa attgatagca aaatc 25

<210> 1125
<211> 20
<212> DNA
<213> synthetic construct

<400> 1125
ttcgctcttg taagaccatt 20

<210> 1126
<211> 20
<212> DNA
<213> synthetic construct

<400> 1126
aatcctcata acgcagaaag 20

<210> 1127
<211> 20
<212> DNA
<213> synthetic construct

<400> 1127
tcagctaaat cgtctgaacc 20

<210> 1128
<211> 20
<212> DNA
<213> synthetic construct

<400> 1128

gctggtgtgg tacttatcct                                                    20

<210>  1129
<211>  19
<212>  DNA
<213>  synthetic construct

<400>  1129
acgcctgtct tttcattgt                                                     19

<210>  1130
<211>  27
<212>  DNA
<213>  synthetic construct

<400>  1130
ttaacaatag aacatttaac aaagaag                                            27

<210>  1131
<211>  23
<212>  DNA
<213>  synthetic construct

<400>  1131
ctcgaaatta agaaagtaac acc                                                23

<210>  1132
<211>  20
<212>  DNA
<213>  synthetic construct

<400>  1132
aaatggttca gtcgtaatgg                                                    20

<210>  1133
<211>  20
<212>  DNA
<213>  synthetic construct

<400>  1133
agttgctctg gatgtcctac                                                    20

<210>  1134
<211>  23
<212>  DNA
<213>  synthetic construct

<400>  1134
ggtattgcat ctgtaacttt agg                                                23

<210>  1135
<211>  20
<212>  DNA
<213>  synthetic construct

<400>  1135

aggttagcac tttgacttgg                                                    20


<210>  1136
<211>  27
<212>  DNA
<213>  synthetic construct

<400>  1136
gttatcaatt aatacaaccc ctgaagc                                            27


<210>  1137
<211>  26
<212>  DNA
<213>  synthetic construct

<400>  1137
tggaactaat tctccttcga ttgtta                                             26


<210>  1138
<211>  26
<212>  DNA
<213>  synthetic construct

<400>  1138
gtagttgaaa atatgcctgt tggtgt                                             26


<210>  1139
<211>  26
<212>  DNA
<213>  synthetic construct

<400>  1139
attacaccat taacgataat tggcat                                             26


<210>  1140
<211>  30
<212>  DNA
<213>  synthetic construct

<400>  1140
agacttatta tctaaacgtg gtgaactagc                                         30


<210>  1141
<211>  24
<212>  DNA
<213>  synthetic construct

<400>  1141
caaatgattt attgccgtct ccta                                               24


<210>  1142
<211>  21
<212>  DNA
<213>  synthetic construct

<400>  1142

aaaattgctg gtatcgctgc a                                                21

<210>  1143
<211>  27
<212>  DNA
<213>  synthetic construct

<400>  1143
gtcattacat catcaacttg catgtta                                          27

<210>  1144
<211>  23
<212>  DNA
<213>  synthetic construct

<400>  1144
agctgagacg acacaagatc aaa                                              23

<210>  1145
<211>  23
<212>  DNA
<213>  synthetic construct

<400>  1145
ttatattgcg tttcaagagc tgc                                              23

<210>  1146
<211>  19
<212>  DNA
<213>  synthetic construct

<400>  1146
cgggattctc tgcattatc                                                   19

<210>  1147
<211>  19
<212>  DNA
<213>  synthetic construct

<400>  1147
gtccgctggt atttctttg                                                   19

<210>  1148
<211>  20
<212>  DNA
<213>  synthetic construct

<400>  1148
ttaattgttc taccgctcca                                                  20

<210>  1149
<211>  23
<212>  DNA
<213>  synthetic construct

<400>  1149

catttgtacc cattattgta acc                                                23

<210>  1150
<211>  21
<212>  DNA
<213>  synthetic construct

<400>  1150
aaagataatt ggtttgctga a                                                  21

<210>  1151
<211>  21
<212>  DNA
<213>  synthetic construct

<400>  1151
tcaaatcgct tcttaatcac t                                                  21

<210>  1152
<211>  25
<212>  DNA
<213>  synthetic construct

<400>  1152
caagggtact taaacaaata gaaac                                              25

<210>  1153
<211>  21
<212>  DNA
<213>  synthetic construct

<400>  1153
ctcgaattat gaatgaagga g                                                  21

<210>  1154
<211>  20
<212>  DNA
<213>  synthetic construct

<400>  1154
actttgtgca attatcagca                                                    20

<210>  1155
<211>  25
<212>  DNA
<213>  synthetic construct

<400>  1155
atgaaacaga aaatatgaaa aatac                                              25

<210>  1156
<211>  24
<212>  DNA
<213>  synthetic construct

<400>  1156

ttatggatag cgtaaagaca ataa                                          24

<210>  1157
<211>  23
<212>  DNA
<213>  synthetic construct

<400>  1157
ttcactggta ataaaacaaa caa                                           23

<210>  1158
<211>  20
<212>  DNA
<213>  synthetic construct

<400>  1158
gattcttggc gctactaaca                                               20

<210>  1159
<211>  22
<212>  DNA
<213>  synthetic construct

<400>  1159
ccatcggtta cattactttc tc                                            22

<210>  1160
<211>  21
<212>  DNA
<213>  synthetic construct

<400>  1160
taatgtttcc ttgccttatg t                                             21

<210>  1161
<211>  22
<212>  DNA
<213>  synthetic construct

<400>  1161
tgataacttc ttctttcacc aa                                            22

<210>  1162
<211>  24
<212>  DNA
<213>  synthetic construct

<400>  1162
gtaaaaacat ttatgaaatc gaaa                                          24

<210>  1163
<211>  20
<212>  DNA
<213>  synthetic construct

<400>  1163

tgtatgttcc cccaattcta                                                    20

<210>  1164
<211>  22
<212>  DNA
<213>  synthetic construct

<400>  1164
aagatacgat ttgttgattg tg                                                 22

<210>  1165
<211>  19
<212>  DNA
<213>  synthetic construct

<400>  1165
ggccatacca taaataccc                                                     19

<210>  1166
<211>  30
<212>  DNA
<213>  synthetic construct

<400>  1166
ttttatctta attaaggaag gagtgatttc                                         30

<210>  1167
<211>  27
<212>  DNA
<213>  synthetic construct

<400>  1167
tagtgaattt gttcactgtg tcgataa                                            27

<210>  1168
<211>  21
<212>  DNA
<213>  synthetic construct

<400>  1168
aaacatcaaa tcgctgtggc t                                                  21

<210>  1169
<211>  21
<212>  DNA
<213>  synthetic construct

<400>  1169
gtgaaagatg cccttgagtg g                                                  21

<210>  1170
<211>  25
<212>  DNA
<213>  synthetic construct

<400>  1170

gggttcttgc tgtctttaag tgatt 25

<210> 1171
<211> 25
<212> DNA
<213> synthetic construct

<400> 1171
tatatctcga agttgctagt tgggg 25

<210> 1172
<211> 25
<212> DNA
<213> synthetic construct

<400> 1172
aaagatagtt ctaagataaa tggtc 25

<210> 1173
<211> 19
<212> DNA
<213> synthetic construct

<400> 1173
ggccattatt ggtctgttg 19

<210> 1174
<211> 22
<212> DNA
<213> synthetic construct

<400> 1174
gctagtgcat ttgttattca ag 22

<210> 1175
<211> 19
<212> DNA
<213> synthetic construct

<400> 1175
ttcacttctg tgcatttgg 19

<210> 1176
<211> 20
<212> DNA
<213> synthetic construct

<400> 1176
ttttagcagc gtcaattttt 20

<210> 1177
<211> 21
<212> DNA
<213> synthetic construct

<400> 1177

ctgatccaga gtttcctacc t                                                21

<210>  1178
<211>  24
<212>  DNA
<213>  synthetic construct

<400>  1178
agaaagaaag tgatttctat gatt                                             24

<210>  1179
<211>  21
<212>  DNA
<213>  synthetic construct

<400>  1179
tggccatatc attcttttaa t                                                21

<210>  1180
<211>  18
<212>  DNA
<213>  synthetic construct

<400>  1180
tcgttacacc gaatggtc                                                    18

<210>  1181
<211>  24
<212>  DNA
<213>  synthetic construct

<400>  1181
aggtgtgatg cttttaattt ttac                                             24

<210>  1182
<211>  23
<212>  DNA
<213>  synthetic construct

<400>  1182
tttaagcgta ctatcacaca gac                                              23

<210>  1183
<211>  25
<212>  DNA
<213>  synthetic construct

<400>  1183
tattgttttc agtttctttt gtatc                                            25

<210>  1184
<211>  22
<212>  DNA
<213>  synthetic construct

<400>  1184

ttgatagcga tttatttgta gg                                              22

<210>  1185
<211>  21
<212>  DNA
<213>  synthetic construct

<400>  1185
caattctgtc ctttcacttt g                                               21

<210>  1186
<211>  22
<212>  DNA
<213>  synthetic construct

<400>  1186
gtcagctcag taacaacaac ac                                              22

<210>  1187
<211>  20
<212>  DNA
<213>  synthetic construct

<400>  1187
gtagcgaagt ctggtgaaaa                                                 20

<210>  1188
<211>  21
<212>  DNA
<213>  synthetic construct

<400>  1188
tgttatcgac cgttttgtat c                                               21

<210>  1189
<211>  21
<212>  DNA
<213>  synthetic construct

<400>  1189
tttcatcttt ggggatattt t                                               21

<210>  1190
<211>  20
<212>  DNA
<213>  synthetic construct

<400>  1190
ggtgtcctat ctcgaaaaca                                                 20

<210>  1191
<211>  25
<212>  DNA
<213>  synthetic construct

<400>  1191

tttttcctta gattgagtat ctatt                                          25

<210>  1192
<211>  24
<212>  DNA
<213>  synthetic construct

<400>  1192
ataaagaaag gaaatgattt tatg                                           24

<210>  1193
<211>  24
<212>  DNA
<213>  synthetic construct

<400>  1193
tatctttaac aaaatcaaac tgaa                                           24

<210>  1194
<211>  22
<212>  DNA
<213>  synthetic construct

<400>  1194
atcattaggt aaaatgtctg ga                                             22

<210>  1195
<211>  25
<212>  DNA
<213>  synthetic construct

<400>  1195
aattatgtcc tttcacttta atttc                                          25

<210>  1196
<211>  21
<212>  DNA
<213>  synthetic construct

<400>  1196
aagttgctca aatacaagct g                                              21

<210>  1197
<211>  20
<212>  DNA
<213>  synthetic construct

<400>  1197
tgatgttagc ccaatctaca                                                20

<210>  1198
<211>  25
<212>  DNA
<213>  synthetic construct

<400>  1198

tgttattatt ctcattttct tcaat                                              25

<210>  1199
<211>  20
<212>  DNA
<213>  synthetic construct

<400>  1199
atgctctgat aaatctggga                                                   20

<210>  1200
<211>  20
<212>  DNA
<213>  synthetic construct

<400>  1200
ttttattcat tgccctaacg                                                   20

<210>  1201
<211>  26
<212>  DNA
<213>  synthetic construct

<400>  1201
ttttcagagt taatcgtttt attatc                                            26

<210>  1202
<211>  20
<212>  DNA
<213>  synthetic construct

<400>  1202
cgtagatgtg tttggagcta                                                   20

<210>  1203
<211>  20
<212>  DNA
<213>  synthetic construct

<400>  1203
cttgagcagt cacctttttc                                                   20

<210>  1204
<211>  20
<212>  DNA
<213>  synthetic construct

<400>  1204
aatttttggc acatgattta                                                   20

<210>  1205
<211>  24
<212>  DNA
<213>  synthetic construct

<400>  1205

cttttatgtc tagttcttga gctg                                              24

<210>  1206
<211>  19
<212>  DNA
<213>  synthetic construct

<400>  1206
ccacctgttg aaggaagag                                                   19

<210>  1207
<211>  23
<212>  DNA
<213>  synthetic construct

<400>  1207
tttaggaaat aagtcaaacc aaa                                              23

<210>  1208
<211>  24
<212>  DNA
<213>  synthetic construct

<400>  1208
gaagatttac acgataaaag tgag                                             24

<210>  1209
<211>  24
<212>  DNA
<213>  synthetic construct

<400>  1209
aaatcataaa tgtcgaatga gtaa                                             24

<210>  1210
<211>  20
<212>  DNA
<213>  synthetic construct

<400>  1210
agtcttatct aacggcgatg                                                  20

<210>  1211
<211>  22
<212>  DNA
<213>  synthetic construct

<400>  1211
agttgtaact gtttgatgct tg                                               22

<210>  1212
<211>  27
<212>  DNA
<213>  synthetic construct

<400>  1212

ggatataaat acggaaataa agttaca                                          27


<210>  1213
<211>  23
<212>  DNA
<213>  synthetic construct

<400>  1213
gaagtcacag aagtagtgtg ttg                                              23


<210>  1214
<211>  21
<212>  DNA
<213>  synthetic construct

<400>  1214
atgaaattta aagcgatagc a                                                21


<210>  1215
<211>  27
<212>  DNA
<213>  synthetic construct

<400>  1215
gtataaacta ctttgtggtt cttcttt                                          27


<210>  1216
<211>  25
<212>  DNA
<213>  synthetic construct

<400>  1216
gtattgaata taaaaatgtg acagg                                            25


<210>  1217
<211>  20
<212>  DNA
<213>  synthetic construct

<400>  1217
gacattgata acatcggaca                                                  20


<210>  1218
<211>  19
<212>  DNA
<213>  synthetic construct

<400>  1218
gcgcaattac agtaacgac                                                   19


<210>  1219
<211>  19
<212>  DNA
<213>  synthetic construct

<400>  1219

tagattgcat tggttgtgg                                                    19

<210>  1220
<211>  26
<212>  DNA
<213>  synthetic construct

<400>  1220
tttaaaagtt agttctttat tcgttg                                            26

<210>  1221
<211>  25
<212>  DNA
<213>  synthetic construct

<400>  1221
acttgaatat aagttacggg tgcat                                             25

<210>  1222
<211>  20
<212>  DNA
<213>  synthetic construct

<400>  1222
tttttatcgt aagccctttg                                                   20

<210>  1223
<211>  20
<212>  DNA
<213>  synthetic construct

<400>  1223
caataaccac ccgttttatc                                                   20

<210>  1224
<211>  25
<212>  DNA
<213>  synthetic construct

<400>  1224
gaaagtattc tgtaggtact gcttc                                             25

<210>  1225
<211>  20
<212>  DNA
<213>  synthetic construct

<400>  1225
cctttatcaa tcgcaatgtc                                                   20

<210>  1226
<211>  20
<212>  DNA
<213>  synthetic construct

<400>  1226

cgggcaaata aataaagatg                                                    20

<210>  1227
<211>  20
<212>  DNA
<213>  synthetic construct

<400>  1227
aactgaagat aagccgtttg                                                    20

<210>  1228
<211>  19
<212>  DNA
<213>  synthetic construct

<400>  1228
aaagttggcg atggtaaag                                                     19

<210>  1229
<211>  21
<212>  DNA
<213>  synthetic construct

<400>  1229
cgatgaagtt tgaatatcct g                                                  21

<210>  1230
<211>  20
<212>  DNA
<213>  synthetic construct

<400>  1230
gtttgggact tattgctctg                                                    20

<210>  1231
<211>  19
<212>  DNA
<213>  synthetic construct

<400>  1231
catcagccac agtttcaag                                                     19

<210>  1232
<211>  19
<212>  DNA
<213>  synthetic construct

<400>  1232
agcctggtga cgacttatc                                                     19

<210>  1233
<211>  18
<212>  DNA
<213>  synthetic construct

<400>  1233

atccgccagt tgcttaac                                                    18

<210>  1234
<211>  19
<212>  DNA
<213>  synthetic construct

<400>  1234
accacgacag gtctttatg                                                   19

<210>  1235
<211>  19
<212>  DNA
<213>  synthetic construct

<400>  1235
agagaggcac cgtcactac                                                   19

<210>  1236
<211>  18
<212>  DNA
<213>  synthetic construct

<400>  1236
atggaattgc gtctgttc                                                    18

<210>  1237
<211>  18
<212>  DNA
<213>  synthetic construct

<400>  1237
aagtttagcc acagcagg                                                    18

<210>  1238
<211>  20
<212>  DNA
<213>  synthetic construct

<400>  1238
ataatcctcg tcatttgcag                                                  20

<210>  1239
<211>  20
<212>  DNA
<213>  synthetic construct

<400>  1239
gacttcgggt gattgataag                                                  20

<210>  1240
<211>  20
<212>  DNA
<213>  synthetic construct

<400>  1240

ctctgtccct cagttctacg                                                    20

<210>  1241
<211>  20
<212>  DNA
<213>  synthetic construct

<400>  1241
tggtattggt tccgttattc                                                    20

<210>  1242
<211>  18
<212>  DNA
<213>  synthetic construct

<400>  1242
gactcggtac agcgattg                                                      18

<210>  1243
<211>  18
<212>  DNA
<213>  synthetic construct

<400>  1243
ctgacgttgg gtatctcg                                                      18

<210>  1244
<211>  18
<212>  DNA
<213>  synthetic construct

<400>  1244
ctttacgacg gttctccc                                                      18

<210>  1245
<211>  19
<212>  DNA
<213>  synthetic construct

<400>  1245
aatcttccct gctgaaatg                                                     19

<210>  1246
<211>  19
<212>  DNA
<213>  synthetic construct

<400>  1246
ttgaaacttc ttactgccg                                                     19

<210>  1247
<211>  21
<212>  DNA
<213>  synthetic construct

<400>  1247

aatttctaat gcagcgtatt g                                                21

<210>  1248
<211>  20
<212>  DNA
<213>  synthetic construct

<400>  1248
atagcagggc tgtttgtatc                                                   20

<210>  1249
<211>  20
<212>  DNA
<213>  synthetic construct

<400>  1249
gacacggaaa ccaaattaac                                                   20

<210>  1250
<211>  18
<212>  DNA
<213>  synthetic construct

<400>  1250
tattgtcatc gcgcagag                                                     18

<210>  1251
<211>  20
<212>  DNA
<213>  synthetic construct

<400>  1251
tgttgggttg aaagagtagc                                                   20

<210>  1252
<211>  20
<212>  DNA
<213>  synthetic construct

<400>  1252
gaataccaaa gcagatcgtc                                                   20

<210>  1253
<211>  18
<212>  DNA
<213>  synthetic construct

<400>  1253
ccgagatcga caacagag                                                     18

<210>  1254
<211>  20
<212>  DNA
<213>  synthetic construct

<400>  1254

ctaactcatt gtggtggagc                                                    20

<210>  1255
<211>  19
<212>  DNA
<213>  synthetic construct

<400>  1255
cttgtcatcg gtcatgttg                                                     19

<210>  1256
<211>  21
<212>  DNA
<213>  synthetic construct

<400>  1256
ggcgttacta tcctctctat g                                                  21

<210>  1257
<211>  18
<212>  DNA
<213>  synthetic construct

<400>  1257
tttccatact gattgccg                                                      18

<210>  1258
<211>  24
<212>  DNA
<213>  synthetic construct

<400>  1258
tttgttgtta ttggtacttc attc                                               24

<210>  1259
<211>  19
<212>  DNA
<213>  synthetic construct

<400>  1259
atcgaaattg ttactggcg                                                     19

<210>  1260
<211>  21
<212>  DNA
<213>  synthetic construct

<400>  1260
ttacgcttcc gatcatagta g                                                  21

<210>  1261
<211>  20
<212>  DNA
<213>  synthetic construct

<400>  1261

gaatacgttt agttgaggcg                                                    20

<210>  1262
<211>  20
<212>  DNA
<213>  synthetic construct

<400>  1262
aagtgaagag gtaatggctg                                                    20

<210>  1263
<211>  19
<212>  DNA
<213>  synthetic construct

<400>  1263
taccatcagt atccttggc                                                     19

<210>  1264
<211>  20
<212>  DNA
<213>  synthetic construct

<400>  1264
gatggtgact ctattgcagg                                                    20

<210>  1265
<211>  19
<212>  DNA
<213>  synthetic construct

<400>  1265
ccatacgatt ctggacctc                                                     19

<210>  1266
<211>  20
<212>  DNA
<213>  synthetic construct

<400>  1266
tgtttgaggt cactttctgg                                                    20

<210>  1267
<211>  18
<212>  DNA
<213>  synthetic construct

<400>  1267
caatagcttt caccaggg                                                      18

<210>  1268
<211>  19
<212>  DNA
<213>  synthetic construct

<400>  1268

tgtttctgca ctcgaaatg                                                    19

<210>  1269
<211>  18
<212>  DNA
<213>  synthetic construct

<400>  1269
ggcaatagct ttcaccag                                                     18

<210>  1270
<211>  20
<212>  DNA
<213>  synthetic construct

<400>  1270
tatgctgctc caactattcc                                                   20

<210>  1271
<211>  20
<212>  DNA
<213>  synthetic construct

<400>  1271
attatctcca tctttcaggg                                                   20

<210>  1272
<211>  20
<212>  DNA
<213>  synthetic construct

<400>  1272
cttggaaatg ttggtaaagc                                                   20

<210>  1273
<211>  19
<212>  DNA
<213>  synthetic construct

<400>  1273
taaactcctt cggttgagc                                                    19

<210>  1274
<211>  21
<212>  DNA
<213>  synthetic construct

<400>  1274
tcaatgctga aactataagg c                                                 21

<210>  1275
<211>  19
<212>  DNA
<213>  synthetic construct

<400>  1275

acttagcacc cagttcgac 19

<210> 1276
<211> 20
<212> DNA
<213> synthetic construct

<400> 1276
catcaggcag ttatcctgtc 20

<210> 1277
<211> 19
<212> DNA
<213> synthetic construct

<400> 1277
agtcgtcctc ctgcattac 19

<210> 1278
<211> 18
<212> DNA
<213> synthetic construct

<400> 1278
ttcacagcgg atatggac 18

<210> 1279
<211> 20
<212> DNA
<213> synthetic construct

<400> 1279
cactttgctc ccagaaatac 20

<210> 1280
<211> 19
<212> DNA
<213> synthetic construct

<400> 1280
agactgggat ttggtcaac 19

<210> 1281
<211> 18
<212> DNA
<213> synthetic construct

<400> 1281
agacaccatc ctgccttc 18

<210> 1282
<211> 20
<212> DNA
<213> synthetic construct

<400> 1282

ggagtatatt gcgtgggtag                                                    20

<210>  1283
<211>  19
<212>  DNA
<213>  synthetic construct

<400>  1283
aagattcacc atagaggcg                                                     19

<210>  1284
<211>  21
<212>  DNA
<213>  synthetic construct

<400>  1284
aaatgaatgt ctggactcaa c                                                  21

<210>  1285
<211>  22
<212>  DNA
<213>  synthetic construct

<400>  1285
tgtaaacaac ggtcataaag tg                                                 22

<210>  1286
<211>  18
<212>  DNA
<213>  synthetic construct

<400>  1286
agcagcatca ggttcttg                                                      18

<210>  1287
<211>  18
<212>  DNA
<213>  synthetic construct

<400>  1287
cagttgttca aacgcgag                                                      18

<210>  1288
<211>  20
<212>  DNA
<213>  synthetic construct

<400>  1288
ttcttcggta tcctattccc                                                    20

<210>  1289
<211>  19
<212>  DNA
<213>  synthetic construct

<400>  1289

tgtgaggtcc acttcttcc                                                    19

<210>  1290
<211>  20
<212>  DNA
<213>  synthetic construct

<400>  1290
aaatggcgac aaattatacc                                                   20

<210>  1291
<211>  20
<212>  DNA
<213>  synthetic construct

<400>  1291
ctgggtctcc tcattacaag                                                   20

<210>  1292
<211>  19
<212>  DNA
<213>  synthetic construct

<400>  1292
aagaagatgt ttatggcgg                                                    19

<210>  1293
<211>  21
<212>  DNA
<213>  synthetic construct

<400>  1293
gattcacagg tactggattt g                                                 21

<210>  1294
<211>  25
<212>  DNA
<213>  synthetic construct

<400>  1294
ccactttttc tacttctaaa acttc                                             25

<210>  1295
<211>  22
<212>  DNA
<213>  synthetic construct

<400>  1295
tattttcaaa gatgcaaaca ca                                                22

<210>  1296
<211>  21
<212>  DNA
<213>  synthetic construct

<400>  1296

ttgataacaa acaaattctg c                                                21

<210>  1297
<211>  24
<212>  DNA
<213>  synthetic construct

<400>  1297
aacgtttaaa tcttatctta actg                                             24

<210>  1298
<211>  19
<212>  DNA
<213>  synthetic construct

<400>  1298
taacactgaa ccccaatga                                                   19

<210>  1299
<211>  20
<212>  DNA
<213>  synthetic construct

<400>  1299
atccggtttt tatggtatga                                                  20

<210>  1300
<211>  20
<212>  DNA
<213>  synthetic construct

<400>  1300
cctcaaacaa gcagaaaaag                                                  20

<210>  1301
<211>  19
<212>  DNA
<213>  synthetic construct

<400>  1301
actaaatggg gtgaattgc                                                   19

<210>  1302
<211>  21
<212>  DNA
<213>  synthetic construct

<400>  1302
cttagggaaa aagatgggta g                                                21

<210>  1303
<211>  27
<212>  DNA
<213>  synthetic construct

<400>  1303

cgtaaaaata acttagatca catacag                                          27

<210>  1304
<211>  19
<212>  DNA
<213>  synthetic construct

<400>  1304
cctcgcatat cagtttgtg                                                   19

<210>  1305
<211>  25
<212>  DNA
<213>  synthetic construct

<400>  1305
gatgatatta atctatttcc gtttg                                            25

<210>  1306
<211>  20
<212>  DNA
<213>  synthetic construct

<400>  1306
atgaagcaca accaccatac                                                  20

<210>  1307
<211>  26
<212>  DNA
<213>  synthetic construct

<400>  1307
gtattattta cggtctattt agcaag                                           26

<210>  1308
<211>  20
<212>  DNA
<213>  synthetic construct

<400>  1308
ctaagcacaa gaaaggctca                                                  20

<210>  1309
<211>  27
<212>  DNA
<213>  synthetic construct

<400>  1309
tcatcatcta tgaatttact actatcc                                          27

<210>  1310
<211>  20
<212>  DNA
<213>  synthetic construct

<400>  1310

acgacgaatg attcataagg                                                    20

<210>  1311
<211>  21
<212>  DNA
<213>  synthetic construct

<400>  1311
ttctatcaat gctggattca c                                                  21

<210>  1312
<211>  21
<212>  DNA
<213>  synthetic construct

<400>  1312
ttaaaccatt aggaaatcgt g                                                  21

<210>  1313
<211>  25
<212>  DNA
<213>  synthetic construct

<400>  1313
aatagctaat atatcttctt cattt                                              25

<210>  1314
<211>  20
<212>  DNA
<213>  synthetic construct

<400>  1314
tcaagacacg ctttctagtg                                                    20

<210>  1315
<211>  18
<212>  DNA
<213>  synthetic construct

<400>  1315
atcgaaccct ttgtttcc                                                      18

<210>  1316
<211>  23
<212>  DNA
<213>  synthetic construct

<400>  1316
gttttcttat tacgaaccac att                                                23

<210>  1317
<211>  22
<212>  DNA
<213>  synthetic construct

<400>  1317

ctgaagtgta aacccatatt ga                                              22

<210>  1318
<211>  24
<212>  DNA
<213>  synthetic construct

<400>  1318
ttattctgct atatgatatt cacg                                            24

<210>  1319
<211>  23
<212>  DNA
<213>  synthetic construct

<400>  1319
aacattagta gatggattca agg                                             23

<210>  1320
<211>  21
<212>  DNA
<213>  synthetic construct

<400>  1320
acattaagtc agcatttgga g                                               21

<210>  1321
<211>  24
<212>  DNA
<213>  synthetic construct

<400>  1321
taactttaag acgactatcc atca                                            24

<210>  1322
<211>  23
<212>  DNA
<213>  synthetic construct

<400>  1322
attagagcca aagtactctc cac                                             23

<210>  1323
<211>  22
<212>  DNA
<213>  synthetic construct

<400>  1323
taacaaacca acctttcgat ac                                              22

<210>  1324
<211>  24
<212>  DNA
<213>  synthetic construct

<400>  1324

tttgatacct gtaatttgtt cttg                                              24

<210>  1325
<211>  21
<212>  DNA
<213>  synthetic construct

<400>  1325
caccaattga tccaatgtta t                                                 21

<210>  1326
<211>  20
<212>  DNA
<213>  synthetic construct

<400>  1326
agcttctcgc actacttgac                                                   20

<210>  1327
<211>  20
<212>  DNA
<213>  synthetic construct

<400>  1327
gaatgaggca gctatcaaac                                                   20

<210>  1328
<211>  24
<212>  DNA
<213>  synthetic construct

<400>  1328
aaagaaatta agtctctagc caaa                                              24

<210>  1329
<211>  22
<212>  DNA
<213>  synthetic construct

<400>  1329
ttattgtgcg taaggataat tg                                                22

<210>  1330
<211>  20
<212>  DNA
<213>  synthetic construct

<400>  1330
aaccaaacga tgctagatga                                                   20

<210>  1331
<211>  24
<212>  DNA
<213>  synthetic construct

<400>  1331

aagaatactt taccaaaagg tgag                                              24

<210>  1332
<211>  24
<212>  DNA
<213>  synthetic construct

<400>  1332
agttttataa tattcagtgc aaaa                                              24

<210>  1333
<211>  24
<212>  DNA
<213>  synthetic construct

<400>  1333
tgaaagataa aatactaggt gtcg                                              24

<210>  1334
<211>  26
<212>  DNA
<213>  synthetic construct

<400>  1334
tttagagaga cagctagata atttga                                            26

<210>  1335
<211>  23
<212>  DNA
<213>  synthetic construct

<400>  1335
caatcttact aattgcttga cct                                               23

<210>  1336
<211>  21
<212>  DNA
<213>  synthetic construct

<400>  1336
tgaaagaagg gatagttttg c                                                 21

<210>  1337
<211>  19
<212>  DNA
<213>  synthetic construct

<400>  1337
ttaaacgatt gggtgatgg                                                    19

<210>  1338
<211>  21
<212>  DNA
<213>  synthetic construct

<400>  1338

atgcaaatta tggagatgaa g                                                21

<210>  1339
<211>  20
<212>  DNA
<213>  synthetic construct

<400>  1339
gacagcttga cctttaccag                                                  20

<210>  1340
<211>  25
<212>  DNA
<213>  synthetic construct

<400>  1340
tgattataga agaaattcaa ggaaa                                            25

<210>  1341
<211>  20
<212>  DNA
<213>  synthetic construct

<400>  1341
tcaatcatgt gaatgtccta                                                  20

<210>  1342
<211>  23
<212>  DNA
<213>  synthetic construct

<400>  1342
ttaacttatt cagatgggat agc                                              23

<210>  1343
<211>  23
<212>  DNA
<213>  synthetic construct

<400>  1343
gaaataaaga ttcgaacatg aac                                              23

<210>  1344
<211>  22
<212>  DNA
<213>  synthetic construct

<400>  1344
tcgtatatgg aatttgtagc ag                                               22

<210>  1345
<211>  20
<212>  DNA
<213>  synthetic construct

<400>  1345

tgtcgatgat aaatcgtttg                                                    20

<210>  1346
<211>  21
<212>  DNA
<213>  synthetic construct

<400>  1346
gcttaacaac gtaaaacaag c                                                  21

<210>  1347
<211>  21
<212>  DNA
<213>  synthetic construct

<400>  1347
tctttctctt caacaatacg c                                                  21

<210>  1348
<211>  20
<212>  DNA
<213>  synthetic construct

<400>  1348
agctcacggc tttaactatg                                                    20

<210>  1349
<211>  20
<212>  DNA
<213>  synthetic construct

<400>  1349
tgtagaacgt ggaaatgttg                                                    20

<210>  1350
<211>  21
<212>  DNA
<213>  synthetic construct

<400>  1350
gtatttacgt gcgtttattt g                                                  21

<210>  1351
<211>  20
<212>  DNA
<213>  synthetic construct

<400>  1351
atgaattgat gaaaagtgtc                                                    20

<210>  1352
<211>  18
<212>  DNA
<213>  synthetic construct

<400>  1352

ggtcaagccc agacagag                                                    18


<210>  1353
<211>  24
<212>  DNA
<213>  synthetic construct

<400>  1353
atgtcacgac ctttcttata tttt                                              24


<210>  1354
<211>  25
<212>  DNA
<213>  synthetic construct

<400>  1354
catttaacag tgaatatact tggtc                                             25


<210>  1355
<211>  24
<212>  DNA
<213>  synthetic construct

<400>  1355
gaaatatatc gaaaaagtgt tgag                                              24


<210>  1356
<211>  23
<212>  DNA
<213>  synthetic construct

<400>  1356
atgtcaaaat tagcagaagc tat                                               23


<210>  1357
<211>  22
<212>  DNA
<213>  synthetic construct

<400>  1357
ttagaaaccg aagattttac ct                                                22


<210>  1358
<211>  21
<212>  DNA
<213>  synthetic construct

<400>  1358
tgacacaata cctcatgaac c                                                 21


<210>  1359
<211>  23
<212>  DNA
<213>  synthetic construct

<400>  1359

tcctttctta taaccactac agg                                              23


<210>  1360
<211>  27
<212>  DNA
<213>  synthetic construct

<400>  1360
gtgacagatg taagacttag aaaaata                                          27


<210>  1361
<211>  19
<212>  DNA
<213>  synthetic construct

<400>  1361
agcttcgtct gattcttcg                                                   19


<210>  1362
<211>  25
<212>  DNA
<213>  synthetic construct

<400>  1362
atgaaaataa tcaactcaga taagg                                            25


<210>  1363
<211>  26
<212>  DNA
<213>  synthetic construct

<400>  1363
tataattcct tcactttacc tatcaa                                           26


<210>  1364
<211>  19
<212>  DNA
<213>  synthetic construct

<400>  1364
atgaacatga gcgacatca                                                   19


<210>  1365
<211>  22
<212>  DNA
<213>  synthetic construct

<400>  1365
atacgttctg caagatgttc ta                                               22


<210>  1366
<211>  18
<212>  DNA
<213>  synthetic construct

<400>  1366

ttgaattggg aacgacag                                                    18

<210>  1367
<211>  20
<212>  DNA
<213>  synthetic construct

<400>  1367
actactattt gggcgaacac                                                  20

<210>  1368
<211>  21
<212>  DNA
<213>  synthetic construct

<400>  1368
aatgacgatg aaacttcctt t                                                21

<210>  1369
<211>  19
<212>  DNA
<213>  synthetic construct

<400>  1369
acccatacgc ttgaaatct                                                   19

<210>  1370
<211>  24
<212>  DNA
<213>  synthetic construct

<400>  1370
catcaattgt gtgataatga taag                                             24

<210>  1371
<211>  20
<212>  DNA
<213>  synthetic construct

<400>  1371
gctgtttgca attctttagc                                                  20

<210>  1372
<211>  27
<212>  DNA
<213>  synthetic construct

<400>  1372
acgtatatcg tcctgaatat tttctaa                                          27

<210>  1373
<211>  20
<212>  DNA
<213>  synthetic construct

<400>  1373

ctacgctagt cgaaggctat                                                    20


<210>  1374
<211>  20
<212>  DNA
<213>  synthetic construct

<400>  1374
aatcgtccac ttgtcttttg                                                    20


<210>  1375
<211>  23
<212>  DNA
<213>  synthetic construct

<400>  1375
aatttcacta ggcgtataat cag                                                23


<210>  1376
<211>  21
<212>  DNA
<213>  synthetic construct

<400>  1376
ccaagatgct aatgtgtctt c                                                  21


<210>  1377
<211>  20
<212>  DNA
<213>  synthetic construct

<400>  1377
tttgacctgg agttaaatgg                                                    20


<210>  1378
<211>  20
<212>  DNA
<213>  synthetic construct

<400>  1378
tgcgaattaa acagttaggc                                                    20


<210>  1379
<211>  18
<212>  DNA
<213>  synthetic construct

<400>  1379
ttttagttgc cgatggtg                                                      18


<210>  1380
<211>  25
<212>  DNA
<213>  synthetic construct

<400>  1380

tttaatgttg ttatttgttg ctaaa                                          25

<210>  1381
<211>  24
<212>  DNA
<213>  synthetic construct

<400>  1381
aaatagagca tgatcaaaga taaa                                           24

<210>  1382
<211>  23
<212>  DNA
<213>  synthetic construct

<400>  1382
tatcaatctt tcaagcagtt atg                                            23

<210>  1383
<211>  25
<212>  DNA
<213>  synthetic construct

<400>  1383
aaataaaata tagcgatggt aaatg                                          25

<210>  1384
<211>  22
<212>  DNA
<213>  synthetic construct

<400>  1384
caaaggagtg tgattttatg tc                                             22

<210>  1385
<211>  20
<212>  DNA
<213>  synthetic construct

<400>  1385
tggattagct ttaggaccag                                                20

<210>  1386
<211>  20
<212>  DNA
<213>  synthetic construct

<400>  1386
gaagtggagc gtaatttgtc                                                20

<210>  1387
<211>  19
<212>  DNA
<213>  synthetic construct

<400>  1387

ctgcttgaac atcatctgg                                                    19

<210>  1388
<211>  21
<212>  DNA
<213>  synthetic construct

<400>  1388
tgtcaggtat cgtagatgca a                                                21

<210>  1389
<211>  26
<212>  DNA
<213>  synthetic construct

<400>  1389
ggaataatcc aataataaag tctaca                                           26

<210>  1390
<211>  19
<212>  DNA
<213>  synthetic construct

<400>  1390
gaaataaccg cattccaac                                                   19

<210>  1391
<211>  25
<212>  DNA
<213>  synthetic construct

<400>  1391
gatttgtata tttagtgttt cagca                                            25

<210>  1392
<211>  19
<212>  DNA
<213>  synthetic construct

<400>  1392
aagagaacca aaccaatcg                                                   19

<210>  1393
<211>  20
<212>  DNA
<213>  synthetic construct

<400>  1393
ttctttcggc ttaaagtcat                                                  20

<210>  1394
<211>  25
<212>  DNA
<213>  synthetic construct

<400>  1394

aaaagatatg acataatgtt acgaa                                          25

<210>  1395
<211>  23
<212>  DNA
<213>  synthetic construct

<400>  1395
atagtatatg tagctggcgg ttg                                            23

<210>  1396
<211>  21
<212>  DNA
<213>  synthetic construct

<400>  1396
catccaattt acagaaccat c                                              21

<210>  1397
<211>  22
<212>  DNA
<213>  synthetic construct

<400>  1397
cgtcatcaga agtagaagtg ag                                             22

<210>  1398
<211>  26
<212>  DNA
<213>  synthetic construct

<400>  1398
tgtatcaact ccactttatt catatt                                         26

<210>  1399
<211>  22
<212>  DNA
<213>  synthetic construct

<400>  1399
tttttcgtat cttatgggtc tt                                             22

<210>  1400
<211>  21
<212>  DNA
<213>  synthetic construct

<400>  1400
agcaagttct ttgttaattg c                                              21

<210>  1401
<211>  21
<212>  DNA
<213>  synthetic construct

<400>  1401

ttcgtttgat tgaacttgaa c 21

<210> 1402
<211> 25
<212> DNA
<213> synthetic construct

<400> 1402
tattgtcaaa gtcacaacaa ttaga 25

<210> 1403
<211> 20
<212> DNA
<213> synthetic construct

<400> 1403
acgttgtatc gcattgtaaa 20

<210> 1404
<211> 25
<212> DNA
<213> synthetic construct

<400> 1404
tcagatataa acaatttaac aagga 25

<210> 1405
<211> 20
<212> DNA
<213> synthetic construct

<400> 1405
gaaggtgcct gatctgtaat 20

<210> 1406
<211> 20
<212> DNA
<213> synthetic construct

<400> 1406
taatcaaacg caacaacaac 20

<210> 1407
<211> 19
<212> DNA
<213> synthetic construct

<400> 1407
catcgccatt atctgtagg 19

<210> 1408
<211> 20
<212> DNA
<213> synthetic construct

<400> 1408

aaacgcattg ttaagagacc                                                    20

<210>  1409
<211>  20
<212>  DNA
<213>  synthetic construct

<400>  1409
acctctagca ccaatcaaag                                                    20

<210>  1410
<211>  21
<212>  DNA
<213>  synthetic construct

<400>  1410
caggtaagtc aaagtctggt g                                                  21

<210>  1411
<211>  20
<212>  DNA
<213>  synthetic construct

<400>  1411
attgccacct tgtatattgg                                                    20

<210>  1412
<211>  24
<212>  DNA
<213>  synthetic construct

<400>  1412
gattaatgac atcaagggtt tagt                                               24

<210>  1413
<211>  19
<212>  DNA
<213>  synthetic construct

<400>  1413
gcaaagcttg cataacatc                                                     19

<210>  1414
<211>  20
<212>  DNA
<213>  synthetic construct

<400>  1414
tctggtgaag gtttaggaag                                                    20

<210>  1415
<211>  20
<212>  DNA
<213>  synthetic construct

<400>  1415

accacttgac tcgaacaaac                                                20

<210>  1416
<211>  20
<212>  DNA
<213>  synthetic construct

<400>  1416
caccaaactc aggcttattc                                                20

<210>  1417
<211>  19
<212>  DNA
<213>  synthetic construct

<400>  1417
agaccatacc cttgaccac                                                 19

<210>  1418
<211>  21
<212>  DNA
<213>  synthetic construct

<400>  1418
gaatgcaaag aaacattgaa a                                              21

<210>  1419
<211>  19
<212>  DNA
<213>  synthetic construct

<400>  1419
ccgtatgcaa atcatcact                                                 19

<210>  1420
<211>  20
<212>  DNA
<213>  synthetic construct

<400>  1420
tgtctgctgc tagtgaatcc                                                20

<210>  1421
<211>  20
<212>  DNA
<213>  synthetic construct

<400>  1421
aagcaatttg agctttagca                                                20

<210>  1422
<211>  19
<212>  DNA
<213>  synthetic construct

<400>  1422

tgacaggttc attggtgtc                                                    19

<210>  1423
<211>  19
<212>  DNA
<213>  synthetic construct

<400>  1423
acagcctcaa attctccac                                                    19

<210>  1424
<211>  20
<212>  DNA
<213>  synthetic construct

<400>  1424
tcagatggtg atgaactgtc                                                   20

<210>  1425
<211>  20
<212>  DNA
<213>  synthetic construct

<400>  1425
gacttcttgt tgtggaggag                                                   20

<210>  1426
<211>  20
<212>  DNA
<213>  synthetic construct

<400>  1426
aactagggct gctaatgttg                                                   20

<210>  1427
<211>  19
<212>  DNA
<213>  synthetic construct

<400>  1427
ttgagcaaga gatcctgaa                                                    19

<210>  1428
<211>  19
<212>  DNA
<213>  synthetic construct

<400>  1428
agtcagagca gggtcaatc                                                    19

<210>  1429
<211>  20
<212>  DNA
<213>  synthetic construct

<400>  1429

atgtcgggta agacacaatc                                                    20

<210>  1430
<211>  19
<212>  DNA
<213>  synthetic construct

<400>  1430
gtcaacaaca aggcaagac                                                     19

<210>  1431
<211>  20
<212>  DNA
<213>  synthetic construct

<400>  1431
ctagccacga ctctttgaac                                                    20

<210>  1432
<211>  20
<212>  DNA
<213>  synthetic construct

<400>  1432
ctgtcaagaa actgacgttg                                                    20

<210>  1433
<211>  22
<212>  DNA
<213>  synthetic construct

<400>  1433
ttgttgtaga attggatgaa tg                                                 22

<210>  1434
<211>  21
<212>  DNA
<213>  synthetic construct

<400>  1434
ttggctcgat taagaaataa a                                                  21

<210>  1435
<211>  19
<212>  DNA
<213>  synthetic construct

<400>  1435
ctgaaacatc accagcaga                                                     19

<210>  1436
<211>  20
<212>  DNA
<213>  synthetic construct

<400>  1436

gctccatcta gcaactcatc                                                    20

<210>  1437
<211>  20
<212>  DNA
<213>  synthetic construct

<400>  1437
gagaccaaca aacacgaaac                                                    20

<210>  1438
<211>  20
<212>  DNA
<213>  synthetic construct

<400>  1438
gacactccgt cagattcaac                                                    20

<210>  1439
<211>  20
<212>  DNA
<213>  synthetic construct

<400>  1439
attcccgtca tcaacattag                                                    20

<210>  1440
<211>  20
<212>  DNA
<213>  synthetic construct

<400>  1440
acatagtcag ccacaaccac                                                    20

<210>  1441
<211>  20
<212>  DNA
<213>  synthetic construct

<400>  1441
gcactcgaat tgttatttcc                                                    20

<210>  1442
<211>  21
<212>  DNA
<213>  synthetic construct

<400>  1442
tcaagaaagc tactgatggt g                                                  21

<210>  1443
<211>  20
<212>  DNA
<213>  synthetic construct

<400>  1443

atttactggt gtccaagacg                                                    20

<210>  1444
<211>  20
<212>  DNA
<213>  synthetic construct

<400>  1444
ctcagtcctt tgctacaacc                                                    20

<210>  1445
<211>  20
<212>  DNA
<213>  synthetic construct

<400>  1445
aacggtatca gtttcaccag                                                    20

<210>  1446
<211>  20
<212>  DNA
<213>  synthetic construct

<400>  1446
aacggtcata tccaaagaag                                                    20

<210>  1447
<211>  20
<212>  DNA
<213>  synthetic construct

<400>  1447
acccatgaaa caaggtgtag                                                    20

<210>  1448
<211>  20
<212>  DNA
<213>  synthetic construct

<400>  1448
aacattagaa acggaacagg                                                    20

<210>  1449
<211>  20
<212>  DNA
<213>  synthetic construct

<400>  1449
ctcagttctg catttggttc                                                    20

<210>  1450
<211>  20
<212>  DNA
<213>  synthetic construct

<400>  1450

caaagttcca ccatttcaac                                                20

<210>  1451
<211>  20
<212>  DNA
<213>  synthetic construct

<400>  1451
attgatccta atgcttccac                                                20

<210>  1452
<211>  20
<212>  DNA
<213>  synthetic construct

<400>  1452
atcgacatca acaggcttac                                                20

<210>  1453
<211>  20
<212>  DNA
<213>  synthetic construct

<400>  1453
tctttggagg atatggacac                                                20

<210>  1454
<211>  19
<212>  DNA
<213>  synthetic construct

<400>  1454
caatagcaca ggcacaatc                                                 19

<210>  1455
<211>  20
<212>  DNA
<213>  synthetic construct

<400>  1455
aaatctcatc cctcttcacc                                                20

<210>  1456
<211>  20
<212>  DNA
<213>  synthetic construct

<400>  1456
ttcattcaaa ccagccttac                                                20

<210>  1457
<211>  21
<212>  DNA
<213>  synthetic construct

<400>  1457

ttcaccatac catcttacac c                                                21

<210>  1458
<211>  24
<212>  DNA
<213>  synthetic construct

<400>  1458
cacaaggtta tacattcaga aaac                                             24

<210>  1459
<211>  22
<212>  DNA
<213>  synthetic construct

<400>  1459
ctacatacca tttcaacacc ac                                               22

<210>  1460
<211>  21
<212>  DNA
<213>  synthetic construct

<400>  1460
aaaccataaa tcaacaacca c                                                21

<210>  1461
<211>  20
<212>  DNA
<213>  synthetic construct

<400>  1461
caccaacaca tctcattgtc                                                  20

<210>  1462
<211>  19
<212>  DNA
<213>  synthetic construct

<400>  1462
tattatggcg attccacag                                                   19

<210>  1463
<211>  18
<212>  DNA
<213>  synthetic construct

<400>  1463
cagcccaaat tcttgttc                                                    18

<210>  1464
<211>  20
<212>  DNA
<213>  synthetic construct

<400>  1464

cgtttgttat ttgctgttcc                                                    20

<210>  1465
<211>  20
<212>  DNA
<213>  synthetic construct

<400>  1465
gctgctttca tagtttcacc                                                    20

<210>  1466
<211>  20
<212>  DNA
<213>  synthetic construct

<400>  1466
agctaaatcc aaagacgatg                                                    20

<210>  1467
<211>  20
<212>  DNA
<213>  synthetic construct

<400>  1467
ccttgattat gcgagaaatg                                                    20

<210>  1468
<211>  20
<212>  DNA
<213>  synthetic construct

<400>  1468
gctaccactc caaacacttc                                                    20

<210>  1469
<211>  20
<212>  DNA
<213>  synthetic construct

<400>  1469
agacgacagc actagattcc                                                    20

<210>  1470
<211>  20
<212>  DNA
<213>  synthetic construct

<400>  1470
ttggtgggtt agaagttgag                                                    20

<210>  1471
<211>  20
<212>  DNA
<213>  synthetic construct

<400>  1471

ttgaaacagc atgaacaatc                                                    20

<210>  1472
<211>  21
<212>  DNA
<213>  synthetic construct

<400>  1472
caagagaaag ggaaagaaga g                                                  21

<210>  1473
<211>  20
<212>  DNA
<213>  synthetic construct

<400>  1473
aggtttcgtc gtatgaagtg                                                    20

<210>  1474
<211>  27
<212>  DNA
<213>  synthetic construct

<400>  1474
cgttaactca gtcatatact acatttt                                            27

<210>  1475
<211>  20
<212>  DNA
<213>  synthetic construct

<400>  1475
gcaataaaag aaaccccaat                                                    20

<210>  1476
<211>  20
<212>  DNA
<213>  synthetic construct

<400>  1476
gatccagatg ctgtaaccac                                                    20

<210>  1477
<211>  20
<212>  DNA
<213>  synthetic construct

<400>  1477
catcgtaaat aacccgtttc                                                    20

<210>  1478
<211>  20
<212>  DNA
<213>  synthetic construct

<400>  1478

taatttccct tgttgtttcc                                                    20

<210>  1479
<211>  20
<212>  DNA
<213>  synthetic construct

<400>  1479
tcaatccaac catcttcttc                                                    20

<210>  1480
<211>  20
<212>  DNA
<213>  synthetic construct

<400>  1480
acattcattg ggttcatctc                                                    20

<210>  1481
<211>  20
<212>  DNA
<213>  synthetic construct

<400>  1481
attggtggta attgtgttcc                                                    20

<210>  1482
<211>  20
<212>  DNA
<213>  synthetic construct

<400>  1482
tgtcccagaa agtgctaaac                                                    20

<210>  1483
<211>  18
<212>  DNA
<213>  synthetic construct

<400>  1483
aataccaccg gaaactgg                                                      18

<210>  1484
<211>  22
<212>  DNA
<213>  synthetic construct

<400>  1484
ttagctcatc aacatcatca ac                                                 22

<210>  1485
<211>  21
<212>  DNA
<213>  synthetic construct

<400>  1485

gctttaattt gacttggagt g                                                21

<210>  1486
<211>  20
<212>  DNA
<213>  synthetic construct

<400>  1486
actgtcgttt ctggtcattc                                                  20

<210>  1487
<211>  20
<212>  DNA
<213>  synthetic construct

<400>  1487
acgagtgaaa tctcttggtg                                                  20

<210>  1488
<211>  20
<212>  DNA
<213>  synthetic construct

<400>  1488
ggtacgaaca gacaaacacc                                                  20

<210>  1489
<211>  20
<212>  DNA
<213>  synthetic construct

<400>  1489
aacagctcta atggcaaatc                                                  20

<210>  1490
<211>  20
<212>  DNA
<213>  synthetic construct

<400>  1490
ttagccaagt ttgaatcgtc                                                  20

<210>  1491
<211>  20
<212>  DNA
<213>  synthetic construct

<400>  1491
aatttcttcg tcatcattgg                                                  20

<210>  1492
<211>  20
<212>  DNA
<213>  synthetic construct

<400>  1492

tcaatcttgg ctgctacttc                                                    20

<210>  1493
<211>  20
<212>  DNA
<213>  synthetic construct

<400>  1493
ggctaatgaa actctgatgg                                                    20

<210>  1494
<211>  20
<212>  DNA
<213>  synthetic construct

<400>  1494
attgggtatc aacaccattc                                                    20

<210>  1495
<211>  20
<212>  DNA
<213>  synthetic construct

<400>  1495
ccatagttgt ttggttcctg                                                    20

<210>  1496
<211>  20
<212>  DNA
<213>  synthetic construct

<400>  1496
attagctgaa catgccagag                                                    20

<210>  1497
<211>  18
<212>  DNA
<213>  synthetic construct

<400>  1497
   atctccattg cacccttc                                                     18

<210>  1498
<211>  19
<212>  DNA
<213>  synthetic construct

<400>  1498
gatttacacg cctcacaaa                                                     19

<210>  1499
<211>  22
<212>  DNA
<213>  synthetic construct

<400>  1499

gcaataacag cagaaatatc aa                                              22


<210>  1500
<211>  20
<212>  DNA
<213>  synthetic construct

<400>  1500
taccacgata gctccatttc                                                 20


<210>  1501
<211>  19
<212>  DNA
<213>  synthetic construct

<400>  1501
acccaaagtt ctcttgctg                                                  19


<210>  1502
<211>  20
<212>  DNA
<213>  synthetic construct

<400>  1502
gtggtgttac tgttggtgaa                                                 20


<210>  1503
<211>  20
<212>  DNA
<213>  synthetic construct

<400>  1503
gtagcatccc aagtcaaatc                                                 20


<210>  1504
<211>  25
<212>  DNA
<213>  synthetic construct

<400>  1504
aagagatgat cctcatacta ttgaa                                           25


<210>  1505
<211>  22
<212>  DNA
<213>  synthetic construct

<400>  1505
caagacaaca catttcttct tc                                              22


<210>  1506
<211>  20
<212>  DNA
<213>  synthetic construct

<400>  1506

taagagagat ggccgtaaag                                                    20

<210>  1507
<211>  19
<212>  DNA
<213>  synthetic construct

<400>  1507
ttaccgtgaa taccgacag                                                     19

<210>  1508
<211>  19
<212>  DNA
<213>  synthetic construct

<400>  1508
ttttggacct caaatggac                                                     19

<210>  1509
<211>  24
<212>  DNA
<213>  synthetic construct

<400>  1509
aaatagtctt cttttatgct tcct                                               24

<210>  1510
<211>  23
<212>  DNA
<213>  synthetic construct

<400>  1510
ccaagaactt accattattg aac                                                23

<210>  1511
<211>  20
<212>  DNA
<213>  synthetic construct

<400>  1511
tttcgtttct agccaatctc                                                    20

<210>  1512
<211>  22
<212>  DNA
<213>  synthetic construct

<400>  1512
ttaaaggatt caaaaagggt gt                                                 22

<210>  1513
<211>  21
<212>  DNA
<213>  synthetic construct

<400>  1513

aataattctt gtgctggttc a                                                21

<210>  1514
<211>  18
<212>  DNA
<213>  synthetic construct

<400>  1514
catggcacca gaaagaac                                                    18

<210>  1515
<211>  20
<212>  DNA
<213>  synthetic construct

<400>  1515
aattgttggt ttgcagtagg                                                  20

<210>  1516
<211>  20
<212>  DNA
<213>  synthetic construct

<400>  1516
tataatgccc cgaaaataaa                                                  20

<210>  1517
<211>  22
<212>  DNA
<213>  synthetic construct

<400>  1517
ttaagcggat atctatcgta cc                                               22

<210>  1518
<211>  21
<212>  DNA
<213>  synthetic construct

<400>  1518
tggaacctgt ttgtacttga c                                                21

<210>  1519
<211>  19
<212>  DNA
<213>  synthetic construct

<400>  1519
ctagcccact ttccaattc                                                   19

<210>  1520
<211>  18
<212>  DNA
<213>  synthetic construct

<400>  1520

gatttcctag ccggaatg                                                    18

<210>  1521
<211>  26
<212>  DNA
<213>  synthetic construct

<400>  1521
attatgtaac cttagtcgta attgtc                                          26

<210>  1522
<211>  18
<212>  DNA
<213>  synthetic construct

<400>  1522
aagacgactg agcgtgtc                                                    18

<210>  1523
<211>  22
<212>  DNA
<213>  synthetic construct

<400>  1523
tggactggaa gataagctag ag                                              22

<210>  1524
<211>  21
<212>  DNA
<213>  synthetic construct

<400>  1524
acatgtcaag aggattgttc a                                               21

<210>  1525
<211>  24
<212>  DNA
<213>  synthetic construct

<400>  1525
tttacattga ttgattaaat ttgc                                            24

<210>  1526
<211>  20
<212>  DNA
<213>  synthetic construct

<400>  1526
gccgaagata ctaaaccaaa                                                 20

<210>  1527
<211>  21
<212>  DNA
<213>  synthetic construct

<400>  1527

tcagaaacat cagcagtaac a                                                21

<210>  1528
<211>  20
<212>  DNA
<213>  synthetic construct

<400>  1528
tctgatgttg ctgtttgttc                                                  20

<210>  1529
<211>  20
<212>  DNA
<213>  synthetic construct

<400>  1529
atcttcagtt tcaccagtcc                                                  20

<210>  1530
<211>  20
<212>  DNA
<213>  synthetic construct

<400>  1530
tcgaccatac catccaatac                                                  20

<210>  1531
<211>  21
<212>  DNA
<213>  synthetic construct

<400>  1531
ttcacaaaga aacctagcaa c                                                21

<210>  1532
<211>  20
<212>  DNA
<213>  synthetic construct

<400>  1532
agaaatttgg cctgatgtta                                                  20

<210>  1533
<211>  20
<212>  DNA
<213>  synthetic construct

<400>  1533
ggtgtttgtc aatcccaata                                                  20

<210>  1534
<211>  20
<212>  DNA
<213>  synthetic construct

<400>  1534

aacagcaacc agaaatcaag                                                    20

<210>  1535
<211>  23
<212>  DNA
<213>  synthetic construct

<400>  1535
ttaaatcaac aggaatcttc aac                                                23

<210>  1536
<211>  20
<212>  DNA
<213>  synthetic construct

<400>  1536
gctaccactt taaccgacac                                                    20

<210>  1537
<211>  20
<212>  DNA
<213>  synthetic construct

<400>  1537
cttctccacc attagcactc                                                    20

<210>  1538
<211>  20
<212>  DNA
<213>  synthetic construct

<400>  1538
tttcatcacc acctcaagtc                                                    20

<210>  1539
<211>  20
<212>  DNA
<213>  synthetic construct

<400>  1539
atttcaacaa gccaatcaac                                                    20

<210>  1540
<211>  20
<212>  DNA
<213>  synthetic construct

<400>  1540
ccagcaaaca attcctaatc                                                    20

<210>  1541
<211>  19
<212>  DNA
<213>  synthetic construct

<400>  1541

ttcccattac cgctagaac                                                    19


<210>  1542
<211>  20
<212>  DNA
<213>  synthetic construct

<400>  1542
gctcgtttga ttagatttgg                                                   20


<210>  1543
<211>  19
<212>  DNA
<213>  synthetic construct

<400>  1543
tgttcacgga ctaaagcag                                                    19


<210>  1544
<211>  20
<212>  DNA
<213>  synthetic construct

<400>  1544
cccaactaat tcagcatcac                                                   20


<210>  1545
<211>  20
<212>  DNA
<213>  synthetic construct

<400>  1545
gtaccaattc ggctttactg                                                   20


<210>  1546
<211>  18
<212>  DNA
<213>  synthetic construct

<400>  1546
aacagcaagc tgctcagt                                                     18


<210>  1547
<211>  22
<212>  DNA
<213>  synthetic construct

<400>  1547
ttacaaaaac ccacttcttt tc                                                22


<210>  1548
<211>  20
<212>  DNA
<213>  synthetic construct

<400>  1548

gtcctttcaa gtgtttgtgg                                                    20

<210>  1549
<211>  20
<212>  DNA
<213>  synthetic construct

<400>  1549
tattgttgtt gtgggtgttg                                                    20

<210>  1550
<211>  20
<212>  DNA
<213>  synthetic construct

<400>  1550
ataacccaca taaggtctgg                                                    20

<210>  1551
<211>  20
<212>  DNA
<213>  synthetic construct

<400>  1551
agctagttcg tggatctttg                                                    20

<210>  1552
<211>  23
<212>  DNA
<213>  synthetic construct

<400>  1552
tgactacgat gactactgaa gaa                                                23

<210>  1553
<211>  20
<212>  DNA
<213>  synthetic construct

<400>  1553
ttttagcaac aatgggtttt                                                    20

<210>  1554
<211>  22
<212>  DNA
<213>  synthetic construct

<400>  1554
attggtttca aacagttact ca                                                 22

<210>  1555
<211>  22
<212>  DNA
<213>  synthetic construct

<400>  1555

attctcttca acatgcttct ct                                                22

<210>  1556
<211>  20
<212>  DNA
<213>  synthetic construct

<400>  1556
aaagagctaa ccacgtcaag                                                    20

<210>  1557
<211>  20
<212>  DNA
<213>  synthetic construct

<400>  1557
gatcgacaaa gtcaaggaac                                                    20

<210>  1558
<211>  23
<212>  DNA
<213>  synthetic construct

<400>  1558
aggtggtgtc atgaaattat tag                                                23

<210>  1559
<211>  20
<212>  DNA
<213>  synthetic construct

<400>  1559
acaggaattc gattggttag                                                    20

<210>  1560
<211>  18
<212>  DNA
<213>  synthetic construct

<400>  1560
ttgcccaatc agcattac                                                      18

<210>  1561
<211>  20
<212>  DNA
<213>  synthetic construct

<400>  1561
acaccttggt cttgtttcac                                                    20

<210>  1562
<211>  24
<212>  DNA
<213>  synthetic construct

<400>  1562

atatcgaagt ggtctattta gagg                                              24

<210>  1563
<211>  23
<212>  DNA
<213>  synthetic construct

<400>  1563
ttgattctga acgatcatag act                                               23

<210>  1564
<211>  21
<212>  DNA
<213>  synthetic construct

<400>  1564
aattaaacaa agcaggaatc a                                                 21

<210>  1565
<211>  19
<212>  DNA
<213>  synthetic construct

<400>  1565
ccaccacatg aaatggtaa                                                    19

<210>  1566
<211>  19
<212>  DNA
<213>  synthetic construct

<400>  1566
agttgcacaa gtagcgatg                                                    19

<210>  1567
<211>  20
<212>  DNA
<213>  synthetic construct

<400>  1567
tgagaagtca agcgagaaac                                                   20

<210>  1568
<211>  19
<212>  DNA
<213>  synthetic construct

<400>  1568
cgcagacaag aaagacaac                                                    19

<210>  1569
<211>  18
<212>  DNA
<213>  synthetic construct

<400>  1569

gtgtggtagc cgtttgag                                                    18

<210>  1570
<211>  24
<212>  DNA
<213>  synthetic construct

<400>  1570
ttttcacttt taggagctat tttt                                            24

<210>  1571
<211>  20
<212>  DNA
<213>  synthetic construct

<400>  1571
ctttttcatc tgccgtagtc                                                  20

<210>  1572
<211>  23
<212>  DNA
<213>  synthetic construct

<400>  1572
caaaaccaac agaagaagaa tta                                              23

<210>  1573
<211>  20
<212>  DNA
<213>  synthetic construct

<400>  1573
cgaacttcta ctcggtgcat                                                  20

<210>  1574
<211>  20
<212>  DNA
<213>  synthetic construct

<400>  1574
ggcttagttg tcagttgtgg                                                  20

<210>  1575
<211>  21
<212>  DNA
<213>  synthetic construct

<400>  1575
aattccttgc atttgataca g                                                21

<210>  1576
<211>  18
<212>  DNA
<213>  synthetic construct

<400>  1576

gtttgattgt tgcgaggt                                                    18

<210>  1577
<211>  24
<212>  DNA
<213>  synthetic construct

<400>  1577
aatctactca attcattttt cctt                                             24

<210>  1578
<211>  26
<212>  DNA
<213>  synthetic construct

<400>  1578
ttttactaaa ccattaggtg taaaat                                           26

<210>  1579
<211>  20
<212>  DNA
<213>  synthetic construct

<400>  1579
caaaaacttg tggaccttct                                                  20

<210>  1580
<211>  23
<212>  DNA
<213>  synthetic construct

<400>  1580
catgtattgg ttgttagata ggg                                              23

<210>  1581
<211>  19
<212>  DNA
<213>  synthetic construct

<400>  1581
gaggataagg ccatgactg                                                   19

<210>  1582
<211>  20
<212>  DNA
<213>  synthetic construct

<400>  1582
gatcaggaag atcaatcagg                                                  20

<210>  1583
<211>  20
<212>  DNA
<213>  synthetic construct

<400>  1583

cttagttccc acaccatcac                                                    20

<210>  1584
<211>  19
<212>  DNA
<213>  synthetic construct

<400>  1584
gttgggctac tcttggttg                                                     19

<210>  1585
<211>  21
<212>  DNA
<213>  synthetic construct

<400>  1585
gtgttgatac gttgttcttc c                                                  21

<210>  1586
<211>  22
<212>  DNA
<213>  synthetic construct

<400>  1586
ttacgttaga agaagcatac ca                                                 22

<210>  1587
<211>  18
<212>  DNA
<213>  synthetic construct

<400>  1587
aggacgatac agggcatt                                                      18

<210>  1588
<211>  18
<212>  DNA
<213>  synthetic construct

<400>  1588
tttatgaagg cccaaaga                                                      18

<210>  1589
<211>  20
<212>  DNA
<213>  synthetic construct

<400>  1589
agccagaatt agcatcaaca                                                    20

<210>  1590
<211>  19
<212>  DNA
<213>  synthetic construct

<400>  1590

tttacctcac cgaccaatc                                                    19

<210>  1591
<211>  18
<212>  DNA
<213>  synthetic construct

<400>  1591
tggctgaaga agctcaac                                                     18

<210>  1592
<211>  20
<212>  DNA
<213>  synthetic construct

<400>  1592
tctgtttacg ttagcggtct                                                   20

<210>  1593
<211>  21
<212>  DNA
<213>  synthetic construct

<400>  1593
tccctaagtt cattgacaac a                                                 21

<210>  1594
<211>  18
<212>  DNA
<213>  synthetic construct

<400>  1594
ggcgacgaag ttaaagtg                                                     18

<210>  1595
<211>  18
<212>  DNA
<213>  synthetic construct

<400>  1595
aatggtgatc ggtccttc                                                     18

<210>  1596
<211>  21
<212>  DNA
<213>  synthetic construct

<400>  1596
gatacgaaga agatagcgaa a                                                 21

<210>  1597
<211>  19
<212>  DNA
<213>  synthetic construct

<400>  1597

acaatagcca tcccagcta                                                    19


<210>  1598
<211>  22
<212>  DNA
<213>  synthetic construct

<400>  1598
aagatggtac gtgtattcgt tt                                                 22


<210>  1599
<211>  20
<212>  DNA
<213>  synthetic construct

<400>  1599
aattacttct gtcgccttca                                                    20


<210>  1600
<211>  20
<212>  DNA
<213>  synthetic construct

<400>  1600
ttatttctgt tgagggcaaa                                                    20


<210>  1601
<211>  20
<212>  DNA
<213>  synthetic construct

<400>  1601
tcttcttcca cttcttctgg                                                    20


<210>  1602
<211>  20
<212>  DNA
<213>  synthetic construct

<400>  1602
ttgatcgttt tgacgtaatg                                                    20


<210>  1603
<211>  21
<212>  DNA
<213>  synthetic construct

<400>  1603
taatcgtttc tttctctttg g                                                  21


<210>  1604
<211>  21
<212>  DNA
<213>  synthetic construct

<400>  1604

agcgactaga gagcatacaa g                                                21

<210>  1605
<211>  22
<212>  DNA
<213>  synthetic construct

<400>  1605
tcagagcatc aaataaatca ac                                               22

<210>  1606
<211>  19
<212>  DNA
<213>  synthetic construct

<400>  1606
aaattgttgc acgtatgga                                                   19

<210>  1607
<211>  18
<212>  DNA
<213>  synthetic construct

<400>  1607
tttgccgact aaacacct                                                    18

<210>  1608
<211>  19
<212>  DNA
<213>  synthetic construct

<400>  1608
caatggaaca aaggccact                                                   19

<210>  1609
<211>  18
<212>  DNA
<213>  synthetic construct

<400>  1609
tgcttggcga actaaagc                                                    18

<210>  1610
<211>  18
<212>  DNA
<213>  synthetic construct

<400>  1610
ctgcggttaa agtcgttg                                                    18

<210>  1611
<211>  20
<212>  DNA
<213>  synthetic construct

<400>  1611

aatcacgtag ccttacatcc                                                    20

<210>  1612
<211>  20
<212>  DNA
<213>  synthetic construct

<400>  1612
atccgactat gcgtttactg                                                    20

<210>  1613
<211>  20
<212>  DNA
<213>  synthetic construct

<400>  1613
tatgttcgcc aatttctttc                                                    20

<210>  1614
<211>  20
<212>  DNA
<213>  synthetic construct

<400>  1614
aggatcaatc gtaaatggtg                                                    20

<210>  1615
<211>  18
<212>  DNA
<213>  synthetic construct

<400>  1615
ttcgctgcaa tcttatcc                                                      18

<210>  1616
<211>  20
<212>  DNA
<213>  synthetic construct

<400>  1616
aagatggtga aacattggtg                                                    20

<210>  1617
<211>  21
<212>  DNA
<213>  synthetic construct

<400>  1617
tctttaatct cagctttttg c                                                  21

<210>  1618
<211>  20
<212>  DNA
<213>  synthetic construct

<400>  1618

gtttccgttc aaataaccac                                                    20

<210>  1619
<211>  21
<212>  DNA
<213>  synthetic construct

<400>  1619
caattcacgc acttctttat c                                                  21

<210>  1620
<211>  18
<212>  DNA
<213>  synthetic construct

<400>  1620
aattgcagga ggttcaca                                                      18

<210>  1621
<211>  20
<212>  DNA
<213>  synthetic construct

<400>  1621
cttcagaggt cgctaaatca                                                    20

<210>  1622
<211>  20
<212>  DNA
<213>  synthetic construct

<400>  1622
aatgcgtgat cagggtgtat                                                    20

<210>  1623
<211>  27
<212>  DNA
<213>  synthetic construct

<400>  1623
tgtaattatg agtcgaacat ttttatt                                            27

<210>  1624
<211>  20
<212>  DNA
<213>  synthetic construct

<400>  1624
ctcaacagct tcaacaatcc                                                    20

<210>  1625
<211>  20
<212>  DNA
<213>  synthetic construct

<400>  1625

ccactattcg atcttccttg                                                    20


<210>  1626
<211>  20
<212>  DNA
<213>  synthetic construct

<400>  1626
aggtttagaa gtgggggagt                                                    20


<210>  1627
<211>  18
<212>  DNA
<213>  synthetic construct

<400>  1627
atcggcgctt taatcgtt                                                      18


<210>  1628
<211>  20
<212>  DNA
<213>  synthetic construct

<400>  1628
aacgcacatc tgaaagctac                                                    20


<210>  1629
<211>  19
<212>  DNA
<213>  synthetic construct

<400>  1629
tacctgaacg agctgaatg                                                     19


<210>  1630
<211>  20
<212>  DNA
<213>  synthetic construct

<400>  1630
atggagggtg ataacatgaa                                                    20


<210>  1631
<211>  23
<212>  DNA
<213>  synthetic construct

<400>  1631
aagatagttc tgataaccaa tcg                                                23


<210>  1632
<211>  19
<212>  DNA
<213>  synthetic construct

<400>  1632

caggaggaac attggttgt                                                    19

<210>  1633
<211>  19
<212>  DNA
<213>  synthetic construct

<400>  1633
cgtcataccc tggattgtt                                                    19

<210>  1634
<211>  22
<212>  DNA
<213>  synthetic construct

<400>  1634
cgacagaact tgctaaagta ga                                                22

<210>  1635
<211>  18
<212>  DNA
<213>  synthetic construct

<400>  1635
gtgattccgc catagttg                                                     18

<210>  1636
<211>  19
<212>  DNA
<213>  synthetic construct

<400>  1636
cttctttcgt gctttcaac                                                    19

<210>  1637
<211>  21
<212>  DNA
<213>  synthetic construct

<400>  1637
aaatttagat catcattttg c                                                 21

<210>  1638
<211>  22
<212>  DNA
<213>  synthetic construct

<400>  1638
aaacctggat gatagtgata gg                                                22

<210>  1639
<211>  19
<212>  DNA
<213>  synthetic construct

<400>  1639

gactcatttc ctgctgatg 19

<210> 1640
<211> 21
<212> DNA
<213> synthetic construct

<400> 1640
caaggagagc atagtgaatg t 21

<210> 1641
<211> 26
<212> DNA
<213> synthetic construct

<400> 1641
tgcaatagga attaagagta ataaaa 26

<210> 1642
<211> 20
<212> DNA
<213> synthetic construct

<400> 1642
gttagggcac ttagcttttg 20

<210> 1643
<211> 27
<212> DNA
<213> synthetic construct

<400> 1643
tgggtatatt agttttaatt tcctatt 27

<210> 1644
<211> 23
<212> DNA
<213> synthetic construct

<400> 1644
tggaaaattt aagtgtagtt cct 23

<210> 1645
<211> 24
<212> DNA
<213> synthetic construct

<400> 1645
ctactcctaa gcctatggta aaac 24

<210> 1646
<211> 20
<212> DNA
<213> synthetic construct

<400> 1646

agcaaagtgg taacgagaag                                                    20

<210>  1647
<211>  21
<212>  DNA
<213>  synthetic construct

<400>  1647
atacgcatca actcaggata g                                                  21

<210>  1648
<211>  20
<212>  DNA
<213>  synthetic construct

<400>  1648
tagttggaat gaccgagaac                                                    20

<210>  1649
<211>  20
<212>  DNA
<213>  synthetic construct

<400>  1649
agtgtaacgg acgataaagg                                                    20

<210>  1650
<211>  20
<212>  DNA
<213>  synthetic construct

<400>  1650
tcaacgtcac aaacaagaac                                                    20

<210>  1651
<211>  21
<212>  DNA
<213>  synthetic construct

<400>  1651
cctaagagac ttccttcatc c                                                  21

<210>  1652
<211>  18
<212>  DNA
<213>  synthetic construct

<400>  1652
tcggaagtat tgcgtttg                                                      18

<210>  1653
<211>  21
<212>  DNA
<213>  synthetic construct

<400>  1653

ttatcgacat tctctgtttc c                                                21

<210>  1654
<211>  19
<212>  DNA
<213>  synthetic construct

<400>  1654
ttgtctttgg gcttctctc                                                   19

<210>  1655
<211>  21
<212>  DNA
<213>  synthetic construct

<400>  1655
tttctgtatt gcttcttggt c                                                21

<210>  1656
<211>  23
<212>  DNA
<213>  synthetic construct

<400>  1656
ctatagattc cctatcttgt tgg                                              23

<210>  1657
<211>  21
<212>  DNA
<213>  synthetic construct

<400>  1657
atatttaccg caatgaaaca c                                                21

<210>  1658
<211>  27
<212>  DNA
<213>  synthetic construct

<400>  1658
atgtatgtat cttttgttct aagttgt                                          27

<210>  1659
<211>  20
<212>  DNA
<213>  synthetic construct

<400>  1659
aaatcccaat cgtctaagtg                                                  20

<210>  1660
<211>  26
<212>  DNA
<213>  synthetic construct

<400>  1660

aaagtactac cttttattgc cttagt                                          26

<210>  1661
<211>  26
<212>  DNA
<213>  synthetic construct

<400>  1661
tgttttataa gtgttctttg tttttc                                          26

<210>  1662
<211>  21
<212>  DNA
<213>  synthetic construct

<400>  1662
tgaacaaaaa gcacaggata g                                               21

<210>  1663
<211>  18
<212>  DNA
<213>  synthetic construct

<400>  1663
tactaaaccc ccgttgtg                                                   18

<210>  1664
<211>  24
<212>  DNA
<213>  synthetic construct

<400>  1664
tgatgaaaat ttaaaagaag aagc                                            24

<210>  1665
<211>  23
<212>  DNA
<213>  synthetic construct

<400>  1665
taaatcctcc attaaatcct cca                                             23

<210>  1666
<211>  21
<212>  DNA
<213>  synthetic construct

<400>  1666
tcaaatacac gtaagccttc t                                               21

<210>  1667
<211>  22
<212>  DNA
<213>  synthetic construct

<400>  1667

aagttttcat ttcgagaagt tg 22

<210> 1668
<211> 25
<212> DNA
<213> synthetic construct

<400> 1668
cattattttc attaggggat attag 25

<210> 1669
<211> 23
<212> DNA
<213> synthetic construct

<400> 1669
agattcctta ctttagcttt tcc 23

<210> 1670
<211> 19
<212> DNA
<213> synthetic construct

<400> 1670
ttgcgatttc tgttgtagg 19

<210> 1671
<211> 19
<212> DNA
<213> synthetic construct

<400> 1671
aaccaaagtc aaacccatc 19

<210> 1672
<211> 18
<212> DNA
<213> synthetic construct

<400> 1672
aatcaagccg ctgaaaag 18

<210> 1673
<211> 20
<212> DNA
<213> synthetic construct

<400> 1673
caaatttagc tttggcttcc 20

<210> 1674
<211> 21
<212> DNA
<213> synthetic construct

<400> 1674

aaatgggtga aggaagatta g                                                21

<210>  1675
<211>  20
<212>  DNA
<213>  synthetic construct

<400>  1675
accagtgctt tctattccag                                                  20

<210>  1676
<211>  20
<212>  DNA
<213>  synthetic construct

<400>  1676
aggtacaggc atctttgttg                                                  20

<210>  1677
<211>  21
<212>  DNA
<213>  synthetic construct

<400>  1677
ttccataatt gttccatctg t                                                21

<210>  1678
<211>  20
<212>  DNA
<213>  synthetic construct

<400>  1678
ctggttcaaa agaagccatt                                                  20

<210>  1679
<211>  22
<212>  DNA
<213>  synthetic construct

<400>  1679
tttagtttgt caacaatcac tg                                               22

<210>  1680
<211>  20
<212>  DNA
<213>  synthetic construct

<400>  1680
gatcgcgtcg taattagagt                                                  20

<210>  1681
<211>  20
<212>  DNA
<213>  synthetic construct

<400>  1681

ttcaacagtg gcaataatgt                                                20

<210>  1682
<211>  19
<212>  DNA
<213>  synthetic construct

<400>  1682
atctcgcgga acaattaga                                                 19

<210>  1683
<211>  21
<212>  DNA
<213>  synthetic construct

<400>  1683
aataagccaa tccacatatc a                                              21

<210>  1684
<211>  20
<212>  DNA
<213>  synthetic construct

<400>  1684
tcggctctaa tggtatgttc                                                20

<210>  1685
<211>  19
<212>  DNA
<213>  synthetic construct

<400>  1685
gttgacgtgc ttgttcttc                                                 19

<210>  1686
<211>  23
<212>  DNA
<213>  synthetic construct

<400>  1686
gcttcattag cattagaaca atc                                            23

<210>  1687
<211>  20
<212>  DNA
<213>  synthetic construct

<400>  1687
aaacctgcat cccatactac                                                20

<210>  1688
<211>  22
<212>  DNA
<213>  synthetic construct

<400>  1688

ttaactgaac aagaaaagca ag                                              22

<210>  1689
<211>  24
<212>  DNA
<213>  synthetic construct

<400>  1689
ttaaatctcc tgaaatagaa actg                                            24

<210>  1690
<211>  21
<212>  DNA
<213>  synthetic construct

<400>  1690
acaaccaaca gtgaaagcta c                                               21

<210>  1691
<211>  20
<212>  DNA
<213>  synthetic construct

<400>  1691
ttgttagcca attctgcttc                                                 20

<210>  1692
<211>  20
<212>  DNA
<213>  synthetic construct

<400>  1692
cattggtggc tatttcattc                                                 20

<210>  1693
<211>  20
<212>  DNA
<213>  synthetic construct

<400>  1693
ctttctgctt ccattgtttc                                                 20

<210>  1694
<211>  20
<212>  DNA
<213>  synthetic construct

<400>  1694
tatcttagct tcgcaaccag                                                 20

<210>  1695
<211>  20
<212>  DNA
<213>  synthetic construct

<400>  1695

attgtctgcc acgtataacc 20

<210> 1696
<211> 19
<212> DNA
<213> synthetic construct

<400> 1696
gagcaacgtc tcttcttcc 19

<210> 1697
<211> 18
<212> DNA
<213> synthetic construct

<400> 1697
gagcgtgtct atcgcatc 18

<210> 1698
<211> 19
<212> DNA
<213> synthetic construct

<400> 1698
ttcatcgcaa taatcgttc 19

<210> 1699
<211> 18
<212> DNA
<213> synthetic construct

<400> 1699
taacacatcc gccatcac 18

<210> 1700
<211> 22
<212> DNA
<213> synthetic construct

<400> 1700
tcttcatttg ttgaatatgc tg 22

<210> 1701
<211> 20
<212> DNA
<213> synthetic construct

<400> 1701
tggaatcgaa cctgtttatc 20

<210> 1702
<211> 19
<212> DNA
<213> synthetic construct

<400> 1702

attattgtcg cctctttcc                                                    19

<210>  1703
<211>  21
<212>  DNA
<213>  synthetic construct

<400>  1703
gctatttgtt atcgcattct g                                                21

<210>  1704
<211>  20
<212>  DNA
<213>  synthetic construct

<400>  1704
ctcctgatcc tcttcttgtg                                                  20

<210>  1705
<211>  23
<212>  DNA
<213>  synthetic construct

<400>  1705
atgttatact ctgctcttca acc                                              23

<210>  1706
<211>  20
<212>  DNA
<213>  synthetic construct

<400>  1706
cggatgaagg aagtaaagaa                                                  20

<210>  1707
<211>  23
<212>  DNA
<213>  synthetic construct

<400>  1707
catatgctcg taaatacttg aaa                                              23

<210>  1708
<211>  18
<212>  DNA
<213>  synthetic construct

<400>  1708
aaagcgattt gttgctga                                                    18

<210>  1709
<211>  25
<212>  DNA
<213>  synthetic construct

<400>  1709

gaaaattaaa ttagcaagga taaaa                                          25

<210>  1710
<211>  18
<212>  DNA
<213>  synthetic construct

<400>  1710
agccggtaaa ctcagtcc                                                   18

<210>  1711
<211>  19
<212>  DNA
<213>  synthetic construct

<400>  1711
caccataagg caattgaag                                                  19

<210>  1712
<211>  19
<212>  DNA
<213>  synthetic construct

<400>  1712
attactcgtt tcccctgac                                                  19

<210>  1713
<211>  20
<212>  DNA
<213>  synthetic construct

<400>  1713
acaatcgtca caactgacac                                                 20

<210>  1714
<211>  22
<212>  DNA
<213>  synthetic construct

<400>  1714
atgttgaaga aagaaacaat ga                                              22

<210>  1715
<211>  24
<212>  DNA
<213>  synthetic construct

<400>  1715
ctgaataatt tcttaaaggg aaaa                                            24

<210>  1716
<211>  21
<212>  DNA
<213>  synthetic construct

<400>  1716

tcagtcagtt tcttgacctt t                                                21

<210>  1717
<211>  20
<212>  DNA
<213>  synthetic construct

<400>  1717
ttagtccgtt tctttccatc                                                  20

<210>  1718
<211>  20
<212>  DNA
<213>  synthetic construct

<400>  1718
ccaattaggt gaagcagaac                                                  20

<210>  1719
<211>  21
<212>  DNA
<213>  synthetic construct

<400>  1719
tttgatggtt gctaaataag g                                                21

<210>  1720
<211>  20
<212>  DNA
<213>  synthetic construct

<400>  1720
gattgccttt cctttctatg                                                  20

<210>  1721
<211>  19
<212>  DNA
<213>  synthetic construct

<400>  1721
acatctttga cggcttctc                                                   19

<210>  1722
<211>  20
<212>  DNA
<213>  synthetic construct

<400>  1722
caagaacaag aaactcagca                                                  20

<210>  1723
<211>  18
<212>  DNA
<213>  synthetic construct

<400>  1723

catgtaacgg cagtggag                                                    18

<210>  1724
<211>  21
<212>  DNA
<213>  synthetic construct

<400>  1724
aaattcactt actgcaccag a                                                21

<210>  1725
<211>  20
<212>  DNA
<213>  synthetic construct

<400>  1725
atggcgaaag aatatgtcaa                                                  20

<210>  1726
<211>  24
<212>  DNA
<213>  synthetic construct

<400>  1726
gcatatttcg cttgatatat aggt                                             24

<210>  1727
<211>  21
<212>  DNA
<213>  synthetic construct

<400>  1727
ttattcatgc tggattattc g                                                21

<210>  1728
<211>  20
<212>  DNA
<213>  synthetic construct

<400>  1728
atatttcatc cccagctctt                                                  20

<210>  1729
<211>  26
<212>  DNA
<213>  synthetic construct

<400>  1729
aagtatgaaa gagataaaac aaacat                                           26

<210>  1730
<211>  20
<212>  DNA
<213>  synthetic construct

<400>  1730

agcagttccg gtatctcttt                                                    20

<210>  1731
<211>  21
<212>  DNA
<213>  synthetic construct

<400>  1731
tgaagaaagg gtaatgtttt g                                                 21

<210>  1732
<211>  21
<212>  DNA
<213>  synthetic construct

<400>  1732
tgaagtcctt tgtctttgtt g                                                 21

<210>  1733
<211>  21
<212>  DNA
<213>  synthetic construct

<400>  1733
caatacacac cgatttagag g                                                 21

<210>  1734
<211>  27
<212>  DNA
<213>  synthetic construct

<400>  1734
tctattaaac agacacaact tatctat                                           27

<210>  1735
<211>  20
<212>  DNA
<213>  synthetic construct

<400>  1735
ccgggatcta attcatattt                                                   20

<210>  1736
<211>  20
<212>  DNA
<213>  synthetic construct

<400>  1736
ttcataagga cgatgtgttg                                                   20

<210>  1737
<211>  20
<212>  DNA
<213>  synthetic construct

<400>  1737

tgttctagct cttgctttcc                                                    20


<210>  1738
<211>  24
<212>  DNA
<213>  synthetic construct

<400>  1738
attatgtgaa gatcaaatta taca                                               24


<210>  1739
<211>  21
<212>  DNA
<213>  synthetic construct

<400>  1739
ttttgttgca tctatccttt c                                                  21


<210>  1740
<211>  19
<212>  DNA
<213>  synthetic construct

<400>  1740
atcttgatct tgccattcc                                                     19


<210>  1741
<211>  20
<212>  DNA
<213>  synthetic construct

<400>  1741
tgctttctca atcttcgttt                                                    20


<210>  1742
<211>  18
<212>  DNA
<213>  synthetic construct

<400>  1742
tgaatcttca gcaacaga                                                      18


<210>  1743
<211>  18
<212>  DNA
<213>  synthetic construct

<400>  1743
tttggttgtt caggtgtt                                                      18


<210>  1744
<211>  20
<212>  DNA
<213>  synthetic construct

<400>  1744

aggatcattt gtctcctacg                                                    20

<210>  1745
<211>  18
<212>  DNA
<213>  synthetic construct

<400>  1745
atggtgcgct tagcaaat                                                      18

<210>  1746
<211>  18
<212>  DNA
<213>  synthetic construct

<400>  1746
ctgatcaacg acgcatgg                                                      18

<210>  1747
<211>  18
<212>  DNA
<213>  synthetic construct

<400>  1747
gcgctgagct ggtatcct                                                      18

<210>  1748
<211>  18
<212>  DNA
<213>  synthetic construct

<400>  1748
ccgatcgagt ccattacc                                                      18

<210>  1749
<211>  18
<212>  DNA
<213>  synthetic construct

<400>  1749
gcgtacggcg aatttact                                                      18

<210>  1750
<211>  20
<212>  DNA
<213>  synthetic construct

<400>  1750
gcctgcttcg ttgatagatt                                                    20

<210>  1751
<211>  18
<212>  DNA
<213>  synthetic construct

<400>  1751

atactgccgc ctgcttgt                                                    18

<210>  1752
<211>  18
<212>  DNA
<213>  synthetic construct

<400>  1752
gccaccttta ttccttcc                                                    18

<210>  1753
<211>  20
<212>  DNA
<213>  synthetic construct

<400>  1753
agcatcccca tcatatacag                                                  20

<210>  1754
<211>  18
<212>  DNA
<213>  synthetic construct

<400>  1754
gtcagcatcg aggcattg                                                    18

<210>  1755
<211>  19
<212>  DNA
<213>  synthetic construct

<400>  1755
cgtcaatcat ctgctcaac                                                   19

<210>  1756
<211>  20
<212>  DNA
<213>  synthetic construct

<400>  1756
gttgttctcc actacccact                                                  20

<210>  1757
<211>  20
<212>  DNA
<213>  synthetic construct

<400>  1757
agatcacctc aatccagaaa                                                  20

<210>  1758
<211>  19
<212>  DNA
<213>  synthetic construct

<400>  1758

gacttccggt ttttcacac                                                    19

<210>  1759
<211>  18
<212>  DNA
<213>  synthetic construct

<400>  1759
cggtacatgc gttttgtc                                                     18

<210>  1760
<211>  18
<212>  DNA
<213>  synthetic construct

<400>  1760
ccagctcgga aaacttct                                                     18

<210>  1761
<211>  19
<212>  DNA
<213>  synthetic construct

<400>  1761
ggcgattgtc cataacgtc                                                    19

<210>  1762
<211>  18
<212>  DNA
<213>  synthetic construct

<400>  1762
taacggcaaa gacgctaa                                                     18

<210>  1763
<211>  19
<212>  DNA
<213>  synthetic construct

<400>  1763
tgaccagggc ttctacttc                                                    19

<210>  1764
<211>  21
<212>  DNA
<213>  synthetic construct

<400>  1764
cctataatat ctttgccacc a                                                 21

<210>  1765
<211>  18
<212>  DNA
<213>  synthetic construct

<400>  1765

ccagggatcc agagtttt                                                    18


<210>  1766
<211>  18
<212>  DNA
<213>  synthetic construct

<400>  1766
gcttgaaatg ccaaagtg                                                    18


<210>  1767
<211>  22
<212>  DNA
<213>  synthetic construct

<400>  1767
cagatcagaa acagagatac ga                                               22


<210>  1768
<211>  19
<212>  DNA
<213>  synthetic construct

<400>  1768
cagtcaggaa cacagcatt                                                   19


<210>  1769
<211>  20
<212>  DNA
<213>  synthetic construct

<400>  1769
tctaaggtat aagccgcatc                                                  20


<210>  1770
<211>  18
<212>  DNA
<213>  synthetic construct

<400>  1770
ccgttaccga tgttgatt                                                    18


<210>  1771
<211>  20
<212>  DNA
<213>  synthetic construct

<400>  1771
gagtgaggag ggtatgttga                                                  20


<210>  1772
<211>  19
<212>  DNA
<213>  synthetic construct

<400>  1772

ccgatcatga gaacatcag                                                    19

<210>  1773
<211>  19
<212>  DNA
<213>  synthetic construct

<400>  1773
ggcatctctt cctggttag                                                    19

<210>  1774
<211>  18
<212>  DNA
<213>  synthetic construct

<400>  1774
agagatgggc tgcaaact                                                     18

<210>  1775
<211>  19
<212>  DNA
<213>  synthetic construct

<400>  1775
cgatccctaa atgctgtct                                                    19

<210>  1776
<211>  23
<212>  DNA
<213>  synthetic construct

<400>  1776
acagattaca ttgtcatttc ctg                                               23

<210>  1777
<211>  19
<212>  DNA
<213>  synthetic construct

<400>  1777
cctcctcaaa aacctgttc                                                    19

<210>  1778
<211>  18
<212>  DNA
<213>  synthetic construct

<400>  1778
atggattttg ccttaccc                                                     18

<210>  1779
<211>  20
<212>  DNA
<213>  synthetic construct

<400>  1779

ccaagtaaca tcaccagacc                                                    20

<210>  1780
<211>  19
<212>  DNA
<213>  synthetic construct

<400>  1780
tatttacctt tcccggtca                                                     19

<210>  1781
<211>  18
<212>  DNA
<213>  synthetic construct

<400>  1781
caatcacgaa ctgcgttt                                                      18

<210>  1782
<211>  20
<212>  DNA
<213>  synthetic construct

<400>  1782
tgctgctgat accaatgtag                                                    20

<210>  1783
<211>  21
<212>  DNA
<213>  synthetic construct

<400>  1783
gtaatttcgt aagtcgcgta g                                                  21

<210>  1784
<211>  19
<212>  DNA
<213>  synthetic construct

<400>  1784
cgcaatacca taccttcac                                                     19

<210>  1785
<211>  18
<212>  DNA
<213>  synthetic construct

<400>  1785
ctcgtggatc tgatacgg                                                      18

<210>  1786
<211>  20
<212>  DNA
<213>  synthetic construct

<400>  1786

gtgcgtttaa tctcctcaag                                                    20

<210>  1787
<211>  18
<212>  DNA
<213>  synthetic construct

<400>  1787
gctatccgga caacttcg                                                      18

<210>  1788
<211>  18
<212>  DNA
<213>  synthetic construct

<400>  1788
aaattgccga agctcaat                                                      18

<210>  1789
<211>  18
<212>  DNA
<213>  synthetic construct

<400>  1789
ctgacggtca tcagcagt                                                      18

<210>  1790
<211>  18
<212>  DNA
<213>  synthetic construct

<400>  1790
agagagcgtc attgagca                                                      18

<210>  1791
<211>  18
<212>  DNA
<213>  synthetic construct

<400>  1791
aattttcgcc agttgatg                                                      18

<210>  1792
<211>  18
<212>  DNA
<213>  synthetic construct

<400>  1792
acagggttga tcctcgtc                                                      18

<210>  1793
<211>  19
<212>  DNA
<213>  synthetic construct

<400>  1793

gttaatgaaa cggcgaaac                                                    19

<210>  1794
<211>  19
<212>  DNA
<213>  synthetic construct

<400>  1794
aaaagacaag ctgttgctg                                                    19

<210>  1795
<211>  19
<212>  DNA
<213>  synthetic construct

<400>  1795
cagataatcg ggttgtgaa                                                    19

<210>  1796
<211>  20
<212>  DNA
<213>  synthetic construct

<400>  1796
atttcataaa ctcggattgg                                                   20

<210>  1797
<211>  18
<212>  DNA
<213>  synthetic construct

<400>  1797
ggcaaacagc actacagg                                                     18

<210>  1798
<211>  20
<212>  DNA
<213>  synthetic construct

<400>  1798
aggttcaggt agctggaaaa                                                   20

<210>  1799
<211>  21
<212>  DNA
<213>  synthetic construct

<400>  1799
atgattctgg gttagatctc c                                                 21

<210>  1800
<211>  22
<212>  DNA
<213>  synthetic construct

<400>  1800

tgattcaatt ttagtgatct gc                                              22

<210>  1801
<211>  18
<212>  DNA
<213>  synthetic construct

<400>  1801
aggtgctatc cggctaat                                                    18

<210>  1802
<211>  18
<212>  DNA
<213>  synthetic construct

<400>  1802
acatgatccc ggagaaat                                                    18

<210>  1803
<211>  21
<212>  DNA
<213>  synthetic construct

<400>  1803
ttacaaaatc tctgcaaatc g                                                21

<210>  1804
<211>  20
<212>  DNA
<213>  synthetic construct

<400>  1804
tggaaccagt gatcaatttc                                                  20

<210>  1805
<211>  18
<212>  DNA
<213>  synthetic construct

<400>  1805
gctgctgaac ttccttcc                                                    18

<210>  1806
<211>  18
<212>  DNA
<213>  synthetic construct

<400>  1806
ggtgtcatcc caaacctg                                                    18

<210>  1807
<211>  18
<212>  DNA
<213>  synthetic construct

<400>  1807

ggtaccgaag tggtggtc                                                    18


<210>  1808
<211>  18
<212>  DNA
<213>  synthetic construct

<400>  1808
cctgctgcta ttgctgtc                                                    18


<210>  1809
<211>  18
<212>  DNA
<213>  synthetic construct

<400>  1809
gtatggtgcc agatgctc                                                    18


<210>  1810
<211>  23
<212>  DNA
<213>  synthetic construct

<400>  1810
aatttaacct ggtttgataa gaa                                              23


<210>  1811
<211>  26
<212>  DNA
<213>  synthetic construct

<400>  1811
caaaatatga actatcagaa agattg                                           26


<210>  1812
<211>  20
<212>  DNA
<213>  synthetic construct

<400>  1812
ggggagaata tccttgtctt                                                  20


<210>  1813
<211>  18
<212>  DNA
<213>  synthetic construct

<400>  1813
gcagcggcca taattttt                                                    18


<210>  1814
<211>  18
<212>  DNA
<213>  synthetic construct

<400>  1814

gactcgcggg tgattaac                                                    18


<210>  1815
<211>  19
<212>  DNA
<213>  synthetic construct

<400>  1815
cagtccctta atcgctttc                                                   19


<210>  1816
<211>  18
<212>  DNA
<213>  synthetic construct

<400>  1816
atgaaacgac ctgattgc                                                    18


<210>  1817
<211>  18
<212>  DNA
<213>  synthetic construct

<400>  1817
gtgtggaccg aagaattg                                                    18


<210>  1818
<211>  23
<212>  DNA
<213>  synthetic construct

<400>  1818
ttgcgtatat agaagtcata cca                                              23


<210>  1819
<211>  18
<212>  DNA
<213>  synthetic construct

<400>  1819
gcgtgagctg tctaccag                                                    18


<210>  1820
<211>  19
<212>  DNA
<213>  synthetic construct

<400>  1820
gattcctgct ctgacaacc                                                   19


<210>  1821
<211>  21
<212>  DNA
<213>  synthetic construct

<400>  1821

gccttgaata ctggaagaaa g                                                21

<210>  1822
<211>  19
<212>  DNA
<213>  synthetic construct

<400>  1822
attgacggga tatctgacc                                                   19

<210>  1823
<211>  21
<212>  DNA
<213>  synthetic construct

<400>  1823
acatttgcag catatttgat t                                                21

<210>  1824
<211>  20
<212>  DNA
<213>  synthetic construct

<400>  1824
cagcagcaag gtgtttaatg                                                  20

<210>  1825
<211>  18
<212>  DNA
<213>  synthetic construct

<400>  1825
gcagtggcca gagtatgt                                                    18

<210>  1826
<211>  19
<212>  DNA
<213>  synthetic construct

<400>  1826
ggggagaaag agaccctca                                                   19

<210>  1827
<211>  21
<212>  DNA
<213>  synthetic construct

<400>  1827
tatttgccca ggacttctgt t                                                21

<210>  1828
<211>  20
<212>  DNA
<213>  synthetic construct

<400>  1828

agtaaattca ggctggctct                                                    20

<210>  1829
<211>  22
<212>  DNA
<213>  synthetic construct

<400>  1829
tttacgtggg ttacagatat gg                                                 22

<210>  1830
<211>  20
<212>  DNA
<213>  synthetic construct

<400>  1830
tcatttgaag aacgacacag                                                    20

<210>  1831
<211>  18
<212>  DNA
<213>  synthetic construct

<400>  1831
gtcaggccat attgttgg                                                      18

<210>  1832
<211>  20
<212>  DNA
<213>  synthetic construct

<400>  1832
tgcctctatt atccaacctc                                                    20

<210>  1833
<211>  20
<212>  DNA
<213>  synthetic construct

<400>  1833
gacgatccag aatgctaaac                                                    20

<210>  1834
<211>  20
<212>  DNA
<213>  synthetic construct

<400>  1834
ggtttaaggc aggtagtcag                                                    20

<210>  1835
<211>  20
<212>  DNA
<213>  synthetic construct

<400>  1835

ccacgataca tgaaacacag                                                20

<210>  1836
<211>  20
<212>  DNA
<213>  synthetic construct

<400>  1836
gtggcattgg tcgttatagt                                                20

<210>  1837
<211>  19
<212>  DNA
<213>  synthetic construct

<400>  1837
caaggacgga ctctttctc                                                 19

<210>  1838
<211>  21
<212>  DNA
<213>  synthetic construct

<400>  1838
tgacctatca ctcggatatt g                                              21

<210>  1839
<211>  22
<212>  DNA
<213>  synthetic construct

<400>  1839
agagctggaa gagaatgtat tt                                             22

<210>  1840
<211>  18
<212>  DNA
<213>  synthetic construct

<400>  1840
ttcaggcgaa atgctatg                                                  18

<210>  1841
<211>  20
<212>  DNA
<213>  synthetic construct

<400>  1841
tggttccgta gaggaataag                                                20

<210>  1842
<211>  20
<212>  DNA
<213>  synthetic construct

<400>  1842

gctatgaact gatcctggtg                                                    20

<210>  1843
<211>  20
<212>  DNA
<213>  synthetic construct

<400>  1843
cgatatggcg tactttatcc                                                    20

<210>  1844
<211>  20
<212>  DNA
<213>  synthetic construct

<400>  1844
ctctgcctct attgctcttg                                                    20

<210>  1845
<211>  20
<212>  DNA
<213>  synthetic construct

<400>  1845
gcactatatc cctgctcttg                                                    20

<210>  1846
<211>  19
<212>  DNA
<213>  synthetic construct

<400>  1846
tatcgatgcg gatgaaaat                                                     19

<210>  1847
<211>  24
<212>  DNA
<213>  synthetic construct

<400>  1847
ttaatataac cttcaagcaa cact                                               24

<210>  1848
<211>  20
<212>  DNA
<213>  synthetic construct

<400>  1848
atccaatgac cagaaatgag                                                    20

<210>  1849
<211>  20
<212>  DNA
<213>  synthetic construct

<400>  1849

agatatgcgt aatccacagg                                                    20

<210>  1850
<211>  20
<212>  DNA
<213>  synthetic construct

<400>  1850
cgtaaatatg ggacaaaggt                                                    20

<210>  1851
<211>  20
<212>  DNA
<213>  synthetic construct

<400>  1851
tagatttcgc acagtcaaag                                                    20

<210>  1852
<211>  20
<212>  DNA
<213>  synthetic construct

<400>  1852
gcaagggtag aggtgtattg                                                    20

<210>  1853
<211>  20
<212>  DNA
<213>  synthetic construct

<400>  1853
tggctgtgaa ctttgtgtag                                                    20

<210>  1854
<211>  20
<212>  DNA
<213>  synthetic construct

<400>  1854
ataagccatg tgtttcttcc                                                    20

<210>  1855
<211>  20
<212>  DNA
<213>  synthetic construct

<400>  1855
cacgttctag cgtctactca                                                    20

<210>  1856
<211>  18
<212>  DNA
<213>  synthetic construct

<400>  1856

gccggtaatc ttgagctg                                                    18

<210>  1857
<211>  18
<212>  DNA
<213>  synthetic construct

<400>  1857
ccgatctgca cgtcgata                                                    18

<210>  1858
<211>  18
<212>  DNA
<213>  synthetic construct

<400>  1858
cctgctctat tccgtcag                                                    18

<210>  1859
<211>  19
<212>  DNA
<213>  synthetic construct

<400>  1859
gatttcctgt acccggatg                                                   19

<210>  1860
<211>  19
<212>  DNA
<213>  synthetic construct

<400>  1860
ggctgtctgc tatggattt                                                   19

<210>  1861
<211>  18
<212>  DNA
<213>  synthetic construct

<400>  1861
ggtcgatcat cagattgg                                                    18

<210>  1862
<211>  18
<212>  DNA
<213>  synthetic construct

<400>  1862
tggcgtttat tttctgga                                                    18

<210>  1863
<211>  18
<212>  DNA
<213>  synthetic construct

<400>  1863

gtagcgttgc atgggata                                                    18

<210>  1864
<211>  18
<212>  DNA
<213>  synthetic construct

<400>  1864
cgttctcccc tgattctt                                                    18

<210>  1865
<211>  18
<212>  DNA
<213>  synthetic construct

<400>  1865
tcatagcggt ggctcagt                                                    18

<210>  1866
<211>  18
<212>  DNA
<213>  synthetic construct

<400>  1866
cggcgcattt aaaatatc                                                    18

<210>  1867
<211>  20
<212>  DNA
<213>  synthetic construct

<400>  1867
cattaaccgg caaatagaac                                                  20

<210>  1868
<211>  18
<212>  DNA
<213>  synthetic construct

<400>  1868
tattgaaggc accaccag                                                    18

<210>  1869
<211>  20
<212>  DNA
<213>  synthetic construct

<400>  1869
gatgaatatc ggacaggaac                                                  20

<210>  1870
<211>  18
<212>  DNA
<213>  synthetic construct

<400>  1870

cctggagtgt gcataagg                                                    18


<210>  1871
<211>  19
<212>  DNA
<213>  synthetic construct

<400>  1871
cagcgaaaga tggataatg                                                   19


<210>  1872
<211>  20
<212>  DNA
<213>  synthetic construct

<400>  1872
acacgcatat aaaccgcaac                                                  20


<210>  1873
<211>  18
<212>  DNA
<213>  synthetic construct

<400>  1873
gaatccagcg tggagagc                                                    18


<210>  1874
<211>  20
<212>  DNA
<213>  synthetic construct

<400>  1874
gcgataagtt ttcgatttca                                                  20


<210>  1875
<211>  18
<212>  DNA
<213>  synthetic construct

<400>  1875
gctgaccgat ccgttttt                                                    18


<210>  1876
<211>  20
<212>  DNA
<213>  synthetic construct

<400>  1876
atgtcatggt tccgatactg                                                  20


<210>  1877
<211>  20
<212>  DNA
<213>  synthetic construct

<400>  1877

ctttatcaag cggatactgg                                                    20


<210>  1878
<211>  20
<212>  DNA
<213>  synthetic construct

<400>  1878
aagctggaac gtactaacga                                                    20


<210>  1879
<211>  20
<212>  DNA
<213>  synthetic construct

<400>  1879
taatcactgc ggctaataca                                                    20


<210>  1880
<211>  19
<212>  DNA
<213>  synthetic construct

<400>  1880
acaggatagc gaacacctc                                                     19


<210>  1881
<211>  18
<212>  DNA
<213>  synthetic construct

<400>  1881
aaagccagct ccgtttag                                                      18


<210>  1882
<211>  20
<212>  DNA
<213>  synthetic construct

<400>  1882
atccataccc tgacactcaa                                                    20


<210>  1883
<211>  18
<212>  DNA
<213>  synthetic construct

<400>  1883
gatcaacaaa ggccgtaa                                                      18


<210>  1884
<211>  19
<212>  DNA
<213>  synthetic construct

<400>  1884

gcttcaggtg ttgaaaatg                                                    19

<210>  1885
<211>  20
<212>  DNA
<213>  synthetic construct

<400>  1885
agcagcagca taaaataacc                                                   20

<210>  1886
<211>  20
<212>  DNA
<213>  synthetic construct

<400>  1886
caagcacaac aagaaatacg                                                   20

<210>  1887
<211>  19
<212>  DNA
<213>  synthetic construct

<400>  1887
tagacctccg aagagttgc                                                    19

<210>  1888
<211>  18
<212>  DNA
<213>  synthetic construct

<400>  1888
gagcgacctt ggattctc                                                     18

<210>  1889
<211>  20
<212>  DNA
<213>  synthetic construct

<400>  1889
ataagaccca aattaacggc                                                   20

<210>  1890
<211>  18
<212>  DNA
<213>  synthetic construct

<400>  1890
atggatgctc gggtactg                                                     18

<210>  1891
<211>  18
<212>  DNA
<213>  synthetic construct

<400>  1891

ctcagctaca gccacgac                                                    18


<210>  1892
<211>  18
<212>  DNA
<213>  synthetic construct

<400>  1892
gatcgtctct gcccagtc                                                    18


<210>  1893
<211>  18
<212>  DNA
<213>  synthetic construct

<400>  1893
acattgatgg tgtcgtcc                                                    18


<210>  1894
<211>  19
<212>  DNA
<213>  synthetic construct

<400>  1894
aggagagaac atgagtcgc                                                   19


<210>  1895
<211>  20
<212>  DNA
<213>  synthetic construct

<400>  1895
tccttgtccc agtagttacc                                                  20


<210>  1896
<211>  18
<212>  DNA
<213>  synthetic construct

<400>  1896
aggagcaact gaagcgac                                                    18


<210>  1897
<211>  18
<212>  DNA
<213>  synthetic construct

<400>  1897
tctgccttta cccaggac                                                    18


<210>  1898
<211>  18
<212>  DNA
<213>  synthetic construct

<400>  1898

aaggttggca ggatcaac 18

<210> 1899
<211> 20
<212> DNA
<213> synthetic construct

<400> 1899
ctagtggcga aattgaacag 20

<210> 1900
<211> 18
<212> DNA
<213> synthetic construct

<400> 1900
aggcatccat cgagctac 18

<210> 1901
<211> 18
<212> DNA
<213> synthetic construct

<400> 1901
aacgtccgag caggatac 18

<210> 1902
<211> 18
<212> DNA
<213> synthetic construct

<400> 1902
gcgaggaggt attcgaca 18

<210> 1903
<211> 19
<212> DNA
<213> synthetic construct

<400> 1903
cccttctgcg agtagtgtt 19

<210> 1904
<211> 18
<212> DNA
<213> synthetic construct

<400> 1904
aaggacttct ggtcggtg 18

<210> 1905
<211> 19
<212> DNA
<213> synthetic construct

<400> 1905

caggaacagg tgctcgtag                                                    19

<210>  1906
<211>  19
<212>  DNA
<213>  synthetic construct

<400>  1906
ttccctaacg aatgctgtc                                                    19

<210>  1907
<211>  18
<212>  DNA
<213>  synthetic construct

<400>  1907
cgttgctccc tcatacac                                                     18

<210>  1908
<211>  18
<212>  DNA
<213>  synthetic construct

<400>  1908
cgagcaccaa tatcgaac                                                     18

<210>  1909
<211>  18
<212>  DNA
<213>  synthetic construct

<400>  1909
gagccgtagg tgttatcg                                                     18

<210>  1910
<211>  20
<212>  DNA
<213>  synthetic construct

<400>  1910
gctttacctt gatcgaactg                                                   20

<210>  1911
<211>  20
<212>  DNA
<213>  synthetic construct

<400>  1911
tcaatagagc cagtcacacc                                                   20

<210>  1912
<211>  18
<212>  DNA
<213>  synthetic construct

<400>  1912

tgccgtgagt gaaatcag                                                    18

<210>  1913
<211>  19
<212>  DNA
<213>  synthetic construct

<400>  1913
cgtagttggc tttccagtt                                                   19

<210>  1914
<211>  21
<212>  DNA
<213>  synthetic construct

<400>  1914
ggtatcaacc cactaaaggt c                                                21

<210>  1915
<211>  20
<212>  DNA
<213>  synthetic construct

<400>  1915
gtccagagct tctaccagag                                                  20

<210>  1916
<211>  18
<212>  DNA
<213>  synthetic construct

<400>  1916
gaagtgaact ccgccaag                                                    18

<210>  1917
<211>  20
<212>  DNA
<213>  synthetic construct

<400>  1917
tcgagcatca tcaggtagac                                                  20

<210>  1918
<211>  20
<212>  DNA
<213>  synthetic construct

<400>  1918
tcgagaagtc gatgttcaag                                                  20

<210>  1919
<211>  18
<212>  DNA
<213>  synthetic construct

<400>  1919

cttgccgtag tgatgcag                                                    18

<210>  1920
<211>  20
<212>  DNA
<213>  synthetic construct

<400>  1920
agacctacaa caaggtttcg                                                  20

<210>  1921
<211>  18
<212>  DNA
<213>  synthetic construct

<400>  1921
tgaggatagt cccttcgc                                                    18

<210>  1922
<211>  19
<212>  DNA
<213>  synthetic construct

<400>  1922
attcctctct gaatcgctg                                                   19

<210>  1923
<211>  20
<212>  DNA
<213>  synthetic construct

<400>  1923
aatatcttca tcgccagttg                                                  20

<210>  1924
<211>  18
<212>  DNA
<213>  synthetic construct

<400>  1924
aggaatgacg gaggcttt                                                    18

<210>  1925
<211>  18
<212>  DNA
<213>  synthetic construct

<400>  1925
gggttggaca tcagcatc                                                    18

<210>  1926
<211>  18
<212>  DNA
<213>  synthetic construct

<400>  1926

ttcaacctca acggactg                                                    18

<210>  1927
<211>  18
<212>  DNA
<213>  synthetic construct

<400>  1927
tgcaaggtac tcaccagc                                                    18

<210>  1928
<211>  18
<212>  DNA
<213>  synthetic construct

<400>  1928
cattgaaagg tcgtagcg                                                    18

<210>  1929
<211>  18
<212>  DNA
<213>  synthetic construct

<400>  1929
cgacgaagtg gatattgg                                                    18

<210>  1930
<211>  19
<212>  DNA
<213>  synthetic construct

<400>  1930
ggtcaagcac atcctagtg                                                   19

<210>  1931
<211>  18
<212>  DNA
<213>  synthetic construct

<400>  1931
acttccttgc ggtactcc                                                    18

<210>  1932
<211>  19
<212>  DNA
<213>  synthetic construct

<400>  1932
ccgaaggact tggtttact                                                   19

<210>  1933
<211>  18
<212>  DNA
<213>  synthetic construct

<400>  1933

ctatggaagg tgcctgtg                                                    18

<210>  1934
<211>  19
<212>  DNA
<213>  synthetic construct

<400>  1934
cactttccgt tattgcctc                                                   19

<210>  1935
<211>  18
<212>  DNA
<213>  synthetic construct

<400>  1935
gaggatgagg atgttggc                                                    18

<210>  1936
<211>  18
<212>  DNA
<213>  synthetic construct

<400>  1936
gactggctga atcgtctc                                                    18

<210>  1937
<211>  18
<212>  DNA
<213>  synthetic construct

<400>  1937
gcaggtcgta ccaggaag                                                    18

<210>  1938
<211>  19
<212>  DNA
<213>  synthetic construct

<400>  1938
attgtcgatg acgaacctc                                                   19

<210>  1939
<211>  20
<212>  DNA
<213>  synthetic construct

<400>  1939
ttcaggtaga gctggaaatg                                                  20

<210>  1940
<211>  18
<212>  DNA
<213>  synthetic construct

<400>  1940

cgtttgggac agattgag                                                    18


<210>  1941
<211>  19
<212>  DNA
<213>  synthetic construct

<400>  1941
gatactctgc tgacctcgc                                                   19


<210>  1942
<211>  18
<212>  DNA
<213>  synthetic construct

<400>  1942
aatgcgataa ccatcagc                                                    18


<210>  1943
<211>  18
<212>  DNA
<213>  synthetic construct

<400>  1943
ccgtcgtact ggaagttg                                                    18


<210>  1944
<211>  19
<212>  DNA
<213>  synthetic construct

<400>  1944
ctgggacgtt agtgtcatc                                                   19


<210>  1945
<211>  18
<212>  DNA
<213>  synthetic construct

<400>  1945
gtcttggcat tgagttcg                                                    18


<210>  1946
<211>  18
<212>  DNA
<213>  synthetic construct

<400>  1946
aagaagtctc tgctcccc                                                    18


<210>  1947
<211>  18
<212>  DNA
<213>  synthetic construct

<400>  1947

acgatttcct ccacctgt                                                    18

<210>  1948
<211>  18
<212>  DNA
<213>  synthetic construct

<400>  1948
atggcagttt cagtgtcg                                                    18

<210>  1949
<211>  18
<212>  DNA
<213>  synthetic construct

<400>  1949
cgaaatagtc gtccagcc                                                    18

<210>  1950
<211>  18
<212>  DNA
<213>  synthetic construct

<400>  1950
aatgtcggca tcattctc                                                    18

<210>  1951
<211>  18
<212>  DNA
<213>  synthetic construct

<400>  1951
gtagacctcg cgcttgaa                                                    18

<210>  1952
<211>  18
<212>  DNA
<213>  synthetic construct

<400>  1952
gacctgctgt tccagttg                                                    18

<210>  1953
<211>  18
<212>  DNA
<213>  synthetic construct

<400>  1953
aattcacggg ttttctcg                                                    18

<210>  1954
<211>  20
<212>  DNA
<213>  synthetic construct

<400>  1954

cctgctcaac accttctatc                                                    20

<210>  1955
<211>  18
<212>  DNA
<213>  synthetic construct

<400>  1955
gtcgaacaac gcgaacag                                                      18

<210>  1956
<211>  19
<212>  DNA
<213>  synthetic construct

<400>  1956
gttgaaaggg tttaccgac                                                     19

<210>  1957
<211>  18
<212>  DNA
<213>  synthetic construct

<400>  1957
aatttctgca tcgggttc                                                      18

<210>  1958
<211>  20
<212>  DNA
<213>  synthetic construct

<400>  1958
atgctcgata atgctattcc                                                    20

<210>  1959
<211>  19
<212>  DNA
<213>  synthetic construct

<400>  1959
ttctcgtagt aaccctcgg                                                     19

<210>  1960
<211>  19
<212>  DNA
<213>  synthetic construct

<400>  1960
gacttcgctg ttcgacttc                                                     19

<210>  1961
<211>  18
<212>  DNA
<213>  synthetic construct

<400>  1961

tcggttcgag ttcatagc                                                    18

<210>  1962
<211>  18
<212>  DNA
<213>  synthetic construct

<400>  1962
gtgttccagg tgttcgac                                                    18

<210>  1963
<211>  20
<212>  DNA
<213>  synthetic construct

<400>  1963
gatagacgtt gtccttgacc                                                  20

<210>  1964
<211>  19
<212>  DNA
<213>  synthetic construct

<400>  1964
acaacctgga acagcaact                                                   19

<210>  1965
<211>  18
<212>  DNA
<213>  synthetic construct

<400>  1965
cgactcttgc gcgtattc                                                    18

<210>  1966
<211>  18
<212>  DNA
<213>  synthetic construct

<400>  1966
gtcaagggtg ttgtctgc                                                    18

<210>  1967
<211>  18
<212>  DNA
<213>  synthetic construct

<400>  1967
ctctgcacaa actcaggg                                                    18

<210>  1968
<211>  20
<212>  DNA
<213>  synthetic construct

<400>  1968

aagtcggtac tggagtcctt 20

<210> 1969
<211> 18
<212> DNA
<213> synthetic construct

<400> 1969
tcagcacatc atggaaca 18

<210> 1970
<211> 18
<212> DNA
<213> synthetic construct

<400> 1970
cttcagttcc gagatgcc 18

<210> 1971
<211> 18
<212> DNA
<213> synthetic construct

<400> 1971
gtaacgaacg ctatcggg 18

<210> 1972
<211> 25
<212> DNA
<213> synthetic construct

<400> 1972
atatacggaa aaagagtttc ttgag 25

<210> 1973
<211> 21
<212> DNA
<213> synthetic construct

<400> 1973
agcacgccat tctttaactt c 21

<210> 1974
<211> 20
<212> DNA
<213> synthetic construct

<400> 1974
tatacggctt cagactttcc 20

<210> 1975
<211> 18
<212> DNA
<213> synthetic construct

<400> 1975

tggcataagt attggcag                                                    18

<210>  1976
<211>  20
<212>  DNA
<213>  synthetic construct

<400>  1976
atccagtata ttcctgctcg                                                  20

<210>  1977
<211>  20
<212>  DNA
<213>  synthetic construct

<400>  1977
tgcaatttct tcttattggc                                                  20

<210>  1978
<211>  20
<212>  DNA
<213>  synthetic construct

<400>  1978
tcgccaataa gaagaaattg                                                  20

<210>  1979
<211>  20
<212>  DNA
<213>  synthetic construct

<400>  1979
agtggtactc gaagggttct                                                  20

<210>  1980
<211>  19
<212>  DNA
<213>  synthetic construct

<400>  1980
atcgttctgg tcttccttg                                                   19

<210>  1981
<211>  20
<212>  DNA
<213>  synthetic construct

<400>  1981
accaaagagt gttgatagcc                                                  20

<210>  1982
<211>  20
<212>  DNA
<213>  synthetic construct

<400>  1982

agtctgttgg tatcggtttg                                                    20

<210>  1983
<211>  18
<212>  DNA
<213>  synthetic construct

<400>  1983
ctccaggtcg aggaaatg                                                      18

<210>  1984
<211>  18
<212>  DNA
<213>  synthetic construct

<400>  1984
aacgctttct cgatcagg                                                      18

<210>  1985
<211>  18
<212>  DNA
<213>  synthetic construct

<400>  1985
gatactgtgc ggttgtga                                                      18

<210>  1986
<211>  20
<212>  DNA
<213>  synthetic construct

<400>  1986
ttcgattact acgcctatgg                                                    20

<210>  1987
<211>  18
<212>  DNA
<213>  synthetic construct

<400>  1987
ggtccattgc aggatctc                                                      18

<210>  1988
<211>  20
<212>  DNA
<213>  synthetic construct

<400>  1988
cccctattcc ataccttacc                                                    20

<210>  1989
<211>  20
<212>  DNA
<213>  synthetic construct

<400>  1989

tattgaggac gctgattttc                                                    20

<210>  1990
<211>  18
<212>  DNA
<213>  synthetic construct

<400>  1990
gtgcgctaca gctacacg                                                      18

<210>  1991
<211>  18
<212>  DNA
<213>  synthetic construct

<400>  1991
cttgccttcc caggtatc                                                      18

<210>  1992
<211>  19
<212>  DNA
<213>  synthetic construct

<400>  1992
actcttggct tgattttgg                                                     19

<210>  1993
<211>  20
<212>  DNA
<213>  synthetic construct

<400>  1993
ttgaagtccc atctcttttc                                                    20

<210>  1994
<211>  23
<212>  DNA
<213>  synthetic construct

<400>  1994
aaatattgtt ggtttacttc gtg                                                23

<210>  1995
<211>  21
<212>  DNA
<213>  synthetic construct

<400>  1995
tcataatttc atctcccttg a                                                  21

<210>  1996
<211>  21
<212>  DNA
<213>  synthetic construct

<400>  1996

ctaaagcatt tggagagatt g                                                21

<210>  1997
<211>  20
<212>  DNA
<213>  synthetic construct

<400>  1997
tggattctat tcgctactcc                                                   20

<210>  1998
<211>  21
<212>  DNA
<213>  synthetic construct

<400>  1998
tgtaggaact gatttggatg a                                                21

<210>  1999
<211>  20
<212>  DNA
<213>  synthetic construct

<400>  1999
ccataactcc cttaacagca                                                   20

<210>  2000
<211>  20
<212>  DNA
<213>  synthetic construct

<400>  2000
tttccagaca taaaccatcc                                                   20

<210>  2001
<211>  20
<212>  DNA
<213>  synthetic construct

<400>  2001
ggcatttcta atcatccaag                                                   20

<210>  2002
<211>  20
<212>  DNA
<213>  synthetic construct

<400>  2002
tcgtcatctg tactggtctg                                                   20

<210>  2003
<211>  22
<212>  DNA
<213>  synthetic construct

<400>  2003

ctcgtctact gacttgaact tg                                              22

<210>  2004
<211>  20
<212>  DNA
<213>  synthetic construct

<400>  2004
gtggaaatct gctggtaaag                                                  20

<210>  2005
<211>  20
<212>  DNA
<213>  synthetic construct

<400>  2005
gctcattcat ctcctcaaag                                                  20

<210>  2006
<211>  20
<212>  DNA
<213>  synthetic construct

<400>  2006
gattatcgga gaaattcgtg                                                  20

<210>  2007
<211>  21
<212>  DNA
<213>  synthetic construct

<400>  2007
gatagtctcc accagatgaa a                                                21

<210>  2008
<211>  18
<212>  DNA
<213>  synthetic construct

<400>  2008
tggacaagca gtgtgtga                                                    18

<210>  2009
<211>  20
<212>  DNA
<213>  synthetic construct

<400>  2009
taggtctgca caaagattcc                                                  20

<210>  2010
<211>  22
<212>  DNA
<213>  synthetic construct

<400>  2010

aaaaatgatg acattcttga aa                                                22

<210>  2011
<211>  23
<212>  DNA
<213>  synthetic construct

<400>  2011
ttaatcattg actttacgat ttg                                               23

<210>  2012
<211>  19
<212>  DNA
<213>  synthetic construct

<400>  2012
atgctggaaa gtctcttgg                                                    19

<210>  2013
<211>  20
<212>  DNA
<213>  synthetic construct

<400>  2013
tttgtttcct taatgcgttt                                                   20

<210>  2014
<211>  20
<212>  DNA
<213>  synthetic construct

<400>  2014
ggctgtagga gacaatgaag                                                   20

<210>  2015
<211>  22
<212>  DNA
<213>  synthetic construct

<400>  2015
ctttgttgac agacgtagag tg                                                22

<210>  2016
<211>  24
<212>  DNA
<213>  synthetic construct

<400>  2016
ttgttagaga gacagaactt gaac                                              24

<210>  2017
<211>  20
<212>  DNA
<213>  synthetic construct

<400>  2017

agatcgacaa acacctcatc                                                    20

<210>  2018
<211>  27
<212>  DNA
<213>  synthetic construct

<400>  2018
aatattttat ccatgttatt atcctac                                            27

<210>  2019
<211>  20
<212>  DNA
<213>  synthetic construct

<400>  2019
gtcggagcat caattctatc                                                    20

<210>  2020
<211>  20
<212>  DNA
<213>  synthetic construct

<400>  2020
tgagggattt attcaggatg                                                    20

<210>  2021
<211>  23
<212>  DNA
<213>  synthetic construct

<400>  2021
ttaaaaaggt tactgatttc cac                                                23

<210>  2022
<211>  22
<212>  DNA
<213>  synthetic construct

<400>  2022
tgctttaact cttttaccaa cc                                                 22

<210>  2023
<211>  21
<212>  DNA
<213>  synthetic construct

<400>  2023
tggacagaaa attccaataa g                                                  21

<210>  2024
<211>  20
<212>  DNA
<213>  synthetic construct

<400>  2024

aaggcaattc caatacaaag                                                    20

<210>  2025
<211>  20
<212>  DNA
<213>  synthetic construct

<400>  2025
gatggtgcaa gaccttaatc                                                    20

<210>  2026
<211>  18
<212>  DNA
<213>  synthetic construct

<400>  2026
tttcaatgcc tctcttgg                                                      18

<210>  2027
<211>  19
<212>  DNA
<213>  synthetic construct

<400>  2027
tcccagctat caagcctac                                                     19

<210>  2028
<211>  22
<212>  DNA
<213>  synthetic construct

<400>  2028
tccattagtc aatgagttga aa                                                 22

<210>  2029
<211>  25
<212>  DNA
<213>  synthetic construct

<400>  2029
gtcacataaa cattctctct acctt                                              25

<210>  2030
<211>  20
<212>  DNA
<213>  synthetic construct

<400>  2030
gaagcatacg acaaacttcc                                                    20

<210>  2031
<211>  20
<212>  DNA
<213>  synthetic construct

<400>  2031

tcaggtagcg atcatactcc                                                    20

<210>  2032
<211>  21
<212>  DNA
<213>  synthetic construct

<400>  2032
gtgatagtga acttgggatt g                                                  21

<210>  2033
<211>  20
<212>  DNA
<213>  synthetic construct

<400>  2033
aacgccagta aagagatgac                                                    20

<210>  2034
<211>  18
<212>  DNA
<213>  synthetic construct

<400>  2034
agagcaaaaa cgctggtt                                                      18

<210>  2035
<211>  22
<212>  DNA
<213>  synthetic construct

<400>  2035
tagtaattcc acacagaaag ca                                                 22

<210>  2036
<211>  20
<212>  DNA
<213>  synthetic construct

<400>  2036
acgaacagtg gacctgatac                                                    20

<210>  2037
<211>  20
<212>  DNA
<213>  synthetic construct

<400>  2037
taaaccttgc gaaataatgg                                                    20

<210>  2038
<211>  20
<212>  DNA
<213>  synthetic construct

<400>  2038

ttgtcttgac tggttgtgtc                                                    20

<210>  2039
<211>  20
<212>  DNA
<213>  synthetic construct

<400>  2039
ctactccgtg aagtgaaagg                                                    20

<210>  2040
<211>  21
<212>  DNA
<213>  synthetic construct

<400>  2040
tgcaaaaggt tagaatgatt g                                                  21

<210>  2041
<211>  21
<212>  DNA
<213>  synthetic construct

<400>  2041
aagtcagagt agggaggaaa g                                                  21

<210>  2042
<211>  20
<212>  DNA
<213>  synthetic construct

<400>  2042
gttcggtaat ccagttgaag                                                    20

<210>  2043
<211>  20
<212>  DNA
<213>  synthetic construct

<400>  2043
ttaacagacg cagtcaagag                                                    20

<210>  2044
<211>  22
<212>  DNA
<213>  synthetic construct

<400>  2044
cgtggaaatt ttagaagaac tc                                                 22

<210>  2045
<211>  20
<212>  DNA
<213>  synthetic construct

<400>  2045

caaggactgg tttttctttg                                                    20

<210>  2046
<211>  19
<212>  DNA
<213>  synthetic construct

<400>  2046
aaaatttggg ggattcagt                                                     19

<210>  2047
<211>  20
<212>  DNA
<213>  synthetic construct

<400>  2047
tcataagcag cagtaaccaa                                                    20

<210>  2048
<211>  20
<212>  DNA
<213>  synthetic construct

<400>  2048
cctctaaggc tatgatggaa                                                    20

<210>  2049
<211>  20
<212>  DNA
<213>  synthetic construct

<400>  2049
ctattaaggg attgcgtttg                                                    20

<210>  2050
<211>  20
<212>  DNA
<213>  synthetic construct

<400>  2050
tgaaagatgg acgagatagg                                                    20

<210>  2051
<211>  20
<212>  DNA
<213>  synthetic construct

<400>  2051
gatgggctag gtaagatttg                                                    20

<210>  2052
<211>  19
<212>  DNA
<213>  synthetic construct

<400>  2052

tacatggatg ctgttggag                                                    19

<210>  2053
<211>  24
<212>  DNA
<213>  synthetic construct

<400>  2053
gatctaggat ggcaagaata taag                                              24

<210>  2054
<211>  24
<212>  DNA
<213>  synthetic construct

<400>  2054
catatggtac atttgattta ttgc                                              24

<210>  2055
<211>  26
<212>  DNA
<213>  synthetic construct

<400>  2055
gtgtcaatat ggtagtcttt aacatc                                            26

<210>  2056
<211>  22
<212>  DNA
<213>  synthetic construct

<400>  2056
ctcctagtgc cctatatctt tg                                                22

<210>  2057
<211>  18
<212>  DNA
<213>  synthetic construct

<400>  2057
tacgatgatt ggccttga                                                     18

<210>  2058
<211>  18
<212>  DNA
<213>  synthetic construct

<400>  2058
cttggtgcac agagtcct                                                     18

<210>  2059
<211>  20
<212>  DNA
<213>  synthetic construct

<400>  2059

ttttcgtcat tcatttttga                                                    20

<210>  2060
<211>  20
<212>  DNA
<213>  synthetic construct

<400>  2060
aagtaggcga tggttatgtc                                                    20

<210>  2061
<211>  20
<212>  DNA
<213>  synthetic construct

<400>  2061
tatttcctga atcctgatgg                                                    20

<210>  2062
<211>  21
<212>  DNA
<213>  synthetic construct

<400>  2062
gcatctctcg ttatgtcttt g                                                  21

<210>  2063
<211>  20
<212>  DNA
<213>  synthetic construct

<400>  2063
tttggagctt tgtatgtgtg                                                    20

<210>  2064
<211>  24
<212>  DNA
<213>  synthetic construct

<400>  2064
gaccattacc actttattcc ttac                                               24

<210>  2065
<211>  20
<212>  DNA
<213>  synthetic construct

<400>  2065
tttgctcttc tgtcaagtcc                                                    20

<210>  2066
<211>  21
<212>  DNA
<213>  synthetic construct

<400>  2066

tttcatttct tatttcctcc a                                                21

<210>  2067
<211>  20
<212>  DNA
<213>  synthetic construct

<400>  2067
gcttattcac aggctcattt                                                   20

<210>  2068
<211>  21
<212>  DNA
<213>  synthetic construct

<400>  2068
gatttctgta tgaggcagtt c                                                 21

<210>  2069
<211>  19
<212>  DNA
<213>  synthetic construct

<400>  2069
cttcctagcg tccaatacc                                                    19

<210>  2070
<211>  20
<212>  DNA
<213>  synthetic construct

<400>  2070
ggctgttagt ccaagtcaag                                                   20

<210>  2071
<211>  20
<212>  DNA
<213>  synthetic construct

<400>  2071
gctacctgta accattgctc                                                   20

<210>  2072
<211>  19
<212>  DNA
<213>  synthetic construct

<400>  2072
cttgtgctgt tcttcgttg                                                    19

<210>  2073
<211>  18
<212>  DNA
<213>  synthetic construct

<400>  2073

cttgcgctac cgtggttt                                                    18

<210>  2074
<211>  23
<212>  DNA
<213>  synthetic construct

<400>  2074
ttgatatcca acaactacaa aaa                                               23

<210>  2075
<211>  23
<212>  DNA
<213>  synthetic construct

<400>  2075
agctttttgg tattcatcta cag                                               23

<210>  2076
<211>  24
<212>  DNA
<213>  synthetic construct

<400>  2076
aggaaacaga aaataaagag aaac                                              24

<210>  2077
<211>  20
<212>  DNA
<213>  synthetic construct

<400>  2077
caaacggagc attgatagac                                                   20

<210>  2078
<211>  21
<212>  DNA
<213>  synthetic construct

<400>  2078
cattgaaacc aggagaaaag g                                                 21

<210>  2079
<211>  26
<212>  DNA
<213>  synthetic construct

<400>  2079
tttttactct caactttctc ttttgc                                            26

<210>  2080
<211>  22
<212>  DNA
<213>  synthetic construct

<400>  2080

aaaagcgaaa gttaagagtg aa                                                22

<210>  2081
<211>  18
<212>  DNA
<213>  synthetic construct

<400>  2081
ggatgggctt ggaagagt                                                    18

<210>  2082
<211>  20
<212>  DNA
<213>  synthetic construct

<400>  2082
aagatattgc caacctcaac                                                  20

<210>  2083
<211>  21
<212>  DNA
<213>  synthetic construct

<400>  2083
ggttatgacc gtagcaacta a                                                21

<210>  2084
<211>  21
<212>  DNA
<213>  synthetic construct

<400>  2084
gcttgtatga cgatagagga g                                                21

<210>  2085
<211>  19
<212>  DNA
<213>  synthetic construct

<400>  2085
tagctcagtc agaccacga                                                   19

<210>  2086
<211>  19
<212>  DNA
<213>  synthetic construct

<400>  2086
ttgtagggct aggaacagg                                                   19

<210>  2087
<211>  20
<212>  DNA
<213>  synthetic construct

<400>  2087

aatctgaact ccatctcgtc                                                    20

<210>  2088
<211>  18
<212>  DNA
<213>  synthetic construct

<400>  2088
atgacgcgct atgctttg                                                      18

<210>  2089
<211>  27
<212>  DNA
<213>  synthetic construct

<400>  2089
ataggcagtc ttagaatagg attcttc                                            27

<210>  2090
<211>  21
<212>  DNA
<213>  synthetic construct

<400>  2090
aaccatgtat tgaggaaagt g                                                  21

<210>  2091
<211>  22
<212>  DNA
<213>  synthetic construct

<400>  2091
tggaagtttg tatgagtgac ag                                                 22

<210>  2092
<211>  21
<212>  DNA
<213>  synthetic construct

<400>  2092
ggacttcctt ttcttatcca g                                                  21

<210>  2093
<211>  20
<212>  DNA
<213>  synthetic construct

<400>  2093
cagtccatag aggctgtttt                                                    20

<210>  2094
<211>  20
<212>  DNA
<213>  synthetic construct

<400>  2094

tttgcagatt tgtgagattg                                                20

<210>  2095
<211>  20
<212>  DNA
<213>  synthetic construct

<400>  2095
tacgtttaca gatgctacgg                                                20

<210>  2096
<211>  20
<212>  DNA
<213>  synthetic construct

<400>  2096
aatagcagta gcagggacag                                                20

<210>  2097
<211>  20
<212>  DNA
<213>  synthetic construct

<400>  2097
tgctccgtat ctctcaaatc                                                20

<210>  2098
<211>  25
<212>  DNA
<213>  synthetic construct

<400>  2098
atgaatttaa catttttagg cttat                                          25

<210>  2099
<211>  27
<212>  DNA
<213>  synthetic construct

<400>  2099
tatttgataa taaatgagaa gacaaga                                        27

<210>  2100
<211>  25
<212>  DNA
<213>  synthetic construct

<400>  2100
atgagtgaaa taggctttaa ataca                                          25

<210>  2101
<211>  24
<212>  DNA
<213>  synthetic construct

<400>  2101

caccttgcca agatatttac tatt                                              24

<210>  2102
<211>  21
<212>  DNA
<213>  synthetic construct

<400>  2102
ttgcacttta tatcctccat t                                                 21

<210>  2103
<211>  20
<212>  DNA
<213>  synthetic construct

<400>  2103
gttacatcaa ttcccatcgt                                                   20

<210>  2104
<211>  18
<212>  DNA
<213>  synthetic construct

<400>  2104
aagtaggggc tttcttgc                                                     18

<210>  2105
<211>  23
<212>  DNA
<213>  synthetic construct

<400>  2105
aactttcttt cttaccacta ccc                                               23

<210>  2106
<211>  25
<212>  DNA
<213>  synthetic construct

<400>  2106
taacgagatt attacaaaac aaaac                                             25

<210>  2107
<211>  20
<212>  DNA
<213>  synthetic construct

<400>  2107
tctttaggct ggaatgtgtc                                                   20

<210>  2108
<211>  19
<212>  DNA
<213>  synthetic construct

<400>  2108

gaaagaattg ggtgcaaag                                                    19

<210>  2109
<211>  20
<212>  DNA
<213>  synthetic construct

<400>  2109
tcatcctcac cattaccaac                                                   20

<210>  2110
<211>  21
<212>  DNA
<213>  synthetic construct

<400>  2110
gcttccatca aatcacttta c                                                 21

<210>  2111
<211>  19
<212>  DNA
<213>  synthetic construct

<400>  2111
gtgcaacatt actgccttc                                                    19

<210>  2112
<211>  20
<212>  DNA
<213>  synthetic construct

<400>  2112
tggaagacct ttatcctgtc                                                   20

<210>  2113
<211>  26
<212>  DNA
<213>  synthetic construct

<400>  2113
aaaatagtta atagcagtaa aacctg                                            26

<210>  2114
<211>  22
<212>  DNA
<213>  synthetic construct

<400>  2114
ctataatggt gagcgatatt tg                                                22

<210>  2115
<211>  20
<212>  DNA
<213>  synthetic construct

<400>  2115

agaacaacac cttcccatac                                                    20

<210>  2116
<211>  18
<212>  DNA
<213>  synthetic construct

<400>  2116
cgtatcaagt cggcattt                                                      18

<210>  2117
<211>  23
<212>  DNA
<213>  synthetic construct

<400>  2117
gcaatttatc aaagttatca tcc                                                23

<210>  2118
<211>  18
<212>  DNA
<213>  synthetic construct

<400>  2118
ggatatgcca gcaaaaac                                                      18

<210>  2119
<211>  19
<212>  DNA
<213>  synthetic construct

<400>  2119
tgtcaaaatc tggaacgag                                                     19

<210>  2120
<211>  18
<212>  DNA
<213>  synthetic construct

<400>  2120
atcaacgact tccaccaa                                                      18

<210>  2121
<211>  22
<212>  DNA
<213>  synthetic construct

<400>  2121
aatatttacg caaggtttca ag                                                 22

<210>  2122
<211>  25
<212>  DNA
<213>  synthetic construct

<400>  2122

agattacact tttacagcta tctcc                                          25


<210>  2123
<211>  20
<212>  DNA
<213>  synthetic construct

<400>  2123
tcaactccct tccataaatc                                                 20


<210>  2124
<211>  20
<212>  DNA
<213>  synthetic construct

<400>  2124
tctggactct cgataattgg                                                 20


<210>  2125
<211>  19
<212>  DNA
<213>  synthetic construct

<400>  2125
aggagaacac accaggaag                                                  19


<210>  2126
<211>  24
<212>  DNA
<213>  synthetic construct

<400>  2126
acatttgtta tagtttcctt gttg                                            24


<210>  2127
<211>  23
<212>  DNA
<213>  synthetic construct

<400>  2127
tttggtaagt tgagatttag gac                                             23


<210>  2128
<211>  20
<212>  DNA
<213>  synthetic construct

<400>  2128
ttttaaccca ccaagttgac                                                 20


<210>  2129
<211>  20
<212>  DNA
<213>  synthetic construct

<400>  2129

gataccgata gcttggactg                                                20

<210>  2130
<211>  19
<212>  DNA
<213>  synthetic construct

<400>  2130
gtggatgctc aagaaactg                                                 19

<210>  2131
<211>  21
<212>  DNA
<213>  synthetic construct

<400>  2131
cttatgttcg gctaattgtt c                                              21

<210>  2132
<211>  19
<212>  DNA
<213>  synthetic construct

<400>  2132
attatcactg gcggaaaga                                                 19

<210>  2133
<211>  20
<212>  DNA
<213>  synthetic construct

<400>  2133
gataagagcc gtctgaatgt                                                20

<210>  2134
<211>  19
<212>  DNA
<213>  synthetic construct

<400>  2134
aatggaatga acggaagtg                                                 19

<210>  2135
<211>  21
<212>  DNA
<213>  synthetic construct

<400>  2135
   tgttctctct ttttccctag c                                               21

<210>  2136
<211>  20
<212>  DNA
<213>  synthetic construct

<400>  2136

ggtcaactgt ccatatctcc 20

<210> 2137
<211> 22
<212> DNA
<213> synthetic construct

<400> 2137
gactgatttc accaatactt cc 22

<210> 2138
<211> 18
<212> DNA
<213> synthetic construct

<400> 2138
agtcgcacta gccacatt 18

<210> 2139
<211> 19
<212> DNA
<213> synthetic construct

<400> 2139
gagaatcaaa gccaccatc 19

<210> 2140
<211> 20
<212> DNA
<213> synthetic construct

<400> 2140
atttcgagtg ttgcttatgg 20

<210> 2141
<211> 20
<212> DNA
<213> synthetic construct

<400> 2141
gtaaagtgag ccgtcaaatc 20

<210> 2142
<211> 20
<212> DNA
<213> synthetic construct

<400> 2142
gggtaactat gcgacttctg 20

<210> 2143
<211> 20
<212> DNA
<213> synthetic construct

<400> 2143

agctttacac catcatctcc                                                    20

<210>  2144
<211>  21
<212>  DNA
<213>  synthetic construct

<400>  2144
gatcaacaag ctgaagaaga c                                                  21

<210>  2145
<211>  18
<212>  DNA
<213>  synthetic construct

<400>  2145
catctttcac ccaacctg                                                      18

<210>  2146
<211>  21
<212>  DNA
<213>  synthetic construct

<400>  2146
gtgtcagcac aaattacgat t                                                  21

<210>  2147
<211>  18
<212>  DNA
<213>  synthetic construct

<400>  2147
tcgtcactca ccttggaa                                                      18

<210>  2148
<211>  20
<212>  DNA
<213>  synthetic construct

<400>  2148
tgttcggatt gagctagaag                                                    20

<210>  2149
<211>  19
<212>  DNA
<213>  synthetic construct

<400>  2149
taattttacg gtggggttg                                                     19

<210>  2150
<211>  22
<212>  DNA
<213>  synthetic construct

<400>  2150

atctgtagaa ggtcttggtt tc                                              22

<210>  2151
<211>  23
<212>  DNA
<213>  synthetic construct

<400>  2151
attctaaatc acctgaactc ttg                                             23

<210>  2152
<211>  21
<212>  DNA
<213>  synthetic construct

<400>  2152
atttgtgagg atcttgttga g                                               21

<210>  2153
<211>  23
<212>  DNA
<213>  synthetic construct

<400>  2153
ggtattcttc tctatcaaac tgg                                             23

<210>  2154
<211>  21
<212>  DNA
<213>  synthetic construct

<400>  2154
caacttctga ttatgccttt g                                               21

<210>  2155
<211>  19
<212>  DNA
<213>  synthetic construct

<400>  2155
tcctaatgtc cgtccaatc                                                  19

<210>  2156
<211>  24
<212>  DNA
<213>  synthetic construct

<400>  2156
agtggaacta tgtgtttaat cttg                                            24

<210>  2157
<211>  20
<212>  DNA
<213>  synthetic construct

<400>  2157

gatgtcagca accatcctac                                                    20

<210>  2158
<211>  23
<212>  DNA
<213>  synthetic construct

<400>  2158
aaagaaacac tcacatacac ctc                                                23

<210>  2159
<211>  22
<212>  DNA
<213>  synthetic construct

<400>  2159
aaaccatagc ttgactatca cc                                                 22

<210>  2160
<211>  18
<212>  DNA
<213>  synthetic construct

<400>  2160
taaacacgaa gcggaaaa                                                      18

<210>  2161
<211>  22
<212>  DNA
<213>  synthetic construct

<400>  2161
tcaataaaaa gttggttagc ac                                                 22

<210>  2162
<211>  20
<212>  DNA
<213>  synthetic construct

<400>  2162
taatcacatt tgttgccatt                                                    20

<210>  2163
<211>  23
<212>  DNA
<213>  synthetic construct

<400>  2163
catccaaaat acccataata act                                                23

<210>  2164
<211>  23
<212>  DNA
<213>  synthetic construct

<400>  2164

cgtacaaaca tacagtttag tgg                                              23

<210>  2165
<211>  20
<212>  DNA
<213>  synthetic construct

<400>  2165
tccataatcg ccgtaagtag                                                   20

<210>  2166
<211>  19
<212>  DNA
<213>  synthetic construct

<400>  2166
tttggagtcg tggctatct                                                    19

<210>  2167
<211>  22
<212>  DNA
<213>  synthetic construct

<400>  2167
gctaagtgcc tctacattaa ca                                                22

<210>  2168
<211>  22
<212>  DNA
<213>  synthetic construct

<400>  2168
tgatcaagaa gtgttcattc aa                                                22

<210>  2169
<211>  26
<212>  DNA
<213>  synthetic construct

<400>  2169
ttatgacaaa acatatcatt atttcc                                            26

<210>  2170
<211>  23
<212>  DNA
<213>  synthetic construct

<400>  2170
tgaggtttga tgagaactta aaa                                               23

<210>  2171
<211>  25
<212>  DNA
<213>  synthetic construct

<400>  2171

atccctttaa tgataagtat ttgac                                          25

<210>  2172
<211>  20
<212>  DNA
<213>  synthetic construct

<400>  2172
gggttgtcag aagctagaaa                                                20

<210>  2173
<211>  20
<212>  DNA
<213>  synthetic construct

<400>  2173
ttattcgctc gtctaatgct                                                20

<210>  2174
<211>  20
<212>  DNA
<213>  synthetic construct

<400>  2174
tattcttggg tggttctgtc                                                20

<210>  2175
<211>  20
<212>  DNA
<213>  synthetic construct

<400>  2175
agtggacgaa tgtgttatcc                                                20

<210>  2176
<211>  20
<212>  DNA
<213>  synthetic construct

<400>  2176
tacactcggg aatttattgg                                                20

<210>  2177
<211>  20
<212>  DNA
<213>  synthetic construct

<400>  2177
aatcaaggga acagacacag                                                20

<210>  2178
<211>  24
<212>  DNA
<213>  synthetic construct

<400>  2178

tcaggactgt ttatttttat gatt                                              24

<210>  2179
<211>  21
<212>  DNA
<213>  synthetic construct

<400>  2179
cgacgataat tccttaattg c                                                 21

<210>  2180
<211>  18
<212>  DNA
<213>  synthetic construct

<400>  2180
aaagttcgta ttgggtca                                                     18

<210>  2181
<211>  23
<212>  DNA
<213>  synthetic construct

<400>  2181
atgagaattc aacaattaca ata                                               23

<210>  2182
<211>  19
<212>  DNA
<213>  synthetic construct

<400>  2182
cgtttcctta gggactgtt                                                    19

<210>  2183
<211>  22
<212>  DNA
<213>  synthetic construct

<400>  2183
ttgccaataa aataagaatg ag                                                22

<210>  2184
<211>  23
<212>  DNA
<213>  synthetic construct

<400>  2184
ttacctatgg ttgtttagat tgg                                               23

<210>  2185
<211>  20
<212>  DNA
<213>  synthetic construct

<400>  2185

cgaaccctct ttacttgttc                                                    20

<210>  2186
<211>  21
<212>  DNA
<213>  synthetic construct

<400>  2186
ctggtgaaga atgtgaagaa g                                                  21

<210>  2187
<211>  22
<212>  DNA
<213>  synthetic construct

<400>  2187
aaatatcagt aatgggttgt cc                                                 22

<210>  2188
<211>  19
<212>  DNA
<213>  synthetic construct

<400>  2188
cgagcataaa cagcatctc                                                     19

<210>  2189
<211>  19
<212>  DNA
<213>  synthetic construct

<400>  2189
ccagaagaat agggctgac                                                     19

<210>  2190
<211>  20
<212>  DNA
<213>  synthetic construct

<400>  2190
tcttatcatc tgctgcttcc                                                    20

<210>  2191
<211>  19
<212>  DNA
<213>  synthetic construct

<400>  2191
tgacgagaga ggacttgtg                                                     19

<210>  2192
<211>  20
<212>  DNA
<213>  synthetic construct

<400>  2192

agcgatatga tgctttcttt                                                    20

<210>  2193
<211>  22
<212>  DNA
<213>  synthetic construct

<400>  2193
gataatgcct tggtaatgtc tt                                                 22

<210>  2194
<211>  22
<212>  DNA
<213>  synthetic construct

<400>  2194
aaaaataggc agtctacatt gg                                                 22

<210>  2195
<211>  22
<212>  DNA
<213>  synthetic construct

<400>  2195
attcttttaa gtaaaccgct tc                                                 22

<210>  2196
<211>  19
<212>  DNA
<213>  synthetic construct

<400>  2196
agtaatggcc aattgttgc                                                     19

<210>  2197
<211>  22
<212>  DNA
<213>  synthetic construct

<400>  2197
tgactaatcc tactttcggt ga                                                 22

<210>  2198
<211>  18
<212>  DNA
<213>  synthetic construct

<400>  2198
aatccctttg gcttgaga                                                      18

<210>  2199
<211>  22
<212>  DNA
<213>  synthetic construct

<400>  2199

aaaactgacc ctaataatcc ac                                                22

<210>  2200
<211>  23
<212>  DNA
<213>  synthetic construct

<400>  2200
ctattataaa gaacgagggc aaa                                               23

<210>  2201
<211>  24
<212>  DNA
<213>  synthetic construct

<400>  2201
agtactattc attttagcac gaca                                              24

<210>  2202
<211>  20
<212>  DNA
<213>  synthetic construct

<400>  2202
ccctaaatca agccataaag                                                   20

<210>  2203
<211>  20
<212>  DNA
<213>  synthetic construct

<400>  2203
tgcaatcata ccacacaaag                                                   20

<210>  2204
<211>  25
<212>  DNA
<213>  synthetic construct

<400>  2204
agttttatct attgacgatt taagc                                             25

<210>  2205
<211>  24
<212>  DNA
<213>  synthetic construct

<400>  2205
caaccgtaat ccctctatta tatc                                              24

<210>  2206
<211>  20
<212>  DNA
<213>  synthetic construct

<400>  2206

tgttcttaac catgctgttg                                                    20

<210>  2207
<211>  20
<212>  DNA
<213>  synthetic construct

<400>  2207
tcattgattg acgaatgtga                                                    20

<210>  2208
<211>  21
<212>  DNA
<213>  synthetic construct

<400>  2208
taatgaatga aggtgtggaa c                                                  21

<210>  2209
<211>  21
<212>  DNA
<213>  synthetic construct

<400>  2209
attctgactt ctggcgtact t                                                  21

<210>  2210
<211>  20
<212>  DNA
<213>  synthetic construct

<400>  2210
aatgggcatt tctcttattg                                                    20

<210>  2211
<211>  24
<212>  DNA
<213>  synthetic construct

<400>  2211
gtatagaaga tgaagcagca taaa                                               24

<210>  2212
<211>  20
<212>  DNA
<213>  synthetic construct

<400>  2212
tcgaaacctt tctgaggtaa                                                    20

<210>  2213
<211>  24
<212>  DNA
<213>  synthetic construct

<400>  2213

gaacaggtgt tatcatacta gcaa                                              24

<210>  2214
<211>  22
<212>  DNA
<213>  synthetic construct

<400>  2214
tgaatctgga atgtttgata ag                                                22

<210>  2215
<211>  18
<212>  DNA
<213>  synthetic construct

<400>  2215
gaccgatgac gtggataa                                                     18

<210>  2216
<211>  20
<212>  DNA
<213>  synthetic construct

<400>  2216
ttattctttc gacgggtatg                                                   20

<210>  2217
<211>  18
<212>  DNA
<213>  synthetic construct

<400>  2217
atcatcttgc gcttcttg                                                     18

<210>  2218
<211>  21
<212>  DNA
<213>  synthetic construct

<400>  2218
gctgaatacg gaataatgaa g                                                 21

<210>  2219
<211>  22
<212>  DNA
<213>  synthetic construct

<400>  2219
tggttagagg aggaattgat ag                                                22

<210>  2220
<211>  18
<212>  DNA
<213>  synthetic construct

<400>  2220

agtcatgctg atgcgatt                                                    18

<210>  2221
<211>  20
<212>  DNA
<213>  synthetic construct

<400>  2221
cgatgaaata tccaaggttt                                                  20

<210>  2222
<211>  20
<212>  DNA
<213>  synthetic construct

<400>  2222
gcatgaccaa gaggaactaa                                                  20

<210>  2223
<211>  20
<212>  DNA
<213>  synthetic construct

<400>  2223
tgtagtaaac cagcgtcaaa                                                  20

<210>  2224
<211>  21
<212>  DNA
<213>  synthetic construct

<400>  2224
aataccttga aatgtgtcgt g                                                21

<210>  2225
<211>  18
<212>  DNA
<213>  synthetic construct

<400>  2225
ccgtgtggac aatacctg                                                    18

<210>  2226
<211>  26
<212>  DNA
<213>  synthetic construct

<400>  2226
acatatgatg tatctatctg gaactc                                           26

<210>  2227
<211>  21
<212>  DNA
<213>  synthetic construct

<400>  2227

ctgttaaggc ttctacacga c                                                21

<210>  2228
<211>  26
<212>  DNA
<213>  synthetic construct

<400>  2228
tatacaaaat caaaacttga taagga                                           26

<210>  2229
<211>  21
<212>  DNA
<213>  synthetic construct

<400>  2229
ttttaatgct gtttgaagtg c                                                21

<210>  2230
<211>  19
<212>  DNA
<213>  synthetic construct

<400>  2230
ggagcgcgtt tagtttacg                                                   19

<210>  2231
<211>  20
<212>  DNA
<213>  synthetic construct

<400>  2231
gccactactt gtccaccatc                                                  20

<210>  2232
<211>  19
<212>  DNA
<213>  synthetic construct

<400>  2232
ctagggaatg gtctgcttg                                                   19

<210>  2233
<211>  20
<212>  DNA
<213>  synthetic construct

<400>  2233
gagacggatt ggttagagtg                                                  20

<210>  2234
<211>  21
<212>  DNA
<213>  synthetic construct

<400>  2234

tcccacttaa ctatgttgct c                                                21

<210>  2235
<211>  21
<212>  DNA
<213>  synthetic construct

<400>  2235
cttgcctgac tatttgatga c                                                21

<210>  2236
<211>  20
<212>  DNA
<213>  synthetic construct

<400>  2236
tagctcatat tgtcgcaaag                                                  20

<210>  2237
<211>  20
<212>  DNA
<213>  synthetic construct

<400>  2237
tgatggacct ctgttacctc                                                  20

<210>  2238
<211>  21
<212>  DNA
<213>  synthetic construct

<400>  2238
ttaaatacgc taaagccctc t                                                21

<210>  2239
<211>  20
<212>  DNA
<213>  synthetic construct

<400>  2239
agggtgctta atttgacaag                                                  20

<210>  2240
<211>  20
<212>  DNA
<213>  synthetic construct

<400>  2240
ccggttatgt taatggaaag                                                  20

<210>  2241
<211>  21
<212>  DNA
<213>  synthetic construct

<400>  2241

tcaaatcatc ccaagttagt g                                                21

<210>  2242
<211>  19
<212>  DNA
<213>  synthetic construct

<400>  2242
gctatttggc cctgtgtag                                                   19

<210>  2243
<211>  20
<212>  DNA
<213>  synthetic construct

<400>  2243
caaacgtaga tcaatgcaag                                                  20

<210>  2244
<211>  22
<212>  DNA
<213>  synthetic construct

<400>  2244
gctcacggtc actaataatc tc                                               22

<210>  2245
<211>  19
<212>  DNA
<213>  synthetic construct

<400>  2245
ctgacaacca gatcaccac                                                   19

<210>  2246
<211>  21
<212>  DNA
<213>  synthetic construct

<400>  2246
cccagttcaa ttagattacc c                                                21

<210>  2247
<211>  20
<212>  DNA
<213>  synthetic construct

<400>  2247
ttgacttagc ctttgctttc                                                  20

<210>  2248
<211>  19
<212>  DNA
<213>  synthetic construct

<400>  2248

tttgatggtg ttgggtatg                                                    19

<210>  2249
<211>  20
<212>  DNA
<213>  synthetic construct

<400>  2249
aaagaggtag gcatttgttg                                                   20

<210>  2250
<211>  22
<212>  DNA
<213>  synthetic construct

<400>  2250
tcgaagagat tttctatgat cc                                                22

<210>  2251
<211>  19
<212>  DNA
<213>  synthetic construct

<400>  2251
cagacatttc cttcctcct                                                    19

<210>  2252
<211>  20
<212>  DNA
<213>  synthetic construct

<400>  2252
caccagacaa cctttctttc                                                   20

<210>  2253
<211>  20
<212>  DNA
<213>  synthetic construct

<400>  2253
caagtgtaac cagagcttcc                                                   20

<210>  2254
<211>  20
<212>  DNA
<213>  synthetic construct

<400>  2254
ttaattgcag tagcagcatc                                                   20

<210>  2255
<211>  19
<212>  DNA
<213>  synthetic construct

<400>  2255

agccaccaat aatgaggag                                                    19

<210>  2256
<211>  23
<212>  DNA
<213>  synthetic construct

<400>  2256
agcagtaatc tttggtactg ttt                                              23

<210>  2257
<211>  26
<212>  DNA
<213>  synthetic construct

<400>  2257
tggtgaagat agatgagata cttaat                                           26

<210>  2258
<211>  23
<212>  DNA
<213>  synthetic construct

<400>  2258
ttatctgatt taggacattt atc                                              23

<210>  2259
<211>  22
<212>  DNA
<213>  synthetic construct

<400>  2259
atatcagtta atggacaagc ag                                               22

<210>  2260
<211>  21
<212>  DNA
<213>  synthetic construct

<400>  2260
cacgagaagt tcatttcaat c                                                21

<210>  2261
<211>  22
<212>  DNA
<213>  synthetic construct

<400>  2261
tcaatcgtga taacagacta gc                                               22

<210>  2262
<211>  20
<212>  DNA
<213>  synthetic construct

<400>  2262

aagaaatgga gcaaacaaac 20

<210> 2263
<211> 20
<212> DNA
<213> synthetic construct

<400> 2263
atatccttct gggctattgg 20

<210> 2264
<211> 20
<212> DNA
<213> synthetic construct

<400> 2264
aacaggttga tcagcatagg 20

<210> 2265
<211> 21
<212> DNA
<213> synthetic construct

<400> 2265
tgacaacaat tagtgctgaa g 21

<210> 2266
<211> 20
<212> DNA
<213> synthetic construct

<400> 2266
tcaacccata ccattgaaac 20

<210> 2267
<211> 20
<212> DNA
<213> synthetic construct

<400> 2267
cgctatacga ttaccgagac 20

<210> 2268
<211> 19
<212> DNA
<213> synthetic construct

<400> 2268
gcaacgacga cataatcac 19

<210> 2269
<211> 19
<212> DNA
<213> synthetic construct

<400> 2269

tggcttcaac ttacactgg 19

<210> 2270
<211> 20
<212> DNA
<213> synthetic construct

<400> 2270
tctggatatt actgccaacc 20

<210> 2271
<211> 21
<212> DNA
<213> synthetic construct

<400> 2271
aacaaccttt agcgtttatt g 21

<210> 2272
<211> 21
<212> DNA
<213> synthetic construct

<400> 2272
cggtaatgtg cttatagatg g 21

<210> 2273
<211> 20
<212> DNA
<213> synthetic construct

<400> 2273
tttctgtggt tccaggtaag 20

<210> 2274
<211> 19
<212> DNA
<213> synthetic construct

<400> 2274
cttactggtc ccaaaggtc 19

<210> 2275
<211> 20
<212> DNA
<213> synthetic construct

<400> 2275
tctaatttgc atcgcactac 20

<210> 2276
<211> 23
<212> DNA
<213> synthetic construct

<400> 2276

aatctactga ctaacaagcc aaa                                              23

<210>  2277
<211>  20
<212>  DNA
<213>  synthetic construct

<400>  2277
atcataatcc gctccaatac                                                   20

<210>  2278
<211>  21
<212>  DNA
<213>  synthetic construct

<400>  2278
tttcaacgta tgggttatgt c                                                 21

<210>  2279
<211>  18
<212>  DNA
<213>  synthetic construct

<400>  2279
tgggaaggtc acatcttg                                                     18

<210>  2280
<211>  23
<212>  DNA
<213>  synthetic construct

<400>  2280
tctcactagg caaacctatt atc                                               23

<210>  2281
<211>  21
<212>  DNA
<213>  synthetic construct

<400>  2281
ccgttcatat aagcaatcat c                                                 21

<210>  2282
<211>  21
<212>  DNA
<213>  synthetic construct

<400>  2282
cagacgatca gcaagataaa g                                                 21

<210>  2283
<211>  21
<212>  DNA
<213>  synthetic construct

<400>  2283

ctactaatgg cgcagacata c                                                21

<210>  2284
<211>  19
<212>  DNA
<213>  synthetic construct

<400>  2284
cagaaaccac aacgacaag                                                   19

<210>  2285
<211>  19
<212>  DNA
<213>  synthetic construct

<400>  2285
aacattggca taggtaggg                                                   19

<210>  2286
<211>  26
<212>  DNA
<213>  synthetic construct

<400>  2286
agtgactaag aaacttgatg ttagag                                           26

<210>  2287
<211>  25
<212>  DNA
<213>  synthetic construct

<400>  2287
ctatatctaa atgcccaatt ttatc                                            25

<210>  2288
<211>  20
<212>  DNA
<213>  synthetic construct

<400>  2288
ttatctgtag ggctcgtctc                                                  20

<210>  2289
<211>  20
<212>  DNA
<213>  synthetic construct

<400>  2289
caataggcca tcttcgttag                                                  20

<210>  2290
<211>  20
<212>  DNA
<213>  synthetic construct

<400>  2290

gtactagaca tggccgaaaa                                                    20

<210>  2291
<211>  22
<212>  DNA
<213>  synthetic construct

<400>  2291
tcagtaacat caacggaata ga                                                 22

<210>  2292
<211>  18
<212>  DNA
<213>  synthetic construct

<400>  2292
tccaagccaa cttcacag                                                      18

<210>  2293
<211>  21
<212>  DNA
<213>  synthetic construct

<400>  2293
ttgagtaaat tctggttcag g                                                  21

<210>  2294
<211>  20
<212>  DNA
<213>  synthetic construct

<400>  2294
agcattaggt ggatttgttc                                                    20

<210>  2295
<211>  20
<212>  DNA
<213>  synthetic construct

<400>  2295
tctttcaacc ctttgttagg                                                    20

<210>  2296
<211>  26
<212>  DNA
<213>  synthetic construct

<400>  2296
tcatactgat ttctacttat ttcacc                                             26

<210>  2297
<211>  22
<212>  DNA
<213>  synthetic construct

<400>  2297

aagtctattt gctcatgttt cc                                                22

<210>  2298
<211>  20
<212>  DNA
<213>  synthetic construct

<400>  2298
ataatctttc atgggtacgg                                                    20

<210>  2299
<211>  20
<212>  DNA
<213>  synthetic construct

<400>  2299
ttgccctttt atgtatggag                                                    20

<210>  2300
<211>  21
<212>  DNA
<213>  synthetic construct

<400>  2300
ttgctagtcc cagtctttat g                                                  21

<210>  2301
<211>  23
<212>  DNA
<213>  synthetic construct

<400>  2301
cgttgtaatc ttcaatcatc ttc                                                23

<210>  2302
<211>  26
<212>  DNA
<213>  synthetic construct

<400>  2302
gatagtagct gaaagtgaga ataaag                                             26

<210>  2303
<211>  19
<212>  DNA
<213>  synthetic construct

<400>  2303
aggccatagt ttgtcatcc                                                     19

<210>  2304
<211>  19
<212>  DNA
<213>  synthetic construct

<400>  2304

ttaactgcgg aaacaacag                                                    19

<210>  2305
<211>  23
<212>  DNA
<213>  synthetic construct

<400>  2305
gagatagcaa atagattggt cag                                              23

<210>  2306
<211>  18
<212>  DNA
<213>  synthetic construct

<400>  2306
taatcggtcc aaatggtg                                                    18

<210>  2307
<211>  20
<212>  DNA
<213>  synthetic construct

<400>  2307
tcagagccgt catattcttt                                                  20

<210>  2308
<211>  19
<212>  DNA
<213>  synthetic construct

<400>  2308
ggaggtttac aaagcagga                                                   19

<210>  2309
<211>  20
<212>  DNA
<213>  synthetic construct

<400>  2309
gctactgtgc atgaaacaaa                                                  20

<210>  2310
<211>  25
<212>  DNA
<213>  synthetic construct

<400>  2310
aattagattt gaccattaca gagtt                                            25

<210>  2311
<211>  23
<212>  DNA
<213>  synthetic construct

<400>  2311

ttactcccat tgtatttcat ttc                                              23

<210>  2312
<211>  20
<212>  DNA
<213>  synthetic construct

<400>  2312
gtcgttgaaa ttgtctttgg                                                    20

<210>  2313
<211>  22
<212>  DNA
<213>  synthetic construct

<400>  2313
acttctatcc ccattttatt tg                                                 22

<210>  2314
<211>  21
<212>  DNA
<213>  synthetic construct

<400>  2314
cttatgaaga ctgctttgga g                                                  21

<210>  2315
<211>  22
<212>  DNA
<213>  synthetic construct

<400>  2315
gaaatagaat gaaacaatgc tg                                                 22

<210>  2316
<211>  20
<212>  DNA
<213>  synthetic construct

<400>  2316
ttctccctac tggtgttctc                                                    20

<210>  2317
<211>  20
<212>  DNA
<213>  synthetic construct

<400>  2317
atttgctctc tcatcgctac                                                    20

<210>  2318
<211>  22
<212>  DNA
<213>  synthetic construct

<400>  2318

agcgatcctt atcttacttg tt                                                22

<210>  2319
<211>  20
<212>  DNA
<213>  synthetic construct

<400>  2319
ctctcctatt caacgcaaag                                                   20

<210>  2320
<211>  21
<212>  DNA
<213>  synthetic construct

<400>  2320
acgtcctctt aacagtcaca a                                                 21

<210>  2321
<211>  18
<212>  DNA
<213>  synthetic construct

<400>  2321
tgcgactagg atggtttc                                                     18

<210>  2322
<211>  19
<212>  DNA
<213>  synthetic construct

<400>  2322
atcaggctgt tatgcgtct                                                    19

<210>  2323
<211>  18
<212>  DNA
<213>  synthetic construct

<400>  2323
tgcccatctt tcatttca                                                     18

<210>  2324
<211>  22
<212>  DNA
<213>  synthetic construct

<400>  2324
aattgtgaac cagttagaaa cc                                                22

<210>  2325
<211>  18
<212>  DNA
<213>  synthetic construct

<400>  2325

tgccacctga tagtgagc                                                    18

<210>  2326
<211>  21
<212>  DNA
<213>  synthetic construct

<400>  2326
gaagaaacgt gaacgattag a                                                21

<210>  2327
<211>  18
<212>  DNA
<213>  synthetic construct

<400>  2327
ttaattggcc atccaaga                                                    18

<210>  2328
<211>  22
<212>  DNA
<213>  synthetic construct

<400>  2328
agatatgatt gcaacaattg aa                                               22

<210>  2329
<211>  20
<212>  DNA
<213>  synthetic construct

<400>  2329
cgcatgattg atttgataag                                                  20

<210>  2330
<211>  20
<212>  DNA
<213>  synthetic construct

<400>  2330
gtgaaaagtc gcatcaaatc                                                  20

<210>  2331
<211>  21
<212>  DNA
<213>  synthetic construct

<400>  2331
gccataaaca tacatgactc c                                                21

<210>  2332
<211>  21
<212>  DNA
<213>  synthetic construct

<400>  2332

cagctgttgc agaagattta g                                                21

<210>  2333
<211>  19
<212>  DNA
<213>  synthetic construct

<400>  2333
ccattagctg cttgtgatg                                                   19

<210>  2334
<211>  23
<212>  DNA
<213>  synthetic construct

<400>  2334
ttctcttaaa tcaaacagga agt                                              23

<210>  2335
<211>  18
<212>  DNA
<213>  synthetic construct

<400>  2335
acccatttgg cattaacc                                                    18

<210>  2336
<211>  22
<212>  DNA
<213>  synthetic construct

<400>  2336
aattgttctc ttattggctt tc                                               22

<210>  2337
<211>  22
<212>  DNA
<213>  synthetic construct

<400>  2337
cctggatttt taataggttt ca                                               22

<210>  2338
<211>  18
<212>  DNA
<213>  synthetic construct

<400>  2338
gtcttggcgc gagtttta                                                    18

<210>  2339
<211>  18
<212>  DNA
<213>  synthetic construct

<400>  2339

ttgaagcctt ttgagcag 18

<210> 2340
<211> 19
<212> DNA
<213> synthetic construct

<400> 2340
cattggttcg atcagtcag 19

<210> 2341
<211> 23
<212> DNA
<213> synthetic construct

<400> 2341
gctttggtag gatatattag tgg 23

<210> 2342
<211> 23
<212> DNA
<213> synthetic construct

<400> 2342
tgattaaagg agttaagttg gtg 23

<210> 2343
<211> 21
<212> DNA
<213> synthetic construct

<400> 2343
tgtcaaaatt agcacagatt g 21

<210> 2344
<211> 23
<212> DNA
<213> synthetic construct

<400> 2344
agaaaaagtt gactacgaaa aag 23

<210> 2345
<211> 20
<212> DNA
<213> synthetic construct

<400> 2345
agataaataa cgcgcacaag 20

<210> 2346
<211> 21
<212> DNA
<213> synthetic construct

<400> 2346

tgatgctgag tacaaggatt t                                                21

<210>  2347
<211>  21
<212>  DNA
<213>  synthetic construct

<400>  2347
ctttaaaatc agccttatcc a                                                21

<210>  2348
<211>  18
<212>  DNA
<213>  synthetic construct

<400>  2348
cggcagacaa gtcagtca                                                    18

<210>  2349
<211>  25
<212>  DNA
<213>  synthetic construct

<400>  2349
tataatttct ctatcatcac caaaa                                            25

<210>  2350
<211>  23
<212>  DNA
<213>  synthetic construct

<400>  2350
gtgatattat ccaaaccatt ctc                                              23

<210>  2351
<211>  19
<212>  DNA
<213>  synthetic construct

<400>  2351
ttaaagcggt caaaaggtc                                                   19

<210>  2352
<211>  20
<212>  DNA
<213>  synthetic construct

<400>  2352
attggacttg tgtcagcttc                                                  20

<210>  2353
<211>  20
<212>  DNA
<213>  synthetic construct

<400>  2353

cctttgtcta ccttcattgg                                                    20

<210>  2354
<211>  21
<212>  DNA
<213>  synthetic construct

<400>  2354
gaaggcaatg aggagactta c                                                  21

<210>  2355
<211>  19
<212>  DNA
<213>  synthetic construct

<400>  2355
ctactcggcc agacaagac                                                     19

<210>  2356
<211>  21
<212>  DNA
<213>  synthetic construct

<400>  2356
ttttattggg aggttttctt t                                                  21

<210>  2357
<211>  19
<212>  DNA
<213>  synthetic construct

<400>  2357
aacaaatatg aaggcagca                                                     19

<210>  2358
<211>  24
<212>  DNA
<213>  synthetic construct

<400>  2358
tgaaaaagtt attattatct gcaa                                               24

<210>  2359
<211>  21
<212>  DNA
<213>  synthetic construct

<400>  2359
atcagtggaa ataagagaag c                                                  21

<210>  2360
<211>  21
<212>  DNA
<213>  synthetic construct

<400>  2360

agatgaaaag cttgctaatg a                                                21

<210>  2361
<211>  20
<212>  DNA
<213>  synthetic construct

<400>  2361
ttaatgaacg atttcttttt                                                  20

<210>  2362
<211>  18
<212>  DNA
<213>  synthetic construct

<400>  2362
tggctgactc tcctgatg                                                    18

<210>  2363
<211>  22
<212>  DNA
<213>  synthetic construct

<400>  2363
ttgcagatag gtattgatat gg                                               22

<210>  2364
<211>  21
<212>  DNA
<213>  synthetic construct

<400>  2364
atggataata agcgaacaca g                                                21

<210>  2365
<211>  19
<212>  DNA
<213>  synthetic construct

<400>  2365
gacatactgc cacaacgac                                                   19

<210>  2366
<211>  23
<212>  DNA
<213>  synthetic construct

<400>  2366
ttatattgaa acactgaagc aac                                              23

<210>  2367
<211>  18
<212>  DNA
<213>  synthetic construct

<400>  2367

ctcctgccca ttgttcta                                                    18

<210>  2368
<211>  20
<212>  DNA
<213>  synthetic construct

<400>  2368
cgctattaac cttgctgaac                                                  20

<210>  2369
<211>  20
<212>  DNA
<213>  synthetic construct

<400>  2369
cctttctcac tcaccacatc                                                  20

<210>  2370
<211>  19
<212>  DNA
<213>  synthetic construct

<400>  2370
ttgttgcaat acagccatc                                                   19

<210>  2371
<211>  24
<212>  DNA
<213>  synthetic construct

<400>  2371
cgataaatta caaaatacgt ttca                                             24

<210>  2372
<211>  22
<212>  DNA
<213>  synthetic construct

<400>  2372
aactttagcc ccactactat ca                                               22

<210>  2373
<211>  19
<212>  DNA
<213>  synthetic construct

<400>  2373
aataccatta tcggggaag                                                   19

<210>  2374
<211>  18
<212>  DNA
<213>  synthetic construct

<400>  2374

actccgccag accttaga                                                    18

<210>  2375
<211>  20
<212>  DNA
<213>  synthetic construct

<400>  2375
atattgaacg ctgtgcttgt                                                  20

<210>  2376
<211>  18
<212>  DNA
<213>  synthetic construct

<400>  2376
gcgattctat ggctgatg                                                    18

<210>  2377
<211>  20
<212>  DNA
<213>  synthetic construct

<400>  2377
ttatgcccgt ttctttgtag                                                  20

<210>  2378
<211>  24
<212>  DNA
<213>  synthetic construct

<400>  2378
tctaatctta attcctctgt tcct                                             24

<210>  2379
<211>  26
<212>  DNA
<213>  synthetic construct

<400>  2379
tgatatagac ttgattaaag gtgatt                                           26

<210>  2380
<211>  20
<212>  DNA
<213>  synthetic construct

<400>  2380
tctttactga gcttccatcc                                                  20

<210>  2381
<211>  22
<212>  DNA
<213>  synthetic construct

<400>  2381

catcactatc tggctctatt tg                                              22


<210>  2382
<211>  20
<212>  DNA
<213>  synthetic construct

<400>  2382
tattgagcgc attgatttac                                                 20


<210>  2383
<211>  20
<212>  DNA
<213>  synthetic construct

<400>  2383
ctatgcacat taggacgttg                                                 20


<210>  2384
<211>  22
<212>  DNA
<213>  synthetic construct

<400>  2384
tcatatcgct gtattacctt ga                                              22


<210>  2385
<211>  19
<212>  DNA
<213>  synthetic construct

<400>  2385
cttattagcc gcccttaac                                                  19


<210>  2386
<211>  20
<212>  DNA
<213>  synthetic construct

<400>  2386
agcgcaaact cttcagatac                                                 20


<210>  2387
<211>  20
<212>  DNA
<213>  synthetic construct

<400>  2387
tttaatcact cgtcctagcc                                                 20


<210>  2388
<211>  19
<212>  DNA
<213>  synthetic construct

<400>  2388

aatattggtc aagctgcaa                                                    19

<210>  2389
<211>  19
<212>  DNA
<213>  synthetic construct

<400>  2389
atgttctgtc cctgtttgg                                                    19

<210>  2390
<211>  20
<212>  DNA
<213>  synthetic construct

<400>  2390
aaatagtggt ggtgtgttcc                                                   20

<210>  2391
<211>  21
<212>  DNA
<213>  synthetic construct

<400>  2391
ttagcgtctt tcccttctaa c                                                 21

<210>  2392
<211>  23
<212>  DNA
<213>  synthetic construct

<400>  2392
atgaaagcaa aaattgtact agg                                               23

<210>  2393
<211>  23
<212>  DNA
<213>  synthetic construct

<400>  2393
ttacttggtt atctagacgc tga                                               23

<210>  2394
<211>  21
<212>  DNA
<213>  synthetic construct

<400>  2394
tgcacttgtt tacgagagat t                                                 21

<210>  2395
<211>  20
<212>  DNA
<213>  synthetic construct

<400>  2395

gaaatggcct ctactgtttg                                                    20

<210>  2396
<211>  20
<212>  DNA
<213>  synthetic construct

<400>  2396
cagacaatgc gtttattttg                                                    20

<210>  2397
<211>  18
<212>  DNA
<213>  synthetic construct

<400>  2397
tacgtaatgc ggcgattc                                                      18

<210>  2398
<211>  23
<212>  DNA
<213>  synthetic construct

<400>  2398
aaacaataga tactttgcca ctt                                                23

<210>  2399
<211>  20
<212>  DNA
<213>  synthetic construct

<400>  2399
ttcgctgtta atatcccact                                                    20

<210>  2400
<211>  19
<212>  DNA
<213>  synthetic construct

<400>  2400
cttgccgtag ttgaaggtc                                                     19

<210>  2401
<211>  20
<212>  DNA
<213>  synthetic construct

<400>  2401
tacattggca ttagcatcag                                                    20

<210>  2402
<211>  24
<212>  DNA
<213>  synthetic construct

<400>  2402

ttaagtaaag ctaaatccag aatg                                              24

<210>  2403
<211>  20
<212>  DNA
<213>  synthetic construct

<400>  2403
ataaatgatg tggcaggttg                                                    20

<210>  2404
<211>  20
<212>  DNA
<213>  synthetic construct

<400>  2404
attattctcg ccagtgtttc                                                    20

<210>  2405
<211>  19
<212>  DNA
<213>  synthetic construct

<400>  2405
cgatacgttt agcaccttg                                                     19

<210>  2406
<211>  25
<212>  DNA
<213>  synthetic construct

<400>  2406
aatgtaaata aagacaatgc taacc                                              25

<210>  2407
<211>  18
<212>  DNA
<213>  synthetic construct

<400>  2407
atcttgccat gcttcaac                                                      18

<210>  2408
<211>  20
<212>  DNA
<213>  synthetic construct

<400>  2408
taaagcattc ggtattccac                                                    20

<210>  2409
<211>  20
<212>  DNA
<213>  synthetic construct

<400>  2409

agcgcattat tccattctac                                                    20

<210>  2410
<211>  21
<212>  DNA
<213>  synthetic construct

<400>  2410
aattggctac tcctcgatta c                                                  21

<210>  2411
<211>  19
<212>  DNA
<213>  synthetic construct

<400>  2411
tgttgtgcta aagggtgtg                                                     19

<210>  2412
<211>  18
<212>  DNA
<213>  synthetic construct

<400>  2412
tgcggcttta gtatttgg                                                      18

<210>  2413
<211>  19
<212>  DNA
<213>  synthetic construct

<400>  2413
gaagttgata acggcttgg                                                     19

<210>  2414
<211>  19
<212>  DNA
<213>  synthetic construct

<400>  2414
tttctcacaa gcagagcag                                                     19

<210>  2415
<211>  19
<212>  DNA
<213>  synthetic construct

<400>  2415
tcatcgcttt gtaaattgg                                                     19

<210>  2416
<211>  20
<212>  DNA
<213>  synthetic construct

<400>  2416

atgccgtatt agatcacacc                                                    20

<210>  2417
<211>  20
<212>  DNA
<213>  synthetic construct

<400>  2417
tttcccacat taccaaagtc                                                    20

<210>  2418
<211>  20
<212>  DNA
<213>  synthetic construct

<400>  2418
aacaggcaca ttaacagagg                                                    20

<210>  2419
<211>  20
<212>  DNA
<213>  synthetic construct

<400>  2419
tttatcttca gggagcaatc                                                    20

<210>  2420
<211>  20
<212>  DNA
<213>  synthetic construct

<400>  2420
gcacactgac ccaattaaag                                                    20

<210>  2421
<211>  20
<212>  DNA
<213>  synthetic construct

<400>  2421
attaaacatg gtgcgatagg                                                    20

<210>  2422
<211>  18
<212>  DNA
<213>  synthetic construct

<400>  2422
tggcttggaa acacaatc                                                      18

<210>  2423
<211>  20
<212>  DNA
<213>  synthetic construct

<400>  2423

aattcgagtg atggacaaac                                                    20

<210>  2424
<211>  21
<212>  DNA
<213>  synthetic construct

<400>  2424
cgagcatgac actaataatg g                                                 21

<210>  2425
<211>  19
<212>  DNA
<213>  synthetic construct

<400>  2425
ccgcttgcag atatagcat                                                    19

<210>  2426
<211>  18
<212>  DNA
<213>  synthetic construct

<400>  2426
tacgtacatc gccaccag                                                     18

<210>  2427
<211>  22
<212>  DNA
<213>  synthetic construct

<400>  2427
aaaggaacag gactactttg ag                                                22

<210>  2428
<211>  20
<212>  DNA
<213>  synthetic construct

<400>  2428
gaaatacgca ttaggctctg                                                   20

<210>  2429
<211>  21
<212>  DNA
<213>  synthetic construct

<400>  2429
gcgttatagc agtttaatcc a                                                 21

<210>  2430
<211>  22
<212>  DNA
<213>  synthetic construct

<400>  2430

caaggtttcg ctaactaaag ag                                                22

<210>  2431
<211>  20
<212>  DNA
<213>  synthetic construct

<400>  2431
actttgattg atccgttttc                                                   20

<210>  2432
<211>  23
<212>  DNA
<213>  synthetic construct

<400>  2432
ttctaaaggt ggtaatgttt ctt                                               23

<210>  2433
<211>  18
<212>  DNA
<213>  synthetic construct

<400>  2433
gcatatcctt ggcctaca                                                     18

<210>  2434
<211>  20
<212>  DNA
<213>  synthetic construct

<400>  2434
gagccttgtt tatccctctt                                                   20

<210>  2435
<211>  19
<212>  DNA
<213>  synthetic construct

<400>  2435
tctatcccag cggttaaaa                                                    19

<210>  2436
<211>  20
<212>  DNA
<213>  synthetic construct

<400>  2436
gctcatatca tcttccatcc                                                   20

<210>  2437
<211>  20
<212>  DNA
<213>  synthetic construct

<400>  2437

cctaagcaaa ttaacgaacc 20

<210> 2438
<211> 24
<212> DNA
<213> synthetic construct

<400> 2438
aagataggta cgcttttaat ttct 24

<210> 2439
<211> 22
<212> DNA
<213> synthetic construct

<400> 2439
agtaataatt tagctcgcca tt 22

<210> 2440
<211> 22
<212> DNA
<213> synthetic construct

<400> 2440
tcacagtcac cactaatctc ac 22

<210> 2441
<211> 18
<212> DNA
<213> synthetic construct

<400> 2441
acgaaacgcc ctctttac 18

<210> 2442
<211> 19
<212> DNA
<213> synthetic construct

<400> 2442
acgtccctga aacactctc 19

<210> 2443
<211> 20
<212> DNA
<213> synthetic construct

<400> 2443
aaccaatgct gtaaccactc 20

<210> 2444
<211> 19
<212> DNA
<213> synthetic construct

<400> 2444

caggaacggg tttcttatc                                                    19

<210>  2445
<211>  26
<212>  DNA
<213>  synthetic construct

<400>  2445
gcttataaaa gactagataa atgtcg                                            26

<210>  2446
<211>  21
<212>  DNA
<213>  synthetic construct

<400>  2446
atcacttcta tccaaaacga a                                                 21

<210>  2447
<211>  20
<212>  DNA
<213>  synthetic construct

<400>  2447
aagttgctaa agcgtcatct                                                   20

<210>  2448
<211>  21
<212>  DNA
<213>  synthetic construct

<400>  2448
caacagtgat ttccatttga g                                                 21

<210>  2449
<211>  19
<212>  DNA
<213>  synthetic construct

<400>  2449
acgccttgaa cctttagac                                                    19

<210>  2450
<211>  22
<212>  DNA
<213>  synthetic construct

<400>  2450
acacaatcca ttcatcagtt ag                                                22

<210>  2451
<211>  19
<212>  DNA
<213>  synthetic construct

<400>  2451

tttcgggaag caattctat                                                    19

<210>  2452
<211>  22
<212>  DNA
<213>  synthetic construct

<400>  2452
gacttaattg ctcctcgtat tg                                                22

<210>  2453
<211>  20
<212>  DNA
<213>  synthetic construct

<400>  2453
cgcgtctaag gtaacatctc                                                   20

<210>  2454
<211>  20
<212>  DNA
<213>  synthetic construct

<400>  2454
attaagcgca aatgaaacag                                                   20

<210>  2455
<211>  20
<212>  DNA
<213>  synthetic construct

<400>  2455
gatattgaga tgccaaggtg                                                   20

<210>  2456
<211>  25
<212>  DNA
<213>  synthetic construct

<400>  2456
aaaaatgtag tgtttcagat tttag                                             25

<210>  2457
<211>  25
<212>  DNA
<213>  synthetic construct

<400>  2457
tcatagcgat actttattat gtttt                                             25

<210>  2458
<211>  26
<212>  DNA
<213>  synthetic construct

<400>  2458

taaattagct ttagttcttg gtttag                                          26

<210>  2459
<211>  18
<212>  DNA
<213>  synthetic construct

<400>  2459
gttttgagca gcacttgg                                                   18

<210>  2460
<211>  19
<212>  DNA
<213>  synthetic construct

<400>  2460
tggcaccact attgctatg                                                  19

<210>  2461
<211>  20
<212>  DNA
<213>  synthetic construct

<400>  2461
ttttggcgta tcgttatttt                                                 20

<210>  2462
<211>  24
<212>  DNA
<213>  synthetic construct

<400>  2462
gatggtaatg ctgataataa caaa                                            24

<210>  2463
<211>  19
<212>  DNA
<213>  synthetic construct

<400>  2463
actaacgcgg tgtaatgct                                                  19

<210>  2464
<211>  18
<212>  DNA
<213>  synthetic construct

<400>  2464
caatctatgc ctcgcaaa                                                   18

<210>  2465
<211>  19
<212>  DNA
<213>  synthetic construct

<400>  2465

agctgccgtt tctgttact                                                    19

<210>  2466
<211>  19
<212>  DNA
<213>  synthetic construct

<400>  2466
aacagtggtt tagcccaag                                                    19

<210>  2467
<211>  18
<212>  DNA
<213>  synthetic construct

<400>  2467
tggcaacaaa atctccac                                                     18

<210>  2468
<211>  25
<212>  DNA
<213>  synthetic construct

<400>  2468
ttactaaatt ctacagcagt aatgg                                             25

<210>  2469
<211>  23
<212>  DNA
<213>  synthetic construct

<400>  2469
attggtattc tgctaataag gtg                                               23

<210>  2470
<211>  18
<212>  DNA
<213>  synthetic construct

<400>  2470
ccggttcata ttgggtct                                                     18

<210>  2471
<211>  24
<212>  DNA
<213>  synthetic construct

<400>  2471
tcagtcataa ttcaaataga aggt                                              24

<210>  2472
<211>  20
<212>  DNA
<213>  synthetic construct

<400>  2472

cctttctaacg ccacttacac                                                    20

<210>  2473
<211>  20
<212>  DNA
<213>  synthetic construct

<400>  2473
atgcgataac caacactttc                                                    20

<210>  2474
<211>  20
<212>  DNA
<213>  synthetic construct

<400>  2474
aacatatgag ggtgtggact                                                    20

<210>  2475
<211>  23
<212>  DNA
<213>  synthetic construct

<400>  2475
gaagcaacgt aaatttctta cac                                                23

<210>  2476
<211>  22
<212>  DNA
<213>  synthetic construct

<400>  2476
ttgattttgc gaatatagat gt                                                 22

<210>  2477
<211>  21
<212>  DNA
<213>  synthetic construct

<400>  2477
caataatgaa aagcatcagg t                                                  21

<210>  2478
<211>  23
<212>  DNA
<213>  synthetic construct

<400>  2478
tggagtatca gagctatttt gtt                                                23

<210>  2479
<211>  25
<212>  DNA
<213>  synthetic construct

<400>  2479

agtcagccat aatttatcta gtttt                                          25

<210>  2480
<211>  20
<212>  DNA
<213>  synthetic construct

<400>  2480
ctatcagcca cctcttcttg                                                20

<210>  2481
<211>  20
<212>  DNA
<213>  synthetic construct

<400>  2481
ctaatgagca tcgtttagcc                                                20

<210>  2482
<211>  24
<212>  DNA
<213>  synthetic construct

<400>  2482
gcactagcta ctattctttc tgct                                           24

<210>  2483
<211>  19
<212>  DNA
<213>  synthetic construct

<400>  2483
aacggttgct tttacatcc                                                 19

<210>  2484
<211>  18
<212>  DNA
<213>  synthetic construct

<400>  2484
gaggtactgc atcgcaaa                                                  18

<210>  2485
<211>  22
<212>  DNA
<213>  synthetic construct

<400>  2485
taccttctct aaatggtgtt ga                                             22

<210>  2486
<211>  20
<212>  DNA
<213>  synthetic construct

<400>  2486

gcgcttgaac taacctctac                                                    20

<210>  2487
<211>  18
<212>  DNA
<213>  synthetic construct

<400>  2487
ggatgaccca ccataagc                                                      18

<210>  2488
<211>  19
<212>  DNA
<213>  synthetic construct

<400>  2488
gctcagcaga aaccttgtc                                                     19

<210>  2489
<211>  19
<212>  DNA
<213>  synthetic construct

<400>  2489
ctggcgtaaa ctgccaata                                                     19

<210>  2490
<211>  24
<212>  DNA
<213>  synthetic construct

<400>  2490
gggatcttct ataactattc aacc                                               24

<210>  2491
<211>  20
<212>  DNA
<213>  synthetic construct

<400>  2491
taacctggaa cattctgacc                                                    20

<210>  2492
<211>  22
<212>  DNA
<213>  synthetic construct

<400>  2492
tttctttgat ctaccttggt tc                                                 22

<210>  2493
<211>  23
<212>  DNA
<213>  synthetic construct

<400>  2493

tgtaggctgc tttactacta tcc                                              23

<210>  2494
<211>  23
<212>  DNA
<213>  synthetic construct

<400>  2494
aagaacaagt agcaggtaaa gag                                              23

<210>  2495
<211>  19
<212>  DNA
<213>  synthetic construct

<400>  2495
cagttagctt tgcgatgtg                                                   19

<210>  2496
<211>  25
<212>  DNA
<213>  synthetic construct

<400>  2496
ccaacttact tctatctacc tgatg                                            25

<210>  2497
<211>  23
<212>  DNA
<213>  synthetic construct

<400>  2497
gttccagagt attggtattg ttc                                              23

<210>  2498
<211>  20
<212>  DNA
<213>  synthetic construct

<400>  2498
catcttattg tgggtccaag                                                  20

<210>  2499
<211>  20
<212>  DNA
<213>  synthetic construct

<400>  2499
aagagcgaat acctgatgac                                                  20

<210>  2500
<211>  19
<212>  DNA
<213>  synthetic construct

<400>  2500

cgtaagcctt atgttcgtg                                                    19

<210>  2501
<211>  19
<212>  DNA
<213>  synthetic construct

<400>  2501
taaacaatta gcgccactg                                                    19

<210>  2502
<211>  22
<212>  DNA
<213>  synthetic construct

<400>  2502
aatcagaatc aacttcctaa gc                                                22

<210>  2503
<211>  21
<212>  DNA
<213>  synthetic construct

<400>  2503
aacaccaata attgcgataa c                                                 21

<210>  2504
<211>  20
<212>  DNA
<213>  synthetic construct

<400>  2504
gcgaagtaga agagaaagca                                                   20

<210>  2505
<211>  19
<212>  DNA
<213>  synthetic construct

<400>  2505
tttcagagtg gcggttatt                                                    19

<210>  2506
<211>  20
<212>  DNA
<213>  synthetic construct

<400>  2506
agctgatgtt gttgttgttg                                                   20

<210>  2507
<211>  19
<212>  DNA
<213>  synthetic construct

<400>  2507

ttgattgcac ctttctctg                                                    19

<210>  2508
<211>  19
<212>  DNA
<213>  synthetic construct

<400>  2508
ctaaatatgg cgcaggaac                                                    19

<210>  2509
<211>  20
<212>  DNA
<213>  synthetic construct

<400>  2509
atagtgccaa tcatccaaag                                                   20

<210>  2510
<211>  22
<212>  DNA
<213>  synthetic construct

<400>  2510
gttcataggc ttcacgtagt tc                                                22

<210>  2511
<211>  22
<212>  DNA
<213>  synthetic construct

<400>  2511
gtcaaatcca ttacttagca ca                                                22

<210>  2512
<211>  20
<212>  DNA
<213>  synthetic construct

<400>  2512
tggctgaaaa tgtcgttaat                                                   20

<210>  2513
<211>  18
<212>  DNA
<213>  synthetic construct

<400>  2513
aatgatttag ccgcttct                                                     18

<210>  2514
<211>  26
<212>  DNA
<213>  synthetic construct

<400>  2514

gaaaggactt aaacttaact atccag                                          26

<210>  2515
<211>  21
<212>  DNA
<213>  synthetic construct

<400>  2515
agggtcgtga atagaaacaa g                                               21

<210>  2516
<211>  21
<212>  DNA
<213>  synthetic construct

<400>  2516
actgacgctt atttgattga c                                               21

<210>  2517
<211>  20
<212>  DNA
<213>  synthetic construct

<400>  2517
gtacgtttca agagtgatgc                                                 20

<210>  2518
<211>  21
<212>  DNA
<213>  synthetic construct

<400>  2518
atggataata aaacgtatga a                                               21

<210>  2519
<211>  23
<212>  DNA
<213>  synthetic construct

<400>  2519
caattcttct atttcatctt gtg                                             23

<210>  2520
<211>  18
<212>  DNA
<213>  synthetic construct

<400>  2520
tagctcgtgc atcaaagg                                                   18

<210>  2521
<211>  21
<212>  DNA
<213>  synthetic construct

<400>  2521

aacctctgtc tccttctcaa c                                                21

<210>  2522
<211>  20
<212>  DNA
<213>  synthetic construct

<400>  2522
acactggctg aattaagtgc                                                   20

<210>  2523
<211>  22
<212>  DNA
<213>  synthetic construct

<400>  2523
aacccgcttt aatactgtta tc                                                22

<210>  2524
<211>  21
<212>  DNA
<213>  synthetic construct

<400>  2524
ttatctgcaa cactgatttc c                                                 21

<210>  2525
<211>  20
<212>  DNA
<213>  synthetic construct

<400>  2525
tttccggcac agtaataaag                                                   20

<210>  2526
<211>  25
<212>  DNA
<213>  synthetic construct

<400>  2526
gaaaattcac gtatgtcatg gaatc                                             25

<210>  2527
<211>  20
<212>  DNA
<213>  synthetic construct

<400>  2527
gcatttttcc cagatggctt                                                   20

<210>  2528
<211>  19
<212>  DNA
<213>  synthetic construct

<400>  2528

acgttctgac tggaggaag                                                    19

<210>  2529
<211>  20
<212>  DNA
<213>  synthetic construct

<400>  2529
ttcaatagag cgaaggattg                                                   20

<210>  2530
<211>  19
<212>  DNA
<213>  synthetic construct

<400>  2530
tcacgctgtt gttaggaag                                                    19

<210>  2531
<211>  19
<212>  DNA
<213>  synthetic construct

<400>  2531
aggctgggtg aagtaagtg                                                    19

<210>  2532
<211>  20
<212>  DNA
<213>  synthetic construct

<400>  2532
cggaactgta taatcccttg                                                   20

<210>  2533
<211>  18
<212>  DNA
<213>  synthetic construct

<400>  2533
agtggcaaca gcaggtag                                                     18

<210>  2534
<211>  20
<212>  DNA
<213>  synthetic construct

<400>  2534
cgtaggcatg atagaaatgg                                                   20

<210>  2535
<211>  20
<212>  DNA
<213>  synthetic construct

<400>  2535

gtatcccgca gataaatcac                                                    20

<210>  2536
<211>  19
<212>  DNA
<213>  synthetic construct

<400>  2536
cacgatagtt gtggcagac                                                     19

<210>  2537
<211>  18
<212>  DNA
<213>  synthetic construct

<400>  2537
actcaaccca tcctaccc                                                      18

<210>  2538
<211>  25
<212>  DNA
<213>  synthetic construct

<400>  2538
agcttttgtt ttatatttct attgg                                              25

<210>  2539
<211>  26
<212>  DNA
<213>  synthetic construct

<400>  2539
tttagtcttt tgtcaattac actttt                                             26

<210>  2540
<211>  18
<212>  DNA
<213>  synthetic construct

<400>  2540
ttgacgaagg cgtttatg                                                      18

<210>  2541
<211>  19
<212>  DNA
<213>  synthetic construct

<400>  2541
tgtgacttgg aacaaccag                                                     19

<210>  2542
<211>  25
<212>  DNA
<213>  synthetic construct

<400>  2542

tttaaagaat ggaaccaaga tcaaa 25

<210> 2543
<211> 23
<212> DNA
<213> synthetic construct

<400> 2543
tcgcctttta aatgtgtagc aaa 23

<210> 2544
<211> 19
<212> DNA
<213> synthetic construct

<400> 2544
gacaataccg cgatgaata 19

<210> 2545
<211> 19
<212> DNA
<213> synthetic construct

<400> 2545
ccggtggtat tgcctttta 19

<210> 2546
<211> 18
<212> DNA
<213> synthetic construct

<400> 2546
aatcatcctc gtggcttt 18

<210> 2547
<211> 20
<212> DNA
<213> synthetic construct

<400> 2547
gctaaattgg gatagtcctg 20

<210> 2548
<211> 20
<212> DNA
<213> synthetic construct

<400> 2548
tacagtcatt tcacgcaaac 20

<210> 2549
<211> 21
<212> DNA
<213> synthetic construct

<400> 2549

tcacgctctt catttagttc t                                                21

<210>  2550
<211>  20
<212>  DNA
<213>  synthetic construct

<400>  2550
agttgtagtt gtcgggtttg                                                  20

<210>  2551
<211>  20
<212>  DNA
<213>  synthetic construct

<400>  2551
tgaagtcgct tttcctagag                                                  20

<210>  2552
<211>  21
<212>  DNA
<213>  synthetic construct

<400>  2552
gccaataagc aagttgaaat a                                                21

<210>  2553
<211>  22
<212>  DNA
<213>  synthetic construct

<400>  2553
atttaaaatg tctcgcaatt ct                                               22

<210>  2554
<211>  19
<212>  DNA
<213>  synthetic construct

<400>  2554
catgcgtgta aatcatcgt                                                   19

<210>  2555
<211>  18
<212>  DNA
<213>  synthetic construct

<400>  2555
cccttgagcg gaagtatc                                                    18

<210>  2556
<211>  21
<212>  DNA
<213>  synthetic construct

<400>  2556

tgaggaaggt agtaagggaa a                                                21

<210>  2557
<211>  21
<212>  DNA
<213>  synthetic construct

<400>  2557
caaacatatt gggttgatag g                                                21

<210>  2558
<211>  21
<212>  DNA
<213>  synthetic construct

<400>  2558
tagcaataca atcgcacata c                                                21

<210>  2559
<211>  21
<212>  DNA
<213>  synthetic construct

<400>  2559
tggtatatct tcaccaacac c                                                21

<210>  2560
<211>  18
<212>  DNA
<213>  synthetic construct

<400>  2560
tgaagatggc agcaagag                                                    18

<210>  2561
<211>  20
<212>  DNA
<213>  synthetic construct

<400>  2561
tcaaaccctc attaacatcc                                                  20

<210>  2562
<211>  20
<212>  DNA
<213>  synthetic construct

<400>  2562
agagatgaat gcaggaacag                                                  20

<210>  2563
<211>  20
<212>  DNA
<213>  synthetic construct

<400>  2563

accaccttgg aaatgtaatg                                                20

<210>  2564
<211>  20
<212>  DNA
<213>  synthetic construct

<400>  2564
caaacaagaa ttagccgaag                                                20

<210>  2565
<211>  19
<212>  DNA
<213>  synthetic construct

<400>  2565
cagaagcaac tgtgattgg                                                 19

<210>  2566
<211>  20
<212>  DNA
<213>  synthetic construct

<400>  2566
tacagatgca gacggtgtag                                                20

<210>  2567
<211>  20
<212>  DNA
<213>  synthetic construct

<400>  2567
ccctgtccat atccagtatc                                                20

<210>  2568
<211>  19
<212>  DNA
<213>  synthetic construct

<400>  2568
gccggtgtat cactaaagg                                                 19

<210>  2569
<211>  20
<212>  DNA
<213>  synthetic construct

<400>  2569
ctgttctagt cccattgctc                                                20

<210>  2570
<211>  28
<212>  DNA
<213>  synthetic construct

<400>  2570

taataaaacg tatgaaatat catctgca                                          28

<210>  2571
<211>  28
<212>  DNA
<213>  synthetic construct

<400>  2571
tttcatcttg tgatagatct tctttttc                                          28

<210>  2572
<211>  21
<212>  DNA
<213>  synthetic construct

<400>  2572
cttggttagc gattcagtta g                                                 21

<210>  2573
<211>  20
<212>  DNA
<213>  synthetic construct

<400>  2573
caattcatcg tctggatagg                                                   20

<210>  2574
<211>  25
<212>  DNA
<213>  synthetic construct

<400>  2574
agcaagttga aatatctatg gctga                                             25

<210>  2575
<211>  25
<212>  DNA
<213>  synthetic construct

<400>  2575
tcatttaaaa tgtctcgcaa ttctt                                             25

<210>  2576
<211>  20
<212>  DNA
<213>  synthetic construct

<400>  2576
acatcgaact ggatctcaac                                                   20

<210>  2577
<211>  21
<212>  DNA
<213>  synthetic construct

<400>  2577

tctcagcgat ctgtctattt c                                                21

<210>  2578
<211>  20
<212>  DNA
<213>  synthetic construct

<400>  2578
tgtgcagtac cagtaaggtg                                                   20

<210>  2579
<211>  19
<212>  DNA
<213>  synthetic construct

<400>  2579
ctccggttgg gtaaagtag                                                    19

<210>  2580
<211>  20
<212>  DNA
<213>  synthetic construct

<400>  2580
acagtgccag ttcttatcgt                                                   20

<210>  2581
<211>  20
<212>  DNA
<213>  synthetic construct

<400>  2581
gaagccaacc tttatttcct                                                   20

<210>  2582
<211>  20
<212>  DNA
<213>  synthetic construct

<400>  2582
ctcacccaaa tggagattta                                                   20

<210>  2583
<211>  20
<212>  DNA
<213>  synthetic construct

<400>  2583
cttgcttttc agatgtttcc                                                   20

<210>  2584
<211>  18
<212>  DNA
<213>  synthetic construct

<400>  2584

acaacatttc gccaaaca                                                    18

<210>  2585
<211>  21
<212>  DNA
<213>  synthetic construct

<400>  2585
ttgagctaaa gacattcctg t                                                21

<210>  2586
<211>  21
<212>  DNA
<213>  synthetic construct

<400>  2586
aatgatggct ttgaagtagt g                                                21

<210>  2587
<211>  20
<212>  DNA
<213>  synthetic construct

<400>  2587
cctgttcttg cttctgtttc                                                  20

<210>  2588
<211>  18
<212>  DNA
<213>  synthetic construct

<400>  2588
ccatcaggca acagaatg                                                    18

<210>  2589
<211>  20
<212>  DNA
<213>  synthetic construct

<400>  2589
tgacgatctg gaacctttac                                                  20

<210>  2590
<211>  20
<212>  DNA
<213>  synthetic construct

<400>  2590
cttactatcg gagctggtca                                                  20

<210>  2591
<211>  18
<212>  DNA
<213>  synthetic construct

<400>  2591

agggcatctc cctgatac                                                    18

<210>  2592
<211>  20
<212>  DNA
<213>  synthetic construct

<400>  2592
tcactcatta accattgctg                                                  20

<210>  2593
<211>  20
<212>  DNA
<213>  synthetic construct

<400>  2593
gtatgtcact caaggtgctg                                                  20

<210>  2594
<211>  25
<212>  DNA
<213>  synthetic construct

<400>  2594
ttatctgttt tgttactgct tacac                                            25

<210>  2595
<211>  20
<212>  DNA
<213>  synthetic construct

<400>  2595
ttctaatcct cccaaagtcc                                                  20

<210>  2596
<211>  20
<212>  DNA
<213>  synthetic construct

<400>  2596
tgacttcgga tgagttcaat                                                  20

<210>  2597
<211>  21
<212>  DNA
<213>  synthetic construct

<400>  2597
gctgttaatt gttgttgctt t                                                21

<210>  2598
<211>  24
<212>  DNA
<213>  synthetic construct

<400>  2598

aagtataatc agttcattgc tcac 24

<210> 2599
<211> 20
<212> DNA
<213> synthetic construct

<400> 2599
gtttcgacta cgacagttgg 20

<210> 2600
<211> 22
<212> DNA
<213> synthetic construct

<400> 2600
tgctttagtt ttaagtgcat gt 22

<210> 2601
<211> 20
<212> DNA
<213> synthetic construct

<400> 2601
tccttcatta cactcttggc 20

<210> 2602
<211> 20
<212> DNA
<213> synthetic construct

<400> 2602
tttgaaggaa ctgaaggttg 20

<210> 2603
<211> 21
<212> DNA
<213> synthetic construct

<400> 2603
ttctatttgc tgtgaatcct g 21

<210> 2604
<211> 18
<212> DNA
<213> synthetic construct

<400> 2604
ggcttacggg atcaagac 18

<210> 2605
<211> 18
<212> DNA
<213> synthetic construct

<400> 2605

attggcctgg aagctcac                                                    18

<210>  2606
<211>  19
<212>  DNA
<213>  synthetic construct

<400>  2606
gcggtaagat ccttgagag                                                   19

<210>  2607
<211>  20
<212>  DNA
<213>  synthetic construct

<400>  2607
agtggtcctg caactttatc                                                  20

<210>  2608
<211>  27
<212>  DNA
<213>  synthetic construct

<400>  2608
agttgaaaat gaaatatgta taagaac                                          27

<210>  2609
<211>  21
<212>  DNA
<213>  synthetic construct

<400>  2609
aactgcatta gcatttcttt c                                                21

<210>  2610
<211>  18
<212>  DNA
<213>  synthetic construct

<400>  2610
tcattcgcac atgtaggc                                                    18

<210>  2611
<211>  18
<212>  DNA
<213>  synthetic construct

<400>  2611
gggatcgtca ccgtaatc                                                    18

<210>  2612
<211>  18
<212>  DNA
<213>  synthetic construct

<400>  2612

gaccgatcac cctacgag                                                    18

<210>  2613
<211>  21
<212>  DNA
<213>  synthetic construct

<400>  2613
cgaaatgctt ctcaagatag g                                               21

<210>  2614
<211>  18
<212>  DNA
<213>  synthetic construct

<400>  2614
aagtttcatt gccagacg                                                    18

<210>  2615
<211>  18
<212>  DNA
<213>  synthetic construct

<400>  2615
tagactgcgt tgctcctc                                                    18

<210>  2616
<211>  19
<212>  DNA
<213>  synthetic construct

<400>  2616
gccgaagtat cgactcaac                                                   19

<210>  2617
<211>  20
<212>  DNA
<213>  synthetic construct

<400>  2617
agcactacat ttcgctcatc                                                  20

<210>  2618
<211>  20
<212>  DNA
<213>  synthetic construct

<400>  2618
gcaggtcaca ttgatacaca                                                  20

<210>  2619
<211>  18
<212>  DNA
<213>  synthetic construct

<400>  2619

catatcgcga cctgaaag                                                    18


<210>  2620
<211>  20
<212>  DNA
<213>  synthetic construct

<400>  2620
gctattggtg tttatggctc                                                  20


<210>  2621
<211>  20
<212>  DNA
<213>  synthetic construct

<400>  2621
ctgattgctt aactgcttca                                                  20


<210>  2622
<211>  22
<212>  DNA
<213>  synthetic construct

<400>  2622
gctaaatcga tctcatatcg tc                                               22


<210>  2623
<211>  19
<212>  DNA
<213>  synthetic construct

<400>  2623
ggcgtgtttg aaccatgta                                                   19


<210>  2624
<211>  19
<212>  DNA
<213>  synthetic construct

<400>  2624
ctcatttggc tcaaaggtc                                                   19


<210>  2625
<211>  18
<212>  DNA
<213>  synthetic construct

<400>  2625
tgaataggac agcgaagg                                                    18


<210>  2626
<211>  20
<212>  DNA
<213>  synthetic construct

<400>  2626

gagaatatca ccggaattga                                                    20

<210>  2627
<211>  20
<212>  DNA
<213>  synthetic construct

<400>  2627
gctcgacata ctgttcttcc                                                    20

<210>  2628
<211>  18
<212>  DNA
<213>  synthetic construct

<400>  2628
cctgaccaag tccaatcc                                                      18

<210>  2629
<211>  20
<212>  DNA
<213>  synthetic construct

<400>  2629
tagatcacgt aagcaccaag                                                    20

<210>  2630
<211>  18
<212>  DNA
<213>  synthetic construct

<400>  2630
tgcgatgctc tatgagtg                                                      18

<210>  2631
<211>  19
<212>  DNA
<213>  synthetic construct

<400>  2631
gtaccttgcc tctcaaacc                                                     19

<210>  2632
<211>  20
<212>  DNA
<213>  synthetic construct

<400>  2632
agatttgcca gaacatgaat                                                    20

<210>  2633
<211>  20
<212>  DNA
<213>  synthetic construct

<400>  2633

tgttgcattt agtctttcca                                                    20

<210>  2634
<211>  25
<212>  DNA
<213>  synthetic construct

<400>  2634
atatcaggaa agattggaaa tacgg                                              25

<210>  2635
<211>  23
<212>  DNA
<213>  synthetic construct

<400>  2635
aaagaggtat agcccattct gca                                                23

<210>  2636
<211>  21
<212>  DNA
<213>  synthetic construct

<400>  2636
aagatacgga aggaatgtct c                                                  21

<210>  2637
<211>  20
<212>  DNA
<213>  synthetic construct

<400>  2637
acaaagatgt tgctgtctcc                                                    20

<210>  2638
<211>  25
<212>  DNA
<213>  synthetic construct

<400>  2638
acacagtcaa aactttatta cttca                                              25

<210>  2639
<211>  24
<212>  DNA
<213>  synthetic construct

<400>  2639
caacaagttt attttctgta gttt                                               24

<210>  2640
<211>  20
<212>  DNA
<213>  synthetic construct

<400>  2640

cgtttaccaa aggagaaggt                                                    20

<210>  2641
<211>  20
<212>  DNA
<213>  synthetic construct

<400>  2641
tgtttagcca attatcagca                                                    20

<210>  2642
<211>  22
<212>  DNA
<213>  synthetic construct

<400>  2642
gtgattacag aaatgaaagc ag                                                 22

<210>  2643
<211>  27
<212>  DNA
<213>  synthetic construct

<400>  2643
agatatatta ccttatcagt attgtca                                            27

<210>  2644
<211>  20
<212>  DNA
<213>  synthetic construct

<400>  2644
gacagatttt cgatccctta                                                    20

<210>  2645
<211>  20
<212>  DNA
<213>  synthetic construct

<400>  2645
cctttttgtt ttgatgcact                                                    20

<210>  2646
<211>  30
<212>  DNA
<213>  synthetic construct

<400>  2646
gatataggat acaaaataga agttgattgg                                         30

<210>  2647
<211>  26
<212>  DNA
<213>  synthetic construct

<400>  2647

ggtctttttc tgttaattca taaccg                                          26


<210>  2648
<211>  19
<212>  DNA
<213>  synthetic construct

<400>  2648
catttaacga cgaaactgg                                                  19


<210>  2649
<211>  20
<212>  DNA
<213>  synthetic construct

<400>  2649
aattgtttac tttggcgtgt                                                 20


<210>  2650
<211>  20
<212>  DNA
<213>  synthetic construct

<400>  2650
ccagaaaaac cctaaagaca                                                 20


<210>  2651
<211>  20
<212>  DNA
<213>  synthetic construct

<400>  2651
aaagaacacg atattcacgg                                                 20


<210>  2652
<211>  20
<212>  DNA
<213>  synthetic construct

<400>  2652
gtccgaatcc tatgaaaatg                                                 20


<210>  2653
<211>  22
<212>  DNA
<213>  synthetic construct

<400>  2653
gcagctgatt tattgtatct tg                                              22


<210>  2654
<211>  20
<212>  DNA
<213>  synthetic construct

<400>  2654

cattagcagg aggatgtttc                                                    20

<210>  2655
<211>  19
<212>  DNA
<213>  synthetic construct

<400>  2655
tgaaccacgt tgatattgg                                                     19

<210>  2656
<211>  26
<212>  DNA
<213>  synthetic construct

<400>  2656
gcatataaat atcaaaacca tacaag                                             26

<210>  2657
<211>  18
<212>  DNA
<213>  synthetic construct

<400>  2657
tttcaatgcc atcgtttc                                                      18

<210>  2658
<211>  20
<212>  DNA
<213>  synthetic construct

<400>  2658
ggtcttgtct atggcttcac                                                    20

<210>  2659
<211>  20
<212>  DNA
<213>  synthetic construct

<400>  2659
cattccttct tgcatttctc                                                    20

<210>  2660
<211>  20
<212>  DNA
<213>  synthetic construct

<400>  2660
gttgagaatg ggagagactg                                                    20

<210>  2661
<211>  18
<212>  DNA
<213>  synthetic construct

<400>  2661

aacgtcgcga ttctaggt                                                    18

<210>  2662
<211>  24
<212>  DNA
<213>  synthetic construct

<400>  2662
actaagaaaa tcgtagctat ttgg                                              24

<210>  2663
<211>  20
<212>  DNA
<213>  synthetic construct

<400>  2663
acttcctttc tcttgcgata                                                   20

<210>  2664
<211>  20
<212>  DNA
<213>  synthetic construct

<400>  2664
caattacctt ggcacttacc                                                   20

<210>  2665
<211>  20
<212>  DNA
<213>  synthetic construct

<400>  2665
   cccttttcta cgcactaaat 20

<210>  2666
<211>  19
<212>  DNA
<213>  synthetic construct

<400>  2666
tggaatgggt agcttcttc                                                    19

<210>  2667
<211>  19
<212>  DNA
<213>  synthetic construct

<400>  2667
gggaaacaca agacagacc                                                    19

<210>  2668
<211>  22
<212>  DNA
<213>  synthetic construct

<400>  2668

gataaccatc acaaacagaa tg                                                22

<210>  2669
<211>  21
<212>  DNA
<213>  synthetic construct

<400>  2669
ttatttgaac caacaaacga c                                                 21

<210>  2670
<211>  22
<212>  DNA
<213>  synthetic construct

<400>  2670
agaaaattgg gatagaaaag aa                                                22

<210>  2671
<211>  18
<212>  DNA
<213>  synthetic construct

<400>  2671
ctgcaaggca actggtat                                                     18

<210>  2672
<211>  19
<212>  DNA
<213>  synthetic construct

<400>  2672
ttagcactct atgcgacga                                                    19

<210>  2673
<211>  21
<212>  DNA
<213>  synthetic construct

<400>  2673
aatacaccaa gaaatgcaag a                                                 21

<210>  2674
<211>  19
<212>  DNA
<213>  synthetic construct

<400>  2674
cacctgcgag tacaaactg                                                    19

<210>  2675
<211>  19
<212>  DNA
<213>  synthetic construct

<400>  2675

cccacaaaga actccaatc                                                    19

<210>  2676
<211>  20
<212>  DNA
<213>  synthetic construct

<400>  2676
gacaaaggta caacgaggac                                                   20

<210>  2677
<211>  20
<212>  DNA
<213>  synthetic construct

<400>  2677
gcacttccat gataaagagg                                                   20

<210>  2678
<211>  19
<212>  DNA
<213>  synthetic construct

<400>  2678
tcttatggca gtacgcaac                                                    19

<210>  2679
<211>  20
<212>  DNA
<213>  synthetic construct

<400>  2679
ccagtttccc gttttctaat                                                   20

<210>  2680
<211>  18
<212>  DNA
<213>  synthetic construct

<400>  2680
gtgcggtatt gggaaaca                                                     18

<210>  2681
<211>  18
<212>  DNA
<213>  synthetic construct

<400>  2681
cggccgttat cttgtaaa                                                     18

<210>  2682
<211>  20
<212>  DNA
<213>  synthetic construct

<400>  2682

aagtgtgggc attactgttt                                                    20

<210>  2683
<211>  18
<212>  DNA
<213>  synthetic construct

<400>  2683
ccttgcttca tctctcca                                                      18

<210>  2684
<211>  21
<212>  DNA
<213>  synthetic construct

<400>  2684
acgagaatta tacggttttc a                                                  21

<210>  2685
<211>  20
<212>  DNA
<213>  synthetic construct

<400>  2685
atcaatggtg tcgttcattt                                                    20

<210>  2686
<211>  21
<212>  DNA
<213>  synthetic construct

<400>  2686
gaattctact tgtcgaggat g                                                  21

<210>  2687
<211>  18
<212>  DNA
<213>  synthetic construct

<400>  2687
tctgcctctg catcaaac                                                      18

<210>  2688
<211>  18
<212>  DNA
<213>  synthetic construct

<400>  2688
aatgatccga gggaaact                                                      18

<210>  2689
<211>  21
<212>  DNA
<213>  synthetic construct

<400>  2689

tttgttagcg ttatcgtttg t                                                21

<210>  2690
<211>  20
<212>  DNA
<213>  synthetic construct

<400>  2690
acatgagttg gaggaaacac                                                   20

<210>  2691
<211>  20
<212>  DNA
<213>  synthetic construct

<400>  2691
tacgatggca agtcctaaac                                                   20

<210>  2692
<211>  19
<212>  DNA
<213>  synthetic construct

<400>  2692
ggcagcaaag accttaaac                                                    19

<210>  2693
<211>  18
<212>  DNA
<213>  synthetic construct

<400>  2693
tcattggttc tcctcctg                                                     18

<210>  2694
<211>  20
<212>  DNA
<213>  synthetic construct

<400>  2694
gaagatggaa caattcaagg                                                   20

<210>  2695
<211>  21
<212>  DNA
<213>  synthetic construct

<400>  2695
tttcccacag taaaggatac a                                                 21

<210>  2696
<211>  19
<212>  DNA
<213>  synthetic construct

<400>  2696

tacagtctat ccgggcatt                                                    19

<210>  2697
<211>  20
<212>  DNA
<213>  synthetic construct

<400>  2697
tgttgtcggg atcaattact                                                   20

<210>  2698
<211>  20
<212>  DNA
<213>  synthetic construct

<400>  2698
tgggataact tcacaggaaa                                                   20

<210>  2699
<211>  19
<212>  DNA
<213>  synthetic construct

<400>  2699
ggtatggctc atcaatcaa                                                    19

<210>  2700
<211>  27
<212>  DNA
<213>  synthetic construct

<400>  2700
atacttaggt tatgactacg ttaatga                                           27

<210>  2701
<211>  19
<212>  DNA
<213>  synthetic construct

<400>  2701
aaatgaaccc gtatttcca                                                    19

<210>  2702
<211>  20
<212>  DNA
<213>  synthetic construct

<400>  2702
gtgcgttcat tatttcgttc                                                   20

<210>  2703
<211>  20
<212>  DNA
<213>  synthetic construct

<400>  2703

gccatctata aacctcgttg                                                    20

<210>  2704
<211>  20
<212>  DNA
<213>  synthetic construct

<400>  2704
ttgtctggta tcccctatgt                                                    20

<210>  2705
<211>  20
<212>  DNA
<213>  synthetic construct

<400>  2705
aaaagatcaa cacgagcaag                                                    20

<210>  2706
<211>  25
<212>  DNA
<213>  synthetic construct

<400>  2706
aggattgcta gctttatatt tagtg                                              25

<210>  2707
<211>  22
<212>  DNA
<213>  synthetic construct

<400>  2707
atttattctt aaatgggtac gg                                                 22

<210>  2708
<211>  20
<212>  DNA
<213>  synthetic construct

<400>  2708
aagctgcctt atgtaggttg                                                    20

<210>  2709
<211>  18
<212>  DNA
<213>  synthetic construct

<400>  2709
agcaccgaca gtcaaaga                                                      18

<210>  2710
<211>  19
<212>  DNA
<213>  synthetic construct

<400>  2710

gacatacgag ttggctgaa                                                    19

<210>  2711
<211>  19
<212>  DNA
<213>  synthetic construct

<400>  2711
ctattggggt atggttcgt                                                    19

<210>  2712
<211>  20
<212>  DNA
<213>  synthetic construct

<400>  2712
cctttgaagc tggtactgac                                                   20

<210>  2713
<211>  19
<212>  DNA
<213>  synthetic construct

<400>  2713
ctgtgaaccg tcttgattg                                                    19

<210>  2714
<211>  22
<212>  DNA
<213>  synthetic construct

<400>  2714
tgtcgaagtt tttcattgat ag                                                22

<210>  2715
<211>  18
<212>  DNA
<213>  synthetic construct

<400>  2715
aaggtcacgg tgatggtc                                                     18

<210>  2716
<211>  23
<212>  DNA
<213>  synthetic construct

<400>  2716
caatggttac aggttgtgga aga                                               23

<210>  2717
<211>  24
<212>  DNA
<213>  synthetic construct

<400>  2717

gcccactaca gattcttcag ctac                                              24


<210>  2718
<211>  18
<212>  DNA
<213>  synthetic construct

<400>  2718
ctcttagacg ccctgtcc                                                    18


<210>  2719
<211>  19
<212>  DNA
<213>  synthetic construct

<400>  2719
gtttccgaga aggtgattg                                                   19


<210>  2720
<211>  19
<212>  DNA
<213>  synthetic construct

<400>  2720
catcgtcaac ataacctcg                                                   19


<210>  2721
<211>  19
<212>  DNA
<213>  synthetic construct

<400>  2721
aattcttgcg gtttctttc                                                   19


<210>  2722
<211>  26
<212>  DNA
<213>  synthetic construct

<400>  2722
gaggtgtaat tatgattcag actatt                                           26


<210>  2723
<211>  24
<212>  DNA
<213>  synthetic construct

<400>  2723
tcgtaccaac taatttagac aatc                                             24


<210>  2724
<211>  18
<212>  DNA
<213>  synthetic construct

<400>  2724

ctcgacccga tctacgtc                                                    18


<210>  2725
<211>  18
<212>  DNA
<213>  synthetic construct

<400>  2725
gtcttcacct cgacaccc                                                    18


<210>  2726
<211>  25
<212>  DNA
<213>  synthetic construct

<400>  2726
tgtcatcata gctcttaaca taatc                                            25


<210>  2727
<211>  18
<212>  DNA
<213>  synthetic construct

<400>  2727
ctaaacgcca agcacaag                                                    18


<210>  2728
<211>  18
<212>  DNA
<213>  synthetic construct

<400>  2728
gaccaggagt tggtggtt                                                    18


<210>  2729
<211>  19
<212>  DNA
<213>  synthetic construct

<400>  2729
aacagctgac aaatcaacg                                                   19


<210>  2730
<211>  20
<212>  DNA
<213>  synthetic construct

<400>  2730
ctggctctgt tagtgctttc                                                  20


<210>  2731
<211>  21
<212>  DNA
<213>  synthetic construct

<400>  2731

ctgttaccgt tgatttacct g                                                21

<210>  2732
<211>  20
<212>  DNA
<213>  synthetic construct

<400>  2732
cctgaaatcc actactgacc                                                  20

<210>  2733
<211>  18
<212>  DNA
<213>  synthetic construct

<400>  2733
ccaacgacag ttccactc                                                    18

<210>  2734
<211>  20
<212>  DNA
<213>  synthetic construct

<400>  2734
gaaatgatat tgacgggact                                                  20

<210>  2735
<211>  19
<212>  DNA
<213>  synthetic construct

<400>  2735
tcaacacaag gacaggttg                                                   19

<210>  2736
<211>  18
<212>  DNA
<213>  synthetic construct

<400>  2736
aaggctggct ttttcttg                                                    18

<210>  2737
<211>  21
<212>  DNA
<213>  synthetic construct

<400>  2737
aattatgagc cccataccta c                                                21

<210>  2738
<211>  20
<212>  DNA
<213>  synthetic construct

<400>  2738

attagaaatt gcgactggtg                                                    20

<210>  2739
<211>  20
<212>  DNA
<213>  synthetic construct

<400>  2739
agcgtgtcat atccttcatc                                                    20

<210>  2740
<211>  27
<212>  DNA
<213>  synthetic construct

<400>  2740
ttggatagtt caacaaaaac attaaca                                            27

<210>  2741
<211>  27
<212>  DNA
<213>  synthetic construct

<400>  2741
catttttatc ttctgttacc actggtt                                            27

<210>  2742
<211>  18
<212>  DNA
<213>  synthetic construct

<400>  2742
tgagcagcgt aaccagac                                                      18

<210>  2743
<211>  19
<212>  DNA
<213>  synthetic construct

<400>  2743
agtcatccac gttcctttc                                                     19

<210>  2744
<211>  20
<212>  DNA
<213>  synthetic construct

<400>  2744
atgaaaaacc ctcatcaaaa                                                    20

<210>  2745
<211>  20
<212>  DNA
<213>  synthetic construct

<400>  2745

gggaaaaaga ccaaaggtaa                                                20

<210>  2746
<211>  20
<212>  DNA
<213>  synthetic construct

<400>  2746
agaacatatc cgcaaacaag                                                20

<210>  2747
<211>  21
<212>  DNA
<213>  synthetic construct

<400>  2747
tgatgtaaac aaaatcgttc c                                              21

<210>  2748
<211>  21
<212>  DNA
<213>  synthetic construct

<400>  2748
aaccgtttat acgttggtga g                                              21

<210>  2749
<211>  18
<212>  DNA
<213>  synthetic construct

<400>  2749
gattaacgcc tgcatcac                                                  18

<210>  2750
<211>  18
<212>  DNA
<213>  synthetic construct

<400>  2750
atgttctcgc tgcagttc                                                  18

<210>  2751
<211>  23
<212>  DNA
<213>  synthetic construct

<400>  2751
accaaaactt tattttcatc atc                                            23

<210>  2752
<211>  22
<212>  DNA
<213>  synthetic construct

<400>  2752

ataaagcaat gaagcctaaa gt                                                22

<210>  2753
<211>  20
<212>  DNA
<213>  synthetic construct

<400>  2753
tttacgatca aggaaccatc                                                    20

<210>  2754
<211>  20
<212>  DNA
<213>  synthetic construct

<400>  2754
ggttacttgt tgctgctttt                                                    20

<210>  2755
<211>  19
<212>  DNA
<213>  synthetic construct

<400>  2755
cgtaatcgca atcgaaata                                                     19

<210>  2756
<211>  18
<212>  DNA
<213>  synthetic construct

<400>  2756
gaactacccc gtgaatcc                                                      18

<210>  2757
<211>  18
<212>  DNA
<213>  synthetic construct

<400>  2757
cactggtcga ggagatgc                                                      18

<210>  2758
<211>  18
<212>  DNA
<213>  synthetic construct

<400>  2758
gcaaggaccg ttctatca                                                      18

<210>  2759
<211>  20
<212>  DNA
<213>  synthetic construct

<400>  2759

cttcaatgac gtgtaaacca                                                    20

<210>  2760
<211>  20
<212>  DNA
<213>  synthetic construct

<400>  2760
tacgatgaca ccagtctttg                                                    20

<210>  2761
<211>  20
<212>  DNA
<213>  synthetic construct

<400>  2761
atcgacaaaa cgtacaggat                                                    20

<210>  2762
<211>  20
<212>  DNA
<213>  synthetic construct

<400>  2762
tagcaacctc cctttgatac                                                    20

<210>  2763
<211>  24
<212>  DNA
<213>  synthetic construct

<400>  2763
acaaaagata tgtgtgaagt tacc                                               24

<210>  2764
<211>  18
<212>  DNA
<213>  synthetic construct

<400>  2764
gcgtagaacg tggacttg                                                      18

<210>  2765
<211>  20
<212>  DNA
<213>  synthetic construct

<400>  2765
tcataaatgg accgaatacc                                                    20

<210>  2766
<211>  18
<212>  DNA
<213>  synthetic construct

<400>  2766

atggcttgtt ctgctgct 18

<210> 2767
<211> 26
<212> DNA
<213> synthetic construct

<400> 2767
ttatttcaag ttagtgaaat cagttt 26

<210> 2768
<211> 20
<212> DNA
<213> synthetic construct

<400> 2768
ctaagatgtc gtcgcaagat 20

<210> 2769
<211> 22
<212> DNA
<213> synthetic construct

<400> 2769
tttcttcatg ttcatatgga tt 22

<210> 2770
<211> 25
<212> DNA
<213> synthetic construct

<400> 2770
gctgaattat ctaaattacc aagag 25

<210> 2771
<211> 19
<212> DNA
<213> synthetic construct

<400> 2771
tgcagtaaca agcatccac 19

<210> 2772
<211> 22
<212> DNA
<213> synthetic construct

<400> 2772
ggtggtaaat taagagattt gg 22

<210> 2773
<211> 20
<212> DNA
<213> synthetic construct

<400> 2773

aggatttctg gtttgaaagg                                                    20

<210>  2774
<211>  20
<212>  DNA
<213>  synthetic construct

<400>  2774
tcatggatta tttgttcgtg                                                    20

<210>  2775
<211>  21
<212>  DNA
<213>  synthetic construct

<400>  2775
ccatatcttt gagcaactgt g                                                  21

<210>  2776
<211>  19
<212>  DNA
<213>  synthetic construct

<400>  2776
aaacggtcca gagttgaag                                                     19

<210>  2777
<211>  20
<212>  DNA
<213>  synthetic construct

<400>  2777
ctctcaaaga accagacacc                                                    20

<210>  2778
<211>  21
<212>  DNA
<213>  synthetic construct

<400>  2778
tgttattggt tatggtgttg g                                                  21

<210>  2779
<211>  20
<212>  DNA
<213>  synthetic construct

<400>  2779
cggtgatttc taatggtctc                                                    20

<210>  2780
<211>  19
<212>  DNA
<213>  synthetic construct

<400>  2780

attccttggt ttggttctg 19

<210> 2781
<211> 22
<212> DNA
<213> synthetic construct

<400> 2781
gtctccaata atgaggtaaa gg 22

<210> 2782
<211> 20
<212> DNA
<213> synthetic construct

<400> 2782
gctctttgct tcaattatcc 20

<210> 2783
<211> 20
<212> DNA
<213> synthetic construct

<400> 2783
ataaaccacc caagaacaac 20

<210> 2784
<211> 25
<212> DNA
<213> synthetic construct

<400> 2784
atccaagttg aaagagataa attga 25

<210> 2785
<211> 20
<212> DNA
<213> synthetic construct

<400> 2785
ttccccttca agtttaccta 20

<210> 2786
<211> 16
<212> DNA
<213> synthetic construct

<400> 2786
agaggcccgt ttgaag 16

<210> 2787
<211> 16
<212> DNA
<213> synthetic construct

<400> 2787

gctcaagggg caaatg                                                    16

<210>  2788
<211>  17
<212>  DNA
<213>  synthetic construct

<400>  2788
tcggagtcaa cggattt                                                   17

<210>  2789
<211>  16
<212>  DNA
<213>  synthetic construct

<400>  2789
ccacgacgta ctcagc                                                    16

<210>  2790
<211>  20
<212>  DNA
<213>  synthetic construct

<400>  2790
atggatgatg atatcgccgc                                                20

<210>  2791
<211>  19
<212>  DNA
<213>  synthetic construct

<400>  2791
gtgtggtgcc agattttct                                                 19

<210>  2792
<211>  18
<212>  DNA
<213>  synthetic construct

<400>  2792
gccaaattgc caaaactc                                                  18

<210>  2793
<211>  16
<212>  DNA
<213>  synthetic construct

<400>  2793
cacaacacga agaccg                                                    16

<210>  2794
<211>  18
<212>  DNA
<213>  synthetic construct

<400>  2794

caccctggat ttgcatac                                                    18

<210>  2795
<211>  17
<212>  DNA
<213>  synthetic construct

<400>  2795
acatctgatc cgttcct                                                     17

<210>  2796
<211>  25
<212>  DNA
<213>  synthetic construct

<400>  2796
atccaagttg aaagagataa attga                                            25

<210>  2797
<211>  20
<212>  DNA
<213>  synthetic construct

<400>  2797
ttccccttca agtttaccta                                                  20

<210>  2798
<211>  18
<212>  DNA
<213>  synthetic construct

<400>  2798
caggcctaac acatgcaa                                                    18

<210>  2799
<211>  19
<212>  DNA
<213>  synthetic construct

<400>  2799
gaccgtgtct cagttccag                                                   19

<210>  2800
<211>  20
<212>  DNA
<213>  synthetic construct

<400>  2800
aggataggta ggagccgtag                                                  20

<210>  2801
<211>  20
<212>  DNA
<213>  synthetic construct

<400>  2801

agatgctttc agcgtttatc                                                    20

<210>  2802
<211>  19
<212>  DNA
<213>  synthetic construct

<400>  2802
gcgtgagtga aagaaggtt                                                     19

<210>  2803
<211>  20
<212>  DNA
<213>  synthetic construct

<400>  2803
cagtttccaa agcgtacatt                                                    20

<210>  2804
<211>  18
<212>  DNA
<213>  synthetic construct

<400>  2804
ccacactggg actgagac                                                      18

<210>  2805
<211>  19
<212>  DNA
<213>  synthetic construct

<400>  2805
gctcgggacc tacgtatta                                                     19

<210>  2806
<211>  18
<212>  DNA
<213>  synthetic construct

<400>  2806
cacggcccag actcctac                                                      18

<210>  2807
<211>  19
<212>  DNA
<213>  synthetic construct

<400>  2807
cccaataaat ccggacaac                                                     19

<210>  2808
<211>  21
<212>  DNA
<213>  synthetic construct

<400>  2808

gtgcattagc tagttggtga g                                                21

<210>  2809
<211>  19
<212>  DNA
<213>  synthetic construct

<400>  2809
ataccgtcaa gggatgaac                                                   19

<210>  2810
<211>  19
<212>  DNA
<213>  synthetic construct

<400>  2810
tgcctataca tgcaagtcg                                                   19

<210>  2811
<211>  18
<212>  DNA
<213>  synthetic construct

<400>  2811
ctatgcatcg tggccttg                                                    18

<210>  2812
<211>  18
<212>  DNA
<213>  synthetic construct

<400>  2812
ggcgtgccta atacatgc                                                    18

<210>  2813
<211>  18
<212>  DNA
<213>  synthetic construct

<400>  2813
ctatgcatcg tcgccttg                                                    18

<210>  2814
<211>  18
<212>  DNA
<213>  synthetic construct

<400>  2814
caggcctaac acatgcaa                                                    18

<210>  2815
<211>  18
<212>  DNA
<213>  synthetic construct

<400>  2815

atcccatctg ggcacatc                                                    18

<210>  2816
<211>  19
<212>  DNA
<213>  synthetic construct

<400>  2816
acacggtcca gactcctac                                                   19

<210>  2817
<211>  20
<212>  DNA
<213>  synthetic construct

<400>  2817
aattaaacca catgctccac                                                  20

<210>  2818
<211>  20
<212>  DNA
<213>  synthetic construct

<400>  2818
gcattagcta gttggtgagg                                                  20

<210>  2819
<211>  18
<212>  DNA
<213>  synthetic construct

<400>  2819
gtcaggggac gttcagtt                                                    18

<210>  2820
<211>  18
<212>  DNA
<213>  synthetic construct

<400>  2820
cgtgcctaat acatgcaa                                                    18

<210>  2821
<211>  18
<212>  DNA
<213>  synthetic construct

<400>  2821
cgatcaccct ctcaggtc                                                    18

<210>  2822
<211>  23
<212>  DNA
<213>  synthetic construct

<400>  2822

acacgtgggt aacctaccta taa                                              23


<210>  2823
<211>  18
<212>  DNA
<213>  synthetic construct

<400>  2823
ccgatcaccc tctcaggt                                                    18


<210>  2824
<211>  22
<212>  DNA
<213>  synthetic construct

<400>  2824
cctttaggtg tattggtagg ag                                               22


<210>  2825
<211>  19
<212>  DNA
<213>  synthetic construct

<400>  2825
atcgtctgca ttccttagc                                                   19


<210>  2826
<211>  18
<212>  DNA
<213>  synthetic construct

<400>  2826
caggcctaac acatgcaa                                                    18


<210>  2827
<211>  18
<212>  DNA
<213>  synthetic construct

<400>  2827
agtcccagtg tggctgat                                                    18


<210>  2828
<211>  18
<212>  DNA
<213>  synthetic construct

<400>  2828
tggttgatca tggctcag                                                    18


<210>  2829
<211>  18
<212>  DNA
<213>  synthetic construct

<400>  2829

aagagcccct gctttggt                                                    18


<210>  2830
<211>  19
<212>  DNA
<213>  synthetic construct

<400>  2830
tctctgatgt tagcggcgg                                                   19


<210>  2831
<211>  17
<212>  DNA
<213>  synthetic construct

<400>  2831
tcaggctttc gcccatt                                                     17


<210>  2832
<211>  20
<212>  DNA
<213>  synthetic construct

<400>  2832
ctaacacatg caagtcgaac                                                  20


<210>  2833
<211>  18
<212>  DNA
<213>  synthetic construct

<400>  2833
cacatccgat ggcaagag                                                    18


<210>  2834
<211>  17
<212>  DNA
<213>  synthetic construct

<400>  2834
ataggtcggc ggttcat                                                     17


<210>  2835
<211>  20
<212>  DNA
<213>  synthetic construct

<400>  2835
cccgagtatc tggaagacag                                                  20


<210>  2836
<211>  22
<212>  DNA
<213>  synthetic construct

<400>  2836

ctaaaattgg tgaaggtgca gc                                                22

<210>  2837
<211>  22
<212>  DNA
<213>  synthetic construct

<400>  2837
atatggtggt tcagattctg cc                                                22

<210>  2838
<211>  21
<212>  DNA
<213>  synthetic construct

<400>  2838
ccttagttat ctcggtgcca g                                                 21

<210>  2839
<211>  22
<212>  DNA
<213>  synthetic construct

<400>  2839
ggaagccaca ctgctacaca gg                                                22

<210>  2840
<211>  22
<212>  DNA
<213>  synthetic construct

<400>  2840
ccaccggttt aacttggaat cc                                                22

<210>  2841
<211>  22
<212>  DNA
<213>  synthetic construct

<400>  2841
aattgatggt acacgaccag tg                                                22

## Description

The present invention provides a DNA microarray for identification and characterisation of microorganisms in a sample or clinical specimen. Furthermore, it provides for a method for rapid identification and strain profiling of different microbial species in clinical specimens, especially in blood cultures, utilizing said DNA microarray.

### Background

Isolation, identification and characterisation of bacteria from clinical specimens is a main task of microbiological routine diagnostics. In fact, microorganisms are ubiquitous in certain areas of the human body. For this reason isolation and identification of pathogenic bacteria from clinical material and discrimination of specific pathogens from contaminations with indigenous or environmentally encountered microorganisms is a requirement for the correct diagnosis of infectious diseases. Additionally, accurate identification of antibiotic resistance and particular virulence factors provide important information enabling the clinician to choose effective antimicrobial therapy.

In the course of infection, many specimen types can be used for direct identification of the pathogens. These include, but are not limited to, liquor in the course of bacterial meningitis, sputum from patients with bacterial pneumonia, urine in the course of upper and lower urinary tract infections, punktate from sites of deep purulent infections (such as abscess, phlegmone, lung emphysema and septic arthritis), stool from patients with gastrointestinal tract infections, pus or wound fluid from purulent infections of the skin and wounds. Sometimes, bacteria are represented in the specimen only in minor numbers, thus, indirect identification of pathogens after culture of specimens in liquid media is employed. Important examples are enrichment cultures of food samples during outbreaks of food borne infections and blood cultures for diagnosis of bloodstream infections.

The invasion of the bloodstream by microorganisms, especially bacteremia and fungemia, represents one of the most serious consequences of infections and is a high ranked cause of death (Mylotte, J.M. and Tayara, A., Eur. Clin. Microbiol. Infect. Dis. 19:157-163 (2000); Reimer, L.G. et al., Clin. Microbiol. Rev. 10:444-465 (1997)). Bacteremia is the means by which local infections spread hematogenously to distant organs. This hematogenous dissemination of bacteria is part of the pathophysiology of, e.g., meningitis and endocarditis, Pott's disease and many other forms of osteomyelitis. In the hospital, indwelling catheters are a frequent cause of bacteremia and subsequent nosocomial infections, since they provide a means by which bacteria normally found on the skin can enter the bloodstream. Other causes of bacteremia include dental procedures, urinary tract infections, intravenous drug use, and colorectal cancer.

Systemic fungal infection is becoming more and more common in modern hospitals. The most common fungal infections are candidiasis and aspergillosis, but other systemic fungal infections such as Histoplasmosis, Blastomycosis, Coccidioidomycosis and Cryptococcosis are also of increasing relevance. Systemic fungal infections in hospitals are commonly seen in immune compromised patients and - like bacteremia - in patients with indewelling catheters. Due to underlying serious illnesses and possible resistance of the pathogens to antifungal agents, patients with system ic fungal infections often have poor clinical outcomes. Infections due to Candida species are the fourth most important cause of nosocomial bloodstream infection.

Bacteremia is operationally defined as the presence of viable bacteria as evidenced by positive blood cultures. Fungemia is similarly defined as the presence of viable fungi as evidenced by positive blood cultures. When bacteremia or fungemia occurs in the presence of systemic symptoms (such as fever or chills) the condition is designated as sepsis; and in the setting of more severe disturbances of temperature, respiration, heart rate or white blood cell count, is characterised as systemic inflammatory response syndrome (SI RS).

Many septic episodes are nosocomial and often due to microorganisms with increased and multiple antimicrobial resistance. *Staphylococcus aureus, Escherichia coli,* Coagulase-negative staphylococci (CoNS), *Klebsiella pneumoniae, Pseudomonas aeruginosa, Enterococcus* spp., *Streptococcus* spp., *Candida albicans* and *Enterobacter cloacae* are the most frequent etiological agents of bacteremia and fungemia in Europe (Decousser, J. W. et al., J. Antimicrob. Chemother. 51:1214-22 (2003); Lyytikainen, O. et al., Clin. Infect. Dis. 35:314-9 (2002); Reacher, M.H. et al., BMJ 320:213-6 (2000); Rosenthal Kreuberger, E.J., Int. J. Antimicrob. Agents 24:196-8 (2004)) and the USA (Bourbeau, P.P. and Pohlman, J.K., J. Clin. Microbiol. 39:2079-82 (2001); Reimer, L.G. et al., Clin. Microbiol. Rev. 10:444-65 (1997); Reisner, L.G. et al., J. Clin. Microbiol. 37:2024-6 (1999); Wilson, M.L. et al., J. Clin. Microbiol. 37:1709-13 (1999)).

Nosocomial bacteremia and especially sepsis require an immediate antibiotic therapy, even when the causative bacteria are still unknown. Thus, said therapy has to be performed as empirical initial therapy (Rello, J. et al., Intensive Care Med. 20:94-98 (1994)), which covers the complete spectrum of relevant pathogens. However, the increase of bacterial resistance lowers the chance of success for such empirical antibiotic treatments considerably (Mylotte, J.M. and Tayara, A., Eur. Clin. Mcrobiol. Infect. Dis. 19:157-163 (2000); Weinstein, M.P. et al., Clin. Infect. Dis. 24:584-602 (1997)). This primary therapy can only be replaced by a specific treatment after a thorough microbial diagnosis which usually takes 76-120 h (Bourbeau, P.P. and Pohlman, J.K., J. Clin. Microbiol. 39:2079-2082 (2001)). A fast track diagnosis which shortens this lag time would increase the chance of therapy success.

Rapid and reliable detection of bloodstream infections, including characterisation of the pathogen to the species level and determination of its antibiotic susceptibility pattern, is crucial for several reasons: (i) Appropriate antimicrobial agents can be selected, and thus, unnecessary treatment with ineffective antibiotics can be avoided; (ii) the prognosis of the patients can be improved; (iii) the acquisition of resistances in pathogens may be decelerated and (iv) expenditures on antimicrobials and overall hospital costs can be reduced (Barenfanger, J. et al., J. Clin. Microbiol. 37:1415-8 (1999); Doern, G.V. et al., J. Clin. Microbiol. 32:1757-62 (1994); Trenholme, G.M. et al., J. Clin. Microbiol. 27:1342-5 (1989); Wheeler, A.P. and Bernard, G.R., N. Engl. J. Med. 340:207-14 (1999)). Therefore, there is a strong need for rapid tests for specific and sensitive identification of bacteria and pathogenic fungi directly from blood cultures.

The diagnosis of bacteremia commonly relies on blood cultures where the growth of microorganisms is continuously monitored by automated devices (James, P.A. and Al-Shafi, K.M., J. Clin. Pathol. 53:231-233 (2000); Reisner, B.S. and Woods, G.L., J. Clin. Microbiol. 37:2024-2026 (1999); Wilson, M.L. et al., J. Clin. Microbiol 37:1709-1713 (1999)). Although such continuous-reading and computed systems decrease the time for detection of positive blood cultures, definitive pathogen identification from positive blood cultures still requires traditional Gram-staining, sub-culturing and susceptibility testing, delaying the identification of pathogens for one to three days (Levi, K and Towner, K.J., J. Clin. Microbiol. 41:3890-3892 (2003); Oliveira, K. et al., J. Clin. Microbiol. 41:889-891 (2003); Oliveira, K. et al., J. Clin. Microbiol. 40:247-251 (2002); Tan, T.Y. et al., J. Clin. Microbiol. 39:4529-4531 (2001)). The subculture procedure with subsequent species identification and determination of antibiotic resistance is time-consuming and elaborate. The biochemical and immunological assays like testing with coagulase, nuclease or latex agglutination are not always reliable. Antigenic and biochemical variations of bacteria grown in blood culture, inhibitory action of blood culture medium components as well as the presence of more than one microbial species may mislead data interpretation.

Staphylococci are the most important and frequent group of pathogens growing in blood culture, responsible for 30% to more than 50% of all bacteremia events (James, P.A. and Al-Shafi, K.M., J. Clin. Pathol. 53:231-233 (2000); Reisner, B.S. and Woods, G.L., J. Clin. Microbiol. 37:2024-2026 (1999); Velasco, E. et al., Sao Paulo Med. J. 118:131-138 (2000)) with a mortality rate ranging from 13 to 50% (McClelland, R.S. et al., Arch. Intern. Med. 159:1244-1247 (1999); Rello, J. et al., Intensive Care Med. 20:94-98 (1994); Weinstein, M.P. et al., Clin. Infect. Dis. 24:584-602 (1997)). The emergence of S. *aureus* strains with multiple resistance to antibiotics makes empirical therapy prone to fail (Tan, T.Y. et al., J. Clin. Microbiol. 39:4529-4531 (2001)). *S. aureus* is generally regarded as a virulent pathogen, whereas CoNS are either considered as a cause of catheter-associated nosocomial bacteremia or, more frequently, as blood culture contamination. Thus, a subgenus identification of gram-positive cocci in clusters (CPCC) is of great clinical significance (Oliveira, K. et al., J. Clin. Microbiol. 41 :889-891 (2003)).

Methods used up to date for direct identification of *S. aureus* growing in blood culture bottles include biochemical tests, like detection of thermostable nuclease or tube coagulase test, or commercial antibody-based kits connected with the disadvantages listed above.

Besides *S. aureus* and coagulase-negative staphylococci, *E. coli, Klebsiella* spp., *Enterobacter* spp., *Proteus* spp. and *P. aeruginosa* belong to the most frequent reported pathogens causing bacteremia (Reimer, L.G. et al., Clin. Microbiol. Rev., 10:444-65 (1997); Reacher, M.H. et al., BMJ, 320:213-6 (2000); Lyytikainen, O. et al., Clin. Infect. Dis., 35:e14-9 (2002)). In order to reduce the time needed for identification and susceptibility testing, the possibility of combining an automated blood culture system with an automated identification and susceptibility testing system by direct inoculation from positive blood cultures has been studied for gram-positive cocci as well as for gram-negative rods by several groups of investigators, but with varying success (Reimer, L.G. et al., Clin. Microbiol. Rev., 10:444-65 (1997); Hansen, D.S. et al., Clin. Microbiol. Infect., 8:38-44 (2002); Ling, T.K. et al., J. Clin. Microbiol., 41:4705-7 (2003); Funke, G. and Funke-Kissling, P., J. Clin. Microbiol., 42:1466-70 (2004)). Although the authors saw some potential of the combined system to allow the agar isolation step to be skipped, the system is hampered by the fact that (i) the blood culture sample has to undergo a time-consuming separation procedure for the enrichment of bacterial cells, (ii) the identification rate varies depending on the employed identification system and (iii) the performance is not equally good for gram-negative and gram-positive pathogens (Reimer, L.G. et al., Clin. Microbiol. Rev., 10:444-65 (1997); Ling, T.K. et al., J. Clin. Microbiol., 41:4705-7 (2003); Funke, G. and Funke-Kissling, P., J. Clin. Microbiol., 42:1466-70 (2004)).

Considerable progress was made using nucleic acid-based methods for the identification and genotyping of bacteria or fungi in blood specimens. Assays employing ribosomal RNA-based oligonucleotide probes like fluorescence *in situ* hybridisation (FISH) (Chapin, K. and Musgnug, M., J. Clin. Microbiol. 41:4324-7 (2003); Jansen, G.J. et al., J. Clin. Microbiol. 38:814-7 (2000); Kempf, V.A. et al., J. Clin. Microbiol. 38:830-8 (2000); Oliveira, K. et al., J. Clin. Microbiol. 41-889-91 (2003)) or microarrays (Anthony, R.M. et al., J. Clin. Microbiol. 38:781-8 (2000); Marlowe, E.M. et al., J. Clin. Microbiol. 41 :5127-33 (2003); Sogaard, M. et al., J. Clin. Microbiol., 43:1947-9 (2005)) provide for rapid species identification in blood cultures. However, methods solely based on ribosomal RNA probes allow species identification only, and do not provide information on antibiotic susceptibility and other strain specific characteristics (e.g. virulence genes). For the molecular detection of antibiotic resistances in staphylococci, several multiplex PCR-based assays were described (Martineau, F. et al., Antimicrob. Agents Chemother. 44:231-8 (2000); Shrestha, N.K. et al., Approved standard M2-4A, Villanova, PA (1990); Strommenger, B.C. et al. J. Clin. Microbiol. 41:4089-94; Tan, T.Y. et al., J. Clin. Microbiol. 39:4529-31 (2001)). Several groups have successfully identified *S. aureus* and more specifically methicillin-resistant *S. aureus* strains (MRSA) from blood cultures by using DNA probes (Levi, K. and Towner, K.J., J. Clin. Microbiol. 41:3890-3892 (2003); Poulsen, A.B. et al., J. Antimicrob. Chemother. 51 :419-421 (2003)), peptide nucleic acid probes (Oliveira, K. et al., J. Clin. Microbiol. 41 :889-891 (2003)), multiplex PCR (Mason, W. J. et al., J. Clin. Microbiol. 39:3332-3338 (2001)), gel-based PCR(Krishnan, P.U. et al., J. Clin Pathol. 55:745-748 (2002)), and real-time PCR (Shrestha N.K. et al., J. Clin. Microbiol. 40:2659-2661 (2002); Tan, T.Y. et al., J. Clin. Microbiol. 39:4529-4531 (2001)).

However, the use of such molecular assays suffers from two main restrictions: First, they rely on a pre-identification of the pathogen since their discriminatory capacity is technically limited, for instance by the number of fluorochromes available for labelling the probes or, in the case of multiplex PCR, by the capacity of resolution in gel electrophoresis. These molecular assays are thus usually not scalable and unfit for high throughput analysis.

The last years have witnessed the emergence of many DNA microchip projects arraying genes of microorganisms (Ye, R.W. et al., J. Microbiol. Methods 47:257-272 (2001)). They can detect tens of thousands of DNA sequences in a single hybridisation step (DeRisi, J.L. et al., Science 278:680-686 (1997); Duggan, D.J. et al., Nat. Genet. 21:10-14 (1999); Lashkari, D.A. et al., Proc. Natl. Acad. Sci. USA 94:13057-13062 (1997)). Originally developed for gene expression profiling, DNA sequence analysis and genotyping, microarrays were recently also used to identify viral (Wang, R.F. et al., FEMS Microbiol. Lett. 213:175-182 (2002)) and bacterial (Bekal, S. et al., J. Clin. Microbiol. 41 :2113-2125 (2003)) pathogens in environmental and clinical samples.

Most of the published reports employed oligonucleotide microarrays containing a reduced number of spotted probes and representing a single bacterial species only (Volokhov, D. et al., J. Appl. Microbiol. 95:787-798 (2003); Volokhov, D. et al., J. Clin. Microbiol. 41:4071-4080 (2003); Volokhov, D. et al., J. Clin. Microbiol. 40:4720-4728 (2002)). Such arrays were used to identify pathogenic strains belonging to a pre-identified species (Chizhikov, V. et al., Appl. Environ. Microbiol. 67:3258-3263 (2001)), to distinguish between species of the same genus (Volokhov, D. et al., J. Clin. Microbiol. 41:4071-4080 (2003); Volokhov, D. et al., J. Clin. Microbiol. 40:4720-4728 (2002)) or to detect genes encoding resistance to a certain antibiotic (Volokhov, D. et al., J. Appl. Microbiol. 95:787-798 (2003)).

Although such specific short-oligonucleotide microarrays could be rapidly designed and built up they carry some intrinsic disadvantages: like all methods based on single and often short DNA sequences they show reduced reliability and sensitivity (Stears, R.L. et al., Nat. Med. 9:140-145 (2003)). To palliate the high probability of non-specific hybridisation due to their short size (20-40bp) it is necessary to design many partially overlapping oligonucleotides in order to confirm the presence of a gene. This consequent increase in complexity makes it extremely difficult to set up the optimal hybridisation conditions necessary for producing trustful results. Moreover, surface-bound short oligonucleotides have poor hybridisation properties and are highly sensitive to single nucleotide polymorphisms (Hughes, T.R. et al., Nat. Biotechnol. 19:342-347 (2001)). For these reasons, oligonucleotide micro-arrays are unsuitable for routine diagnostics.

Up to now, diagnosis of bacteremia by microarrays is limited to species identification by oligonucleotides for 23S RNA sequences, which is still strictly experimental (Anthony, R.M. et al., J. Clin. Microbiol. 38:781-788 (2000)) and carries along the methodological weakness associated to the use of oligonucleotides as hybridisation probes.

A DNA microarray employing capture probes of more than 40 nt length amplified by PCR was described by Fitzgerald et al. (Fitzgerald, J.R. at al., Proc. Natl. Acad. Sci. USA 98(15):8821-8826 (2001)). To investigate molecular population genetics of *Staphylococcus aureus* on a genome scale, a microarray comprising 2817 complete ORFs of *S. aureus* strain COL was constructed, representing >90% of the *S. aureus* genome. The microarray was able to discriminate 36 *S. aureus* strains. However, since it was not designed for the identification of different bacterial species, it was not tested for possible cross reactions with other bacteria besides *S. aureus.* Due to the conservative nature of many house-keeping proteins and genes, respectively, cross reactions of the microarray with CoNS strains and other bacterial species will occur. Unspecific cross reactions combined with the high number of probes (2817) result in a high complexity of the microarray data, not applicable to routine diagnostics. Furthermore, PCR amplification of long ORFs is a difficult procedure, in particular for bacteria with DNA of high GC-content.

The aim of present invention is to provide a gene-segment based microarray for identification and characterisation of different microorganisms, especially different bacteria and pathogenic fungi, present in a sample or clinical specimen.

### Summary of the Invention

The present invention provides a DNA microarray for the identification and characterisation of microorganisms in biological samples, especially of microorganisms connected with bacteremia, fungemia and sepsis. Species specific gene probes in this microarray allow the identification of different microbial species, whilst antibiotic resistance and virulence gene probes allow for the genotypic discrimination within a species. The microarray can be designed to allow species identification, virulence determination and resistance determination independently from each other or simultaneously, and furthermore said determinations can be performed for one or more different microbial species and strains with one microarray. Furthermore, different microbial species and strains are discriminated, even in a polymicrobial sample (specimen with more than one pathogen).

The DNA microarray according to present invention thus demonstrates the feasibility of simultaneously identifying and characterising different microbial species in a sample or clinical specimen, especially in blood samples, without prior PCR amplification of target DNA or pre-identification of the pathogen. This can reduce sample processing time to a single day and less.

The invention furthermore provides a method for rapid identification and characterisation of microorganisms, especially of bacteria, yeasts and filamentous fungi, using the microarray of the invention. The method is quick, can be automated, leads to reproducible results and allows an early choice of specific antibiotics for treatment of bacteremia, fungemia or sepsis.

I n particular, the present invention provides
(1) a DNA microarray for direct identification and characterisation of microorganisms in a sample or clinical specimen, wherein the microarray comprises gene probes being derived from DNA sequences or partial DNA sequences of the microorganisms to be identified or DNA sequences complementary or homologous thereto and having a length of at least 100 nucleotides (nt);
(2) the use of the DNA microarray as defined in (1) above for *in vitro* identification and characterisation of microorganisms in a sample or in a clinical specimen, preferably for the diagnosis of bacteremia, fungemia or sepsis;
(3) an *in vitro* method for identification and characterisation of microorganisms in a sample or in a clinical specimen comprising
   (a) isolating the total DNA from the sample or clinical specimen and labelling the DNA with a reporter molecule, preferably a fluorochrome;
   (b) applying the DNA thus obtained to the DNA microarray as defined in (1) above and hybridising the DNA with the gene probes of the DNA microarray; and
   (c) detecting DNA bound to the DNA microrarray by determination of the amount of the reporter molecules bound to the array; and
(4) a kit for detection of microorganisms in a sample or clinical specimen comprising the m icroarray of embodiment (1).

### Brief description of the Figures

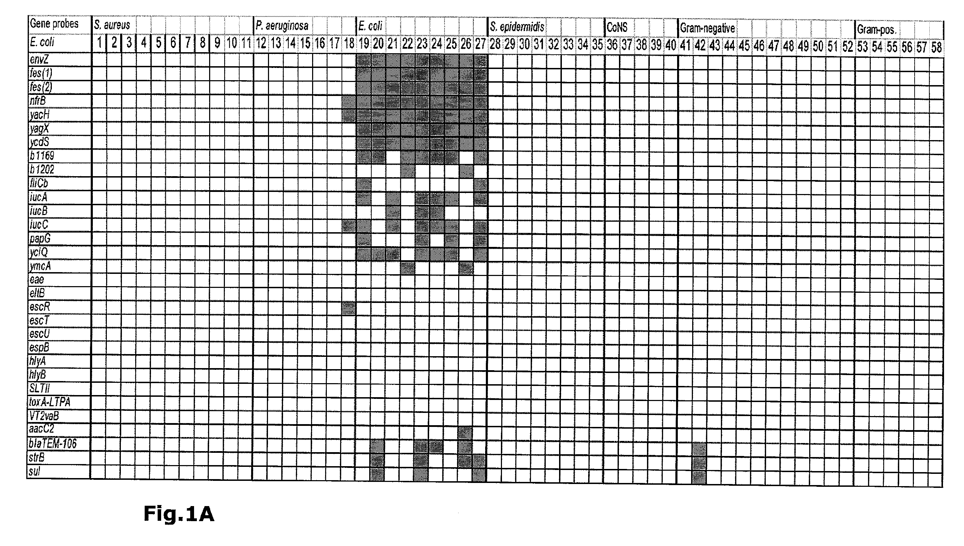

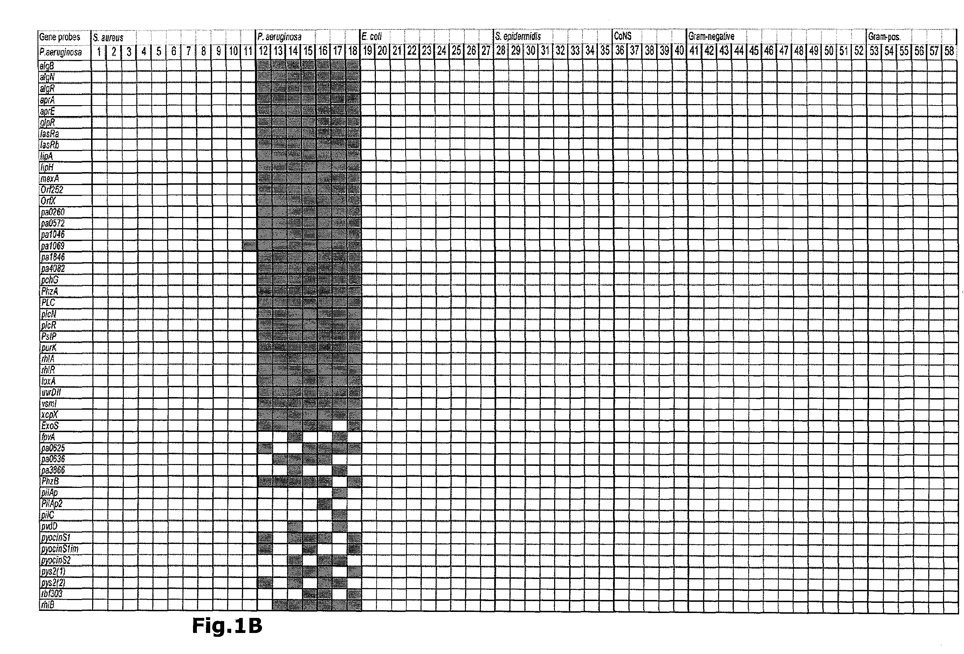

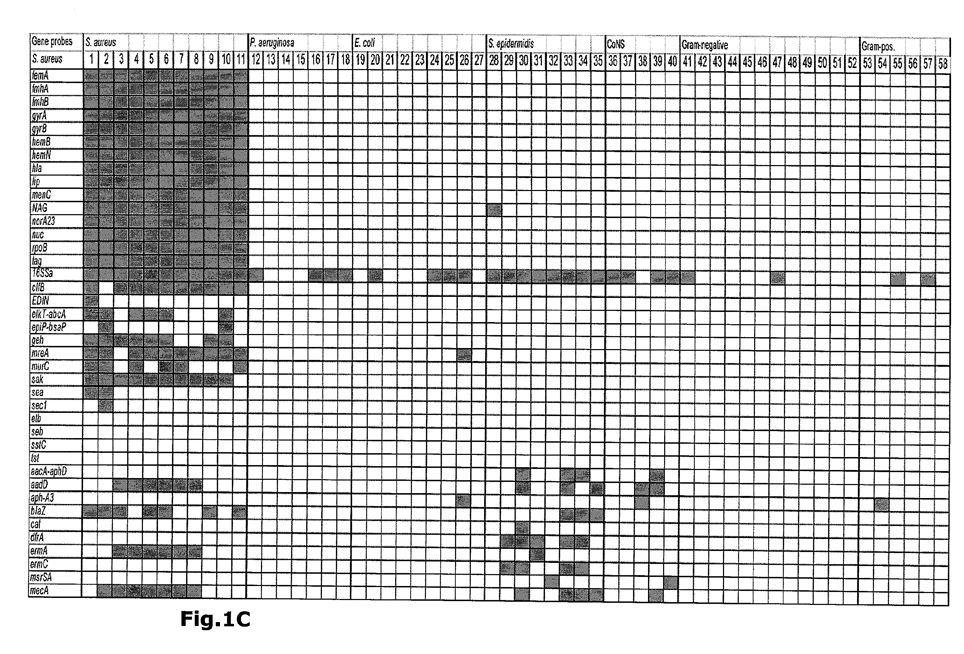

Fig. 1: DNA microarray analyses of 58 clinical isolates, reference strains and blood cultures.
   Each column shows the results of an individual hybridisation with target DNA prepared from: *S. aureus* ATCC 29213 (1), MW2 (2), clinical isolates (3-7), positive blood cultures (8-11); *P. aeruginosa* ATCC 27853 (12), clinical isolates (13-17), positive blood culture (18); *E. coli* ATCC 25922 (19), clinical isolates (20-25), positive blood cultures (26-27); *S. epidermidis* clinical isolates (28-32), positive blood cultures (33-35); clinical isolates of *S. auricularis* (36), *S. capitis (37), S. haemolyticus* (38), *S. hominis* (39), and *S. warneri* (40). Other Gram-negative species included a *Proteus mirabilis* positive blood culture (41), clinical isolates of *Proteus mirabilis* (42-43), *Serratia marcescens* (44-45), *Klebsiella pneumonia* (46-48), *Stenotrophomonas maltophilia* (49), *Acinetobacter baumannii* (50), *Enterobacter cloacae* (51) and *Enterobacter aerogenes* (52); other Gram-positive species included clinical isolates of *Micrococcus* spp. (53), *Enterococcus* spp. (54), *Enterococcus faecalis* (55) and *Streptococcus pneumoniae* (56) and two positive blood cultures of *S*. *pneumoniae* (57-58).
   (A) Hybridisation of DNA prepared from bacterial isolates, reference strains and blood cultures with *E*. *coli* gene probes;
   (B) hybridisation with *P. aeruginosa* gene probes;
   (C) hybridisation with *S. aureus* gene probes.
   Grey boxes represent gene probes which hybridised with the respective target DNA, white boxes represent gene probes which showed no hybridisation with the respective target DNA.

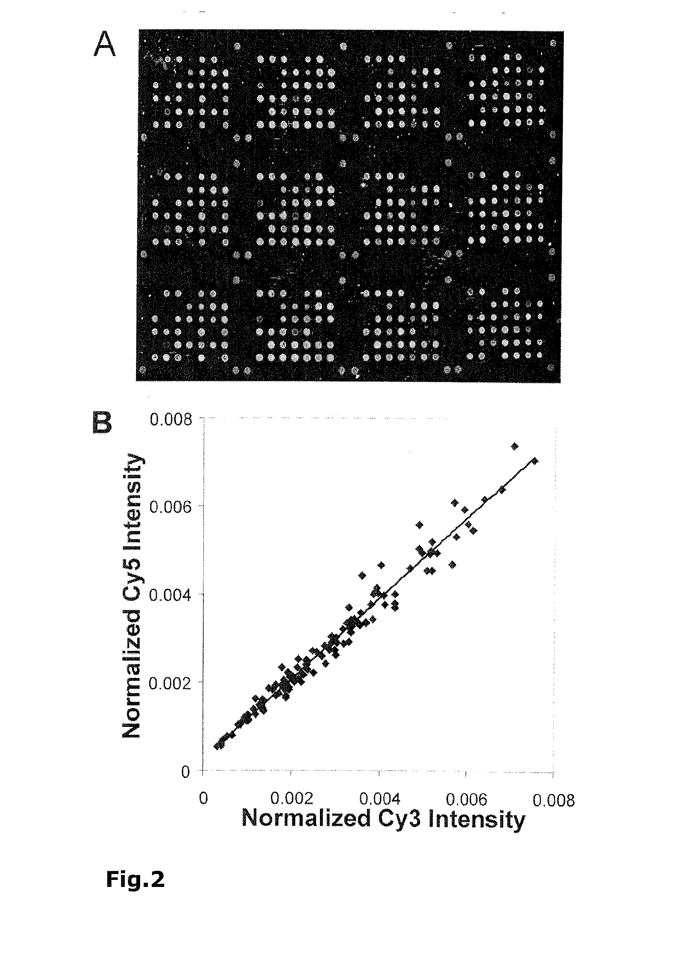

Fig. 2: Validation of the *S. aureus* microarray of example 11. 2 µg genomic DNA from *S. aureus* strain T94 were labelled either with Cy3 or Cy5, combined and hybridised as described in Example 11. Cy3: green signal; Cy5: red signal; double-hybridisation: yellow signal.
   A) Overlay of microarray scanned using Cy3 and Cy5 filter sets;
   B) Scatterplot of normalized fluorescence intensities of individual gene probes after microarray hybridisation. The signal intensities from both channels correlate highly with each other (r² = 0.97).

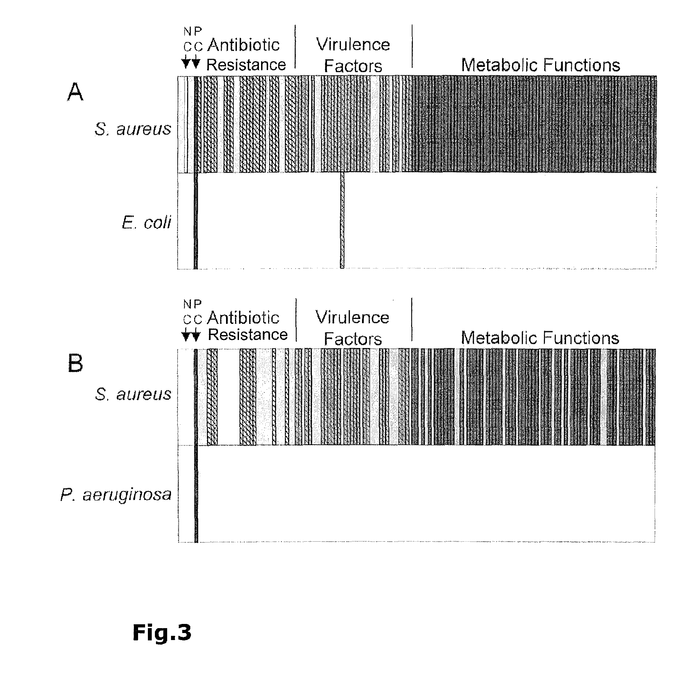

Fig. 3: Specific identification of *S. aureus* from distantly related bacteria using the microarray of example 11. 2 µg of *S. aureus* DNA were co-hybridised with 2 µg of pure *E. coli* (A) or *P. aeruginosa* (B) genomic DNA. Obtained hybridisation patterns are represented as bar codes, where the 140 spotted gene segments appear subsequently and are clustered in categories (NC: negative control; PC: positive control; Antibiotic Resistance Determinants; Virulence Factors and Metabolic Functions (see Tab. 6)). Positive hybridisation is indicated by a bar while negative spots are represented by an em pty area. Both assays show clear *S. aureus* discrimination with practically no cross hybridisation between DNA from said gram negative bacteria and *S. aureus* selected genes, while the positive control (16S RNA sequence) reveals the good quality of hybridisation.

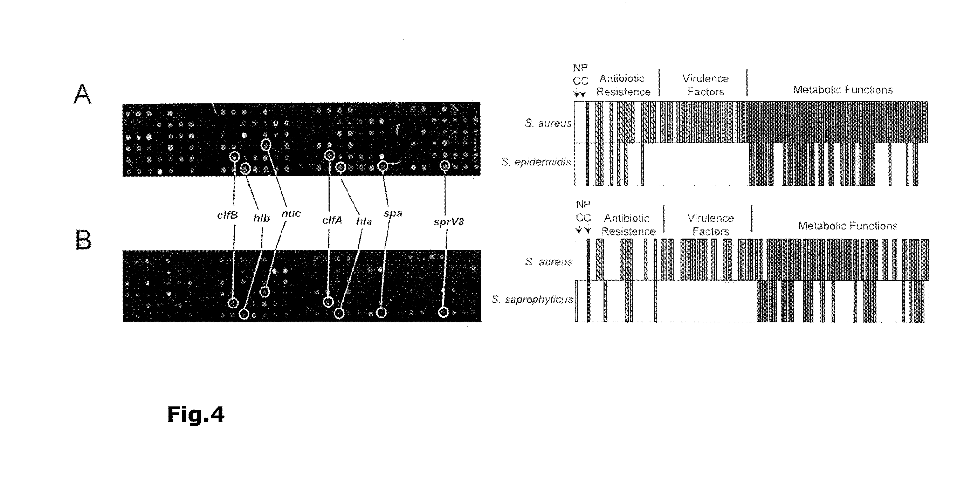

Fig. 4: Specific identification of *S. aureus* from coagulase negative staphylococci using the microarray of example 11. 2 µg of *S*. *aureus* DNA were co-hybridised with 2 µg of *S*. *epidermidis* (A) or *S*. *saprophyticus* (B) genomic DNA. Obtained hybridisation patterns are illustrated by scanned fluorescent picture data (A: *S*. *aureus:* green signal; *S*. *epidermidis:* red signal; B: *S*. *aureus:* red signal; *S*. *saprophyticus:* green signal) and transformed in bar codes (see legend of Fig. 3). All specific *S*. *aureus* virulence factor genes hybridised exclusively with *S. aureus* DNA. Yellow spots showing cross-hybridisation correspond to some shared antibiotic resistance determinants and genes associated to metabolic functions.

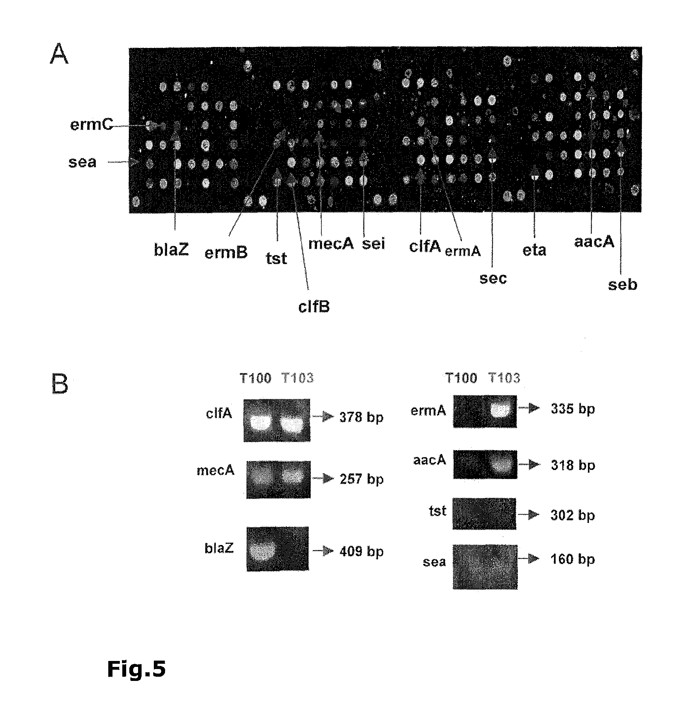

Fig. 5: Specificity of the *S. aureus* m icroarray of example 11.
   A) Scan of microarray hybridised with 2 µg each of genomic DNA from *S. aureus* strain T103 (Cy3, represented in green) or T100 (Cy5, represented in red), showing remarkable genotypic differences between strains.
   B) PCR amplification of the genes from genomic DNA of *S. aureus* (strains T100 and T103) validating results of the microarray hybridisation shown in (A).

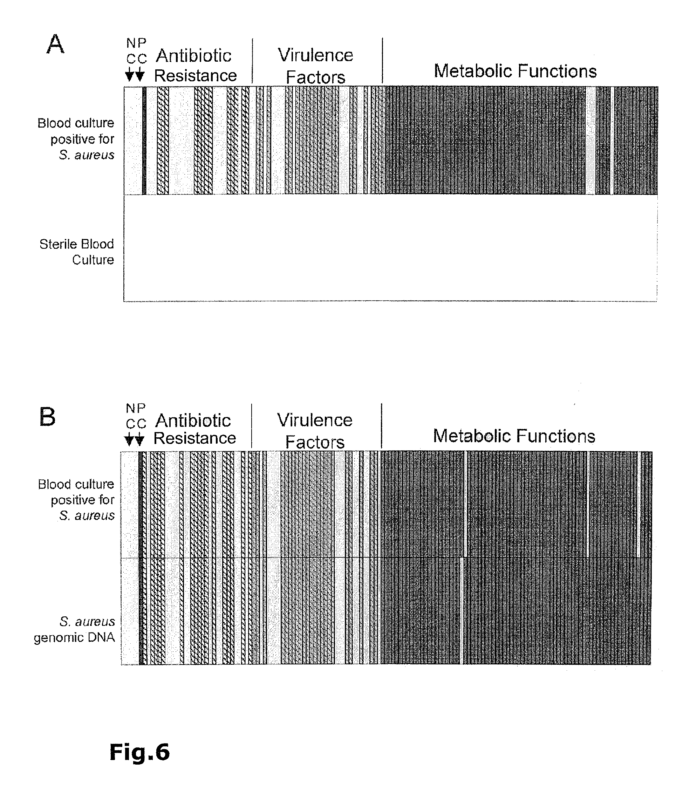

Fig. 6: Identification and characterisation of *S. aureus* from positive blood culture using the microarray of example 11.
   2 µg of DNA prepared from blood culture positive for *S. aureus* (strain T95) was co-hybridised with 2 µg of DNA prepared from sterile blood culture or with 2 µg of pure *S. aureus* genomic DNA for 4 hours. Positive and negative spots are transformed in a bar code scheme (see legend of Fig. 3).
   Sterile blood culture DNA did not cross-hybridise with spotted *S. aureus* genes (A). Blood culture positive for *S. aureus* produced a fluorescent hybridisation pattern almost identical to the pattern obtained with pure *S. aureus* genomic DNA (B).

### Definitions

In the framework of the present invention the following terms and definitions are used.

A "DNA microarray" consists of a collection of nucleic acid sequences, preferably DNA sequences, immobilized onto a solid support, such as glass, plastic or silicon chips, in a latticed pattern (forming an "array"), Each unique sequence of said sequences forms a tiny feature on the microarray called a "spot" or "capture probe". The size of these spots varies from one system to another, but is usually less than two hundred micrometers in diameter, thus up to tens of thousands of spots can be arrayed in a total area of a few square centimeters. DNA microarrays provide a means to detect and quantity large numbers of discrete nucleic sequences in parallel. In a microarray hybridisation the nucleic acids in the sample that is being analysed (called "target") are expected to form duplexes specifically with the corresponding capture probes. Occurrence or absence of duplex formation indicate the presence or absence of said target, For routine microarray analysis, said target is commonly converted to a labelled population of nucleic acids, using reporter molecules. Hybridisation of said labelled target DNA molecules from the tested samples with complementary DNA sequences affixed in specific spots on the array can thus be detected by examination for the presence of said label on the array using a microarray scanner (Müller, H.-J., Röder, T., "Der Experimentator: Microarrays, Spektrum Akademischer Verlag, Heidelberg (2004)).

"Gene probe" or "gene probe derived from..." refers to a DNA sequence present on the microarray of present invention and used as a capture probe. It is complementary to a target DNA sequence, preferably to a microbial, more preferably to a bacterial or fungal gene or gene segment. Said gene probe is prepared by any known method of DNA synthesis, and preferably prepared by cloning the respective PCR-amplified gene or gene segment into a plasmid/vector. The recombinant gene or gene segment is then amplified by PCR, isolated from the amplification mix, purified (preferably by ethanol-purification) and finally spotted onto the array.

A "clinical isolate" is a microbial, especially a fungal or bacterial strain isolated from a clinical specimen, wherein the isolation includes at least one *in vitro* propagation.

An "isolated DNA" is a DNA separated or purified from the organism it is naturally associated with or from the clinical specimen in which it occurs. This comprises biochemically or biophysically purified native DNA, recombinant DNA, chemically synthesized DNA and DNA analogues (e.g. peptide nucleic acids).

"Native" is synonymous to "naturally (occuring)".

A "DNA segment" or "gene segment" is an isolated DNA which contains or consists of a part of the native full-length sequence of a gene which is still able to hybridize to the native sequence under stringent hybridisation conditions. Although the present invention is in the following exclusively described as relating to "DNA" sequences, it is not to be construed as being lim ited thereto. Rather, if the term "DNA" is used in connection with the gene probes or target sequences of present invention, it includes other polynucleotides (like RNA or RNA/DNA hybrids), and DNA analogues such as PNA, phosphonate backbone DNA, artificial pentose or hexose backbone DNA which is able to hybridize with native DNA etc.. Furthermore, modified bases like deoxy bases, inosine or aminoallylcytosine may be used on all DNA, RNA and PNA backbones. However, DNA itself is the preferred polynucleotide for performance of the invention.

The DNA sequences used as gene probes in present invention are either identical, substantially identical or homologous to the complementary native target sequences. I n the context of present invention, when a specific DNA sequence is denominated, this encompasses not only said specific sequence, but also the sequences substantially identical or homologous thereto, i.e. its substitution mutants. "Substantially identical" means that the DNA contains mutations of up to 10% of the total number of nt in comparison with the native DNA sequence and/or has a nucleotide identity of > 90% to the corresponding native DNA segment. Said mutations are preferably single nucleotide polymorphisms or point mutations and include the mutation of not only a single but also a few (up to 10 nt, preferably up to 5 nt) consecutive nt. "Homologous" or "homologue" refers to a DNA sequence which has a sequence identity of more than 70% of the corresponding native DNA sequence and encompasses the substantially identical DNA sequences. Preferably, the sequences used as gene probes are at least substantially identical to the corresponding native DNA sequence.

Preferred gene probes of the present invention are the DNA sequences listed in the sequence protocol, their complementary sequences or their corresponding native DNA segment.

The DNA sequences used as gene probes in present invention may also be deletion or addition mutants of the corresponding native DNA segments. In case of deletion mutants, the minimum length of the DNA sequences suitable as probes in present invention is 100 nt. Preferably, the deletions take place at the 5' - and/or 3' -terminus of the native DNA segment. In case of addition mutants, the added nucleotides may sum up to a total of 90% of the nucleotide number of the native DNA segment, if added at the 5' - or 3' - terminus of the DNA sequence. Alternatively, the additions and deletions may be of one isolated nucleotide or of 2 or more consecutive nucleotides at one or more internal site(s) of the native DNA segment. Preferably, 0-30% nucleotides of the corresponding native DNA segment are added or deleted. It is most preferred that the addition or deletion mutants used as gene probes in present invention comprise one or more segment(s) of at least 100 consecutive nt each, which are derived from one gene, and/or sequences homologous (70% homology) or complementary thereto. These segments may be embedded in or fused to other DNA sequences, which will not hybridize under stringent conditions with either human or bacterial DNA or the DNA of the target microorganism. Said other DNA sequences preferably have a maximum length wich adds up with the length of the enclosed segment(s) to not more than the upper limit for the length of gene probes suitable for present invention.

A "positive blood culture" is an *in vitro* culture started from whole blood or blood components wherein the growth of microorganisms has been detected. Said growth is indicated by a positive growth index. The detection is preferably done by monitoring CO₂ production in the blood culture.

"Direct identification" of microorganisms refers to an identification method which comprises isolation of DNA from a sample or clinical specimen, but does not require an amplification of the genetic material of the microorganisms after said isolation in order to identify the microorganisms using the method of present invention. The isolated genetic material is labelled and applied to the DNA microarray of present invention without prior amplification, i.e. directly after isolation or after a short workup step.

A "detection method" in the context of the present invention is a method for determination of hybridisation of DNA molecules contained in a sample to the probes on the solid support of the microarray of present invention. This method may be any textbook method for detection of DNA hybridisation on microarrays, e.g. direct detection or labelling of target DNA with a reporter molecule and consecutive visualisation of the reporter molecule. Preferred detection methods are said labelling method and the direct detection by electrical biosensors or mass spectrometry (Liu, R. H. et al., Anal. Chem. 76(7):1824-31 (2004); Stomakhin, A. A. et al., Nucleic Acids Res. 28(5):1193-8 (2000)).

A "reporter molecule" in the context of the method of the present invention is a chemical or physical marker which allows differentiation of labelled from unlabelled DNA by physical, chemical or immunological methods. The labelling method includes, but is not limited to radioactive labelling (e.g. with ³³P, ³²P), fluorescent/luminescent/chromophor labelling and hapten labelling (i.e. psoralen or DIG). It is followed by an appropriate detection step necessary to determine the presence and/or quantity of the reporter molecule, namely scintillation counting (e.g. phosphoimaging); photooptic measurement (e.g. fluorescence measurement, luminescence measurement) and antibody-based detection (including colorimetric, luminescence or fluorescence detection), respectively. Preferably, the reporter molecule is a fluorochrome/fluorophor (both terms are used as synonyms in the context of present invention) which includes but is not limited to cyanines, fluoresceins and rhodamines. More preferably, it is of the cyanine group of fluorophores. Most preferably, it is selected from the group consisting of the fluorophores Cy3, Cy5 or Alexa Fluor 647 and Alexa Fluor 546. The ratio of base to dye molecules (BDR) in DNA labelled with such reporter molecules is preferably less or equal to 60.

### Detailed description of the invention

The present invention provides a DNA microarray and its use for rapid identification and characterisation of microorganisms in a sample or clinical specimen (embodiments (1) to (3)).

The DNA microarray of embodiment (1) of the invention comprises gene specific DNA sequences as capture probes, which allow the identification of microbial species ("target species"), especially of bacterial and fungal species, and/or their further characterisation with regard to antibiotic resistance and virulence. Preferably, it allows the identification and characterisation of the target species. It is specific, applicable to the analysis of DNA isolated from blood cultures and suitable to detect resistance genes.

One important feature of the microarray of the present invention is that the panel of probes can be continually extended to include sequences for additional species, variant isolates or antibiotic resistance determinants as they are characterised and available. The accuracy, range and discriminatory power of the gene-segment based microarray can be refined by adding or removing gene probes to the panel without significantly increasing complexity or costs. In a pilot study, three important species causing bacteremia were selected to provide a proof of principle (examples 1-10). The range of organisms that can be identified can be easily expanded by increasing the number of gene probes on the array. For example, addition of a few probes specific for *S. epidermidis* and other CoNS will allow for the species identification of coagulase-negative staphylococci. Furthermore, due to a specific hybridisation pattern for each species it will also allow the identification of mixed blood cultures with more than one pathogen.

A second important feature of this microarray format is the length of the DNA sequences used as gene probes. They are at least 100 nt, preferably 100-3000 nt long. In an especially preferred aspect of embodiment (1) the length of the gene probes is from 100 to 1000 nt, most preferably from 200 to 800 nt. Thus, one probe per gene is usually sufficient to produce strong signals and high specificity (Stears, R.L. et al., Nat. Med., 9:140-5 (2003)). For long probes like these, minor point mutations are likely to only slightly reduce duplex formation, which does not lead to the loss of hybridisation signals. In contrast, short oligonucleotide microarrays sometimes lack specificity and require multiple short oligonucleotides per one gene.

The microorganims or microbial DNA to be detected using the microarray of present invention are preferably bacteria (such as *Staphylococci, Enterococci, Streptococci, E. coli, P. aeruginosa*) or fungi (such as yeasts and filamentous fungi, in particular *Candida* spp., *Aspergillus* spp., *Cryptococcus* spp., *Malassezia* spp., *Trichosporin* spp.), respectively bacterial or fungal DNA. The microarray is especially suitable for direct identification and characterisation of bacteria and *C. albicans.*

In one preferred aspect of embodiments (1), (2) and (3), the DNA microarray is feasible to identify and characterize any of the microorganisms, including the fungi and bacteria as defined above, known as etiological agents of fungemia, bacteremia or sepsis. I n another preferred aspect of (1), it is feasible to characterize the bacteria known as etiological agents of bacteremia or sepsis. More preferably, it is feasible to identify and characterize at least 90 % of said microorganisms or bacteria. Equally more preferably it is feasible to identify and characterize microorganisms selected from the group consisting of *S. aureus, Coagulase-negative staphylococci, Enterococci, Streptococci, E. coli, Klebsiella* spp., *Proteus* spp., *Enterobacter* spp., *P. aeruginosa, Stenotrophomonas* spp., *Acinetobacter* spp. and *Candida albicans,* most preferably microorganisms selected from the group consisting of *C. albicans, Enterococcus faecalis, Enterococcus faecium, E. coli, Klebsiella oxytoca, Klebsiella pneumoniae, Proteus mirabilis, Proteus vulgaris, Enterobacter cloacae, P. aeruginosa, Stenotrophomonas maltophilia, Acinetobacter baumannii, S. aureus, Staphylococcus epidermidis, Staphylococcus haemolyticus, Staphylococcus lugdunensis, Staphylococcus warneri, Streptococcus agalactiae, Streptococcus bovis, Streptococcus dysgalactiae, Streptococcus mitis, Streptococcus mutans, Streptococcus pneumoniae, Streptococcus pyogenes.* Most preferably, it is feasible to identify and characterize at least *S*. *aureus, E. coli* and *P. aeruginosa.*

The practicability and specificity of the DNA microarray for the identification and characterisation of *Staphylococcus aureus, Escherichia coli* and *Pseudomonas aeruginosa* grown in blood culture specimens was evaluated with clinical isolates and positive blood cultures (Examples 1-10). Especially preferred is a microarray which allows identification and characterisation of *S. aureus.* The latter microarray allows the detection of every *S. aureus* isolate, unambiguously identifies most of important virulence genes such as *tsst-1, sea, seb, eta* and antibiotic resistance genes such as *mecA, aacA-aphD, blaZ, ermA* and specifically distinguishes *S. aureus* from unrelated gram negative bacteria, e.g. *Escherichia coli* or *Pseudomonas aeruginosa,* as well as from closely related CoNS (Example 11, Fig. 2-6).

In another preferred aspect of the invention, the microarray of (1) is suitable for diagnosis of fungemia, bacteremia or sepsis; especially for diagnosis of bacteremia, candidemia, and bacterial or *Candida* sepsis.

The present invention provides a novel approach for detection of microorganisms, especially of bacteria and fungi, by microarrays: using gene-segments it allows species identification by probing a large and diverse set of species-specific genes. Such an approach is reliable since it makes possible to identify a pathogen even when some genes have been deleted from its genome. Furthermore, the selected DNA probes are at least 100 nt, preferably 200 to 800 nt long and are therefore not sensitive to single nucleotide polymorphisms or CG-content variations in the targets. Therefore, a gene segment array according to present invention is useful for indicating the presence of a gene even though the sequence may be slightly altered e.g. by point mutations (Southern, E. et al., Nat. Genet. 21 :5-9 (1999)). Additionally, it permits species virulence and antibiotics resistance profiling all together in a single-step test. Thus, present invention provides for a significant improvement compared to the classical approach focused on the detection of a short evolutionary conserved sequence like 16S RNA.

The number and perfect composition of gene-segments necessary for a correct species identification, virulence determination and resistance profiling must be determined by empiric specificity tests. Thus, in a preferred aspect of the invention, the DNA microarray of embodiment (1) comprises the minimal number of species specific gene probes which is sufficient for species identification, the minimal number of virulence gene probes which is sufficient for virulence determination, and/or the minimal number of resistance gene probes which is sufficient for determination of resistance of a specific microorganism. Preferably, the minimal number of gene probes in this aspect of the invention is: for correct species identification at least 2 different species specific gene probes per target species, more preferably at least 10, most preferably at least 20; for virulence determination at least 1 gene probe per target species, more preferably at least 5 different gene probes, even more preferably at least 20 different gene probes, most preferably gene probes for all known virulence factors of each target species; for determination of resistance at least 1 gene probe per antibiotic class or resistance factor, more preferably at least 5 different gene probes, most preferably all known gene-coded resistance determinants in the target species.

Generally, the DNA microarray of embodiment (1) comprises gene probes which are specific for a microbial species, bacterial/fungal species or a group of microorganisms to be identified. Said gene probes are preferably DNA sequences selected from three different groups, namely (a) species specific gene probes; (b) virulence gene probes; and/or (c) resistance gene probes. Preferably, the species specific set of gene probes for each species to be identified and characterised is selected from species specific gene probes (a) for
(i) *Staphylococcus aureus* including gene probes derived from *cataSaur, clfA, clfB, coa, I-clpC, I-clpP, I-ctaA, I-ctsR, I-dltA, I-dltB, I-dltC, I-dnaK, I-elkT, I-femD, I-glnA, I-glnR, I-grlA, I-grlB, I-groEL, I-groES, I-hemA, I-hemE, I-hemH, I-hemL, I-hemY, I-lepA, I-lrgA, I-lrgB, I-lytM, I-menB, I-menD, I-menE, I-menF, I-mreB, I-mreR, I-mutL, I-mutS, I-NAG, I-pbg, I-pbpF, I-pdhB, I-pdhC, I-rsbU, I-rsbV, I-rsbW, I-sgp, I-sirR, I-sodA, I-sodB, I-sstA, I-sstB, I-sstC, I-sstD, I-trx, I-yhiN, epiP-bsaP, geh, gyrA, gyrB, hemB, hemC, hemD, hemN, hsdS, hsdS, lip, menC, nuc, pdhD, rpoB, SAV0431, SAV0439, SAV0440, SAV0441, sigB, spa, sstC, tag, tyrA, I-aroC, I-aroA, I-cna, I-ebpS, I-eno, I-fbpA, I-fib, I-fnbB, I-srtA, I-stpC, I-fnbA, I-spa, I-aroE, I-aroF, I-aroG, I-asp23, I-atl*;
(ii) *Escherichia coli* including gene probes derived from *b1169, envZ, fliCb, nfrB, nlpA, pilAe, yacH, yagX, ycdS, yciQ, ymcA*;
(iii) *Staphylococcus epidermidis* including gene probes derived from *ardeSE0106, ardeSE0107, aroiSE0105, atlE, agrB, agrC, alphSE1368, gad, glucSE1191, hsp10, icaA, icaB, m vaSSepid, nitreSE1972, nitreSE1974, nitreSE1975, oiamtSE1209, ORF1Sepid, ORF3bSepid, qacR, sin, ureSE1861, ureSE1863, ureSE1864, ureSE1865, ureSE1867;*
(iv) *Staphylococcus haemolyticus* including gene probes derived from *folQShaemolyt, mvaCShaemolyticus, mvaDShaemolyt, mvaK1Shaemolyticus, mvaSShaemolyticus, RNApolsigm;*
(v) *Staphylococcus lugdunensis* including gene probes derived from *agrB2Stalugd, agrC2Stalugd, agrCStalugd, slamStalugd;*
(vi) *Staphylococcus warneri* including gene probes derived from *msrw1Stwar, nukMStwar, proDStwar, proMStwar, sigrpoStwar, tnpStwar;*
(vii) *Candida albicans* including gene probes derived from *ARG56, ASL43f, BGL2, CACHS3, CCT8, CDC37, CEF3, CHS1, CHS2, CHS4, CHS5, CHT1, CHT2, CHT4, CSA1, 5triphosphatase, AAF1, ADH1, ALS1, ALS7, EDT1, ELF, ESS1, FAL1, GAP1, GNA1, GSC1, GSL1, HIS1, HTS1, HWP1, HYR1, INT1a, KRE15f, KRE6, KRE9, MIG1, MLS1, MP65, NDE1, PFK2, PHR1, PHR2, PHR3, PRA1, PRS1, RBT1, RBT4, RHO1, RNR1, RPB7, RPL13, RVS167, SHA3, SKN1, SRB1, TCA1, TRP1, YAE1, YRB1, YST1exon2;*
(viii) *Enterococcus faecalis* including gene probes derived from *arcA, arcC, bkdA, cad, camE1, csrA, dacA, dfr, dhoD1a, ABC-eltA, agrBfs, agrCfs, dnaE, ebsA, ebsB, eep, efaR, gls24_glsB, gph, gyrAEf, metEf, mntHCb2, mob2, mvaD, mvaE, parC, pcfG, phoZ, polC, ptb, recS1, rpoN, tms, tyrDC, tyrs;*
(ix) *Enterococcus faecium* including gene probes derived from *bglB, bglR, bglS, efmA, efmB, efmC, mreC, mreD, mvaDEfaecium, mvaEEfaecium, mvaK1Efaecium, m vaK2Efaecium, m vaSEfaecium, orf3_4Efaeciumb, orf6_7Efaecium, orf7_8Efaecium, orf9_10Efaecium;*
*(x) Klebsiella pneumonia* including gene probes derived from *atsA, atsB, budC, citA, citW, citX, dalD, dalK, dalT, acoA, acoB, acoC, ahlK, fimK, glfKPN2, ItrA, mdcC, mdcF, mdcH, mrkA, mtrK, nifF, nifK, nifN, tyrP, ureA, wbbO, wza, wzb, wzmKPN2, wztKPN2, yojH, liac;*
(xi) *Klebsiella oxytoca* including gene probes derived from *cymA, cymD, cymE, cymH, cymI, cymJ, ddrA, fdt-1, fdt-2, fdt-3, gatY, hydH, masA, nasA, nasE, nasF, pehX, pelX, tagH, tagK, tagT;*
(xii) *Pseudomonas aeruginosa* including gene probes derived from *glpR, lasRb, OrfX, pa0260, pa0572, pa0625, pa0636, pa1046, pa1069, pa1846, pa3866, pa4082, pilAp, PilAp2, pilC, PstP, purK, uvrDII, vsml, vsmR, xcpX;*
(xiii) *Streptococcus pneumoniae* including gene probes derived from *cap1EStrpneu, cap1FStrpneu, cap1GStrpneu, cap3AStrpneu, cap3BStrpneu, celAStrpneu, celBStrpneu, cglAStrpneu, cglBStrpneu, cgICStrpneu, cglDStrpneu, cinA, cps14EStrpneum, cps14FStrpneum, cps14GStrpneum, cps14HStrpneum, cps19aHStrpneum, cps19aIStrpneum, cps19aKStrpneum, cps19fGStrpneum, cps23fGStrpneum, dexB, dinF, 1760Strpneu, acyPStrpneu, endAStrpneu, exoAStrpneu, exp72, fnlAStrpneu, fnlBStrpneu, fnlCStrpneu, gct18Strpneum, hexB1, hftsHstrpneu, immunofrag1Strpneu, immunofrag2Strpneu, immunofrag3Strpneu, KdtBStrpneu, lyAStrpneu, pcpBStrpneu, pflCStrpneu, plpA, prtA1Strpneu, pspC1Strpneu, pspC2, purRStrpneu, pyrDAStrpneum, SP0828Strpneu, SP0830Strpneu, SP0833Strpneu, SP0837_38Strpneu, SP0839Strpneu, ugdStrpneu, uncC, vicXStrepneu, wchA6bStrpneum, wci4Strpneum, wciK4Strpneum, wciL4Strpneum, wciN6bStrpneum, wciO6bStrpneum, wciP6bStrpneum, wciY18Strpneum, wzdbStrpneum, wze6bStrpneum, wzy18Strpneum, wzy4Strpneum, wzy6bStrpneum, xpt;*
(xiv) *Streptococcus agalactiae* including gene probes derived from *cpsA1Strgal, cpsB1Strgal, cpsC1Strgal, cpsD1Strgal, cpsE1Strgal, cpsG1Strgal, cpsIStragal, cpsJStragal, cpsKStragal, cpsMStragal, cpsYStragal, cylBStraga, cylEStraga, cylFStraga, cylHStraga, cylIStraga, cylJStraga, cylKStraga, 0487Straga, 0488Straga, 0493Straga, 0495Straga, 0498Straga, 0500Straga, 0502Straga, 0504Straga, folDStraga, neuA1Strgal, neuB1Strgal, neuC1Strgal, neuD1Strgal, recNStraga, ileSStraga;*
(xv) *Streptococcus pyogenes* including gene probes derived from *cyclStrpyog, fah_rph_hlo_Strpyog, int, int315.5, murEStrpyog, oppA, oppCStrpyog, oppD, SPy0382Strpyog, SPy0390Strpyog, SpyM3_1351, vicXStrpyog;*
*(xvi) Streptococcus viridans* including gene probes derived from *573Stprmut, 580SStprmut, 581_582SStprmut, 584SStprmut, dltAStrmut, dltBStrmut, dltCppx1Strmut, dltDStrmut, lichStrbov, lytRStprmut, lytSStprmut, pepQStrrmut, pflCStrmut, recNStprmut, ytqBStrmut;*
(xvii) *Proteus mirabilis* including gene probes derived from *atfA, atfB, atfC, ccmPrmi1, cyaPrmi, aad, flfB, flfD, flfN, flhD, floA, ftsK, gstB, hemCPrmi, hemDPrmi, hev, katA, lpp1, menE, mfd, nrpA, nrpB, nrpG, nrpS, nrpT, nrpU, pat, pmfA, pmfC, pmfE, ppaA, rsbA, rsbC, speB, stmA, stmB, terA, terD, umoA, umoB, umoC, ureR, xerC, ygbA;*
(xviii) *Proteus vulgaris* including gene probes derived from *envZPrvu, frdC, frdD, infBPrvu, lad, tna2.*

Preferably, the virulence specific set of gene probes for each species to be identified and characterised is selected from virulence gene probes (b) for
(i) *Staphylococcus aureus* including gene probes derived from *bsaE, bsaG, cap5h, cap5i, cap5j, cap5k, cap8H, cap8I, cap8J, cap8K, I-hld, I-hysA, I-IgGbg, EDIN, eta, etb, hglA, hglB, hglC, hla, hlb, lukF, lukS, NAG, sak, sea, seb, sec1, seg, seh, sel, set15, set6, set7, set8, sprV8, tst, I-sdrC, I-sdrD, I-sdrE;*
(ii) *Escherichia coli* including gene probes derived from *b1202, eae, eltB, escR, escT, escU, espB, fes, fteA, hlyA, hlyB, iucA, iucB, iucC, papG, rfbE, shuA, SLTII, toxA-LTPA, VT2vaB;*
(iii) *Staphylococcus epidermidis* including gene probes derived from *gcaD, hld_orf5, icaC, icaD, icaR, psm_beta1and2, purR, spoVG, yabJ;*
(iv) *Staphylococcus haemolyticus* including gene probes derived from *IipShaemolyt;*
(v) *Staphylococcus lugdunensis* including gene probes derived from *fblStalugd, slushABCStalugd;*
(vi) *Staphylococcus warneri* including gene probes derived from *gehAStwar;*
(vii) *Candida albicans* including gene probes derived from *CCN1, CDC28, CLN2, CPH1, CYB1, EFG1, MNT1, RBF1, RBF1, RIM101, RIM8, SEC14, SEC4, TUP1, YPT1, ZNF1 CZF1;*
(viii) *Enterococcus faecalis* including gene probes derived from *asa1, asp1, cgh, cylA, cylB, cylI, cylL_cylS, cylM, ace, ef00108, ef00109, ef0011, ef00113, ef0012, ef0022, ef0031, ef0032, ef0040, ef0058, enlA, esa, esp, gelE, groEL, groES, rt1, sala, salb, sea1, sep1, vicK, yycH, yycl, yycJ;*
(ix) *Enterococcus faecium* including gene probes derived from *entA_entl, entD, entR, oep, sagA;*
(x) *Klebsiella pneumonia* including gene probes derived from *cim, aldA, hemly, pSL017, pSL020, rcsA, rmlC, rmlD, waaG, wbbD, wbbM, wbbN, wbdA, wbdC, wztKpn, yibD;*
(xi) *P. aeruginosa* including gene probes derived from *aprA, aprE, ctx, algB, algN, algR, ExoS, fpvA, lasRa, lipA, lipH, Orf159, Orf252, pchG, PhzA, PhzB, PLC, plcN, plcR, pvdD, pvdF, pyocinS1, pyocinS1im, pyocinS2, pys2, rbf303, rhlA, rhlB, rhlR, TnAP41, toxA;*
(xii) *Streptococcus pneumoniae* including gene probes derived from *igaStrpneu, lytA, nanA, nanBStrpneu, pcpCStrpneu, ply, prtAStrpneu, pspA, SP0834Strpneu, sphtraStrpneu, wciJStrpneu, wziyStrpneu, wzxStrpneu;*
(xiii) *Streptococcus agalactiae* including gene probes derived from *CAMPfactor, 0499Straga, hylStragal, lipStragal;*
(xiv) *Streptococcus pyogenes* including gene probes derived from *DNaselStrpyog, fba2Strpyog, fhuAStrpyog, fhuB1Strpyog, fhuDStrpyog, fhuGStrpyog, hylA, hylP, hylp2, oppB, ropB, scpAStrpyog, sloStrpyog, smez-Strpyog, sof, speA, speB2Strpyog, speCStrpyog, speJStrpyog, srtBStrpyog, srtCStrpyog, srtEStrpyog, srtFStrpyog, srtGStrpyog, srtlStrpyog, srtKStrpyog, srtRStrpyog, srtTStrpyog, vicKStrpyog;*
(xvi) *Streptococcus viridans* including gene probes derived from *hlyXStrmut, igaStrmitis, igaStrsanguis, perMStrmut;*
(xvii) *Proteus mirabilis* including gene probes derived from *flaA, laD, fliA, hpmA, hpmB, IpsPrmi, mrpA, mrpB, mrpC, mrpD, mrpE, mrpF, mrpG, mrpH, mrpI, mrpJ, patA, putA, uca, ureDPrmi, ureEPrmi, ureFPrmi, zapA, zapB, zapD, zapE.*

Preferably, the resistance specific set of gene probes is selected from resistance gene probes (c) derived from genes coding for
(i) beta-lactams resistance including gene probes derived from *blalMP-7, meclSepid, blaOXA-10, blaB, ampC, I-blaR, blaOXA-32, bla-CTX-M-22, pbp2aStrpneu, blaSHV-1, blaOXA-2, blaRShaemolyt, blalMP-7, I-mecR, blaOXY, dacCStrpyog, femA, mecA, blalShaemolyt, blavim, pbp2b, pbp2prim eSepid, pbp2x, pbp3Saureuc, pbp4, pbp5Efaecium, pbpC, I-mecl, pbp1a, I-blal, blaTEM-106, blaOXY-KLOX, ftsWEF, fmhB, cumA, femBShaemolyt, blaPER-1, bla_FOX-3, blaA, psrb, fmhA, mecR1Sepid, blaZ, blaOXA-1, fox-6, blaPrmi;*
(ii) aminoglycosides resistance including gene probes derived from *aacA_aphDStwar, aacC1, aacC2, strB, aadA, aadB, aadD, aacA4, strA, aph-A3, aacC1, aacA4, aacA-aphD, I-spc, aphA3;*
(iii) macrolides-lincosamines-streptogramins resistance including gene probes derived from *ermC, linB, satSA, mdrSA, I-linA, ermB, ermA, satA, msrA, mphBM, mefA, mrx;*
(iv) trim ethoprim resistance including gene probes derived from *dfrA, dfrStrpneu;*
(v) chloramphenicol resistance including gene probes derived from *cat, catEfaecium, cmlA5;*
(vi) tetracyclines resistance including gene probes derived from *tetAJ, tetL, tetM*
(vii) glycopeptides resistance including gene probes derived from *vanH(tn), vanA, vanHB2, vanR, vanRB2, vanS(tn), vanSB2, vanVllB2, ddl, ble, vanXB2, vanY(tn), vanYB2, vanB, vanZ(tn), vanC-2, vanX(tn);*
(viii) multiple target resistance including gene probes derived from *acrB, m exB, I-qacA, sull, sul, cadBStalugd, mexA, acrR, emeA, acrA, rtn, abcXStrpmut, qacEdelta1, elkT-abcA, 1-cadA, albA, wzm, msrCb, nov, wzt, wbbl, norA23, mexR, arr2, mreA, I-cadC, uvrA;*
(ix) fungicides resistance, especially *C. albicans* fungicide resistance, including gene probes derived from *CRD2, CDR1, MET3, FET3, FTR2, MDR1-7, ERG11, SEC20.*

Furthermore, the microarray may contain a set of gene probes which serve as controls. Preferably, such a set of control gene probes is selected from group (d) consisting of control gene probes coding for
(i) negative controls, namely DNA sequences which will not hybridise with human DNA or bacterial, fungal or the microbial target DNA under the hybridisation conditions of the method of present invention, including gene probes derived neither from fungal, bacterial or target microbial nor from human genes, preferably gene probes derived from plant genes, more preferably from *Arabidopsis thaliana* or *Glycine max* genes;
(ii) positive controls including segments of ribosomal DNA from bacterial target species, preferably 16S DNA, and segments of conserved human genes;
(iii) positive controls specific for DNA added to the sample ("spiked DNA"), namely DNA sequences which will not hybridise with human DNA or the fungal, bacterial or microbial target DNA under the hybridisation conditions of the method of present invention, including gene probes derived neither from fungal, bacterial or target microbial nor from human genes, preferably gene probes derived from mouse or amoeba genes, most preferably from *Mus musculus* or *Dictyostelium discoideum* genes.

These control gene probes are necessary to
a) detect non-specific hybridisation;
b) optimise hybridisation conditions and image acquisition and analysis;
c) provide positive controls for the quality of probe preparation, hybridisation and detection; and/or
d) control technical aspects of the entire detection procedure including labelling, hybridisation and detection steps.

In a preferred aspect of embodiment (1), the microarray contains DNA sequences selected from the group consisting of the SEQ ID NOs: 1-918, complementary sequences thereto, addition mutants, deletion mutants, substitution mutants and homologues thereof as gene probes.

More preferably, in order to identify a specific microbial species, bacterial species or group of bacteria, the gene probes of group (a) are selected from SEQ ID NO: 1-99, 142-152, 174-199, 209-214, 216-219, 222-229, 231-291, 308-342, 377-393, 399-431, 449-490, 523-591, 606-639, 645-656, 687-701, 706-749 and 776-781 (compare Tab. 1). Equally, in order to determine virulence of a specific micororganism or bacterial species, the gene probes of group (b) are selected from SEQ ID NO: 100-141, 153-173, 200-208, 215, 220-221 , 230, 292-307, 343-376, 394-398, 432-448, 491-522, 592-605, 640-644, 657-686, 702-705, 750-775 and 782-784 (compare Tab. 1). Equally, in order to determine antibiotic resistance of a specific microbial or bacterial species, the gene probes of group (c) are selected from SEQ I D NO:785-918, preferably from SEQ ID NO:785-882 (compare Tab. 1). Equally, in order to provide the required controls (negative, positive, hybridisation controls), the gene probes of group (d) are selected from SEQ ID NO:919-947, preferably from SEQ I D NO:919-925 and 944-947, more preferably from SEQ I D NO: 919 and 921 (compare Tab. 1).

Tab. 1: Preferred gene probes for species identification, virulence determination and resistance determination of microorganisms
a) probes for species identification

| SEQ ID NO | Probe |
|---|---|
| *Staphylococcus aureus* identification | |
| 1 | cataSaur_1_1 |
| 2 | cataSaur_1_2 |
| 3 | clfA_1_1 |
| 4 | clfB_1_1 |
| 5 | coa_1_1 |
| 6 | coa_1_2 |
| 7 | I-clpC_1_1 |
| 8 | I-clpP_1_1 |
| 9 | I-ctaA_1_1 |
| 10 | I-ctsR_1_1 |
| 11 | I-dltA_1_1 |
| 12 | I-dltB_1_1 |
| 13 | I-dltC_1_1 |
| 14 | I-dnaK_1_1 |
| 15 | I-elkT_1_1 |
| 16 | I-femD_1_1 |
| 17 | I-glnA_1_1 |
| 18 | I-glnR_1_1 |
| 19 | I-qrlA_1_1 |
| 20 | I-grlB_1_1 |
| 21 | I-groEL_1_1 |
| 22 | I-groES_1_1 |
| 23 | I-hemA_1_1 |
| 24 | I-hemE_1_1 |
| 25 | I-hemH_1_1 |
| 26 | I-hemL_1_1 |
| 27 | I-hemY_1_1 |
| 28 | I-lepA_1_1 |
| 29 | I-IrgA_1_1 |
| 30 | I-IrgB_1_1 |
| 31 | I-lytM_1_1 |
| 32 | I-menB_1_1 |
| 33 | I-menD_1_1 |
| 34 | I-menE_1_1 |
| 35 | I-menF_1_1 |
| 36 | I-mreB_1_1 |
| 37 | I-mreR_1_1 |
| 38 | I-mutL_1_1 |
| 39 | I-mutS_1_1 |
| 40 | I-NAG_1_1 |
| 41 | I-pbg_1_1 |
| 42 | I-pbpF_1_1 |
| 43 | I-pdhB_1_1 |
| 44 | I-pdhC_1_1 |
| 45 | I-rsbU_1_1 |
| 46 | I-rsbV_1_1 |
| 47 | I-rsbW_1_1 |
| 48 | I-sgp_1_1 |
| 49 | I-sirR_1_1 |
| 50 | I-sodA_1_1 |
| 51 | I-sodB_1_1 |
| 52 | I-sstA_1_1 |
| 53 | I-sstB_1_1 |
| 54 | I-sstC_1_1 |
| 55 | I-sstD_1_1 |
| 56 | I-trx_1_1 |
| 57 | I-yhiN_1_1 |
| 58 | epiP-bsaP_1_1 |
| 59 | geh_1_1 |
| 60 | gyrA_1_1 |
| 61 | gyrB_1_1 |
| 62 | hemB_1_1 |
| 63 | hemC_1_1 |
| 64 | hemD_1_1 |
| 65 | hemN_1_1 |
| 66 | hsdS_1_1 |
| 67 | hsdS_2_1 |
| 68 | lip_1_1 |
| 69 | menC_1_1 |
| 70 | murC_1_1 |
| 71 | nuc_1_1 |
| 72 | pdhD_1_1 |
| 73 | rpoB_1_1 |
| 74 | SAV0431_1_1 |
| 75 | SAV0439_1_1 |
| 76 | SAV0440_1_1 |
| 77 | SAV0441_1_1 |
| 78 | sigB_1_1 |
| 79 | spa_1_2 |
| 80 | sstC_1_1 |
| 81 | tag_1_1 |
| 82 | tyrA_1_1 |
| 83 | I-aroC_1_1 |
| 84 | I-aroA_1_1 |
| 85 | I-cna_1_1 |
| 86 | I-ebpS_1_1 |
| 87 | I-eno_1_1 |
| 88 | I-fbpA_1_1 |
| 89 | I-fib_1_1 |
| 90 | I-fnbB_1_1 |
| 91 | I-srtA_1_1 |
| 92 | I-stpC_1 _1 |
| 93 | I-fnbA_1 _1 |
| 94 | I-spa_1_1 |
| 95 | I-aroE_1_1 |
| 96 | I-aroF_1_1 |
| 97 | I-aroG_1_1 |
| 98 | I-asp23_1_1 |
| 99 | I-atl_1_1 |

| *Escherichia coli* identification | |
|---|---|
| 142 | b1169_1_1 |
| 143 | envZ_1_1 |
| 144 | fliCb_1_1 |
| 145 | nfrB_1_1 |
| 146 | nlpA_1_1 |
| 147 | pilAe_1_1 |
| 148 | yacH_1_1 |
| 149 | yagX_1_1 |
| 150 | ycdS_1_1 |
| 151 | yciQ_1_1 |
| 152 | ymcA_1_1 |

| *Staphylococcus epidermidis* identification | |
|---|---|
| 174 | ardeSE0106_1_1 |
| 175 | ardeSE0107_1_1 |
| 176 | aroiSE0105_1_1 |
| 177 | atlE_1_1 |
| 178 | agrB_1_1 |
| 179 | agrC_1_1 |
| 180 | alphSE1368_1_1 |
| 181 | gad_1_1 |
| 182 | glucSE1191_1_1 |
| 183 | hspl0_1_1 |
| 184 | icaA_1_1 |
| 185 | icaB_1_1 |
| 186 | mvaSSepid_1_1 |
| 187 | nitreSE1972_1_1 |
| 188 | nitreSE1974_1_1 |
| 189 | nitreSE1975_1_1 |
| 190 | oiamtSE1209_1_1 |
| 191 | ORF1Sepid_1_1 |
| 192 | ORF3bSepid_1_1 |
| 193 | qacR_1_1 |
| 194 | sin_1_1 |
| 195 | ureSE1861_1_1 |
| 196 | ureSE1863_1_1 |
| 197 | ureSE1864_1_1 |
| 198 | ureSE1865_1_1 |
| 199 | ureSE1867_1_1 |

| *Staphylococcus haemolyticus* identification | |
|---|---|
| 209 | folQShaemolyt_1_1 |
| 210 | mvaCShaemolyticus_1_1 |
| 211 | mvaDShaemolyt_1_1 |
| 212 | mvaK1 Shaemolyticus_1_1 |
| 213 | mvaSShaemolyticus_1_1 |
| 214 | RNApolsigm_1_1 |

| *Staphylococcus lugdunensis* identification | |
|---|---|
| 216 | agrB2Stalugd_1_1 |
| 217 | agrC2Stalugd_1_1 |
| 218 | agrCStalugd_1_1 |
| 219 | slamStalugd_1_1 |

| *Staphylococcus saprophyticus* identification | |
|---|---|
| 222 | RNApoIsigmSsapro_1_1 |
| 223 | RNApolsigmSsapro_1_2 |

| *Staphylococcus warneri* identification | |
|---|---|
| 224 | msrw1Stwar_1_1 |
| 225 | nukMStwar_1_1 |
| 226 | proDStwar_1_1 |
| 227 | proMStwar_1_1 |
| 228 | sigrpoStwar_1_1 |
| 229 | tnpStwar_1_1 |

| *Candida albicans* identification | |
|---|---|
| 231 | ARG56_1_1 |
| 232 | ASL43f_1_1 |
| 233 | BGL2_1_1 |
| 234 | CACHS3_1_1 |
| 235 | CCT8_1_1 |
| 236 | CDC37_1_1 |
| 237 | CEF3_1_1 |
| 238 | CHS1_1_1 |
| 239 | CHS2_1_1 |
| 240 | CHS4_1_1 |
| 241 | CHS5_1_1 |
| 242 | CHT1_1_1 |
| 243 | CHT2_1_1 |
| 244 | CHT4_1_1 |
| 245 | CSA1_1_1 |
| 246 | 5triphosphatase_1_1 |
| 247 | AAF1_1_1 |
| 248 | ADH1_1_1 |
| 249 | ALS1_1_1 |
| 250 | ALS7_1_1 |
| 251 | EDT1_1_1 |
| 252 | ELF_1_1 |
| 253 | ESS1_1_1 |
| 254 | FAL1_1_1 |
| 255 | GAP1_1_1 |
| 256 | GNA1_1_1 |
| 257 | GSC1_1_1 |
| 258 | GSL1_1_1 |
| 259 | HIS1_1_1 |
| 260 | HTS1_1_1 |
| 261 | HWP1_2_1 |
| 262 | HYR1_1_1 |
| 263 | INT1a_1_1 |
| 264 | KRE15f_1_1 |
| 265 | KRE6_1_1 |
| 266 | KRE9_1_1 |
| 267 | MIG1_1_1 |
| 268 | MLS_1_1 |
| 269 | MP65_1_1 |
| 270 | NDE1_1_1 |
| 271 | PFK2_1_1 |
| 272 | PHR1_1_1 |
| 273 | PHR2_1_1 |
| 274 | PHR3_1_1 |
| 275 | PRA1_1_1 |
| 276 | PRS_1_1 |
| 277 | RBT1_1_1 |
| 278 | RBT4_1_1 |
| 279 | RHO1_1_1 |
| 280 | RNR1_1_1 |
| 281 | RPB7_1_1 |
| 282 | RPL13_1_1 |
| 283 | RVS167_1_1 |
| 284 | SHA3_1_1 |
| 285 | SKN1_1_1 |
| 286 | SRB1_1_1 |
| 287 | TCA1_1_1 |
| 288 | TRP1_1_1 |
| 289 | YAE1_1_1 |
| 290 | YRB1_1_1 |
| 291 | YST1exon2_1_1 |

| *Enterococcus faecalis* identification | |
|---|---|
| 308 | arcA_1_1 |
| 309 | arcC_1_1 |
| 310 | bkdA_1_1 |
| 311 | cad_1_1 |
| 312 | camE1_1_1 |
| 313 | csrA_1_1 |
| 314 | dacA_1_1 |
| 315 | dfr_1_1 |
| 316 | dhoD1a_1_1 |
| 317 | ABC-eltA_1_1 |
| 318 | agrBfs_1_1 |
| 319 | agrCfs_1_1 |
| 320 | dnaE_1_1 |
| 321 | ebsA_1_1 |
| 322 | ebsB_1_1 |
| 323 | eep_1_1 |
| 324 | efaR_1_1 |
| 325 | gls24_glsB_1_1 |
| 326 | gph_1_1 |
| 327 | gyrAEf_1_1 |
| 328 | metEf_1_1 |
| 329 | mntHCb2_1_1 |
| 330 | mob2_1_1 |
| 331 | mvaD_1_1 |
| 332 | mvaE_1_1 |
| 333 | parC_1_1 |
| 334 | pcfG_1_1 |
| 335 | phoZ_1_1 |
| 336 | polC_1_1 |
| 337 | ptb_1_1 |
| 338 | recS1_1_1 |
| 339 | rpoN_1_1 |
| 340 | tms_1_1 |
| 341 | tyrDC_1_1 |
| 342 | tyrS_1_1 |

| *Enterococcus faecium* identification | |
|---|---|
| 377 | bglB_1_1 |
| 378 | bglR_1_1 |
| 379 | bglS_1_1 |
| 380 | efmA_1_1 |
| 381 | efmB_1_1 |
| 382 | efmC_1_1 |
| 383 | mreC_1_1 |
| 384 | mreD_1_1 |
| 385 | mvaDEfaecium_1_1 |
| 386 | mvaEEfaecium_1_1 |
| 387 | mvaK1Efaecium_1_1 |
| 388 | mvaK2Efaecium_1_1 |
| 389 | mvaSEfaecium_1_1 |
| 390 | orf3_4Efaeciumb_1_1 |
| 391 | orf6_7Efaecium_1_1 |
| 392 | orf7_8Efaecium_1_1 |
| 393 | orf9_10Efaecium_1_1 |

| *Klebsiella pneumoniae* identification | |
|---|---|
| 399 | atsA_1_1 |
| 400 | atsB_1_1 |
| 401 | budC_1_1 |
| 402 | citA_1_1 |
| 403 | citW_1_1 |
| 404 | citX_1_1 |
| 405 | dalD_1_1 |
| 406 | dalK_1_1 |
| 407 | dalT_1 _1 |
| 408 | acoA_1_1 |
| 409 | acoB_1_1 |
| 410 | acoC_1_1 |
| 411 | ahlK_1_1 |
| 412 | fimK_1_1 |
| 413 | glfKPN2_1_1 |
| 414 | ltrA_1_1 |
| 415 | mdcC_1_1 |
| 416 | mdcF_1_1 |
| 417 | mdcH_1_1 |
| 418 | mrkA_1_1 |
| 419 | mtrK_1_1 |
| 420 | nifF_1_1 |
| 421 | nifK_1_1 |
| 422 | nifN_1_1 |
| 423 | tyrP_1_1 |
| 424 | ureA_1_1 |
| 425 | wbbO_1_1 |
| 426 | wza_1_1 |
| 427 | wzb_1_1 |
| 428 | wzmKPN2_1_1 |
| 429 | wztKPN2_1_1 |
| 430 | yojH_1_1 |
| 431 | liac_1_1 |

| *Klebsiella oxytoca* identification | |
|---|---|
| 449 | cymA_1_1 |
| 450 | cymD_1_1 |
| 451 | cymE_1_1 |
| 452 | cymH_1_1 |
| 453 | cyml_1_1 |
| 454 | cymJ_1_1 |
| 455 | ddrA_1_1 |
| 456 | fdt-1_1_1 |
| 457 | fdt-2_1_1 |
| 458 | fdt-3_1_1 |
| 459 | gatY_1_1 |
| 460 | hydH_1_1 |
| 461 | masA_1_1 |
| 462 | nasA_1_1 |
| 463 | nasE_1_1 |
| 464 | nasF_1_1 |
| 465 | pehX_1_1 |
| 466 | pelX_1_1 |
| 467 | tagH_1_1 |
| 468 | tagK_1_1 |
| 469 | tagT_1_1 |

| *Pseudomonas aeruginosa* identification | |
|---|---|
| 470 | glpR_1_1 |
| 471 | lasRb_1_1 |
| 472 | OrfX_1_1 |
| 473 | pa0260_1_1 |
| 474 | pa0572_1_1 |
| 475 | pa0625_1_1 |
| 476 | pa0636_1_1 |
| 477 | pa1046_1_1 |
| 478 | pa1069_1_1 |
| 479 | pa1846_1_1 |
| 480 | pa3866_1_1 |
| 481 | pa4082_1_1 |
| 482 | pilAp_1_1 |
| 483 | PilAp2_1_1 |
| 484 | pilC_1_1 |
| 485 | PstP_1_1 |
| 486 | purK_1_1 |
| 487 | uvrDII_1_1 |
| 488 | vsmI_1_1 |
| 489 | vsmR_1_2 |
| 490 | xcpX_1_1 |

| *Streptococcus pneumoniae* identification | |
|---|---|
| 523 | cap1EStrpneu_1_1 |
| 524 | cap1FStrpneu_1_1 |
| 525 | cap1GStrpneu_1_1 |
| 526 | cap3AStrpneu_1_1 |
| 527 | cap3BStrpneu_1_1 |
| 528 | celAStrpneu_1_1 |
| 529 | celBStrpneu_1_1 |
| 530 | cglAStrpneu_1_1 |
| 531 | cglBStrpneu_1_1 |
| 532 | cglCStrpneu_1_1 |
| 533 | cgIDStrpneu_1_1 |
| 534 | cinA_1_1 |
| 535 | cps14EStrpneum_1_1 |
| 536 | cps14FStrpneum_1_1 |
| 537 | cps14GStrpneum_1_1 |
| 538 | cps14HStrpneum_1_1 |
| 539 | cps19aHStrpneum_1_1 |
| 540 | cps19alStrpneum_1_1 |
| 541 | cps19aKStrpneum_1_1 |
| 542 | cps19fGStrpneum_1_1 |
| 543 | cps23fGStrpneum_1_1 |
| 544 | dexB_1_1 |
| 545 | dinF_1_1 |
| 546 | 1760Strpneu_1_1 |
| 547 | acyPStrpneu_1_1 |
| 548 | endAStrpneu_1_1 |
| 549 | exoAStrpneu_1_1 |
| 550 | exp72_1_1 |
| 551 | fnlAStrpneu_1_1 |
| 552 | fnlBStrpneu_1_1 |
| 553 | fnlCStrpneu_1_1 |
| 554 | gct18Strpneum_1_1 |
| 555 | hexB1_1_1 |
| 556 | hftsHstrpneu_1_1 |
| 557 | immunofrag1Strpneu_1_1 |
| 558 | immunofrag2Strpneu_2_1 |
| 559 | immunofraq3Strpneu_2_1 |
| 560 | kdtBStrpneu_1_1 |
| 561 | lysAStrpneu_1_1 |
| 562 | pcpBStrpneu_1_1 |
| 563 | pflCStrpneu_1_1 |
| 564 | plpA_1_1 |
| 565 | prtA1Strpneu_1_1 |
| 566 | pspC1Strpneu_1_1 |
| 567 | pspC2_1_1 |
| 568 | purRStrpneu_1_1 |
| 569 | pyrDAStrpneum_1_1 |
| 570 | SP0828Strpneu_1_1 |
| 571 | SP0830Strpneu_1_1 |
| 572 | SP0833Strpneu_1_1 |
| 573 | SP0837_38Strpneu_1_1 |
| 574 | SP0839Strpneu_1_1 |
| 575 | ugdStrpneu_1_1 |
| 576 | uncC_1_1 |
| 577 | vicXStrepneu_1_1 |
| 578 | wchA6bStrpneum_1_1 |
| 579 | wci4Strpneum_1_1 |
| 580 | wciK4Strpneum_1_1 |
| 581 | wciL4Strpneum_1_1 |
| 582 | wciN6bStrpneum_1_1 |
| 583 | wciO6bStrpneum_1_1 |
| 584 | wciP6bStrpneum_1_1 |
| 585 | wciY18Strpneum_1_1 |
| 586 | wzdbStrpneum_1_1 |
| 587 | wze6bStrpneum_1_1 |
| 588 | wzy18Strpneum_1_1 |
| 589 | wzy4Strpneum_1_1 |
| 590 | wzy6bStrpneum_1_1 |
| 591 | xpt_1_1 |

| *Streptococcus agalactiae* identification | |
|---|---|
| 606 | cpsA1Strqal_1_1 |
| 607 | cpsB1Strgal_1_1 |
| 608 | cpsC1Strgal_1_1 |
| 609 | cpsD1Strgal_1_1 |
| 610 | cpsE1Strgal_1_1 |
| 611 | cpsG1Strgal_1_1 |
| 612 | cpsIStragal_1_1 |
| 613 | cpsJStragal_1_1 |
| 614 | cpsKStraqal_1_1 |
| 615 | cpsMStragal_1_1 |
| 616 | cpsYStragal_1_1 |
| 617 | cpsYStragal_2_1 |
| 618 | cyIBStraga_1_1 |
| 619 | cylEStraga_1_1 |
| 620 | cylFStraga_1_1 |
| 621 | cylHStraga_1_1 |
| 622 | cylIStraga_1_1 |
| 623 | cylJStraga_1_1 |
| 624 | cylKStraga_1_1 |
| 625 | 0487Straga_1_1 |
| 626 | 0488Straga_1_1 |
| 627 | 0493Straga_1_1 |
| 628 | 0495Straga_1_1 |
| 629 | 0498Straga_1_1 |
| 630 | 0500Straga_1_1 |
| 631 | 0502Straga_1_1 |
| 632 | 0504Straga_1_1 |
| 633 | foIDStraga_1_1 |
| 634 | neuA1Strgal_1_1 |
| 635 | neuB1Strgal_1_1 |
| 636 | neuC1Strgal_1_1 |
| 637 | neuD1Strgal_1_1 |
| 638 | recNStraga_1_1 |
| 639 | ileSStraga_1_1 |

| *Streptococcus pyogenes identification* | |
|---|---|
| 645 | cyclStrpyog_1_1 |
| 646 | fah_rph_hlo_Strpyog_1_1 |
| 647 | int_1_1 |
| 648 | int315.5_1_1 |
| 649 | murEStrpyog_1_1 |
| 650 | oppA_1_1 |
| 651 | oppCStrpyog_1_1 |
| 652 | oppD_1_1 |
| 653 | SPy0382Strpyog_1_1 |
| 654 | SPy0390Strpyog_1_1 |
| 655 | SpyM3_1351_1_1 |
| 656 | vicXStrpyog_1_1 |

| *Streptococcus viridans* identification | |
|---|---|
| 687 | 573Stprmut_1_1 |
| 688 | 580SStprmut_1_1 |
| 689 | 581_582SStprmut_1_1 |
| 690 | 584SStprmut_1_1 |
| 691 | dltAStrmut_1_1 |
| 692 | dItBStrmut_1_1 |
| 693 | dltCppx1Strmut_1_1 |
| 694 | dItDStrmut_1_1 |
| 695 | lichStrbov_1_1 |
| 696 | lytRStprmut_1_1 |
| 697 | lytSStprmut_1_1 |
| 698 | pepQStrrmut_1_1 |
| 699 | pflCStrmut_1_1 |
| 700 | recNStprmut_1_1 |
| 701 | ytqBStrmut_1_1 |

| *Proteus mirabilis* identification | |
|---|---|
| 706 | atfA_1_1 |
| 707 | atfB_1_1 |
| 708 | atfC_1_1 |
| 709 | ccmPrmi1_1_1 |
| 710 | cyaPrmi_1_1 |
| 711 | aad_1_1 |
| 712 | flfB_1_1 |
| 713 | flfD_1_1 |
| 714 | flfN_1_1 |
| 715 | flhD_1_1 |
| 716 | floA_1_1 |
| 717 | ftsK_1_1 |
| 718 | gstB_1_1 |
| 719 | hemCPrmi_1_1 |
| 720 | hemDPrmi_1_1 |
| 721 | hev_1_1 |
| 722 | katA_1_1 |
| 723 | lpp1_1_1 |
| 724 | menE_1_1 |
| 725 | mfd_1_1 |
| 726 | nrpA_1_1 |
| 727 | nrpB_1_1 |
| 728 | nrpG_1_1 |
| 729 | nrpS_1_1 |
| 730 | nrpT_1_1 |
| 731 | nrpU_1_1 |
| 732 | pat_1_1 |
| 733 | pmfA_1_1 |
| 734 | pmfC_1_1 |
| 735 | pmfE_1_1 |
| 736 | ppaA_1_1 |
| 737 | rsbA_1_1 |
| 738 | rsbC_1_1 |
| 739 | speB_1_1 |
| 740 | stmA_1_1 |
| 741 | stmB_1_1 |
| 742 | terA_1_1 |
| 743 | terD_1_1 |
| 744 | umoA_1_1 |
| 745 | umoB_1 _1 |
| 746 | umoC_1_1 |
| 747 | ureR_1_1 |
| 748 | xerC_1_1 |
| 749 | ygbA_1_1 |

| *Proteus vulgaris* identification | |
|---|---|
| 776 | envZPrvu_1_1 |
| 777 | frdC_1_1 |
| 778 | frdD_1_1 |
| 779 | infBPrvu_1_1 |
| 780 | lad_1_1 |
| 781 | tna2_1_1 |

b) virulence gene probes

| SEQ ID NO | Probe |
|---|---|
| *Staphylococcus aureus* virulence | |
| 100 | bsaE_1_1 |
| 101 | bsaG_1_1 |
| 102 | cap5h_1_1 |
| 103 | cap5i_1_1 |
| 104 | cap5j_1_1 |
| 105 | cap5k_1_1 |
| 106 | cap8H_1_1 |
| 107 | cap81_1_1 |
| 108 | cap8J_1_1 |
| 109 | cap8K_1_1 |
| 110 | I-hld_1_1 |
| 111 | I-hysA_1_1 |
| 112 | I-IgGbg_1_1 |
| 113 | EDIN_1_1 |
| 114 | eta_1_1 |
| 115 | etb_1_1 |
| 116 | hglA_1_1 |
| 117 | hglA_2_1 |
| 118 | hglB_1_1 |
| 119 | hglC_2_1 |
| 120 | hla_1_1 |
| 121 | hlb_1_2 |
| 122 | lukF_1_1 |
| 123 | lukS_1_1 |
| 124 | lukS_2_1 |
| 125 | NAG_1_1 |
| 126 | sak_1_1 |
| 127 | sea_1_1 |
| 128 | seb_1_1 |
| 129 | sec1_1_1 |
| 130 | seg_1_1 |
| 131 | seh_1_1 |
| 132 | sel_1_1 |
| 133 | set15_1_1 |
| 134 | set6_1_1 |
| 135 | set7_1_1 |
| 136 | set8_1_1 |
| 137 | sprV8_1_1 |
| 138 | tst_1_1 |
| 139 | I-sdrC_1_1 |
| 140 | I-sdrD_1_1 |
| 141 | I-sdrE_1_1 |

| *Escherichia coli* virulence | |
|---|---|
| 153 | b1202_1_1 |
| 154 | eae_1_1 |
| 155 | eltB_1_1 |
| 156 | escR_1_1 |
| 157 | escT_1_1 |
| 158 | escU_1_1 |
| 159 | espB_1_1 |
| 160 | fes_1_1 |
| 161 | fes_2_1 |
| 162 | fteA_1_1 |
| 163 | hlyA_1_1 |
| 164 | hlyB_1_1 |
| 165 | iucA_1_1 |
| 166 | iucB_1_1 |
| 167 | iucC_1_1 |
| 168 | papG_1_1 |
| 169 | rfbE_1_1 |
| 170 | shuA_1_1 |
| 171 | SLTII_1_1 |
| 172 | toxA-LTPA_1_1 |
| 173 | VT2vaB_1_1 |

| *Staphylococcus epidermidis* virulence | |
|---|---|
| 200 | gcaD_1_1 |
| 201 | hld_orf5_1_1 |
| 202 | icaC_1_1 |
| 203 | icaD_1_1 |
| 204 | icaR_1_1 |
| 205 | psm_beta1and2_1_1 |
| 206 | purR_1_1 |
| 207 | spoVG_1_1 |
| 208 | yabJ_1_1 |

| *Staphylococcus haemolyticus* virulence | |
|---|---|
| 215 | lipShaem olyt_1_1 |

| *Staphylococcus lugdunensis* virulence | |
|---|---|
| 220 | slushABCStalugd_1_1 |
| 221 | fblStalugd_1_1 |

| *Staphylococcus warneri* virulence | |
|---|---|
| 230 | gehAStwar_1_1 |

| *Candida albicans* virulence | |
|---|---|
| 292 | CCN1_1_1 |
| 293 | CDC28_1_1 |
| 294 | CLN2_1_1 |
| 295 | CPH1_1_1 |
| 296 | CYB1_1_1 |
| 297 | EFG1_1_1 |
| 298 | MNT1_1_1 |
| 299 | RBF1_1_1 |
| 300 | RBF1_2_1 |
| 301 | RIM101_1_1 |
| 302 | RIM8_1_1 |
| 303 | SEC14_1_1 |
| 304 | SEC4_1_1 |
| 305 | TUP1_1_1 |
| 306 | YPT1_1_1 |
| 307 | ZNF1CZF1_2_1 |

| *Enterococcus faecalis* virulence | |
|---|---|
| 343 | asa1_1_1 |
| 344 | asp1_1_1 |
| 345 | cgh_1_1 |
| 346 | cylA_1_1 |
| 347 | cylB_1_1 |
| 348 | cyll_1_1 |
| 349 | cylL_cylS_1_1 |
| 350 | cylM_1_1 |
| 351 | ace_1_1 |
| 352 | ef00108_1_1 |
| 353 | ef00109_1_1 |
| 354 | ef0011_1_1 |
| 355 | ef00113_1_1 |
| 356 | ef0012_1_1 |
| 357 | ef0022_1_1 |
| 358 | ef0031_1_1 |
| 359 | ef0032_1_1 |
| 360 | ef0040_1_1 |
| 361 | ef0058_1_1 |
| 362 | enlA_1_1 |
| 363 | esa_1_1 |
| 364 | esp_1_1 |
| 365 | gelE_1_1 |
| 366 | groEL_1_1 |
| 367 | groES_1_1 |
| 368 | rt1_1_1 |
| 369 | sala_1_1 |
| 370 | salb_1_1 |
| 371 | sea1_1_1 |
| 372 | sep1_1_1 |
| 373 | vicK_1_1 |
| 374 | yycH_1_1 |
| 375 | yycl_1_1 |
| 376 | yycJ_1_1 |

| *Enterococcus faecium* virulence | |
|---|---|
| 394 | entA_entl_1 _1 |
| 395 | entD_1_1 |
| 396 | entR_1_1 |
| 397 | oep_1_1 |
| 398 | sagA_1_2 |

| *Klebsiella pneumoniae* virulence | |
|---|---|
| 432 | cim_1_1 |
| 433 | aldA_1 _1 |
| 434 | aldA_2_1 |
| 435 | hemly_1_1 |
| 436 | pSL017_1_1 |
| 437 | pSL020_1_1 |
| 438 | rcsA_1_1 |
| 439 | rmlC_1_1 |
| 440 | rmlD_1_1 |
| 441 | waaG_1_1 |
| 442 | wbbD_1_1 |
| 443 | wbbM_1_1 |
| 444 | wbbN_1_1 |
| 445 | wbdA_1_1 |
| 446 | wbdC_1_1 |
| 447 | wztKpn_1_1 |
| 448 | yibD_1_1 |

| *Pseudomonas aeruginosa* virulence | |
|---|---|
| 491 | aprA_1_1 |
| 492 | aprE_1_1 |
| 493 | ctx_1_2 |
| 494 | algB_1_1 |
| 495 | algN_1_1 |
| 496 | algR_1_1 |
| 497 | ExoS_1_1 |
| 498 | fpvA_1_1 |
| 499 | lasRa_1_1 |
| 500 | lipA_1_1 |
| 501 | lipH_1_1 |
| 502 | Orf159_1_2 |
| 503 | Orf252_1_1 |
| 504 | pchG_1 _1 |
| 505 | PhzA_1_1 |
| 506 | PhzB_1_1 |
| 507 | PLC_1_1 |
| 508 | plcN_1_1 |
| 509 | plcR_1 _1 |
| 510 | pvdD_1_1 |
| 511 | pvdF_1_2 |
| 512 | pyocinS1_1_1 |
| 513 | pyocinS1im_1_1 |
| 514 | pyocinS2_1 _1 |
| 515 | pys2_1_1 |
| 516 | pys2_2_1 |
| 517 | rbf303_1 _1 |
| 518 | rhlA_1_1 |
| 519 | rhlB_1_1 |
| 520 | rhlR_1_1 |
| 521 | TnAP41_1_2 |
| 522 | toxA_1_1 |

| *Streptococcus pneumoniae* virulence | |
|---|---|
| 592 | igaStrpneu_1_1 |
| 593 | lytA_1_1 |
| 594 | nanA_1_1 |
| 595 | nanBStrpneu_1_1 |
| 596 | pcpCStrpneu_1_1 |
| 597 | ply_1_1 |
| 598 | prtAStrpneu_1_1 |
| 599 | pspA_1_2 |
| 600 | SP0834Strpneu_1_1 |
| 601 | SP0834Strpneu_1_2 |
| 602 | sphtraStrpneu_1_1 |
| 603 | wciJStrpneu_1_1 |
| 604 | wziyStrpneu_1_1 |
| 605 | wzxStrpneu_1_1 |

| *Streptococcus agalactiae* virulence | |
|---|---|
| 640 | CAMPfactor_1_1 |
| 641 | CAMPfactor_2_1 |
| 642 | 0499Straqa_1_1 |
| 643 | hylStragal_1_1 |
| 644 | lipStragal_1_1 |

| *Streptococcus pyogenes* virulence | |
|---|---|
| 657 | DNaselStrpyog_1_1 |
| 658 | fba2Strpyog_1_1 |
| 659 | fhuAStrpyog_1_1 |
| 660 | fhuB1Strpyog_1_1 |
| 661 | fhuDStrpyog_1_1 |
| 662 | fhuGStrpyog_1_1 |
| 663 | hylA_1_1 |
| 664 | hylP_1_1 |
| 665 | hylp2_1_1 |
| 666 | oppB_1_1 |
| 667 | ropB_1_1 |
| 668 | scpAStrpyog_1_1 |
| 669 | sloStrpyog_1_1 |
| 670 | smez-4Strpyog_1_1 |
| 671 | sof_1_1 |
| 672 | sof_2_1 |
| 673 | speA_1_1 |
| 674 | speB2Strpyog_1_1 |
| 675 | speCStrpyog_1_1 |
| 676 | speJStrpyog_1_1 |
| 677 | srtBStrpyog_1_1 |
| 678 | srtCStrpyog_1_1 |
| 679 | srtEStrpyog_1_1 |
| 680 | srtFStrpyog_1_1 |
| 681 | srtGStrpyog_1_1 |
| 682 | srtlStrpyog_1_1 |
| 683 | srtKStrpyog_1_1 |
| 684 | srtRStrpyog_1_1 |
| 685 | srtTStrpyog_1_1 |
| 686 | vicKStrpyog_1_1 |

| *Streptococcus viridans* virulence | |
|---|---|
| 702 | hlyXStrmut_1_1 |
| 703 | igaStrmitis_1_1 |
| 704 | igaStrsanguis_1_1 |
| 705 | perMStrmut_1_1 |

| *Proteus mirabilis* virulence | |
|---|---|
| 750 | flaA_1_1 |
| 751 | flaD_1_1 |
| 752 | fliA_1_1 |
| 753 | hpmA_1_1 |
| 754 | hpmB_1_1 |
| 755 | IpsPrmi_1_1 |
| 756 | mrpA_1_1 |
| 757 | mrpB_1_1 |
| 758 | mrpC_1_1 |
| 759 | mrpD_1_1 |
| 760 | mrpE_1_1 |
| 761 | mrpF_1_1 |
| 762 | mrpG_1_1 |
| 763 | mrpH_1_1 |
| 764 | mrpI_1_1 |
| 765 | mrpJ_1_1 |
| 766 | patA_1_1 |
| 767 | putA_1_1 |
| 768 | uca_1_1 |
| 769 | ureDPrmi_1_1 |
| 770 | ureEPrmi_1_1 |
| 771 | ureFPrmi_1_1 |
| 772 | zapA_1_1 |
| 773 | zapB_1_1 |
| 774 | zapD_1_1 |
| 775 | zapE_1_1 |

| *Proteus vulgaris* virulence | |
|---|---|
| 782 | end_1_1 |
| 783 | pqrA_1_1 |
| 784 | urg_1_1 |

c) resistance gene probes

| SEQ ID NO | Probe |
|---|---|
| Beta-lactams resistance | |
| 785 | blalMP-7_1_1 |
| 786 | meclSepid_1_1 |
| 787 | blaOXA-10_1_2 |
| 788 | blaB_1_1 |
| 789 | ampC_1_1 |
| 790 | I-blaR_1_1 |
| 791 | blaOXA-32_1_1 |
| 792 | bla-CTX-M-22_1_1 |
| 793 | pbp2aStrpneu_1_1 |
| 794 | blaSHV-1_1_1 |
| 795 | blaOXA-2_1_1 |
| 796 | blaRShaemolyt_1_1 |
| 797 | blalMP-7_1_2 |
| 798 | I-mecR_1_1 |
| 799 | blaOXY_1_1 |
| 800 | dacCStrpyog_1_1 |
| 801 | femA_1_1 |
| 802 | mecA_1_1 |
| 803 | blalShaemolyt_1_1 |
| 804 | blavim_1_1 |
| 805 | pbp2b_1_1 |
| 806 | pbp2primeSepid_1_1 |
| 807 | pbp2x_1_1 |
| 808 | pbp3Saureuc_1_1 |
| 809 | pbp4_1_1 |
| 810 | pbp5Efaecium_1_1 |
| 811 | pbpC_1_1 |
| 812 | I-mecl_1_1 |
| 813 | pbp1a_1_1 |
| 814 | I-blal_1_1 |
| 815 | blaTEM-106_1_1 |
| 816 | blaOXY-KLOX_1_1 |
| 817 | ftsWEF_1_1 |
| 818 | fmhB_1_1 |
| 819 | cumA_1_1 |
| 820 | fem BShaem olyt_1_1 |
| 821 | blaPER-1_1_1 |
| 822 | bla_FOX-3_1_1 |
| 823 | blaA_1_1 |
| 824 | psrb_1_1 |
| 825 | fmhA_1_1 |
| 826 | mecRiSepid_1_1 |
| 827 | blaZ_1_1 |
| 828 | blaOXA-1_1_1 |
| 829 | fox-6_1_1 |
| 830 | blaPrmi_1_1 |

| Aminoglycosides resistance | |
|---|---|
| 831 | aacA_aphDStwar_1_1 |
| 832 | aacC1_1_2 |
| 833 | aacC2_1_1 |
| 834 | strB_1_1 |
| 835 | aadA_1_1 |
| 836 | aadB_1_2 |
| 837 | aadD_1_1 |
| 838 | aacA4_1_2 |
| 839 | strA_1_1 |
| 840 | aph-A3_1_1 |
| 841 | aacC1_1_1 |
| 842 | aacA4_1_1 |
| 843 | aacA-aphD_1_1 |
| 844 | l-spc_1_1 |
| 845 | aphA3_1_1 |

| Macrolide-Lincosamide-Streptogramin resistance | |
|---|---|
| 846 | ermC_1_1 |
| 847 | linB_1_1 |
| 848 | satSA_1_1 |
| 849 | mdrSA_1_1 |
| 850 | I-linA_1_1 |
| 851 | ermB_1_2 |
| 852 | ermA_1_1 |
| 853 | satA_1_1 |
| 854 | msrA_1_1 |
| 855 | mphBM_1_1 |
| 856 | mefA_1_1 |
| 857 | mrx_1_1 |

| Trymethoprim resistance | |
|---|---|
| 858 | dfrStrpneu_1_1 |
| 859 | dfrA_1_1 |

| Chloramphenicol resistance | |
|---|---|
| 860 | cmlA5_1_1 |
| 861 | catEfaecium_1_1 |
| 862 | cat_1_1 |

| Tetracyclines resistance | |
|---|---|
| 863 | tetAJ_1_1 |
| 864 | tetL_1_1 |
| 865 | tetM_1_1 |

| Glycopeptides resistance | |
|---|---|
| 866 | vanH(tn)_1_1 |
| 867 | vanA_1_1 |
| 868 | vanHB2_1_1 |
| 869 | vanR_1_1 |
| 870 | vanRB2_1_1 |
| 871 | vanS(tn)_1_1 |
| 872 | vanSB2_1_1 |
| 873 | vanWB2_1_1 |
| 874 | ddl_1_1 |
| 875 | ble_1_1 |
| 876 | vanXB2_1_1 |
| 877 | vanY(tn)_1_1 |
| 878 | vanYB2_1_1 |
| 879 | vanB_1_1 |
| 880 | vanZ(tn)_1_1 |
| 881 | vanC-2_1_1 |
| 882 | vanX(tn)_1_1 |

| Other / multiple substances resistance | |
|---|---|
| 883 | acrB_1_1 |
| 884 | mexB_1_2 |
| 885 | I-qacA_1_1 |
| 886 | sull_1_1 |
| 887 | sul_1_1 |
| 888 | cadBStalugd_1_1 |
| 889 | mexA_1_1 |
| 890 | acrR_1_1 |
| 891 | emeA_1_1 |
| 892 | acrA_1_1 |
| 893 | rtn_1_1 |
| 894 | abcXStrpmut_1_1 |
| 895 | qacEdelta1_1_1 |
| 896 | elkT-abcA_1_1 |
| 897 | I-cadA_1_1 |
| 898 | albA_1_1 |
| 899 | wzm_1_1 |
| 900 | msrCb_1_1 |
| 901 | nov_1_1 |
| 902 | wzt_1_1 |
| 903 | wbbl_1_1 |
| 904 | norA23_1_1 |
| 905 | mexR_1_1 |
| 906 | arr2_1_1 |
| 907 | mreA_1_1 |
| 908 | I-cadC_1_1 |
| 909 | uvrA_1_1 |

| *Candida albicans* drug resistance | |
|---|---|
| 910 | CRD2_1_1 |
| 911 | CDR1_1_1 |
| 912 | CDR1_2_1 |
| 913 | MET3_1_1 |
| 914 | FET3_1_1 |
| 915 | FTR2_1 _1 |
| 916 | MDR1-7_1_1 |
| 917 | ERG11_1_1 |
| 918 | SEC20_1_1 |

d) controls and utility genes

| SEQ I D NO | Probe |
|---|---|
| Negative Controls | |
| 919 | rbcL_1_1 |
| 925 | rbcL_1_1 1_2 |

| Positive controls / human genes | |
|---|---|
| 920 | LDHA(hu)_1_1 |
| 921 | GAPD(hu)_1_1 |
| 922 | b-Act(hu)_1_1 |
| 923 | ARHGDIA(hu)_1_1 |
| 924 | PGK1 (hu)_1_1 |

| Positive controls / 16S | |
|---|---|
| 926 | 16SPa_1_1 |
| 927 | 23SEfaecium_2_1 |
| 928 | 16SStrepyog_1_1 |
| 929 | 16SStrepneu_1_1 |
| 930 | 16SStrepagalactiae_1_1 |
| 931 | 16SEfaecium_1_1 |
| 932 | 16SEfaecium_2_1 |
| 933 | 16SRNAEf_2_1 |
| 934 | 16SKpn_1_1 |
| 935 | 16SSa_3_1 |
| 936 | 16SRNAEf_1 _1 |
| 937 | 16SShominis_1_1 |
| 938 | 16SShaemolyt_1_1 |
| 939 | 23SEfaecium_1_1 |
| 940 | 16SrRNAPrmi_1_1 |
| 941 | 16SrRNAPrvu1_1_1 |
| 942 | 16SSa_1_1 |
| 943 | 16SKlox_1_1 |

| Positive controls / Spiked Controls | |
|---|---|
| 944 | p53_1_1 |
| 945 | 0135mihck_1_1 |
| 946 | FAN_1_1 |
| 947 | 0270cap_1_1 |

The DNA microarray of (1) is preferably suitable for
(I) identification of *Staphylococcus aureus* and comprises one or more or all gene probes of group (a) selected from SEQ ID NO:1-99, preferably at least the gene probes represented by SEQ I D NO:71 and 68; and/or
(II) identification of *Escherichia coli* and comprises one or more or all gene probes of group (a) selected from SEQ I D NO:142-152, preferably at least the gene probes represented by SEQ I D NO: 143 and 149; and/or
(III) identification of *Staphylococcus epidermidis* and comprises gene probes of group (a) selected from SEQ ID NO:174-199, preferably at least the gene probes represented by SEQ I D NO: 177 and 184; and/or
(IV) identification of *Staphylococcus haemolyticus* and comprises one or more or all gene probes of group (a) selected from SEQ ID NO:209-214, preferably at least the gene probes represented by SEQ I D NO:209 and 210; and/or
(V) identification of *Staphylococcus lugdunensis* and comprises one or more or all gene probes of group (a) selected from SEQ ID NO:216-219, preferably at least the gene probes represented by SEQ I D NO:216 and 219; and/or
(VI) identification of *Staphylococcus warneri* and comprises one or more or all gene probes of group (a) selected from SEQ ID NO:224-229, preferably at least the gene probes represented by SEQ I D NO:224 and 225; and/or
(VII) identification of *Candida albicans* and comprises one or more or all gene probes of group (a) selected from SEQ I D NO:231-291, preferably at least the gene probes represented by SEQ I D NO:231 and 232; and/or
(VIII) identification of *Enterococcus faecalis* and comprises one or more or all gene probes of group (a) selected from SEQ ID NO:308-342, preferably at least the gene probes represented by SEQ I D NO:308 and 310; and/or
(IX) identification of *Enterococcus faecium* and comprises one or more or all gene probes of group (a) selected from SEQ ID NO:377-393, preferably at least the gene probes represented by SEQ ID NO:377 and 380; and/or
(X) identification of *Klebsiella pneumonia* and comprises one or more or all gene probes of group (a) selected from SEQ ID NO:399-431, preferably at least the gene probes represented by SEQ I D NO:399 and 402; and/or
(XI) identification of *Klebsiella oxytoca* and comprises one or more or all gene probes of group (a) selected from SEQ ID NO:449-469, preferably at least the gene probes represented by SEQ I D NO:449 and 455; and/or
(XII) identification of *Pseudomonas aeruginosa* and comprises one or more or all gene probes of group (a) selected from SEQ I D NO:470-490, preferably at least the gene probes represented by SEQ I D NO:470 and 471 ; and/or
(XIII) identification of *Streptococcus pneumoniae* and comprises one or more or all gene probes of group (a) selected from SEQ ID NO:523-591, preferably at least the gene probes represented by SEQ I D NO:523 and 524; and/or
(XIV) identification of *Streptococcus agalactiae* and comprises one or more or all gene probes of group (a) selected from SEQ I D NO:606-639, preferably at least the gene probes represented by SEQ I D NO:606 and 619; and/or
(XV) identification of *Streptococcus pyogenes* and comprises one or more or all gene probes of group (a) selected from SEQ I D NO:645-656, preferably at least the gene probes represented by SEQ I D NO:645 and 646; and/or
(XVI) identification of *Streptococcus viridans* and comprises one or more or all gene probes of group (a) selected from SEQ ID NO:687-701, preferably at least the gene probes represented by SEQ I D NO:687 and 691 ; and/or
(XVII) identification of *Proteus mirabilis* and comprises one or more or all gene probes of group (a) selected from SEQ I D NO:706-749, preferably at least the gene probes represented by SEQ I D NO:706 and 710; and/or
(XVIII) identification of *Proteus vulgaris* and comprises one or more or all gene probes of group (a) selected from SEQ I D NO:776-781, preferably at least the gene probes represented by SEQ I D NO:776 and 777.

I n a further especially preferred aspect, the DNA m icroarray of (1) is suitable for
(I) virulence determination of *Staphylococcus aureus* and comprises one or more or all of the gene probes of group (b) selected from SEQ ID NO:100-141 ; and/or
(II) virulence determination of *Escherichia coli* and comprises one or more or all of the gene probes of group (b) selected from SEQ I D NO: 153-173; and/or
(III) virulence determination of *Staphylococcus epidermidis* and comprises one or more or all of the gene probes of group (b) selected from SEQ ID NO:200-208; and/or
(IV) virulence determination of *Staphylococcus haemolyticus* and comprises the gene probe of group (b) represented by SEQ I D NO:215; and/or
(V) virulence determination of *Staphylococcus lugdunensis* and comprises one or more or all of the gene probes of group (b) selected from SEQ ID NO:220-221 ; and/or
(VI) virulence determination of *Staphylococcus warneri* and comprises the gene probe of group (b) represented by SEQ I D NO:230; and/or
(VII) virulence determination of *Candida albicans* and comprises one or more or all of the gene probes of group (b) selected from SEQ ID NO:292-307; and/or
(VIII) virulence determination of *Enterococcus faecalis* and comprises one or more or all of the gene probes of group (b) selected from SEQ ID NO:343-376; and/or
(IX) virulence determination of *Enterococcus faecium* and comprises one or more or all of the gene probes of group (b) selected from SEQ ID NO:394-398; and/or
(X) virulence determination of *Klebsiella pneumonia* and comprises one or more or all of the gene probes of group (b) selected from SEQ ID NO:432-448; and/or
(XI) virulence determination of *Klebsiella oxytoca*; and/or
(XII) virulence determination of *Pseudomonas aeruginosa* and comprises one or more or all of the gene probes of group (b) selected from SEQ ID NO:491-522; and/or
(XIII) virulence determination of *Streptococcus pneumoniae* and comprises one or more or all of the gene probes of group (b) selected from SEQ ID NO:592-605; and/or
(XIV) virulence determination of *Streptococcus agalactiae* and comprises one or more or all of the gene probes of group (b) selected from SEQ ID NO:640-644; and/or
(XV) virulence determination of *Streptococcus pyogenes* and comprises one or more or all of the gene probes of group (b) selected from SEQ ID NO:657-686; and/or
(XVI) virulence determination of *Streptococcus viridans* and comprises one or more or all of the gene probes of group (b) selected from SEQ ID NO:702-705; and/or
(XVII) virulence determination of *Proteus mirabilis* and comprises one or more or all of the gene probes of group (b) selected from SEQ ID NO:750-775; and/or
(XVIII) virulence determination of *Proteus vulgaris* and comprises one or more or all of the gene probes of group (b) selected from SEQ I D NO: 782-784.

I n a further especially preferred aspect, the DNA m icroarray of (1) is suitable for antibiotic resistance determination of (I) *Staphylococcus aureus,* (II) *Escherichia coli,* (III) *Staphylococcus epidermidis, (*IV*) Staphylococcus haemolyticus,* (V) *Staphylococcus lugdunensis,* (VI) *Staphylococcus warneri,* (VIII) *Enterococcus faecalis,* (IX) *Enterococcus faecium, (*X*) Klebsiella pneumonia,* (XI) *Klebsiella oxytoca,* (XII) *Pseudomonas aeruginosa,* (XIII) *Streptococcus pneumoniae,* (XIV) *Streptococcus agalactiae,* (XV) *Streptococcus pyogenes,* (XVI) *Streptococcus viridans,* (XVII) *Proteus mirabilis,* and/or (XVIII) *Proteus vulgaris* and comprises one or more or all of the gene probes of group (c) selected from SEQ ID NO:785-909; and/or

it is suitable for antibiotic resistance determination of (VII) *Candida albicans* and comprises one or more or all of the gene probes of group (c) selected from SEQ ID NO:910-918.

In a preferred embodiment, the microarray of (1) is suitable for identification and characterisation, i.e. virulence and/or resistance determination, of the target microorganism and comprises one or more or all of the gene probes of group (a) and additionally one or more or all of the gene probes of group (b) and group (c) for each organism as listed above

If the identification and/or characterisation of *S*. *aureus, E. coli* and/or *P*. *aeruginosa* is the aim of a test using the array, then the array comprises preferably at least the core gene probes designated in example 7, more preferably all the sequences listed in Tab. 2 and/or Tab. 6. Even more preferred, it consists of said sequences.

In a most especially preferred aspect, the DNA microarray of (1) comprises the following gene probes, even more preferably consists of the following gene probes:
(I) When the DNA microarray is suitable for identification and characterisation of *Staphylococcus aureus,* it comprises
   (a) the gene probes represented by SEQ I D NO: 1-99; and
   (b) the gene probes represented by SEQ I D NO:100-141 and/or
   (c) the gene probes represented by SEQ I D NO:785-909.
(II) When the DNA microarray is suitable for identification and characterisation of *Escherichia coli,* it comprises
   (a) the gene probes represented by SEQ ID NO: 142-152; and
   (b) the gene probes represented by SEQ I D NO: 153-173 and/or
   (c) the gene probes represented by SEQ I D NO: 785-909.
(III) When the DNA microarray is suitable for identification and characterisation of *Staphylococcus epidermidis,* it comprises
   (a) the gene probes represented by SEQ I D NO: 174-199; and
   (b) the gene probes represented by SEQ I D NO: 200-208 and/or
   (c) the gene probes represented by SEQ I D NO: 785-909.
(IV) When the DNA m icroarray is suitable for identification and characterisation of *Staphylococcus haemolyticus,* it comprises
   (a) the gene probes represented by SEQ I D NO: 209-214; and
   (b) the gene probes represented by SEQ I D NO: 215 and/or
   (c) the gene probes represented by SEQ I D NO: 785-909.
(V) When the DNA microarray is suitable for identification and characterisation of *Staphylococcus lugdunensis,* it comprises
   (a) the gene probes represented by SEQ I D NO: 216-219; and
   (b) the gene probes represented by SEQ I D NO: 220-221 and/or
   (c) the gene probes represented by SEQ I D NO: 785-909.
(VI) When the DNA m icroarray is suitable for identification and characterisation of *Staphylococcus warneri,* it comprises
   (a) the gene probes represented by SEQ I D NO: 224-229; and
   (b) the gene probes represented by SEQ I D NO: 230 and/or
   (c) the gene probes represented by SEQ I D NO: 785-909.
(VII) When the DNA microarray is suitable for identification and characterisation of *Candida albicans,* it comprises
   (a) the gene probes represented by SEQ I D NO: 231 -291 ; and
   (b) the gene probes represented by SEQ ID NO: 292-307 and/or
   (c) the gene probes represented by SEQ ID NO: 910-918.
(VIII) When the DNA microarray is suitable for identification and characterisation of *Enterococcus faecalis,* it comprises
   (a) the gene probes represented by SEQ I D NO: 308-342; and
   (b) the gene probes represented by SEQ ID NO: 343-376 and/or
   (c) the gene probes represented by SEQ I D NO: 785-909.
(IX) When the DNA microarray is suitable for identification and characterisation of *Enterococcus faecium,* it comprises
   (a) the gene probes represented by SEQ I D NO: 377-393; and
   (b) the gene probes represented by SEQ I D NO: 394-398 and/or
   (c) the gene probes represented by SEQ I D NO: 785-909.
(X) When the DNA microarray is suitable for identification and characterisation of *Klebsiella pneumonia,* it comprises
   (a) the gene probes represented by SEQ I D NO: 399-431; and
   (b) the gene probes represented by SEQ ID NO: 432-448 and/or
   (c) the gene probes represented by SEQ I D NO: 785-909.
(XI) When the DNA microarray is suitable for identification and characterisation of *Klebsiella oxytoca,* it comprises
   (a) the gene probes represented by SEQ I D NO: 449-469, and
   (c) the gene probes represented by SEQ I D NO: 785-909.
(XII) When the DNA microarray is suitable for identification and characterisation of *Pseudomonas aeruginosa,* it comprises
   (a) the gene probes represented by SEQ I D NO: 470-490; and
   (b) the gene probes represented by SEQ I D NO: 491 -522 and/or
   (c) the gene probes represented by SEQ I D NO: 785-909.
(XIII) When the DNA microarray is suitable for identification and characterisation of *Streptococcus pneumoniae,* it comprises
   (a) the gene probes represented by SEQ I D NO: 523-591 ; and
   (b) the gene probes represented by SEQ I D NO: 592-605 and/or
   (c) the gene probes represented by SEQ I D NO: 785-909.
(XIV) When the DNA microarray is suitable for identification and characterisation of *Streptococcus agalactiae,* it comprises
   (a) the gene probes represented by SEQ I D NO: 606-639; and
   (b) the gene probes represented by SEQ I D NO: 640-644 and/or
   (c) the gene probes represented by SEQ I D NO: 785-909.
(XV) When the DNA microarray is suitable for identification and characterisation of *Streptococcus pyogenes,* it comprises
   (a) the gene probes represented by SEQ I D NO: 645-656; and
   (b) the gene probes represented by SEQ ID NO: 657-686 and/or
   (c) the gene probes represented by SEQ I D NO: 785-909.
(XVI) When the DNA microarray is suitable for identification and characterisation of *Streptococcus viridans,* it comprises
   (a) the gene probes represented by SEQ I D NO: 687-701 ; and
   (b) the gene probes represented by SEQ I D NO: 702-705 and/or
   (c) the gene probes represented by SEQ I D NO: 785-909.
(XVII) When the DNA microarray is suitable for identification and characterisation of *Proteus mirabilis,* it comprises
   (a) the gene probes represented by SEQ I D NO: 706-749; and
   (b) the gene probes represented by SEQ I D NO: 750-775 and/or
   (c) the gene probes represented by SEQ I D NO: 785-909.
(XVIII) When the DNA microarray is suitable for identification and characterisation of *Proteus vulgaris,* it comprises
   (a) the gene probes represented by SEQ I D NO: 776-781 ; and
   (b) the gene probes represented by SEQ I D NO: 782-784 and/or
   (c) the gene probes represented by SEQ I D NO: 785-909.

The microarray of embodiment (1) can be fabricated using textbook methods for microarray production, including printing with fine-pointed pins onto the solid support, photolithography using pre-made masks or dynamic micromirror devices, ink-jet printing or electrochemistry on microelectrode arrays (Müller, H.-J., Röder, T., "Der Experimentator: Microarrays, Spektrum Akademischer Verlag, Heidelberg (2004)). Preferred fabrication methods are printing methods spotting the gene probes onto the solid surface of the microarray. The attachment of the spotted DNA to the surface is achieved by covalent or non-covalent binding, preferably by non-covalent binding, more preferably by electrostatic interaction (ionic binding), most preferably by ionic binding of the DNA to amino groups present on the surface of the solid support. Any amino-functionalized microarray support can be used, but gamma aminopropyl silane (GAPS^{™}) coated slides, especially UltraGAPS^{™} coated glass slides, are preferred in present invention.

The amount of DNA per spot printed onto the array is from 0.1 to 15.0 ng, preferably from 0.1 to 0.2 ng.

Thus, the present invention also pertains to a method for fabrication of a microarray of embodiment (1), which method comprises spotting the gene probes listed above to an appropirate solid support.

The sample or clinical specimen of embodiment (1) is preferably selected from the group consisting of whole blood, serum, urine, saliva, liquor, sputum, punktate, stool, pus, wound fluid and positive blood cultures, more preferably is whole blood or a positive blood culture, most preferably is a positive blood culture. If blood culture is used as DNA source, 0.5 ml positive blood culture is sufficient for identification and characterisation of the microorganisms and bacteria present without prior amplification of the target DNA.

Thus, the microarray of present application is
(i) a robust diagnostic tool, detecting all tested bacterial reference strains and clinical isolates;
(ii) sensitive enough to yield positive signals with e.g. only 20 ng of purified genomic *S. aureus* DNA or 2 µg of DNA extracted from blood culture which contains a high percentage of human DNA;
(iii) highly specific, distinguishing e.g. *S. aureus* from distantly related gram-negative bacteria like *Escherichia coli* or *Pseudomonas aeruginosa* as well as from closely related CoNS;
(iv) precise enough to identify virulence factors and antibiotic resistance determinant genes without previous amplification by PCR.

Moreover, the whole procedure can be accomplished the same day after blood cultures become positive (e.g. in the Bactec^{®}). Rapid identification of the causative pathogen in fungemia, bacteremia and sepsis is crucial for several reasons:
(i) appropriate antimicrobial therapy should be started as early as possible and unnecessary treatment avoided;
(ii) the prognosis of the patients with sepsis may be improved; and
(iii) expenditures on antimicrobials and prolonged hospitalisation can be reduced.

With the gene-segment based microarray of (1) there is an excellent correlation between genotypic detection of antibiotic resistance determinants and phenotypic typing using conventional susceptibility testing. I n one aspect of the invention, the detection of the resistance genes *mecA, blaZ, ermA, ermC, msrSA, aadD* and *aacA-aphD* by microarray hybridisation allows for reliable prediction of oxacillin, penicillin, erythromycin, tobramycin and gentamicin resistance in a single assay.

By microarray hybridisation according to present invention it is furthermore possible to discriminate multi-resistant and multi-susceptible MRSA (strain MW2). Multi-susceptible MRSA have been shown to be susceptible to tobramycin and erythromycin (Polyzou, A. et al., J. Antimicrob. Chemother. 48:231-4 (2001); Pournaras, S. et al., J. Clin. Microbiol. 39:779-81 (2001)).

In a preferred aspect of the invention, simultaneous comprehensive resistance genotyping for oxacillin, macrolide and aminoglycoside resistance genes (preferably *mecA, aadD, aacA-aphD, ermA,B,C* and *msrSA)* by microarray hybridisation allows the rapid discrimination of multi-resistant or multi-susceptible strains and in consequence other therapeutic options with e.g. macrolides and may reduce reliance on vancomycin (Polyzou, A. et al., J. Antimicrob. Chemother. 48:231-4 (2001); Pournaras, S. et al., J. Clin. Microbiol. 39:779-81 (2001)).

One preferred aspect of embodiment (1) is a DNA microarray for the identification and characterisation of the three important bacteremia causing species *Staphylococcus aureus, Escherichia coli* and *Pseudomonas aeruginosa* in a sample, preferably in blood culture. The microarray allows simultaneous species identification and detection of important virulence and antibiotic resistance genes in a single assay. Preferably, this array consists of 2-20 species specific gene probes, 1-20 virulence gene probes and 1-20 resistance gene probes of at least 100 nt length, more preferably of 200-800 nt length. One especially preferred embodiment is an array comprising or consisting of the gene probes listed in Tab. 2. The probes may be amplified from recombinant plasmids or synthesized by any other method know in the art. These probes represent genes encoding house-keeping proteins, virulence factors and antibiotic resistance determinants. Evaluation with 42 clinical isolates, 3 reference strains and 13 positive blood cultures revealed that this DNA microarray is highly specific in identifying *S. aureus, E. coli* and *P*. *aeruginosa* strains and in discriminating them from closely related Gram-positive and Gram-negative bacterial strains also known to be etiological agents of bacteremia. In Example 6 and 7, this array was successful in identifying all tested 27 *E. coli, P. aeruginosa* and *S. aureus* strains and in discriminating them from 21 closely related Gram positive and Gram negative bacterial strains. There is a nearly perfect correlation between genotypic antibiotic resistance by hybridisation to the *S. aureus* resistance gene probes *mecA* (oxacillin/methicillin resistance), *aacA-aphD* (gentamicin resistance), *ermA* (erythromycin resistance) and *blaZ* (penicillin resistance) and the *E. coli* resistance gene probes *blaTEM-106* (penicillin resistance) and *aacC2* (aminoglycoside resistance) and phenotypic antibiotic resistance determined by conventional susceptibility testing (Example 10).

One further preferred aspect of embodiment (1) of the invention is a DNA microrarray for the identification and characterisation of *S. aureus* in a sample, preferably in blood culture. Evaluation with 10 clinical isolates, 6 reference strains and 10 positive blood cultures revealed that this DNA microarray is highly specific in identifying *S. aureus* and in discriminating them from closely related Gram-positive and Gram-negative bacterial strains also known to be etiological agents of bacterem ia (Example 11).

The method of embodiment (3) comprises - after isolating the total DNA (including non-microbial DNA) from a sample - the steps of immediate labelling and microarray-based detection of this isolated DNA with or without, preferably without, further DNA amplification steps after the DNA isolation. It is one advantage of the method (3) that it can be performed without said further DNA amplification steps, i.e. the isolated DNA is labelled and applied to the microarray without prior amplification. The use of a single protocol for all microbial species comprising all steps of a microarray procedure including DNA preparation and DNA-chip hybridisation, is essential for testing blood cultures or other clinical specimens, where the bacterial diagnosis is usually uncertain. Preferably, a DNA preparation protocol employing sonication for simultaneous cell disruption and target DNA fragmentation is the method of choice to increase the sensitivity of the microarray, in particular towards low-copy number and/or plasmid encoded genes which may be underrepresented in the target DNA.

The method of embodiment (3) is preferably a method for diagnosis of bacteremia or sepsis. Furthermore, the sample or clinical specimen used in embodiment (3) is preferably blood or derived from blood, more preferably is a blood culture. Most preferably, the clinical specimen is a positive blood culture.

To obtain positive signals in the method of embodiment (3), 100 pg of purified genomic microbial DNA may be sufficient (lower detection limit), but preferably at least 1 ng of said DNA should be present in the sample. Usually, at least 10 ng, preferably at least 20 ng, more preferably at least 1 µg of purified genomic microbial DNA or at least 1 µg, preferably at least 2 µg of DNA extracted from blood culture are required. 500 µl of positive blood culture yield enough DNA for several hybridisations.

In the method of embodiment (3), the ratio of microbial DNA to total DNA isolated from said sample or clinical specimen is less than or equal to 100 %, preferably is from 1% to 99%, m ore preferably from 30 to 60%.

The labelling reaction of the method of embodiment (3) may be any DNA labelling reaction known in the art. However, chemical labelling reactions consisting of chemical attachement of a reporter molecule to the sample DNA and labelling by integration of labelled nucleotides into the sample DNA are preferred. Preferably the reporter molecules are fluorophores, more preferably are of the cyanine group of fluorophores. Most preferably, the DNA is labelled with Cy3, Cy5 and/or Alexa Fluor 647 and Alexa Fluor 546. The ratio of bases to dye molecules (BDR) is preferably less or equal to 60.

The detection of the reporter molecule in the method of embodiment (3) of the invention is preferably done by using a suitable detection system for the bound reporter molecule. This detection system is preferably based on visualization of the reporter molecule, more preferably on fluorescence detection. Furthermore, the detection is preferably done by a microarray scanner.

In the method of embodiment (3) of the invention, the DNA microarray can be substituted by any other solid support onto which DNA gene probes are attached in a way permitting hybridisation of the DNA in the sample and subsequent detection of the bound DNA. This includes the use of microtiter plates coated with one or several DNA gene probes per well, of glass surfaces (like, e.g., microscopic slides) with DNA spots, of filter paper disks, membranes, gold electrodes and beads (particles with a diameter of from 1 nm to several µm made of glass, plastic, metal etc.) coated with DNA, etc.

The kit of embodiment (4) of the invention may additionally comprise reagents for the labelling reactions of embodiment (3) and/or reagents necessary for the hybridisation step of the method of embodiment (3).

The present invention is described in more detail by reference to the following examples. It should be understood that these examples are for illustrative purpose only and are not to be construed as limiting the invention.

### Examples

In the experimental examples described below, standard techniques of recombinant DNA technology were used that were described in various publications, e.g. Sambrook et al. (1989), Molecular Cloning: A Laboratory Manual, Cold Spring Harbor Laboratory, or Ausubel et al. (1987), Current Protocols in Molecular Biology 1987-1988, Wiley Interscience. Unless otherwise indicated, all enzymes and kits were used according to the manufacturers' specifications.

### Example 1 : Materials and Methods

Reference strains, clinical isolates and culture conditions: Bacterial reference strains were obtained from the American Type Culture Collection (ATCC, Manassas, Va.), the Deutsche Sammlung von Mikroorganismen und Zellkulturen (DSMZ, Braunschweig, Germany) or the network on antimicrobial resistance in *Staphylococcus aureus* (NARSA, Herndon, Virginia). Clinical isolates were obtained from the inventors' clinical routine microbiology laboratory.

The following bacteria were used for evaluation of the specificity of the microarray in Examples 2-10: *Staphylococcus aureus* (ATCC 25923, NRS123 alias MW2, 5 clinical isolates), *Staphylococcus epidermidis* (5 clinical isolates), *Staphylococcus capitis* (clinical isolate), *Staphylococcus haemolyticus* (clinical isolate), *Staphylococcus hominis* (clinical isolate), *Staphylococcus warneri* (clinical isolate), *Staphylococcus auricularis* (clinical isolate), *Micrococcus* spp. (clinical isolate), *Escherichia coli* (ATCC 25922, 6 clinical isolates), *Pseudomonas aeruginosa* (ATCC27853, 5 clinical isolates), *Klebsiella pneumoniae* (3 clinical isolates), *Proteus mirabilis* (2 clinical isolates), *Serratia marcescens* (2 clinical isolates), *Enterobacter cloacae* (clinical isolate), *Enterobacter aerogenes* (clinical isolate), *Acinetobacter baumannii* (clinical isolate), *Stenotrophomonas maltophilia* (clinical isolate), *Enterococcus* spp. (clinical isolate), *Enterococcus faecalis* (clinical isolate) and *Streptococcus pneumoniae* (clinical isolate).

Bacterial strains and clinical isolates were grown over night at 37 °C with constant shaking in 5 ml Luria-Bertani (LB) broth or tryptic soy broth (TSB, 30 g/I, Merck) containing 3 g/I yeast extract. Enterococci and streptococci were grown in 10 ml TSB plus yeast without agitation under 5% CO₂. Overnight cultures were harvested at 2,560 g for 10 min. After discarding the supernatant the pellet was washed in 1 ml TE (10 mM Tris-HCl, pH 7.5 and 1 mM EDTA) and recovered by centrifugation at 17,900 g for 10 min. Cell pellets were used for DNA preparation.

Blood cultures: Aerobic and anaerobic blood culture bottles (BACTEC^{®}, Becton Dickinson, Heidelberg, Germany) were inoculated with blood from patients with suspected sepsis and placed in a BACTEC^{®} 9240 blood culture system (Becton Dickinson), a continuous-reading, automated, and computed blood culture system that detects the growth of microorganisms by monitoring CO₂ production. Incubation was performed according to the manufacturer's recommendations. Bottles with a positive growth index were removed from the incubator, and aliquots of 1 ml of the blood culture suspensions were taken aseptically with a needle syringe. 1 ml-aliquots of the blood culture suspensions were mixed with 1 ml 0.1% Triton^{®}-X-100 and kept at room temperature for 5 min in order to disrupt human blood cells. Bacterial cells were then harvested at 17,900 g for 10 min, pellets were washed in 1 ml TE, recovered by centrifugation and used for DNA preparation. For conventional identification and susceptibility testing, a second 1 ml-aliquot was examined by Gram-stain and subcultured on agar plates. The organisms grown on agar plates were characterised and tested for susceptibility using a VITEK-2 system (bioMerieux, Inc., Nürtingen, Germany), Etest strips (AB BIODISK, Solna, Sweden) or disk diffusion tests following the method recommended by the National Committee for Clinical Laboratory Standards (NCCLS) (Standards, N.C.f.C.L., Approved standard M2-4a, Villanova, PA (1990)).

For microarray hybridisation experiments, DNA was prepared from 13 blood cultures positive for *S. aureus (4), S*. *epidermidis (3), S. pneumoniae (2), P. aeruginosa* (1), *E. coli* (2) and *P. mirabilis* (1).

### Example 2: DNA preparation

Total cellular DNA was extracted and purified either by using the First-DNA All-tissue kit (GEN-IAL GmbH, Troisdorf, Germany) following the instructions of the supplier or by enzymatic lysis followed by phenol/chloroform extraction. For the latter protocol, cell pellets were resuspended in 500 µl lysis buffer (20 mM Tris-HCl, pH 8.0, 2 mM EDTA, pH 8.0, and 1.2% Triton^{®}-X-100) and lysozyme (Sigma, Taufkirchen, Germany) was added to reach a final concentration of 0.8 mg/ml. In addition, lysostaphin (Sigma) was added to a final concentration of 0.2 mg/ml to promote staphylococcal lysis or mutanolysin (0.5 U/µl; Sigma) was added to lyse Streptococci and Enterococci. After incubation at 37°C for one hour, cell lysates were treated with Proteinase K (1 mg/ml; Sigma) for 1 hour at 55°C and then with RNase A (0.2 mg/ml; Qiagen, Hilden, Germany) for 1 hour at 37°C. The volume was increased by the addition of 200 µl TE and the salt concentration was adjusted to 0.7 M by addition of 5 M NaCl. A 10% CTAB (cetyltrimethylammonium bromide) solution in 0.7 M NaCl was added to a final concentration of 1% and incubated at 65°C for 20 min in order to release DNA from polysaccharide DNA complexes. DNA was then extracted once with phenol/chloroform/isoamyl alcohol (25:24:1) and once with chloroform/isoamyl alcohol (24:1) prior to precipitation with one volume of isopropanol. After centrifugation at 17,900 g for 30 min, DNA pellets were washed in 70% ethanol and resuspended in 50-100 µl TE.

Concentration, purity and size of the purified DNA preparations were determined by UV-spectrophotometry (lambda 40, PerkinElmer, Boston USA) and 1% agarose gel electrophoresis.

### Example 3: DNA labelling

Total DNA from commercially available reference strains, clinical isolates and blood cultures was labelled by a non-enzymatic chemical labelling method using the Label It Cy3/Cy5 kits (Mirus, Madison, USA) or the ULYSIS Alexa Fluor 467 Nucleic Acid Labelling Kit (Molecular Probes; Eugene, USA). Prior to labelling, each target DNA was spiked with three gene segments (1 µl each, 30 ng/µl) amplified by PCR from selected recombinant plasmids to serve as internal positive controls.

For labelling with the Label It Cy3/Cy5 kit 5 µg of high molecular weight DNA (>20 kb) were mixed with 7.5 µl reagent in a total volume of 50 µl and incubated for 2 hours at 37°C according to the recommendations by the supplier. After adjusting the volume to 200 µl with H₂O and adding 0.1 volume of 5 M NaCl, unbound label was removed by precipitation with 2 volumes of ice-cold absolute ethanol for at least 30 min at -20 °C. The labelled DNA was recovered by centrifugation at 17,900 g for 30 min. The pellet was washed with 70% ethanol and resuspended in 70 µl TE.

For labelling with the Ulysis Alexa Fluor 647 kit, 1 µg DNA was denatured at 95°C for 5 min, cooled on ice, mixed with 20 µl labelling buffer and 5 µl reagent and incubated at 80 °C for 15 min according to the instructions of the manufacturer. Unbound dye was removed by ethanol precipitation as described above. The relative labelling efficiency of a reaction was evaluated by calculating the approximate ratio of bases to dye molecules (acceptable labelling ratios for nucleic acid were =60). This ratio and the amount of recovered labelled DNA was determined by measuring the absorbance of the nucleic acids at 260 nm and the absorbance of the dye at its absorbance maximum using a Iambda40 UV-spectrophotometer (PerkinElmer) and plastic disposable cuvettes for the range from 220 nm to 1,600 nm (UVette; Eppendorf, Hamburg, Germany).

### Example 4: Microarray construction

Cloned PCR-products were used to generate probes for the DNA microarray. All together 120 gene segments representing virulence genes, antibiotic resistant determinants and species specific metabolic and structural genes from *S. aureus* (40), *E. coli* (31) and *P. aeruginosa* (49) were represented on the microarray (Tab. 2).

**Tab. 2: Gene probes with SEQ ID NOs, function, gi numbers and primer sequences. E. coli gene probes (1-31), P. aeruginosa gene probes (32-80), S. aureus gene probes (81-120).**

| Array No. | Symbol | Function | gi number | gene probe SEQ ID NO | Primer forward [SEQ ID NO] | Primer reverse [SEQ ID NO] |
|---|---|---|---|---|---|---|
| 1 | *envZ* | Inner membrane osmosensor | 453286 | 143 | AGCCTGGTGACGA CTTATC [1233] | ATCCGCCAGTTGCTT AAC [1234] |
| 2 | *fes(2)* | Enterochelin esterase (siderophore) | 145916 | 161 | TGTTTCTGCACTCG AAATG [1269] | GGCAATAGCTTTCAC CAG [1270] |
| 3 | *fes(1)* | Enterochelin esterase (siderophore) | 145916 | 160 | TGTTTGAGGTCACT TTCTGG [1267] | CAATAGCTTTCACCA GGG [1268] |
| 4 | *nfrB* | Bacteriophage N4 receptor, inner membrane protein | 16127994 | 145 | ATGGAATTGCGTCT GTTC [1237] | AAGTTTAGCCACAGC AGG [1238] |
| 5 | *yacH* | Putative membrane protein | 16127994 | 148 | GACTCGGTACAGC GATTG [1242] | CTGACGTTGGGTATC TCG [1243] |
| 6 | *yagX* | Putative enzyme | 16127994 | 149 | CTTTACGACGGTTC TCCC [1244] | AATCTTCCCTGCTGA AATG [1245] |
| 7 | *ycdS* | Putative outer membrane protein | 16127994 | 150 | TTGAAACTTCTTAC TGCCG [1246] | AATTTCTAATGCAGC GTATTG [1247] |
| 8 | *b1169* | | 16127994 | 142 | GTTTGGGACTTATT GCTCTG [1230] | CATCAGCCACAGTTT CAAG [1231] |
| 9 | *b1202* | Putative outer membrane protein | 16127994 | 153 | GAATACCAAAGCA GATCGTC [1252] | CCGAGATCGACAACA GAG [1253] |
| 10 | *fliCb* | Flagellar H antigen | 8071787 | 144 | ACCACGACAGGTC TTTATG [1234] | AGAGAGGCACCGTC ACTAC [1235] |
| 11 | *iucA* | Aerobactin synthesis (siderophore) | 474189 | 165 | CATCAGGCAGTTAT CCTGTC [1276] | AGTCGTCCTCCTGCA TTAČ [1277] |
| 12 | *iucB* | Aerobactin synthesis (siderophore) | 474189 | 166 | TTCACAGCGGATAT GGAC [1278] | CACTTTGCTCCCAGA AATAC [1279] |
| 13 | *iucC* | Aerobactin synthesis (siderophore) | 474189 | 167 | AGACTGGGATTTG GTCAAC [1280] | AGACACCATCCTGCC TTC [1281] |
| 14 | *papG* | Adhesin, P-pill protein | 42307 | 168 | GGAGTATATTGCGT GGGTAG [1282] | AAGATTCACCATAGA GGCG [1283] |
| 15 | *yciQ* | Putative membrane protein | 16127994 | 151 | ATAGCAGGGCTGT TTGTATC [1248] | GACACGGAAACCAA ATTAAC [1249] |
| 16 | *ymcA* | Hypothetical protein | 16127994 | 152 | TATTGTCATCGCGC AGAG [1250] | TGTTGGGTTGAAAGA GTAGC [1251] |
| 17 | *eae* | Genetic locus necessary for the production of attaching and effacing lesions on tissue culture, OM protein adhesin | 145852 | 154 | CTAACTCATTGTGG TGGAGC [1254] | CTTGTCATCGGTCAT GTTG [1255] |
| 18 | *eltB* | Enterotoxin subunit B | 145830 | 155 | GGCGTTACTATCCT CTCTATG [1256] | TTTCCATACTGATTG CCG [1257] |
| 19 | *escR* | Secretion | 2897961 | 156 | TTTGTTGTTATTGG TACTTCATTC [1258] | ATCGAAATTGTTACT GGCG [1259] |
| 20 | *escT* | Secretion | 2897961 | 157 | TTACGCTTCCGATC ATAGTAG [1260] | GAATACGTTTAGTTG AGGCG [1261] |
| 21 | *escU* | Secretion | 2897961 | 158 | AAGTGAAGAGGTA ATGGCTG [1262] | TACCATCAGTATCCT TGGC [1263] |
| 22 | *espB* | Protein secreted by enteropathoge nic E. coli | 1657262 | 159 | GATGGTGACTCTAT TGCAGG [1264] | CCATACGATTCTGGA CCTC [1265] |
| 23 | *hlyA* | Enterohemorrh agic Escherichia coli hemolysin | 525328 | 163 | CTTGGAAATGTTGG TAAAGC [1272] | TAAACTCCTTCGGTT GAGC [1273] |
| 24 | *hlyB* | Enterohemorrh agic Escherichia coli hemolysin | 1247757 | 164 | TCAATGCTGAAACT ATAAGGC [1274] | ACTTAGCACCCAGTT CGAC [1275] |
| 25 | *SLTII* | Shiga-like toxin type II | 304950 | 171 | TTCTTCGGTATCCT ATTCCC [1288] | TGTGAGGTCCACTTC TTCC [1289] |
| 26 | *toxA-LTPA* | Subunit A of heat-labile enterotoxin | 148027 | 172 | AAATGGCGACAAAT TATACC [1290] | CTGGGTCTCCTCATT ACAAG [1291] |
| 27 | *VT2va B* | Verotoxin-2 variant, beta-subunit, shiga-like toxin | 148261 | 173 | AAGAAGATGTTTAT GGCGG [1292] | GATTCACAGGTACTG GATTTG [1293] |
| 28 | *aacC2* | aminoglycoside-(3)-N-acetyltransferase | 45769 | 833 | GACCGATCACCCTA CGAG [2612] | CGAAATGCTTCTCAA GATAGG [2613] |
| 29 | *blaTE M-106* | Class A beta-lactamase | 21464484 | 815 | ACATCGAACTGGAT CTCAAC [2576] | TCTCAGCGATCTGTC TATTTC [2577] |
| 30 | *strB* | Streptomycin resistance protein B | 17129524 | 834 | AAGTTTCATTGCCA GACG [2614] | TAGACTGCGTTGCTC CTC [2615] |
| 31 | *sul* | Dihydropteroat e synthase, sulfonamide resistance | 17129524 | 887 | CATCGTCAACATAA CCTCG [2720] | AATTCTTGCGGTTTC TTTC [2721] |
| 32 | *algB* | Alginate biosynthesis (exopolysacch aride) | 150990 | 494 | CACTTTCCGTTATT GCCTC [1934] | GAGGATGAGGATGT TGGC [1935] |
| 33 | *algN* | Alginate biosynthesis (exopolysaccharide) | 150999 | 495 | GACTGGCTGAATC GTCTC [1936] | GCAGGTCGTACCAG GAAG [1937] |
| 34 | *algR* | Alginate biosynthesis (exopolysaccharide) | 151003 | 496 | ATTGTCGATGACGA ACCTC [1938] | TTCAGGTAGAGCTG GAAATG [1939] |
| 35 | *aprA* | Alkaline protease | 45279 | 491 | CATTGAAAGGTCGT AGCG [1928] | CGACGAAGTGGATA TTGG [1929] |
| 36 | *aprE* | Alkaline protease secretion | 45279 | 492 | GGTCAAGCACATC CTAGTG [1930] | ACTTCCTTGCGGTAC TCC [1931] |
| 37 | *glpR* | Repression of glycerol metabolic enzymes (glp=glycerol-3-phosphate) | 1399486 | 470 | CAAGCACAACAAG AAATACG [1886] | TAGACCTCCGAAGA GTTGC [1887] |
| 38 | *IasRa* | Elastase, virulence protein | 309873 | 499 | CTGGGACGTTAGT GTCATC [1944] | GTCTTGGCATTGAGT TCG [1945] |
| 39 | *lasRb* | Transcriptional activator of elastase | 151325 | 471 | GAGCGACCTTGGA TTCTC [1888] | ATAAGACCCAAATTA ACGGC [1889] |
| 40 | *lipA* | Extracellular triacylglycerol lipase | 45340 | 500 | AAGAAGTCTCTGCT CCCC [1946] | ACGATTTCCTCCACC TGT [1947] |
| 41 | *lipH* | Lipophilic protein necessary for the expression of active lipase | 483463 | 501 | ATGGCAGTTTCAGT GTCG [1948] | CGAAATAGTCGTCCA GCC [1949] |
| 42 | *mexA* | Multidrug resistance protein MexA precursor | 5616092 | 889 | CTCGACCCGATCTA CGTC [2724] | GTCTTCACCTCGACA CCC [2725] |
| 43 | *Orf25 2* | DnaJ-like protein | 4545242 | 503 | GACCTGCTGTTCCA GTTG [1952] | AATTCACGGGTTTTC TCG [1953] |
| 44 | *OrfX* | Regulatory protein, glycerol metabolism | 1399486 | 472 | ATGGATGCTCGGG TACTG [1890] | CTCAGCTACAGCCAC GAC [1891] |
| 45 | *pa026 0* | Hypothetical protein | 15595198 | 473 | GATCGTCTCTGCCC AGTC [1892] | ACATTGATGGTGTCG TCC [1893] |
| 46 | *pa057* 2 | Hypothetical protein | 15595198 | 474 | AGGAGAGAACATG AGTCGC [1894] | TCCTTGTCCCAGTAG TTACC [1895] |
| 47 | *pa104* 6 | Hypothetical protein | 15595198 | 477 | AGGCATCCATCGA GCTAC [1900] | AACGTCCGAGCAGG ATAC [1901] |
| 48 | *pa106 9* | Hypothetical protein | 15595198 | 478 | GCGAGGAGGTATT CGACA [1902] | CCCTTCTGCGAGTAG TGTT [1903] |
| 49 | *pa184* 6 | Hypothetical protein | 15595198 | 479 | AAGGACTTCTGGTC GGTG [1904] | CAGGAACAGGTGCT CGTAG [1905] |
| 50 | *pa408 2* | Hypothetical protein | 15595198 | 481 | CGAGCACCAATATC GAAC [1908] | GAGCCGTAGGTGTT ATCG [1909] |
| 51 | *pchG* | Necessary for formation of siderophore pyochelin | 4325021 | 504 | CCTGCTCAACACCT TCTATC [1954] | GTCGAACAACGCGA ACAG [1955] |
| 52 | *PhzA* | Phenazine biosynthesis proteins (low molecular weight toxins) | 5616088 | 505 | GTTGAAAGGGTTTA CCGAC [1956] | AATTTCTGCATCGGG TTC [1957] |
| 53 | *PLC* | Phospholipase C (heat labile-hemolysin) | 151492 | 507 | GACTTCGCTGTTCG ACTTC [1960] | TCGGTTCGAGTTCAT AGC [1961] |
| 54 | *plcN* | Non-hemolytic phospholipase C | 151497 | 508 | GTGTTCCAGGTGTT CGAC [1962] | GATAGACGTTGTCCT TGACC [1963] |
| 55 | *plcR* | Phospholipase C regulation | 151499 | 509 | ACAACCTGGAACA GCAACT [1964] | CGACTCTTGCGCGTA TTC [1965] |
| 56 | *PstP* | Phosphoenolpy ruvate-protein phosphotransf erase | 4545246 | 485 | GAAGTGAACTCCG CCAAG [1916] | TCGAGCATCATCAGG TAGAC [1917] |
| 57 | *purK* | AIR carboxylase II, purine biosynthesis | 1621599 | 486 | TCGAGAAGTCGAT GTTCAAG [1918] | CTTGCCGTAGTGATG CAG [1919] |
| 58 | *rhlA* | Rhamnosyl-transferase involved in rhamnolipid biosu rfactant synthesis | 452502 | 518 | AGTCTGTTGGTATC GGTTTG [1982] | CTCCAGGTCGAGGA AATG [1983] |
| 59 | *rhlR* | Rhamnolipid regulation | 1117916 | 520 | TTCGATTACTACGC CTATGG [1986] | GGTCCATTGCAGGAT CTC [1987] |
| 60 | *toxA* | Exotoxin A precursor | 15595198 | 522 | GTGCGCTACAGCT ACACG [1990] | CTTGCCTTCCCAGGT ATC [1991] |
| 61 | *uvrDII* | DNA helicase | 3249556 | 487 | AGACCTACAACAAG GTTTCG [1920] | TGAGGATAGTCCCTT CGC [1921] |
| 62 | *vsml* | Autoinducer synthesis protein | 695153 | 488 | ATTCCTCTCTGAAT CGCTG [1922] | AATATCTTCATCGCC AGTTG [1923] |
| 63 | *xcpX* | Secretion protein, translocation of exoproteins across outer membrane | 45433 | 490 | TTCAACCTCAACGG ACTG [1926] | TGCAAGGTACTCACC AGC [1927] |
| 64 | *ExoS* | Exoenzyme S, secreted toxin | 13892017 | 497 | CGTTTGGGACAGA TTGAG [1940] | GATACTCTGCTGACC TCGC [1941] |
| 65 | *fpvA* | Ferripyoverdine receptor | 1633044 | 498 | AATGCGATAACCAT CAGC [1942] | CCGTCGTACTGGAA GTTG [1943] |
| 66 | *pa0625* | Hypothetical protein | 15595198 | 475 | AGGAGCAACTGAA GCGAC [1896] | TCTGCCTTTACCCAG GAC [1897] |
| 67 | *pa0636* | Hypothetical protein | 15595198 | 476 | AAGGTTGGCAGGA TCAAC [1898] | CTAGTGGCGAAATTG AACAG [1899] |
| 68 | *pa3866* | Hypothetical protein | 15595198 | 480 | TTCCCTAACGAATG CTGTC [1906] | CGTTGCTCCCTCATA CAC [1907] |
| 69 | *PhzB* | Phenazine biosynthesis proteins (low molecular weight toxins) | 5616088 | 506 | ATGCTCGATAATGC TATTCC [1958] | TTCTCGTAGTAACCC TCGG [1959] |
| 70 | *pilAp* | Type IV pilin, involved in twitching motility and attachment | 18535593 | 482 | GCTTTACCTTGATC GAACTG [1910] | TCAATAGAGCCAGTC ACACC [1911] |
| 71 | *PilAp2* | type IV pilin, involved in twitching motility and attachment | 21629637 | 483 | TGCCGTGAGTGAA ATCAG [1912] | CGTAGTTGGCTTTCC AGTT [1913] |
| 72 | *pilC* | Pilin biogenesis protein | 18535591 | 484 | GGTATCAACCCACT AAAGGTC [1914] | GTCCAGAGCTTCTAC CAGAG [1915] |
| 73 | *pvdD* | Pyoverdine synthetase D (siderophore) | 1633044 | 510 | GTCAAGGGTGTTG TCTGC [1966] | CTCTGCACAAACTCA GGG [1967] |
| 74 | *pyocin S1* | PyocinS1, bacteriocin | 286179 | 512 | CTTCAGTTCCGAGA TGCC [1970] | GTAACGAACGCTATC GGG [1971] |
| 75 | *pyocin S1im* | Immunity protein of pyocin S1 | 286179 | 513 | ATATACGGAAAAAG AGTTTCTTGAG [1972] | AGCACGCCATTCTTT AACTTC [1973] |
| 76 | *pyocin S2* | PyocinS2 | 286182 | 514 | TATACGGCTTCAGA CTTTCC [1974] | TGGCATAAGTATTGG CAG [1975] |
| 77 | *pys2( 1)* | PyocinS2 | 15595198 | 515 | TCGCCAATAAGAAG AAATTG [1976] | AGTGGTACTCGAAG GGTTCT [1977] |
| *78* | *pys2( 2)* | PyocinS2 | 15595198 | 516 | ATCCAGTATATTCC TGCTCG [1978] | TGCAATTTCTTCTTAT TGGC [1979] |
| 79 | *rbf30* 3 | B-band LPS (O-antigen) biosynthesis | 836903 | 517 | ATCGTTCTGGTCTT CCTTG [1980] | ACCAAAGAGTGTTGA TAGCC [1981] |
| 80 | *rhlB* | Rhamnosyl- transferase involved in rhamnolipid biosurfactant synthesis | 452502 | 519 | AACGCTTTCTCGAT CAGG [1984] | GATACTGTGCGGTTG TGA [1985] |
| 81 | *femA* | Factor essential for methicillin resistance | 4929298 | 801 | TACAGTCATTTCAC GCAAAC [2548] | TCACGCTCTTCATTT AGTTCT [2549] |
| 82 | *fmhA* | Factor essential for methicillin resistance | 4574232 | 825 | TGACTTCGGATGA GTTCAAT [2596] | GCTGTTAATTGTTGT TGCTTT [2597] |
| 83 | *fmhB* | Factor essential for methicillin resistance, putative | 4574234 | 818 | CTCACCCAAATGGA GATTTA [2582] | CTTGCTTTTCAGATG TTTCC [2583] |
| 84 | *gyrA* | DNA gyrase subunit A | 296393 | 60 | AGGCTCGTATGATT GAAAAA [1066] | GGTTTTGAGCACGAT ATGTAG [1067] |
| 85 | *gyrB* | DNA gyrase subunit B | 296393 | 61 | TTGGCACAACTGAT AAGACA [1068] | AAAAATCGTTCAAAG TGCTC [1069] |
| 86 | *hemB* | Porphobilinogene synthase | 2589180 | 62 | ATCATCAGCGACAA TGAGAG [1070] | TTTTTAACATCTCGA ACTATATCTAA [1071] |
| 87 | *hemN* | Oxygen-independent coproporphyrin ogen oxidase | 14349226 | 65 | TCTTCCATTCTCTC AGTCAAA [1076] | AGACCATGTATGTAG GTGGC [1077] |
| 88 | *hla* | α-Hemolysin | 46763 | 120 | GTCAGCTCAGTAAC AACAACAC [1186] | GTAGCGAAGTCTGG TGAAAA [1187] |
| 89 | *lip* | Lipase | 393265 | 68 | TGCATCTTCCATTT TAATAGC [1082] | GTCATTGTCCTTTGT TGGTT [1083] |
| 90 | *menC* | o-Succinyl-benzoic acid synthetase | 1255258 | 69 | TTGACAGCTTTGCA TTTTTA [1084] | GGCTTTGTTGCTTTT AATGA [1085] |
| 91 | *NAG* | N-acetyl-glucosaminidase | 2506026 | 125 | AAGTTGCTCAAATA CAAGCTG [1196] | TGATGTTAGCCCAAT CTACA [1197] |
| 92 | *norA2 3* | Quinolone resistance protein | 4115706 | 904 | GGTTACTTGTTGCT GCTTTT [2754] | CGTAATCGCAATCGA AATA [2755] |
| 93 | *nuc* | Nuclease | 46623 | 71 | TGGCTATCAGTAAT GTTTCG [1088] | GAATCAGCGTTGTCT TCG [1089] |
| 94 | *rpoB* | RNA polymerase B-subunit | 677848 | 73 | TGGAAGACATCGT AAACGTA [1092] | TGGATCAAAGAAACG TGAAT [1093] |
| 95 | *tag* | DNA- 3-methyladenine glycosidase | 6434027 | 81 | TTTTGATTTATCTTC TGACGG [1108] | CATTCATTTTATTCCC ACCT [1109] |
| 96 | *16SSa* | 16S rRNA | 46498 | 942 | TCTCTGATGTTAGC GGCGG [2830] | TCAGGCTTTCGCCCA TT [2831] |
| 97 | *clfB* | Clumping factor B | 3393010 | 4 | TAGCATAGCAACAA ACAGTGA [954] | GTTTTGACCTGAAGC TGTATC [955] |
| 98 | *EDIN* | Epidermal cell differentiation inhibitor | 152997 | 113 | AAAGATAGTTCTAA GATAAATGGTC [1172] | GGCCATTATTGGTCT GTTG [1173] |
| 99 | *elkT-abcA* | Lantibiotic epilancin K7 tranlocator | 1841513 | 896 | ATTAGAAATTGCGA CTGGTG [2738] | AGCGTGTCATATCCT TCATC [2739] |
| 100 | *epiP-bsaP* | Biosynthesis of lantibiotic epidermin; serine protease | 21204850 | 58 | CTTAGATGTCCCAT GCTGAT [1062] | GTCAAACGAGTGCTA ATGGT [1063] |
| 101 | *geh* | Lipase precursor; glycerol ester hyderolase | 153019 | 59 | TTCAATAGGCGTG GTGTC [1064] | TTATCTGTCGGTTTC TCTGG [1065] |
| 102 | *mreA* | ABC transporter | 7548683 | 907 | TACGATGACACCA GTCTTTG [2760] | ATCGACAAAACGTAC AGGAT [2761] |
| 103 | *murC* | UDP-N-acetylmuramoyl-L-alanine synthetase | 2642658 | 70 | GTATTATTGCTTGG GGTGAT [1086] | GGATATTTCTTTCGT GCTGT [1087] |
| 104 | *sak* | Staphylokinase | 47425 | 126 | TGTTATTATTCTCA TTTTCTTCAAT [1198] | ATGCTCTGATAAATC TGGGA [1199] |
| 105 | *sea* | Enterotoxin A | 153120 | 127 | TTTTATTCATTGCC CTAACG [1200] | TTTTCAGAGTTAATC GTTTTATTATC [1201] |
| 106 | *sec1* | Enterotoxin C | 46566 | 129 | AATTTTTGGCACAT GATTTA [1204] | CTTTTATGTCTAGTT CTTGAGCTG [1205] |
| 107 | *etb* | Exfoliative toxine B precursor | 15301 | 115 | TTTTAGCAGCGTCA ATTTTT [1176] | CTGATCCAGAGTTTC CTACCT [1177] |
| 108 | *seb* | Enterotoxin B | 152999 | 128 | CGTAGATGTGTTTG GAGCTA [1202] | CTTGAGCAGTCACCT TTTTC [1203] |
| 109 | *sstC* | Iron transport protein | 3724154 | 80 | TGATATTGGAAGAT ATTAGCATAGA [1106] | TGACAATCGCTTTAT TCATTT [1107] |
| 110 | *tst* | Toxic shock syndrome toxin | 18266750 | 138 | TTTTTATCGTAAGC CCTTTG [1222] | CAATAACCACCCGTT TTATC [1223] |
| 111 | *aacA-aphD* | Bifunctional aminoglycoside modifying enzyme | 3676412 | 843 | AGATTTGCCAGAAC ATGAAT [2632] | TGTTGCATTTAGTCT TTCCA [2633] |
| 112 | *aadD* | Aminoglycoside acetyl transferase | 21623792 | 837 | GCTATTGGTGTTTA TGGCTC [2620] | CTGATTGCTTAACTG CTTCA [2621] |
| 113 | *aph-A3* | 3'5'-aminoglycoside acetyl-transferase | 1272325 | 840 | GAGAATATCACCG GAATTGA [2626] | GCTCGACATACTGTT CTTCC [2627] |
| 114 | *blaZ* | β-lactam ase | 1575124 | 827 | TGCTTTAGTTTTAA GTGCATGT [2600] | TCCTTCATTACACTC TTGGC [2601] |
| 115 | *cat* | Chloramphenicol acetyl-transferase | 46651 | 862 | AGAAAATTGGGATA GAAAAGAA [2670] | CTGCAAGGCAACTG GTAT [2671] |
| 116 | *dfrA* | S1 dihydrofolate reductase | 3676404 | 859 | CAATTACCTTGGCA CTTACC [2664] | CCCTTTTCTACGCAC TAAAT [2665] |
| 117 | *ermA* | rRNA methylase | 13785452 | 852 | CCAGAAAAACCCTA AAGACA [2650] | AAAGAACACGATATT CACGG [2651] |
| 118 | *ermC* | Adenine methylase | 4138444 | 846 | ACACAGTCAAAACT TTATTACTTCA [2638] | CAACAAGTTTATTTT CTGTAGTTT [2639] |
| 119 | *msrSA* | Macrolide antibiotic resistance | 3892641 | 854 | GACAGATTTTCGAT CCCTTA [2654] | CCTTTTTGTTTTGAT GCACT [2655] |
| 120 | *mecA* | Penicillin binding protein 2' | 13785452 | 802 | AGTTGTAGTTGTCG GGTTTG [2550] | TGAAGTCGCTTTTCC TAGAG [2551] |

*S. aureus, E. coli* and *P. aeruginosa* genes were selected from the literature and databases, and compared by BLAST analysis to all other sequences available in the NCBI database. Primers were designed to amplify gene segments of 200-810 bp length and devoid of apparent homology with genes of other bacterial species and *Homo sapiens.* Gene segments were amplified by using the puReTaq Ready-To-Go PCR beads (Amersham Biosciences, Freiburg, Germany) and cloned into the pDrive Cloning Vector (Qiagen, Hilden, Germany) according to the recommendations of the suppliers and transformed into competent *Escherichia coli* (XL-1-Blue) cells using the calcium chloride protocol (Sambrook, J., Russel D.W., Molecular Cloning: A Laboratory Manual. Cold Spring Harbor Laboratory Press, NY (2001)).

For quality control purposes, all gene probes were partially sequenced and verified (with the BigDye kit 1.1 and an 377 DNA sequencer; Applied Biosystems, Foster City, USA). All sequences obtained were identical or substantially identical (>90% sequence identity) to those obtained from the database.

For DNA-probe production 120 recombinant plasmids containing *S. aureus, E. coli* and *P. aeruginosa* gene segments were used for re-amplification. Amplicons were purified and spotted in 4 replicates per slide on UltraGAPS^{™} Coated Slides (gamma amino propyl silane coated slides, Corning, NY, USA). Approximately 1 nl DNA (with a concentration of about 0.1 to about 0.2 ng/nl) per spot was spotted onto the slide with a Biorobotics Microgrid Microarrayer (Genomic Solutions, Ann Arbor, MI, USA).

### Example 5: Hybridisation and scanning

All experiments described represent dual co-hybridisations of two different target DNA samples labelled respectively with Cy3, Cy5 or Alexa647. After removal of unbound label, Cy3 and Cy5/Alexa647 labelled DNAs were pooled and mixed with 10 µg of Salmon Sperm DNA and 50 µg of poly-A-DNA. The mixture was frozen in liquid nitrogen and lyophilised in the dark. Prior to hybridisation the target DNA was reconstituted in 33 µl H₂O and 55 µl 2x hybridisation solution (Memorec Biotec GmbH, Cologne, Germany) and chemically denatured with 11 µl denaturation buffer D1 (Mirus) and neutralized with 11 µl buffer N1 (Mirus) according the instructions of the supplier. Hybridisation was automatically performed with a TECAN Hybridisation Station (HS400, TECAN, Salzburg, Austria). The arrays were prewashed at 60 °C for 1 min with 0.2% SDS and 4x SSC and prehybridised in 120 µl denatured prehybridisation buffer (Memorec) for 30 min at 60°C at mild agitation. After injection of 110 µl labelled DNA, hybridisation was performed at 60°C for 18 hours at mild agitation. The arrays were washed at 50°C in primary wash buffer (Memorec) - five cycles of 1 min wash time and 30 s soak time - and in secondary wash buffer (Memorec) - five cycles of 20 s wash time and 30 s soak time -, and finally dried at 30°C with N₂ (2.7 bar) for 3 min. Hybridised arrays were scanned with a Scan Array 5000 laser scanner (PerkinElmer). Laser light of wavelengths at 532 and 635 nm was used to excite Cy3 dye and Cy5/Alexa647 dye, respectively. Fluorescent images were analysed by the ImaGene software (BioDiscovery, El Segundo, CA, USA).

### Example 6: Specificity

In order to allow the simultaneous and rapid identification of *S. aureus, E. coli* and *P. aeruginosa* grown in blood culture specimens from septicemic patients, a microarray comprising a set of 40 *S. aureus,* 31 *E*. *coli* and 49 *P. aeruginosa* gene probes of 200 to 810 bp length was developed (Tab. 2).

The specificity of the DNA-chip was validated firstly (compare Example 1) with 45 well characterised clinical isolates and reference strains of the three target species as well as other related bacteria and secondly (compare Example 2) with 13 blood cultures from sepsis patients.

In all assays, three PCR-amplified DNA-segments, which had been added to each DNA preparation as a positive control, hybridised with the corresponding probes, indicating that labelling and hybridisation had performed efficiently.

Hybridisation experiments with *S. aureus, E. coli* and *P. aeruginosa* target DNAs, respectively, revealed specific hybridisation with the species-specific gene probes (Fig. 1). There was no cross-hybridisation between the three species with the exception of the *S. aureus* 16S rRNA gene probe (16SSa, Fig. 1 C), which hybridised also with *E. coli* and *P. aeruginosa* target DNA.

Identification of *E. coli, P. aeruginosa* and *S. aureus* reference strains, clinical isolates and blood cultures (BC) by m icroarray analysis corresponded by 100% with the conventional identification results (Fig. 1).

### Example 7: Detection and discrimination

### Example 7A: Detection and discrimination of E. coli

All DNA samples from 9 *E. coli* strains hybridised always with seven *E. coli* gene probes (*envZ, fes* (1) and (2), *nfrB, yacH, yagX, ycdS*) (Fig. 1 A, columns 19 to 27); in the following these genes are designated as core genes. With 14 *E. coli* gene probes variable hybridisation was observed including the antibiotic resistance gene probes *bla-TEM106, sul, strB* and *aacC2.* Such a variable hybridisation profile is expected for antibiotic resistance genes since acquired resistance to antimicrobials is strain specific. For 11 *E. coli* virulence gene probes *(eae, eltB, escR, escT, escU, espB, hlyA, hlyB, SLTII, toxA-LTPA, VT2vaB)* no hybridisation signals were detected with any of the tested *E. coli* isolates and blood cultures. Since these virulence genes are known to be specific for particular *E. coli* pathotypes (Bekal, S. et al., J. Clin. Microbiol., 41:2113-25 (2003)), it was not surprising that they were not present in the tested strains. The *eae, esc* and *esp* genes for example are encoded on a chromosomal pathogenicity island, which is typical for enteropathogenic *E*. *coli* exhibiting the unique virulence mechanism known as attaching and effacing (AE) (Elliott, S.J. et al., Mol. Microbiol., 28:1-4 (1998)). The alpha-hemolysin *(hly)* operon is encoded on a large plasmid of enterohemorrhagic *E. coli* strains (Schmidt, H. et al., Infect. Immun. 63:1055-61 (1995)).

### Example 7B: Detection and discrimination of Pseudomonas aeruginosa

DNA samples obtained from *P. aeruginosa* uniform ly hybridised with 32 out of 49 *P. aeruginosa* specific gene segments including the *mexA* gene probe (core genes). Variable hybridisation was observed with 17 probes allowing for discrimination of individual *P. aeruginosa* isolates (Fig. 1 B, columns 12 to 18).

### Example 7C: Detection and discrimination of S. aureus

Hybridisation experiments performed with 11 *S*. *aureus* target DNAs revealed signals in all assays with 16 *S. aureus* gene segments (core genes) (Fig. 1C, columns 1 to 11). Variable hybridisation was observed with 14 *S. aureus* gene probes including the 6 antibiotic resistance gene segments *aadD, aacA-aphD, blaZ, dfrA, ermA* and *mecA* and the virulence genes *sak, sea, sec1* and *EDIN.* The gene probes *geh, mreA, clfB* and *elkT-abcA* hybridised with 8, 10 (*mreA* and *clfB*) and 6 target DNAs respectively. However, PCR am plification of the four genes was positive for all 11 *S. aureus* target DNAs (not shown) suggesting that the four genes were present in all strains investigated and that these gene probes did not allow reliable detection of the four genes in *S. aureus.*

No hybridisation was observed with 10 probes including the toxin genes *seb, tst* and *etb.* In contrast to the community-acquired, multi-susceptible MRSA strain MW2 that hybridised to *mecA* and *blaZ* only, all six clinical MRSA strains showed the same multiresistant hybridisation pattern and their DNA hybridised to *ermA* (erythromycin resistance), *mecA* (oxacillin resistance) and the *aadD* gene (tobramycin resistance). As for the majority of multiresistant MRSA strains the *ermA* and *aadD* genes were shown to be located upstream and downstream, respectively, of the *mecA* gene in the mec chromosomal region (Chambers, H.F., Clin. Microbiol. Rev., 10:781-91 (1997); Polyzou, A. et al., J. Antimicrob. Chemother., 48:231-4 (2001)) . Hybridisation to the core gene probes permitted the identification of *S. aureus,* while hybridisation to antibiotic resistance gene probes allowed for discrimination of strains.

### Example 7D: Discrimination of E. coli. P. aeruginosa and S. aureus from related bacterial species

Co-hybridisation experiments performed with related bacterial species confirmed the high specificity of the DNA-chip (Fig. 1): For *S. epidermidis* and all other Coagulase-negative staphylococci, cross-hybridisation was observed only with the *S. aureus* 16S rRNA gene probe (16SSa, Fig. 1 C) and several common staphylococcal antibiotic resistance determinants (*aadD, aacA-aphD, aph-A3, blaZ, cat, dfrA, ermA, ermC, mdrSA, mecA*) (Fig. 1C, columns 28 to 36). There was no cross-hybridisation with other metabolic or virulence genes of *S. aureus.*

The *Micrococcus* spp. isolate showed no hybridisation with the DNA-chip (column 53). Streptococci (column 56 to 58) and enterococci (columns 54 and 55) showed hybridisation with the staphylococcal 16S RNA gene probe and once with the staphylococcal *aph-A3* aminoglycoside resistance gene probe *(Enterococcus* spp.) (Fig. 1C). Out of 12 strains of seven Gram-negative species (columns 41 to 52), two hybridised with the *S. aureus* 16S rRNA gene probe (*Klebsiella pneumoniae* and *Proteus mirabilis,* Fig. 1C, columns 41 and 47) and one clinical isolate of *Proteus mirabilis* hybridised with the *E. coli* resistance genes *bla-TEM106* (β-lactam resistance), *sul* (sulfonamide resistance) and *strB* (streptomycin resistance) (Fig. 1A, column 42). *Serratia, Stenotrophomonas, Acinetobacter* and *Enterobacter* species showed no cross-hybridisation with any gene probe.

### Example 8: Sensitivity

While the majority of *P. aeruginosa* probes allowed unambiguous identification, some probes showed variable hybridisation patterns when microarray hybridisation was performed with different target DNA samples prepared from the same isolate (Tab. 3).

**Tab. 3: Microarray hybridisation signals obtained with different target DNA preparations of Pseudomonas aeruginosa isolates.**

| Isolate | | | | | | | | | |
|---|---|---|---|---|---|---|---|---|---|
| | C4242 | | | C3853 | | C3045 | | C3755 | |
| DNA amount [ng] | 130^{a} | 382^{a} | 1350^{b} | 510^{a} | > 2400^{b} | 550^{a} | 2950^{b} | 1180^{b} | > 1600^{b} |
| BDR^{c} | 22 | 75 | 48 | 29 | 30 | 90 | 41 | 139 | 40 |
| No. of hybridised gene probes^{d} | 38 (88%) | 31 (72%) | 43 (100%) | 36 (88%) | 41 (100%) | 34 (89%) | 38 (100%) | 41 (95%) | 43 (100%) |

| | | | | | | | | | |
|---|---|---|---|---|---|---|---|---|---|
| ^{a} Labelled with Alexa647 ^{b} Labelled with Cy3 or Cy5 ^{c} BDR: Base to dye ratio; number of nucleotides per one dye molecule ^{d} Number of signals obtained with *P. aeruginosa* capture probes (total 49) after hybridisation with different DNA preparations. The percentage of specific hybridisations is compared to the highest number of signals obtained for each isolate (100%). | | | | | | | | | |

Successful hybridisation with strong fluorescent signals depends on efficiency of DNA labelling (ratio of bases per one dye molecule) and amount of labelled DNA. For the different target DNA preparations of four clinical isolates, variable hybridisation was observed with 14 gene probes (*uvrDII, vsml, pa1069, rhlR, rhlA, rhlB, 1046, pyocinS, pyocinS1im, plcR, plcN, PHZb, rbf303* and *pllAp2*). For example, for three different DNA preparations of isolate C4242, hybridisation to *Pseudomonas*-gene probes varied from 31 to 43 probes, respectively, depending on the labelling efficiency and amount of DNA (Tab. 3). The lowest number of signals was detected with 382 ng target DNA, that, however, showed a high base to dye ratio of 75. Overall, the results suggest that varying amounts of DNA and base to dye ratios influenced the hybridisation results of few gene probes. However, irrespective of the varying quality and quantity of the labelled target DNA, 35 of the 49 *P. aeruginosa* gene probes showed robust hybridisation results in all performed experiments.

### Example 9: Detection and characterisation of pathogens in blood cultures

Although DNA prepared from blood cultures comprises a mixture of human and bacterial DNA, the resulting hybridisation signals obtained with DNA from 1 ml positive blood culture allowed a clear and unambiguous characterisation of *S. aureus, E. coli* and *P. aeruginosa* present in 13 tested blood specimens (Fig. 1). In accordance to the VITEK2 characterisation, positive BACTEC^{®} cultures were identified by microarray hybridisation as multi-resistant MRSA (Fig. 1C, column 8), penicillin-resistant *S. aureus* (column 9 and 11), multi-susceptible *S. aureus* (column 10), *E. coli* (Fig. 1A, columns 26 and 27), *P*. *aeruginosa* (Fig. 1B, column18), and discriminated from oxacillin resistant *Staphylococcus epidermidis* (columns 33-35), *Proteus mirabilis* (column 43) and *Streptococcus pneumoniae* (columns 57 and 58).

### Example 10: Correlation between susceptibility testing and microarray hybridisation of selected antibiotic resistance genes

*S. aureus:* For 11 *Staphylococcus aureus* strains and blood cultures, susceptibility results determined by the VITEK2 system, Etest strips and disk diffusion tests were compared with the results of the m icroarray hybridisation assay for the simultaneous detection of antibiotic resistance genes (Tab. 4). The presence or absence of resistance genes as indicated by microarray hybridisation was confirmed by PCR with gene specific primers (results not shown).

**Tab. 4: Correlation between phenotypic and genotypic antibiotic resistance for 11 S. aureus isolates and blood cultures.**

| a) Penicillin resistance^{a} | Hybridisation with *mecA*/*blaZ* | |
|---|---|---|
| | No. pos. | No. neg. |
| 10 (resistant) | 10 | 0 |
| 1 (susceptible) | 0 | 1 |

| b) Oxacillin resistance | Hybridisation | with *mecA* |
|---|---|---|
| | No. pos. | No. neg. |
| 7 (resistant) | 7 | 0 |
| 4 (susceptible) | 0 | 4 |

| c) Erythromycin resistance | Hybridisation with *ermA, ermC* or *msrA* | |
|---|---|---|
| | No. pos. | No. neg. |
| 6 (resistant) | 6 | 0 |
| 5 (susceptible) | 0 | 5 |

| d) Tobramycin resistance | Hybridisation with *aadD* | |
|---|---|---|
| | No. pos. | No. neg. |
| 5 (resistant) | 5 | 0 |
| 6 (susceptible) | 0 | 6 |

| e) Gentamicin resistance | Hybridisation with *aacA-aphD* | |
|---|---|---|
| | No. pos. | No. neg. |
| 0 (resistant) | 0 | 0 |
| 11 (susceptible) | 0 | 11 |

| f) Trimethoprim resistance | Hybridisation with *dfrA* | |
|---|---|---|
| | No. pos. | No. neg. |
| 1 (resistant) | 0 | 1^{b} |
| 10 (susceptible) | 0 | 10 |

| | | |
|---|---|---|
| ^{a} Number of strains tested for resistance ^{b} *dfrA* gene detected by PCR | | |

For the *S. aureus* strains there was a 100% correlation between phenotypic resistance to penicillin and hybridisation to the *mecA* and/or *blaZ gene* (both genes confer resistance to penicillin, Tab. 4a). Phenotypic resistance to oxacillin correlated 100% with the hybridisation of the *mecA* gene (Table 4b), between resistance to erythromycin and hybridisation to the erythromycin resistance genes *ermA, ermC* or *msrSA* (Tab. 4c) and between resistance to tobramycin and hybridisation to the *aadD* gene (Tab. 4d). Furthermore, they all showed 100% correlation between phenotypic susceptibility to gentamicin and no hybridisation to the resistance genes *aacA-aphD* (Tab. 4e). Notably the *dfrA* gene of the trimethoprim resistant strain MW2 (MIC of 1 µg/ml) was not detected by microarray hybridisation (Tab. 4f), whereas PCR amplification revealed the presence of the *dfrA* gene.

*E. coli* and other Gram negative bacteria: The prototype microarray harboured only four *E. coli* and one *P. aeruginosa* resistance gene probes which do not yet allow a comprehensive prediction of antibiotic resistances. Nevertheless, hybridisation with the *E. coli* resistance gene probe *blaTEM106* was observed in one *P. mirabilis* and four *E. coli* strains and correlated with phenotypic ampicillin resistance for all five strains (Tab. 5).

**Tab. 5: Correlation between ampicillin/penicillin resistance, gentamicin/tobramycin resistance and streptomycin resistance and hybridisation with the resistance gene probes blaTEM-106, aacC2, aph-A3 and strB, respectively.**

| Species | Resistance phenotype^{a} | Hybridisation with | | | |
|---|---|---|---|---|---|
| | | *blaTEM-106*^{b} | *aacC2*^{b} | *aph-A3*^{c} | *strB*^{b} |
| *E. coli* ATCC | susceptible | - | - | - | - |
| 25922 | | | | | |
| *E. coli* C4821 | AMP, STR | + | - | - | + |
| *E. coli* F3437 | AMP | + | - | - | - |
| *E. coli* C3941 | AMP, STR | + | - | - | + |
| *E. coli* F1806^{d} | AMP, GEN, TOB, STR | + | + | + | + |
| *E. coli* C4547 | AMPi | - | - | - | - |
| *E. coli* C4230 | AMP | - | - | - | - |
| *E. coli* C3940 | susceptible | - | - | - | - |
| *E. coli* F1642^{d} | STR | - | - | - | + |
| *P. mirabilis* C4024 | AMP, STR | + | - | - | + |
| *P. mirabilis* C4403 | susceptible | - | - | - | - |
| *P. mirabilis* F1738 | susceptible | - | - | - | - |

| | | | | | |
|---|---|---|---|---|---|
| ^{a} AMP, ampicillin; GEN, gentamicin; STR, streptomycin; TOB, tobramycin; i, intermediate ^{b} *E. coli* gene probes ^{c} *S. aureus* gene probes ^{d} Positive blood culture | | | | | |

One *E. coli* blood culture showed also resistance to tobramycin and gentamicin. This phenotypic resistance correlated with the hybridisation of the *aacC2* gene probe for am inoglycoside resistance and the *S. aureus aph-A3* probe for tobramycin/kanamycin resistance (Tab. 5). For one *P. mirabilis* and four *E*. *coli* strains, phenotypic resistance to streptomycin correlated with hybridisation to the *strB* probe (Tab. 5).

All *P. aeruginosa* strains hybridised with the *mexA* gene probe (Fig. 1) and showed phenotypic resistance to tetracycline, trimethoprim/sulfamehoxazole, penicillins (ampicillin, mezlocillin) and cephalosporines (cefazolin, cefixime, cefuroxime). The *mexA-mexB-oprM* operon is a determinant for a three component efflux system responsible for intrinsic and acquired multiresistance in *P. aeruginosa* (β-lactams, fluoroquinolones, trimethoprim, sulphonamides, chloramphenicol and others) (Poole, K., Clin. Microbiol. Infect. 10:12-26 (2004) ).

### Example 11: Microarray for specific detection of S. aureus

### A) Strains and Cultures

Reference strains and clinical isolates: The following bacteria were purchased from the American Type Culture Collection (ATCC, Manassas, Va.) or the Deutsche Sammlung für Mikroorganismen und Zellkulturen (DMSZ, Braunschweig, Germany) and were used for evaluation of the specificity of the microarray: *Staphylococcus aureus* (ATCC 29213), *Staphylococcus epidermidis* (ATCC 12228; ATCC 18610) *Staphylococcus saprophyticus* (ATCC 14953), *Escherichia coli* (ATCC 25922), *Pseudomonas aeruginosa* (ATCC 27853). Ten clinical MRSA (methicillin resistant *S. aureus)* isolates were obtained from the inventors' clinical routine microbiology laboratory.

Bacterial cultures: Bacterial strains and clinical isolates were plated either onto sheep blood or onto Mueller-Hinton agar from 50% glycerol stocks. One colony was then picked and transferred to 5 ml Luria-Bertani (LB) broth and cultured overnight at 37°C.

Blood cultures: Aerobic blood culture bottles (BACTEC^{®} Plus aerobic, Becton Dickinson, Heidelberg, Germany) were inoculated with 100 CFU of *S. aureus* after adding 10 ml blood from healthy volunteers. A BACTEC^{®} 9240 blood culture system (Becton Dickinson) - a continuous reading, automated, and computed system detecting the growth of microorganisms by monitoring CO₂ production - was used for incubation according to the manufacturer's recommendations. Bottles with a positive growth index were removed from the incubator, and an aliquot of 1 ml of the blood culture suspension was taken aseptically with a needle syringe. The aliquot was equally divided, with one part for subculture on agar plates and CFU determination, and one part for DNA isolation.

Additionally, in order to test the microarray upon real conditions, samples were collected from ten clinical positive blood culture specimens cultivated under the same conditions as described above. Six of them were positive for different *S. aureus* strains and four for other bacterial species *(Staphylococcus epidermidis, Streptococcus mitis, E. coli* and *Klebsiella oxytoca*). Blood culture aliquots of 500 µl were used for DNA preparation.

### B) Generation of the S. aureus specific microarray

About 140 gene segments of *S. aureus* genes, but also a few of CoNS (SEQ I D NO: 177,178,179), were selected from the literature and nucleotide databases in order to cover different functional categories (virulence factors, species-specific metabolic and structural features, antibiotic resistance determinants). Tab. 6 provides the complete list of selected genes with gene symbol, gene function and SEQ ID NO of the segments.

**Tab. 6: Selected S. aureus genes, selected segments (SEQ ID NO) and primers used for segment amplification (SEQ ID NO)**

| Gene symbol | Functions | gene probe SEQ ID NO | Primer forward [SEQ ID NO] | Primer reverse [SEQ ID NO] |
|---|---|---|---|---|
| *atl* | autolysin | 99 | AGCTGAGACGACACA AGATCAAA [1144] | TTATATTGCGTTTCAAGA GCTGC [1145] |
| *aroA* | 3-phosphoshikimate 1-carboxyvinyl-transferase | 84 | ACCTTCAATATTCGCA TCC [1114] | TATTCCGATTATTAGGCG TAG [1115] |
| *aroC* | Chorismatsynthase | 83 | ATGAGATACCTAACAT CAGGAGAATCA [1112] | GCTATTCTTCCATCTAATT TACGATCATA [1113] |
| *aroE* | Shikimatdehydrogen ase | 95 | GTTATCAATTAATACA ACCCCTGAAGC [1136] | TGGAACTAATTCTCCTTC GATTGTTA [1137] |
| *aroF* | 3-deoxy-D-arabino-heptulosonate-7-phosphate synthase | 96 | GTAGTTGAAAATATG CCTGTTGGTGT [1138] | ATTACACCATTAACGATA ATTGGCAT [1139] |
| *aroG* | Chorismat-Mutase | 97 | AGACTTATTATCTAAA CGTGGTGAACTAGC [1140] | CAAATGATTTATTGCCGT CTCCTA [1141] |
| *asp23* | alkaline shock protein | 98 | AAAATTGCTGGTATC GCTGCA [1142] | GTCATTACATCATCAACTT GCATGTTA [1143] |
| *cata* | catalase | 1 | TAAATTGTTTAGATTA CAATCAGAGG [948] | TTCAAAGTTTTCGTATGTT TCA [949] |
| *clpC* | endopeptidase | 7 | AATGCTGCTAACCTG CGTGAT [960] | CACGTCTAACCGCTTTAC TGATTG [961] |
| *clpP* | endopeptidase | 8 | AAAGTAAAGAGTAGA CTAAGCTGTCTGCTC [962] | ACCTAATAAAATTCAAGC ATTGGGA [963] |
| *ctaA* | cytochrome biosynthesis | 9 | AAGAATTTAAAATGGT TAGGTGTCGTA [964] | ACGTAATCGTTTTGTTGC CAAATA [965] |
| *ctsR* | transcription repressor of class III stress genes homologue | 10 | AACGTCCCATGCCATT AATTTT [966] | TTGCGTTTCTATTTAGCTC AGACA [967] |
| *dltA* | D-alanine-D-alanyl carrier protein ligase | 11 | ACAGAGCAGCAAAAG CGTTAGTG [968] | GACCTTGAATGAACCATT GACCAT [969] |
| *dltB* | hypothethecal membrane transporter | 12 | CATATGGTGATTTTAC ATTCTTCTTAATTG [970] | CCTAACCATGTACTTTGT AACACTTTCA [971] |
| *dltC* | D-alanyl carrier protein | 13 | AAATTTATTAGCAGAA GTAGCAGAAAATG [972] | CTGAACTCTTCTAATGCTT CAACGATT [973] |
| *dnak* | Heat-shock-protein | 14 | TTTAGGCGAAAATATT GGTGAAGA [974] | TTTGTCGTCGTCTTTTACT TCGTT [975] |
| *elk T* | lantibiotic epilancin K7 translocator | 15 | GGTCTTATCGTTGCA GCTATCACTAT [976] | GAGCGTATCGCATAAATA ATCTTTTC [977] |
| *eno* | 2-phosphoglycerate dehydrogenase | 87 | CGATGTTCATCATTGG TACTGGTA [1120] | GGTGTTACTAAAGCAGTT GAAAACG [1121] |
| *glnA* | glutamine synthetase; belongs to the fem C locus | 17 | TAGTCACCATGAAGTT GCCCC [980] | CCTCTTGAAGATGGTACA CGGAT [981] |
| *gInR* | glutamine synthetase repressor; belongs to the fem C locus | 18 | CGAATGATGCAATCA GACGAAA [982] | CACCACGATTTATTGGCA AAGTT [983] |
| *grlA* | DNA topoisomerase IV subunit A | 19 | TTGAATCACCAAATTG AGGTTGT[984] | CAGTCGTTCAGATTTGAA TTTCTTT [985] |
| *grlB* | gyrase-like protein beta subunit B | 20 | AAATCCATCGAGATG GTAATATATATCA [986] | AAACTTAAAATACTTTCTG AATATTGATCAT [987] |
| *groEL* | stress response; heat shock protein | 21 | GTATGCAATTTGATCG TGGTTAT [988] | TGTTAATGCATCGCCTTC AAC[989] |
| *groES* | stress response; heat shock protein | 22 | [?] ATGTATGTTAGCACTC TTTAATGTTAAGTG [990] | GTTTAGTTGTGTTTCATTT TCGTT [991] |
| *gyrA* | DNA gyrase subunit A | 60 | CATCATTAATTCGATT CCCTGAAT [1066] | TCATTTACTTCATCTGCAT CCTCTT [1067] |
| *gyrB* | DNA gyrase subunit B | 61 | TCAATTTGACTTAAAA GAAGTTGGC [1068] | AAGATTTGTGGCATATCC TGAGTTA [1069] |
| *hemA* | Glutamyl-transfer RNA reductase | 23 | TGTCATATTATCAACA TGTAATCGAACTG [992] | AATATCAGTAATTCCAGA ACCAAGAAGAT [993] |
| *hemB* | Porphobilinogene synthase | 62 | TTGATAGACATAGAA GATTGAGATCATCAG [1070] | ACTTGAGAAATTGCTGTT TTAACAAGTAG [1071] |
| *hemC* | Porphobilinogene deaminase | 63 | GTAAATTAGTCGTTG GCTCCAGAAG [1072] | GGGATAGTGGTGTATGTG TTTTAGAAATA [1073] |
| *hemD* | Uroporphyrinogene III synthase | 64 | TGTTGATAACATTGCT GTGATAGGAA [1074] | AATGCATCGATTTGTTGA TGTTCTA [1075] |
| *hemE* | Uroporphyrinogene decarboxylase | 24 | AAAATGATCAAAGGT GAAGAAACATC [994] | AATCCTCGACATTTAATG CACCTAC [995] |
| *hemH* | Ferrochelatase | 25 | AATGGGATTATTAGTT ATGGCTTATGG [996] | GTGGATATGGATCATTAT TCTTTTCG [997] |
| *hemL* | GSA-1-Aminotransferase | 26 | ATGAGATATACGAAAT CAGAAGAAGCA [998] | CTAATCTTAAAGTATCCAA TGTAGCTTCTGTA [999] |
| *hemN* | oxygen-independent coproporphyrinogen oxidase | 65 | ACAGAATCAACCTGT AGATGAGTACTTAGA T [1076] | TGATATTCGTATAACGCA CACCATC [1077] |
| *hemY* | putative involved in a late step of protoheme IX synthesis | 27 | AAACAGCAAGATCCT AATATTGATGTAAC [1000] | CTCTACGTACAATCGATA CTAATTCATTATCT [1001] |
| *IepA* | GTP-binding protein | 28 | ATTAACAAAATTGATT TACCTGCTGC [1002] | CTATAACCAAAACCTAAT GCTTGTGAC [1003] |
| *IrgA* | holin-like protein LrgA | 29 | AAAGACGCATCAAAA CCAGCA [1004] | GGCTAATGACACCTAAAG AGTTAACAACT [1005] |
| *IrgB* | holin-like protein LrgA | 30 | GATTAACCACTTAGCA CTAAACACACCT [1006] | AATGTTTAACAAGCACTT CACGCT [1007] |
| *lytM* | peptidoglycan hydrolase | 31 | CGACAAACACCCAAC AAGCA [1008] | TGGCTGTTATACGCTTGG TTGT [1009] |
| *menB* | naphthoate synthase | 32 | GTTATCGTATTAACTG GTGAAGGTGATT [1010] | ACATTTAGTACATTACCG CCACCTAC [1011] |
| *menC* | o-succinylbenzoic acid synthetase | 69 | TTTAAGTCACAAATTG TAACACCGAA [1084] | TTAATTTAATTCTGGTCG GCTTTGT [1085] |
| *menD* | 2-Succinyl-6-hydroxy-2,4-cyclohexadiene-1-carboxylase | 33 | CGTAAGGGAAGTAGT TATCAGTCCG [1012] | TTAGCTGTATACTCGAAA TCCAATCC [1013] |
| *menE* | O-succinylbenzoic acid-CoA ligase | 34 | ATGGACTTTTGGTTAT ATAAACAAGCAC [1014] | TATTTCAGCAATGTCACC CGTATTA [1015] |
| *menF* | Isochorismate-Synthase | 35 | ATTGATAATTTACATC CAACACCTGC [1016] | TCACTATCTGGATCAGAA TCTTTAACAAT [1017] |
| *murC* | UDP-N-acetylmuramoyl-L-alanine synthetase | 70 | CTTGGGGTGATGATG AACATCTA [1086] | AAGTGTGTGGTTGAAATA CTGCAA [1087] |
| *mutL* | DNA mismatch repair protein | 38 | TCGTTTACATCATAAT AATCATCAGAC [1022] | ACACAGAGAATAACCAGG AGAAGA [1023] |
| *mutS* | DNA mismatch repair protein | 39 | TTGTAATTCACTTAAC TTCACCAATG [1024] | TCAAGTTGCGAAATTAGC TGA [1025] |
| *pbg* | porphobilinogen synthase | 41 | GGTGTTCCAAACTCA AAAGATGATATA [1028] | TTGACACCATAACTCATTA TAGGAATATTG [1029] |
| *pdhB* | pyruvate dehydrogenase (lipoamlde): subunit E1beta | 43 | TGACATTTCAAATCAA TCACATCG [1032] | TTGGTAACCAAACATTTTC AGCTT [1033] |
| *pdhC* | dihydrolipoamide acetyltransferase: subunit E2 | 44 | CTGGAGATACTATTG AAGAAGACGATG [1034] | TTGCTTTTACAGTTCTGTT TTCATCTAC [1035] |
| *pdhD* | dihydrolipoamide dehydrogenase: subunit E3 | 72 | CAGGTAAATTAGTTGT AGTTGGTGGAG [1090] | AGTGGTAAACCTGGAACG ATATCA [1091] |
| *rpoB* | RNA polymerase B-subunit | 73 | ATTGTTACGTGCATTA GGTTTCTCA [1092] | TTTCTACTGGCTCGTCTAT AACGC [1093] |
| *rsbU* | putative operon encoding alternate sigma factor | 45 | TAGTTATCGAGATTAT CAAAGATTGGTAGA [1036] | GTAATTGTGAGT GTCCAT AAGAATCCA [1037] |
| *rsbV* | putative operon encoding alternate sigma factor | 46 | TGAATCTTAATATAGA AACAACCACTCAAG [1038] | ACGATCTGACACACCTAA AATGTA [1039] |
| *rsbW* | putative operon encoding alternate sigma factor | 47 | TCTAAAGAAGATTTTA TCGAAATG [1040] | CCCACATTGTTATTTTCTT TGTAT [1041] |
| *sdrC* | serine-aspartate repeat protein multigene family | 139 | GAAAGTATTCTGTAG GTACTGCTTC [1224] | CCTTTATCAATCGCAATG TC [1225] |
| *sdrD* | serine-aspartate repeat protein multigene family | 140 | CGGGCAAATAAATAA AGATG [1226] | AACTGAAGATAAGCCGTT TG [1227] |
| *sdrE* | serine-aspartate repeat protein multigene family | 141 | TCTGTCGCAGTTTTAT CAGTTGAAG [1228] | GCAAAACAAGATGATGCA ACG [1229] |
| sgp | G protein | 48 | TGAGATAGATGCAAT CATGTTTATGG [1042] | GAAATAGGTACAATCTCT GTAAAGTCCATATA [1043] |
| *sigB* | sigma factor B | 78 | GATGGTTCAACTGTTA CGCTATTA [1102] | CTCTGAAGTCGTGATACA TGCA [1103] |
| *sirR* | sit operon metal dependent repressor | 49 | AATATAATTGGGAAG AAGTACATCAAGAAG [1044] | ATATTAGCAAATCGGTCT TATCTCTCA [1045] |
| *sodA* | superoxide dismutase | 50 | TTGAATTACCAAAATT ACCATACG [1046] | CTCCCAGAATAATGAATG GTTTAAAT [1047] |
| *sodB* | superoxide dismutase | 51 | GCGCATTTTGAAAAG GCA [1048] | GGGATAGCACGTAAAAGT GGAA [1049] |
| *srtA* | tanspeptidase;sorta se that anchors surface proteins to the cell wall | 91 | CTGGTCCTGGATATA CTGGTTCTTT [1128] | GATTAATGACAATCGCTG GTGTG [1129] |
| *sstA* | iron transport proteins | 52 | TTCGTTGTTCATAGGT GCGAGT [1050] | CTTTGAACAGCACTCGTG CG [1051] |
| *sstB* | iron transport protein | 53 | TATTGCCTTATTTAGA TGTATTGCTTTT [1052] | TCGTAGCTTCAAACACAT TTTCAA [1053] |
| *sstC* | iron transport protein | 54 | AATCAAATGATATTGG AAGATATTAGCA [1054] | TATTCAGTATCTTGTGCTA TTGTCATTG [1055] |
| *sstD* | iron transport protein | 55 | CATGCGGTAACAATT CTGATAAAGA [1056] | AATTTTCGCTTTAGGTGC AGCT [1057] |
| *stpC* | Potential ABC transporter | 92 | TTAACAATAGAACATT TAACAAAGAAG [1130] | CTCGAAATTAAGAAAGTA ACACC [1131] |
| *tag* | DNA-3-methyladenine glycosidase | 81 | GCATTTGGTACTAAA GATCCAGTCTACT [1108] | AACGAAAATACTGTTACT GGACCTAAAA [1109] |
| *trx* | thioredoxin reductase | 56 | GCTGACTATGAAGGT AAAGCTGACA [1058] | CAGCTAAGTTTTCTTTTG GTTGGA [1059] |
| *tyrA* | prephenate dehydrogenase | 82 | ATTCATTTAGTCAGTG GTCATCCAAT [1110] | GCTGTCGAATCATTTCTA AAATATACGT [1111] |
| *yhiN* | yhiN-protein | 57 | CAATTGGCTTTCGATT ATTGTTGTA [1060] | AACCAATGATCTAGTGTA AATGTTAAACCT [1061] |
| | Virulence Factors | | | |
| *clfA* | clumping factor A | 3 | GCTTCAGTGCTTGTA GGTACGTTAA [952] | TTGATTCACTAATTCCTCC GCAT [953] |
| *clfB* | clumping factor B | 4 | TAATGATACATCTGAT ATTAGTGCAAACAC [954] | TTTAGCATCAGCAGCATT TACTACC [955] |
| *cna* | collagen adhesin | 85 | TCGAGGAATTAACAA AGGTC [1116] | ATCAGGTTTAGTTGGTGG TG [1117] |
| *coa* | staphylocoagulase | 5 | TGTTAGGGATACACA ACATAAAACTGA [956] | GATTTTGTTTCAGATTCAC CGTATTT [957] |
| *ebpS* | cell surface elastin binding protein | 86 | GAACCTAGCCATCAA GACAG [1118] | GCATTATTAGAGGCATGT GG [1119] |
| *EDIN* | Epidermal cell differentiation inhibitor | 113 | TATCTTTAGCATTAAG CGTTTATTCAAT [1172] | TTTCTAACTAGATTTTCAT CATACTGGC [ 1173] |
| *eta* | exfoliative toxine A precursor | 114 | TGCATTTAATTTACCA AAAGAGCTT [1174] | TGGATAGCCTATTAATTC GAGTTTG [1175] |
| *etb* | exfoliative toxine B precursor | 115 | AAGAGCTTTATACACA CATTACGGATAA [1176] | CAAAATATTGAGAATCAT TGAACATTTC [1177] |
| *fbpA* | fibrinogen binding protein | 88 | CTCTTTTTACCTTTGA CGTTGGATT [1122] | GCCAAAATAGTGCTTCAA TATCAGA [1123] |
| *fib* | fibrinogen binding protein | 89 | GCTTTTCTGTGTGCAC TGACAGT [1124] | AGCGAAGGATACGGTCC AAG [1125] |
| *fnbA* | fibronectin-binding protein | 93 | TTACATCTGTACCCGT TTCCACTT [1132] | AAACTGCACAACCAGCAA ATATAGA [1133] |
| *fnbB* | fibronectin-binding protein | 90 | CCGCCTTAATTCCTTC TCCAAA [1126] | GCGAGTTGATTTGCCATC GG [1127] |
| *geh* | lipase precursor; glycerol ester hydrolase | 59 | GAACAAGGGAATGCG ATAACG [1064] | AGGTGCAGTTTTATCATT AGACGG [1065] |
| *hla* | alpha-hemolysin | 120 | ATGATGAAAATGAAA ACACGTATAGTC [1186] | ATTTGAGCTACTTCATTAT CAGGTAGTTG [1187] |
| *hlb* | beta-hemolysin | 121 | TGTTAATAAAGGCACT CCAGAGTTC [1188] | CTTTGATTGGGTAATGAT CTGAAAA [1189] |
| *hld* | delta-hemolysin | 110 | TTTTATCTTAATTAAG GAAGGAGTGATTTC [1166] | TAGTGAATTTGTTCACTG TGTCGATAA [1167] |
| *hlgA_C* | gamma-hemolysin component A; C-terminus | 117 | ACTGAAGTAGAAAGT CAGAACTCTAAAGGT [1180] | GTGTTTTCCAGTTCACTTC ATATTTAACT [1181] |
| *hlgA_N* | gamma-hemolysin component A; N-terminus | 116 | CTTAAAATTAAATAGA AAGAAAGT [1178] | ATGTTTTGAGTTATAGCT AATCGTT [1179] |
| *hlgB* | gamma-hemolysin component B | 118 | ATAGCTTCCACCCAAC ATATGGTAA [1182] | ATTTCACTTTGTGATTTTC CCAATC [1183] |
| *hlgC_C* | gamma-hemolysin component C; C-terminus | 119 | AATCAGCATTTGATAG CGATTTATTT [1184] | CCAATTGACTTCATATTTC ACAGTGTA [1185] |
| *hysA* | hyaluronate lyase | 111 | AAACATCAAATCGCT GTGGCT [1168] | GTGAAAGATGCCCTTGAG TGG [1169] |
| *lgGbg* | IgGbinding protein | 112 | GGGTTCTTGCTGTCTT TAAGTGATT [1170] | TATATCTCGAAGTTGCTA GTTGGGG [1171] |
| *lip* | lipase; glycerol ester hydrolase | 68 | TTTTAAGTGGTGGAC AAGCACAA [1082] | GATTGTTATTAGCGTTTG AATCTTGAC [1083] |
| *lukF* | leucocidin F | 122 | CATATGGCAGAGATA GTTATCATTCAACT [1190] | GATGTATGAGTTGCTCTT ATGTGATCTTTA [1191] |
| *lukS_C* | leucocidin S; C-terminus | 124 | AGTGTTCAATGGGGA ATAAAAGCTA [1194] | GATCCTTCTAAATAACTAT TGCCATAGTG [1195] |
| *lukS*_*N* | leucocidin S; C-terminus | 123 | AACATTGTCGTTAGG AATAATCACT [1192] | AATCAAAGCATCTTTGTTA TACTTT [1193] |
| *NAG* | N-acetylglucosaminidase; cytotoxin | 125 | ACTCAAACAGTTAGC AAGATTGCTC [1196] | TGCATTTACCCAACCAGT GC [1197] |
| *nuc* | nuclease | 71 | GCGATTGATGGTGAT ACGGTT [1088] | TTTTCGCTTGTGCTTCACT TTT [1089] |
| *sak* | staphylokinase | 126 | CGAGTTATTTTGAACC AACAGGC [1198] | GCGCAAAGATCGAAGTCA CTTAT [1199] |
| *sea* | staphylococcal enterotoxin A precursor | 127 | CTGATGTTTTTGATGG GAAGGTT [1200] | TGCATGTTTTCAGAGTTA ATCGTTT [1201] |
| *seb* | staphylococcal enterotoxin B precursor | 128 | ATATATTCTATTAAGG ACACTAAGTTAGGGA AT [1202] | AGTTAGGTAATCTAATTCT TGAGCAGTCA [ 1203] |
| *sec* | staphylococcal enterotoxin C precursor | 129 | GGCACATGATTTAATT TATAACATTAGTG [1204] | ATTCCTAGCTTTTATGTCT AGTTCTTGAG [1205] |
| *spa* | immunoglobulin G binding protein A precursor | 94 | GGTATTGCATCTGTAA CTTTAGG [1134] | AGGTTAGCACTTTGACTT GG [1135] |
| *sprV8* | V8 serine protease gene | 137 | ACAAACGCAGTCAAG CAAACA [1220] | CATTGTTGCTGGTTTAAC TACTTCAC [1221] |
| *tst* | toxic shock syndrom toxin | 138 | AAAATTACCTACTCCA ATAGAACTACCTTT [1222] | TTTCTGCTTCTATAGTTTT TATTTCATCA [1223] |
| | Antibiotic Resistance Determinants | | | |
| *aacA-aphD* | bifunctional aminoglycoside modifying enzyme | 843 | ACCCTCATAAAAATAA C TCCAAGAGC [2632] T | CTTTTTCTTTTGCATAACC TTTTTTC [2633] |
| *aadD* | aminoglycoside acetyl transferase; kanamycin resistance | 837 | AAGCAGAGTTCAGCC ATGAATG [2620] | CAGATGCGATGATGCAGA CC [2621] |
| *aphA3* | 3' 5'-aminoglycoside acetyltransferase; kanamycin resistance | 845 | CTGGTGGGAGAAAT GAAAACC [2636] | CCAGTTTTCGCAATCCAC ATC [2637] |
| *blal* | regulator protein | 814 | AGCAAGTTGAAATAT CTATGGCTGA [2574] | TCATTTAAAATGTCTCGCA ATTCTT [2575] |
| *blaR* | beta lactamase repressor | 790 | GAAAATTCACGTATGT CATGGAATC [2526] | GCATTTTTCCCAGATGGC TT [2527] |
| *blaZ* | beta-lactamase | 827 | GATAAGAGATTTGCC TATGCTTCAA [2600] | TGCTTAATTTTCCATTTGC GAT [2601] |
| *cadA* | Probable cadmium-transporting ATPase (Cadmium efflux ATPase) | 897 | TTGGATAGTTCAACAA AAACATTAACA [2740] | CATTTTTATCTTCTGTTAC CACTGGTT [2741] |
| *cadC* | Cadmium efflux system accessory protein homolog | 908 | TAGCAACCTCCCTTTG ATAC [2762] | ACAAAAGATATGTGTGAA GTTACC [2763] |
| *cat* | chloramphenicol acetyltransferase | 862 | CCTTCTTTGATTTATG CAATTATGG [2670] | GAAGCATGGTAACCATCA CATACA [2671] |
| *dfrA* | S1 dihydrofolate reductase; trimethoprim resistance | 859 | ATGACATTATCAATAA TTGTCGCTCA [2664] | AACATGACCAGATAACTC TTTAATTTCAT [2665] |
| *ermA* | rRNA methylase | 852 | TAGCTATCTTATCGTT GAGAAGGGAT [2650] | AAAGAAATTGTTCCTTCG ATAGTTTATT [2651] |
| *ermB* | adenine methylase | 851 | AACCGATACCGTTTAC GAAATTG [2648] | CGCTTGTAGAATCCTTCT TCAACA [2649] |
| *ermC* | adenine methylase | 846 | AACACAGTCAAAACTT TATTACTTCAAAAC [2638] | TTGCATAATTTATGGTCTA TTTCAATG [2639] |
| *femA* | factor essential for methicillin resistance | 801 | TAGGATTTGAACATAC TGGATTCCA [2548] | AAAGGCACTAACACACGG TCTTT [2549] |
| *fem D* | putative factor essential for methicillin resistance | 16 | TCAGGTGAAATGTTA GAATCAGCA [978] | TAAGTCACCAAATAAGAA TGGCG [ 979] |
| *fmhA* | similar to Staphylococcus aureus FemA and FemB proteins | 825 | GTTAACGATTGATGA AACGCAAA [2596] | TGCACCATCTTGTTCAATT TGTT [2597] |
| *fmhB* | essential for addition of glycine 1 to peptidoglycan precursor | 818 | GAGTTATTAAATAGTT TTGAACGCCG [2582] | TTCAGGATGTTCCTTTTCT AAAGCT [2583] |
| *linA* | lincosaminide nucleotidyltransferase | 850 | GATATAGGATACAAA ATAGAAGTTGATTGG [2646] | GGTCTTTTTCTGTTAATTC ATAACCG [2647] |
| *mecA* | penicillin binding protein 2' | 802 | ATATGAGATAGGCAT CGTTCCAAA [2550] | CTAATAGATGTGAAGTCG CTTTTCCT [2551] |
| *mecl* | mecl protein | 812 | TAATAAAACGTATGAA ATATCATCTGCA [2570] | TTTCATCTTGTGATAGATC TTCTTTTTC [2571] |
| *mecR* | mecl protein | 798 | TTTAAAGAATGGAAC CAAGATCAAA [2542] | TCGCCTTTTAAATGTGTA GCAAA [2543] |
| *mreA* | ABC transporter | 907 | GCAGTATTAGTACTTG ATGAACCAACG [2760] | GACAAAACGTACAGGATG TCCATAA [2761] |
| *mreB* | ABC transporter | 36 | ATGAGGTACTCTTTAA TTAGTGGTATCTTGA [1018] | ATCAGCTAATGAAATGAA GATTGCA [1019] |
| *mreR* | ABC transporter | 37 | GAAAATACAGAACTT GATGGTGAAATG [1020] | GCAAGACTCACATACACC ATAAACTTC [1021] |
| *msrA* | methionine sulfoxide reductase | 854 | TCATAAGCTGACAGA TTTTCGATCC [2654] | CTTTTAGATGAACCTACA AATCACTTGG [2655] |
| *norA* | quinolone resistance protein | 904 | TTAGCTTTCATAATGT CAGTTGTATTGA [2754] | ACAGTGTTTCAAATGCCG ATAAA [2755] |
| *pbpF* | penicillin-binding protein Pbp2b | 42 | AACACAATCGGAAAT GTTGGATAC [1030] | CTATCCCAATCCATAGAC GTGTTAA [1031] |
| *qacA* | quaternary ammonium compound resistance protein | 885 | CAATGGTTACAGGTT GTGGAAGA [2716] | GCCCACTACAGATTCTTC AGCTAC [2717] |
| *spc* | adenyltransferase AAD9 | 844 | ATATCAGGAAAGATT GGAAATACGG [2634] | AAAGAGGTATAGCCCATT CTGCA [2635] |

In order to obtain a high specificity level, each selected gene was compared to all other gene sequences available in the NCBI database using the BLAST algorithm. From that comparison, regions (ranging from 104 to 1434 bp) devoid of apparent homology with genes of other bacterial species and *Homo sapiens* were defined and amplified by PCR using specifically designed primers (see Tab. 6). A mixture of the total DNA from three different *S. aureus* reference strains and 100 clinical isolates was used as template for amplification of *S. aureus* gene segments, increasing therefore the chances to amplify more seldom occurring virulence and antibiotic resistance genes. PCR products were cloned into the plasm id pCR 2.1 -Topo Vector (Invitrogen, Karlruhe, Germany) which were used to transform competent *Escherichia coli* (XL-1-Blue) cells using the Calcium Chloride protocol (Seidman, C.E. et al., in: Ausubel, F.M. (ed.), Current Protocols in Molecular Biology, John Wiley & Sons, Inc. (2000)). Recombinant plasmids containing selected gene segments were screened by restriction analysis and verified by sequencing. The plasmid library constructed was used for re-amplification and production of the bulk DNA (10 µg at a concentration of 1 µM) from each clone necessary for printing the microchips. A Microgrid II spotter (BioRobotics, Cambridge, UK) and CMT-GAPS^{™} coated glass slides (Corning Incorporated, Corning, USA) were used. The complete array of 140 segments of genes was spotted in 3 replicates per slide.

### C) DNA purification

### a) Sample preparation

Bacterial cultures: Overnight cultures (5 ml) were harvested at 2,560g for 10 minutes. After discarding the supernatant the pellet was washed in 1 ml TE (10 mM Tris-HCl, pH 7.5 - 1 mM EDTA) and recovered by centrifugation at 17,900 g for 2 min.

Blood cultures: One ml of blood culture was mixed with 1 ml 0.1% Triton^{®}-X-100 and kept at room temperature for 5 min in order to disrupt blood human cells and resolve bacterial clumps. Bacterial cells were then harvested at 17,900 g for 10 min. Pellets were washed in 1 ml TE and recovered as described above.

### b) Purification of DNA

Pellets of harvested cells were resuspended in 500 µl lysis buffer (20 m M Tris-HCl, pH 8.0 - 2 mM EDTA, pH 8.0 - 1.2% Triton^{®}-X-100). To promote bacterial lysis, lysozyme and lysostaphin (Sigma, Taufkirchen, Germany) were added to reach a final concentration of 0.8 mg/ml and 0.2 mg/ml respectively. To lyse Gram negative bacterial cells, only lysozyme in the indicated concentration was used. Samples were then incubated for one hour at 37°C. After treatment with Proteinase K (1 mg/ml) (Sigma, Taufkirchen, Germany) for 5 hours at 55 °C under mild agitation, the samples were heated at 65°C for 30 min to inactivate Proteinase K and then cooled down to 37°C. Finally, a RNAse A treatment (0.2 mg/ml) was carried out for 1 hour at 37°C. A pretreatment with CTAB (Cethyltrimethylammonium bromide) was performed in order to release DNA from polysaccharide DNA complexes (Murray, M.G. and Thopson, W.F., Nucl. Acid Res. 8:4321-4325 (1980)). Salt concentration was adjusted to 0.7 M by adding 5 M NaCl. After thoroughly mixing, a 10% CTAB-0.7M NaCl solution was added to adjust the CTAB concentration to 1%.

The mixture was subsequently incubated under rotation for 20 min at 65°C and then extracted with one volume of chloroform/isoamyl alcohol (24:1). The samples were spun in a microcentrifuge (17,900 g) at room temperature. The aqueous phase was extracted once with chloroform/isoamyl alcohol (24:1), once with phenol/chloroform/isoamyl alcohol (25:24:1) and finally with chloroform/ isoamyl alcohol (25:24:1). Genomic DNA in the aqueous phase was sonified (3 x 10 s at 12% amplitude with 20 s breaks between pulses) in a Digital Sonifier (Branson, Schwaebisch Gmuend, Germany) to obtain fragments of around 1 kb, then precipitated with one volume of isopropanol and pelleted by centrifugation for 30 min at 4°C in a microcentrifuge at 17,900 g. The pellets were washed in 70% ethanol and resuspended in 50-100 µl TE (10 mM Tris-HCl, pH 7.5 - 1 m M EDTA). This DNA preparation was used when a high yield (hundreds of µg) was necessary, for example to prepare samples for several hybridisations experiments.

A second protocol using DNeasy Tissue Kit (QIAGEN, Hilden, Germany) adapted to bacterial cells and allowing DNA preparation in two hours, was also used when fast preparation was the priority. The abbreviations below pertain to the manufacturer's abbreviations for buffers used in the kit. The bacterial pellet was resuspended in 1 ml ddH₂O and the cell suspension frozen in liquid N₂ for 1 minute and then placed in a 60°C thermo-block for 2 minutes. Such a treatment was repeated once and bacteria were centrifuged again for 5 minutes at 14,000g. The resulting pellet was resuspended in 180 µl lysis buffer (20 mM Tris-HCl, pH 8.0 - 2 mM EDTA, pH 8.0 - 1.2% Triton-X-100). Specifically for *S. aureus* DNA preparation, lysostaphin (0.2mg/ml) was added and incubated 1 hour at 37°C. After, 200 µl of buffer AL (for gram positive bacteria) or buffer ATL (for gram negative) and 25 µl of the Proteinase K solution delivered with the kit were added and incubated at 70°C for 30 minutes. 200 µl of 100% ethanol were added and the suspension transferred to a DNeasy Mini Column placed into a collection tube. The column was centrifuged at 6,000 g for 1 minute, washed first with 500 µl of buffer AW1, centrifuged at 6,000 g for 1 minute, washed then with 500 µl of buffer AW2, and centrifuged at 14,000 g for 3 minutes. The column was then placed in a 1.5 ml tube and centrifuged once more at 14,000 g for 1 minute. DNA was eluted with 130 µl of buffer AE. After one minute the column was centrifuged at 6,000g for 1 minute. The eluate was re-loaded in the column and centrifuged again under the same conditions in order to increase the DNA yield.

### D) DNA labelling

Different amounts of DNA (5 ng to 5 µg) were labelled with 3 µl either of Cy5-dCTP or Cy3-dCTP (Amersham Pharmacia Biotech Europe, Freiburg, Germany) by random priming (1 x random primer/Klenow reaction buffer) using Klenow Polymerase (50units) (both from BioPrime DNA labelling Kit, Invitrogen, Karlsruhe, Germany) in the presence of 0.12 mM dATP's, dGTP's and dTTP's and 0.06 mM dCTP's, in a total volume of 50 µl. After 2 hours incubation at 37°C, the reaction was interrupted by adding 5 µl of 0.5 M EDTA and the probe purified either by MiniElute PCR or QlAquick Purification Kits (QIAGEN, Hilden, Germany), depending on the amount of labelled DNA applying two wash and two elution steps.

### E) Hybridisation and detection procedure

All experiments described in the present example represent co-hybridisation of two different DNA samples labelled respectively with Cy3 and Cy5. Cy3 and Cy5 belong to the cyanine family of fluorophores and were used as reporter molecules. The photochemical properties of the two CyDye fluors were as follows: Absorption maximum at 550 nm and emission maximum at 570 nm for Cy3 and for Cy5 at 649 nm and 670 nm, respectively.

After purification, Cy3 and Cy5 labelled DNA were pooled and 10 µg of Salmon Sperm DNA and 50 µg of polyA DNA were added. The mixture was frozen in liquid nitrogen and lyophilized in the dark. DNA microchips were automatically hybridised in a GeneTac Hybridisation Station (Genomic Solutions, Harvard, USA) following the Corning protocol.

Shortly, 110 µl of pre-hybridisation buffer (25% Formamide, 5x SSC, 0.1% SDS, 10 mg/ml BSA) were added to each slide and incubated for one hour at 42°C. Lyophilized samples were resuspended in 110µl of hybridisation buffer (25% Formamide, 5x SSC, 0.1% SDS), denatured for 3 minutes at 90°C, added to the slides, and incubated 4 hours at 42°C. After several washing steps using successively 2 x SSC/0.1 % SDS, 0.1 x SSC/0.1 % SDS, and 0.1 x SSC, slides were dried by a 2 min centrifugation step (1000 g) and read in a Scan Array 5000 (Perkin Elmer, Boston, USA) using emission filters for Cy3 and Cy5 in two separate channels. Fluorescence intensities as hybridisation indicators were then analyzed by the software ImaGene (BioBiscovery, Marina Del Rey, USA). Spots were found and segmented in order to select areas of recognizable signals for analysis. Intensity of fluorescence of each spot was measured, signal to local background ratios were calculated, spot morphology and deviation from expected spot position were considered. Cut off values for those parameters were empirically determined in pilot experiments and used to tag spots either as positive or as negative.

### F) Validation of the detection system

The experimental approach adopted in present example required dual-dye hybridisations. It was therefore necessary to verify at first whether DNA samples from the same source, labelled with one or the other fluorochrome, would produce the same hybridisation pattern. Co-hybridisation experiments, combining two identical samples of 2 µg of *S. aureus* DNA, produced strictly similar hybridisation results whatever fluorochrome was used for labelling (Fig. 2A). For better presentation gray scale images from scanning were converted in false-color, where green and red color represent intensity of Cy3 and Cy5 fluorochromes respectively. All spots showed double-hybridisation - yellow color meaning the overlay between green (here assigned to Cy3 labelled DNA) and red signals (Cy5 labelled DNA). Signal intensities from both channels strongly correlated (r²=0,97) (Fig. 2B).

### G) Sensitivity of detection

*S. aureus* DNA samples in decreasing amounts (from 2 µg to 5 ng) were labelled and hybridised in order to determine the minimum amount of DNA producing the expected hybridisation pattern for a certain strain. Such expected patterns were defined as those produced by the hybridisation of 2 µg of DNA. From 2 µg to 50 ng no significant differences in the hybridisation pattern were observed with no false negative spots. Detection of 20 ng DNA was still satisfying with only 5% of false negative and false positive. However, 5 ng of labelled DNA yielded weak signals with almost 95% of false negative spots (data not shown). The limit of sensitivity of the *S. aureus* microarray was then considered as being 20 ng DNA which corresponds approximately to 7 x 10⁶ *S. aureus* CFU *(S. aureus* genome 2.5 x 10⁶ bp. 2.8 fg DNA per cell).

### H) Specificity of detection

The specificity of the *S. aureus* microchip was demonstrated by six independently performed co-hybridisation experiments. Visual examination of pictures showing results of co-hybridisation of *S. aureus* DNA with *Pseudomonas aeruginosa* or *Escherichia coli* DNA revealed no cross-hybridisation between *S. aureus* selected gene segments and DNA probes from those Gram negative bacteria (data not shown). Transcribing these data in a bar code showing positive or negative spots (Fig. 3A and B) confirmed that only the *S. aureus* DNA sample hybridised with spotted probes.

The specificity of the microarray could be demonstrated even below the genus level. As shown in Fig. 4, some spotted S. *aureus* probes cross-hybridised with *S. epidermidis* and *S. saprophyticus* DNA samples. This is not surprising as these species are phylogenetically closely related. However, genes coding for *S. aureus* specific proteins as nuclease (*nuc*)*,* clumping factors A and B (*clfA* and *B*)*,* protein A (*spa*)*,* V8 serine protease (*sprV8*) and alpha and beta hemolysins *(hla* and *hlb)* exclusively hybridised with *S. aureus* DNA. The presence/absence of such genes allowed unambiguous discrimination between *S. aureus* and CoNS.

### I) S. aureus strain profiling

The principle of the *S. aureus* microarray was tested as a tool for strain profiling. A distinctive hybridisation pattern could be established for reference strains and 10 selected clinical isolates. For instance when DNA from clinical isolates T100 and T103 were labelled with Cy5 and Cy3, respectively, and co-hybridised, both isolates were identified as *S. aureus,* since both contained species-specific genes as e.g. clumping factor A and B (Fig. 5A).

Moreover, both strains are methicillin resistant (*mecA* positive), but only T100 contained the beta-lactamase gene. The hybridisation of T103 DNA reveals the presence of *ermA, ermB* and *aacA* genes indicating that the strain is resistant to erythromycin and aminoglycosides.

Apparently, T103 harbors the genes encoding enterotoxines A (*eta*) and B (*etb*) while in T100 the gene encoding enterotoxin C *(etc)* is present. The presence or absence of these genes was confirmed by PCR assays (Fig. 5B) and the antibiotic resistance was verified by classical antibiograms ( Sahm, D. & Washington, J. A. (1991). Antibacterial susceptibility tests: dilution methods. In: Manual of Clinical Microbiology (Balows, A., Ed.), pp. 1105-16. American Society for Microbiology, Washington DC, USA) (data not shown).

### J) Detection of S. aureus in spiked positive BACTEC^{®} cultures

One possible application of the *S. aureus* microarray is to detect the bacterium growing in blood culture, i.e. after the BACTEC^{®} signals bacterial growth. Blood culture bottles were spiked with 100 CFU of *S. aureus.* After the automated culturing system indicated bacterial growth, 1 ml was withdrawn for DNA extraction.

As shown in Fig. 6A, DNA samples prepared from sterile blood culture show no crosshybridisation with spotted S. *aureus* probes. A 2 µg DNA sample derived from blood culture containing *S. aureus* cells revealed a hybridisation pattern almost completely identical to a DNA sample isolated from an overnight LB culture inoculated with a *S. aureus* colony (Fig. 6B).

These data underscore the high sensitivity and specificity of the detection system since blood culture DNA comprises a mixture of human and bacterial DNA. Co-hybridisation between DNA from blood culture positive for *S. aureus* and CoNS DNA also allowed clear identification since only the *S. aureus* probe hybridised to *S. aureus* species-specific genes (data not shown).

### K) Detection of S. aureus in positive BACTEC^{®} cultures inoculated with clinical specimens

Co-hybridisation with DNA from clinical blood cultures positive for *S.aureus* and CoNS (*Staphylococus epidermidis), Streptococcus mitis, E. coli* and *Klebsiella oxytoca* allowed clear species identification since the *S.aureus* probes hybridised to *S.aureus* species-specific genes only. *Staphylococcus epidermidis* positive blood culture DNA hybridised to staphylococcal metabolic genes and to some antibiotic resistance determinant genes only. No cross-hybridisation was detected between DNA from the two gram-negative strains and the *Streptococcus* strain and *S*. *aureus* spotted gene probes (data not shown).

### Sequence Listing - Free text

a) Probe sequences

| SEQ ID NO | Probe name | Template source |
|---|---|---|
| 1 | cataSaur_1_1 | *Staphylococcus aureus* |
| 2 | cataSaur_1_2 | *Staphylococcus aureus* |
| 3 | clfA_1_1 | *Staphylococcus aureus* |
| 4 | clfB_1_1 | *Staphylococcus aureus* |
| 5 | coa_1_1 | *Staphylococcus aureus* |
| 6 | coa_1_2 | *Staphylococcus aureus* |
| 7 | I-clpC_1_1 | *Staphylococcus aureus* |
| 8 | I-clpP_1_1 | *Staphylococcus aureus* |
| 9 | I-ctaA_1_1 | *Staphylococcus aureus* |
| 10 | I-ctsR_1_1 | *Staphylococcus aureus* |
| 11 | I-dltA_1_1 | *Staphylococcus aureus* |
| 12 | I-dltB_1_1 | *Staphylococcus aureus* |
| 13 | I-dltC_1_1 | *Staphylococcus aureus* |
| 14 | I-dnaK_1_1 | *Staphylococcus aureus* |
| 15 | I-elkT_1_1 | *Staphylococcus aureus* |
| 16 | I-femD_1_1 | *Staphylococcus aureus* |
| 17 | I-glnA_1_1 | *Staphylococcus aureus* |
| 18 | I-glnR_1_1 | *Staphylococcus aureus* |
| 19 | I-grlA_1_1 | *Staphylococcus aureus* |
| 20 | I-grlB_1_1 | *Staphylococcus aureus* |
| 21 | I-groEL_1_1 | *Staphylococcus aureus* |
| 22 | I-groES_1_1 | *Staphylococcus aureus* |
| 23 | I-hemA_1_1 | *Staphylococcus aureus* |
| 24 | I-hemE_1_1 | *Staphylococcus aureus* |
| 25 | I-hemH_1_1 | *Staphylococcus aureus* |
| 26 | I-hemL_1_1 | *Staphylococcus aureus* |
| 27 | I-hemY_1_1 | *Staphylococcus aureus* |
| 28 | I-lepA_1_1 | *Staphylococcus aureus* |
| 29 | I-lrgA_1_1 | *Staphylococcus aureus* |
| 30 | I-lrgB_1_1 | *Staphylococcus aureus* |
| 31 | I-lytM_1_1 | *Staphylococcus aureus* |
| 32 | I-menB_1_1 | *Staphylococcus aureus* |
| 33 | I-menD_1_1 | *Staphylococcus aureus* |
| 34 | I-menE_1_1 | *Staphylococcus aureus* |
| 35 | I-menF_1_1 | *Staphylococcus aureus* |
| 36 | I-mreB_1_1 | *Staphylococcus aureus* |
| 37 | I-mreR_1_1 | *Staphylococcus aureus* |
| 38 | I-mutL_1_1 | *Staphylococcus aureus* |
| 39 | I-mutS_1_1 | *Staphylococcus aureus* |
| 40 | I-NAG_1_1 | *Staphylococcus aureus* |
| 41 | I-pbg_1_1 | *Staphylococcus aureus* |
| 42 | I-pbpF_1_1 | *Staphylococcus aureus* |
| 43 | I-pdhB_1_1 | *Staphylococcus aureus* |
| 44 | I-pdhC_1_1 | *Staphylococcus aureus* |
| 45 | I-rsbU_1_1 | *Staphylococcus aureus* |
| 46 | I-rsbV_1_1 | *Staphylococcus aureus* |
| 47 | I-rsbW_1_1 | *Staphylococcus aureus* |
| 48 | I-sgp_1_1 | *Staphylococcus aureus* |
| 49 | I-sirR_1_1 | *Staphylococcus aureus* |
| 50 | I-sodA_1_1 | *Staphylococcus aureus* |
| 51 | I-sodB_1_1 | *Staphylococcus aureus* |
| 52 | I-sstA_1_1 | *Staphylococcus aureus* |
| 53 | I-sstB_1_1 | *Staphylococcus aureus* |
| 54 | I-sstC_1_1 | *Staphylococcus aureus* |
| 55 | I-sstD_1_1 | *Staphylococcus aureus* |
| 56 | I-trx_1_1 | *Staphylococcus aureus* |
| 57 | I-yhiN_1_1 | *Staphylococcus aureus* |
| 58 | epiP-bsaP_1_1 | *Staphylococcus aureus* |
| 59 | geh_1_1 | *Staphylococcus aureus* |
| 60 | gyrA_1_1 | *Staphylococcus aureus* |
| 61 | gyrB_1_1 | *Staphylococcus aureus* |
| 62 | hemB_1_1 | *Staphylococcus aureus* |
| 63 | hemC_1_1 | *Staphylococcus aureus* |
| 64 | hemD_1_1 | *Staphylococcus aureus* |
| 65 | hemN_1_1 | *Staphylococcus aureus* |
| 66 | hsdS_1_1 | *Staphylococcus aureus* |
| 67 | hsdS_2_1 | *Staphylococcus aureus* |
| 68 | lip_1_1 | *Staphylococcus aureus* |
| 69 | menC_1_1 | *Staphylococcus aureus* |
| 70 | murC_1_1 | *Staphylococcus aureus* |
| 71 | nuc_1_1 | *Staphylococcus aureus* |
| 72 | pdhD_1_1 | *Staphylococcus aureus* |
| 73 | rpoB_1_1 | *Staphylococcus aureus* |
| 74 | SAV0431_1_1 | *Staphylococcus aureus* |
| 75 | SAV0439_1_1 | *Staphylococcus aureus* |
| 76 | SAV0440_1_1 | *Staphylococcus aureus* |
| 77 | SAV0441_1_1 | *Staphylococcus aureus* |
| 78 | sigB_1_1 | *Staphylococcus aureus* |
| 79 | spa_1 _2 | *Staphylococcus aureus* |
| 80 | sstC_1_1 | *Staphylococcus aureus* |
| 81 | tag_1 _1 | *Staphylococcus aureus* |
| 82 | tyrA_1_1 | *Staphylococcus aureus* |
| 83 | I-aroC_1_1 | *Staphylococcus aureus* |
| 84 | I-aroA_1_1 | *Staphylococcus aureus* |
| 85 | I-cna_1_1 | *Staphylococcus aureus* |
| 86 | I-ebpS_1_1 | *Staphylococcus aureus* |
| 87 | I-eno_1_1 | *Staphylococcus aureus* |
| 88 | I-fbpA_1_1 | *Staphylococcus aureus* |
| 89 | I-fib_1_1 | *Staphylococcus aureus* |
| 90 | I-fnbB_1_1 | *Staphylococcus aureus* |
| 91 | I-srtA_1_1 | *Staphylococcus aureus* |
| 92 | I-stpC_1_1 | *Staphylococcus aureus* |
| 93 | I-fnbA_1_1 | *Staphylococcus aureus* |
| 94 | I-spa_1_1 | *Staphylococcus aureus* |
| 95 | I-aroE_1_1 | *Staphylococcus aureus* |
| 96 | I-aroF_1_1 | *Staphylococcus aureus* |
| 97 | I-aroG_1_1 | *Staphylococcus aureus* |
| 98 | I-asp23_1_1 | *Staphylococcus aureus* |
| 99 | I-atl_1_1 | *Staphylococcus aureus* |
| 100 | bsaE_1_1 | *Staphylococcus aureus* |
| 101 | bsaG_1_1 | *Staphylococcus aureus* |
| 102 | cap5h_1_1 | *Staphylococcus aureus* |
| 103 | cap5i_1_1 | *Staphylococcus aureus* |
| 104 | cap5j_1_1 | *Staphylococcus aureus* |
| 105 | cap5k_1_1 | *Staphylococcus aureus* |
| 106 | cap8H_1_1 | *Staphylococcus aureus* |
| 107 | cap81_1_1 | *Staphylococcus aureus* |
| 108 | cap8J_1_1 | *Staphylococcus aureus* |
| 109 | cap8K_1_1 | *Staphylococcus aureus* |
| 110 | I-hld_1_1 | *Staphylococcus aureus* |
| 111 | I-hysA_1_1 | *Staphylococcus aureus* |
| 112 | I-lgGbg_1_1 | *Staphylococcus aureus* |
| 113 | EDIN_1_1 | *Staphylococcus aureus* |
| 114 | eta_1_1 | *Staphylococcus aureus* |
| 115 | etb_1_1 | *Staphylococcus aureus* |
| 116 | hglA_1_1 | *Staphylococcus aureus* |
| 117 | hglA_2_1 | *Staphylococcus aureus* |
| 118 | hglB_1_1 | *Staphylococcus aureus* |
| 119 | hglC_2_1 | *Staphylococcus aureus* |
| 120 | hla_1_1 | *Staphylococcus aureus* |
| 121 | hlb_1_2 | *Staphylococcus aureus* |
| 122 | lukF_1_1 | *Staphylococcus aureus* |
| 123 | lukS_1_1 | *Staphylococcus aureus* |
| 124 | lukS_2_1 | *Staphylococcus aureus* |
| 125 | NAG_1_1 | *Staphylococcus aureus* |
| 126 | sak_1_1 | *Staphylococcus aureus* |
| 127 | sea_1_1 | *Staphylococcus aureus* |
| 128 | seb_1_1 | *Staphylococcus aureus* |
| 129 | sec1_1_1 | *Staphylococcus aureus* |
| 130 | seg_1_1 | *Staphylococcus aureus* |
| 131 | seh_1_1 | *Staphylococcus aureus* |
| 132 | sel_1_1 | *Staphylococcus aureus* |
| 133 | set 15_1_1 | *Staphylococcus aureus* |
| 134 | set6_1 _1 | *Staphylococcus aureus* |
| 135 | set7_1_1 | *Staphylococcus aureus* |
| 136 | set8_1 _1 | *Staphylococcus aureus* |
| 137 | sprV8_1_1 | *Staphylococcus aureus* |
| 138 | tst_1 _1 | *Staphylococcus aureus* |
| 139 | I-sdrC_1_1 | *Staphylococcus aureus* |
| 140 | I-sdrD_1_1 | *Staphylococcus aureus* |
| 141 | I-sdrE_1_1 | *Staphylococcus aureus* |
| 142 | b1169_1_1 | *Escherichia coli* |
| 143 | envZ_1_1 | *Escherichia coli* |
| 144 | fliCb_1_1 | *Escherichia coli* |
| 145 | nfrB_1_1 | *Escherichia coli* |
| 146 | nlpA_1_1 | *Escherichia coli* |
| 147 | pilAe_1_1 | *Escherichia coli* |
| 148 | yacH_1_1 | *Escherichia coli* |
| 149 | yagX_1_1 | *Escherichia coli* |
| 150 | ycdS_1_1 | *Escherichia coli* |
| 151 | yciQ_1_1 | *Escherichia coli* |
| 152 | ym cA_1_1 | *Escherichia coli* |
| 153 | b1202_1_1 | *Escherichia coli* |
| 154 | eae_1_1 | *Escherichia coli* |
| 155 | eltB_1_1 | *Escherichia coli* |
| 156 | escR_1_1 | *Escherichia coli* |
| 157 | escT_1_1 | *Escherichia coli* |
| 158 | escU_1_1 | *Escherichia coli* |
| 159 | espB_1_1 | *Escherichia coli* |
| 160 | fes_1_1 | *Escherichia coli* |
| 161 | fes_2_1 | *Escherichia coli* |
| 162 | fteA_1_1 | *Escherichia coli* |
| 163 | hlyA_1_1 | *Escherichia coli* |
| 164 | hlyB_1_1 | *Escherichia coli* |
| 165 | iucA_1_1 | *Escherichia coli* |
| 166 | iucB_1_1 | *Escherichia coli* |
| 167 | iucC_1_1 | *Escherichia coli* |
| 168 | papG_1_1 | *Escherichia coli* |
| 169 | rfbE_1_1 | *Escherichia coli* |
| 170 | shuA_1_1 | *Escherichia coli* |
| 171 | SLTII_1_1 | *Escherichia coli* |
| 172 | toxA- LTPA_1_1 | *Escherichia coli* |
| 173 | VT2vaB_1_1 | *Escherichia coli* |
| 174 | ardeSE0106_1_1 | *Staphylococcus epidermidis* |
| 175 | ardeSE0107_1_1 | *Staphylococcus epidermidis* |
| 176 | aroiSE0105_1_1 | *Staphylococcus epidermidis* |
| 177 | atlE_1_1 | *Staphylococcus epidermidis* |
| 178 | agrB_1_1 | *Staphylococcus epidermidis* |
| 179 | agrC_1_1 | *Staphylococcus epidermidis* |
| 180 | alphSE1368_1_1 | *Staphylococcus epidermidis* |
| 181 | gad_1_1 | *Staphylococcus epidermidis* |
| 182 | glucSE1191_1_1 | *Staphylococcus epidermidis* |
| 183 | hsp10_1_1 | *Staphylococcus epidermidis* |
| 184 | icaA_1_1 | *Staphylococcus epidermidis* |
| 185 | icaB_1_1 | *Staphylococcus epidermidis* |
| 186 | mvaSSepid_1_1 | *Staphylococcus epidermidis* |
| 187 | nitreSE1972_1_1 | *Staphylococcus epidermidis* |
| 188 | nitreSE1974_1_1 | *Staphylococcus epidermidis* |
| 189 | nitreSE1975_1_1 | *Staphylococcus epidermidis* |
| 190 | oiamtSE1209_1_1 | *Staphylococcus epidermidis* |
| 191 | ORF1Sepid_1_1 | *Staphylococcus epidermidis* |
| 192 | ORF3bSepid_1_1 | *Staphylococcus epidermidis* |
| 193 | qacR_1_1 | *Staphylococcus epidermidis* |
| 194 | sin_1_1 | *Staphylococcus epidermidis* |
| 195 | ureSE1861_1_1 | *Staphylococcus epidermidis* |
| 196 | ureSE1863_1_1 | *Staphylococcus epidermidis* |
| 197 | ureSE1864_1_1 | *Staphylococcus epidermidis* |
| 198 | ureSE1865_1_1 | *Staphylococcus epidermidis* |
| 199 | ureSE1867_1_1 | *Staphylococcus epidermidis* |
| 200 | gcaD_1_1 | *Staphylococcus epidermidis* |
| 201 | hld_orf5_1_1 | *Staphylococcus epidermidis* |
| 202 | icaC_1_1 | *Staphylococcus epidermidis* |
| 203 | icaD_1_1 | *Staphylococcus epidermidis* |
| 204 | icaR_1_1 | *Staphylococcus epidermidis* |
| 205 | psm_betaiand2_1_1 | *Staphylococcus epidermidis* |
| 206 | purR_1_1 | *Staphylococcus epidermidis* |
| 207 | spoVG_1_1 | *Staphylococcus epidermidis* |
| 208 | yabJ_1_1 | *Staphylococcus epidermidis* |
| 209 | folQShaemolyt_1_1 | *Staphylococcus haemolyticus* |
| 210 | mvaCShaemolyticus_1_1 | *Staphylococcus haemolyticus* |
| 211 | mvaDShaemolyt_1_1 | *Staphylococcus haemolyticus* |
| 212 | mvaK1Shaemolyticus_1_1 | *Staphylococcus haemolyticus* |
| 213 | mvaSShaemolyticus_1_1 | *Staphylococcus haemolyticus* |
| 214 | RNApolsigm_1_1 | *Staphylococcus haemolyticus* |
| 215 | IipShaemolyt_1_1 | *Staphylococcus haemolyticus* |
| 216 | agrB2Stalugd_1_1 | *Staphylococcus lugdunensis* |
| 217 | agrC2Stalugd_1_1 | *Staphylococcus lugdunensis* |
| 218 | agrCStalugd_1_1 | *Staphylococcus lugdunensis* |
| 219 | slamStalugd_1_1 | *Staphylococcus lugdunensis* |
| 220 | fblStalugd_1_1 | *Staphylococcus lugdunensis* |
| 221 | slushABCStalugd_1_1 | *Staphylococcus lugdunensis* |
| 222 | RNApoIsigmSsapro_1_1 | *Staphylococcus saprophyticus* |
| 223 | RNApoIsigmSsapro_1_2 | *Staphylococcus saprophyticus* |
| 224 | msrw1Stwar_1_1 | *Staphylococcus warneri* |
| 225 | nukMStwar_1_1 | *Staphylococcus warneri* |
| 226 | proDStwar_1_1 | *Staphylococcus warneri* |
| 227 | proMStwar_1_1 | *Staphylococcus warneri* |
| 228 | sigrpoStwar_1_1 | *Staphylococcus warneri* |
| 229 | tnpStwar_1_1 | *Staphylococcus warneri* |
| 230 | gehAStwar_1_1 | *Staphylococcus warneri* |
| 231 | ARG56_1_1 | *Candida albicans* |
| 232 | ASL43f_1_1 | *Candida albicans* |
| 233 | BGL2_1_1 | *Candida albicans* |
| 234 | CACHS3_1_1 | *Candida albicans* |
| 235 | CCT8_1_1 | *Candida albicans* |
| 236 | CDC37_1_1 | *Candida albicans* |
| 237 | CEF3_1_1 | *Candida albicans* |
| 238 | CHS1_1_1 | *Candida albicans* |
| 239 | CHS2_1_1 | *Candida albicans* |
| 240 | CHS4_1_1 | *Candida albicans* |
| 241 | CHS5_1_1 | *Candida albicans* |
| 242 | CHT1_1_1 | *Candida albicans* |
| 243 | CHT2_1_1 | *Candida albicans* |
| 244 | CHT4_1_1 | *Candida albicans* |
| 245 | CSA1_1_1 1 | *Candida albicans* |
| 246 | 5triphosphatase_1_1 | *Candida albicans* |
| 247 | AAF1_1_1 1 | *Candida albicans* |
| 248 | ADH1_1_1 | *Candida albicans* |
| 249 | ALS1_1_1 | *Candida albicans* |
| 250 | ALS7_1_1 | *Candida albicans* |
| 251 | EDT1_1_1 | *Candida albicans* |
| 252 | ELF_1_1 | *Candida albicans* |
| 253 | ESS1_1_1 | *Candida albicans* |
| 254 | FAL1_1_1 | *Candida albicans* |
| 255 | GAP1_1_1 | *Candida albicans* |
| 256 | GNA1_1_1 | *Candida albicans* |
| 257 | GSC1_1_1 | *Candida albicans* |
| 258 | GSL1_1_1 | *Candida albicans* |
| 259 | HIS1_1_1 | *Candida albicans* |
| 260 | HTS1_1_1 | *Candida albicans* |
| 261 | HWP1_2_1 | *Candida albicans* |
| 262 | HYR1_1_1 | *Candida albicans* |
| 263 | NT1a_1_1 | *Candida albicans* |
| 264 | KRE15f_1_1 | *Candida albicans* |
| 265 | KRE6_1_1 | *Candida albicans* |
| 266 | KRE9_1_1 | *Candida albicans* |
| 267 | MIG1_1_1 | *Candida albicans* |
| 268 | MLS1_1_1 | *Candida albicans* |
| 269 | MP65_1_1 | *Candida albicans* |
| 270 | NDE1_1_1 | *Candida albicans* |
| 271 | PFK2_1_1 | *Candida albicans* |
| 272 | PHR1_1_1 | *Candida albicans* |
| 273 | PHR2_1_1 | *Candida albicans* |
| 274 | PHR3_1_1 | *Candida albicans* |
| 275 | PRA1_1_1 | *Candida albicans* |
| 276 | PRS1_1_1 | *Candida albicans* |
| 277 | RBT1_1_1 | *Candida albicans* |
| 278 | RBT4_1_1 | *Candida albicans* |
| 279 | RHO1_1_1 | *Candida albicans* |
| 280 | RNR1_1_1 | *Candida albicans* |
| 281 | RPB7_1_1 | *Candida albicans* |
| 282 | RPL13_1_1 | *Candida albicans* |
| 283 | RVS167_1_1 | *Candida albicans* |
| 284 | SHA3_1_1 | *Candida albicans* |
| 285 | SKN1_1_1 | *Candida albicans* |
| 286 | SRB1_1_1 | *Candida albicans* |
| 287 | TCA1_1_1 | *Candida albicans* |
| 288 | TRP1_1_1 | *Candida albicans* |
| 289 | YAE1_1_1 | *Candida albicans* |
| 290 | YRB1_1_1 | *Candida albicans* |
| 291 | YST1exon2_1_1 | *Candida albicans* |
| 292 | CCN1_1_1 | *Candida albicans* |
| 293 | CDC28_1_1 | *Candida albicans* |
| 294 | CLN2_1_1 | *Candida albicans* |
| 295 | CPH1_1_1 | *Candida albicans* |
| 296 | CYB1_1_1 | *Candida albicans* |
| 297 | EFG1_1_1 | *Candida albicans* |
| 298 | MNT1_1_1 | *Candida albicans* |
| 299 | RBF1_1_1 | *Candida albicans* |
| 300 | RBF1_2_1 | *Candida albicans* |
| 301 | RIM101_1_1 | *Candida albicans* |
| 302 | RIM8_1_1 | *Candida albicans* |
| 303 | SEC14_1_1 | *Candida albicans* |
| 304 | SEC4_1_1 | *Candida albicans* |
| 305 | TUP1_1_1 | *Candida albicans* |
| 306 | YPT1_1_1 | *Candida albicans* |
| 307 | ZNF1CZF1_2_1 | *Candida albicans* |
| 308 | arcA_1_1 | *Enterococcus faecalis* |
| 309 | arcC_1_1 | *Enterococcus faecalis* |
| 310 | bkdA_1_1 | *Enterococcus faecalis* |
| 311 | cad_1_1 | *Enterococcus faecalis* |
| 312 | camE1_1_1 | *Enterococcus faecalis* |
| 313 | esrA_1_1 | *Enterococcus faecalis* |
| 314 | dacA_1_1 | *Enterococcus faecalis* |
| 315 | dfr_1_1 | *Enterococcus faecalis* |
| 316 | dhoD1a_1_1 | *Enterococcus faecalis* |
| 317 | ABC-eltA_1_1 | *Enterococcus faecalis* |
| 318 | agrBfs_1_1 | *Enterococcus faecalis* |
| 319 | agrCfs_1_1 | *Enterococcus faecalis* |
| 320 | dnaE_1_1 | *Enterococcus faecalis* |
| 321 | ebsA_1_1 | *Enterococcus faecalis* |
| 322 | ebsB_1_1 | *Enterococcus faecalis* |
| 323 | eep_1_1 | *Enterococcus faecalis* |
| 324 | efaR_1_1 | *Enterococcus faecalis* |
| 325 | gls24_glsB_1_1 | *Enterococcus faecalis* |
| 326 | gph_1_1 | *Enterococcus faecalis* |
| 327 | gyrAEf_1_1 | *Enterococcus faecalis* |
| 328 | metEf_1_1 | *Enterococcus faecalis* |
| 329 | mntHCb2_1_1 | *Enterococcus faecalis* |
| 330 | mob2_1_1 | *Enterococcus faecalis* |
| 331 | mvaD_1_1 | *Enterococcus faecalis* |
| 332 | mvaE_1_1 | *Enterococcus faecalis* |
| 333 | parC_1 _1 | *Enterococcus faecalis* |
| 334 | pcfG_1_1 | *Enterococcus faecalis* |
| 335 | phoZ_1_1 | *Enterococcus faecalis* |
| 336 | polC_1_1 | *Enterococcus faecalis* |
| 337 | ptb_1 _1 | *Enterococcus faecalis* |
| 338 | reeS1_1_1 | *Enterococcus faecalis* |
| 339 | rpoN_1_1 | *Enterococcus faecalis* |
| 340 | tms_1_1 | *Enterococcus faecalis* |
| 341 | tyrDC_1_1 | *Enterococcus faecalis* |
| 342 | tyrS_1_1 | *Enterococcus faecalis* |
| 343 | asa1_1_1 | *Enterococcus faecalis* |
| 344 | asp1_1_1 | *Enterococcus faecalis* |
| 345 | cgh_1_1 | *Enterococcus faecalis* |
| 346 | cylA_1_1 | *Enterococcus faecalis* |
| 347 | cylB_1_1 | *Enterococcus faecalis* |
| 348 | cyll_1_1 | *Enterococcus faecalis* |
| 349 | cylL_cylS_1_1 | *Enterococcus faecalis* |
| 350 | cylM_1_1 | *Enterococcus faecalis* |
| 351 | ace_1_1 | *Enterococcus faecalis* |
| 352 | ef00108_1_1 | *Enterococcus faecalis* |
| 353 | ef00109_1_1 | *Enterococcus faecalis* |
| 354 | ef0011_1_1 | *Enterococcus faecalis* |
| 355 | ef00113_1_1 | *Enterococcus faecalis* |
| 356 | ef0012_1_1 | *Enterococcus faecalis* |
| 357 | ef0022_1_1 | *Enterococcus faecalis* |
| 358 | ef0031_1_1 | *Enterococcus faecalis* |
| 359 | ef0032_1_1 | *Enterococcus faecalis* |
| 360 | ef0040_1_1 | *Enterococcus faecalis* |
| 361 | ef0058_1_1 | *Enterococcus faecalis* |
| 362 | enlA_1_1 | *Enterococcus faecalis* |
| 363 | esa_1_1 | *Enterococcus faecalis* |
| 364 | esp_1_1 | *Enterococcus faecalis* |
| 365 | gelE_1_1 | *Enterococcus faecalis* |
| 366 | groEL_1_1 | *Enterococcus faecalis* |
| 367 | groES_1_1 | *Enterococcus faecalis* |
| 368 | rt1_1_1 | *Enterococcus faecalis* |
| 369 | sala_1_1 | *Enterococcus faecalis* |
| 370 | salb_1_1 | *Enterococcus faecalis* |
| 371 | sea1_1_1 | *Enterococcus faecalis* |
| 372 | sep1_1_1 | *Enterococcus faecalis* |
| 373 | vicK_1_1 | *Enterococcus faecalis* |
| 374 | yycH_1_1 | *Enterococcus faecalis* |
| 375 | yycl_1_1 | *Enterococcus faecalis* |
| 376 | yycJ_1_1 | *Enterococcus faecalis* |
| 377 | bglB_1_1 | *Enterococcus faecium* |
| 378 | bglR_1_1 | *Enterococcus faecium* |
| 379 | bglS_1_1 | *Enterococcus faecium* |
| 380 | efmA_1_1 | *Enterococcus faecium* |
| 381 | efmB_1_1 | *Enterococcus faecium* |
| 382 | efmC_1_1 | *Enterococcus faecium* |
| 383 | mreC_1_1 | *Enterococcus faecium* |
| 384 | mreD_1_1 | *Enterococcus faecium* |
| 385 | mvaDEfaecium_1_1 | *Enterococcus faecium* |
| 386 | mvaEEfaecium_1_1 | *Enterococcus faecium* |
| 387 | mvaK1 Efaecium_1_1 | *Enterococcus faecium* |
| 388 | mvaK2Efaecium_1_1 | *Enterococcus faecium* |
| 389 | mvaSEfaecium_1_1 | *Enterococcus faecium* |
| 390 | orf3_4Efaeciumb_1_1 | *Enterococcus faecium* |
| 391 | orf6_7Efaecium_1_1 | *Enterococcus faecium* |
| 392 | orf7_8Efaecium_1_1 | *Enterococcus faecium* |
| 393 | orf9_10Efaecium_1_1 | *Enterococcus faecium* |
| 394 | entA_entl_1_1 | *Enterococcus faecium* |
| 395 | entD_1_1 | *Enterococcus faecium* |
| 396 | entR_1_1 | *Enterococcus faecium* |
| 397 | oep_1_1 | *Enterococcus faecium* |
| 398 | sagA_1 _2 | *Enterococcus faecium* |
| 399 | atsA_1_1 | *Klebsiella pneumoniae* |
| 400 | atsB_1_1 | *Klebsiella pneumoniae* |
| 401 | budC_1_1 | *Klebsiella pneumoniae* |
| 402 | citA_1_1 | *Klebsiella pneumoniae* |
| 403 | citW_1_1 | *Klebsiella pneumoniae* |
| 404 | citX_1_1 | *Klebsiella pneumoniae* |
| 405 | dalD_1_1 | *Klebsiella pneumoniae* |
| 406 | dalK_1_1 | *Klebsiella pneumoniae* |
| 407 | dalT_1_1 | *Klebsiella pneumoniae* |
| 408 | acoA_1 _1 | *Klebsiella pneumoniae* |
| 409 | acoB_1 _1 | *Klebsiella pneumoniae* |
| 410 | acoC_1_1 | *Klebsiella pneumoniae* |
| 411 | ahlK_1_1 | *Klebsiella pneumoniae* |
| 412 | fimK_1_1 | *Klebsiella pneumoniae* |
| 413 | glfKPN2_1_1 | *Klebsiella pneumoniae* |
| 414 | ltrA_1_1 | *Klebsiella pneumoniae* |
| 415 | mdcC_1_1 | *Klebsiella pneumoniae* |
| 416 | mdcF_1_1 | *Klebsiella pneumoniae* |
| 417 | mdcH_1_1 | *Klebsiella pneumoniae* |
| 418 | mrkA_1_1 | *Klebsiella pneumoniae* |
| 419 | mtrK_1_1 | *Klebsiella pneumoniae* |
| 420 | nifF_1_1 | *Klebsiella pneumoniae* |
| 421 | nifK_1_1 | *Klebsiella pneumoniae* |
| 422 | nifN_1_1 | *Klebsiella pneumoniae* |
| 423 | tyrP_1_1 | *Klebsiella pneumoniae* |
| 424 | ureA_1_1 | *Klebsiella pneumoniae* |
| 425 | wbbO_1_1 | *Klebsiella pneumoniae* |
| 426 | wza_1_1 | *Klebsiella pneumoniae* |
| 427 | wzb_1_1 | *Klebsiella pneumoniae* |
| 428 | wzm KPN2_1 _1 | *Klebsiella pneumoniae* |
| 429 | wztKPN2_1_1 | *Klebsiella pneumoniae* |
| 430 | yojH_1_1 | *Klebsiella pneumoniae* |
| 431 | liac_1_1 | *Klebsiella pneumoniae* |
| 432 | cim_1_1 | *Klebsiella pneumoniae* |
| 433 | aldA_1_1 | *Klebsiella pneumoniae* |
| 434 | aldA_2_1 | *Klebsiella pneumoniae* |
| 435 | hemly_1_1 | *Klebsiella pneumoniae* |
| 436 | pSL017_1_1 | *Klebsiella pneumoniae* |
| 437 | pSL020_1_1 | *Klebsiella pneumoniae* |
| 438 | rcsA_1_1 | *Klebsiella pneumoniae* |
| 439 | rmlC_1_1 | *Klebsiella pneumoniae* |
| 440 | rmID_1_1 | *Klebsiella pneumoniae* |
| 441 | waaG_1_1 | *Klebsiella pneumoniae* |
| 442 | wbbD_1_1 | *Klebsiella pneumoniae* |
| 443 | wbbM_1_1 | *Klebsiella pneumoniae* |
| 444 | wbbN_1_1 | *Klebsiella pneumoniae* |
| 445 | wbdA_1 _1 | *Klebsiella pneumoniae* |
| 446 | wbdC_1 _1 | *Klebsiella pneumoniae* |
| 447 | wztKpn_1_1 | *Klebsiella pneumoniae* |
| 448 | yibD_1_1 | *Klebsiella pneumoniae* |
| 449 | cymA_1_1 | *Klebsiella oxytoca* |
| 450 | cymD_1_1 | *Klebsiella oxytoca* |
| 451 | cymE_1_1 | *Klebsiella oxytoca* |
| 452 | cymH_1_1 | *Klebsiella oxytoca* |
| 453 | cyml_1_1 | *Klebsiella oxytoca* |
| 454 | cymd_1_1 | *Klebsiella oxytoca* |
| 455 | ddrA_1_1 | *Klebsiella oxytoca* |
| 456 | fdt-1_1_1 | *Klebsiella oxytoca* |
| 457 | fdt-2_1_1 | *Klebsiella oxytoca* |
| 458 | fdt-3_1_1 | *Klebsiella oxytoca* |
| 459 | gatY_1_1 | *Klebsiella oxytoca* |
| 460 | hydH_1_1 | *Klebsiella oxytoca* |
| 461 | masA_1_1 | *Klebsiella oxytoca* |
| 462 | nasA_1_1 | *Klebsiella oxytoca* |
| 463 | nasE_1_1 | *Klebsiella oxytoca* |
| 464 | nasF_1_1 | *Klebsiella oxytoca* |
| 465 | pehX_1_1 | *Klebsiella oxytoca* |
| 466 | pelX_1_1 | *Klebsiella oxytoca* |
| 467 | tagH_1_1 | *Klebsiella oxytoca* |
| 468 | tagK_1_1 | *Klebsiella oxytoca* |
| 469 | tagT_1 _1 | *Klebsiella oxytoca* |
| 470 | glpR_1_1 | *Pseudomonas aeruginosa* |
| 471 | lasRb_1_1 | *Pseudomonas aeruginosa* |
| 472 | OrfX_1 _1 | *Pseudomonas aeruginosa* |
| 473 | pa0260_1_1 | *Pseudomonas aeruginosa* |
| 474 | pa0572_1_1 | *Pseudomonas aeruginosa* |
| 475 | pa0625_1_1 | *Pseudomonas aeruginosa* |
| 476 | pa0636_1_1 | *Pseudomonas aeruginosa* |
| 477 | pa1046_1_1 | *Pseudomonas aeruginosa* |
| 478 | pa1069_1_1 | *Pseudomonas aeruginosa* |
| 479 | pa1846_1_1 | *Pseudomonas aeruginosa* |
| 480 | pa3866_1_1 | *Pseudomonas aeruginosa* |
| 481 | pa4082_1_1 | *Pseudomonas aeruginosa* |
| 482 | pilAp_1_1 | *Pseudomonas aeruginosa* |
| 483 | PilAp2_1_1 | *Pseudomonas aeruginosa* |
| 484 | pilC_1_1 | *Pseudomonas aeruginosa* |
| 485 | PstP_1_1 | *Pseudomonas aeruginosa* |
| 486 | purK_1_1 | *Pseudomonas aeruginosa* |
| 487 | uvrDII_1_1 | *Pseudomonas aeruginosa* |
| 488 | vsml_1_1 | *Pseudomonas aeruginosa* |
| 489 | vsm R_1 _2 | *Pseudomonas aeruginosa* |
| 490 | xcpX_1_1 | *Pseudomonas aeruginosa* |
| 491 | aprA_1_1 | *Pseudomonas aeruginosa* |
| 492 | aprE_1_1 | *Pseudomonas aeruginosa* |
| 493 | ctx_1_2 | *Pseudomonas aeruginosa* |
| 494 | algB_1_1 | *Pseudomonas aeruginosa* |
| 495 | algN_1_1 | *Pseudomonas aeruginosa* |
| 496 | algR_1_1 | *Pseudomonas aeruginosa* |
| 497 | ExoS_1_1 | *Pseudomonas aeruginosa* |
| 498 | fpvA_1_1 | *Pseudomonas aeruginosa* |
| 499 | lasRa_1_1 | *Pseudomonas aeruginosa* |
| 500 | lipA_1_1 | *Pseudomonas aeruginosa* |
| 501 | lipH_1_1 | *Pseudomonas aeruginosa* |
| 502 | Orf159_1_2 | *Pseudomonas aeruginosa* |
| 503 | Orf252_1_1 | *Pseudomonas aeruginosa* |
| 504 | pchG_1_1 | *Pseudomonas aeruginosa* |
| 505 | PhzA_1_1 | *Pseudomonas aeruginosa* |
| 506 | PhzB_1_1 | *Pseudomonas aeruginosa* |
| 507 | PLC_1_1 | *Pseudomonas aeruginosa* |
| 508 | plcN_1_1 | *Pseudomonas aeruginosa* |
| 509 | plcR_1_1 | *Pseudomonas aeruginosa* |
| 510 | pvdD_1_1 | *Pseudomonas aeruginosa* |
| 511 | pvdF_1_2 | *Pseudomonas aeruginosa* |
| 512 | pyocinS1_1_1 | *Pseudomonas aeruginosa* |
| 513 | pyocinS1im_1_1 | *Pseudomonas aeruginosa* |
| 514 | pyocinS2_1_1 | *Pseudomonas aeruginosa* |
| 515 | pys2_1_1 | *Pseudomonas aeruginosa* |
| 516 | pys2_2_1 | *Pseudomonas aeruginosa* |
| 517 | rbf303_1_1 | *Pseudomonas aeruginosa* |
| 518 | rhlA_1_1 | *Pseudomonas aeruginosa* |
| 519 | rhlB_1_1 | *Pseudomonas aeruginosa* |
| 520 | rhlR_1_1 | *Pseudomonas aeruginosa* |
| 521 | TnAP41_1_2 | *Pseudomonas aeruginosa* |
| 522 | toxA_1_1 | *Pseudomonas aeruginosa* |
| 523 | cap1EStrpneu_1_1 | *Streptococcus pneumoniae* |
| 524 | cap1FStrpneu_1_1 | *Streptococcus pneumoniae* |
| 525 | cap1GStrpneu_1_1 | *Streptococcus pneumoniae* |
| 526 | cap3AStrpneu_1_1 | *Streptococcus pneumoniae* |
| 527 | cap3BStrpneu_1_1 | *Streptococcus pneumoniae* |
| 528 | ceIAStrpneu_1_1 | *Streptococcus pneumoniae* |
| 529 | ceIBStrpneu_1_1 | *Streptococcus pneumoniae* |
| 530 | cgIAStrpneu_1_1 | *Streptococcus pneumoniae* |
| 531 | cgIBStrpneu_1_1 | *Streptococcus pneumoniae* |
| 532 | cgICStrpneu_1_1 | *Streptococcus pneumoniae* |
| 533 | cgIDStrpneu_1_1 | *Streptococcus pneumoniae* |
| 534 | cinA_1_1 | *Streptococcus pneumoniae* |
| 535 | cps14EStrpneum_1_1 | *Streptococcus pneumoniae* |
| 536 | cps14FStrpneum_1_1 | *Streptococcus pneumoniae* |
| 537 | cps14GStrpneum_1_1 | *Streptococcus pneumoniae* |
| 538 | cps14HStrpneum_1_1 | *Streptococcus pneumoniae* |
| 539 | cps19aHStrpneum_1_1 | *Streptococcus pneumoniae* |
| 540 | cps19alStrpneum_1_1 | *Streptococcus pneumoniae* |
| 541 | cps19aKStrpneum_1_1 | *Streptococcus pneumoniae* |
| 542 | cps19fGStrpneum_1_1 | *Streptococcus pneumoniae* |
| 543 | cps23fGStrpneum_1_1 | *Streptococcus pneumoniae* |
| 544 | dexB_1_1 | *Streptococcus pneumoniae* |
| 545 | dinF_1_1 | *Streptococcus pneumoniae* |
| 546 | 1760Strpneu_1_1 | *Streptococcus pneumoniae* |
| 547 | acyPStrpneu_1_1 | *Streptococcus pneumoniae* |
| 548 | endAStrpneu_1 _1 | *Streptococcus pneumoniae* |
| 549 | exoAStrpneu_1_1 | *Streptococcus pneumoniae* |
| 550 | exp72_1_1 | *Streptococcus pneumoniae* |
| 551 | fnlAStrpneu_1_1 | *Streptococcus pneumoniae* |
| 552 | fnlBStrpneu_1_1 | *Streptococcus pneumoniae* |
| 553 | fnlCStrpneu_1_1 | *Streptococcus pneumoniae* |
| 554 | gct18Strpneum_1_1 | *Streptococcus pneumoniae* |
| 555 | hexB1_1_1 | *Streptococcus pneumoniae* |
| 556 | hftsHstrpneu_1_1 | *Streptococcus pneumoniae* |
| 557 | immunofrag 1 Strpneu_1_1 | *Streptococcus pneumoniae* |
| 558 | immunofrag2Strpneu_2_1 | *Streptococcus pneumoniae* |
| 559 | immunofrag3Strpneu_2_1 | *Streptococcus pneumoniae* |
| 560 | kdtBStrpneu_1_1 | *Streptococcus pneumoniae* |
| 561 | lysAStrpneu_1_1 | *Streptococcus pneumoniae* |
| 562 | pcpBStrpneu_1_1 | *Streptococcus pneumoniae* |
| 563 | pfICStrpneu_1_1 | *Streptococcus pneumoniae* |
| 564 | plpA_1_1 | *Streptococcus pneumoniae* |
| 565 | prtAlStrpneu_1_1 | *Streptococcus pneumoniae* |
| 566 | pspC1Strpneu_1 _1 | *Streptococcus pneumoniae* |
| 567 | pspC2_1_1 | *Streptococcus pneumoniae* |
| 568 | purRStrpneu_1_1 | *Streptococcus pneumoniae* |
| 569 | pyrDAStrpneum_1_1 | *Streptococcus pneumoniae* |
| 570 | SP0828Strpneu_1_1 | *Streptococcus pneumoniae* |
| 571 | SP0830Strpneu_1_1 | *Streptococcus pneumoniae* |
| 572 | SP0833Strpneu_1_1 | *Streptococcus pneumoniae* |
| 573 | SP0837_38Strpneu_1_1 | *Streptococcus pneumoniae* |
| 574 | SP0839Strpneu_1_1 | *Streptococcus pneumoniae* |
| 575 | ugdStrpneu_1_1 | *Streptococcus pneumoniae* |
| 576 | uncC_1_1 | *Streptococcus pneumoniae* |
| 577 | vicXStrepneu_1_1 | *Streptococcus pneumoniae* |
| 578 | wchA6bStrpneum_1_1 | *Streptococcus pneumoniae* |
| 579 | wci4Strpneum_1_1 | *Streptococcus pneumoniae* |
| 580 | wciK4Strpneum_1_1 | *Streptococcus pneumoniae* |
| 581 | wciL4Strpneum_1_1 | *Streptococcus pneumoniae* |
| 582 | wciN6bStrpneum_1_1 | *Streptococcus pneumoniae* |
| 583 | wciO6bStrpneum_1_1 | *Streptococcus pneumoniae* |
| 584 | wciP6bStrpneum_1_1 | *Streptococcus pneumoniae* |
| 585 | wciY18Strpneum_1_1 | *Streptococcus pneumoniae* |
| 586 | wzdbStrpneum_1_1 | *Streptococcus pneumoniae* |
| 587 | wze6bStrpneum_1_1 | *Streptococcus pneumoniae* |
| 588 | wzy18Strpneum_1_1 | *Streptococcus pneumoniae* |
| 589 | wzy4Strpneum_1_1 | *Streptococcus pneumoniae* |
| 590 | wzy6bStrpneum_1_1 | *Streptococcus pneumoniae* |
| 591 | xpt_1_1 | *Streptococcus pneumoniae* |
| 592 | igaStrpneu_1_1 | *Streptococcus pneumoniae* |
| 593 | IytA_1_1 | *Streptococcus pneumoniae* |
| 594 | nanA_1 _1 | *Streptococcus pneumoniae* |
| 595 | nanBStrpneu_1_1 | *Streptococcus pneumoniae* |
| 596 | pcpCStrpneu_1_1 | *Streptococcus pneumoniae* |
| 597 | ply_1_1 | *Streptococcus pneumoniae* |
| 598 | prtAStrpneu_1_1 | *Streptococcus pneumoniae* |
| 599 | pspA_1 _2 | *Streptococcus pneumoniae* |
| 600 | SP0834Strpneu_1_1 | *Streptococcus pneumoniae* |
| 601 | SP0834Strpneu_1_2 | *Streptococcus pneumoniae* |
| 602 | sphtraStrpneu_1_1 | *Streptococcus pneumoniae* |
| 603 | wciJStrpneu_1_1 | *Streptococcus pneumoniae* |
| 604 | wziyStrpneu_1_1 | *Streptococcus pneumoniae* |
| 605 | wzxStrpneu_1_1 | *Streptococcus pneumoniae* |
| 606 | cpsA1Strgal_1_1 | *Streptococcus agalactiae* |
| 607 | cpsB1 Strgal_1_1 | *Streptococcus agalactiae* |
| 608 | cpsC1Strgal_1_1 | *Streptococcus agalactiae* |
| 609 | cpsD1Strgal_1_1 | *Streptococcus agalactiae* |
| 610 | cpsE1Strgal_1_1 | *Streptococcus agalactiae* |
| 611 | cpsG1Strgal_1_1 | *Streptococcus agalactiae* |
| 612 | cpsIStragal_1_1 | *Streptococcus agalactiae* |
| 613 | cpsJStragal_1_1 | *Streptococcus agalactiae* |
| 614 | cpsKStragal_1_1 | *Streptococcus agalactiae* |
| 615 | cpsMStragal_1_1 | *Streptococcus agalactiae* |
| 616 | cpsYStragal_1_1 | *Streptococcus agalactiae* |
| 617 | cpsYStragal_2_1 | *Streptococcus agalactiae* |
| 618 | cylBStraga_1_1 | *Streptococcus agalactiae* |
| 619 | cylEStraga_1_1 | *Streptococcus agalactiae* |
| 620 | cylFStraga_1_1 | *Streptococcus agalactiae* |
| 621 | cylHStraga_1_1 | *Streptococcus agalactiae* |
| 622 | cylIStraga_1_1 | *Streptococcus agalactiae* |
| 623 | cylJStraga_1_1 | *Streptococcus agalactiae* |
| 624 | cylKStraga_1_1 | *Streptococcus agalactiae* |
| 625 | 0487Straga_1_1 | *Streptococcus agalactiae* |
| 626 | 0488Straga_1_1 | *Streptococcus agalactiae* |
| 627 | 0493Straga_1_1 | *Streptococcus agalactiae* |
| 628 | 0495Straga_1_1 | *Streptococcus agalactiae* |
| 629 | 0498Straga_1_1 | *Streptococcus agalactiae* |
| 630 | 0500Straga_1_1 | *Streptococcus agalactiae* |
| 631 | 0502Straga_1_1 | *Streptococcus agalactiae* |
| 632 | 0504Straga_1_1 | *Streptococcus agalactiae* |
| 633 | folDStraga_1_1 | *Streptococcus agalactiae* |
| 634 | neuA1Strgal_1_1 | *Streptococcus agalactiae* |
| 635 | neuB1Strgal_1_1 | *Streptococcus agalactiae* |
| 636 | neuC1Strgal_1_1 | *Streptococcus agalactiae* |
| 637 | neuD1Strgal_1_1 | *Streptococcus agalactiae* |
| 638 | recNStraga_1_1 | *Streptococcus agalactiae* |
| 639 | ileSStraga_1_1 | *Streptococcus agalactiae* |
| 640 | CAMPfactor_1_1 | *Streptococcus agalactiae* |
| 641 | CAMPfactor_2_1 | *Streptococcus agalactiae* |
| 642 | 0499Straga_1_1 | *Streptococcus agalactiae* |
| 643 | hylStragal_1_1 | *Streptococcus agalactiae* |
| 644 | IipStragal_1_1 | *Streptococcus agalactiae* |
| 645 | cyclStrpyog_1_1 | *Streptococcus pyogenes* |
| 646 | fah_rph_hlo_Strpyog_1_1 | *Streptococcus pyogenes* |
| 647 | int_1_1 | *Streptococcus pyogenes* |
| 648 | int315.5_1_1 | *Streptococcus pyogenes* |
| 649 | murEStrpyog_1_1 | *Streptococcus pyogenes* |
| 650 | oppA_1_1 | *Streptococcus pyogenes* |
| 651 | oppCStrpyog_1_1 | *Streptococcus pyogenes* |
| 652 | oppD_1_1 | *Streptococcus pyogenes* |
| 653 | SPy0382Strpyog_1_1 | *Streptococcus pyogenes* |
| 654 | SPy0390Strpyog_1_1 | *Streptococcus pyogenes* |
| 655 | SpyM3_1351_1_1 | *Streptococcus pyogenes* |
| 656 | vicXStrpyog_1_1 | *Streptococcus pyogenes* |
| 657 | DNaseIStrpyog_1_1 | *Streptococcus pyogenes* |
| 658 | fba2Strpyog_1_1 | *Streptococcus pyogenes* |
| 659 | fhuAStrpyog_1_1 | *Streptococcus pyogenes* |
| 660 | fhuBiStrpyog_1_1 | *Streptococcus pyogenes* |
| 661 | fhuDStrpyog_1_1 | *Streptococcus pyogenes* |
| 662 | fhuGStrpyog_1_1 | *Streptococcus pyogenes* |
| 663 | hylA_1_1 | *Streptococcus pyogenes* |
| 664 | hylP_1_1 | *Streptococcus pyogenes* |
| 665 | hylp2_1_1 | *Streptococcus pyogenes* |
| 666 | oppB_1_1 | *Streptococcus pyogenes* |
| 667 | ropB_1 _1 | *Streptococcus pyogenes* |
| 668 | scpAStrpyog_1_1 | *Streptococcus pyogenes* |
| 669 | sloStrpyog_1_1 | *Streptococcus pyogenes* |
| 670 | smez-4Strpyog_1_1 | *Streptococcus pyogenes* |
| 671 | sof_1_1 | *Streptococcus pyogenes* |
| 672 | sof_2_1 | *Streptococcus pyogenes* |
| 673 | speA_1_1 | *Streptococcus pyogenes* |
| 674 | speB2Strpyog_1_1 | *Streptococcus pyogenes* |
| 675 | speCStrpyog_1 _1 | *Streptococcus pyogenes* |
| 676 | speJStrpyog_1_1 | *Streptococcus pyogenes* |
| 677 | srtBStrpyog_1_1 | *Streptococcus pyogenes* |
| 678 | srtCStrpyog_1_1 | *Streptococcus pyogenes* |
| 679 | srtEStrpyog_1_1 | *Streptococcus pyogenes* |
| 680 | srtFStrpyog_1_1 | *Streptococcus pyogenes* |
| 681 | srtGStrpyog_1_1 | *Streptococcus pyogenes* |
| 682 | srtlStrpyog_1_1 | *Streptococcus pyogenes* |
| 683 | srtKStrpyog_1_1 | *Streptococcus pyogenes* |
| 684 | srtRStrpyog_1_1 | *Streptococcus pyogenes* |
| 685 | srtTStrpyog_1_1 | *Streptococcus pyogenes* |
| 686 | vicKStrpyog_1_1 | *Streptococcus pyogenes* |
| 687 | 573Stprmut_1_1 | *Streptococcus viridans* |
| 688 | 580SStprm ut_1 _1 | *Streptococcus viridans* |
| 689 | 581_582SStprmut_1_1 | *Streptococcus viridans* |
| 690 | 584SStprmut_1_1 | *Streptococcus viridans* |
| 691 | dltAStrmut_1_1 | *Streptococcus viridans* |
| 692 | dltBStrmut_1_1 | *Streptococcus viridans* |
| 693 | dltCppx1Strmut_1_1 | *Streptococcus viridans* |
| 694 | dltDStrmut_1_1 | *Streptococcus viridans* |
| 695 | IichStrbov_1_1 | *Streptococcus viridans* |
| 696 | lytRStprmut_1_1 | *Streptococcus viridans* |
| 697 | lytSStprmut_1_1 | *Streptococcus viridans* |
| 698 | pepQStrrmut_1_1 | *Streptococcus viridans* |
| 699 | pflCStrmut_1_1 | *Streptococcus viridans* |
| 700 | recNStprmut_1_1 | *Streptococcus viridans* |
| 701 | ytqBStrmut_1_1 | *Streptococcus viridans* |
| 702 | hlyXStrmut_1_1 | *Streptococcus viridans* |
| 703 | igaStrmitis_1_1 | *Streptococcus viridans* |
| 704 | igaStrsanguis_1_1 | *Streptococcus viridans* |
| 705 | perMStrmut_1_1 | *Streptococcus viridans* |
| 706 | atfA_1_1 | *Proteus mirabilis* |
| 707 | atfB_1_1 | *Proteus mirabilis* |
| 708 | atfC_1_1 | *Proteus mirabilis* |
| 709 | ccmPrmi1_1_1 | *Proteus mirabilis* |
| 710 | cyaPrmi_1_1 | *Proteus mirabilis* |
| 711 | aad_1_1 | *Proteus mirabilis* |
| 712 | flfB_1_1 | *Proteus mirabilis* |
| 713 | flfD_1_1 | *Proteus mirabilis* |
| 714 | flfN_1_1 | *Proteus mirabilis* |
| 715 | flhD_1_1 | *Proteus mirabilis* |
| 716 | floA_1_1 | *Proteus mirabilis* |
| 717 | ftsK_1_1 | *Proteus mirabilis* |
| 718 | gstB_1_1 | *Proteus mirabilis* |
| 719 | hem CPrmi_1_1 | *Proteus mirabilis* |
| 720 | hem DPrmi_1_1 | *Proteus mirabilis* |
| 721 | hev_1_1 | *Proteus mirabilis* |
| 722 | katA_1_1 | *Proteus mirabilis* |
| 723 | lpp1_1_1 | *Proteus mirabilis* |
| 724 | menE_1_1 | *Proteus mirabilis* |
| 725 | mfd_1_1 | *Proteus mirabilis* |
| 726 | nrpA_1_1 | *Proteus mirabilis* |
| 727 | nrpB_1_1 | *Proteus mirabilis* |
| 728 | nrpG_1_1 | *Proteus mirabilis* |
| 729 | nrpS_1_1 | *Proteus mirabilis* |
| 730 | nrpT_1_1 | *Proteus mirabilis* |
| 731 | nrpU_1_1 | *Proteus mirabilis* |
| 732 | pat_1_1 | *Proteus mirabilis* |
| 733 | pmfA_1_1 | *Proteus mirabilis* |
| 734 | pmfC_1_1 | *Proteus mirabilis* |
| 735 | pmfE_1_1 | *Proteus mirabilis* |
| 736 | ppaA_1_1 | *Proteus mirabilis* |
| 737 | rsbA_1_1 | *Proteus mirabilis* |
| 738 | rsbC_1_1 | *Proteus mirabilis* |
| 739 | speB_1_1 | *Proteus mirabilis* |
| 740 | stmA_1_1 | *Proteus mirabilis* |
| 741 | stmB_1_1 | *Proteus mirabilis* |
| 742 | terA_1_1 | *Proteus mirabilis* |
| 743 | terD_1_1 | *Proteus mirabilis* |
| 744 | umoA_1_1 | *Proteus mirabilis* |
| 745 | umoB_1_1 | *Proteus mirabilis* |
| 746 | umoC_1_1 | *Proteus mirabilis* |
| 747 | ureR_1_1 | *Proteus mirabilis* |
| 748 | xerC_1_1 | *Proteus mirabilis* |
| 749 | ygbA_1_1 | *Proteus mirabilis* |
| 750 | flaA_1_1 | *Proteus mirabilis* |
| 751 | flaD_1_1 | *Proteus mirabilis* |
| 752 | fliA_1_1 | *Proteus mirabilis* |
| 753 | hpmA_1_1 | *Proteus mirabilis* |
| 754 | hpmB_1_1 | *Proteus mirabilis* |
| 755 | lpsPrmi_1_1 | *Proteus mirabilis* |
| 756 | mrpA_1_1 | *Proteus mirabilis* |
| 757 | mrpB_1_1 | *Proteus mirabilis* |
| 758 | mrpC_1_1 | *Proteus mirabilis* |
| 759 | mrpD_1_1 | *Proteus mirabilis* |
| 760 | mrpE_1_1 | *Proteus mirabilis* |
| 761 | mrpF_1_1 | *Proteus mirabilis* |
| 762 | mrpG_1_1 | *Proteus mirabilis* |
| 763 | mrpH_1_1 | *Proteus mirabilis* |
| 764 | mrpl_1_1 | *Proteus mirabilis* |
| 765 | mrpJ_1_1 | *Proteus mirabilis* |
| 766 | patA_1_1 | *Proteus mirabilis* |
| 767 | putA_1_1 | *Proteus mirabilis* |
| 768 | uca_1_1 | *Proteus mirabilis* |
| 769 | ureDPrmi_1_1 | *Proteus mirabilis* |
| 770 | ureEPrmi_1_1 | *Proteus mirabilis* |
| 771 | ureFPrmi_1_1 | *Proteus mirabilis* |
| 772 | zapA_1_1 | *Proteus mirabilis* |
| 773 | zapB_1_1 | *Proteus mirabilis* |
| 774 | zapD_1_1 | *Proteus mirabilis* |
| 775 | zapE_1_1 | *Proteus mirabilis* |
| 776 | envZPrvu_1_1 | *Proteus vulgaris* |
| 777 | frdC_1_1 | *Proteus vulgaris* |
| 778 | frdD_1_1 | *Proteus vulgaris* |
| 779 | infBPrvu_1 _1 | *Proteus vulgaris* |
| 780 | lad_1_1 | *Proteus vulgaris* |
| 781 | tna2_1_1 | *Proteus vulgaris* |
| 782 | end_1 _1 | *Proteus vulgaris* |
| 783 | pqrA_1_1 | *Proteus vulgaris* |
| 784 | urg_1_1 | *Proteus vulgaris* |
| 785 | blaIMP-7_1_1 | *Pseudomonas aeruginosa* |
| 786 | meclSepid_1_1 | *Staphylococcus epidermidis* |
| 787 | blaOXA-10_1_2 | *Pseudomonas aeruginosa* |
| 788 | blaB_1_1 | *Proteus vulgaris* |
| 789 | ampC_1_1 | *Klebsiella oxytoca* |
| 790 | I-blaR_1_1 | *Staphylococcus aureus* |
| 791 | blaOXA-32_1_1 | *Pseudomonas aeruginosa* |
| 792 | bla- CTX-M-22_1_1 | *Klebsiella pneumoniae* |
| 793 | pbp2aStrpneu_1_1 | *Streptococcus pneumoniae* |
| 794 | blaSHV-1_1_1 | *Klebsiella pneumoniae* |
| 795 | blaOXA-2_1_1 | *Salmonella typhimurium* |
| 796 | blaRShaemolyt_1_1 | *Staphylococcus haemolyticus* |
| 797 | blalMP-7_1_2 | *Pseudomonas aeruginosa* |
| 798 | I-mecR_1_1 | *Staphylococcus aureus* |
| 799 | blaOXY_1_1 | *Klebsiella oxytoca* |
| 800 | dacCStrpyog_1_1 | *Streptococcus pyogenes* |
| 801 | femA_1_1 | *Staphylococcus aureus* |
| 802 | mecA_1_1 | *Staphylococcus aureus* |
| 803 | blalShaemolyt_1_1 | *Staphylococcus haemolyticus* |
| 804 | blavim_1_1 | *Pseudomonas aeruginosa* |
| 805 | pbp2b_1 _1 | *Streptococcus pneumoniae* |
| 806 | pbp2primeSepid_1_1 | *Staphylococcus epidermidis* |
| 807 | pbp2x_1_1 | *Streptococcus pneumoniae* |
| 808 | pbp3Saureuc_1_1 | *Staphylococcus aureus* |
| 809 | pbp4_1_1 | *Enterococcus faecalis* |
| 810 | pbp5Efaecium_1_1 | *Enterococcus faecium* |
| 811 | pbpC_1_1 | *Enterococcus faecalis* |
| 812 | I-mecl_1_1 | *Staphylococcus aureus* |
| 813 | pbp1a_1_1 | *Streptococcus pneumoniae* |
| 814 | I-blal_1_1 | *Staphylococcus aureus* |
| 815 | blaTEM-106_1_1 | *Escherichia coli* |
| 816 | blaOXY-KLOX_1_1 | *Klebsiella oxytoca* |
| 817 | ftsWEF_1_1 | *Enterococcus faecium* |
| 818 | fmhB_1_1 | *Staphylococcus aureus* |
| 819 | cumA_1_1 | *Proteus vulgaris* |
| 820 | femBShaemolyt_1_1 | *Staphylococcus haemolyticus* |
| 821 | blaPER-1_1_1 | *Pseudomonas aeruginosa* |
| 822 | bla_FOX-3_1_1 | *Klebsiella oxytoca* |
| 823 | blaA_1_1 | *Proteus vulgaris* |
| 824 | psrb_1_1 | *Enterococcus faecium* |
| 825 | fmhA_1_1 | *Staphylococcus aureus* |
| 826 | mecR1Sepid_1_1 | *Staphylococcus epidermidis* |
| 827 | blaZ_1 _1 | *Staphylococcus aureus* |
| 828 | blaOXA-1_1_1 | *Plasmid FGN238* |
| 829 | fox-6_1_1 | *Klebsiella pneumoniae* |
| 830 | b!aPrmi_1_1 | *Proteus mirabilis* |
| 831 | aacA_aphDStwar_1_1 | *Staphylococcus warneri* |
| 832 | aacC1_1_2 | *Pseudomonas aeruginosa* |
| 833 | aacC2_1_1 | *Escherichia coli* |
| 834 | strB_1_1 | *Escherichia coli* |
| 835 | aadA_1_1 | *Enterococcus faecalis* |
| 836 | aadB_1_2 | *Escherichia coli* |
| 837 | aadD_1_1 | *Staphylococcus aureus* |
| 838 | aacA4_1_2 | *Pseudomonas aeruginosa* |
| 839 | strA_1_1 | *Escherichia coli* |
| 840 | aph-A3_1_1 | *Staphylococcus aureus* |
| 841 | aacC1_1_1 | *Pseudomonas aeruginosa* |
| 842 | aacA4_1_1 | *Pseudomonas aeruginosa* |
| 843 | aacA-aphD_1_1 | *Staphylococcus aureus* |
| 844 | I-spc_1_1 | *Staphylococcus aureus* |
| 845 | aphA3_1_1 | *synthetic construct* |
| 846 | ermC_1_1 | *Staphylococcus aureus* |
| 847 | linB_1_1 | *Enterococcus faecium* |
| 848 | satSA_1_1 | *Staphylococcus aureus* |
| 849 | mdrSA_1_1 | *Staphylococcus aureus* |
| 850 | I-linA_1_1 | *Staphylococcus aureus* |
| 851 | erm B_1 _2 | *Staphylococcus aureus* |
| 852 | ermA_1_1 | *Staphylococcus aureus* |
| 853 | satA_1_1 | *Enterococcus faecium* |
| 854 | msrA_1_1 | *Staphylococcus aureus* |
| 855 | mphBM_1_1 | *Staphylococcus aureus* |
| 856 | mefA_1_1 | *Streptococcus pyogenes* |
| 857 | mrx_1_1 | *Escherichia coli* |
| 858 | dfrStrpneu_1 _1 | *Streptococcus pneumoniae* |
| 859 | dfrA_1_1 | *Staphylococcus aureus* |
| 860 | cm lA5_1_1 | *Escherichia coli* |
| 861 | catEfaecium_1_1 | *Enterococcus faecium* |
| 862 | cat_1 _1 | *Staphylococcus aureus* |
| 863 | tetAJ_1_1 | *Proteus mirabilis* |
| 864 | tetL_1_1 | *Enterococcus faecalis* |
| 865 | tetM_1_1 | *Enterococcus faecalis* |
| 866 | vanH(tn)_1_1 | *Enterococcus faecium* |
| 867 | vanA_1_1 | *Enterococcus faecium* |
| 868 | vanHB2_1_1 | *Enterococcus faecium* |
| 869 | vanR_1_1 | *Enterococcus faecium* |
| 870 | vanRB2_1_1 | *Enterococcus faecium* |
| 871 | vanS(tn)_1_1 | *Enterococcus faecium* |
| 872 | vanSB2_1_1 | *Enterococcus faecium* |
| 873 | vanWB2_1_1 | *Enterococcus faecium* |
| 874 | ddl_1_1 | *Enterococcus faecalis* |
| 875 | ble_1_1 | *Staphylococcus aureus* |
| 876 | vanXB2_1_1 | *Enterococcus faecium* |
| 877 | vanY(tn)_1_1 | *Enterococcus faecium* |
| 878 | vanYB2_1_1 | *Enterococcus faecium* |
| 879 | vanB_1_1 | *Enterococcus faecalis* |
| 880 | vanZ(tn)_1_1 | *Enterococcus faecium* |
| 881 | vanC-2_1_1 | *Enterococcus flavescens* |
| 882 | vanX(tn)_1_1 | *Enterococcus faecium* |
| 883 | acrB_1_1 | *Proteus mirabilis* |
| 884 | mexB_1_2 | *Pseudomonas aeruginosa* |
| 885 | I-qacA_1_1 | *Staphylococcus aureus* |
| 886 | sull_1_1 | *Escherichia coli* |
| 887 | sul_1_1 | *Escherichia coli* |
| 888 | cadBStalugd_1_1 | *Staphylococcus lugdunensis* |
| 889 | mexA_1_1 | *Pseudomonas aeruginosa* |
| 890 | acrR_1_1 | *Proteus mirabilis* |
| 891 | emeA_1_1 | *Enterococcus faecalis* |
| 892 | acrA_1_1 | *Proteus mirabilis* |
| 893 | rtn_1_1 | *Proteus vulgaris* |
| 894 | abcXStrpmut_1_1 | *Streptococcus mutans* |
| 895 | qacEdelta1_1 _1 | *Escherichia coli* |
| 896 | elkT-abcA_1_1 | *Staphylococcus aureus* |
| 897 | l-cadA_1_1 | *Staphylococcus aureus* |
| 898 | albA_1_1 | *Klebsiella oxytoca* |
| 899 | wzm_1_1 | *Klebsiella pneumoniae* |
| 900 | msrCb_1_1 | *Enterococcus faecium* |
| 901 | nov_1_1 | *Escherichia coli* |
| 902 | wzt_1_1 | *Klebsiella pneumoniae* |
| 903 | wbbl_1_1 | *Klebsiella pneumoniae* |
| 904 | norA23_1_1 | *Staphylococcus aureus* |
| 905 | mexR_1_1 | *Pseudomonas aeruginosa* |
| 906 | arr2_1_1 | *Escherichia coli* |
| 907 | mreA_1_1 | *Staphylococcus aureus* |
| 908 | I-cadC_1_1 | *Staphylococcus aureus* |
| 909 | uvrA_1_1 | *Enterococcus faecalis* |
| 910 | CRD2_1_1 | *Candida albicans* |
| 911 | CDR1_1_1 | *Candida albicans* |
| 912 | CDR1_2_1 | *Candida albicans* |
| 913 | MET3_1_1 | *Candida albicans* |
| 914 | FET3_1 _1 | *Candida albicans* |
| 915 | FTR2_1_1 | *Candida albicans* |
| 916 | MDR1-7_1_1 | *Candida albicans* |
| 917 | ERG11_1_1 | *Candida albicans* |
| 918 | SEC20_1_1 | *Candida albicans* |
| 919 | rbcL_1_1 | *Glycine max* |
| 920 | LDHA(hu)_1_1 | *Homo sapiens* |
| 921 | GAPD(hu)_1_1 | *Homo sapiens* |
| 922 | b-Act(hu)_1_1 | *Homo sapiens* |
| 923 | ARHGDIA(hu)_1_1 | *Homo sapiens* |
| 924 | PGK1(hu)_1_1 | *Homo sapiens* |
| 925 | rbcL_1_2 | *Glycine max* |
| 926 | 16SPa_1_1 | *Pseudomonas aeruginosa* |
| 927 | 23SEfaecium_2_1 | *Enterococcus faecium* |
| 928 | 16SStrepyog_1_1 | *Streptococcus pyogenes* |
| 929 | 16SStrepneu_1_1 | *Streptococcus pneumoniae* |
| 930 | 16SStrepagalactiae_1_1 | *Streptococcus agalactiae* |
| 931 | 16SEfaecium_1_1 | *Enterococcus faecium* |
| 932 | 16SEfaecium_2_1 | *Enterococcus faecium* |
| 933 | 16SRNAEf_2_1 | *Enterococcus faecalis* |
| 934 | 16SKpn_1_1 | *Klebsiella pneumoniae* |
| 935 | 16SSa_3_1 | *Staphylococcus aureus* |
| 936 | 16SRNAEf_1_1 | *Enterococcus faecalis* |
| 937 | 16SShominis_1_1 | *Staphylococcus hominis* |
| 938 | 16SShaemolyt_1_1 | *Staphylococcus haemolyticus* |
| 939 | 23SEfaecium_1_1 | *Enterococcus faecium* |
| 940 | 16SrRNAPrmi_1_1 | *Proteus mirabilis* |
| 941 | 16SrRNAPrvu1_1_1 | *Proteus vulgaris* |
| 942 | 16SSa_1_1 | *Staphylococcus aureus* |
| 943 | 16SKlox_1_1 | *Klebsiella oxytoca* |
| 944 | p53_1_1 | *Mus musculus* |
| 945 | 0135mihck_1_1 | *Dictyostelium discoideum* |
| 946 | FAN_1_1 | *Mus musculus* |
| 947 | 0270cap_1 _1 | *Dictyostelium discoideum* |

b) primer sequences

| SEQ ID NO | Probe name | Direction |
|---|---|---|
| 948 | cataSaur_1_1 | F(orward) |
| 949 | cataSaur_1_1 | R(everse) |
| 950 | cataSaur_1_2 | F |
| 951 | cataSaur_1_2 | R |
| 952 | clfA_1_1 | F |
| 953 | clfA_1_1 | R |
| 954 | clfB_1_1 | F |
| 955 | clfB_1_1 | R |
| 956 | coa_1_1 | F |
| 957 | coa_1_1 | R |
| 958 | coa_1_2 | F |
| 959 | coa_1_2 | R |
| 960 | I-clpC_1_1 | F |
| 961 | I-clpC_1_1 | R |
| 962 | I-clpP_1_1 | F |
| 963 | I-clpP_1_1 | R |
| 964 | I-ctaA_1_1 | F |
| 965 | I-ctaA_1_1 | R |
| 966 | I-ctsR_1_1 | F |
| 967 | I-ctsR_1_1 | R |
| 968 | I-dltA_1_1 | F |
| 969 | I-dltA_1_1 | R |
| 970 | I-dltB_1_1 | F |
| 971 | I-dltB_1_1 | R |
| 972 | I-dltC_1_1 | F |
| 973 | I-dltc_1_1 | R |
| 974 | I-dnaK_1_1 | F |
| 975 | I-dnaK_1_1 | R |
| 976 | I-elkT_1_1 | F |
| 977 | I-elkT_1_1 | R |
| 978 | I-femD_1_1 | F |
| 979 | I-femD_1_1 | R |
| 980 | I-glnA_1 _1 | F |
| 981 | I-glnA_1_1 | R |
| 982 | I-glnR_1_1 | F |
| 983 | I-glnR_1_1 | R |
| 984 | I-grlA_1_1 | F |
| 985 | I-grlA_1_1 | R |
| 986 | I-grlB_1_1 | F |
| 987 | I-grlB_1_1 | R |
| 988 | I-groEL_1_1 | F |
| 989 | I-groEL_1_1 | R |
| 990 | I-groES_1_1 | F |
| 991 | I-groES_1_1 | R |
| 992 | I-hemA_1_1 | F |
| 993 | I-hemA_1_1 | R |
| 994 | I-hemE_1_1 | F |
| 995 | I-hemE_1_1 | R |
| 996 | I-hemH_1_1 | F |
| 997 | I-hemH_1_1 | R |
| 998 | I-hemL_1_1 | F |
| 999 | I-hemL_1_1 | R |
| 1000 | I-hemY_1_1 | F |
| 1001 | I-hemY_1_1 | R |
| 1002 | I-lepA_1 _1 | F |
| 1003 | I-lepA_1 _1 | R |
| 1004 | I-lrgA_1_1 | F |
| 1005 | I-lrgA_1_1 | R |
| 1006 | I-IrgB_1_1 | F |
| 1007 | I-IrgB_1_1 | R |
| 1008 | I-IytM_1_1 | F |
| 1009 | I-IytM_1_1 | R |
| 1010 | I-menB_1_1 | F |
| 1011 | I-menB_1_1 | R |
| 1012 | I-menD_1_1 | F |
| 1013 | I-menD_1_1 | R |
| 1014 | I-menE_1_1 | F |
| 1015 | I-menE_1_1 | R |
| 1016 | I-menF_1_1 | F |
| 1017 | I-menF_1_1 | R |
| 1018 | I-mreB_1_1 | F |
| 1019 | I-mreB_1_1 | R |
| 1020 | I-mreR_1_1 | F |
| 1021 | I-mreR_1_1 | R |
| 1022 | I-mutL_1_1 | F |
| 1023 | I-mutL_1_1 | R |
| 1024 | I-mutS_1_1 | F |
| 1025 | I-mutS_1_1 | R |
| 1026 | I-NAG_1_1 | F |
| 1027 | I-NAG_1_1 | R |
| 1028 | I-pbg_1_1 | F |
| 1029 | I-pbg_1_1 | R |
| 1030 | I-pbpF_1_1 | F |
| 1031 | I-pbpF_1_1 | R |
| 1032 | I-pdhB_1_1 | F |
| 1033 | I-pdhB_1_1 | R |
| 1034 | I-pdhC_1_1 | F |
| 1035 | I-pdhC_1_1 | R |
| 1036 | I-rsbU_1_1 | F |
| 1037 | I-rsbU_1_1 | R |
| 1038 | I-rsbV_1_1 | F |
| 1039 | I-rsbV_1_1 | R |
| 1040 | I-rsbW_1_1 | F |
| 1041 | I-rsbW_1_1 | R |
| 1042 | I-sgp_1_1 | F |
| 1043 | I-sgp_1_1 | R |
| 1044 | I-sirR_1_1 | F |
| 1045 | I-sirR_1_1 | R |
| 1046 | I-sodA_1_1 | F |
| 1047 | I-sodA_1_1 | R |
| 1048 | I-sodB_1_1 | F |
| 1049 | I-sodB_1_1 | R |
| 1050 | I-sstA_1_1 | F |
| 1051 | I-sstA_1_1 | R |
| 1052 | I-sstB_1_1 | F |
| 1053 | I-sstB_1_1 | R |
| 1054 | I-sstC_1_1 | F |
| 1055 | I-sstC_1_1 | R |
| 1056 | I-sstD_1_1 | F |
| 1057 | I-sstD_1_1 | R |
| 1058 | I-trx_1_1 | F |
| 1059 | I-trx_1_1 | R |
| 1060 | I-yhiN_1_1 | F |
| 1061 | I-yhiN_1_1 | R |
| 1062 | epiP-bsaP_1_1 | F |
| 1063 | epiP-bsaP_1_1 | R |
| 1064 | geh_1_1 | F |
| 1065 | geh_1_1 | R |
| 1066 | gyrA_1_1 | F |
| 1067 | gyrA_1_1 | R |
| 1068 | gyrB_1_1 | F |
| 1069 | gyrB_1_1 | R |
| 1070 | hemB_1_1 | F |
| 1071 | hemB_1_1 | R |
| 1072 | hemC_1_1 | F |
| 1073 | hemC_1_1 | R |
| 1074 | hemD_1_1 | F |
| 1075 | hemD_1_1 | R |
| 1076 | hemN_1_1 | F |
| 1077 | hemN_1_1 | R |
| 1078 | hsdS_1_1 | F |
| 1079 | hsdS_1_1 | R |
| 1080 | hsdS_2_1 | F |
| 1081 | hsdS_2_1 | R |
| 1082 | lip_1 _1 | F |
| 1083 | lip_1_1 | R |
| 1084 | menC_1_1 | F |
| 1085 | menC_1_1 | R |
| 1086 | murC_1_1 | F |
| 1087 | murC_1_1 | R |
| 1088 | nuc_1_1 | F |
| 1089 | nuc_1_1 | R |
| 1090 | pdhD_1_1 | F |
| 1091 | pdhD_1_1 | R |
| 1092 | rpoB_1_1 | F |
| 1093 | rpoB_1_1 | R |
| 1094 | SAV0431_1_1 | F |
| 1095 | SAV0431_1_1 | R |
| 1096 | SAV0439_1_1 | F |
| 1097 | SAV0439_1_1 | R |
| 1098 | SAV0440_1_1 | F |
| 1099 | SAV0440_1_1 | R |
| 1100 | SAV0441_1_1 | F |
| 1101 | SAV0441_1_1 | R |
| 1102 | sigB_1_1 | F |
| 1103 | sigB_1_1 | R |
| 1104 | spa_1_2 | F |
| 1105 | spa_1_2 | R |
| 1106 | sstC_1_1 | F |
| 1107 | sstC_1_1 | R |
| 1108 | tag_1_1 | F |
| 1109 | tag_1_1 | R |
| 1110 | tyrA_1_1 | F |
| 1111 | tyrA_1_1 | R |
| 1112 | I-aroC_1_1 | F |
| 1113 | I-aroC_1_1 | R |
| 1114 | I-aroA_1_1 | F |
| 1115 | I-aroA_1_1 | R |
| 1116 | I-cna_1_1 | F |
| 1117 | I-cna_1_1 | R |
| 1118 | I-ebpS_1_1 | F |
| 1119 | I-ebpS_1_1 | R |
| 1120 | I-eno_1_1 | F |
| 1121 | I-eno_1_1 | R |
| 1122 | I-fbpA_1_1 | F |
| 1123 | I-fbpA_1_1 | R |
| 1124 | I-fib_1_1 | F |
| 1125 | I-fib_1_1 | R |
| 1126 | I-fnbB_1_1 | F |
| 1127 | I-fnbB_1_1 | R |
| 1128 | I-srtA_1_1 | F |
| 1129 | I-srtA_1_1 | R |
| 1130 | I-stpC_1_1 | F |
| 1131 | I-stpC_1_1 | R |
| 1132 | I-fnbA_1_1 | F |
| 1133 | I-fnbA_1_1 | R |
| 1134 | I-spa_1_1 | F |
| 1135 | I-spa_1_1 | R |
| 1136 | I-aroE_1_1 | F |
| 1137 | I-aroE_1_1 | R |
| 1138 | I-aroF_1_1 | F |
| 1139 | I-aroF_1_1 | R |
| 1140 | I-aroG_1_1 | F |
| 1141 | I-aroG_1_1 | R |
| 1142 | I-asp23_1_1 | F |
| 1143 | I-asp23_1_1 | R |
| 1144 | I-atl_1_1 | F |
| 1145 | I-atl_1_1 | R |
| 1146 | bsaE_1_1 | F |
| 1147 | bsaE_1_1 | R |
| 1148 | bsaG_1_1 | F |
| 1149 | bsaG_1_1 | R |
| 1150 | cap5h_1_1 | F |
| 1151 | cap5h_1_1 | R |
| 1152 | cap5i_1_1 | F |
| 1153 | cap5i_1_1 | R |
| 1154 | cap5j_1_1 | F |
| 1155 | cap5j_1_1 | R |
| 1156 | cap5k_1_1 | F |
| 1157 | cap5k_1_1 | R |
| 1158 | capBH_1_1 | F |
| 1159 | cap8H_1_1 | R |
| 1160 | cap8I_1_1 | F |
| 1161 | cap8I_1_1 | R |
| 1162 | cap8J_1_1 | F |
| 1163 | cap8J_1_1 | R |
| 1164 | cap8K_1_1 | F |
| 1165 | cap8K_1_1 | R |
| 1166 | I-hld_1_1 | F |
| 1167 | I-hld_1_1 | R |
| 1168 | I-hysA_1_1 | F |
| 1169 | I-hysA_1_1 | R |
| 1170 | I-IgGbg_1_1 | F |
| 1171 | I-IgGbg_1_1 | R |
| 1172 | EDIN_1_1 | F |
| 1173 | EDIN_1_1 | R |
| 1174 | eta_1_1 | F |
| 1175 | eta_1_1 | R |
| 1176 | etb_1_1 | F |
| 1177 | etb_1_1 | R |
| 1178 | hglA_1_1 | F |
| 1179 | hglA_1_1 | R |
| 1180 | hglA_2_1 | F |
| 1181 | hglA_2_1 | R |
| 1182 | hglB_1_1 | F |
| 1183 | hglB_1_1 | R |
| 1184 | hglC_2_1 | F |
| 1185 | hglC_2_1 | R |
| 1186 | hla_1_1 | F |
| 1187 | hla_1_1 | R |
| 1188 | hlb_1_2 | F |
| 1189 | hlb_1_2 | R |
| 1190 | lukF_1_1 | F |
| 1191 | lukF_1_1 | R |
| 1192 | lukS_1_1 | F |
| 1193 | lukS_1_1 | R |
| 1194 | lukS_2_1 | F |
| 1195 | lukS_2_1 | R |
| 1196 | NAG_1_1 | F |
| 1197 | NAG_1_1 | R |
| 1198 | sak_1_1 | F |
| 1199 | sak_1_1 | R |
| 1200 | sea_1_1 | F |
| 1201 | sea_1_1 | R |
| 1202 | seb_1_1 | F |
| 1203 | seb_1_1 | R |
| 1204 | sec1_1_1 | F |
| 1205 | sec1_1_1 | R |
| 1206 | seg_1_1 | F |
| 1207 | seg_1_1 | R |
| 1208 | seh_1_1 | F |
| 1209 | seh_1_1 | R |
| 1210 | sel_1_1 | F |
| 1211 | sel_1_1 | R |
| 1212 | set15_1_1 | F |
| 1213 | set15_1_1 | R |
| 1214 | set6_1_1 | F |
| 1215 | set6_1_1 | R |
| 1216 | set7_1_1 | F |
| 1217 | set7_1_1 | R |
| 1218 | set8_1_1 | F |
| 1219 | set8_1_1 | R |
| 1220 | sprV8_1_1 | F |
| 1221 | sprV8_1_1 | R |
| 1222 | tst_1_1 | F |
| 1223 | tst_1_1 | R |
| 1224 | I-sdrC_1_1 | F |
| 1225 | I-sdrC_1_1 | R |
| 1226 | I-sdrD_1_1 | F |
| 1227 | I-sdrD_1_1 | R |
| 1228 | I-sdrE_1_1 | F |
| 1229 | I-sdrE_1_1 | R |
| 1230 | b1169_1_1 | F |
| 1231 | b1169_1_1 | R |
| 1232 | envZ_1_1 | F |
| 1233 | envZ_1_1 | R |
| 1234 | fliCb_1_1 | F |
| 1235 | fliCb_1_1 | R |
| 1236 | nfrB_1_1 | F |
| 1237 | nfrB_1_1 | R |
| 1238 | nlpA_1_1 | F |
| 1239 | nlpA_1_1 | R |
| 1240 | pilAe_1_1 | F |
| 1241 | pilAe_1_1 | R |
| 1242 | yacH_1_1 | F |
| 1243 | yacH_1_1 | R |
| 1244 | yagX_1_1 | F |
| 1245 | yagX_1_1 | R |
| 1246 | ycdS_1_1 | F |
| 1247 | ycdS_1_1 | R |
| 1248 | yciQ_1_1 | F |
| 1249 | yciQ_1_1 | R |
| 1250 | ymcA_1_1 | F |
| 1251 | ymcA_1_1 | R |
| 1252 | b1202_1_1 | F |
| 1253 | b1202_1_1 | R |
| 1254 | eae_1_1 | F |
| 1255 | eae_1_1 | R |
| 1256 | eltB_1_1 | F |
| 1257 | eltB_1_1 | R |
| 1258 | escR_1_1 | F |
| 1259 | escR_1_1 | R |
| 1260 | escT_1_1 | F |
| 1261 | escT_1_1 | R |
| 1262 | escU_1_1 | F |
| 1263 | escU_1_1 | R |
| 1264 | espB_1_1 | F |
| 1265 | espB_1_1 | R |
| 1266 | fes_1_1 | F |
| 1267 | fes_1_1 | R |
| 1268 | fes_2_1 | F |
| 1269 | fes_2_1 | R |
| 1270 | fteA_1_1 | F |
| 1271 | fteA_1_1 | R |
| 1272 | hlyA_1_1 | F |
| 1273 | hlyA_1_1 | R |
| 1274 | hlyB_1_1 | F |
| 1275 | hlyB_1_1 | R |
| 1276 | iucA_1_1 | F |
| 1277 | iucA_1_1 | R |
| 1278 | iucB_1_1 | F |
| 1279 | iucB_1_1 | R |
| 1280 | iucC_1_1 | F |
| 1281 | iucC_1_1 | R |
| 1282 | papG_1_1 | F |
| 1283 | papG_1_1 | R |
| 1284 | rfbE_1_1 | F |
| 1285 | rfbE_1_1 | R |
| 1286 | shuA_1_1 | F |
| 1287 | shuA_1_1 | R |
| 1288 | SLTII_1_1 | F |
| 1289 | SLTII_1_1 | R |
| 1290 | toxA-LTPA_1_1 | F |
| 1291 | toxA-LTPA_1_1 | R |
| 1292 | VT2vaB_1_1 | F |
| 1293 | VT2vaB_1_1 | R |
| 1294 | ardeSE0106_1_1 | F |
| 1295 | ardeSE0106_1_1 | R |
| 1296 | ardeSE0107_1_1 | F |
| 1297 | ardeSE0107_1_1 | R |
| 1298 | aroiSE0105_1_1 | F |
| 1299 | aroiSE0105_1_1 | R |
| 1300 | atlE_1_1 | F |
| 1301 | atlE_1_1 | R |
| 1302 | agrB_1_1 | F |
| 1303 | agrB_1_1 | R |
| 1304 | agrC_1_1 | F |
| 1305 | agrC_1_1 | R |
| 1306 | alphSE1368_1_1 | F |
| 1307 | alphSE1368_1_1 | R |
| 1308 | gad_1_1 | F |
| 1309 | gad_1_1 | R |
| 1310 | glucSE1191_1_1 | F |
| 1311 | glucSE1191_1_1 | R |
| 1312 | hsp10_1_1 | F |
| 1313 | hsp10_1_1 | R |
| 1314 | icaA_1_1 | F |
| 1315 | icaA_1_1 | R |
| 1316 | icaB_1_1 | F |
| 1317 | icaB_1_1 | R |
| 1318 | mvaSSepid_1_1 | F |
| 1319 | mvaSSepid_1_1 | R |
| 1320 | nitreSE1972_1_1 | F |
| 1321 | nitreSE1972_1_1 | R |
| 1322 | nitreSE1974_1_1 | F |
| 1323 | nitreSE1974_1_1 | R |
| 1324 | nitreSE1975_1_1 | F |
| 1325 | nitreSE1975_1_1 | R |
| 1326 | oiamtSE1209_1_1 | F |
| 1327 | oiamtSE1209_1_1 | R |
| 1328 | ORF1Sepid_1_1 | F |
| 1329 | ORF1Sepid_1_1 | R |
| 1330 | ORF3bSepid_1_1 | F |
| 1331 | ORF3bSepid_1_1 | R |
| 1332 | qacR_1_1 | F |
| 1333 | qacR_1_1 | R |
| 1334 | sin_1_1 | F |
| 1335 | sin_1_1 | R |
| 1336 | ureSE1861_1_1 | F |
| 1337 | ureSE1861_1_1 | R |
| 1338 | ureSE1863_1_1 | F |
| 1339 | ureSE1863_1_1 | R |
| 1340 | ureSE1864_1_1 | F |
| 1341 | ureSE1864_1_1 | R |
| 1342 | ureSE1865_1_1 | F |
| 1343 | ureSE1865_1_1 | R |
| 1344 | ureSE1867_1_1 | F |
| 1345 | ureSE1867_1_1 | R |
| 1346 | 9caD_1_1 | F |
| 1347 | gcaD_1_1 | R |
| 1348 | hld_orf5_1_1 | F |
| 1349 | hld_orf5_1_1 | R |
| 1350 | icaC_1_1 | F |
| 1351 | icaC_1_1 | R |
| 1352 | icaD_1_1 | F |
| 1353 | icaD_1_1 | R |
| 1354 | icaR_1_1 | F |
| 1355 | icaR_1_1 | R |
| 1356 | psm_beta 1 and2_1_1 | F |
| 1357 | psm_beta1and2_1_1 | R |
| 1358 | purR_1_1 | F |
| 1359 | purR_1_1 | R |
| 1360 | spoVG_1_1 | F |
| 1361 | spoVG_1_1 | R |
| 1362 | yabJ_1_1 | F |
| 1363 | yabJ_1_1 | R |
| 1364 | folQShaemolyt_1_1 | F |
| 1365 | folQShaemolyC_1_1 | R |
| 1366 | mvaCShaem olyticus_1_1 | F |
| 1367 | mvaCShaem olyticus_1 _1 | R |
| 1368 | mvaDShaemolyt_1_1 | F |
| 1369 | mvaDShaemolyt_1_1 | R |
| 1370 | mvaK1Shaemolyticus_1_1 | F |
| 1371 | mvaKiShaemolyticus_1_1 | R |
| 1372 | mvaSShaemolyticus_1_1 | F |
| 1373 | mvaSShaemolyticus_1_1 | R |
| 1374 | RNApolsigm_1_1 | F |
| 1375 | RNApolsigm_1_1 | R |
| 1376 | lipShaemolyC1_1 | F |
| 1377 | lipShaemolyt_1_1 | R |
| 1378 | agrB2Stalugd_1_1 | F |
| 1379 | agrB2Stalugd_1_1 | R |
| 1380 | agrC2Stalugd_1_1 | F |
| 1381 | agrC2Stalugd_1_1 | R |
| 1382 | agrCStalugd_1_1 | F |
| 1383 | agrCStalugd_1_1 | R |
| 1384 | slamStalugd_1_1 | F |
| 1385 | slamStalugd_1_1 | R |
| 1386 | fblStalugd_1_1 | F |
| 1387 | fblStalugd_1_1 | R |
| 1388 | slushABCStalugd_1_1 | F |
| 1389 | slushABCStalugd_1_1 | R |
| 1390 | RNApolsigmSsapro_1_1 | F |
| 1391 | RNApolsigmSsapro_1_1 | R |
| 1392 | RNApolsigmSsapro_1_2 | F |
| 1393 | RNApolsigmSsapro_1_2 | R |
| 1394 | msrw1Stwar_1_1 | F |
| 1395 | msrw1Stwar_1_1 | R |
| 1396 | nukMStwar_1_1 | F |
| 1397 | nukMStwar_1_1 | R |
| 1398 | proDStwar_1_1 | F |
| 1399 | proDStwar_1_1 | R |
| 1400 | proMStwar_1_1 | F |
| 1401 | proMStwar_1_1 | R |
| 1402 | sigrpoStwar_1_1 | F |
| 1403 | sigrpoStwar_1_1 | R |
| 1404 | tnpStwar_1 _1 | F |
| 1405 | tnpStwar_1 _1 | R |
| 1406 | gehAStwar_1 _1 | F |
| 1407 | gehAStwar_1_1 | R |
| 1408 | ARG56_1_1 | F |
| 1409 | ARG56_1_1 | R |
| 1410 | ASL43f_1_1 | F |
| 1411 | ASL43f_1_1 | R |
| 1412 | BGL2_1_1 | F |
| 1413 | BGL2_1_1 | R |
| 1414 | CACHS3_1_1 | F |
| 1415 | CACHS3_1_1 | R |
| 1 41 6 | CCT8_1_1 | F |
| 1417 | CCT8_1_1 | R |
| 1418 | CDC37_1_1 | F |
| 1419 | CDC37_1_1 | R |
| 1420 | CEF3_1_1 | F |
| 1421 | CEF3_1_1 | R |
| 1422 | CHS1_1_1 | F |
| 1423 | CHS1_1_1 | R |
| 1424 | CHS2_1_1 | F |
| 1425 | CHS2_1_1 | R |
| 1426 | CHS4_1_1 | F |
| 1427 | CHS4_1_1 | R |
| 1428 | CHS5_1_1 | F |
| 1429 | CHS5_1_1 | R |
| 1430 | CHT1_1_1 | F |
| 1431 | CHT1_1_1 | R |
| 1432 | CHT2_1_1 | F |
| 1433 | CHT2_1_1 | R |
| 1434 | CHT4_1_1 | F |
| 1435 | CHT4_1_1 | R |
| 1436 | CSA1_1_1 | F |
| 1437 | CSA1_1_1 | R |
| 1438 | 5triphosphatase_1 _1 | F |
| 1439 | 5triphosphatase_1 _1 | R |
| 1440 | AAF1_1_1 | F |
| 1441 | AAF1_1_1 | R |
| 1442 | ADH1_1_1 | F |
| 1443 | ADH1_1_1 | R |
| 1444 | ALS1_1_1 | F |
| 1445 | ALS1_1_1 | R |
| 1446 | ALS7_1_1 | F |
| 1447 | ALS7_1_1 | R |
| 1448 | EDT1_1_1 | F |
| 1449 | EDT1_1_1 | R |
| 1450 | ELF_1_1 | F |
| 1451 | ELF_1_1 | R |
| 1452 | ESS1_1_1 | F |
| 1453 | ESS1_1_1 | R |
| 1454 | FAL1_1_1 | F |
| 1455 | FAL1_1_1 | R |
| 1456 | GAP1_1_1 | F |
| 1457 | GAP1_1_1 | R |
| 1458 | GNA1_1_1 | F |
| 1459 | GNA1_1_1 | R |
| 1460 | GSC1_1_1 | F |
| 1461 | GSC1_1_1 | R |
| 1462 | GSL1_1_1 | F |
| 1463 | GSL1_1_1 | R |
| 1464 | HlS1_1_1 | F |
| 1465 | HIS1_1_1 | R |
| 1466 | HTS1_1_1 | F |
| 1467 | HTS1_1_1 | R |
| 1468 | HWP1_2_1 | F |
| 1469 | HWP1_2_1 | R |
| 1470 | HYR1_1_1 | F |
| 1471 | HYR1_1_1 | R |
| 1472 | INT1a_1_1 | F |
| 1473 | INT1a_1_1 | R |
| 1474 | KRE15f_1_1 | F |
| 1475 | KRE15f_1_1 | R |
| 1476 | KRE6_1_1 | F |
| 1477 | KRE6_1_1 | R |
| 1478 | KRE9_1_1 | F |
| 1479 | KRE9_1_1 | R |
| 1480 | MIG1_1_1 | F |
| 1481 | MIG1_1_1 | R |
| 1482 | MLS1_1_1 | F |
| 1483 | MLS1_1_1 | R |
| 1484 | MP65_1_1 | F |
| 1485 | MP65_1_1 | R |
| 1486 | NDE1_1_1 | F |
| 1487 | NDE1_1_1 | R |
| 1488 | PFK2_1_1 | F |
| 1489 | PFK2_1_1 | R |
| 1490 | PHR1_1_1 | F |
| 1491 | PHR1_1_1 | R |
| 1492 | PHR2_1_1 | F |
| 1493 | PHR2_1_1 | R |
| 1494 | PHR3_1_1 | F |
| 1495 | PHR3_1_1 | R |
| 1496 | PRA1_1_1 | F |
| 1497 | PRA1_1_1 | R |
| 1498 | PRS1_1_1 | F |
| 1499 | PRS1_1_1 | R |
| 1500 | RBT1_1_1 | F |
| 1501 | RBT1_1_1 | R |
| 1502 | RBT4_1_1 | F |
| 1503 | RBT4_1_1 | R |
| 1504 | RHO1_1_1 | F |
| 1505 | RHO1_1_1 | R |
| 1506 | RNR1_1_1 | F |
| 1507 | RNR1_1_1 | R |
| 1508 | RPB7_1_1 | F |
| 1509 | RPB7_1_1 | R |
| 1510 | RPL13_1_1 | F |
| 1511 | RPL13_1_1 | R |
| 1512 | RVS167_1_1 | F |
| 1513 | RVS167_1_1 | R |
| 1514 | SHA3_1_1 | F |
| 1515 | SHA3_1_1 | R |
| 1516 | SKN1_1_1 | F |
| 1517 | SKN1_1_1 | R |
| 1518 | SRB1_1_1 | F |
| 1519 | SRB1_1_1 | R |
| 1520 | TCA1_1_1 | F |
| 1521 | TCA1_1_1 | R |
| 1522 | TRP1_1_1 | F |
| 1523 | TRP1_1_1 | R |
| 1524 | YAE1_1_1 | F |
| 1525 | YAE1_1_1 | R |
| 1526 | YRB1_1_1 | F |
| 1527 | YRB1_1_1 | R |
| 1528 | YST1exon2_1_1 | F |
| 1529 | YST1exon2_1_1 | R |
| 1530 | CCN1_1_1 | F |
| 1531 | CCN1_1_1 | R |
| 1532 | CDC28_1_1 | F |
| 1533 | CDC28_1_1 | R |
| 1534 | CLN2_1_1 | F |
| 1535 | CLN2_1_1 | R |
| 1536 | CPH1_1_1 | F |
| 1537 | CPH1_1_1 | R |
| 1538 | CYB1_1_1 | F |
| 1539 | CYB1_1_1 | R |
| 1540 | EFG1_1_1 | F |
| 1541 | EFG1_1_1 | R |
| 1542 | MNT1_1_1 | F |
| 1543 | MNT1_1_1 | R |
| 1544 | RBF1_1_1 | F |
| 1545 | RBF1_1_1 | R |
| 1546 | RBF1_2_1 | F |
| 1547 | RBF1_2_1 | R |
| 1548 | RIM101_1_1 | F |
| 1549 | RIM101_1_1 | R |
| 1550 | RIM8_1_1 | F |
| 1551 | RIM8_1_1 | R |
| 1552 | SEC14_1_1 | F |
| 1553 | SEC14_1_1 | R |
| 1554 | SEC4_1_1 | F |
| 1555 | SEC4_1_1 | R |
| 1556 | TUP1_1_1 | F |
| 1557 | TUP1_1_1 | R |
| 1558 | YPT1_1_1 | F |
| 1559 | YPT1_1_1 | R |
| 1560 | ZNF1CZF1_2_1 | F |
| 1561 | ZNF1CZF1_2_1 | R |
| 1562 | arcA_1_1 | F |
| 1563 | arcA_1_1 | R |
| 1564 | arcC_1_1 | F |
| 1565 | arcC_1_1 | R |
| 1566 | bkdA_1_1 | F |
| 1567 | bkdA_1_1 | R |
| 1568 | cad_1_1 | F |
| 1569 | cad_1_1 | R |
| 1570 | camE1_1_1 | F |
| 1571 | camE1_1_1 | R |
| 1572 | csrA_1_1 | F |
| 1573 | csrA_1_1 | R |
| 1574 | dacA_1_1 | F |
| 1575 | dacA_1_1 | R |
| 1576 | dfr_1_1 | F |
| 1577 | dfr_1_1 | R |
| 1578 | dhoD1a_1_1 | F |
| 1579 | dhoD1a_1_1 | R |
| 1580 | ABC-eltA_1_1 | F |
| 1581 | ABC-eltA_1_1 | R |
| 1582 | agrBfs_1_1 | F |
| 1583 | agrBfs_1_1 | R |
| 1584 | agrCfs_1_1 | F |
| 1585 | agrCfs_1_1 | R |
| 1586 | dnaE_1_1 | F |
| 1587 | dnaE_1_1 | R |
| 1588 | ebsA_1_1 | F |
| 1589 | ebsA_1_1 | R |
| 1590 | ebsB_1_1 | F |
| 1591 | ebsB_1_1 | R |
| 1592 | eep_1_1 | F |
| 1593 | eep_1_1 | R |
| 1594 | efaR_1_1 | F |
| 1595 | efaR_1_1 | R |
| 1596 | gls24_glsB_1_1 | F |
| 1597 | gls24_glsB_1_1 | R |
| 1598 | gph_1_1 | F |
| 1599 | gph_1_1 | R |
| 1600 | gyrAEf_1 _1 | F |
| 1601 | gyrAEf_1 _1 | R |
| 1602 | metEf_1_1 | F |
| 1603 | metEf_1_1 | R |
| 1604 | mntHCb2_1_1 | F |
| 1605 | mntHCb2_1_1 | R |
| 1606 | mob2_1_1 | F |
| 1607 | mob2_1_1 | R |
| 1608 | mvaD_1_1 | F |
| 1609 | mvaD_1_1 | R |
| 1610 | mvaE_1_1 | F |
| 1611 | mvaE_1_1 | R |
| 1612 | parC_1 _1 | F |
| 1613 | parC_1 _1 | R |
| 1614 | pcfG_1_1 | F |
| 1615 | pcfG_1 _1 | R |
| 1616 | phoZ_1_1 | F |
| 1617 | phoZ_1_1 | R |
| 1618 | polC_1_1 | F |
| 1619 | polC_1_1 | R |
| 1620 | ptb_1_1 | F |
| 1621 | ptb_1_1 | R |
| 1622 | recS1_1_1 | F |
| 1623 | recS1_1_1 | R |
| 1624 | rpoN_1_1 | F |
| 1625 | rpoN_1_1 | R |
| 1626 | tms_1_1 | F |
| 1627 | tms_1_1 | R |
| 1628 | tyrDC_1_1 | F |
| 1629 | tyrDC_1 _1 | R |
| 1630 | tyrS_1_1 | F |
| 1631 | tyrS_1_1 | R |
| 1632 | asa1_1_1 | F |
| 1633 | asa1_1_1 | R |
| 1634 | asp1_1_1 | F |
| 1635 | asp1_1_1 | R |
| 1636 | cgh_1_1 | F |
| 1637 | cgh_1_1 | R |
| 1638 | cylA_1_1 | F |
| 1639 | cylA_1_1 | R |
| 1640 | cyIB_1_1 | F |
| 1641 | cylB_1_1 | R |
| 1642 | cylI_1_1 | F |
| 1643 | cylI_1_1 | R |
| 1644 | cylL_cylS_1_1 | F |
| 1645 | cylL_cylS_1_1 | R |
| 1646 | cylM_1_1 | F |
| 1647 | cylM_1_1 | R |
| 1648 | ace_1_1 | F |
| 1649 | ace_1_1 | R |
| 1650 | ef00108_1_1 | F |
| 1651 | ef00108_1_1 | R |
| 1652 | ef00109_1_1 | F |
| 1653 | ef00109_1_1 | R |
| 1654 | ef0011_1_1 | F |
| 1655 | ef0011_1_1 | R |
| 1656 | ef00113_1_1 | F |
| 1657 | ef00113_1_1 | R |
| 1658 | ef0012_1_1 | F |
| 1659 | ef0012_1_1 | R |
| 1660 | ef0022_1_1 | F |
| 1661 | ef0022_1_1 | R |
| 1662 | ef0031_1_1 | F |
| 1663 | ef0031_1_1 | R |
| 1664 | ef0032_1_1 | F |
| 1665 | ef0032_1_1 | R |
| 1666 | ef0040_1_1 | F |
| 1667 | ef0040_1_1 | R |
| 1668 | ef0058_1_1 | F |
| 1669 | ef0058_1_1 | R |
| 1670 | enlA_1_1 | F |
| 1671 | enlA_1_1 | R |
| 1672 | esa_1_1 | F |
| 1673 | esa_1_1 | R |
| 1674 | esp_1_1 | F |
| 1675 | esp_1_1 | R |
| 1676 | gelE_1_1 | F |
| 1677 | gelE_1_1 | R |
| 1678 | groEL_1_1 | F |
| 1679 | groEL_1_1 | R |
| 1680 | groES_1_1 | F |
| 1681 | groES_1_1 | R |
| 1682 | rt1_1_1 | F |
| 1683 | rt1_1_1 | R |
| 1684 | sala_1_1 | F |
| 1685 | sala_1_1 | R |
| 1686 | salb_1_1 | F |
| 1687 | salb_1_1 | R |
| 1688 | sea1_1_1 | F |
| 1689 | sea1_1_1 | R |
| 1690 | sep1_1_1 | F |
| 1691 | sep1_1_1 | R |
| 1692 | vicK_1_1 | F |
| 1693 | vicK_1_1 | R |
| 1694 | yycH_1_1 | F |
| 1695 | yycH_1_1 | R |
| 1696 | yycl_1_1 | F |
| 1697 | yycl_1_1 | R |
| 1698 | yycJ_1_1 | F |
| 1699 | yycJ_1_1 | R |
| 1700 | bglB_1_1 | F |
| 1701 | bglB_1_1 | R |
| 1702 | bglR_1_1 | F |
| 1703 | bglR_1_1 | R |
| 1704 | bglS_1_1 | F |
| 1705 | bglS_1_1 | R |
| 1706 | efmA_1_1 | F |
| 1707 | efmA_1_1 | R |
| 1708 | efmb_1_1 | F |
| 1709 | efmB_1_1 | R |
| 1710 | efmC_1_1 | F |
| 1711 | efmC_1_1 | R |
| 1712 | mreC_1_1 | F |
| 1713 | mreC_1_1 | R |
| 1714 | mreD_1_1 | F |
| 1715 | mreD_1_1 | R |
| 1716 | mvaDEfaecium_1_1 | F |
| 1717 | mvaDEfaecium_1_1 | R |
| 1718 | mvaEEfaecium_1_1 | F |
| 1719 | mvaEEfaecium_1_1 | R |
| 1720 | mvaK1Efaecium_1_1 | F |
| 1721 | mvaK1Efaecium_1_1 | R |
| 1722 | mvaK2Efaecium_1_1 | F |
| 1723 | mvaK2Efaecium_1_1 | R |
| 1724 | mvaSEfaecium_1_1 | F |
| 1725 | mvaSEfaecium_1_1 | R |
| 1726 | orf3_4Efaeciumb_1_1 | F |
| 1727 | orf3_4Efaeciumb_1_1 | R |
| 1728 | orf6_7Efaecium_1_1 | F |
| 1729 | orf6_7Efaecium_1_1 | R |
| 1730 | orf7_8Efaecium_1_1 | F |
| 1731 | orf7_8Efaecium_1_1 | R |
| 1732 | orf9_10Efaecium_1_1 | F |
| 1733 | orf9_10Efaecium_1_1 | R |
| 1734 | entA_entl_1_1 | F |
| 1735 | entA_entl_1_1 | R |
| 1736 | entD_1_1 | F |
| 1737 | entD_1_1 | R |
| 1738 | entR_1_1 | F |
| 1739 | entR_1_1 | R |
| 1740 | oep_1_1 | F |
| 1741 | oep_1_1 | R |
| 1742 | sagA_1_2 | F |
| 1743 | sagA_1_2 | R |
| 1744 | atsA_1_1 | F |
| 1745 | atsA_1_1 | R |
| 1746 | atsB_1_1 | F |
| 1747 | atsB_1_1 | R |
| 1748 | budC_1_1 | F |
| 1749 | budC_1_1 | R |
| 1750 | citA_1_1 | F |
| 1751 | citA_1_1 | R |
| 1752 | citW_1_1 | F |
| 1753 | citW_1_1 | R |
| 1754 | citX_1_1 | F |
| 1755 | citX_1_1 | R |
| 1756 | dalD_1_1 | F |
| 1757 | dalD_1_1 | R |
| 1758 | dalK_1_1 | F |
| 1759 | dalK_1_1 | R |
| 1760 | dalT_1_1 | F |
| 1761 | dalT_1_1 | R |
| 1762 | acoA_1_1 | F |
| 1763 | acoA_1_1 | R |
| 1764 | acoB_1_1 | F |
| 1765 | acoB_1_1 | R |
| 1766 | acoC_1_1 | F |
| 1767 | acoC_1_1 | R |
| 1768 | ahlK_1_1 | F |
| 1769 | ahlK_1_1 | R |
| 1770 | fimK_1_1 | F |
| 1771 | fimK_1_1 | R |
| 1772 | glfKPN2_1_1 | F |
| 1773 | glfKPN2_1_1 | R |
| 1774 | ltrA_1_1 | F |
| 1775 | ltrA_1_1 | R |
| 1776 | mdcC_1_1 | F |
| 1777 | mdcC_1_1 | R |
| 1778 | mdcF_1_1 | F |
| 1779 | mdcF_1_1 | R |
| 1780 | mdcH_1_1 | F |
| 1781 | mdcH_1_1 | R |
| 1782 | mrkA_1_1 | F |
| 1783 | mrkA_1_1 | R |
| 1784 | mtrK_1_1 | F |
| 1785 | mtrK_1_1 | R |
| 1786 | nifF_1_1 | F |
| 1787 | nifF_1_1 | R |
| 1788 | nifK_1_1 | F |
| 1789 | nifK_1_1 | R |
| 1790 | nifN_1_1 | F |
| 1791 | nifN_1_1 | R |
| 1792 | tyrP_1_1 | F |
| 1793 | tyrP_1_1 | R |
| 1794 | ureA_1_1 | F |
| 1795 | ureA_1_1 | R |
| 1796 | wbbO_1_1 | F |
| 1797 | wbbO_1_1 | R |
| 1798 | wza_1_1 | F |
| 1799 | wza_1_1 | R |
| 1800 | wzb_1_1 | F |
| 1801 | wzb_1_1 | R |
| 1802 | wzmKPN2_1_1 | F |
| 1803 | wzmKPN2_1_1 | R |
| 1804 | wztKPN2_1_1 | F |
| 1805 | wztKPN2_1_1 | R |
| 1806 | yojH_1_1 | F |
| 1807 | yojH_1_1 | R |
| 1808 | liac_1_1 | F |
| 1809 | liac_1_1 | R |
| 1810 0 | cim_1_1 | F |
| 1811 | cim_1_1 | R |
| 1812 | aldA_1_1 | F |
| 1813 | aldA_1_1 | R |
| 1814 | aldA_2_1 | F |
| 1815 | aldA_2_1 | R |
| 1816 | hemly_1_1 | F |
| 1817 | hemly_1_1 | R |
| 1818 | pSL017_1_1 | F |
| 1819 | pSL017_1_1 | R |
| 1820 | pSL020_1_1 | F |
| 1821 | pSL020_1_1 | R |
| 1822 | rcsA_1_1 | F |
| 1823 | rcsA_1_1 | R |
| 1824 | rmlC_1_1 | F |
| 1825 | rmlC_1_1 | R |
| 1826 | rmlD_1_1 | F |
| 1827 | rmlD_1_1 | R |
| 1828 | waaG_1_1 | F |
| 1829 | waaG_1_1 | R |
| 1830 | wbbD_1_1 | F |
| 1831 | wbbD_1_1 | R |
| 1832 | wbbM_1_1 | F |
| 1833 | wbbM_1_1 | R |
| 1834 | wbbN_1_1 | F |
| 1835 | wbbN_1_1 | R |
| 1836 | wbdA_1_1 | F |
| 1837 | wbdA_1_1 | R |
| 1838 | wbdC_1_1 | F |
| 1839 | wbdC_1_1 | R |
| 1840 | wztKpn_1_1 | F |
| 1841 | wztKpn_1_1 | R |
| 1842 | yibD_1_1 | F |
| 1843 | yibD_1_1 | R |
| 1844 | cymA_1_1 | F |
| 1845 | cymA_1_1 | R |
| 1846 | cymD_1_1 | F |
| 1847 | cymD_1_1 | R |
| 1848 | cymE_1_1 | F |
| 1849 | cymE_1_1 | R |
| 1850 | cymH_1_1 | F |
| 1851 | cymH_1_1 | R |
| 1852 | cymI_1_1 | F |
| 1853 | cymI_1_1 | R |
| 1854 | cymJ_1_1 | F |
| 1855 | cymJ_1_1 | R |
| 1856 | ddrA_1_1 | F |
| 1857 | ddrA_1_1 | R |
| 1858 | fdt-1_1_1 | F |
| 1859 | fdt-1_1_1 | R |
| 1860 | fdt-2_1_1 | F |
| 1861 | fdt-2_1_1 | R |
| 1862 | fdt-3_1_1 | F |
| 1863 | fdt-3_1_1 | R |
| 1864 | gatY_1_1 | F |
| 1865 | gatY_1_1 | R |
| 1866 | hydH_1_1 | F |
| 1867 | hydH_1_1 | R |
| 1868 | masA_1_1 | F |
| 1869 | masA_1_1 | R |
| 1870 | nasA_1_1 | F |
| 1871 | nasA_1_1 | R |
| 1872 | nasE_1_1 | F |
| 1873 | nasE_1_1 | R |
| 1874 | nasF_1_1 | F |
| 1875 | nasF_1_1 | R |
| 1876 | pehX_1_1 | F |
| 1877 | pehX_1_1 | R |
| 1878 | pelX_1_1 | F |
| 1879 | pelX_1_1 | R |
| 1880 | tagH_1_1 | F |
| 1881 | tagH_1 _1 | R |
| 1882 | tagK_1_1 | F |
| 1883 | tagK_1_1 | R |
| 1884 | tagT_1_1 | F |
| 1885 | tagT_1_1 | R |
| 1886 | glpR_1 _1 | F |
| 1887 | glpR_1 _1 | R |
| 1888 | lasRb_1_1 | F |
| 1889 | lasRb_1_1 | R |
| 1890 | OrfX_1_1 | F |
| 1891 | OrfX_1_1 | R |
| 1892 | pa0260_1_1 | F |
| 1893 | pa0260_1_1 | R |
| 1894 | pa0572_1_1 | F |
| 1895 | pa0572_1_1 | R |
| 1896 | pa0625_1_1 | F |
| 1897 | pa0625_1_1 | R |
| 1898 | pa0636_1_1 | F |
| 1899 | pa0636_1_1 | R |
| 1900 | pa1046_1_1 | F |
| 1901 | pa1046_1_1 | R |
| 1902 | pa1069_1_1 | F |
| 1903 | pa1069_1_1 | R |
| 1904 | pa1846_1_1 | F |
| 1905 | pa1846_1_1 | R |
| 1906 | pa3866_1_1 | F |
| 1907 | pa3866_1_1 | R |
| 1908 | pa4082_1_1 | F |
| 1909 | pa4082_1_1 | R |
| 1910 | pilAp_1_1 | F |
| 1911 | pilAp_1_1 | R |
| 1912 | PilAp2_1_1 | F |
| 1913 | PilAp2_1_1 | R |
| 1914 | pilC_1_1 | F |
| 1915 | pilC_1_1 | R |
| 1916 | PstP_1_1 | F |
| 1917 | PstP_1_1 | R |
| 1918 | purK_1_1 | F |
| 1919 | purK_1_1 | R |
| 1920 | uvrDII_1_1 | F |
| 1921 | uvrDII_1_1 | R |
| 1922 | vsml_1_1 | F |
| 1923 | vsml_1_1 | R |
| 1924 | vsmR_1_2 | F |
| 1925 | vsmR_1_2 | R |
| 1926 | xcpX_1_1 | F |
| 1927 | xcpX_1_1 | R |
| 1928 | aprA_1_1 | F |
| 1929 | aprA_1_1 | R |
| 1930 | aprE_1_1 | F |
| 1931 | aprE_1_1 | R |
| 1932 | ctx_1_2 | F |
| 1933 | ctx_1_2 | R |
| 1934 | algB_1_1 | F |
| 1935 | algB_1_1 | R |
| 1936 | algN_1_1 | F |
| 1937 | algN_1_1 | R |
| 1938 | algR_1_1 | F |
| 1939 | algR_1_1 | R |
| 1940 | ExoS_1_1 | F |
| 1941 | ExoS_1_1 | R |
| 1942 | fpvA_1_1 | F |
| 1943 | fpvA_1_1 | R |
| 1944 | lasRa_1_1 | F |
| 1945 | lasRa_1_1 | R |
| 1946 | lipA_1_1 | F |
| 1947 | lipA_1_1 | R |
| 1948 | lipH_1_1 | F |
| 1949 | lipH_1_1 | R |
| 1950 | Orf159_1_2 | F |
| 1951 | Orf159_1_2 | R |
| 1952 | Orf252_1_1 | F |
| 1953 | Orf252_1_1 | R |
| 1954 | pchG_1_1 | F |
| 1955 | pchG_1_1 | R |
| 1956 | PhzA_1_1 | F |
| 1957 | PhzA_1_1 | R |
| 1958 | PhzB_1_1 | F |
| 1959 | PhzB_1_1 | R |
| 1960 | PLC_1_1 | F |
| 1961 | PLC_1_1 | R |
| 1962 | plcN_1_1 | F |
| 1963 | plcN_1_1 | R |
| 1964 | plcR_1_1 | F |
| 1965 | plcR_1 _1 | R |
| 1966 | pvdD_1_1 | F |
| 1967 | pvdD_1_1 | R |
| 1968 | pvdF_1_2 | F |
| 1969 | pvdF_1_2 | R |
| 1970 | pyocinS1_1_1 | F |
| 1971 | pyocinS1_1_1 | R |
| 1972 | pyocinS1im_1_1 | F |
| 1973 | pyocinS1im_1_1 | R |
| 1974 | pyocinS2_1 _1 | F |
| 1975 | pyocinS2_1 _1 | R |
| 1976 | pys2_1_1 | F |
| 1977 | pys2_1_1 | R |
| 1978 | pys2_2_1 | F |
| 1979 | pys2_2_1 | R |
| 1980 | rbf303_1_1 | F |
| 1981 | rbf303_1_1 | R |
| 1982 | rhlA_1_1 | F |
| 1983 | rhlA_1_1 | R |
| 1984 | rhlB_1_1 | F |
| 1985 | rhlB_1_1 | R |
| 1986 | rhlR_1_1 | F |
| 1987 | rhlR_1_1 | R |
| 1988 | TnAP41_1_2 | F |
| 1989 | TnAP41_1_2 | R |
| 1990 | toxA_1_1 | F |
| 1991 | toxA_1_1 | R |
| 1992 | cap1 EStrpneu_1_1 | F |
| 1993 | cap1 EStrpneu_1_1 | R |
| 1994 | cap1 FStrpneu_1_1 | F |
| 1995 | cap1 FStrpneu_1_1 | R |
| 1996 | cap1 GStrpneu_1_1 | F |
| 1997 | cap1 GStrpneu_1_1 | R |
| 1998 | cap3AStrpneu_1_1 | F |
| 1999 | cap3AStrpneu_1 _1 | R |
| 2000 | cap3BStrpneu_1_1 | F |
| 2001 | cap3BStrpneu_1_1 | R |
| 2002 | ceIAStrpneu_1_1 | F |
| 2003 | celAStrpneu_1_1 | R |
| 2004 | celBStrpneu_1_1 | F |
| 2005 | ceIBStrpneu_1_1 | R |
| 2006 | cgIAStrpneu_1_1 | F |
| 2007 | cgIAStrpneu_1_1 | R |
| 2008 | cgIBStrpneu_1_1 | F |
| 2009 | cgIBStrpneu_1_1 | R |
| 2010 | cgICStrpneu_1_1 | F |
| 2011 | cgICStrpneu_1_1 | R |
| 2012 | cgIDStrpneu_1_1 | F |
| 2013 | cgIDStrpneu_1_1 | R |
| 2014 | cinA_1_1 | F |
| 2015 | cinA_1_1 | R |
| 2016 | cps14EStrpneum_1_1 | F |
| 2017 | cps14EStrpneum_1_1 | R |
| 2018 | cps14FStrpneum_1_1 | F |
| 2019 | cps14FStrpneum_1_1 | R |
| 2020 | cps14GStrpneum_1_1 | F |
| 2021 | cps14GStrpneum_1_1 | R |
| 2022 | cps14HStrpneum_1_1 | F |
| 2023 | cps14HStrpneum_1_1 | R |
| 2024 | cps19aHStrpneum_1_1 | F |
| 2025 | cps19aHStrpneum_1_1 | R |
| 2026 | cps19alStrpneum_1_1 | F |
| 2027 | cps19aIStrpneum_1_1 | R |
| 2028 | cps19aKStrpneum_1_1 | F |
| 2029 | cps19aKStrpneum_1_1 | R |
| 2030 | cps19fGStrpneum_1_1 | F |
| 2031 | cps19fGStrpneum_1_1 | R |
| 2032 | cps23fGStrpneum_1_1 | F |
| 2033 | cps23fGStrpneum_1_1 | R |
| 2034 | dexB_1_1 | F |
| 2035 | dexB_1_1 | R |
| 2036 | dinF_1_1 | F |
| 2037 | dinF_1_1 | R |
| 2038 | 1760Strpneu_1_1 | F |
| 2039 | 1760Strpneu_1_1 | R |
| 2040 | acyPStrpneu_1_1 | F |
| 2041 | acyPStrpneu_1_1 | R |
| 2042 | endAStrpneu_1_1 | F |
| 2043 | endAStrpneu_1_1 | R |
| 2044 | exoAStrpneu_1_1 | F |
| 2045 | exoAStrpneu_1_1 | R |
| 2046 | exp72_1_1 | F |
| 2047 | exp72_1_1 | R |
| 2048 | fnlAStrpneu_1_1 | F |
| 2049 | fnlAStrpneu_1_1 | R |
| 2050 | fnlBStrpneu_1_1 | F |
| 2051 | fnlBStrpneu_1_1 | R |
| 2052 | fnlCStrpneu_1_1 | F |
| 2053 | fnlCStrpneu_1_1 | R |
| 2054 | gct18Strpneum_1_1 | F |
| 2055 | gct18Strpneum_1_1 | R |
| 2056 | hexB1_1_1 | F |
| 2057 | hexB1_1_1 | R |
| 2058 | hftsHstrpneu_1 _1 | F |
| 2059 | hftsHstrpneu_1_1 | R |
| 2060 | immunofrag1Strpneu_1_1 | F |
| 2061 | immunofrag1Strpneu_1_1 | R |
| 2062 | immunofrag2Strpneu_2_1 | F |
| 2063 | immunofrag2Strpneu_2_1 | R |
| 2064 | immunofrag3Strpneu_2_1 | F |
| 2065 | immunofrag3Strpneu_2_1 | R |
| 2066 | kdtBStrpneu_1_1 | F |
| 2067 | kdtBStrpneu_1_1 | R |
| 2068 | lysAStrpneu_1_1 | F |
| 2069 | lysAStrpneu_1_1 | R |
| 2070 | pcpBStrpneu_1_1 | F |
| 2071 | pcpBStrpneu_1 _1 | R |
| 2072 | pflCStrpneu_1_1 | F |
| 2073 | pflCStrpneu_1_1 | R |
| 2074 | plpA_1_1 | F |
| 2075 | plpA_1_1 | R |
| 2076 | prtA1 Strpneu_1_1 | F |
| 2077 | prtA1 Strpneu_1_1 | R |
| 2078 | pspC1Strpneu_1_1 | F |
| 2079 | pspC1Strpneu_1_1 | R |
| 2080 | pspC2_1_1 | F |
| 2081 | pspC2_1_1 | R |
| 2082 | purRStrpneu_1_1 | F |
| 2083 | purRStrpneu_1_1 | R |
| 2084 | pyrDAStrpneum_1_1 | F |
| 2085 | pyrDAStrpneum_1_1 | R |
| 2086 | SP0828Strpneu_1_1 | F |
| 2087 | SP0828Strpneu_1_1 | R |
| 2088 | SP0830Strpneu_1_1 | F |
| 2089 | SP0830Strpneu_1_1 | R |
| 2090 | SP0833Strpneu_1_1 | F |
| 2091 | SP0833Strpneu_1 _1 | R |
| 2092 | SP0837_38Strpneu_1_1 | F |
| 2093 | SP0837_38Strpneu_1 _1 | R |
| 2094 | SP0839Strpneu_1 _1 | F |
| 2095 | SP0839Strpneu_1 _1 | R |
| 2096 | ugdStrpneu_1_1 | F |
| 2097 | ugdStrpneu_1_1 | R |
| 2098 | uncC_1_1 | F |
| 2099 | uncC_1_1 | R |
| 2100 | vicXStrepneu_1_1 | F |
| 2101 | vicXStrepneu_1 _1 | R |
| 2102 | wchA6bStrpneum_1 _1 | F |
| 2103 | wchA6bStrpneum_1 _1 | R |
| 2104 | wci4Strpneum_1_1 | F |
| 2105 | wci4Strpneum_1_1 | R |
| 2106 | wciK4Strpneum_1_1 | F |
| 2107 | wciK4Strpneum_1_1 | R |
| 2108 | wciL4Strpneum_1_1 | F |
| 2109 | wciL4Strpneum_1_1 | R |
| 2110 | wciN6bStrpneum_1_1 | F |
| 2111 | wciN6bStrpneum_1_1 | R |
| 2112 | wciO6bStrpneum_1_1 | F |
| 2113 | wciO6bStrpneum_1_1 | R |
| 2114 | wciP6bStrpneum_1_1 | F |
| 2115 | wciP6bStrpneum_1_1 | R |
| 2116 | wciY18Strpneum_1 _1 | F |
| 2117 | wciY18Strpneum_1_1 | R |
| 2118 | wzdbStrpneum_1_1 | F |
| 2119 | wzdbStrpneum_1_1 | R |
| 2120 | wze6bStrpneum_1_1 | F |
| 2121 | wze6bStrpneum_1_1 | R |
| 2122 | wzy18Strpneum_1_1 | F |
| 2123 | wzy18Strpneum_1_1 | R |
| 2124 | wzy4Strpneum_1_1 | F |
| 2125 | wzy4Strpneum_1_1 | R |
| 2126 | wzy6bStrpneum_1_1 | F |
| 2127 | wzy6bStrpneum_1_1 | R |
| 2128 | xpt_1 _1 | F |
| 2129 | xpt_1 _1 | R |
| 2130 | igaStrpneu_1 _1 | F |
| 2131 | igaStrpneu_1 _1 | R |
| 2132 | lytA_1_1 | F |
| 2133 | lytA_1_1 | R |
| 2134 | nanA_1_1 | F |
| 2135 | nanA_1_1 | R |
| 2136 | nanBStrpneu_1_1 | F |
| 2137 | nanBStrpneu_1_1 | R |
| 2138 | pcpCStrpneu_1_1 | F |
| 2139 | pcpCStrpneu_1_1 | R |
| 2140 | ply_1_1 | F |
| 2141 | ply_1_1 | R |
| 2142 | prtAStrpneu_1_1 | F |
| 2143 | prtAStrpneu_1_1 | R |
| 2144 | pspA_1_2 | F |
| 2145 | pspA_1_2 | R |
| 2146 | SP0834Strpneu_1_1 | F |
| 2147 | SP0834Strpneu_1_1 | R |
| 2148 | SP0834Strpneu_1_2 | F |
| 2149 | SP0834Strpneu_1_2 | R |
| 2150 | sphtraStrpneu_1_1 | F |
| 2151 | sphtraStrpneu_1_1 | R |
| 2152 | wciJStrpneu_1_1 | F |
| 2153 | wciJStrpneu_1_1 | R |
| 2154 | wziyStrpneu_1_1 | F |
| 2155 | wziyStrpneu_1_1 | R |
| 2156 | wzxStrpneu_1_1 | F |
| 2157 | wzxStrpneu_1_1 | R |
| 2158 | cpsA1Strgal_1_1 | F |
| 2159 | cpsA1Strgal_1_1 | R |
| 2160 | cpsB1Strgal_1_1 | F |
| 2161 | cpsB1Strgal_1_1 | R |
| 2162 | cpsC1Strgal_1_1 | F |
| 2163 | cpsC1Strgal_1_1 | R |
| 2164 | cpsD1Strgal_1_1 | F |
| 2165 | cpsD1Strgal_1_1 | R |
| 2166 | cpsE1Strgal_1_1 | F |
| 2167 | cpsE1Strgal_1_1 | R |
| 2168 | cpsG1Strgal_1_1 | F |
| 2169 | cpsG1Strgal_1_1 | R |
| 2170 | cpslStragal_1_1 | F |
| 2171 | cpslStragal_1_1 | R |
| 2172 | cpsJStragal_1_1 | F |
| 2173 | cpsJStragal_1_1 | R |
| 2174 | cpsKStragal_1_1 | F |
| 2175 | cpsKStragal_1_1 | R |
| 2176 | cpsMStragal_1_1 | F |
| 2177 | cpsMStragal_1_1 | R |
| 2178 | cpsYStragal_1_1 | F |
| 2179 | cpsYStragal_1 _1 | R |
| 2180 | cpsYStragal_2_1 | F |
| 2181 | cpsYStragal_2_1 | R |
| 2182 | cylBStraga_1_1 | F |
| 2183 | cylBStraga_1_1 | R |
| 2184 | cylEStraga_1_1 | F |
| 2185 | cylEStraga_1_1 | R |
| 2186 | cylFStraga_1_1 | F |
| 2187 | cylFStraga_1_1 | R |
| 2188 | cylHStraga_1_1 | F |
| 2189 | cylHStraga_1_1 | R |
| 2190 | cylIStraga_1_1 | F |
| 2191 | cylIStraga_1_1 | R |
| 2192 | cylJStraga_1_1 | F |
| 2193 | cyIJStraga_1_1 | R |
| 2194 | cylKStraga_1_1 | F |
| 2195 | cylKStraga_1_1 | R |
| 2196 | 0487Straga_1_1 | F |
| 2197 | 0487Straga_1_1 | R |
| 2198 | 0488Straga_1_1 | F |
| 2199 | 0488Straga_1_1 | R |
| 2200 | 0493Straga_1_1 | F |
| 2201 | 0493Straga_1_1 | R |
| 2202 | 0495Straga_1_1 | F |
| 2203 | 0495Straga_1_1 | R |
| 2204 | 0498Straga_1_1 | F |
| 2205 | 0498Straga_1_1 | R |
| 2206 | 0500Straga_1_1 | F |
| 2207 | 0500Straga_1_1 | R |
| 2208 | 0502Straga_1_1 | F |
| 2209 | 0502Straga_1_1 | R |
| 2210 | 0504Straga_1_1 | F |
| 2211 | 0504Straga_1_1 | R |
| 2212 | foIDStraga_1_1 | F |
| 2213 | foIDStraga_1_1 | R |
| 2214 | neuA1Strgal_1_1 | F |
| 2215 | neuA1Strgal_1_1 | R |
| 2216 | neuB1Strgal_1_1 | F |
| 2217 | neuB1Strgal_1_1 | R |
| 2218 | neuC1Strgal_1_1 | F |
| 2219 | neuC1Strgal_1_1 | R |
| 2220 | neuD1Strgal_1 _1 | F |
| 2221 | neuD1Strgal_1_1 | R |
| 2222 | recNStraga_1_1 | F |
| 2223 | recNStraga_1_1 | R |
| 2224 | ileSStraga_1_1 | F |
| 2225 | ileSStraga_1_1 | R |
| 2226 | CAMPfactor_1_1 | F |
| 2227 | CAMPfactor_1_1 | R |
| 2228 | CAMPfactor_2_1 | F |
| 2229 | CAMPfactor_2_1 | R |
| 2230 | 0499Straga_1_1 | F |
| 2231 | 0499Straga_1_1 | R |
| 2232 | hylStragal_1_1 | F |
| 2233 | hylStragal_1_1 | R |
| 2234 | lipStragal_1_1 | F |
| 2235 | lipStragal_1_1 | R |
| 2236 | cyclStrpyog_1_1 | F |
| 2237 | cyclStrpyog_1_1 | R |
| 2238 | fah_rph_hlo_Strpyog_1_1 | F |
| 2239 | fah_rph_hlo_Strpyog_1_1 | R |
| 2240 | int_1_1 | F |
| 2241 | int_1_1 | R |
| 2242 | int315.5_1_1 | F |
| 2243 | int315.5_1_1 | R |
| 2244 | murEStrpyog_1_1 | F |
| 2245 | murEStrpyog_1_1 | R |
| 2246 | oppA_1_1 | F |
| 2247 | oppA_1_1 | R |
| 2248 | oppCStrpyog_1_1 | F |
| 2249 | oppCStrpyog_1_1 | R |
| 2250 | oppD_1_1 | F |
| 2251 | oppD_1_1 | R |
| 2252 | SPy0382Strpyog_1_1 | F |
| 2253 | SPy0382Strpyog_1_1 | R |
| 2254 | SPy0390Strpyog_1_1 | F |
| 2255 | SPy0390Strpyog_1_1 | R |
| 2256 | SpyM3_1351_1_1 | F |
| 2257 | SpyM3_1351_1_1 | R |
| 2258 | vicXStrpyog_1_1 | F |
| 2259 | vicXStrpyog_1 _1 | R |
| 2260 | DNaseIStrpyog_1_1 | F |
| 2261 | DNaseIStrpyog_1_1 | R |
| 2262 | fba2Strpyog_1_1 | F |
| 2263 | fba2Strpyog_1 _1 | R |
| 2264 | fhuAStrpyog_1 _1 | F |
| 2265 | fhuAStrpyog_1 _1 | R |
| 2266 | fhuB1Strpyog_1_1 | F |
| 2267 | fhuB1Strpyog_1_1 | R |
| 2268 | fhuDStrpyog_1_1 | F |
| 2269 | fhuDStrpyog_1_1 | R |
| 2270 | fhuGStrpyog_1_1 | F |
| 2271 | fhuGStrpyog_1_1 | R |
| 2272 | hylA_1_1 | F |
| 2273 | hylA_1_1 | R |
| 2274 | hylP_1_1 | F |
| 2275 | hylP_1_1 | R |
| 2276 | hylp2_1_1 | F |
| 2277 | hylp2_1_1 | R |
| 2278 | oppB_1_1 | F |
| 2279 | oppB_1_1 | R |
| 2280 | ropB_1_1 | F |
| 2281 | ropB_1_1 | R |
| 2282 | scpAStrpyog_1_1 | F |
| 2283 | scpAStrpyog_1_1 | R |
| 2284 | sloStrpyog_1_1 | F |
| 2285 | sloStrpyog_1_1 | R |
| 2286 | smez-4Strpyog_1_1 | F |
| 2287 | smez-4Strpyog_1_1 | R |
| 2288 | sof_1_1 | F |
| 2289 | sof_1_1 | R |
| 2290 | sof_2_1 | F |
| 2291 | sof_2_1 | R |
| 2292 | speA_1_1 | F |
| 2293 | speA_1_1 | R |
| 2294 | speB2Strpyog_1_1 | F |
| 2295 | speB2Strpyog_1_1 | R |
| 2296 | speCStrpyog_1_1 | F |
| 2297 | speCStrpyog_1_1 | R |
| 2298 | speJStrpyog_1_1 | F |
| 2299 | speJStrpyog_1_1 | R |
| 2300 | srtBStrpyog_1_1 | F |
| 2301 | srtBStrpyog_1_1 | R |
| 2302 | srtCStrpyog_1_1 | F |
| 2303 | srtCStrpyog_1_1 | R |
| 2304 | srtEStrpyog_1_1 | F |
| 2305 | srtEStrpyog_1_1 | R |
| 2306 | srtFStrpyog_1_1 | F |
| 2307 | srtFStrpyog_1_1 | R |
| 2308 | srtGStrpyog_1_1 | F |
| 2309 | srtGStrpyog_1_1 | R |
| 2310 | srtlStrpyog_1_1 | F |
| 2311 | srtIStrpyog_1_1 | R |
| 2312 | srtKStrpyog_1_1 | F |
| 2313 | srtKStrpyog_1_1 | R |
| 2314 | srtRStrpyog_1_1 | F |
| 2315 | srtRStrpyog_1_1 | R |
| 2316 | srtTStrpyog_1_1 | F |
| 2317 | srtTStrpyog_1_1 | R |
| 2318 | vicKStrpyog_1_1 | F |
| 2319 | vicKStrpyog_1_1 | R |
| 2320 | 573Stprmut_1_1 | F |
| 2321 | 573Stprmut_1_1 | R |
| 2322 | 580SStprmut_1_1 | F |
| 2323 | 580SStprmut_1_1 | R |
| 2324 | 581_582SStprmut_1_1 | F |
| 2325 | 581_582SStprmut_1_1 | R |
| 2326 | 584SStprmut_1_1 | F |
| 2327 | 584SStprmut_1_1 | R |
| 2328 | dltAStrmut_1_1 | F |
| 2329 | dltAStrmut_1_1 | R |
| 2330 | dltBStrmut_1_1 | F |
| 2331 | dltBStrmut_1_1 | R |
| 2332 | dltCppx1Strmut_1_1 | F |
| 2333 | dltCppx1Strmut_1_1 | R |
| 2334 | dltDStrmut_1_1 | F |
| 2335 | dltDStrmut_1_1 | R |
| 2336 | lichStrbov_1_1 | F |
| 2337 | lichStrbov_1_1 | R |
| 2338 | lytRStprmut_1_1 | F |
| 2339 | lytRStprmut_1_1 | R |
| 2340 | lytSStprmut_1_1 | F |
| 2341 | lytSStprmut_1_1 | R |
| 2342 | pepQStrrmut_1_1 | F |
| 2343 | pepQStrrmut_1_1 | R |
| 2344 | pflCStrmut_1_1 | F |
| 2345 | pflCStrmut_1_1 | R |
| 2346 | recNStprmut_1_1 | F |
| 2347 | recNStprmut_1_1 | R |
| 2348 | ytqBStrm ut_1 _1 | F |
| 2349 | ytqBStrmut_1_1 | R |
| 2350 | hlyXStrmut_1_1 | F |
| 2351 | hlyXStrmut_1_1 | R |
| 2352 | igaStrmitis_1_1 | F |
| 2353 | igaStrmitis_1_1 | R |
| 2354 | igaStrsanguis_1_1 | F |
| 2355 | igaStrsanguis_1_1 | R |
| 2356 | perMStrmut_1_1 | F |
| 2357 | perMStrmut_1_1 | R |
| 2358 | atfA_1_1 | F |
| 2359 | atfA_1_1 | R |
| 2360 | atfB_1_1 | F |
| 2361 | atfB_1_1 | R |
| 2362 | atfC_1_1 | F |
| 2363 | atfC_1_1 | R |
| 2364 | ccmPrmi1_1_1 | F |
| 2365 | ccmPrmi1_1_1 | R |
| 2366 | cyaPrmi_1_1 | F |
| 2367 | cyaPrmi_1_1 | R |
| 2368 | aad_1_1 | F |
| 2369 | aad_1_1 | R |
| 2370 | flfB_1_1 | F |
| 2371 | flfB_1_1 | R |
| 2372 | flfD_1_1 | F |
| 2373 | flfD_1_1 | R |
| 2374 | flfN_1_1 | F |
| 2375 | flfN_1_1 | R |
| 2376 | flhD_1_1 | F |
| 2377 | flhD_1_1 | R |
| 2378 | floA_1_1 | F |
| 2379 | floA_1_1 | R |
| 2380 | ftsK_1_1 | F |
| 2381 | ftsK_1_1 | R |
| 2382 | gstB_1_1 | F |
| 2383 | gstB_1_1 | R |
| 2384 | hemCPrmi_1_1 | F |
| 2385 | hemCPrmi_1_1 | R |
| 2386 | hemDPrmi_1_1 | F |
| 2387 | hemDPrmi_1_1 | R |
| 2388 | hev_1_1 | F |
| 2389 | hev_1_1 | R |
| 2390 | katA_1_1 | F |
| 2391 | katA_1_1 | R |
| 2392 | lpp1_1_1 | F |
| 2393 | Ipp1_1_1 | R |
| 2394 | menE_1_1 | F |
| 2395 | menE_1_1 | R |
| 2396 | mfd_1_1 | F |
| 2397 | mfd_1_1 | R |
| 2398 | nrpA_1_1 | F |
| 2399 | nrpA_1_1 | R |
| 2400 | nrpB_1_1 | F |
| 2401 | nrpB_1_1 | R |
| 2402 | nrpG_1_1 | F |
| 2403 | nrpG_1_1 | R |
| 2404 | nrpS_1_1 | F |
| 2405 | nrpS_1_1 | R |
| 2406 | nrpT_1_1 | F |
| 2407 | nrpT_1_1 | R |
| 2408 | nrpU_1_1 | F |
| 2409 | nrpU_1_1 | R |
| 2410 | pat_1_1 | F |
| 2411 | pat_1_1 | R |
| 2412 | pmfA_1_1 | F |
| 2413 | pmfA_1_1 | R |
| 2414 | pmfC_1_1 | F |
| 2415 | pmfC_1_1 | R |
| 2416 | pmfE_1_1 | F |
| 2417 | pmfE_1_1 | R |
| 2418 | ppaA_1_1 | F |
| 2419 | ppaA_1_1 | R |
| 2420 | rsbA_1_1 | F |
| 2421 | rsbA_1_1 | R |
| 2422 | rsbC_1_1 | F |
| 2423 | rsbC_1_1 | R |
| 2424 | speB_1_1 | F |
| 2425 | speB_1_1 | R |
| 2426 | stmA_1_1 | F |
| 2427 | stmA_1_1 | R |
| 2428 | stmB_1_1 | F |
| 2429 | stmB_1_1 | R |
| 2430 | terA_1_1 | F |
| 2431 | terA_1_1 | R |
| 2432 | terD_1_1 | F |
| 2433 | terD_1_1 | R |
| 2434 | umoA_1_1 | F |
| 2435 | umoA_1_1 | R |
| 2436 | umoB_1_1 | F |
| 2437 | umoB_1_1 | R |
| 2438 | umoC_1_1 | F |
| 2439 | umoC_1_1 | R |
| 2440 | ureR_1_1 | F |
| 2441 | ureR_1_1 | R |
| 2442 | xerC_1_1 | F |
| 2443 | xerC_1_1 | R |
| 2444 | ygbA_1_1 | F |
| 2445 | ygbA_1_1 | R |
| 2446 | flaA_1_1 | F |
| 2447 | flaA_1_1 | R |
| 2448 | flaD_1_1 | F |
| 2449 | flaD_1_1 | R |
| 2450 | fliA_1_1 | F |
| 2451 | fliA_1_1 | R |
| 2452 | hpmA_1_1 | F |
| 2453 | hpmA_1_1 | R |
| 2454 | hpmB_1_1 | F |
| 2455 | hpmB_1_1 | R |
| 2456 | IpsPrmi_1_1 | F |
| 2457 | IpsPrmi_1_1 | R |
| 2458 | mrpA_1_1 | F |
| 2459 | mrpA_1_1 | R |
| 2460 | mrpB_1_1 | F |
| 2461 | mrpB_1_1 | R |
| 2462 | mrpC_1_1 | F |
| 2463 | mrpC_1_1 | R |
| 2464 | mrpD_1_1 | F |
| 2465 | mrpD_1_1 | R |
| 2466 | mrpE_1_1 | F |
| 2467 | mrpE_1_1 | R |
| 2468 | mrpF_1_1 | F |
| 2469 | mrpF_1_1 | R |
| 2470 | mrpG_1_1 | F |
| 2471 | mrpG_1_1 | R |
| 2472 | mrpH_1_1 | F |
| 2473 | mrpH_1_1 | R |
| 2474 | mrpl_1_1 | F |
| 2475 | mrpl_1_1 | R |
| 2476 | mrpJ_1_1 | F |
| 2477 | mrpJ_1_1 | R |
| 2478 | patA_1_1 | F |
| 2479 | patA_1_1 | R |
| 2480 | putA_1_1 | F |
| 2481 | putA_1_1 | R |
| 2482 | uca_1_1 | F |
| 2483 | uca_1_1 | R |
| 2484 | ureDPrmi_1_1 | F |
| 2485 | ureDPrmi_1_1 | R |
| 2486 | ureEPrmi_1_1 | F |
| 2487 | ureEPrmi_1_1 | R |
| 2488 | ureFPrmi_1_1 | F |
| 2489 | ureFPrmi_1_1 | R |
| 2490 | zapA_1_1 | F |
| 2491 | zapA_1_1 | R |
| 2492 | zapB_1_1 | F |
| 2493 | zapB_1_1 | R |
| 2494 | zapD_1_1 | F |
| 2495 | zapD_1_1 | R |
| 2496 | zapE_1_1 | F |
| 2497 | zapE_1_1 | R |
| 2498 | envZPrvu_1_1 | F |
| 2499 | envZPrvu_1_1 | R |
| 2500 | frdC_1_1 | F |
| 2501 | frdC_1_1 | R |
| 2502 | frdD_1_1 | F |
| 2503 | frdD_1_1 | R |
| 2504 | infBPrvu_1_1 | F |
| 2505 | infBPrvu_1_1 | R |
| 2506 | lad_1_1 | F |
| 2507 | lad_1_1 | R |
| 2508 | tna2_1_1 | F |
| 2509 | tna2_1_1 | R |
| 2510 | end_1_1 | F |
| 2511 | end_1_1 | R |
| 2512 | pqrA_1_1 | F |
| 2513 | pqrA_1_1 | R |
| 2514 | urg_1_1 | F |
| 2515 | urg_1_1 | R |
| 2516 | blalMP-7_1_1 | F |
| 2517 | blalMP-7_1_1 | R |
| 2518 | meclSepid_1_1 | F |
| 2519 | meclSepid_1_1 | R |
| 2520 | blaOXA-10_1_2 | F |
| 2521 | blaOXA-10_1_2 | R |
| 2522 | blaB_1_1 | F |
| 2523 | blaB_1_1 | R |
| 2524 | ampC_1_1 | F |
| 2525 | ampC_1_1 | R |
| 2526 | I-blaR_1_1 | F |
| 2527 | I-blaR_1_1 | R |
| 2528 | blaOXA- 32_1_1 | F |
| 2529 | blaOXA- 32_1_1 | R |
| 2530 | bla-CTX-M-22_1 _1 | F |
| 2531 | bla-CTX-M-22_1_1 | R |
| 2532 | pbp2aStrpneu_1_1 | F |
| 2533 | pbp2aStrpneu_1_1 | R |
| 2534 | blaSHV-1_1_1 | F |
| 2535 | blaSHV-1_1_1 | R |
| 2536 | blaOXA- 2_1_1 | F |
| 2537 | blaOXA-2_1_12_1_1 | R |
| 2538 | blaRShaemolyt_1_1 | F |
| 2539 | blaRShaemolyt_1_1 | R |
| 2540 | blalMP-7_1_2 | F |
| 2541 | blaIMP-7_1_2 | R |
| 2542 | I-mecR_1_1 | F |
| 2543 | I-mecR_1_1 | R |
| 2544 | blaOXY_1_1 | F |
| 2545 | blaOXY_1_1 | R |
| 2546 | dacCStrpyog_1_1 | F |
| 2547 | dacCStrpyog_1_1 | R |
| 2548 | femA_1_1 | F |
| 2549 | femA_1_1 | R |
| 2550 | mecA_1_1 | F |
| 2551 | mecA_1_1 | R |
| 2552 | blaIShaemolyt_1_1 | F |
| 2553 | blaIShaemolyt_1_1 | R |
| 2554 | blavim_1_1 | F |
| 2555 | blavim_1_1 | R |
| 2556 | pbp2b_1_1 | F |
| 2557 | pbp2b_1_1 | R |
| 2558 | pbp2primeSepid_1_1 | F |
| 2559 | pbp2primeSepid_1_1 | R |
| 2560 | pbp2x_1_1 | F |
| 2561 | pbp2x_1_1 | R |
| 2562 | pbp3Saureuc_1_1 | F |
| 2563 | pbp3Saureuc_1_1 | R |
| 2564 | pbp4_1_1 | F |
| 2565 | pbp4_1_1 | R |
| 2566 | pbp5Efaecium_1_1 | F |
| 2567 | pbp5Efaecium_1_1 | R |
| 2568 | pbpC_1_1 | F |
| 2569 | pbpC_1_1 | R |
| 2570 | I-mecl_1_1 | F |
| 2571 | I-mecl_1_1 | R |
| 2572 | pbp1a_1_1 | F |
| 2573 | pbp1a_1_1 | R |
| 2574 | I-blal_1_1 | F |
| 2575 | I-blal_1_1 | R |
| 2576 | blaTEM-106_1_1 | F |
| 2577 | blaTEM-106_1_1 | R |
| 2578 | blaOXY-KLOX_1_1 | F |
| 2579 | blaOXY-KLOX_1_1 | R |
| 2580 | ftsWEF_1_1 | F |
| 2581 | ftsWEF_1_1 | R |
| 2582 | fmhB_1_1 | F |
| 2583 | fmhB_1_1 | R |
| 2584 | cumA_1_1 | F |
| 2585 | cumA_1_1 | R |
| 2586 | femBShaemolyt_1_1 | F |
| 2587 | femBShaemolyt_1_1 | R |
| 2588 | blaPER-1_1_1 | F |
| 2589 | blaPERl-1_1_1 | R |
| 2590 | bla_FOX-3_1_1 | F |
| 2591 | bla_FOX-3_1_1 | R |
| 2592 | blaA_1_1 | F |
| 2593 | blaA_1_1 | R |
| 2594 | psrb_1_1 | F |
| 2595 | Psrb_1_1 | R |
| 2596 | fmhA_1_1 | F |
| 2597 | fmhA_1_1 | R |
| 2598 | mecR1Sepid_1_1 | F |
| 2599 | mecR1Sepid_1_1 | R |
| 2600 | blaZ_1_1 | F |
| 2601 | blaZ_1_1 | R |
| 2602 | blaOXA-1_1_1 | F |
| 2603 | blaOXA-1_1_1 | R |
| 2604 | fox-6_1_1 | F |
| 2605 | fox-6_1_1 | R |
| 2606 | blaPrmi_1_1 | F |
| 2607 | blaPrmi_1_1 | R |
| 2608 | aacA_aph DStwar_1 _1 | F |
| 2609 | aacA_aphDStwar_1_1 | R |
| 2610 | aacC1_1_2 | F |
| 2611 | aacC1_1_2 | R |
| 2612 | aacC2_1_1 | F |
| 2613 | aacC2_1_1 | R |
| 2614 | strB_1_1 | F |
| 2615 | strB_1_1 | R |
| 2616 | aadA_1_1 | F |
| 2617 | aadA_1_1 | R |
| 2618 | aadB_1_2 | F |
| 2619 | aadB_1_2 | R |
| 2620 | aadD_1_1 | F |
| 2621 | aadD_1_1 | R |
| 2622 | aacA4_1_2 | F |
| 2623 | aacA4_1_2 | R |
| 2624 | strA_1_1 | F |
| 2625 | strA_1_1 | R |
| 2626 | aph-A3_1_1 | F |
| 2627 | aph-A3_1_1 | R |
| 2628 | aacC1_1_1 | F |
| 2629 | aacC1_1_1 | R |
| 2630 | aacA4_1_1 | F |
| 2631 | aacA4_1_1 | R |
| 2632 | aacA-aphD_1_1 | F |
| 2633 | aacA-aphD_1_1 | R |
| 2634 | I-spc_1_1 | F |
| 2635 | I-spc_1_1 | R |
| 2636 | aphA3_1_1 | F |
| 2637 | aphA3_1_1 | R |
| 2638 | ermC_1_1 | F |
| 2639 | ermC_1_1 | R |
| 2640 | linB_1_1 | F |
| 2641 | linB_1_1 | R |
| 2642 | satSA_1_1 | F |
| 2643 | satSA_1_1 | R |
| 2644 | mdrSA_1_1 | F |
| 2645 | mdrSA_1_1 | R |
| 2646 | l-linA_1_1 | F |
| 2647 | l-linA_1_1 | R |
| 2648 | ermB_1_2 | F |
| 2649 | ermB_1_2 | R |
| 2650 | ermA_1_1 | F |
| 2651 | ermA_1_1 | R |
| 2652 | satA_1_1 | F |
| 2653 | satA_1_1 | R |
| 2654 | msrA_1_1 | F |
| 2655 | msrA_1_1 | R |
| 2656 | mphBM_1_1 | F |
| 2657 | mphBM_1_1 | R |
| 2658 | mefA_1_1 | F |
| 2659 | mefA_1_1 | R |
| 2660 | mrx_1_1 | F |
| 2661 | mrx_1_1 | R |
| 2662 | dfrStrpneu_1_1 | F |
| 2663 | dfrStrpneu_1_1 | R |
| 2664 | dfrA_1_1 | F |
| 2665 | dfrA_1_1 | R |
| 2666 | cmlA5_1_1 | F |
| 2667 | cmIA5_1_1 | R |
| 2668 | catEfaecium_1_1 | F |
| 2669 | catEfaecium_1_1 | R |
| 2670 | cat_1_1 | F |
| 2671 | cat_1_1 | R |
| 2672 | tetAJ_1_1 | F |
| 2673 | tetAJ_1_1 | R |
| 2674 | tetL_1_1 | F |
| 2675 | tetL_1_1 | R |
| 2676 | tetM_1_1 | F |
| 2677 | tetM_1_1 | R |
| 2678 | vanH(tn)_1 _1 | F |
| 2679 | vanH(tn)_1 _1 | R |
| 2680 | vanA_1_1 | F |
| 2681 | vanA_1_1 | R |
| 2682 | vanHB2_1_1 | F |
| 2683 | vanHB2_1_1 | R |
| 2684 | vanR_1_1 | F |
| 2685 | vanR_1_1 | R |
| 2686 | vanRB2_1_1 | F |
| 2687 | vanRB2_1_1 | R |
| 2688 | vanS(tn)_1_1 | F |
| 2689 | vanS(tn)_1_1 | R |
| 2690 | vanSB2_1_1 | F |
| 2691 | vanSB2_1_1 | R |
| 2692 | vanWB2_1_1 | F |
| 2693 | vanWB2_1_1 | R |
| 2694 | ddl_1_1 | F |
| 2695 | ddl_1_1 | R |
| 2696 | ble_1_1 | F |
| 2697 | ble_1_1 | R |
| 2698 | vanXB2_1_1 | F |
| 2699 | vanXB2_1_1 | R |
| 2700 | vanY(tn)_1_1 | F |
| 2701 | vanY(tn)_1_1 | R |
| 2702 | vanYB2_1_1 | F |
| 2703 | vanYB2_1_1 | R |
| 2704 | vanB_1_1 | F |
| 2705 | vanB_1_1 | R |
| 2706 | vanZ(tn)_1_1 | F |
| 2707 | vanZ(tn)_1_1 | R |
| 2708 | vanC-2_1_1 | F |
| 2709 | vanC-2_1_1 | R |
| 2710 | vanX(tn)_1_1 | F |
| 2711 | vanX(tn)_1_1 | R |
| 2712 | acrB_1_1 | F |
| 2713 | acrB_1_1 | R |
| 2714 | mexB_1_2 | F |
| 2715 | mexB_1_2 | R |
| 2716 | I-qacA_1 _1 | F |
| 2717 | I-qacA_1_1 | R |
| 2718 | sull_1_1 | F |
| 2719 | sull_1_1 | R |
| 2720 | sul_1_1 | F |
| 2721 | sul_1_1 | R |
| 2722 | cadBStalugd_1_1 | F |
| 2723 | cadBStalugd_1_1 | R |
| 2724 | mexA_1_1 | F |
| 2725 | mexA_1_1 | R |
| 2726 | acrR_1_1 | F |
| 2727 | acrR_1_1 | R |
| 2728 | emeA_1_1 | F |
| 2729 | emeA_1_1 | R |
| 2730 | acrA_1_1 | F |
| 2731 | acrA_1_1 | R |
| 2732 | rtn_1_1 | F |
| 2733 | rtn_1_1 | R |
| 2734 | abcXStrpmut_1_1 | F |
| 2735 | abcXStrpmut_1_1 | R |
| 2736 | qacEdelta1_1_1 | F |
| 2737 | qacEdelta1_1_1 | R |
| 2738 | elkT-abcA_1_1 | F |
| 2739 | elkT-abcA_1_1 | R |
| 2740 | I-cadA_1_1 | F |
| 2741 | I-cadA_1_1 | R |
| 2742 | albA_1_1 | F |
| 2743 | albA_1_1 | R |
| 2744 | wzm_1_1 | F |
| 2745 | wzm_1_1 | R |
| 2746 | msrCb_1_1 | F |
| 2747 | msrCb_1_1 | R |
| 2748 | nov_1_1 | F |
| 2749 | nov_1_1 | R |
| 2750 | wzt_1_1 | F |
| 2751 | wzt_1_1 | R |
| 2752 | wbbI_1_1 | F |
| 2753 | wbbI_1_1 | R |
| 2754 | norA23_1_1 | F |
| 2755 | norA23_1_1 | R |
| 2756 | mexR_1_1 | F |
| 2757 | mexR_1_1 | R |
| 2758 | arr2_1_1 | F |
| 2759 | arr2_1_1 | R |
| 2760 | mreA_1_1 | F |
| 2761 | mreA_1_1 | R |
| 2762 | I-cadC_1_1 | F |
| 2763 | I-cadC_1_1 | R |
| 2764 | uvrA_1_1 | F |
| 2765 | uvrA_1_1 | R |
| 2766 | CRD2_1_1 | F |
| 2767 | CRD2_1_1 | R |
| 2768 | CDR1_1_1 | F |
| 2769 | CDR1_1_1 | R |
| 2770 | CDR1_2_1 | F |
| 2771 | CDR1_2_1 | R |
| 2772 | MET3_1_1 | F |
| 2773 | MET3_1_1 | R |
| 2774 | FET3_1_1 | F |
| 2775 | FET3_1_1 | R |
| 2776 | FTR2_1_1 | F |
| 2777 | FTR2_1_1 | R |
| 2778 | MDR1-7_1_1 | F |
| 2779 | MDR1-7_1_1 | R |
| 2780 | ERG11_1_1 | F |
| 2781 | ERG11_1_1 | R |
| 2782 | SEC20_1_1 | F |
| 2783 | SEC20_1_1 | R |
| 2784 | rbcL_1_1 | F |
| 2785 | rbcL_1_1 | R |
| 2786 | LDHA(hu)_1_1 | F |
| 2787 | LDHA(hu)_1_1 | R |
| 2788 | GAPD(hu)_1_1 | F |
| 2789 | GAPD(hu)_1_1 | R |
| 2790 | b-Act(hu)_1_1 | F |
| 2791 | b-Act(hu)_1_1 | R |
| 2792 | ARHGDIA(hu)_1_1 | F |
| 2793 | ARHGDIA(hu)_1_1 | R |
| 2794 | PGK1(hu)_1_1 | F |
| 2795 | PGK1(hu)_1_1 | R |
| 2796 | rbcL_1_2 | F |
| 2797 | rbcL_1_2 | R |
| 2798 | 16SPa_1_1 | F |
| 2799 | 16SPa_1_1 | R |
| 2800 | 23SEfaecium_2_1 | F |
| 2801 | 23SEfaecium_2_1 | R |
| 2802 | 16SStrepyog_1_1 | F |
| 2803 | 16SStrepyog_1_1 | R |
| 2804 | 16SStrepneu_1_1 | F |
| 2805 | 16SStrepneu_1_1 | R |
| 2806 | 16SStrepagalactiae_1_1 | F |
| 2807 | 16SStrepagalactiae_1_1 | R |
| 2808 | 16SEfaecium_1_1 | F |
| 2809 | 16SEfaecium_1_1 | R |
| 2810 | 16SEfaecium_2_1 | F |
| 2811 | 16SEfaecium_2_1 | R |
| 2812 | 16SRNAEf_2_1 | F |
| 2813 | 16SRNAEf_2_1 | R |
| 2814 | 16SKpn_1_1 | F |
| 2815 | 16SKpn_1_1 | R |
| 2816 | 16SSa_3_1 | F |
| 2817 | 16SSa_3_1 | R |
| 2818 | 16SRNAEf_1_1 | F |
| 2819 | 16SRNAEf_1_1 | R |
| 2820 | 16SShominis_1_1 | F |
| 2821 | 16SShominis_1_1 | R |
| 2822 | 16SShaemolyt_1_1 | F |
| 2823 | 16SShaemolyt_1_1 | R |
| 2824 | 23SEfaecium_1_1 | F |
| 2825 | 23SEfaecium_1_1 | R |
| 2826 | 16SrRNAPrmi_1_1 | F |
| 2827 | 16SrRNAPrmi_1_1 | R |
| 2828 | 16SrRNAPrvu1_1_1 | F |
| 2829 | 16SrRNAPrvu1_1_1 | R |
| 2830 | 16SSa_1_1 | F |
| 2831 | 16SSa_1_1 | R |
| 2832 | 16SKlox_1_1 | F |
| 2833 | 16SKlox_1_1 | R |
| 2834 | p53_1_1 | F |
| 2835 | p53_1_1 | R |
| 2836 | 0135mihck_1_1 | F |
| 2837 | 0135mihck_1_1 | R |
| 2838 | FAN_1_1 | F |
| 2839 | FAN_1_1 | R |
| 2840 | 0270cap_1_1 | F |
| 2841 | 0270cap_1_1 | R |

## Claims

1. A DNA microarray for direct identification and characterisation of microorganisms in a sample or clinical specimen, wherein the microarray comprises gene probes being derived from DNA sequences or partial DNA sequences of the microorganisms to be identified or DNA sequences complementary or homologous thereto, and having a length of at least 100 nucletides (nt).

2. The DNA microarray of claim 1, wherein
(i) the length of the gene probes is from 100 to 1000 nt, preferably from 200 to 800 nt; and/or
(ii) the gene probes are specific for a specific microbial species or group of microorganisms to be identified and preferably are DNA sequences selected from the groups consisting of (a) species specific gene probes, (b) virulence gene probes and (c) resistance gene probes; and/or
(iii) the microorganisms to be detected are microorganisms which cause bacteremia, fungemia or sepsis and include bacteria and fungi, preferably the microorganisms are selected from the group consisting of *Candida albicans, Enterococcus faecalis, Enterococcus faecium, Escherichia coli, Klebsiella oxytoca, Klebsiella pneum oniae, Proteus mirabilis, Proteus vulgaris, Enterobacter cloacae, Pseudomonas aeruginosa, Stenotrophomonas maltophilia, Acinetobacter baumannii, Staphylococcus aureus, Staphylococcus epidermidis, Staphylococcus haemolyticus, Staphylococcus lugdunensis, Staphylococcus warneri, Streptococcus agalactiae, Streptococcus bovis, Streptococcus dysgalactiae, Streptococcus mitis, Streptococcus mutans, Streptococcus pneumoniae, Streptococcus pyogenes,* most preferably are *S. aureus, E. coli* and/or *P. aeruginosa;* and/or
(iv) the sample is selected from whole blood, serum, urine, saliva, liquor, sputum, punktate, stool, pus, wound fluid, positive blood cultures, preferably is positive blood cultures; and/or
(v) the array further comprises DNA sequences selected from the group (d) consisting of contrtol gene probes coding for negative controls and positive controls.

3. The DNA microarray of claim 2, which is suitable for identification of bacteremia, fungemia or sepsis and wherein the set of gene probes preferably comprises gene probes selected from
(a) species specific gene probes for
(i) *Staphylococcus aureus* including gene probes derived from *cataSaur, clfA, clfB, coa, I-clpC, I-clpP, I-ctaA, I-ctsR, I-dltA, I-dltB, I-dltC, I-dnaK, I-elkT, I-femD, I-glnA, I-glnR, I-grlA, I-grlB, I-groEL, I-groES, I-hemA, I-hemE, I-hemH, I-hemL, I-hemY, I-lepA, I-lrgA, I-lrgB, I-lytM, I-menB, I-menD, I-menE, I-menF, I-mreB, I-mreR, I-mutL, I-mutS, I-NAG, I-pbg, I-pbpF, I-pdhB, I-pdhC, I-rsbU, I-rsbV, I-rsbW, I-sgp, I-sirR, I-sodA, I-sodB, I-sstA, I-sstB, I-sstC, I-sstD, I-trx, I-yhiN, epiP-bsaP, geh, gyrA, gyrB, hemB, hemC, hemD, hemN, hsdS, hsdS, lip, menC, nuc, pdhD, rpoB, SAV0431, SAV0439, SAV0440, SAV0441, sigB, spa, sstC, tag, tyrA, I-aroC, I-aroA, I-cna, I-ebpS, I-eno, I-fbpA, I-fib, I-fnbB, I-srtA, I-stpC, I-fnbA, I-spa, I-aroE, I-aroF, I-aroG, I-asp23, I-atl*;
(ii) *Escherichia coli* including gene probes derived from *b1169, envZ, fliCb, nfrB, nlpA, pilAe, yacH, yagX, ycdS, yciQ, ymcA;*
(iii) *Staphylococcus epidermidis* including gene probes derived from *ardeSE0106, ardeSE0107, aroiSE0105, atlE, agrB, agrC, alphSE1368, gad, glucSE1191, hsp10, icaA, icaB, mvaSSepid, nitreSE1972, nitreSE1974, nitreSE1975, oiamtSE1209, ORFISepid, ORF3bSepid, qacR, sin, ureSE1861, ureSE1863, ureSE1864, ureSE1865, ureSE1867;*
(iv) *Staphylococcus haemolyticus* including gene probes derived from *folQShaemolyt, m vaCShaem olyticus, mvaDShaemolyt, mvaK1Shaemolyticus, m vaSShaem olyticus, RNApolsigm;*
(v) *Staphylococcus lugdunensis* including gene probes derived from *agrB2Stalugd, agrC2Stalugd, agrCStalugd, slam Stalugd;*
(vi) *Staphylococcus warneri* including gene probes derived from *msrw1Stwar, nukMStwar, proDStwar, proMStwar, sigrpoStwar, tnpStwar;*
(vii) *Candida albicans* including gene probes derived from *ARG56, ASL43f, BGL2, CACHS3, CCT8, CDC37, CEF3, CHS1, CHS2, CHS4, CHS5, CHT1, CHT2, CHT4, CSA1, 5triphosphatase, AAF1, ADH1, ALS1, ALS7, EDT1, ELF, ESS1, FAL1, GAP1, GNA1, GSC1, GSL1, HIS1, HTS1, HWP1, HYR1, INT1a, KRE15f, KRE6, KRE9, MIG1, MLS1, MP65, NDE1, PFK2, PHR1, PHR2, PHR3, PRA1, PRS1, RBT1, RBT4, RHO1, RNR1, RPB7, RPL13, RVS167, SHA3, SKN1, SRB1, TCA1, TRP1, YAE1, YRB1, YST1exon2;*
(viii) *Enterococcus faecalis* including gene probes derived from *arcA, arcC, bkdA, cad, camE1, csrA, dacA, dfr, dhoD1a, ABC-eltA, agrBfs, agrCfs, dnaE, ebsA, ebsB, eep, efaR, gls24_glsB, gph, gyrAEf, metEf, mntHCb2, mob2, mvaD, mvaE, parC, pcfG, phoZ, polC, ptb, recS1, rpoN, tms, tyrDC, tyrS;*
(ix) *Enterococcus faecium* including gene probes derived from *bglB, bglR, bglS, efmA, efmB, efmC, mreC, mreD, mvaDEfaecium, mvaEEfaecium, mvaK1Efaecium, m vaK2Efaecium, mvaSEfaecium, orf3_4Efaeciumb, orf6_7Efaecium, orf7_8Efaecium, orf9_10Efaecium;*
(x) *Klebsiella pneumonia* including gene probes derived from *atsA, atsB, budC, citA, citW, citX, dalD, dalK, dalT, acoA, acoB, acoC, ahlK, fimK, glfKPN2, ItrA, mdcC, mdcF, mdcH, mrkA, mtrK, nifF, nifK, nifN, tyrP, ureA, wbbO, wza, wzb, wzmKPN2, wztKPN2, yojH, liac;*
(xi) *Klebsiella oxytoca* including gene probes derived from *cymA, cymD, cymE, cymH, cymI, cymJ, ddrA, fdt-1, fdt-2, fdt-3, gatY, hydH, masA, nasA, nasE, nasF, pehX, pelX, tagH, tagK, tagT;*
(xii) *Pseudomonas aeruginosa* including gene probes derived from *glpR, lasRb, OrfX, pa0260, pa0572, pa0625, pa0636, pa1046, pa1069, pa1846, pa3866, pa4082, pilAp, PilAp2, pilC, PstP, purK, uvrDII, vsml, vsmR, xcpX;*
(xiii) *Streptococcus pneumoniae* including gene probes derived from *cap1EStrpneu, cap1FStrpneu, cap1GStrpneu, cap3AStrpneu, cap3BStrpneu, celAStrpneu, celBStrpneu, cglAStrpneu, cglBStrpneu, cglCStrpneu, cglDStrpneu, cinA, cps14EStrpneum, cps14FStrpneum, cps14GStrpneum, cps14H-Strpneum, cps19aHStrpneum, cps19alStrpneum, cps19aKStrpneum, cps19f-GStrpneum, cps23fGStrpneum, dexB, dinF, 1760Strpneu, acyPStrpneu, endAStrpneu, exoAStrpneu, exp72, fnlAStrpneu, fnlBStrpneu, fnlCStrpneu, gct18Strpneum, hexB1, hftsHstrpneu, immunofrag1Strpneu, immunofrag-2Strpneu, immunofrag3Strpneu, kdtBStrpneu, lysAStrpneu, pcpBStrpneu, pflCStrpneu, plpA, prtA1Strpneu, pspC1Strpneu, pspC2, purRStrpneu, pyrDAStrpneum, SP0828Strpneu, SP0830Strpneu, SP0833Strpneu, SP0837_38-Strpneu, SP0839Strpneu, ugdStrpneu, uncC, vicXStrepneu, wchA6bStrpneum, wci4Strpneum, wciK4Strpneum, wciL4Strpneum, wciN6bStrpneum, wciO6b-Strpneum, wciP6bStrpneum, wciY18Strpneum, wzdbStrpneum, wze6b-Strpneum, wzy18Strpneum, wzy4Strpneum, wzy6bStrpneum, xpt;*
(xiv) *Streptococcus agalactiae* including gene probes derived from *cpsA1Strgal, cpsB1Strgal, cpsC1Strgal, cpsD1Strgal, cpsE1Strgal, cpsG1Strgal, cpslStragal, cpsJStragal, cpsKStragal, cpsMStragal, cpsYStragal, cylBStraga, cylEStraga, cylFStraga, cylHStraga, cyllStraga, cylJStraga, cylKStraga, 0487Straga, 0488Straga, 0493Straga, 0495Straga, 0498Straga, 0500Straga, 0502Straga, 0504Straga, folDStraga, neuA1Strgal, neuB1Strgal, neuC1Strgal, neuD1Strgal, recNStraga, ileSStraga;*
(xv) *Streptococcus pyogenes* including gene probes derived from *cyclStrpyog, fah_rph_hlo_Strpyog, int, int315.5, murEStrpyog, oppA, oppCStrpyog, oppD, SPy0382Strpyog, SPy0390Strpyog, SpyM3_1351, vicXStrpyog;*
(xvi) *Streptococcus viridans* including gene probes derived from *573Stprmut, 580SStprmut, 581_582SStprmut, 584SStprmut, dltAStrmut, dltBStrmut, dltCppx1Strmut, dltDStrmut, lichStrbov, lytRStprmut, lytSStprmut, pepQStrrmut, pflCStrmut, recNStprmut, ytqBStrmut;*
(xvii) *Proteus mirabilis* including gene probes derived from *atfA, atfB, atfC, ccmPrmi1, cyaPrmi, aad, flfB, flfD, flfN, flhD, floA, ftsK, gstB, hemCPrmi, hemDPrmi, hev, katA, Ipp1, menE, mfd, nrpA, nrpB, nrpG, nrpS, nrpT, nrpU, pat, pmfA, pmfC, pmfE, ppaA, rsbA, rsbC, speB, stmA, stmB, terA, terD, umoA, umoB, umoC, ureR, xerC, ygbA;*
(xviii) *Proteus vulgaris* including gene probes derived from *envZPrvu, frdC, frdD, infBPrvu, lad, tna2;* and/or
(b) virulence gene probes for
(i) *Staphylococcus aureus* including gene probes derived from *bsaE, bsaG, cap5h, cap5i, cap5j, cap5k, cap8H, cap8l, capBJ, cap8K, I-hld, I-hysA, I-IgGbg, EDIN, eta, etb, hglA, hglB, hglC, hla, hlb, lukF, lukS, NAG, sak, sea, seb, sec1, seg, seh, sel, setl5, set6, set7, set8, sprV8, tst, I-sdrC, I-sdrD, I-sdrE;*
(ii) *Escherichia coli* including gene probes derived from *b1202, eae, eltB, escR, escT, escU, espB, fes, fteA, hlyA, hlyB, iucA, iucB, iucC, papG, rfbE, shuA, SLTII, toxA-LTPA, VT2vaB;*
(iii) *Staphylococcus epidermidis* including gene probes derived from *gcaD, hld_orf5, icaC, icaD, icaR, psm_beta1and2, purR, spoVG, yabJ;*
(iv) *Staphylococcus haemolyticus* including gene probes derived from *lipShaemolyt;*
(v) *Staphylococcus lugdunensis* including gene probes derived from *fblStalugd, slushABCStalugd;*
(vi) *Staphylococcus warneri* including gene probes derived from *gehAStwar;*
(vii) *Candida albicans* including gene probes derived from *CCN1, CDC28, CLN2, CPH1, CYB1, EFG1, MNT1, RBF1, RBF1, RIM101, RIM8, SEC14, SEC4, TUP1, YPT1, ZNF1 CZF1 ;*
(viii) *Enterococcus faecalis* including gene probes derived from *asa1, asp1, cgh, cylA, cylB, cylI, cylL_cylS, cylM, ace, ef00108, ef00109, ef0011, ef00113, ef0012, ef0022, ef0031, ef0032, ef0040, ef0058, enlA, esa, esp, gelE, groEL, groES, rt1, sala, salb, sea1, sep1, vicK, yycH, yycI, yycJ;*
(ix) *Enterococcus faecium* including gene probes derived from *entA_entl, entD, entR, oep, sagA;*
(x) *Klebsiella pneumonia* including gene probes derived from *cim, aldA, hemly, pSL017, pSL020, rcsA, rmlC, rmlD, waaG, wbbD, wbbM, wbbN, wbdA, wbdC, wztKpn, yibD;*
(xi) *P. aeruginosa* including gene probes derived from *aprA, aprE, ctx, algB, algN, algR, ExoS, fpvA, lasRa, lipA, lipH, Orf159, Orf252, pchG, PhzA, PhzB, PLC, plcN, plcR, pvdD, pvdF, pyocinS1, pyocinS1im, pyocinS2, pys2, rbf303, rhlA, rhlB, rhlR, TnAP41, toxA;*
(xii) *Streptococcus pneumoniae* including gene probes derived from *igaStrpneu, lytA, nanA, nanBStrpneu, pcpCStrpneu, ply, prtAStrpneu, pspA, SP0834Strpneu, sphtraStrpneu, wciJStrpneu, wziyStrpneu, wzxStrpneu;*
(xiii) *Streptococcus agalactiae* including gene probes derived from *CAMPfactor, 0499Straga, hylStragal, lipStragal;*
(xiv) *Streptococcus pyogenes* including gene probes derived from *DNaselStrpyog, fba2Strpyog, fhuAStrpyog, fhuB1Strpyog, fhuDStrpyog, fhuGStrpyog, hylA, hylP, hylp2, oppB, ropB, scpAStrpyog, sloStrpyog, smez-Strpyog, sof, speA, speB2Strpyog, speCStrpyog, speJStrpyog, srtBStrpyog, srtCStrpyog, srtEStrpyog, srtFStrpyog, srtGStrpyog, srtlStrpyog, srtKStrpyog, srtRStrpyog, srtTStrpyog, vicKStrpyog;*
(xvi) *Streptococcus viridans* including gene probes derived from *hlyXStrmut, igaStrmitis, igaStrsanguis, perMStrmut;*
(xvii) *Proteus mirabilis* including gene probes derived from *flaA, laD, fliA, hpmA, hpmB, IpsPrmi, mrpA, mrpB, mrpC, mrpD, mrpE, mrpF, mrpG, mrpH, mrpI, mrpJ, patA, putA, uca, ureDPrmi, ureEPrmi, ureFPrmi, zapA, zapB, zapD, zapE;* and/or
(c) resistance gene probes derived from genes coding for
(i) beta-lactams resistance including gene probes derived from *blaIMP-7, meclSepid, blaOXA-10, blaB, ampC, I-blaR, blaOXA-32, bla-CTX-M-22, pbp2aStrpneu, blaSHV-1, blaOXA-2, blaRShaemolyt, blalMP-7, I-mecR, blaOXY, dacCStrpyog, femA, mecA, blalShaemolyt, blavim, pbp2b, pbp2prim eSepid, pbp2x, pbp3Saureuc, pbp4, pbp5Efaecium, pbpC, I-mecl, pbp1a, I-blal, blaTEM-106, blaOXY-KLOX, ftsWEF, fmhB, cumA, femBShaemolyt, blaPER-1, bla_FOX-3, blaA, psrb, fmhA, mecR1Sepid, blaZ, blaOXA-1, fox-6, blaPrmi;*
(ii) aminoglycosides resistance including gene probes derived from *aacA_aphDStwar, aacC1, aacC2, strB, aadA, aadB, aadD, aacA4, strA, aph-A3, aacC1, aacA4, aacA-aphD, I-spc, aphA3;*
(iii) macrolides-lincosamines-streptogramins resistance including gene probes derived from *ermC, linB, satSA, mdrSA, I-linA, ermB, ermA, satA, msrA, mphBM, mefA, mrx;*
(iv) trimethoprim resistance including gene probes derived from *dfrA, dfrStrpneu;*
(v) chloramphenicol resistance including gene probes derived from *cat, catEfaecium, cmlA5;*
(vi) tetracyclines resistance including gene probes derived from *tetAJ, tetL, tetM*
(vii) glycopeptides resistance including gene probes derived from *vanH(tn), vanA, vanHB2, vanR, vanRB2, vanS(tn), vanSB2, vanVllB2, ddl, ble, vanXB2, vanY(tn), vanYB2, vanB, vanZ(tn), vanC-2, vanX(tn);*
(viii) multiple target resistance including gene probes derived from *acrB, m exB, I-qacA, sull, sul, cadBStalugd, mexA, acrR, emeA, acrA, rtn, abcXStrpmut, qacEdelta1, elkT-abcA, I-cadA, albA, wzm, msrCb, nov, wzt, wbbl, norA23, mexR, arr2, mreA, I-cadC, uvrA;*
(ix) fungicide resistance, especially *C. albicans* fungicide resistance, including gene probes derived from *CRD2, CDR1, MET3, FET3, FTR2, MDR1-7, ERG11, SEC20.*

4. The DNA microarray of claim 2 or 3, wherein
(i) the array comprises the minimal number of species specific gene probes of group (a) which is sufficient for species identification, preferably the array comprises at least 2 different gene probes per target species of group (a); and/or
(ii) the array comprises the minimal number of virulence gene probes of group (b) sufficient for virulence determination, preferably at least 1 gene probe, more preferably at least 5 different gene probes per target species of group (b); and/or
(iii) the array comprises the minimal number of resistance gene probes of group (c) sufficient for determination of resistance, preferably at least 1 gene probe, more preferably at least 5 different gene probes of group (c); and/or
(iv) the DNA sequences are selected from the group consisting of SEQ I D NOs 1-918, complementary sequences thereto, addition mutants, deletion mutants, substitution mutants and homologues thereof.

5. The DNA microarray of claim 4, wherein
(i) the gene probes of group (a) are selected from SEQ I D NO: 1-99, 142-152, 174-199, 209-214, 216-219, 222-229, 231-291, 308-342, 377-393, 399-431, 449-490, 523-591, 606-639, 645-656, 687-701 , 706-749 and 776-781;
(ii) the gene probes of group (b) are selected from SEQ ID NO: 100-141, 153-173, 200-208, 215, 220-221, 230, 292-307, 343-376, 394-398, 432-448, 491-522, 592-605, 640-644, 657-686, 702-705, 750-775 and 782-784; and/or
(iii) the gene probes of group (c) are selected from SEQ ID NO:785-918, preferably from SEQ I D NO:785-882.

6. The DNA microarray of claim 4 or 5, which
(I) is suitable for identification of *Staphylococcus aureus* and comprises one or more or all of the gene probes of group (a) selected from SEQ ID NO:1-99, preferably comprises at least the gene probes represented by SEQ ID NO:71 and 68; and/or
(II) is suitable for identification of *Escherichia coli* and comprises one or more or all of the gene probes of group (a) selected from SEQ ID NO:142-152, preferably at least the gene probes represented by SEQ I D NO:143 and 149; and/or
(III) is suitable for identification of *Staphylococcus epidermidis* and comprises gene probes of group (a) selected from SEQ ID NO:174-199, preferably at least the gene probes represented by SEQ I D NO:177 and 184; and/or
(IV) is suitable for identification of *Staphylococcus haemolyticus* and comprises one or more or all of the gene probes of group (a) selected from SEQ ID NO:209-214, preferably at least the gene probes represented by SEQ I D NO:209 and 210; and/or
(V) is suitable for identification of *Staphylococcus lugdunensis* and comprises one or more or all of the gene probes of group (a) selected from SEQ ID NO:216-219, preferably at least the gene probes represented by SEQ ID NO:216 and 219; and/or
(VI) is suitable for identification of *Staphylococcus warneri* and comprises one or more or all of the gene probes of group (a) selected from SEQ I D NO: 224-229, preferably at least the gene probes represented by SEQ ID NO: 224 and 225; and/or
(VII) is suitable for identification of *Candida albicans* and comprises one or more or all of the gene probes of group (a) selected from SEQ ID NO:231-291, preferably at least the gene probes represented by SEQ ID NO:231 and 232; and/or
(VIII) is suitable for identification of *Enterococcus faecalis* and comprises one or more or all of the gene probes of group (a) selected from SEQ ID NO:308-342, preferably at least the gene probes represented by SEQ ID NO:308 and 310; and/or
(IX) is suitable for identification of *Enterococcus faecium* and comprises one or more or all of the gene probes of group (a) selected from SEQ ID NO:377-393, preferably at least the gene probes represented by SEQ ID NO:377 and 380; and/or
(X) is suitable for identification of *Klebsiella pneumonia* and comprises one or more or all of the gene probes of group (a) selected from SEQ ID NO:399-431, preferably at least the gene probes represented by SEQ ID NO:399 and 402; and/or
(XI) is suitable for identification of *Klebsiella oxytoca* and comprises one or more or all of the gene probes of group (a) selected from SEQ ID NO:449-469, preferably at least the gene probes represented by SEQ ID NO:449 and 455; and/or
(XII) is suitable for identification of *Pseudomonas aeruginosa* and comprises one or more or all of the gene probes of group (a) selected from SEQ ID NO:470-490, preferably at least the gene probes represented by SEQ ID NO:470 and 471; and/or
(XIII) is suitable for identification of *Streptococcus pneumoniae* and comprises one or more or all of the gene probes of group (a) selected from SEQ ID NO:523-591, preferably at least the gene probes represented by SEQ ID NO:523 and 524; and/or
(XIV) is suitable for identification of *Streptococcus agalactiae* and comprises one or more or all of the gene probes of group (a) selected from SEQ ID NO:606-639, preferably at least the gene probes represented by SEQ ID NO:606 and 619; and/or
(XV) is suitable for identification of *Streptococcus pyogenes* and comprises one or more or all of the gene probes of group (a) selected from SEQ ID NO:645-656, preferably at least the gene probes represented by SEQ ID NO:645 and 646; and/or
(XVI) is suitable for identification of *Streptococcus viridans* and comprises one or more or all of the gene probes of group (a) selected from SEQ ID NO:687-701, preferably at least the gene probes represented by SEQ ID NO:687 and 691 ; and/or
(XVII) is suitable for identification of *Proteus mirabilis* and comprises one or more or all of the gene probes of group (a) selected from SEQ ID NO:706-749, preferably at least the gene probes represented by SEQ ID NO:706 and 710; and/or
(XVIII) is suitable for identification of *Proteus vulgaris* and comprises one or more or all of the gene probes of group (a) selected from SEQ ID NO:776-781, preferably at least the gene probes represented by SEQ ID NO:776 and 777.

7. The DNA microarray of claim 6, which further comprises
(I) for the characterisation of *Staphylococcus aureus:* one or more or all of the gene probes of group (b) selected from SEQ ID NO:100-141, and/or of the gene probes of group (c) selected from SEQ I D NO:785-909; and/or
(II) for the characterisation of *Escherichia coli:* one or m ore or all of the gene probes of group (b) selected from SEQ I D NO:153-173, and/or of the gene probes of group (c) selected from SEQ I D NO:785-909; and/or
(III) for the characterisation of *Staphylococcus epidermidis:* one or more or all of the gene probes of group (b) selected from SEQ I D NO:200-208, and/or of the gene probes of group (c) selected from SEQ I D NO:785-909; and/or
(IV) for the characterisation of *Staphylococcus haemolyticus:* one or more or all of the gene probe of group (b) represented by SEQ I D NO:215, and/or of the gene probes of group (c) selected from SEQ I D NO:785-909; and/or
(V) for the characterisation of *Staphylococcus lugdunensis:* one or more or all of the gene probes of group (b) selected from SEQ I D NO:220-221, and/or of the gene probes of group (c) selected from SEQ ID NO:785-909; and/or
(VI) for the characterisation of *Staphylococcus warneri:* one or more or all of the gene probe of group (b) represented by SEQ I D NO:230, and/or of the gene probes of group (c) selected from SEQ I D NO:785-909; and/or
(VII) for the characterisation of *Candida albicans:* one or more or all of the gene probes of group (b) selected from SEQ I D NO:292-307, and/or of the gene probes of group (c) selected from SEQ ID NO:910-918; and/or
(VIII) for the characterisation of *Enterococcus faecalis:* one or more or all of the gene probes of group (b) selected from SEQ I D NO:343-376, and/or of the gene probes of group (c) selected from SEQ I D NO:785-909; and/or
(IX) for the characterisation of *Enterococcus faecium:* one or more or all of the gene probes of group (b) selected from SEQ I D NO:394-398, and/or of the gene probes of group (c) selected from SEQ I D NO:785-909; and/or
(X) for the characterisation of *Klebsiella pneumonia:* one or more or all of the gene probes of group (b) selected from SEQ ID NO:432-448, and/or of the gene probes of group (c) selected from SEQ I D NO:785-909; and/or
(XI) for the characterisation of *Klebsiella oxytoca:* one or more or all of the gene probes of group (c) selected from SEQ I D NO:785-909; and/or
(XII) for the characterisation of *Pseudomonas aeruginosa:* one or more or all of the gene probes of group (b) selected from SEQ ID NO:491-522, and/or of the gene probes of group (c) selected from SEQ I D NO:785-909; and/or
(XIII) for the characterisation of *Streptococcus pneumoniae:* one or more or all of the gene probes of group (b) selected from SEQ I D NO:592-605, and/or of the gene probes of group (c) selected from SEQ I D NO:785-909; and/or
(XIV) for the characterisation of *Streptococcus agalactiae:* one or more or all of the gene probes of group (b) selected from SEQ ID NO:640-644, and/or of the gene probes of group (c) selected from SEQ I D NO:785-909; and/or
(XV) for the characterisation of *Streptococcus pyogenes:* one or more or all of the gene probes of group (b) selected from SEQ ID NO:657-686, and/or of the gene probes of group (c) selected from SEQ I D NO:785-909; and/or
(XVI) for the characterisation of *Streptococcus viridans:* one or more or all of the gene probes of group (b) selected from SEQ ID NO:702-705, and/or of the gene probes of group (c) selected from SEQ I D NO:785-909; and/or
(XVII) for the characterisation of *Proteus mirabilis:* one or more or all of the gene probes of group (b) selected from SEQ ID NO:750-775, and/or of the gene probes of group (c) selected from SEQ ID NO:785-909; and/or
(XVIII) for the characterisation of *Proteus vulgaris:* one or more or all of the gene probes of group (b) selected from SEQ I D NO:782-784, and/or of the gene probes of group (c) selected from SEQ I D NO:785-909.

8. Use of the DNA microarray of any of claims 1 - 7 for *in vitro* identification and characterisation of microorganisms in a sample or in a clinical specimen, preferably for the diagnosis of bacteremia or sepsis.

9. An *in vitro* method for identification and characterisation of microorganisms in a sample or in a clinical specimen comprising
(a) isolating the total DNA from the sample or clinical specimen and labelling the DNA with a reporter molecule;
(b) applying the DNA thus obtained to the DNA microarray of anyone of claims 1-7 and hybridising the DNA with the gene probes of the DNA m icroarray; and
(c) detecting DNA bound to the DNA microrarray by determination of the amount of the reporter molecules bound to the array.

10. The method of claim 9,
(i) which is a method for diagnosis of bacteremia, fungemia or sepsis; and/or
(ii) wherein the clinical specimen is a positive blood culture; and/or
(iii) wherein the ratio of microbial DNA to total DNA isolated from said sample or clinical specim en is less than 100 %, preferably from 1% to 99%; and/or
(iv) wherein the reporter molecule is a fluorochrome; and/or
(v) wherein the determination of the amount of reporter molecules bound to the array is achieved by visualization of the reporter molecule; and/or
(vi) wherein the DNA isolated in step (a) is labelled and applied to the DNA microarray without prior amplification.

11. A kit for detection of microorgamisms in a sample or clinical specimen comprising the microarray of anyone of claims 1 to 7.
